(19) Europäisches Patentamt
European Patent Office
Office européen des brevets

(11) EP 3 578 659 B1

(12) **EUROPEAN PATENT SPECIFICATION**

(45) Date of publication and mention of the grant of the patent:
**01.11.2023 Bulletin 2023/44**

(21) Application number: **19171200.9**

(22) Date of filing: **27.03.2013**

(51) International Patent Classification (IPC):
***C12N 15/67*** *(2006.01)*

(52) Cooperative Patent Classification (CPC):
**C12N 15/67**

(54) **ARTIFICIAL NUCLEIC ACID MOLECULES FOR IMPROVED PROTEIN OR PEPTIDE EXPRESSION**

KÜNSTLICHE NUKLEINSÄUREMOLEKÜLE ZUR VERBESSERTEN PROTEIN- ODER PEPTIDEXPRESSION

MOLÉCULES D'ACIDE NUCLÉIQUE ARTIFICIELLES POUR UNE EXPRESSION PROTÉIQUE OU PEPTIDIQUE AMÉLIORÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.03.2012 PCT/EP2012/001336**
**08.06.2012 PCT/EP2012/002447**

(43) Date of publication of application:
**11.12.2019 Bulletin 2019/50**

(62) Document number(s) of the earlier application(s) in accordance with Art. 76 EPC:
**17001061.5 / 3 260 541**
**13716727.6 / 2 831 241**

(73) Proprietor: **CureVac SE**
**72076 Tübingen (DE)**

(72) Inventor: **Theß, Andreas**
**72706 Tübingen (DE)**

(74) Representative: **Graf von Stosch Patentanwaltsgesellschaft mbH**
**Prinzregentenstraße 22**
**80538 München (DE)**

(56) References cited:
**WO-A1-2012/019630**

- **LEDDA M ET AL: "Effect of 3'UTR length on the translational regulation of 5'-terminal oligopyrimidine mRNAs", GENE, ELSEVIER, AMSTERDAM, NL, vol. 344, 3 January 2005 (2005-01-03), pages 213-220, XP027872598, ISSN: 0378-1119 [retrieved on 2005-01-03]**
- **CHAKRABARTI PARTHA ET AL: "The mammalian target of rapamycin complex 1 regulates leptin biosynthesis in adipocytes at the level of translation: The role of the 5 '-untranslated region in the expression of leptin messenger ribonucleic acid", MOLECULAR ENDOCRINOLOGY, vol. 22, no. 10, October 2008 (2008-10), pages 2260-2267, XP002689369, ISSN: 0888-8809**
- **ZHU JIANFENG ET AL: "Binding of the La autoantigen to the 5' untranslated region of a chimeric human translation elongation factor 1A reporter mRNA inhibits translation in vitro", 31 October 2001 (2001-10-31), BIOCHIMICA ET BIOPHYSICA ACTA, VOL. 1521, NR. 1-3, PAGE(S) 19-29, XP002689370, ISSN: 0006-3002 * page 20, left-hand column, paragraph 3 - page 20, right-hand column, paragraph 1; figure 1 ***
- **V. IADEVAIA ET AL: "All translation elongation factors and the e, f, and h subunits of translation initiation factor 3 are encoded by 5'-terminal oligopyrimidine (TOP) mRNAs", RNA, vol. 14, no. 9, 1 January 2008 (2008-01-01), pages 1730-1736, XP055044697, ISSN: 1355-8382, DOI: 10.1261/rna.1037108**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SÁNCHEZ R ET AL: "The oligo(A) tail on histone mRNA plays an active role in translational silencing of histone mRNA during Xenopus oogenesis", MOLECULAR AND CELLULAR BIOLOGY, AMERICAN SOCIETY FOR MICROBIOLOGY, WASHINGTON, US, vol. 24, no. 6, 1 March 2004 (2004-03-01), pages 2513-2525, XP002616167, ISSN: 0270-7306, DOI: 10.1128/MCB.24.6.2513-2525.2004**
- **SHEN ET AL: "Structures required for poly(A) tail-independent translation overlap with, but are distinct from, cap-independent translation and RNA replication signals at the 3' end of Tobacco necrosis virus RNA", VIROLOGY, ACADEMIC PRESS,ORLANDO, US, vol. 358, no. 2, 25 January 2007 (2007-01-25), pages 448-458, XP005853995, ISSN: 0042-6822, DOI: 10.1016/J.VIROL.2006.08.054**


Remarks:

The file contains technical information submitted after the application was filed and not included in this specification

## Description

The invention relates to artificial nucleic acid molecules comprising a 5'UTR element as defined in the claims, an open reading frame, and optionally a histone stem-loop, a 3'UTR element, a poly(A) sequence and/or a polyadenylation signal. The invention relates further to a vector comprising a 5'UTR element as defined in the claims, an open reading frame and/or a cloning site, to a pharmaceutical composition or a cell comprising the artificial nucleic acid molecule or the vector, and to a kit comprising the artificial nucleic acid molecule, the vector and/or the pharmaceutical composition, preferably for use in the field of gene therapy and/or genetic vaccination.

Gene therapy and genetic vaccination belong to the most promising and quickly developing methods of modern medicine. They may provide highly specific and individual options for therapy of a large variety of diseases. Particularly, inherited genetic diseases but also autoimmune diseases, cancerous or tumour-related diseases as well as inflammatory diseases may be the subject of such treatment approaches. Also, it is envisaged to prevent (early) onset of such diseases by these approaches.

The main conceptual rational behind gene therapy is appropriate modulation of impaired gene expression associated with pathological conditions of specific diseases. Pathologically altered gene expression may result in lack or overproduction of essential gene products, for example, signalling factors such as hormones, housekeeping factors, metabolic enzymes, structural proteins or the like. Altered gene expression may not only be due to mis-regulation of transcription and/or translation, but also due to mutations within the ORF coding for a particular protein. Pathological mutations may be caused by e.g. chromosomal aberration, or by more specific mutations, such as point or frame-shift-mutations, all of them resulting in limited functionality and, potentially, total loss of function of the gene product. However, misregulation of transcription or translation may also occur, if mutations affect genes encoding proteins which are involved in the transcriptional or translational machinery of the cell. Such mutations may lead to pathological up- or down-regulation of genes which are - as such - functional. Genes encoding gene products which exert such regulating functions, may be, e.g., transcription factors, signal receptors, messenger proteins or the like. However, loss of function of such genes encoding regulatory proteins may, under certain circumstances, be reversed by artificial introduction of other factors acting further downstream of the impaired gene product. Such gene defects may also be compensated by gene therapy via substitution of the affected gene itself.

Genetic vaccination allows to evoke a desired immune response to selected antigens, such as characteristic components of bacterial surfaces, viral particles, tumour antigens or the like. Generally, vaccination is one of the pivotal achievements of modern medicine. However, effective vaccines are currently available only for a smaller number of diseases. Accordingly, infections that are not preventable by vaccination still affect millions of people every year.

Commonly, vaccines may be subdivided into "first", "second" and "third" generation vaccines. "First generation" vaccines are, typically, whole-organism vaccines. They are based on either live and attenuated or killed pathogens, e.g. viruses, bacteria or the like. The major drawback of live and attenuated vaccines is the risk for a reversion to life-threatening variants. Thus, although attenuated, such pathogens may still intrinsically bear unpredictable risks. Killed pathogens may not be as effective as desired for generating a specific immune response. In order to minimize these risks, "second generation" vaccines were developed. These are, typically, subunit vaccines, consisting of defined antigens or recombinant protein components which are derived from pathogens.

Genetic vaccines, i.e. vaccines for genetic vaccination, are usually understood as "third generation" vaccines. They are typically composed of genetically engineered nucleic acid molecules which allow expression of peptide or protein (antigen) fragments characteristic for a pathogen or a tumor antigen *in vivo.* Genetic vaccines are expressed upon administration to a patient and uptake by competent cells. Expression of the administered nucleic acids results in production of the encoded proteins. In the event these proteins are recognized as foreign by the patient's immune system, an immune response is triggered.

As can be seen from the above, both methods, gene therapy and genetic vaccination, are essentially based on the administration of nucleic acid molecules to a patient and subsequent transcription and/or translation of the encoded genetic information. Alternatively, genetic vaccination or gene therapy may also comprise methods which include isolation of specific body cells from a patient to be treated, subsequent *in vitro* transfection of such cells, and re-administration of the treated cells to the patient.

DNA as well as RNA may be used as nucleic acid molecules for administration in the context of gene therapy or genetic vaccination. DNA is known to be relatively stable and easy to handle. However, the use of DNA bears the risk of undesired insertion of the administered DNA-fragments into the patient's genome potentially resulting in loss of function of the impaired genes. As a further risk, the undesired generation of anti-DNA antibodies has emerged. Another drawback is the limited expression level of the encoded peptide or protein that is achievable upon DNA administration and its transcription/translation. Among other reasons, the expression level of the administered DNA will be dependent on the presence of specific transcription factors which regulate DNA transcription. In the absence of such factors, DNA transcription will not yield satisfying amounts of RNA. As a result, the level of translated peptide or protein obtained is limited.

By using RNA instead of DNA for gene therapy or genetic vaccination, the risk of undesired genomic integration and generation of anti-DNA antibodies is minimized or avoided. However, RNA is considered to be a rather unstable molecular species which may readily be degraded by ubiquitous RNAses.

*In vivo,* RNA-degradation contributes to the regulation of the RNA half-life time. That effect was considered and proven to fine tune the regulation of eukaryotic gene expression (Friedel et al., Conserved principles of mammalian transcriptional regulation revealed by RNA half-life, Nucleic Acid Research, 2009, 1-12). Accordingly, each naturally occurring mRNA has its individual half-life depending on the gene from which the mRNA is derived. It contributes to the regulation of the expression level of this gene. Unstable RNAs are important to realize transient gene expression at distinct points in time. However, long-lived RNAs may be associated with accumulation of distinct proteins or continuous expression of genes. *In vivo,* the half life of mRNAs may also be dependent on environmental factors, such as hormonal treatment, as has been shown, e.g., for insulin-like growth factor I, actin, and albumin mRNA (Johnson et al., Newly synthesized RNA: Simultaneous measurement in intact cells of transcription rates and RNA stability of insulin-like growth factor I, actin, and albumin in growth hormone-stimulated hepatocytes, Proc. Natl. Acad. Sci., Vol. 88, pp. 5287-5291, 1991).

For gene therapy and genetic vaccination, usually stable RNA is desired. This is, on the one hand, due to the fact that the product encoded by the RNA-sequence shall accumulate *in vivo.* On the other hand, the RNA has to maintain its structural and functional integrity when prepared for a suitable dosage form, in the course of its storage, and when administered. Thus, considerable attention was dedicated to provide stable RNA molecules for gene therapy or genetic vaccination in order to prevent them from being subject to early degradation or decay.

It has been reported that the G/C-content of nucleic acid molecules may influence their stability. Thus, nucleic acids comprising an increased amount of guanine (G) and/or cytosine (C) residues may be functionally more stable than nucleic acids containing a large amount of adenine (A) and thymine (T) or uracil (U) nucleotides. In this context, WO02/098443 provides a pharmaceutical composition containing an mRNA that is stabilised by sequence modifications in the translated region. Such a sequence modification takes advantage of the degeneracy of the genetic code. Accordingly, codons which contain a less favourable combination of nucleotides (less favourable in terms of RNA stability) may be substituted by alternative codons without altering the encoded amino acid sequence. This method of RNA stabilization is limited by the provisions of the specific nucleotide sequence of each single RNA molecule which is not allowed to leave the space of the desired amino acid sequence. Also, that approach is restricted to coding regions of the RNA.

As an alternative option for mRNA stabilisation, it has been found that naturally occurring eukaryotic mRNA molecules contain characteristic stabilising elements. For example, they may comprise so-called untranslated regions (UTR) at their 5'-end (5'UTR) and/or at their 3'-end (3'UTR) as well as other structural features, such as a 5'-cap structure or a 3'-poly(A) tail. Both, 5'UTR and 3'UTR are typically transcribed from the genomic DNA and are, thus, an element of the premature mRNA. Characteristic structural features of mature mRNA, such as the 5'-cap and the 3'-poly(A) tail (also called poly(A) tail or poly(A) sequence) are usually added to the transcribed (premature) mRNA during mRNA processing.

A 3'-poly(A) tail is typically a monotonous sequence stretch of adenine nucleotides added to the 3'-end of the transcribed mRNA. It may comprise up to about 400 adenine nucleotides.

It was found that the length of such a 3'-poly(A) tail is a potentially critical element for the stability of the individual mRNA.

Nearly all eukaryotic mRNAs end with such a poly(A) sequence that is added to their 3' end by the ubiquitous cleavage/polyadenylation machinery. The presence of a poly(A) sequence at the 3' end is one of the most recognizable features of eukaryotic mRNAs. After cleavage, most pre-mRNAs, with the exception of replication-dependent histone transcripts, acquire a polyadenylated tail. In this context, 3' end processing is a nuclear co-transcriptional process that promotes transport of mRNAs from the nucleus to the cytoplasm and affects the stability and the translation of mRNAs. Formation of this 3' end occurs in a two step reaction directed by the cleavage/polyadenylation machinery and depends on the presence of two sequence elements in mRNA precursors (pre-mRNAs); a highly conserved hexanucleotide AAUAAA (polyadenylation signal) and a downstream G/U-rich sequence. In a first step, pre-mRNAs are cleaved between these two elements. In a second step tightly coupled to the first step the newly formed 3' end is extended by addition of a poly(A) sequence consisting of 200-250 adenylates which affects subsequently all aspects of mRNA metabolism, including mRNA export, stability and translation (Dominski, Z. and W. F. Marzluff (2007), Gene 396(2): 373-90.).

The only known exception to this rule are the replication-dependent histone mRNAs which terminate with a histone stem-loop instead of a poly(A) sequence. Exemplary histone stem-loop sequences are described in Lopez *et al.* (Dávila López, M., & Samuelsson, T. (2008), RNA (New York, N.Y.), 14(1), 1-10. doi:10.1261/rna.782308.).

The stem-loops in histone pre-mRNAs are typically followed by a purine-rich sequence known as the histone downstream element (HDE). These pre-mRNAs are processed in the nucleus by a single endonucleolytic cleavage approximately 5 nucleotides downstream of the stem-loop, catalyzed by the U7 snRNP through base pairing of the U7 snRNA with the HDE.

Due to the requirement to package newly synthesized DNA into chromatin, histone synthesis is regulated in

concert with the cell cycle. Increased synthesis of histone proteins during S phase is achieved by transcriptional activation of histone genes as well as posttranscriptional regulation of histone mRNA levels. It could be shown that the histone stem-loop is essential for all posttranscriptional steps of histone expression regulation. It is necessary for efficient processing, export of the mRNA into the cytoplasm, loading onto polyribosomes, and regulation of mRNA stability.

In the above context, a 32 kDa protein was identified, which is associated with the histone stem-loop at the 3'-end of the histone messages in both the nucleus and the cytoplasm. The expression level of this stem-loop binding protein (SLBP) is cell-cycle regulated and is highest during S-phase when histone mRNA levels are increased. SLBP is necessary for efficient 3'-end processing of histone pre-mRNA by the U7 snRNP. After completion of processing, SLBP remains associated with the stem-loop at the end of mature histone mRNAs and stimulates their translation into histone proteins in the cytoplasm. (Dominski, Z. and W. F. Marzluff (2007), Gene 396(2): 373-90). Interestingly, the RNA binding domain of SLBP is conserved throughout metazoa and protozoa (Dávila López, M., & Samuelsson, T. (2008), RNA (New York, N.Y.), 14(1), 1-10. doi:10.1261/rna.782308) and it could be shown that its binding to the histone stem-loop sequence is dependent on the stem-loop structure and that the minimum binding site contains at least 3 nucleotides 5' and 2 nucleotides 3' of the stem-loop (Pandey, N. B., et al. (1994), Molecular and Cellular Biology, 14(3), 1709-1720 and Williams, A. S., & Marzluff, W. F., (1995), Nucleic Acids Research, 23(4), 654-662.).

Even though histone genes are generally classified as either "replication-dependent", giving rise to mRNA ending in a histone stem-loop, or "replacement-type", giving rise to mRNA bearing a poly(A)-tail instead, naturally occurring mRNAs containing both a histone stem-loop and poly(A) or oligo(A) 3' thereof have been identified in some very rare cases. Sanchez *et al.* examined the effect of naturally occurring oligo(A) tails appended 3' of the histone stem-loop of histone mRNA during Xenopus oogenesis using Luciferase as a reporter protein and found that the oligo(A) tail is an active part of the translation repression mechanism that silences histone mRNA during oogenesis and its removal is part of the mechanism that activates translation of histone mRNAs (Sanchez, R. and W. F. Marzluff (2004), Mol Cell Biol 24(6): 2513-25).

Furthermore, the requirements for regulation of replication dependent histones at the level of pre-mRNA processing and mRNA stability have been investigated using artificial constructs coding for the marker protein alpha globin, taking advantage of the fact that the globin gene contains introns as opposed to the intron-less histone genes. For this purpose constructs were generated in which the alpha globin coding sequence was followed by a histone stem-loop signal (histone stem-loop followed by the histone downstream element) and a polyadenylation signal (Whitelaw, E., et al. (1986). Nucleic Acids Research, 14(17), 7059-7070; Pandey, N. B., & Marzluff, W. F. (1987). Molecular and Cellular Biology, 7(12), 4557-4559; Pandey, N. B., et al. (1990). Nucleic Acids Research, 18(11), 3161-3170).

Also, it was shown that the 3'UTR of $\alpha$-globin mRNA may be an important factor for the well-known stability of $\alpha$-globin mRNA (Rodgers et al., Regulated $\alpha$-globin mRNA decay is a cytoplasmic event proceeding through 3'-to-5' exosome-dependent decapping, RNA, 8, pp. 1526-1537, 2002). The 3'UTR of $\alpha$-globin mRNA is obviously involved in the formation of a specific ribonucleoprotein-complex, the $\alpha$-complex, whose presence correlates with mRNA stability *in vitro* (Wang et al., An mRNA stability complex functions with poly(A)-binding protein to stabilize mRNA in vitro, Molecular and Cellular biology, Vol 19, No. 7, July 1999, p. 4552-4560).

Irrespective of factors influencing mRNA stability, effective translation of the administered nucleic acid molecules by the target cells or tissue is crucial for any approach using nucleic acid molecules for gene therapy or genetic vaccination. Along with the regulation of stability, also translation of the majority of mRNAs is regulated by structural features like UTRs, 5'-cap and 3'-poly(A) tail. In this context, it has been reported that the length of the poly(A) tail may play an important role for translational efficiency as well. Stabilizing 3'-elements, however, may also have an attenuating effect on translation.

Further regulative elements, which may have an influence on expression levels, may be found in the 5'UTR. For example, it has been reported that the 5'UTR of leptin mRNA is involved in the regulation of its translation (Chakrabarti P. et al., The Mammalian Target of Rapamycin Complex 1 Regulates Leptin Biosynthesis in Adipocytes at the Level of Translation: The Role of the 5'-Untranslated Region in the Expression of Leptin Messenger Ribonucleic Acid; Molecular Endocrinology, 2008, 22(10):2260-2267). It was further reported that the synthesis of particular proteins, e.g. proteins belonging to the translational apparatus, may be regulated not only at the transcriptional but also at the translational level. For example, translation of proteins encoded by so called 'TOP-genes' may be down-regulated by translational repression. Therein, the term 'TOP-gene' relates to a gene corresponding to an mRNA that is characterized by the presence of a TOP sequence at the 5'end and in most cases by a growth-associated translation regulation (Iadevaia et al., All translation elongation factors and the e, f, and h subunits of translation initiation factor 3 are encoded by 5'-terminal oligopyrimidine (TOP) mRNAs; RNA, 2008, 14:1730-1736). In this context, a TOP sequence - also called the '5'-terminal oligopyrimidine tract' - typically consists of a C residue at the cap site, followed by an uninterrupted sequence of up to 13 or even more pyrimidines (Avni et al., Vertebrate mRNAs with a 5'-terminal pyrimidine tract are Candidates for translational repression in quiescent cells: characterization of the translational cis-regulatory element, Molecular and Cellular Biology, 1994, p. 3822-3833). These TOP sequences are reported to be present in many mRNAs encoding components of the translational machinery and to be responsible for selective repression of the translation of these TOP

containing mRNAs due to growth arrest (Meyuhas, et al., Translational Control of Ribosomal Protein mRNAs in Eukaryotes, Translational Control. Cold Spring Harbor Monograph Archive. Cold Spring Harbor Laboratory Press, 1996, p. 363-388). The mechanism of translational regulation of TOP mRNAs is also the subject of another study, which reported that TOP mRNA translation may be modulated through binding of the La autoantigen to the 5'-untranslated region of said mRNA (Zhu J., Binding of the La autoantigen to the 5' untranslated region of a chimeric human translation elongation factor 1A reporter mRNA inhibits translation in vitro; Biochemica et Biophysica Acta, 2001, 1521:19-29). The results obtained in a further study indicate that the length of a 3'-UTR may also play a role in the translational regulation of 5'TOP mRNAs (Ledda, M. et al., Effect of 3' UTR length on the translational regulation of 5'-terminal oligopyrimidine mRNAs; Gene, 2005, 344:213-220).

It is the object of the invention to provide nucleic acid molecules which may be suitable for application in gene therapy and/or genetic vaccination. Particularly, it is the object of the invention to provide artificial nucleic acid molecules, such as an mRNA species, which provide for increased protein production from said artificial nucleic acid molecules, preferably which exhibit increased translational efficiency. Another object of the present invention is to provide nucleic acid molecules coding for such a superior mRNA species which may be amenable for use in gene therapy and/or genetic vaccination. It is a further object of the present invention to provide a pharmaceutical composition for use in gene therapy and/or genetic vaccination. In summary, it is the object of the present invention to provide improved nucleic acid species which overcome the above discussed disadvantages of the prior art by a cost-effective and straight-forward approach.

The object underlying the present invention is solved by the claimed subject-matter.

For the sake of clarity and readability the following definitions are provided. Any technical feature mentioned for these definitions may be read on each and every embodiment of the invention. Additional definitions and explanations may be specifically provided in the context of these embodiments.

Adaptive immune response: The adaptive immune response is typically understood to be an antigen-specific response of the immune system. Antigen specificity allows for the generation of responses that are tailored to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naive T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are, typically, responsible for presentation of an antigen to T-cells. Therein, presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Adaptive immune system: The adaptive immune system is essentially dedicated to eliminate or prevent pathogenic growth. It typically regulates the adaptive immune response by providing the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of such a cell will then inherit genes encoding the same receptor specificity, including the Memory B cells and Memory T cells that are the keys to long-lived specific immunity.

Adjuvant/adjuvant component: An adjuvant or an adjuvant component in the broadest sense is typically a pharmacological and/or immunological agent that may modify, e.g. enhance, the effect of other agents, such as a drug or vaccine. It is to be interpreted in a broad sense and refers to a broad spectrum of substances. Typically, these substances are able to increase the immunogenicity of antigens. For example, adjuvants may be recognized by the innate immune systems and, e.g., may elicit an innate immune response. "Adjuvants" typically do not elicit an adaptive immune response. Insofar, "adjuvants" do not qualify as antigens. Their mode of action is distinct from the effects triggered by antigens resulting in an adaptive immune response.

Antigen: In the context of the present invention "antigen" refers typically to a substance which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells.

Artificial nucleic acid molecule: An artificial nucleic acid molecule may typically be understood to be a nucleic acid molecule, e.g. a DNA or an RNA, that does not occur naturally. In other words, an artificial nucleic acid molecule may be understood as a non-natural nucleic acid molecule. Such nucleic acid molecule may be non-natural due to its individual sequence (which does not occur naturally) and/or due to other modifications, e.g. structural modifications of nucleotides which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term 'wild type' may be understood as a sequence occurring in nature. Further, the term 'artificial nucleic acid molecule' is not restricted to mean 'one single molecule' but is, typically, understood to comprise an ensemble of identical molecules. Accordingly, it may relate to a plurality of identical molecules contained in an aliquot.

Bicistronic RNA, multicistronic RNA: A bicistronic or multicistronic RNA is typically an RNA, preferably an mRNA, that typically may have two (bicistronic) or more (multicistronic) open reading frames (ORF). An open reading frame in this context is a sequence of codons that is translatable into a peptide or protein.

Carrier / polymeric carrier: A carrier in the context of the invention may typically be a compound that facilitates transport and/or complexation of another compound (cargo). A polymeric carrier is typically a carrier that is formed of a polymer. A carrier may be associated to its cargo by covalent or non-covalent interaction. A carrier may transport nucleic acids, e.g. RNA or DNA, to the target cells. The carrier may - for some embodiments - be a cationic component.

Cationic component: The term "cationic component" typically refers to a charged molecule, which is positively charged (cation) at a pH value typically from 1 to 9, preferably at a pH value of or below 9 (e.g. from 5 to 9), of or below 8 (e.g. from 5 to 8), of or below 7 (e.g. from 5 to 7), most preferably at a physiological pH, e.g. from 7.3 to 7.4. Accordingly, a cationic component may be any positively charged compound or polymer, preferably a cationic peptide or protein which is positively charged under physiological conditions, particularly under physiological conditions *in vivo.* A 'cationic peptide or protein' may contain at least one positively charged amino acid, or more than one positively charged amino acid, e.g. selected from Arg, His, Lys or Orn. Accordingly, 'polycationic' components are also within the scope exhibiting more than one positive charge under the conditions given.

5'-cap: A 5'-cap is an entity, typically a modified nucleotide entity, which generally 'caps' the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety.

Cellular immunity/cellular immune response: Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

DNA: DNA is the usual abbreviation for deoxy-ribonucleic-acid. It is a nucleic acid molecule, i.e. a polymer consisting of nucleotides. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerise by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA-sequence. DNA may be single stranded or double stranded. In the double stranded form, the nucleotides of the first strand typically hybridize with the nucleotides of the second strand, e.g. by A/T-base-pairing and G/C-base-pairing.

Epitope: Epitopes (also called 'antigen determinant') can be distinguished in T cell epitopes and B cell epitopes. T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Fragment of a sequence: A fragment of a sequence may typically be a shorter portion of a full-length sequence of e.g. a nucleic acid molecule or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 20%, preferably at least 30%, more preferably at least 40%, more preferably at least 50%, even more preferably at least 60%, even more preferably at least 70%, and most preferably at least 80% of the total (i.e. full-length) molecule from which the fragment is derived.

G/C modified: A G/C-modified nucleic acid may typically be a nucleic acid, preferably an artificial nucleic acid molecule as defined herein, based on a modified wild-type sequence comprising a preferably increased number of guanosine and/or cytosine nucleotides as compared to the wild-type sequence. Such an increased number may be generated by substitution of codons containing adenosine or thymidine nucleotides by codons containing guanosine or cytosine nucleotides. If the enriched G/C content occurs in a coding region of DNA or RNA, it makes use of the degeneracy of the genetic code. Accordingly, the codon substitutions preferably do not alter the encoded amino acid residues, but exclusively increase the G/C content of the nucleic acid molecule.

Gene therapy: Gene therapy may typically be understood to mean a treatment of a patient's body or isolated elements of a patient's body, for example isolated tissues/cells, by nucleic acids encoding a peptide or protein. It typically may comprise at least one of the steps of a) administration of a nucleic acid, preferably an artificial nucleic acid molecule as defined herein, directly to the patient - by whatever administration route - or *in vitro* to isolated cells/tissues of the patient, which results in transfection of the patient's cells either *in vivo*/*ex vivo* or *in vitro;* b) transcription and/or translation of the introduced nucleic acid molecule; and optionally c) re-administration of isolated, transfected cells to the patient, if the nucleic acid has not been administered directly to the patient.

Genetic vaccination: Genetic vaccination may typically be understood to be vaccination by administration of a nucleic acid molecule encoding an antigen or an immunogen or fragments thereof. The nucleic acid molecule may be administered to a subject's body or to isolated cells of a subject. Upon transfection of certain cells of the body or upon transfection of the isolated cells, the antigen or immunogen may be expressed by those cells and subsequently presented to the immune system, eliciting an adaptive, i.e. antigen-specific immune response. Accordingly, genetic vaccination typically comprises at least one of the steps of a) administration of a nucleic acid, preferably an artificial nucleic acid molecule as defined herein, to a subject, preferably a patient, or to isolated cells of a subject, preferably a patient, which usually results in transfection of the subject's cells either *in vivo* or *in vitro;* b) transcription and/or translation of the introduced nucleic acid molecule; and optionally c) re-administration of isolated, transfected cells to the subject, preferably the patient, if the nucleic acid has not been administered directly to the patient.

Heterologous sequence: Two sequences are typically understood to be 'heterologous' if they are not derivable from the same gene. I.e., although heterologous sequences may be derivable from the same organism, they naturally (in nature) do not occur in the same nucleic acid molecule, such as in the same mRNA.

Humoral immunity/humoral immune response: Humoral immunity refers typically to antibody production and optionally to accessory processes accompanying antibody production. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis

and pathogen elimination.

Immunogen: In the context of the present invention an immunogen may be typically understood to be a compound that is able to stimulate an immune response. Preferably, an immunogen is a peptide, polypeptide, or protein. In a particularly preferred embodiment, an immunogen in the sense of the present invention is the product of translation of a provided nucleic acid molecule, preferably an artificial nucleic acid molecule as defined herein. Typically, an immunogen elicits at least an adaptive immune response.

Immunostimulatory composition: In the context of the invention, an immunostimulatory composition may be typically understood to be a composition containing at least one component which is able to induce an immune response or from which a component which is able to induce an immune response is derivable. Such immune response may be preferably an innate immune response or a combination of an adaptive and an innate immune response. Preferably, an immunostimulatory composition in the context of the invention contains at least one artificial nucleic acid molecule, more preferably an RNA, for example an mRNA molecule. The immunostimulatory component, such as the mRNA may be complexed with a suitable carrier. Thus, the immunostimulatory composition may comprise an mRNA/carrier-complex. Furthermore, the immunostimulatory composition may comprise an adjuvant and/or a suitable vehicle for the immunostimulatory component, such as the mRNA.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

Immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Immunostimulatory RNA: An immunostimulatory RNA (isRNA) in the context of the invention may typically be an RNA that is able to induce an innate immune response. It usually does not have an open reading frame and thus does not provide a peptide-antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

Innate immune system: The innate immune system, also known as non-specific (or unspecific) immune system, typically comprises the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be, e.g., activated by ligands of Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. The pharmaceutical composition according to the present invention may comprise one or more such substances. Typically, a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system; and/or acting as a physical and chemical barrier to infectious agents.

Cloning site: A cloning site is typically understood to be a segment of a nucleic acid molecule, which is suitable for insertion of a nucleic acid sequence, e.g., a nucleic acid sequence comprising an open reading frame. Insertion may be performed by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Nucleic acid molecule: A nucleic acid molecule is a molecule comprising, preferably consisting of nucleic acid components. The term nucleic acid molecule preferably refers to DNA or RNA molecules. It is preferably used synonymous with the term "polynucleotide". Preferably, a nucleic acid molecule is a polymer comprising or consisting of nucleotide

monomers which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

Open reading frame: An open reading frame (ORF) in the context of the invention may typically be a sequence of several nucleotide triplets which may be translated into a peptide or protein. An open reading frame preferably contains a start codon, i.e. a combination of three subsequent nucleotides coding usually for the amino acid methionine (ATG or AUG), at its 5'-end and a subsequent region which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG or AUG) and which preferably terminates with a stop codon (e.g., TAA, TGA, or TAG or UAA, UAG, UGA, respectively). The open reading frame may be isolated or it may be incorporated in a longer nucleic acid sequence, for example in a vector or an mRNA. An open reading frame may also be termed 'protein coding region'.

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Pharmaceutically effective amount: A pharmaceutically effective amount in the context of the invention is typically understood to be an amount that is sufficient to induce a pharmaceutical effect, such as an immune response, altering a pathological level of an expressed peptide or protein, or substituting a lacking gene product, e.g., in case of a pathological situation.

Protein A protein typically comprises one or more peptides or polypeptides. A protein is typically folded into 3-dimensional form, which may be required for to protein to exert its biological function.

Poly(A) sequence: A poly(A) sequence, also called poly(A) tail or 3'-poly(A) tail, is typically understood to be a sequence of adenine nucleotides, e.g., of up to about 400 adenine nucleotides, e.g. from about 20 to about 400, preferably from about 50 to about 400, more preferably from about 50 to about 300, even more preferably from about 50 to about 250, most preferably from about 60 to about 250 adenine nucleotides. A poly(A) sequence is typically located at the 3'end of an mRNA. In the context of the present invention, a poly(A) sequence may be located within an mRNA or any other nucleic acid molecule, such as, e.g., in a vector, for example, in a vector serving as template for the generation of an RNA, preferably an mRNA, e.g., by transcription of the vector.

Polyadenylation: Polyadenylation is typically understood to be the addition of a poly(A) sequence to a nucleic acid molecule, such as an RNA molecule, e.g. to a premature mRNA.

Polyadenylation may be induced by a so called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

Restriction site: A restriction site, also termed 'restriction enzyme recognition site', is a nucleotide sequence recognized by a restriction enzyme. A restriction site is typically a short, preferably palindromic nucleotide sequence, e.g. a sequence comprising 4 to 8 nucleotides. A restriction site is preferably specifically recognized by a restriction enzyme. The restriction enzyme typically cleaves a nucleotide sequence comprising a restriction site at this site. In a double-stranded nucleotide sequence, such as a double-stranded DNA sequence, the restriction enzyme typically cuts both strands of the nucleotide sequence.

RNA, mRNA: RNA is the usual abbreviation for ribonucleic-acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. *In vivo,* transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino acid sequence of a particular peptide or protein. Typically, a mature mRNA comprises a 5'-cap, a 5'UTR, an open reading frame, a 3'UTR and a poly(A) sequence. Aside from messenger RNA, several non-coding types of RNA exist which may be involved in regulation of transcription and/or translation.

Sequence of a nucleic acid molecule: The sequence of a nucleic acid molecule is typically understood to be the particular and individual order, i.e. the succession of its nucleotides. The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an alignment.

Stabilized nucleic acid molecule: A stabilized nucleic acid molecule is a nucleic acid molecule, preferably a DNA or RNA molecule that is modified such, that it is more stable to disintegration or degradation, e.g., by environmental factors or enzymatic digest, such as by an exo- or endonuclease degradation, than the nucleic acid molecule without the modification. Preferably, a stabilized nucleic acid molecule in the context of the present invention is stabilized in a cell, such as a prokaryotic or eukaryotic cell, preferably in a mammalian cell, such as a human cell. The stabilization effect may also be exerted outside of cells, e.g. in a buffer solution etc., for example, in a manufacturing process for a pharmaceutical composition comprising the stabilized nucleic acid molecule.

Transfection: The term 'transfection' refers to the introduction of nucleic acid molecules, such as DNA or RNA (e.g. mRNA) molecules, into cells, preferably into eukaryotic cells. In the context of the present invention, the term 'transfection' encompasses any method known to the skilled person for introducing nucleic acid molecules into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. Preferably, the introduction is non-viral.

Vaccine: A vaccine is typically understood to be a prophylactic or therapeutic material providing at least one antigen, preferably an immunogen. The antigen or immunogen may be derived from any material that is suitable for vaccination. For example, the antigen or immunogen may be derived from a pathogen, such as from bacteria or virus particles etc., or from a tumor or cancerous tissue. The antigen or immunogen stimulates the body's adaptive immune system to provide an adaptive immune response.

Vector: The term 'vector' refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector which allows the convenient storage of a nucleic acid molecule, for example, of an mRNA molecule. Thus, the vector may comprise a sequence corresponding, e.g., to a desired mRNA sequence or a part thereof, such as a sequence corresponding to the open reading frame and the 3'UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

Vehicle: A vehicle is typically understood to be a material that is suitable for storing, transporting, and/or administering a compound, such as a pharmaceutically active compound. For example, it may be a physiologically acceptable liquid which is suitable for storing, transporting, and/or administering a pharmaceutically active compound.

3'-untranslated region (3'UTR): A 3'UTR is typically the part of an mRNA which is located between the protein coding region (i.e. the open reading frame) and the poly(A) sequence of the mRNA. A 3'UTR of the mRNA is not translated into an amino acid sequence. The 3'UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excise optional introns and modifications of the 3'-end, such as polyadenylation of the 3'-end of the pre-mature mRNA and optional endo- or exonuclease cleavages etc. In the context of the present invention, a 3'UTR corresponds to the sequence of

a mature mRNA which is located 3' to the stop codon of the protein coding region, preferably immediately 3' to the stop codon of the protein coding region, and which extends to the 5'-side of the poly(A) sequence, preferably to the nucleotide immediately 5' to the poly(A) sequence. The term "corresponds to" means that the 3'UTR sequence may be an RNA sequence, such as in the mRNA sequence used for defining the 3'UTR sequence, or a DNA sequence which corresponds to such RNA sequence. In the context of the present invention, the term "a 3'UTR of a gene", such as "a 3'UTR of an albumin gene", is the sequence which corresponds to the 3'UTR of the mature mRNA derived from this gene, i.e. the mRNA obtained by transcription of the gene and maturation of the pre-mature mRNA. The term "3'UTR of a gene" encompasses the DNA sequence and the RNA sequence of the 3'UTR.

5'-untranslated region (5'UTR): A 5'UTR is typically understood to be a particular section of messenger RNA (mRNA). It is located 5' of the open reading frame of the mRNA. Typically, the 5'UTR starts with the transcriptional start site and ends one nucleotide before the start codon of the open reading frame. The 5'UTR may comprise elements for controlling gene expression, also called regulatory elements. Such regulatory elements may be, for example, ribosomal binding sites or a 5'-Terminal Oligopyrimidine Tract. The 5'UTR may be posttranscriptionally modified, for example by addition of a 5'-cap. In the context of the present invention, a 5'UTR corresponds to the sequence of a mature mRNA which is located between the 5'cap and the start codon. Preferably, the 5'UTR corresponds to the sequence which extends from a nucleotide located 3' to the 5'-cap, preferably from the nucleotide located immediately 3' to the 5'cap, to a nucleotide located 5' to the start codon of the protein coding region, preferably to the nucleotide located immediately 5' to the start codon of the protein coding region. The nucleotide located immediately 3' to the 5'cap of a mature mRNA typically corresponds to the transcriptional start site. The term "corresponds to" means that the 5'UTR sequence may be an RNA sequence, such as in the mRNA sequence used for defining the 5'UTR sequence, or a DNA sequence which corresponds to such RNA sequence. In the context of the present invention, the term "a 5'UTR of a gene", such as "a 5'UTR of a TOP gene", is the sequence which corresponds to the 5'UTR of the mature mRNA derived from this gene, i.e. the mRNA obtained by transcription of the gene and maturation of the pre-mature mRNA. The term "5'UTR of a gene" encompasses the DNA sequence and the RNA sequence of the 5'UTR.

5'Terminal Oligopyrimidine Tract (TOP): The 5'terminal oligopyrimidine tract (TOP) is typically a stretch of pyrimidine nucleotides located at the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. Messenger RNA that contains a S'terminal oligopyrimidine tract is often referred to as 5' TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purin residue in said gene or mRNA. A TOP motif in the sense of the present invention is preferably located at the 5'end of a sequence which represents a 5'UTR or at the 5'end of a sequence which codes for a 5'UTR. Thus, preferably, a stretch of 3 or more pyrimidine nucleotides is called "TOP motif" in the sense of the present invention if this stretch is located at the 5'end of a respective sequence, such as the artificial nucleic acid molecule according to the present invention, the 5'UTR element of the artificial nucleic acid molecule according to the present invention, or the nucleic acid sequence which is derived from the 5'UTR of a TOP gene as described herein. In other words, a stretch of 3 or more pyrimidine nucleotides which is not located at the 5'-end of a 5'UTR or a 5'UTR element but anywhere within a 5'UTR or a 5'UTR element is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes with a tissue specific translational regulation are known. As defined above, the 5'UTR of a TOP gene corresponds to the sequence of a 5'UTR of a mature mRNA derived from a TOP gene, which preferably extends from the nucleotide located 3' to the 5'cap to the nucleotide located 5' to the start codon. A 5'UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'UTRs of TOP genes are generally rather short. The lengths of 5'UTRs of TOP genes may vary between 20 nucleotides up to 500 nucleotides, and are

typically less than about 200 nucleotides, preferably less than about 150 nucleotides, more preferably less than about 100 nucleotides. Exemplary 5'UTRs of TOP genes in the sense of the present invention are the nucleic acid sequences extending from the nucleotide at position 5 to the nucleotide located immediately 5' to the start codon (e.g. the ATG) in the sequences according to SEQ ID NOs. 1-1363, 1435, 1461 and 1462.

In a first aspect, the present invention relates to an artificial nucleic acid molecule comprising:

a. at least one 5'-untranslated region element (5'UTR element) which comprises or consists of a nucleic acid sequence which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit Vlc gene (COX6C), or a N-acylsphingosine amidohydrolase (acid ceramidase) 1 gene (ASAH1) or from a functional fragment or a functional variant thereof, wherein the functional fragment or variant fulfils at least one function of the naturally occuring 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit Vlc gene (COX6C), or a N-acylsphingosine amidohydrolase (acid ceramidase) 1 gene (ASAH1), wherein the at least one function is stabilizing the mRNA and/or stabilizing and/or prolonging protein production from an mRNA; and

b. at least one open reading frame (ORF),
wherein the 5'UTR element and the open reading frame are heterologous.

Further disclosed herein is also an artificial nucleic acid molecule comprising:

a. at least one 5'-untranslated region element (5'UTR element) which comprises or consists of a nucleic acid sequence which is derived from the 5'UTR of a TOP gene or which is derived from a variant of the 5'UTR of a TOP gene; and

b. at least one open reading frame (ORF).

Preferably, the artificial nucleic acid molecule further comprises:
c. at least one histone stem-loop.

Such an artificial nucleic acid molecule may be DNA or RNA. In case the artificial nucleic acid molecule is DNA it may be used for providing RNA, preferably an mRNA with a corresponding sequence as is described further below. The artificial nucleic acid molecule disclosed herein is particularly useful in gene therapy and genetic vaccination because it may provide increased and/or prolonged protein production of the protein encoded by the open reading frame.

In this context, the term '5'UTR element' preferably refers to a nucleic acid sequence which represents a 5'UTR of an artificial nucleic acid sequence, such as an artificial mRNA, or which codes for a 5'UTR of an artificial nucleic acid molecule. Thus, preferably, a 5'UTR element may be the 5'UTR of an mRNA, preferably of an artificial mRNA, or it may be the transcription template for a 5'UTR of an mRNA. Thus, a 5'UTR element preferably is a nucleic acid sequence which corresponds to the 5'UTR of an mRNA, preferably to the 5'UTR of an artificial mRNA, such as an mRNA obtained by transcription of a genetically engineered vector construct. Preferably, a 5'UTR element in the sense of the present disclosure functions as a 5'UTR or codes for a nucleotide sequence that fulfils the function of a 5'UTR. The term '5'UTR element' furthermore refers to a fragment or part of a 5'UTR of an artificial nucleic acid sequence, such as an artificial mRNA, or which codes for a part or fragment of a 5'UTR of an artificial nucleic acid molecule. This means that the 5'UTR element in the sense of the present disclosure may be comprised in the 5'UTR of an artificial nucleic acid sequence, such as an artificial mRNA, or which codes for a 5'UTR of an artificial nucleic acid molecule.

According to the present disclosure, the 5'UTR element comprises or consists of a nucleic acid sequence that is derived from the 5'UTR of a TOP gene or from a variant of the 5'UTR of a TOP gene.

The term 'a nucleic acid sequence which is derived from the 5'UTR of a TOP gene' preferably refers to a nucleic acid sequence which is based on the 5'UTR sequence of a TOP gene or on a fragment thereof. This term includes sequences corresponding to the entire 5'UTR sequence, i.e. the full length 5'UTR sequence of a TOP gene, and sequences corresponding to a fragment of the 5'UTR sequence of a TOP gene. Preferably, a fragment of a 5'UTR of a TOP gene consists of a continuous stretch of nucleotides corresponding to a continuous stretch of nucleotides in the full-length 5'UTR of a TOP gene, which represents at least 20%, preferably at least 30%, more preferably at least 40%, more preferably at least 50%, even more preferably at least 60%, even more preferably at least 70%, even more preferably at least 80%, and most preferably at least 90% of the full-length 5'UTR of a TOP gene. Such a fragment, in the sense of the present disclosure, is preferably a functional fragment as described herein. A particularly preferred fragment of a

5'UTR of a TOP gene is a 5'UTR of a TOP gene lacking the 5'TOP motif. The term '5'UTR of a TOP gene' preferably refers to the 5'UTR of a naturally occurring TOP gene.

The terms 'variant of the 5'UTR of a TOP gene' and 'variant thereof' in the context of a 5'UTR of a TOP gene refers to a variant of the 5'UTR of a naturally occurring TOP gene, preferably to a variant of the 5'UTR of a vertebrate TOP gene, preferably to a variant of the 3'UTR of a mammalian TOP gene, more preferably to a variant of the 3'UTR of a human TOP gene. Such variant may be a modified 5'UTR of a TOP gene. For example, a variant 5'UTR may exhibit one or more nucleotide deletions, insertions, additions and/or substitutions compared to the naturally occurring 5'UTR from which the variant is derived. Preferably, a variant of a 5'UTR of a TOP gene is at least 40%, preferably at least 50%, more preferably at least 60%, more preferably at least 70%, even more preferably at least 80%, even more preferably at least 90%, most preferably at least 95% identical to the naturally occurring 5'UTR the variant is derived from. Preferably, the variant is a functional variant as described herein.

The term "a nucleic acid sequence that is derived from a variant of the 5'UTR of a TOP gene" preferably refers to a nucleic acid sequence which is based on a variant of a 5'UTR sequence of a TOP gene or on a fragment thereof. This term includes sequences corresponding to the entire variant 5'UTR sequence, i.e. the full length variant 5'UTR sequence of a TOP gene, and sequences corresponding to a fragment of the variant 5'UTR sequence of a TOP gene. Preferably, a fragment of a variant of the 5'UTR of a TOP gene consists of a continuous stretch of nucleotides corresponding to a continuous stretch of nucleotides in the full-length variant 5'UTR of a TOP gene, which represents at least 20%, preferably at least 30%, more preferably at least 40%, more preferably at least 50%, even more preferably at least 60%, even more preferably at least 70%, even more preferably at least 80%, and most preferably at least 90% of the full-length variant 5'UTR of a TOP gene. Such a fragment of a variant, in the sense of the present disclosure, is preferably a functional fragment as described herein.

Thus, the 5'UTR element of the artificial nucleic acid molecule may comprise or consist of a fragment of the 5'UTR of a TOP gene or of a fragment of a variant of the 5'UTR of a TOP gene or may comprise or consist of the entire 5'UTR of a TOP gene or may comprise or consist of a variant of the 5'UTR of a TOP gene.

The 5'UTR element is preferably suitable for increasing protein production from the artificial nucleic acid molecule.

Preferably, the at least one 5'UTR element is functionally linked to the ORF. This means preferably that the 5'UTR element is associated with the ORF such that it may exert a function, such as a protein production increasing function for the protein encoded by the ORF or a stabilizing function on the artificial nucleic acid molecule. Preferably, the 5'UTR element and the ORF are associated in 5'→3' direction. Thus, preferably, the artificial nucleic acid molecule comprises the structure 5'-5'UTR element-(optional)linker-ORF-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

Preferably, the 5'UTR element and the at least one open reading frame are heterologous. The term 'heterologous' in this context means that the open reading frame and the 5'UTR element are not occurring naturally (in nature) in this combination. Preferably, the 5'UTR element is derived from a different gene than the open reading frame. For example, the ORF may be derived from a different gene than the 5'UTR element, e.g. encoding a different protein or the same protein but of a different species etc. For example, the ORF does not encode the protein which is encoded by the gene from which the 5'UTR element is derived.

In a preferred embodiment, the 5'UTR element, preferably the artificial nucleic acid molecule, does not comprise a complete TOP-motif or 5'TOP sequence. Thus, preferably, the 5'UTR element, preferably the artificial nucleic acid molecule, does not comprise the complete TOP-motif of the TOP gene from which the nucleic acid sequence of the 5'UTR element is derived. For example, the 5'UTR element or the artificial nucleic acid molecule disclosed herein may comprise 1, 2, 3, 4, 5, 6, 7, 8, 9, 10 or more pyrimidine residues of the TOP-motif or 5'TOP, preferably 1, 2, 3, 4, 5, 6, 7, 8, 9, 10 or more pyrimidine residues of the TOP-motif located at the 3'side of the TOP-motif or 5'TOP. For example, the 5'UTR element may comprise or consist of a nucleic acid sequence which starts at its 5'end with a pyrimidine residue that corresponds to residue 2, 3, 4, 5, 6, 7, 8, 9, 10 etc. of the TOP-motif or 5'TOP of the TOP gene from which the nucleic acid sequence of the 5'UTR element is derived.

It is particularly preferred that the 5'UTR element, preferably the artificial nucleic acid molecule disclosed herein, does not comprise a TOP-motif or 5'TOP. For example, the nucleic acid sequence of the 5'UTR element which is derived from a 5'UTR of a TOP gene starts at its 5'-end with a nucleotide located at position 1, 2, 3, 4, 5, 6, 7, 8, 9 or 10 downstream of the S'terminal oligopyrimidine tract (TOP) of the 5'UTR of a TOP gene. Position 1 downstream of the S'terminal oligopyrimidine tract (TOP) is the first purine based nucleotide 3' of the TOP-motif or the 5'TOP. Accordingly, position 1 downstream of the S'terminal oligopyrimidine tract is the first nucleotide following the 3'-end of the S'terminal oligopyrimidine tract in 5'-3'-direction. Likewise, position 2 downstream of the 5'TOP is the second nucleotide following the end of the 5'terminal oligopyrimidine tract, position 3 the third nucleotide and so on.

Therefore, the 5'UTR element preferably starts 5, 10, 15, 20, 25, 30, 40 or 50 nucleotides downstream of the transcriptional start site of the 5'UTR of a TOP gene.

In some embodiments, the nucleic acid sequence of the 5'UTR element which is derived from a 5'UTR of a TOP gene terminates at its 3'-end with a nucleotide located at position 1, 2, 3, 4, 5, 6, 7, 8, 9 or 10 upstream of the start codon (e.g. A(U/T)G) of the gene or mRNA it is derived from. Thus, the 5'UTR element does not comprise any part of the protein coding region. Thus, preferably, the only protein coding part of the artificial nucleic acid molecule disclosed herein is provided by the open reading frame. However, the open reading frame is preferably derived - as said above - from a gene that is different to the gene the 5'UTR element is derived from.

It is particularly preferred that the 5'UTR element does not comprise a start codon, such as the nucleotide sequence A(U/T)G. Thus, preferably, the artificial nucleic acid molecule will not comprise any upstream AUGs (or upstream ATGs in case it is a DNA molecule). In other words, in some embodiments, it may be preferred that the AUG or ATG, respectively, of the open reading frame is the only start codon of the artificial nucleic acid molecule.

Additionally, it is preferred that the 5'UTR element does not comprise an open reading frame. Thus, preferably, the artificial nucleic acid molecule will not comprise any upstream open reading frames.

The nucleic acid sequence which is derived from the 5'UTR of a TOP gene is derived from a eukaryotic TOP gene, preferably a plant or animal TOP gene, more preferably a chordate TOP gene, even more preferably a vertebrate TOP gene, most preferably a mammalian TOP gene, such as a human or mouse TOP gene.

Preferably, the artificial nucleic acid molecule disclosed herein comprises a 5'UTR element which comprises or consists of a nucleic acid sequence which is derived from the 5'UTR of a TOP gene or which is derived from a variant of the 5'UTR of a TOP gene, wherein the TOP gene is a plant or animal TOP gene, more preferably a chordate TOP gene, even more preferably a vertebrate TOP gene, most preferably a mammalian TOP gene, such as a human or mouse TOP gene and which optionally does not comprise the nucleotide sequence A(U!T)G and optionally does not comprise an open reading frame; at least one open reading frame (ORF); and optionally at least one histone-stem loop; wherein optionally the 5'UTR element does not comprise a TOP motif and wherein optionally the 5'UTR element starts at its 5'-end with a nucleotide located at position 1, 2, 3, 4, 5, 6, 7, 8, 9 or 10 downstream of the S'terminal oligopyrimidine tract (TOP) of the 5'UTR of a TOP gene and wherein further optionally the 5'UTR element which is derived from a 5'UTR of a TOP gene terminates at its 3'-end with a nucleotide located at position 1, 2, 3, 4, 5, 6, 7, 8, 9 or 10 upstream of the start codon (A(U/T)G) of the gene or mRNA it is derived from.For example, the 5'UTR element comprises or consists of a nucleic acid sequence which is derived from a nucleic acid sequence selected from the group consisting of SEQ ID NOs. 1-1363, SEQ ID NO. 1435, SEQ ID NO. 1461 or SEQ ID NO. 1462, from the homologs of SEQ ID NOs. 1-1363, SEQ ID NO. 1435, SEQ ID NO. 1461 or SEQ ID NO. 1462, from a variant thereof, or a corresponding RNA sequence. The term "homologs of SEQ ID NOs. 1-1363, SEQ ID NO. 1435, SEQ ID NO. 1461 or SEQ ID NO. 1462, " refers to sequences of other species than Homo sapiens (human) or Mus musculus (mouse), which are homologous to the sequences according to SEQ ID NOs. 1-1363, SEQ ID NO. 1435, SEQ ID NO. 1461 or SEQ ID NO. 1462. For example, SEQ ID NO. 1 relates to a sequence comprising the 5'UTR of Homo sapiens alpha 2 macroglobulin (A2M). A homolog of SEQ ID NO. 1 in the context of the present disclosure is any such sequence derived from an alpha 2 macroglobulin (A2M) gene or mRNA of another species than Homo sapiens (human), such as any vertebrate, preferably any mammalian alpha 2 macroglobulin (A2M) gene other than the human alpha 2 macroglobulin (A2M) gene, such as a mouse, rat, rabbit, monkey etc. alpha 2 macroglobulin (A2M) gene.

In a preferred embodiment, the 5'UTR element comprises or consists of a nucleic acid sequence which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, from the homologs of SEQ ID NOs. 1-1363, SEQ ID NO. 1435, SEQ ID NO. 1461 or SEQ ID NO. 1462, from a variant thereof, or a corresponding RNA sequence. It is particularly preferred that the 5' UTR element is derived from a nucleic acid sequence extending from the nucleotide position immediately 3' to the 5'-TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, from the homologs of SEQ ID NOs. 1-1363, SEQ ID NO. 1435, SEQ ID NO. 1461 or SEQ ID NO. 1462, from a variant thereof, or a corresponding RNA sequence.

In a preferred embodiment, the 5'UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence extending from nucleotide position 5 to the nucleotide position immediately 5' to the start codon (located at the 3' end of the sequences), e.g. the nucleotide position immediately 5' to the ATG sequence of a nucleic acid sequence, selected from SEQ ID NOs. 1-1363, SEQ ID NO. 1435, SEQ ID NO. 1461 or SEQ ID NO. 1462, or a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more

preferably of at least about 80%, more preferably of at least about 90%, even more preferably of at least about 95%, even more preferably of at least about 99% to a nucleic acid sequence extending from nucleotide position 5 to the nucleotide position immediately 5' to the start codon (located at the 3' end of the sequences), e.g. the nucleotide position immediately 5' to the ATG sequence of a nucleic acid sequence, selected from SEQ ID NOs. 1-1363, SEQ ID NO. 1435, SEQ ID NO. 1461 or SEQ ID NO. 1462, or a corresponding RNA sequence, wherein, preferably, the fragment is as described above, i.e. being a continuous stretch of nucleotides representing at least 20% etc. of the full-length 5'UTR the fragment is derived from.

Preferably, the 5'UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence extending from the nucleotide position immediately 3' to the S'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, or a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, or a corresponding RNA sequence, wherein, preferably, the fragment is as described above, i.e. being a continuous stretch of nucleotides representing at least 20% etc. of the full-length 5'UTR the fragment is derived from.

Preferably, the above defined fragments and variants (e.g. exhibiting at least 40% identity) of the sequences according to SEQ ID NOs. 1-1363, SEQ ID NO. 1435, SEQ ID NO. 1461 or SEQ ID NO. 1462, are functional fragments and variants as described herein.

Furthermore, the artificial nucleic acid molecule disclosed herein may comprise more than one 5'UTR elements as described above. For example, the artificial nucleic acid molecule disclosed herein may comprise one, two, three, four or more 5'UTR elements, wherein the individual 5'UTR elements may be the same or they may be different. For example, the artificial nucleic acid molecule disclosed herein may comprise two essentially identical 5'UTR elements as described above

In a particularly preferred embodiment, the 5'UTR element comprises or consists of a nucleic acid sequence which is derived from a 5'UTR of a TOP gene encoding a ribosomal protein or from a variant of a 5'UTR of a TOP gene encoding a ribosomal protein. Particularly preferred 5'UTR elements comprise or consist of a nucleic acid sequence which are derived from a 5' UTR of a TOP gene coding for a ribosomal protein selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, UBA52. Particularly preferred are nucleic acid sequences which are derived from a 5' UTR of TOP genes vertebrate coding for ribosomal proteins, such as mammalian ribosomal proteins e.g. human or mouse ribosomal proteins.

For example, the 5'UTR element comprises or consists of a nucleic acid sequence which is derived from a 5'UTR of a nucleic acid sequence according to any ofSEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360; a corresponding RNA sequence, a homolog thereof, or a variant thereof as described herein, preferably lacking the 5'TOP motif. As described above, the sequence extending from position 5 to the nucleotide immediately 5' to the ATG (which is located at the 3'end of the sequences) corresponds to the 5'UTR of said sequences.

Preferably, the 5'UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the 5'UTR of a nucleic acid sequence according to any of SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317,

1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360; or a corresponding RNA sequence, preferably lacking the 5'TOP motif, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the 5'UTR of a nucleic acid sequence according to SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360; or a corresponding RNA sequence, wherein, preferably, the fragment is as described above, i.e. being a continuous stretch of nucleotides representing at least 20% etc. of the full-length 5'UTR, preferably lacking the 5'TOP motif. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Preferably, the 5'UTR element comprises or consists of a nucleic acid sequence which is derived from a 5'UTR of a TOP gene encoding a ribosomal Large protein (RPL) or from a variant of a 5'UTR of a TOP gene encoding a ribosomal Large protein (RPL). For example, the 5'UTR element comprises or consists of a nucleic acid sequence which is derived from a 5'UTR of a nucleic acid sequence according to any of SEQ ID NOs: 67, 259, 1284-1318, 1344, 1346, 1348-1354, 1357, 1461 and 1462, a corresponding RNA sequence, a homolog thereof, or a variant thereof as described herein, preferably lacking the 5'TOP motif.

Preferably, the 5'UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the 5'UTR of a nucleic acid sequence according to any of SEQ ID NOs. 67, 259, 1284-1318, 1344, 1346, 1348-1354, 1357, and 1358 or a corresponding RNA sequence, preferably lacking the 5'TOP motif, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the 5'UTR of a nucleic acid sequence according to SEQ ID NOs: 67, 259, 1284-1318, 1344, 1346, 1348-1354, 1357, 1461 and 1462 or a corresponding RNA sequence, wherein, preferably, the fragment is as described above, i.e. being a continuous stretch of nucleotides representing at least 20% etc. of the full-length 5'UTR, preferably lacking the 5'TOP motif. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In a particularly preferred embodiment, the 5'UTR element comprises or consists of a nucleic acid sequence which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (1 7-beta) dehydrogenase 4 gene (HSD1 7B4), an androgen-induced 1 gene (AIG1), cytochrome c oxidase subunit VIc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit VIc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cytochrome c oxidase subunit Vic gene (COX6C), or a mammalian N-acylsphingosine amidohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit VIc gene (COX6C), or a human N-acylsphingosine amidohydrolase (acid ceramidase) 1 gene (ASAH1) or from a variant thereof, wherein preferably the 5'UTR element does not comprise the 5'TOP of said gene.

Accordingly, in a particularly preferred embodiment, the 5'UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 1368, or SEQ ID NOs 1452-1460 or a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 1368, or SEQ ID NOs 1452-1460 wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Preferably, the at least one 5'UTR element exhibits a length of at least about 20 nucleotides or more, preferably of at least about 30 nucleotides or more, more preferably of at least about 40 nucleotides or more. However, it may be preferred if the 5'UTR element of the artificial nucleic acid molecule is rather short. Accordingly, it may have a length of less than about 200, preferably less than 150, more preferably less than 100 nucleotides. For example, the 5'UTR may have a length of less than about 25, 30, 35, 40, 45, 50, 55, 60, 65, 70, 80, 85, 90, 95, 100, 105, 110, 115, 120, 125, 130, 135, 140, 145, 150, 155, 160, 165, 170, 175, 180, 185, 190, 195, 200 nucleotides Preferably, the 5'UTR element may have a length of about 20-25, 26-30, 31-35, 36-40, 41-45, 46-50, 51-55, 56-60, 61-65, 66-70, 71-80, 81-85, 86-90, 91-95, 96-100, 101-105, 106-110, 111-115, 116-120, 121-125, 126-130, 131-135, 136-140, 141-145, 146-150, 151-155, 156-160, 161-165, 166-170, 171-175, 176-180, 181-185, 186-190, 191-195, 196-200 or more nucleotides. For example, the 5'UTR element may have a length of about 20, 26, 31, 36, 41, 46, 51, 56, 61, 66, 71, 81, 86, 91, 96, 101, 106, 111, 116, 121, 126, 131, 136, 141, 146, 151, 156, 161, 166, 171, 176, 181, 186, 191 or 196 nucleotides. Preferably, the 5'UTR element may have a length from about 20, 30, 40 or more to less than about 200 nucleotides, more preferably from about 20, 30, 40 or more to less than about 150 nucleotides, most preferably from about 20, 30, 40 or more to less than about 100 nucleotides.

Preferred 5'UTR elements are derived from a 5' UTR of a TOP gene selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17,RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB or from a variant thereof.

In some embodiments, the artificial nucleic acid molecule comprises a 5'UTR element which comprises or consists of a nucleic acid sequence which is derived from the 5'UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB or from a variant thereof, wherein preferably the 5'UTR element does not comprise a TOP motif or the 5'TOP of said genes, and wherein optionally the 5'UTR element starts at its 5'-end with a nucleotide located at position 1, 2, 3, 4, 5, 6, 7, 8, 9 or 10 downstream of the S'terminal oligopyrimidine tract (TOP) and wherein further optionally the 5'UTR element which is derived from a 5'UTR of a TOP gene terminates at its 3'-end with a nucleotide located at position 1, 2, 3, 4, 5, 6, 7, 8, 9 or 10 upstream of the start codon (A(U/T)G) of the gene it is derived from.

In a particularly preferred embodiment, the artificial nucleic acid molecule further comprises a histone stem-loop.

Accordingly, it is particularly preferred that the artificial nucleic acid molecule disclosed herein comprises:

a. at least one 5'-untranslated region element (5'UTR element) which comprises or consists of a nucleic acid sequence which is derived from the 5'UTR of a TOP gene or which is derived from a variant of the 5'UTR of a TOP gene as described above;

b. at least one open reading frame (ORF); and

c. at least one histone stem-loop.

The combination of a 5'UTR element as described above with a histone stem-loop may have a particularly advantageous effect in providing prolonged and possibly also enhanced translation of an RNA molecule.

In the context of the present disclosure, such a histone stem-loop is typically derived from a histone gene and comprises an intramolecular base pairing of two neighbored entirely or partially reverse complementary sequences, thereby forming a stem-loop. A stem-loop can occur in single-stranded DNA or, more commonly, in RNA. The structure is also known as a hairpin or hairpin loop and usually consists of a stem and a (terminal) loop within a consecutive sequence, wherein the stem is formed by two neighbored entirely or partially reverse complementary sequences separated by a short sequence as sort of spacer, which builds the loop of the stem-loop structure. The two neighbored entirely or partially reverse complementary sequences may be defined as e.g. stem-loop elements stem1 and stem2. The stem loop is formed when these two neighbored entirely or partially reverse complementary sequences, e.g. stem-loop elements stem1 and stem2, form base-pairs with each other, leading to a double stranded nucleic acid sequence comprising an unpaired loop at its terminal ending formed by the short sequence located between stem-loop elements stem1 and stem2 on the consecutive sequence. The unpaired loop thereby typically represents a region of the nucleic acid which is not capable of base pairing with either of these stem-loop elements. The resulting lollipop-shaped structure is a key building block of many RNA secondary structures. The formation of a stem-loop structure is thus dependent on the stability of the resulting stem and loop regions, wherein the first prerequisite is typically the presence of a sequence that can fold back on itself to form a paired double strand. The stability of paired stem-loop elements is determined by the length, the number of mismatches or bulges it contains (a small number of mismatches is typically tolerable, especially in a long double strand), and the base composition of the paired region. In the context of the present disclosure, optimal loop length is 3-10 bases, more preferably 3 to 8, 3 to 7, 3 to 6 or even more preferably 4 to 5 bases, and most preferably 4 bases.

Preferably, the at least one histone stem-loop is functionally associated to the ORF. This means that the at least one histone stem-loop is preferably positioned within the artificial nucleic acid molecule such that it is able to exert its function, for example, its function of increasing protein production from the ORF or stabilizing the artificial nucleic acid molecule.

Preferably, the histone stem-loop is located 3' to the ORF. For example, the histone stem-loop may be connected to the 3'-end of the ORF directly or via a linker, for example via a stretch of nucleotides, such as 2, 4, 6, 8, 10 etc. nucleotides, e.g. comprising one or more restriction sites, or the histone stem-loop may be located within or between or downstream of other structures located 3' to the ORF, such as within a 3'UTR element, or between a poly(A) sequence and a poly(C) sequence, or down-stream of a poly(A) and/or a poly(C) sequence, or the histone stem-loop may be located at the 3'-end of the artificial nucleic acid molecule. The term "located at the 3'-end" also includes embodiments, wherein the histone stem-loop is followed in 3'-direction by few nucleotides which remain, e.g., after a restriction enzyme cleavage.

Preferably, the 5'UTR element and the histone stem-loop are chosen and positioned such that they exert at least an an additive, preferably a synergistic function on protein production from the ORF of the artificial nucleic acid molecule. Preferably, protein production from the ORF is increased at least in an additive, preferably in a synergistic way by the 5'UTR element and the histone stem-loop. Thus, the protein amount of the protein encoded by the ORF, such as a reporter protein, e.g. luciferase, at a certain time point after initiation of expression of the ORF, e.g. after transfection of a test cell line, is at least the same, preferably higher than what would be expected if the protein production increasing effects of the 5'UTR element and the histone stem-loop were purely additive. The additive, preferably synergistic effect may, for example, be determined by the following assay. Four artificial nucleic acid molecules, e.g. mRNAs, comprising an ORF encoding, e.g. a reporter protein such as luciferase, are generated, i.e. (i) lacking a 5'UTR element and a histone stem-loop (E0), (ii) containing a 5'UTR element derived from a 5'UTR of a TOP gene or of a variant thereof (E1), (iii) containing a histone stem-loop (E2), and (iv) containing both the 5'UTR element and the histone stem-loop (E1E2). Expression of the ORF contained in the artificial nucleic acid molecules is initiated, for example, by transfecting a test cell line, such as a mammalian cell line, e.g. HELA cells, or primary cells, e.g. HDF cells. Samples are taken at specific time points after initiation of expression, for example, after 6 hours, 24 hours, 48 hours, and/or 72 hours and the amount of protein produced by expression of the ORF contained in the artificial nucleic acid molecules is measured, for example, by an ELISA assay or a luciferase test, depending on the type of protein encoded by the ORF. The predicted amount of protein at a certain time point after initiation of expression obtained by construct E1E2 if the effects of the 3'UTR element and the 5'UTR element were purely additive (PPA) may be calculated as follows:

$$PPA_x = (E1_x - E0_x) + (E2_x - E0_x) + E0_x,$$

E0 is the amount of protein obtained for the construct E0 (lacking a 5'UTR and a histone stem-loop), E1 is the amount of protein obtained for the construct E1, E2 is the protein amount obtained for the construct E2, and x is the time point after initiation of expression. The effect on increasing protein production is additive if $E1E2_x = PPA_x$, and synergistic in the sense of the present disclosure if $E1E2_x > PPA_x$, wherein $E1E2_x$ is the amount of protein obtained from construct E1E2 at time point x. Preferably, E1E2 is at least 1.0, more preferably at least 1.1, more preferably at least 1.3, more preferably at least 1.5, even more preferably at least 1.75 times PPA at a given time point post initiation of expression, such as 24 hours, 48 hours or 72 hours post initiation of expression.

Thus, in a preferred embodiment, the present disclosure provides an artificial nucleic acid molecule comprising (a.) at least one 5'UTR element which comprises or consists of a nucleic acid sequence which is derived from the 5'UTR of a TOP gene or which is derived from a variant of the 5'UTR of a TOP geneas described above; (b.) at least one open reading frame (ORF); and (c.) at least one histone stem-loop as described herein, wherein the histone stem-loop and the 5'UTR element act at least additively, preferably synergistically to increase protein production from the ORF, preferably wherein E1E2 ≥ PPA, preferably E1E2 is at least PPA, more preferably E1E2 is at least 1.1 times PPA, more preferably E1 E2 is at least 1.3 times PPA, even more preferably wherein E1E2 is at least 1.5 times PPA at a given time point post initiation of expression of the ORF, for example 24 hours, preferably 48 hours post initiation of expression, wherein E1E2 and PPA are as described above.

Furthermore, it is preferred that the at least one histone stem-loop and the at least one 5'UTR element have an at least additive, preferably a synergistic effect on total protein production from the artificial nucleic acid molecule in a certain time span, such as within 24 hours, 48 hours, or 72 hours post initiation of expression. The additive, preferably the synergistic effect may be determined as described above, with the difference that the area under the curve (AUC) for the amount of protein over time predicted for E1E2 if the effects are additive is compared to the actual AUC measured for E1E2.

In a preferred embodiment disclosed herein, the artificial nucleic acid molecule disclosed herein comprises or codes for (a.) at least one 5'UTR element as described above, (b.) at least one open reading frame; and (c.) at least one histone stem-loop, preferably according to at least one of the following formulae (I) or (II):

formula (I) (stem-loop sequence without stem bordering elements):

$$\underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}$$

formula (II) (stem-loop sequence with stem bordering elements):

$$\underbrace{N_{1-6}}_{\substack{\text{stem1}\\ \text{bordering element}}}\ \underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}\ \underbrace{N_{1-6}}_{\substack{\text{stem2}\\ \text{bordering element}}}$$

wherein:

stem1 or stem2 bordering element $N_{1-6}$ is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;

stem1 $[N_{0-2}GN_{3-5}]$ is reverse complementary or partially reverse complementary with element stem2, and is a consecutive sequence of between 5 to 7 nucleotides;

wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof;

wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof, and

wherein G is guanosine or an analogue thereof, and may be optionally replaced by a cytidine or an analogue thereof, provided that its complementary nucleotide cytidine in stem2 is replaced by guanosine;

loop sequence [$N_{0-4}$(U/T)$N_{0-4}$] is located between elements stem1 and stem2, and is a consecutive sequence of 3 to 5 nucleotides, more preferably of 4 nucleotides;

wherein each $N_{0-4}$ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and wherein U/T represents uridine, or optionally thymidine;

stem2 [$N_{3-5}$C$N_{0-2}$] is reverse complementary or partially reverse complementary with element stem1, and is a consecutive sequence of between 5 to 7 nucleotides;

wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof;

wherein $N_{0-2}$ is a consecutive sequence of 0 to 2, preferably of 0 to 1, more preferably of 1 N, wherein each N is independently from another selected from a nucleotide selected from A, U, T, G or C or a nucleotide analogue thereof; and

wherein C is cytidine or an analogue thereof, and may be optionally replaced by a guanosine or an analogue thereof provided that its complementary nucleotide guanosine in stem! is replaced by cytidine;

wherein stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one ore more bases in one stem do not have a complementary base in the reverse complementary sequence of the other stem.

In the above context, a wobble base pairing is typically a non-Watson-Crick base pairing between two nucleotides. The four main wobble base pairs in the present context, which may be used, are guanosine-uridine, inosine-uridine, inosine-adenosine, inosine-cytidine (G-U/T, I-U/T, I-A and I-C) and adenosine-cytidine (A-C).

Accordingly, in the context of the present disclosure, a wobble base is a base, which forms a wobble base pair with a further base as described above. Therefore, non-Watson-Crick base pairing, e.g. wobble base pairing, may occur in the stem of the histone stem-loop structure disclosed herein.

In the above context, a partially reverse complementary sequence comprises maximally two, preferably only one mismatch in the stem-structure of the stem-loop sequence formed by base pairing of stem1 and stem2. In other words, stem1 and stem2 are preferably capable of (full) base pairing with each other throughout the entire sequence of stem1 and stem2 (100% of possible correct Watson-Crick or non-Watson-Crick base pairings), thereby forming a reverse complementary sequence, wherein each base has its correct Watson-Crick or non-Watson-Crick base pendant as a complementary binding partner. Alternatively, stem! and stem2 are preferably capable of partial base pairing with each other throughout the entire sequence of stem1 and stem2, wherein at least about 70%, 75%, 80%, 85%, 90%, or 95% of the 100% possible correct Watson-Crick or non-Watson-Crick base pairings are occupied with the correct Watson-Crick or non-Watson-Crick base pairings and at most about 30%, 25%, 20%, 15%, 10%, or 5% of the remaining bases are unpaired.

According to a preferred embodiment disclosed herein, the at least one histone stem-loop sequence (with stem bordering elements) of the nucleic acid sequence as defined herein comprises a length of about 15 to about 45 nucleotides, preferably a length of about 15 to about 40 nucleotides, preferably a length of about 15 to about 35 nucleotides, preferably a length of about 15 to about 30 nucleotides and even more preferably a length of about 20 to about 30 and most preferably a length of about 24 to about 28 nucleotides.

Furthermore, the at least one histone stem-loop sequence (without stem bordering elements) of the artificial nucleic acid molecule as defined herein may comprise a length of about 10 to about 30 nucleotides, preferably a length of about 10 to about 20 nucleotides, preferably a length of about 12 to about 20 nucleotides, preferably a length of about 14 to about 20 nucleotides and even more preferably a length of about 16 to about 17 and most preferably a length of about 16 nucleotides.

Preferably, the artificial nucleic acid molecule disclosed herein may comprise or code for (a.) at least one 5'UTR element as described above; at least one open reading frame; and (c.) at least one histone stem-loop sequence according to at least one of the following specific formulae (Ia) or (IIa):

formula (Ia) (stem-loop sequence without stem bordering elements):

$$\underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}$$

formula (IIa) (stem-loop sequence with stem bordering elements):

$$\underbrace{N_{2-5}}_{\substack{\text{stem1}\\ \text{bordering element}}}\ \underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}\ \underbrace{N_{2-5}}_{\substack{\text{stem2}\\ \text{bordering element}}}$$

wherein N, C, G, T and U are as defined above.

Preferably, the artificial nucleic acid molecule disclosed herein may comprise or code for (a.) at least one 5'UTR element as described above; at least one open reading frame; and (c.) at least one histone stem-loop sequence according to at least one of the following specific formulae (Ib) or (IIb):

formula (Ib) (stem-loop sequence without stem bordering elements):

$$\underbrace{[N_{1}GN_{4}]}_{\text{stem1}}\ \underbrace{[N_{2}(U/T)N_{1}]}_{\text{loop}}\ \underbrace{[N_{4}CN_{1}]}_{\text{stem2}}$$

formula (IIb) (stem-loop sequence with stem bordering elements):

$$\underbrace{N_{4-5}}_{\substack{\text{stem1}\\ \text{bordering element}}}\ \underbrace{[N_{1}GN_{4}]}_{\text{stem1}}\ \underbrace{[N_{2}(U/T)N_{1}]}_{\text{loop}}\ \underbrace{[N_{4}CN_{1}]}_{\text{stem2}}\ \underbrace{N_{4-5}}_{\substack{\text{stem2}\\ \text{bordering element}}}$$

wherein N, C, G, T and U are as defined above.

Preferably, the artificial nucleic acid molecule disclosed herein may comprise or code for (a.) at least one 5'UTR element as described above; at least one open reading frame; and (c.) at least one histone stem-loop sequence according to at least one of the following specific formulae (Ic) to (Ih) or (IIc) to (IIh), shown alternatively in its stem-loop structure and as a linear sequence representing histone stem-loop sequences as generated according to Example 1:

formula (Ic): (metazoan and protozoan histone stem-loop consensus sequence without stem bordering elements):

```
   N U
  N   N
   N-N
   N-N
   N-N
   N-N
   G-C
   N-N  (stem-loop structure)
```

NGNNNNNNUNNNNNCN
(linear sequence) (SEQ ID NO: 1391)

formula (IIc): (metazoan and protozoan histone stem-loop consensus sequence with stem bordering elements):

```
             N U
            N   N
             N-N
             N-N
             N-N
             N-N
             G-C
N*N*NNNN-NNNN*N*N*  (stem-loop structure)
```

N*N*NNNNGNNNNNNUNNNNNCNNNN*N*N*
(linear sequence) (SEQ ID NO: 1392)

formula (Id): (without stem bordering elements)

```
   N U
  N   N
   N-N
   N-N
   N-N
   N-N
   C-G
   N-N  (stem-loop structure)
```

NCNNNNNNUNNNNNGN
(linear sequence) (SEQ ID NO: 1393)

formula (IId): (with stem bordering elements)

```
            N U
           N   N
            N-N
            N-N
            N-N
            N-N
            C-G
N*N*NNNN-NNNN*N*N*  (stem-loop structure)
```

N*N*NNNNCNNNNNNUNNNNNGNNNN*N*N*
(linear sequence) (SEQ ID NO: 1394)

formula (Ie): (protozoan histone stem-loop consensus sequence without stem bordering elements)

```
 N U
N   N
 N-N
 N-N
 N-N
 N-N
 G-C
 D-H  (stem-loop structure)
```

DGNNNNNNUNNNNNCH
(linear sequence) (SEQ ID NO: 1395)

formula (IIe): (protozoan histone stem-loop consensus sequence with stem bordering elements)

```
          N U
         N   N
          N-N
          N-N
          N-N
          N-N
          G-C
N*N*NNND-HNNN*N*N*  (stem-loop structure)
```

N*N*NNNDGNNNNNNUNNNNNCHNNN*N*N*
(linear sequence) (SEQ ID NO: 1396)

formula (If): (metazoan histone stem-loop consensus sequence without stem bordering elements)

```
 N U
N   N
 Y-V
 Y-N
 B-D
 N-N
 G-C
 N-N  (stem-loop structure)
```

NGNBYYNNUNVNDNCN
(linear sequence) (SEQ ID NO: 1397)

formula (IIf): (metazoan histone stem-loop consensus sequence with stem bordering elements)

```
                N U
               N   N
                Y-V
                Y-N
                B-D
                N-N
                G-C
N*N*NNNN-NNNN*N*N*  (stem-loop structure)
```

N*N*NNNNGNBYYNNUNVNDNCNNNN*N*N*
(linear sequence) (SEQ ID NO: 1398)

formula (Ig): (vertebrate histone stem-loop consensus sequence without stem bordering elements)

```
 N U
D   H
 Y-A
 Y-B
 Y-R
 H-D
 G-C
 N-N  (stem-loop structure)
```

NGHYYYDNUHABRDCN
(linear sequence) (SEQ ID NO: 1399)

formula (IIg): (vertebrate histone stem-loop consensus sequence with stem bordering elements)

```
        N U
       D   H
        Y-A
        Y-B
        Y-R
        H-D
        G-C
N*N*HNNN-NNNN*N*H*  (stem-loop structure)
```

N*N*HNNNGHYYYDNUHABRDCNNNN*N*H*
(linear sequence) (SEQ ID NO: 1400)

formula (Ih): (human histone stem-loop consensus sequence (Homo sapiens) without stem bordering elements)

```
 Y U
D   H
 U-A
 C-S
 Y-R
 H-R
 G-C
 D-C  (stem-loop structure)
```

DGHYCUDYUHASRRCC
(linear sequence) (SEQ ID NO: 1401)

formula (IIh): (human histone stem-loop consensus sequence (Homo sapiens) with stem bordering elements)

```
          Y U
         D   H
          U-A
          C-S
          Y-R
          H-R
          G-C
N*H*AAHD-CVHB*N*H*  (stem loop structure)
```

N*H*AAHDGHYCUDYUHASRRCCVHB*N*H*
(linear sequence) (SEQ ID NO: 1402)

wherein in each of above formulae (Ic) to (Ih) or (IIc) to (IIh):
N, C, G, A, T and U are as defined above;
each U may be replaced by T;
each (highly) conserved G or C in the stem elements 1 and 2 may be replaced by its complementary nucleotide base C or G, provided that its complementary nucleotide in the corresponding stem is replaced by its complementary nucleotide in parallel; and/or
G, A, T, U, C, R, Y, M, K, S, W, H, B, V, D, and N are nucleotide bases as defined in the following Table:

| abbreviation | Nucleotide bases | remark |
|---|---|---|
| G | G | Guanine |
| A | A | Adenine |
| T | T | Thymine |
| U | U | Uracile |
| C | C | Cytosine |
| R | G or A | Purine |
| Y | T/U or C | Pyrimidine |
| M | A or C | Amino |
| K | G or T/U | Keto |
| 5 | G or C | Strong (3H bonds) |
| W | A or T/U | Weak (2H bonds) |
| H | A or C or T/U | Not G |
| B | G or T/U or C | Not A |
| V | G or C or A | Not T/U |
| D | G or A or T/U | Not C |
| N | G or C or T/U or A | Any base |
| * | Present or not | Base may be present or not |

In this context, it is particularly preferred that the histone stem-loop sequence according to at least one of the formulae (I) or (Ia) to (Ih) or (II) or (IIa) to (IIh) of the present disclosure is selected from a naturally occurring histone stem-loop sequence, more particularly preferred from protozoan or metazoan histone stem-loop sequences, and even more particularly preferred from vertebrate and mostly preferred from mammalian histone stem-loop sequences especially from human histone stem-loop sequences.

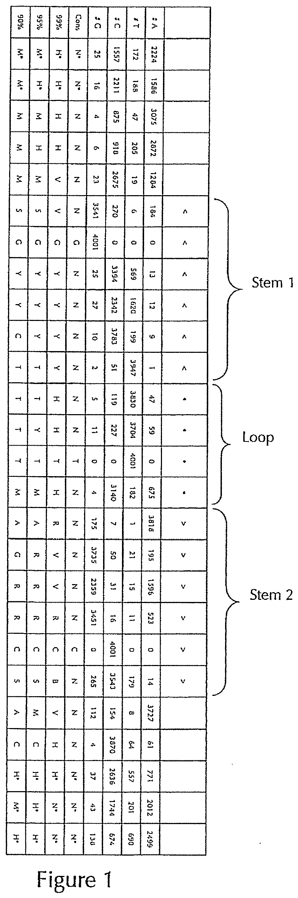

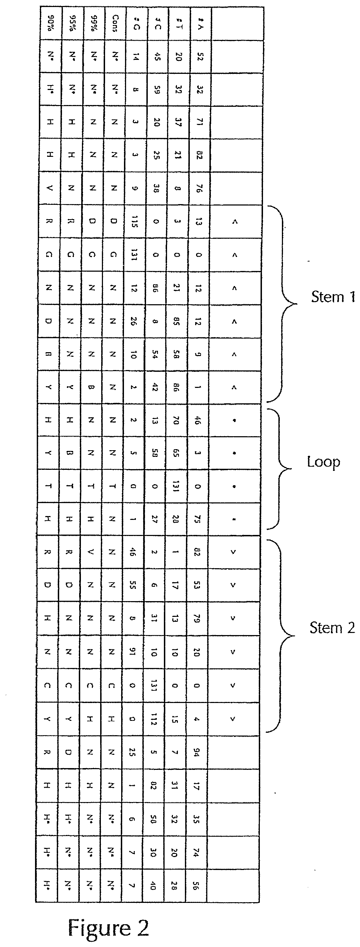

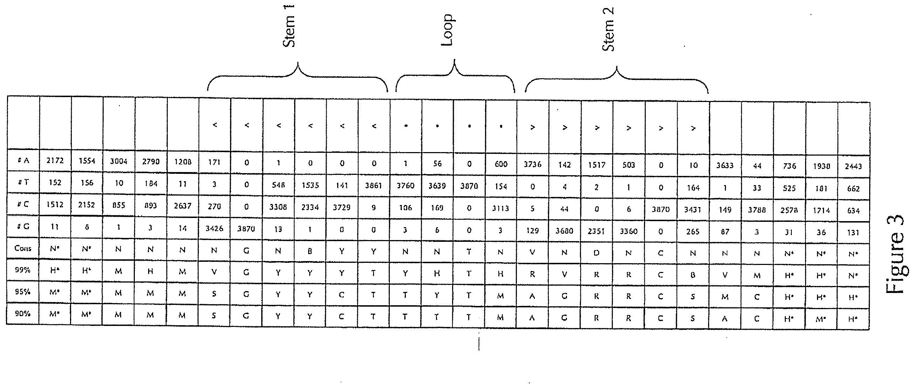

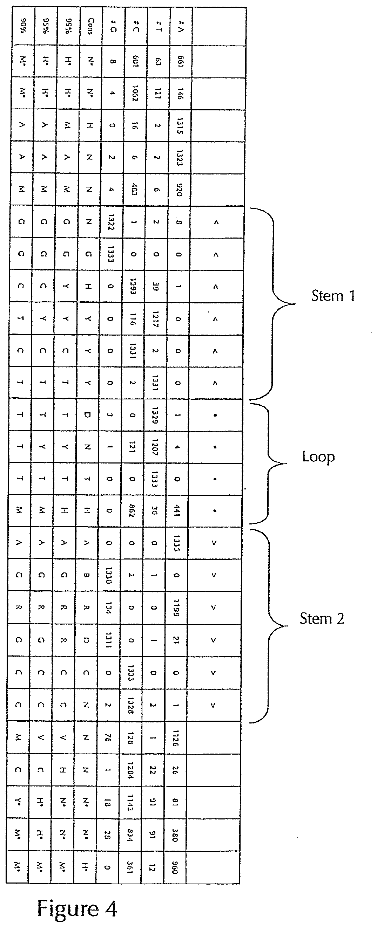

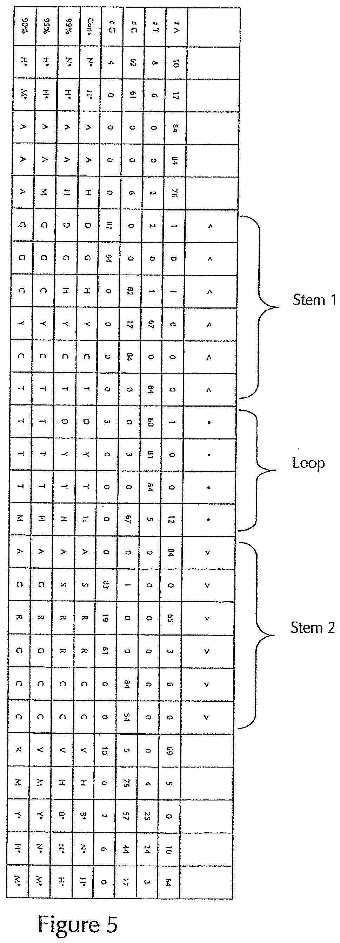

Further preferably, the histone stem-loop sequence according to at least one of the specific formulae (I) or (Ia) to (Ih) or (II) or (IIa) to (IIh) of the present disclosure is a histone stem-loop sequence comprising at each nucleotide position the most frequently occurring nucleotide, or either the most frequently or the second-most frequently occurring nucleotide of naturally occurring histone stem-loop sequences in metazoa and protozoa (Fig. 1), protozoa (Fig. 2), metazoa (Fig. 3), vertebrates (Fig. 4) and humans (Fig. 5) as shown in figures 1-5. In this context, it is particularly preferred that at least 80%, preferably at least 85%, or most preferably at least 90% of all nucleotides correspond to the most frequently occurring nucleotide of naturally occurring histone stem-loop sequences.

Further preferably, the histone stem-loop sequence according to at least one of the specific formulae (I) or (Ia) to (Ih) of the present disclosure may be selected from following histone stem-loop sequences or corresponding RNA sequences (without stem-bordering elements) representing histone stem-loop sequences as generated according to Example 1:

VGYYYYHHTHRVVRCB (SEQ ID NO: 1403 according to formula (Ic))
SGYYYTTYTMARRRCS (SEQ ID NO: 1404 according to formula (Ic))
SGYYCTTTTMAGRRCS (SEQ ID NO: 1405 according to formula (Ic))
DGNNNBNNTHVNNNCH (SEQ ID NO: 1406 according to formula (Ie))
RGNNNYHBTHRDNNCY (SEQ ID NO: 1407 according to formula (Ie))
RGNDBYHYTHRDHNCY (SEQ ID NO: 1408 according to formula (Ie))
VGYYYTYHTHRVRRCB (SEQ ID NO: 1409 according to formula (If))
SGYYCTTYTMAGRRCS (SEQ ID NO: 1410 according to formula (If))
SGYYCTTTTMAGRRCS (SEQ ID NO: 1411 according to formula (If))
GGYYCTTYTHAGRRCC (SEQ ID NO: 1412 according to formula (Ig))
GGCYCTTYTMAGRGCC (SEQ ID NO: 1413 according to formula (Ig))
GGCTCTTTTMAGRGCC (SEQ ID NO: 1414 according to formula (Ig))

DGHYCTDYTHASRRCC (SEQ ID NO: 1415 according to formula (Ih))
GGCYCTTTTHAGRGCC (SEQ ID NO: 1416 according to formula (Ih))
GGCYCTTTTMAGRGCC (SEQ ID NO: 1417 according to formula (Ih))

Furthermore, in this context, following histone stem-loop sequences (with stem bordering elements) as generated according to Example 1 according to one of specific formulae (II) or (IIa) to (IIh) and the corresponding RNA sequences are particularly preferred:

H*H*HHVVGYYYYHHTHRVVRCBVHH*N*N*
(SEQ ID NO: 1418 according to formula (IIc))

M*H*MHMSGYYYTTYTMARRRCSMCH*H*H*
(SEQ ID NO: 1419 according to formula (IIc))

M*M*MMMSGYYCTTTTMAGRRCSACH*M*H*
(SEQ ID NO: 1420 according to formula (IIc))

N*N*NNNDGNNNBNNTHVNNNCHNHN*N*N*
(SEQ ID NO: 1421 according to formula (IIe))

N*N*HHNRGNNNYHBTHRDNNCYDHH*N*N*
(SEQ ID NO: 1422 according to formula (Iie))

N*H*HHVRGNDBYHYTHRDHNCYRHH*H*H*
(SEQ ID NO: 1423 according to formula (Iie))

H*H*MHMVGYYYTYHTHRVRRCBVMH*H*N*
(SEQ ID NO: 1424 according to formula (IIf))

M*M*MMMSGYYCTTYTMAGRRCSMCH*H*H*
(SEQ ID NO: 1425 according to formula (IIf))

M*M*MMMSGYYCTTTTMAGRRCSACH*M*H*
(SEQ ID NO: 1426 according to formula (IIf))

H*H*MAMGGYYCTTYTHAGRRCCVHN*N*M*
(SEQ ID NO: 1427 according to formula (IIg))

H*H*AAMGGCYCTTYTMAGRGCCVCH*H*M*
(SEQ ID NO: 1428 according to formula (IIg))

M*M*AAMGGCTCTTTTMAGRGCCMCY*M*M*
(SEQ ID NO: 1429 according to formula (IIg))

N*H*AAHDGHYCTDYTHASRRCCVHB*N*H*
(SEQ ID NO: 1430 according to formula (llh))

H*H*AAMGGCYCTTTTHAGRGCCVMY*N*M*
(SEQ ID NO: 1431 according to formula (llh))

H*M*AAAGGCYCTTTTMAGRGCCRMY*H*M*
(SEQ ID NO: 1432 according to formula (IIh))

A particular preferred histone stem-loop sequence is the sequence according to SEQ ID NO: 1433 (CAAAGGCTCTTTTCAGAGCCACCA) or the corresponding RNA sequence.

Thus, in a particularly preferred embodiment, the artificial nucleic acid molecule disclosed herein comprises (a.) at least one 5'UTR element as described above; (b.) at least one open reading frame; and (c.) at least one histone-stem loop which comprises or consists of a sequence having a sequence identity of at least about 75%, preferably of

at least about 80%, preferably at least about 85%, more preferably at least about 90%, even more preferably at least about 95% to the sequence according to SEQ ID NO. 1433 or the corresponding RNA sequence, wherein preferably positions 6, 13 and 20 of the sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence are conserved, i.e. are identical to the nucleotides at positions 6, 13 and 20 of SEQ ID NO. 1433.

According to a further preferred embodiment, the artificial nucleic acid molecule disclosed herein comprises or codes for at least one histone stem-loop sequence showing at least about 80%, preferably at least about 85%, more preferably at least about 90%, or even more preferably at least about 95% sequence identity with the not to 100% conserved nucleotides in the histone stem-loop sequences according to at least one of specific formulae (I) or (Ia) to (Ih) or (II) or (IIa) to (IIh) or with a naturally occurring histone stem-loop sequence.

Furthermore, the artificial nucleic acid molecule disclosed herein may comprise more than one histone stem-loop as described herein. For example, the artificial nucleic acid molecule may comprise one, two, three, four or more histone stem-loops, wherein the individual histone stem-loops may be the same or they may be different. For example, the artificial nucleic acid molecule disclosed herein may comprise two histone stem-loops, wherein each histone stem-loop sequence may be selected from the group consisting of SEQ ID NOs. 1391-1433.

In a particularly preferred embodiment, the present disclosure provides an artificial nucleic acid molecule comprising:

a. at least one 5'-untranslated region element (5'UTR element) which comprises or consists of a nucleic acid sequence which is derived from the 5'UTR of a TOP gene or which is derived from a variant of the 5'UTR of a TOP gene as described above;

b. at least one open reading frame (ORF); and

c. at least one histone stem-loop, wherein preferably the sequence of the histone stem-loop is selected from the group consisting of sequences according to formulae (I) or (Ia) to (Ih) or (II) or (IIa) to (IIh), such as a sequence selected from the group consisting of SEQ ID NOs: 1391-1433, preferably from the group consisting of SEQ ID NOs. 1403-1433.

Thus, for example, the artificial nucleic acid molecule disclosed herein may comprise at least one 5'UTR element which is derived from the 5'UTR of a sequence selected from the group consisting of SEQ ID NOs. 1-1363, SEQ ID NO. 1435, SEQ ID NO. 1461 or SEQ ID NO. 1462, from a homolog thereof, from a variant thereof, or from a corresponding RNA sequence, such as a 5'UTR element which comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence extending from nucleotide position 5 to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, or a corresponding RNA sequence, or at least one 5'UTR element which comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence extending from nucleotide position 5 to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, or a corresponding RNA sequence, preferably lacking the 5'TOP motif, wherein, preferably, the fragment is as described above, i.e. being a continuous stretch of nucleotides representing at least 20% etc. of the full-length 5'UTR the fragment is derived from, (b.) at least one open reading frame, and (c.) at least one histone stem-loop sequence selected from the group consisting of SEQ ID NOs: 1391-1433, preferably from the group consisting of SEQ ID NOs: 1403-1433, preferably wherein the at least one histone histone-stem loop comprises or consists of a sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence, wherein preferably positions 6, 13 and 20 of the sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence are conserved, i.e. are identical to the nucleotides at positions 6, 13 and 20 of SEQ ID NO. 1433.

Furthermore, for example, the artificial nucleic acid molecule disclosed herein may comprise at least one 5'UTR element which comprises or consists of a nucleic acid sequence which is derived from a 5'UTR of a TOP gene encoding a ribosomal protein or from a variant of a 5'UTR of a TOP gene encoding a ribosomal protein, e.g. which comprises or consists of a nucleic acid sequence which is derived from a 5'UTR of a nucleic acid sequence according to any of SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360;, a corresponding RNA sequence, a homolog thereof, or a variant thereof as described herein, and at least one histone stem-loop sequence selected from the group consisting of SEQ ID NOs: 1391-1433, preferably from the group consisting of SEQ ID NOs: 1403-1433, preferably wherein the at least one histone-stem loop comprises or consists of a sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence, wherein preferably positions 6, 13 and 20 of the sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence are conserved, i.e. are identical to the nucleotides at positions 6, 13 and 20 of SEQ ID NO. 1433.

In a further embodiment, the artificial nucleic acid molecule disclosed herein may comprise at least one 5'UTR element which comprises or consists of a nucleic acid sequence which is derived from a 5'UTR of a TOP gene encoding a ribosomal Large protein or from a variant of a 5'UTR of a TOP gene encoding a ribosomal Large protein, e.g. which comprises or consists of a nucleic acid sequence which is derived from a 5'UTR of a nucleic acid sequence according to any of SEQ ID NOs: 67, 259, 1284-1318, 1344, 1346, 1348-1354, 1357, 1461 and 1462, a corresponding RNA sequence, a homolog thereof, or a variant thereof as described herein, and at least one histone stem-loop sequence selected from the group consisting of SEQ ID NOs: 1391-1433, preferably from the group consisting of SEQ ID NOs: 1403-1433, preferably wherein the at least one histone histone-stem loop comprises or consists of a sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence, wherein preferably positions 6, 13 and 20 of the sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence are conserved, i.e. are identical to the nucleotides at positions 6, 13 and 20 of SEQ ID NO. 1433.

As preferred example, the artificial nucleic acid molecule disclosed herein may comprise a 5'UTR element which comprises or consists of a nucleic acid sequence which has an identity of at least about 90%, preferably of at least about 95%, even more preferably of at least about 99% to the nucleic acid sequence according to SEQ ID NO: 1368 or SEQ ID NOs: 1452-1460 and a histone stem-loop sequence selected from the group consisting of SEQ ID NOs: 1403-1433, e.g. according to SEQ ID NO: 1433, or wherein the histone histone-stem loop comprises or consists of a sequence having a sequence identity of about 90%, even more preferably at least about 95% to the sequence according to SEQ ID NO. 1433 or the corresponding RNA sequence, wherein positions 6, 13 and 20 of the sequence having a sequence identity of at least about 90%, even more preferably at least about 95% to the sequence according to SEQ ID NO. 1433 or the corresponding RNA sequence are conserved, i.e. are identical to the nucleotides at positions 6, 13 and 20 of SEQ ID NO. 1433.

In some embodiments, the histone stem-loop sequence according to component (c.) is not derived from a mouse histone gene, e.g. from mouse histone gene H2A614. In one embodiment, the artificial nucleic acid molecule disclosed herein neither contains a mouse histone stem-loop sequence nor contains mouse histone gene H2A614. Furthermore, in one embodiment, the artificial nucleic acid molecule disclosed herein does not contain a stem-loop processing signal, more specifically, a mouse histone processing signal and, most specifically, does not contain mouse histone stem-loop processing signal H2kA614. Also, in one embodiment, the nucleic acid molecule disclosed herein may contain at least one mammalian histone gene. However, in one embodiment, the at least one mammalian histone gene is not Seq. ID No. 7 of WO 01/12824.

Preferably, the artificial nucleic acid molecule disclosed herein comprises no histone downstream element (HDE).

The term "histone downstream element (HDE)" refers to a purine-rich polynucleotide stretch of about 15 to 20 nucleotides 3' of naturally occurring stem-loops, which represents the binding site for the U7 snRNA involved in processing of histone pre-mRNA into mature histone mRNA. For example in sea urchins the HDE is CAAGAAAGA (Dominski, Z. and W. F. Marzluff (2007), Gene 396(2): 373-90).

Preferably, the artificial nucleic acid molecule disclosed herein further comprises a poly(A) sequence or a poly(A) signal.

Therefore, it is particularly preferred that the artificial nucleic acid molecule disclosed herein comprises or codes for (a.) at least one 5'UTR element as described above, (b.) at least one open reading frame, preferably encoding a peptide or protein; (c.) at least one histone stem-loopas described herein, and (d.) a poly(A) sequence or a polyadenylation signal.

A polyadenylation signal is defined herein as a signal which conveys polyadenylation to a (transcribed) mRNA by specific protein factors (e.g. cleavage and polyadenylation specificity factor (CPSF), cleavage stimulation factor (CstF), cleavage factors I and II (CF I and CF II), poly(A) polymerase (PAP)).

Preferably, the polyadenylation signal comprises the consensus sequence NN(UAT)ANA, with N = A or U, preferably AA(U/T)AAA or A(U/T)(U/T)AAA. Such consensus sequence may be recognised by most animal and bacterial cell-systems, for example by the polyadenylation-factors, such as cleavage/polyadenylation specificity factor (CPSF) cooperating with CstF, PAP, PAB2, CFI and/or CFII. The polyadenylation signal is preferably located within the artificial nucleic acid molecule such that the above described machinery is able to effect polyadenylation of the artificial nucleic acid molecule. For example, the polyadenylation signal may be located less than about 50 nucleotides, more preferably less than about 30 nucleotides, most preferably less than about 25 nucleotides, for example 21 nucleotides, upstream of the 3'-end of the artificial nucleic acid molecule.

Additionally or alternatively to the polyadenylation signal, in some embodiments, the artificial nucleic acid molecule disclosed herein may further comprise a poly(A) sequence. The length of the poly(A) sequence may vary. For example, the poly(A) sequence may have a length of about 20 adenine nucleotides up to about 400 adenine nucleotides, such as about 20 adenine nucleotides up to about 300 adenine nucleotides, preferably about 40 to about 200 adenine nucleotides, more preferably about 50 to about 100 adenine nucleotides, such as about 60, 70, 80, 90 or 100 adenine nucleotides. The term about refers to a deviation of + 10%.

The poly(A) sequence is preferably located 3' to the ORF. For example, the poly(A) sequence may be connected to the 3'-end of the ORF directly or via a linker, for example via a stretch of nucleotides, such as 2, 4, 6, 8, 10, 20 etc. nucleotides, such as via a linker of 1-50, preferably 1-20 nucleotides, e.g. comprising one or more restriction sites, or the poly(A) sequence may be located within or between or downstream of other structures located 3' to the ORF, such as between a 3'UTR element and a poly(C) sequence, or down-stream of a 3'UTR element and/or a poly(C) sequence, or the poly(A) sequence may be located at the 3'-end of the artificial nucleic acid molecule. The term "located at the 3'-end" also includes embodiments, wherein the poly(A) sequence is followed in 3'-direction by few nucleotides which remain, e.g. after a restriction enzyme cleavage.

It is particularly preferred that the artificial nucleic acid molecule disclosed herein comprises in 5'- to 3'-direction or codes in 5'- to 3'-direction for

(a.) at least one 5'UTR element derived from a TOP gene as described herein;
(b.) at least one open reading frame, preferably encoding a peptide or protein;
(c.) at least one histone stem-loop, optionally without a histone downstream element 3' to the histone stem-loop, as described herein; and
(d.) a poly(A) sequence and/or a polyadenylation signal.

In another particularly preferred embodiment, the nucleic acid molecule disclosed herein comprises in 5'- to 3'-direction or codes in 5'- to 3'-direction for:

(a.) at least one 5'UTR element derived from a TOP gene as described above;
(b.) at least one open reading frame, preferably encoding a peptide or protein;
(d.) a poly(A) sequence; and
(c.) at least one histone stem-loop as described herein.

Thus, the poly(A) sequence and the histone stem-loop of an artificial nucleic acid molecule disclosed herein may be positioned in any desired order from 5' to 3'. Particularly, the poly(A) sequence may be located 5' as well as 3' of the histone stem-loop.

Accordingly, in one embodiment, the artificial nucleic acid molecule disclosed herein comprises

(a.) at least one 5'-untranslated region element (5'UTR element) which comprises or consists of a nucleic acid sequence which is derived from the 5'UTR of a TOP gene or which is derived from a variant of the 5'UTR of a TOP gene;

(b.) at least one open reading frame (ORF);

(c.) a histone stem-loop; and

(d.) a poly(A) sequence and/or a polyadenylation signal, wherein the poly(A) sequence is located 5' or 3' of the histone stem-loop.

In a further preferred embodiment, the artificial nucleic acid molecule disclosed herein further comprises a poly(C) sequence. A poly(C) sequence in the context of the present disclosure preferably consists of about 10 to about 200 cytidine nucleotides, more preferably of about 10 to about 100 cytidine nucleotides, more preferably of about 10 to about 50 cytidine nucleotides, even more preferably of about 20 to about 40 cytidine nucleotides, such as about 20, about 25, about 30, about 35, about 40, preferably about 30 cytidine nucleotides. The poly(C) sequence is preferably located 3' to the ORF of the artificial nucleic acid molecule. For example, the poly(C) sequence may be connected to the 3'-end of the ORF directly or via a linker of a stretch of nucleotides, such as 2, 4, 6, 8, 10, 20 etc. nucleotides, such as via a linker of 1-50, preferably of 1-20 nucleitides, e.g. comprising one or more restriction sites, or the poly(C) sequence may be located within, between or downstream of any other structures located 3' to the ORF. For example, the poly(C) sequence may be part of a 3'UTR element or may be located between a poly(A) sequence and a histone stem-loop, or the poly(C) sequence may be located at the 3'-end of the artificial nucleic acid molecule. The term "located at the 3'-end" also includes embodiments, wherein the poly(C) sequence is followed in 3'-direction by a few nucleotides which remain, e.g., after a restriction enzyme cleavage. In a particularly preferred embodiment, the poly(C) sequence is located between a poly(A) sequence and a histone stem-loop.

In a particularly preferred embodiment, the poly(C) sequence is located 5' to the histone stem-loop.

Thus, in a particularly preferred embodiment, the artificial nucleic acid molecule according to the present application comprises the structure 5'-[ORF]-[optional linker]-[3'UTR element]-[optional linker]-[poly(A) sequence]-[optional linker]-[poly(C) sequence]-[optional linker]-[histone stem-loop]-3', wherein the optional linkers may be independently of each other present or absent and may be a stretch of 1-50 nucleotides, e.g. comprising one or more restriction sites.

In a further embodiment, the artificial nucleic acid molecule disclosed herein further comprises a 3'UTR element. Thus, in some embodiments, the artificial nucleic acid molecule disclosed herein may comprise at least one 5'UTR element as described above, at least one open reading frame, at least one histone stem-loop as described herein and at least one 3'UTR element as described herein. Furthermore, in some embodiments, the artificial nucleic acid disclosed herein may comprise at least one 5'UTR element as described above, at least one open reading frame, at least one histone stem-loop as described herein, at least one 3'UTR element as described herein, and a poly(A) sequence and/or a polyadenylation signal as described herein. In some embodiments, the histone stem-loop may be part of the 3'UTR element.

The term '3'UTR element' refers to a nucleic acid sequence which comprises or consists of a nucleic acid sequence that is derived from a 3'UTR or from a variant of a 3'UTR. A 3'UTR element in the sense of the present disclosure may represent the 3'UTR of an mRNA, e.g., in the event that the artificial nucleic acid molecule is an mRNA, or it may represent a sequence in a nucleic acid construct, such as a vector construct, that when transcribed represents the 3'UTR of the transcription product, such as the mRNA. Thus, in the sense of the present disclosure, preferably, a 3'UTR element may be the 3'UTR of an mRNA, preferably of an artificial mRNA, or it may be the transcription template for a 3'UTR of an mRNA. Thus, a 3'UTR element preferably is a nucleic acid sequence which corresponds to the 3'UTR of an mRNA, preferably to the 3'UTR of an artificial mRNA, such as an mRNA obtained by transcription of a genetically engineered vector construct. Preferably, the 3'UTR element fulfils the function of a 3'UTR or encodes a sequence which fulfils the function of a 3'UTR. The term '3UTR element' furthermore refers to a fragment or part of a 3'UTR of an artificial nucleic acid sequence, such as an artificial mRNA, or which codes for a part or fragment of a 3'UTR of an artificial nucleic acid molecule. This means that the 3'UTR element in the sense of the present disclosure may be comprised in the 3'UTR of an artificial nucleic acid sequence, such as an artificial mRNA, or which codes for a 3'UTR of an artificial nucleic acid molecule.

In the context of the present disclosure, the 3'UTR element may be derived from any 3'UTR of a gene or from a variant thereof, such as from a 3'UTR which is naturally associated with the ORF of the artificial nucleic acid molecule disclosed herein or any other 3'UTR of a naturally occurring gene or of a variant thereof.

Preferably, the 3'UTR element is functionally linked to the ORF. This means preferably that the 3'UTR element is associated with the ORF such that it may exert a function, such as a stabilizing function on the expression of the ORF or a stabilizing function on the artificial nucleic acid molecule. Preferably, the ORF and the 3'UTR element are associated in 5'→3' direction. Thus, preferably, the artificial nucleic acid molecule comprises the structure 5'-ORF-(optional)linker-3'UTR element-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

Preferably, the at least one 5'UTR element and the at least one 3'UTR element are functionally linked to the ORF. This means preferably that the 5'UTR element and the 3'UTR element are associated with the ORF such that they may exert a function, preferably in an additive, more preferably in a synergistic manner, such as a stabilizing function on the expression of the ORF, a protein production increasing function for the protein encoded by the ORF, or a stabilizing

function on the artificial nucleic acid molecule. Preferably, the 5'UTR element, the ORF, and the 3'UTR element are associated in 5'→3' direction. Thus, preferably, the artificial nucleic acid molecule comprises the structure 5'-5'UTR element-(optional)linker-ORF-(optional)linker-3'UTR element-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

In a particularly preferred embodiment, the 5'UTR element and the 3'UTR element are heterologous, e.g. preferably the 5'UTR and the 3'UTR are derived from different genes of the same or of different species. Preferably, the 3'UTR is not derived from the TOP gene the 5'UTR is derived from.

In a preferred embodiment, the 3'UTR element is chosen such that it exerts at least an additive, preferably a synergistic function with the 5'UTR element on the protein production from the ORF of the artificial nucleic acid molecule. Preferably, the protein production is increased in at least an additive, preferably a synergistic way by the 3'UTR element and the 5'UTR element. Thus, the protein amount of the protein encoded by the ORF, such as a reporter protein, e.g. luciferase, at a certain time point after initiation of expression of the ORF, e.g. after transfection of a test cell or cell line, is preferably at least the same, preferably higher than what would be expected if the protein production increasing effects of the 3'UTR element and the 5'UTR element were purely additive. The additive, preferably the synergistic effect may, for example, be determined by the following assay. Four artificial nucleic acid molecules, e.g. mRNAs, comprising an ORF encoding, e.g. a reporter protein such as luciferase, are generated, i.e. (i) lacking UTR elements (E0), (ii) containing a 5'UTR element derived from a 5'UTR of a TOP gene or of a variant thereof (E1), (iii) containing a test 3'UTR element (E2), and (iv) containing both the 5'UTR element and the test 3'UTR element (E1 E2). Expression of the ORF contained in the artificial nucleic acid molecules is initiated, for example, by transfecting a test cell line, such as a mammalian cell line, e.g. HELA cells, or primary cells, e.g. HDF cells. Samples are taken at specific time points after initiation of expression, for example, after 6 hours, 24 hours, 48 hours, and 72 hours and the amount of protein produced by expression of the ORF contained in the artificial nucleic acid molecules is measured, for example, by an ELISA assay or a luciferase test, depending on the type of protein encoded by the ORF. The predicted amount of protein at a certain time point after initiation of expression obtained by construct E1 E2 if the effects of the 3'UTR element and the 5'UTR element were purely additive (PPA) may be calculated as follows:

$$PPA_x = (E1_x - E0_x) + (E2_x - E0_x) + E0_x,$$

E0 is the amount of protein obtained for the construct E0 (lacking UTRs), E1 is the amount of protein obtained for the construct E1, E2 is the protein amount obtained for the construct E2, and x is the time point after initiation of expression. The effect on increasing protein production is additive if $E1E2_x = PPA_x$ and synergistic in the sense of the present disclosure if $E1E2_x > PPA_x$, wherein $E1E2_x$ is the amount of protein obtained from construct E1E2 at time point x. Preferably, E1E2 is at least 1.0, preferably at least 1.1, more preferably at least 1.3, more preferably at least 1.5, even more preferably at least 1.75 times PPA at a given time point post initiation of expression, such as 24 hours, 48 hours or 72 hours post initiation of expression.

Thus, in a preferred embodiment, the present disclosure provides an artificial nucleic acid molecule comprising (a.) at least one 5'UTR element which comprises or consists of a nucleic acid sequence which is derived from the 5'UTR of a TOP gene or which is derived from a variant of the 5'UTR of a TOP gene; (b.) at least one open reading frame (ORF); (c.) at least one histone stem-loop, and at least one 3'UTR element, wherein preferably the 3'UTR element and the 5'UTR element act at least additively, preferably synergistically to increase protein production from the ORF, preferably wherein E1E2 ≥ PPA, preferably E1E2 is at least 1.0 times PPA, preferably E1E2 is at least 1.1 times PPA, more preferably E1E2 is at least 1.3 times PPA, even more preferably wherein E1E2 is at least 1.5 times PPA at a given time point post initiation of expression of the ORF, for example 24 hours, preferably 48 hours post initiation of expression, wherein E1E2 and PPA are as described above.

Furthermore, it is preferred that the 3'UTR element and the 5'UTR element have at least an additive, preferably a synergistic effect on the total protein production from the artificial nucleic acid molecule in a certain time span, such as within 24 hours, 48 hours, or 72 hours post initiation of expression. The additive or the synergistic effect may be determined as described above, with the difference that the area under the curve (AUC) for the amount of protein over time predicted for E1E2 if the effects were purely additive is compared to the actual AUC measured for E1 E2.

In a preferred embodiment, the 3'UTR element comprises or consists of a nucleic acid sequence which is derived from the 3'UTR of a stable mRNA or from a variant of the 3'UTR of a stable mRNA. Thus, in a preferred embodiment, the 3'UTR element comprises or consists of a sequence which is derived from a gene providing a stable mRNA or from a variant of a 3'UTR of a gene providing a stable mRNA. The term "stable mRNA", preferably refers to mRNAs which exhibit a longer half-life in mammalian cells than the average half-life of mRNA molecules in mammalian cells. Preferably, a stable mRNA in the sense of the present application refers to an mRNA which exhibits a half-life of more than 5 hours, preferably more than 8 hours, in a mammalian cell, such as in a mammalian cell line, e.g. in HELA

cells, or in primary cells, e.g. in HDF cells, preferably determined by using a transcription inhibitor such as actinomycin D.

For example, the half-life of an mRNA in mammalian cells, such as HELA or HDF cells, may be determined by culturing the cells in presence of a transcription inhibitor, e.g. actinomycin D, 5,6-dichloro-1-β-D-ribofuranosylbenzimidazole (DRB), or α-amanitin, harvesting the cells at different time points after inhibition of transcription, and determining the amount of the mRNA present in the cell samples by methods well known to the person skilled in the art, e.g. by quantitative RT-PCR. The half-life of a particular mRNA may be calculated based on the amounts of the particular mRNA measured at the different time points post inhibition of transcription. Alternatively, pulse-chase methods, e.g. using radioactively labelled nucleotides, or constructs comprising inducible promoters may be used for determining the half-life of an mRNA in mammalian cells.

It is particularly preferred that the enhanced stability of a stable mRNA in the sense of the present disclosure is affected by its 3'UTR. Thus, preferably, the 3'UTR element comprises or consists of a nucleic acid sequence which is derived from the 3'UTR of a stable mRNA which exhibits a half-life of more than 5 hours, preferably more than 8 hours, in a mammalian cell, such as in a mammalian cell line, e.g. in HELA cells, or in mammalian primary cells, such as HDF cells, preferably determined by using a transcription inhibitor such as actinomycin D, wherein the enhanced stability of said stable mRNA is effected by its 3'UTR. The ability of a 3'UTR for enhancing stability may be tested as described herein, e.g. by using a reporter open reading frame such as a luciferase encoding open reading frame. Alternatively, an artificial construct encoding the test stable mRNA may be generated, wherein the 3'UTR of the stable mRNA is replaced with a reference 3'UTR, such as a 3'UTR of a short lived mRNA, e.g. a Myc 3'UTR. The stability of the wild type stable mRNA and the 3'UTR modified mRNA may be determined as described above. In the event the 3'UTR modified mRNA exhibits a shorter half-life than the wild type stable mRNA, it may be concluded that a stability enhancing effect is exerted by the 3'UTR of the stable mRNA.

In a particularly preferred embodiment, the 3'UTR element comprises or consists of a nucleic acid sequence which is derived from a 3'UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene, or from a variant of a 3'UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene. In a particularly preferred embodiment, the 3'UTR element comprises or consists of a nucleic acid sequence which is derived from a 3'UTR of an albumin gene, preferably a vertebrate albumin gene, more preferably a mammalian albumin gene, most preferably a human albumin gene. In another particularly preferred embodiment, the 3'UTR element comprises or consists of a nucleic acid sequence which is derived from a 3'UTR of an α-globin gene, preferably a vertebrate α-globin gene, more preferably a mammalian α-globin gene, most preferably a human α-globin gene. For example, the 3'UTR element may comprise or consist of the center, α-complex-binding portion of the 3'UTR of an α-globin gene, such as of a human α-globin gene.

Preferably, the at least one 3'UTR element comprises or consists of a nucleic acid sequence which is derived from the 3'UTR of a vertebrate albumin gene, a vertebrate α-globin gene, a vertebrate β-globin gene, a vertebrate tyrosine hydroxylase gene, a vertebrate lipoxygenase gene, and a vertebrate collagen alpha gene, such as a vertebrate collagen alpha 1(I) gene, or from a variant thereof, preferably from the 3'UTR of a mammalian albumin gene, a mammalian α-globin gene, a mammalian β-globin gene, a mammalian tyrosine hydroxylase gene, a mammalian lipoxygenase gene, and a mammalian collagen alpha gene, such as a mammalian collagen alpha 1(I) gene, or from a variant thereof, more preferably from the 3'UTR of a human albumin gene, a human α-globin gene, a human β-globin gene, a human tyrosine hydroxylase gene, a human lipoxygenase gene, and a human collagen alpha gene, such as a human collagen alpha 1(I) gene, or from a variant thereof, even more preferably from the 3'UTR of the human albumin gene according to GenBank Accession number NM_000477.5 or from a variant thereof. In a preferred embodiment, the 3'UTR element is not derived from the 3'UTR of a *Xenopus* albumin gene. Preferably, the 3'UTR element does not comprise a poly(A) limiting element B (PLEB) of a 3'UTR from a *Xenopus* albumin gene. Preferably, the 3'UTR element does not consist of a PLEB of a 3'UTR from a *Xenopus* albumin gene.

In one embodiment, the 3'UTR element and the at least one open reading frame are heterologous, e.g. preferably the 3'UTR element and the ORF are derived from different genes of the same or of different species. Preferably, the ORF does not encode an α-globin protein if the 3'UTR element is derived from an α-globin gene. Preferably, the ORF does not encode a β-globin protein if the 3'UTR element is derived from a β-globin gene. Preferably, the ORF does not encode an albumin protein if the 3'UTR element is derived from an albumin gene. Preferably, the ORF does not encode a tyrosine hydroxylase protein if the 3'UTR element is derived from a tyrosine hydroxylase gene. Preferably, the ORF does not encode a lipoxygenase protein if the 3'UTR element is derived from a lipoxygenase gene. Preferably, the ORF does not encode a collagen alpha protein if the 3'UTR element is derived from a collagene alpha gene.

In one embodiment, the artificial nucleic acid molecule may consist of at least two sequence parts that are derivable from two different genes, the 5'UTR element which is derivable from a TOP gene and the open reading frame and the 3'UTR which may be derivable from the gene encoding the desired protein product. More preferably, the artificial nucleic acid molecule consists of three sequence parts that are derivable from three different genes: the 5'UTR element

which is derivable from a TOP gene, the open reading frame which is derivable from the gene encoding the desired gene product and the 3'UTR element which may be derivable from a gene that relates to an mRNA with an enhanced half-life, for example a 3'UTR element as defined and described below.

In some embodiments, the 3'UTR element consists of a histone stem-loop. In some embodiments, the 3'UTR element of the artificial nucleic acid molecule may comprise a histone stem-loop in addition to the nucleic acid sequence derived from the 3'UTR of a gene, such as of a gene providing a stable mRNA, such as of an albumin gene, an α-globin gene, a β-globin gene, a tyrosine hydroxylase gene, a lipoxygenase gene, or a collagen alpha gene, such as a collagen alpha 1(I) gene as described above. Such artificial nucleic acid molecule disclosed herein, for example, may comprise in 5'-to-3'-direction a 5'UTR element, an ORF, a 3'UTR element, preferably comprising a polyadenylation signal, a histone stem-loop and an optional poly(A) sequence. It may also comprise in 5'-to-3'-direction a 5'UTR element as described above, an ORF, a 3'UTR element, e.g. comprising a polyadenylation signal, a poly(A) sequence and a histone stem-loop.

The term 'a nucleic acid sequence which is derived from the 3'UTR of a [...] gene' preferably refers to a nucleic acid sequence which is based on the 3'UTR sequence of a [...] gene or on a part thereof, such as on the 3'UTR of an albumin gene, an α-globin gene, a β-globin gene, a tyrosine hydroxylase gene, a lipoxygenase gene, or a collagen alpha gene, such as a collagen alpha 1(I) gene, preferably of an albumin gene or an α-globin gene on a part thereof. This term includes sequences corresponding to the entire 3'UTR sequence, i.e. the full length 3'UTR sequence of a gene, and sequences corresponding to a fragment of the 3'UTR sequence of a gene, such as an albumin gene, α-globin gene, β-globin gene, tyrosine hydroxylase gene, lipoxygenase gene, or collagen alpha gene, such as a collagen alpha 1(I) gene, preferably of an albumin or α-globin gene. A fragment in this context preferably consists of a continuous stretch of nucleotides corresponding to a continuous stretch of nucleotides in the full-length 3'UTR, which represents at least 20%, preferably at least 30%, more preferably at least 40%, more preferably at least 50%, even more preferably at least 60%, even more preferably at least 70%, even more preferably at least 80%, and most preferably at least 90% of the full-length 3'UTR. Such a fragment, in the sense of the present disclosure, is preferably a functional fragment as described herein. The term '3'UTR of a [...] gene' preferably refers to the 3'UTR of a naturally occurring gene, such as of a naturally occurring albumin gene, α-globin gene, β-globin gene, tyrosine hydroxylase gene, lipoxygenase gene, or collagen alpha gene, such as a collagen alpha 1(I) gene, preferably of a naturally occurring albumin or α-globin gene.

The terms 'variant of the 3'UTR of a [...] gene' and 'variant thereof' in the context of a 3'UTR refers to a variant of the 3'UTR of a naturally occurring gene, such as a naturally occurring albumin gene, a naturally occurring α-globin gene, a naturally occurring β-globin gene, a naturally occurring tyrosine hydroxylase gene, a naturally occurring lipoxygenase gene, or a naturally occurring collagen alpha gene, such as a collagen alpha 1(I) gene, preferably to a variant of the 3'UTR of a vertebrate albumin gene, a vertebrate α-globin gene, a vertebrate β-globin gene, a vertebrate tyrosine hydroxylase gene, a vertebrate lipoxygenase gene, and a vertebrate collagen alpha gene, such as a vertebrate collagen alpha 1(I) gene, preferably to a variant of the 3'UTR of a mammalian albumin gene, a mammalian α-globin gene, a mammalian β-globin gene, a mammalian tyrosine hydroxylase gene, a mammalian lipoxygenase gene, and a mammalian collagen alpha gene, such as a mammalian collagen alpha 1(I) gene, or to a variant of the 3'UTR of a human albumin gene, a human α-globin gene, a human β-globin gene, a human tyrosine hydroxylase gene, a human lipoxygenase gene, and a human collagen alpha gene, such as a human collagen alpha 1(I) gene. Such variant may be a modified 3'UTR of a gene. For example, a variant 3'UTR may exhibit one or more nucleotide deletions, insertions, additions and/or substitutions compared to the naturally occurring 3'UTR from which the variant is derived. Preferably, a variant of a 3'UTR is at least 40%, preferably at least 50%, more preferably at least 60%, more preferably at least 70%, even more preferably at least 80%, even more preferably at least 90%, most preferably at least 95% identical to the naturally occurring 3'UTR the variant is derived from. Preferably, the variant is a functional variant as described herein.

The term 'a nucleic acid sequence which is derived from a variant of the 3'UTR of a [...] gene' preferably refers to a nucleic acid sequence which is based on a variant of the 3'UTR sequence of a gene, such as on a variant of the 3'UTR of an albumin gene, an α-globin gene, a β-globin gene, a tyrosine hydroxylase gene, a lipoxygenase gene, or a collagen alpha gene, such as a collagen alpha 1(I) gene, or on a part thereof as described above. This term includes sequences corresponding to the entire sequence of the variant of the 3'UTR of a gene, i.e. the full length variant 3'UTR sequence of a gene, and sequences corresponding to a fragment of the variant 3'UTR sequence of a gene. A fragment in this context preferably consists of a continuous stretch of nucleotides corresponding to a continuous stretch of nucleotides in the full-length variant 3'UTR, which represents at least 20%, preferably at least 30%, more preferably at least 40%, more preferably at least 50%, even more preferably at least 60%, even more preferably at least 70%, even more preferably at least 80%, and most preferably at least 90% of the full-length variant 3'UTR. Such a fragment of a variant, in the sense of the present disclosure, is preferably a functional fragment of a variant as described herein.

The terms 'functional variant', 'functional fragment', and 'functional fragment of a variant' (also termed 'functional variant fragment') in the context of the present disclosure, mean that the fragment of the 5'UTR or the 3'UTR, the variant of the 5'UTR or the 3'UTR, or the fragment of a variant of the 5'UTR or the 3'UTR of a gene fulfils at least one, preferably more than one, function of the naturally occurring 5'UTR or 3'UTR of the gene of which the variant, the fragment, or the

fragment of a variant is derived. Such function may be, for example, stabilizing mRNA and/or stabilizing and/or prolonging protein production from an mRNA and/or increasing protein production from an mRNA, preferably in a mammalian cell, such as in a human cell. It is particularly preferred that the variant, the fragment, and the variant fragment in the context of the present disclosure fulfil the function of stabilizing an mRNA, preferably in a mammalian cell, such as a human cell, compared to an mRNA comprising a reference 5'UTR or lacking a 5'UTR and/or a 3'UTR, and/or the function of stabilizing and/or prolonging protein production from an mRNA, preferably in a mammalian cell, such as in a human cell, compared to an mRNA comprising a reference 5'UTR or lacking a 5'UTR and/or a 3'UTR, and/or the function of increasing protein production from an mRNA, preferably in a mammalian cell, such as in a human cell, compared to an mRNA comprising a reference 5'UTR or lacking a 5'UTR and/or a 3'UTR. A reference 5'UTR may be, for example, a 5'UTR naturally occurring in combination with the ORF. Furthermore, a functional variant, a functional fragment, or a functional variant fragment of a 5'UTR or of a 3'UTR of a gene preferably does not have a substantially diminishing effect on the efficiency of translation of the mRNA which comprises such variant of a 5'UTR and/or such variant of a 3'UTR compared to the wild type 5'UTR and/or 3'UTR from which the variant is derived. A particularly preferred function of a "functional fragment", a "functional variant" or a "functional fragment of a variant" of the 3'UTR of a gene, such as an albumin gene, α-globin gene, P-globin gene, tyrosine hydroxylase gene, lipoxygenase gene, or collagen alpha gene, such as a collagen alpha 1(I) gene, in the context of the present disclosure is the stabilization and/or prolongation of protein production by expression of an mRNA carrying the functional fragment, functional variant or functional fragment of a variant as described above. A particularly preferred function of a "functional fragment", a "functional variant" or a "functional fragment of a variant" of the 5'UTR in the context of the present disclosure is the protein production increasing function.

Preferably, the efficiency of the one or more functions exerted by the functional variant, the functional fragment, or the functional variant fragment, such as mRNA and/or protein production stabilizing efficiency and/or the protein production increasing efficiency, is at least 40%, more preferably at least 50%, more preferably at least 60%, even more preferably at least 70%, even more preferably at least 80%, most preferably at least 90% of the mRNA and/or protein production stabilizing efficiency and/or the protein production increasing efficiency exhibited by the naturally occurring 5'UTR and/or 3'UTR of which the variant, the fragment or the variant fragment is derived.

In the context of the present disclosure, a fragment or part of the 3'UTR of a gene, such as an albumin gene, α-globin gene, β-globin gene, tyrosine hydroxylase gene, lipoxygenase gene, or collagen alpha gene, such as a collagen alpha 1(I) gene, or of a variant thereof preferably exhibits a length of at least about 40 nucleotides, preferably of at least about 50 nucleotides, preferably of at least about 75 nucleotides, more preferably of at least about 100 nucleotides, even more preferably of at least about 125 nucleotides, most preferably of at least about 150 nucleotides. Preferably, such fragment of the 3'UTR of a gene or of a variant of the 3'UTR of a gene is a functional fragment as described above.

In the context of the present disclosure, a fragment or part of the 5'UTR of a TOP gene or of a variant thereof preferably exhibits a length of at least about 20 nucleotides, preferably of at least about 30 nucleotides, more preferably of at least about 50 nucleotides. Preferably, such fragment of the 5'UTR of a TOP gene or of a variant of the 5'UTR of a TOP gene is a functional fragment as described above.

In some embodiments, the 3'UTR element of the artificial nucleic acid molecule disclosed herein comprises or consists of a "functional fragment", a "functional variant" or a "functional fragment of a variant" of the 3'UTR of a gene, such as of an albumin gene, α-globin gene, β-globin gene, tyrosine hydroxylase gene, lipoxygenase gene, or collagen alpha gene, such as a collagen alpha 1(I) gene, or of a variant thereof.

In some embodiments, the at least one 5'UTR element of the artificial nucleic acid molecule disclosed herein comprises or consists of a "functional fragment", a "functional variant" or a "functional fragment of a variant" of the 5'UTR of a TOP gene.

Preferably, the 3'UTR element of the artificial nucleic acid molecule disclosed herein increases the stability of the artificial nucleic acid molecule, e.g. increases the stability of an mRNA disclosed herein, compared to a respective mRNA (reference mRNA) lacking a 3'UTR element. Preferably, the at least one 3'UTR element of the artificial nucleic acid molecule disclosed herein increases the stability of protein production from the artificial nucleic acid molecule disclosed herein, e.g. from an mRNA disclosed herein, compared to a respective mRNA lacking a 3'UTR element. Preferably, the at least one 3'UTR element of the artificial nucleic acid molecule disclosed herein prolongs protein production from the artificial nucleic acid molecule disclosed herein, e.g. from an mRNA disclosed herein, compared to a respective mRNA lacking a 3'UTR element. Preferably, the at least one 3'UTR element of the artificial nucleic acid molecule disclosed herein increases the protein production from the artificial nucleic acid molecule disclosed herein, e.g. from an mRNA disclosed herein, compared to a respective mRNA lacking a 3'UTR element. Preferably, the at least one 3'UTR element of the artificial nucleic acid molecule disclosed herein does not negatively influence translational efficiency of an mRNA compared to the translational efficiency of a respective mRNA lacking a 3'UTR element. The term 'respective mRNA' in this context means that - apart from the different 3'UTR - the reference mRNA is comparable, preferably identical, to the mRNA comprising the 3'UTR element.

Preferably, the at least one 5'UTR element of the artificial nucleic acid molecule disclosed herein increases the stability of the artificial nucleic acid molecule, e.g. increases the stability of an mRNA disclosed herein, compared to a

respective mRNA (reference mRNA) lacking a 5'UTR element or comprising a reference 5'UTR element, such as a 5'UTR naturally occurring in combination with the ORF. Preferably, the at least one 5'UTR element of the artificial nucleic acid molecule disclosed herein increases protein production from the artificial nucleic acid molecule disclosed herein, e.g. from an mRNA disclosed herein, compared to a respective mRNA lacking a 5'UTR element or comprising a reference 5'UTR element, such as a 5'UTR naturally occurring in combination with the ORF. The term 'respective mRNA' in this context means that - apart from the different 5'UTR - the reference mRNA is comparable, preferably identical, to the mRNA comprising the inventive 5'UTR element.

Preferably, the histone stem-loop of the artificial nucleic acid molecule disclosed herein increases the stability of the artificial nucleic acid molecule, e.g. increases the stability of an mRNA disclosed herein, compared to a respective mRNA (reference mRNA) lacking a histone stem-loop. Preferably, the histone stem-loop of the artificial nucleic acid molecule disclosed herein increases protein production from the artificial nucleic acid molecule disclosed herein, e.g. from an mRNA disclosed herein, compared to a respective mRNA lacking a histone stem-loop. The term 'respective mRNA' in this context means that - apart from the histone stem loop - the reference mRNA is comparable, preferably identical, to the mRNA comprising the a histone stem-loop.

Preferably, the at least one 5'UTR element and the at least one 3'UTR element act synergistically to increase protein production from the artificial nucleic acid molecule disclosed herein, e.g. from an mRNA disclosed herein, as described above.

Preferably, the at least one 5'UTR element and the histone stem-loop act synergistically to increase protein production from the artificial nucleic acid molecule disclosed herein, e.g. from an mRNA disclosed herein, as described above.

The term 'stabilizing and/or prolonging protein production from an mRNA' preferably means that the protein production from the mRNA is stabilized and/or prolonged compared to the protein production from a reference mRNA, e.g. lacking a 3'UTR element.

'Stabilized protein expression' in this context preferably means that there is more uniform protein production from the artificial nucleic acid molecule disclosed herein over a predetermined period of time, such as over 24 hours, more preferably over 48 hours, even more preferably over 72 hours, when compared to a reference nucleic acid molecule, for example, lacking a 3'UTR element. Thus, the level of protein production, e.g. in a mammalian system, from the artificial nucleic acid molecule comprising a 3'UTR element disclosed herein, e.g. from an mRNA disclosed herein, preferably does not drop to the extent observed for a reference nucleic acid molecule. For example, the amount of a protein (encoded by the ORF) observed 6 hours after initiation of expression, e.g. 6 hours post transfection of the artificial nucleic acid molecule disclosed herein into a cell, such as a mammalian cell, may be comparable to the amount of protein observed 48 hours after initiation of expression, e.g. 48 hours post transfection. Thus, the ratio of the amount of protein encoded by the ORF, such as of a reporter protein, e.g., luciferase, observed at 48 hours post initiation of expression, e.g. 48 hours post transfection, to the amount of protein observed 6 hours after initiation of expression, e.g. 6 hours post transfection, is preferably above 0.4, preferably above 0.5, more preferably above 0.6, even more preferably above 0.7, e.g. between about 0.4 and about 4, preferably between about 0.65 and about 3, more preferably between about 0.7 and 2 for a nucleic acid molecule disclosed herein. Thus, in one embodiment, the present disclosure provides an artificial nucleic acid molecule as described above, wherein the ratio of the (reporter) protein amount observed 48 hours after initiation of expression to the (reporter) protein amount observed 6 hours after initiation of expression, preferably in a mammalian expression system, such as in mammalian cells, is preferably between about 0.4 and 4, preferably between about 0.65 and about 3, more preferably between about 0.7 and 2.

'Increased protein expression' in the context of the present disclosure may refer to increased protein expression at one time point after initiation of expression compared to a reference molecule or to an increased total protein production within a certain time period after initiation of expression. Thus, the protein level observed at a certain time point after initiation of expression, e.g. after transfection, of the artificial nucleic acid molecule disclosed herein, e.g. after transfection of an mRNA disclosed herein, for example, 24, 48, or 72 hours post transfection, or the total protein produced in a time span of, e.g. 24, 48 or 72 hours, is preferably higher than the protein level observed at the same time point after initiation of expression, e.g. after transfection, or the total protein produced within the same time span, for a reference nucleic acid molecule, such as a reference mRNA comprising a reference 5'UTR element or lacking a 5'UTR element and/or 3'UTR element and/or a histone stem-loop. As set forth above, it is a particularly preferred function of the 5'UTR element and the histone stem-loop to effect an increase in protein production from the artificial nucleic acid molecule. Preferably, the increase in protein production effected by the 5'UTR element and the histone stem-loop compared to a reference nucleic acid molecule lacking such 5'UTR element and a histone stem-loop at a given time point post initiation of expression is at least 1.5-fold, more preferably at least 2-fold, more preferably at least 3-fold, more preferably at least 4-fold, more preferably at least 5-fold, even more preferably at least 10-fold, even more preferably at least 15-fold of the protein production observed for a reference nucleic acid molecule lacking the 5'UTR element and a histone stem-loop. The same holds preferably for the total protein production in a given time period, for example in a time period of 24, 48 or 72 hours post initiation of expression.

Said increase in stability of the artificial nucleic acid molecule, said increase in stability of protein production, said prolongation of protein production and/or said increase in protein production is preferably determined by comparison with a respective reference nucleic acid molecule lacking a 5'UTR element and/or a 3'UTR element and/or a histone stem-loop, e.g. an mRNA lacking a 5'UTR element and/or a 3'UTR element and/or a histone stem-loop, or a reference nucleic acid molecule comprising a reference 5'UTR element and/or a reference 3'UTR element, such as a 3'UTR and/or a 5'UTR naturally occurring with the ORF or a 5'UTR and/or a 3'UTR of a reference gene.

The mRNA and/or protein production stabilizing effect and efficiency and/or the protein production increasing effect and efficiency of the variants, fragments and/or variant fragments of the 3'UTR of an albumin gene as well as the mRNA and/or protein production stabilizing effect and efficiency and/or the protein production increasing effect and efficiency of the 3'UTR element, the at least one 5'UTR element, or the histone stem-loop of the artificial nucleic acid molecule disclosed herein may be determined by any method suitable for this purpose known to the skilled person. For example, artificial mRNA molecules may be generated comprising a coding sequence for a reporter protein, such as luciferase, and no 3'UTR and/or no 5'UTR and/or no histone stem-loop, a 5'UTR derived from a TOP gene and/or a 3'UTR derived from a gene as described above and/or a histone stem-loop as described above, a 5'UTR derived from a reference gene and/or a 3'UTR derived from a reference gene (i.e., a reference 3'UTR or a reference 5'UTR, such as a 5'UTR or a 3'UTR naturally occurring with the ORF), as 3'UTR a variant of a 3'UTR of a gene as described above, as 3'UTR a fragment of a 3'UTR of a gene as described above, or as 3'UTR a fragment of a variant of a 3'UTR of a gene as described above, as 5'UTR a variant of a 5'UTR of a TOP gene, as 5'UTR a fragment of a 5'UTR of a TOP gene, or as 5'UTR a fragment of a variant of a 5'UTR of a TOP gene. Such mRNAs may be generated, for example, by *in vitro* transcription of respective vectors such as plasmid vectors, e.g. comprising a T7 promoter and a sequence encoding the respective mRNA sequences. The generated mRNA molecules may be transfected into cells by any transfection method suitable for transfecting mRNA, for example they may be electroporated into mammalian cells, such as HELA or HDF cells, and samples may be analyzed certain time points after transfection, for example, 6 hours, 24 hours, 48 hours, and 72 hours post transfection. Said samples may be analyzed for mRNA quantities and/or protein quantities by methods well known to the skilled person. For example, the quantities of reporter mRNA present in the cells at the sample time points may be determined by quantitative PCR methods. The quantities of reporter protein encoded by the respective mRNAs may be determined, e.g., by ELISA assays or reporter assays such as luciferase assays depending on the reporter protein used. The effect of stabilizing protein expression and/or prolonging protein expression may be, for example, analyzed by determining the ratio of the protein level observed 48 hours post transfection and the protein level observed 6 hours post transfection. The closer said value is to 1, the more stable the protein expression is within this time period. Said value may also be above 1 if the protein level is higher at the later time point. Such measurements may of course also be performed at 72 or more hours and the ratio of the protein level observed 72 hours post transfection and the protein level observed 6 hours post transfection may be determined to determine stability of protein expression.

Preferably, the 3'UTR element of the artificial nucleic acid molecule disclosed herein comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to a nucleic acid sequence selected from SEQ ID NOs. 1369-1377 and 1434 and corresponding RNA sequences, wherein the variants of the sequences according to SEQ ID NOs. 1369-1377 and 1434 are preferably functional variants as described above. SEQ ID NOs. 1369, 1371 and 1434, variants thereof, and corresponding RNA sequences are particularly preferred.

The 3'UTR element of the artificial nucleic acid molecule disclosed herein may also comprise or consist of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the nucleic acid sequence according to SEQ ID No. 1369-1377 and 1434 and of corresponding RNA sequences, wherein the fragment is preferably a functional fragment or a functional variant fragment as described above. Preferably, the fragment is as described above, i.e. being a continuous stretch of nucleotides representing at least 20% etc. of the full-length 3'UTR the fragment is derived from. Such fragment preferably exhibits a length of at least about 40 nucleotides, preferably of at least about 50 nucleotides, preferably of at least about 75 nucleotides, more preferably of at least about 100 nucleotides, even more preferably of at least about 125 nucleotides, most preferably of at least about 150 nucleotides.

For example, such fragment may exhibit a nucleic acid sequence according to SEQ ID Nos. 1378-1390, such as:

AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATT (SEQ ID No. 1378)

CATCACATTT AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG (SEQ ID No. 1379)

AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC (SEQ ID No. 1380)

CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT (SEQ ID No. 1381)

TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT (SEQ ID No. 1382)

AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT (SEQ ID No. 1383)

TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT (SEQ ID No. 1384)

AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA (SEQ ID No. 1385)

ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA AAAATGGAAA (SEQ ID No. 1386)

CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No. 1387)

TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No. 1388)

CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No. 1389)

AAGCTTATTC ATCTGTTTTT CTTTTTCGTT GGTGTAAAGC CAACACCCTG TCTAAAAAAC (SEQ ID No. 1390)

or the corresponding RNA sequence, or a nucleic acid sequence which is at least 40%, preferably at least about 50%, preferably at least about 60%, preferably at least about 70%, more preferably at least about 80%, more preferably at least about 90%, even more preferably at least about 95%, even more preferably at least about 99% identical to said nucleic acid sequences or the corresponding RNA sequence. Thus, the at least one 3'UTR element of the artificial nucleic acid molecule disclosed herein may comprise or consist of a nucleic acid fragment as described above. Obviously, the thymidine nucleotides comprised in the fragments according to SEQ ID Nos. 1378-1390 may be replaced by uridine nucleotides.

Preferably, said variants, fragments or variant fragments are functional variants, functional fragments, or functional variant fragments as described above, exhibiting at least one function of the nucleic acid sequence according to SEQ ID Nos. 1369-1377 and 1434, such as stabilization of the artificial nucleic acid molecule disclosed herein, stabilizing and/or prolonging protein expression from the artificial nucleic acid molecule disclosed herein, and/or increasing protein production, preferably with an efficiency of at least 40%, more preferably of at least 50%, more preferably of at least 60%, even more preferably of at least 70%, even more preferably of at least 80%, most preferably of at least 90% of the stabilizing efficiency and/or protein production increasing efficiency exhibited by the nucleic acid sequence according to SEQ ID Nos. 1369-1377 and 1434. Preferably, variants, fragments or variant fragments are functional variants, functional fragments, or functional variant fragments exhibit the function of acting synergistically with the 5'UTR element to increase protein production from the artificial nucleic acid molecule.

Preferably, the 3'UTR element of the artificial nucleic acid molecule disclosed herein exhibits a length of at least about 40 nucleotides, preferably of at least about 50 nucleotides, preferably of at least about 75 nucleotides, more preferably of at least about 100 nucleotides, even more preferably of at least about 125 nucleotides, most preferably of at least about 150 nucleotides. For example, the 3'UTR may exhibit a length of about 50 to about 300 nucleotides, preferably of about 100 to about 250 nucleotides, more preferably of about 150 to about 200 nucleotides.

Furthermore, the artificial nucleic acid molecule disclosed herein may comprise more than one 3'UTR elements as described above. For example, the artificial nucleic acid molecule disclosed herein may comprise one, two, three, four or more 3'UTR elements, wherein the individual 3'UTR elements may be the same or they may be different. For example, the artificial nucleic acid molecule disclosed herein may comprise two essentially identical 3'UTR elements as described above, e.g. two 3'UTR elements comprising or consisting of a nucleic acid sequence which is derived from the 3'UTR of an albumin gene or an α-globin gene or from a variant of the 3'UTR of an albumin gene or of an α-globin gene, such as a nucleic acid sequence according to SEQ ID No. 1369, 1371, 1376, or 1434, functional variants thereof, functional fragments thereof, or functional variant fragments thereof as described above.

In a preferred embodiment, the artificial nucleic acid molecule comprises (a.) at least one 5'UTR element which comprises or consists of a nucleic acid sequence which is derived from the 5'UTR of a TOP gene encoding a ribosomal protein as described above, for example, encoding a ribosomal Large protein, or from a variant thereof, (b.) at least one open reading frame, (c.) at least one histone stem-loop as described herein, such as at least one histone stem-loop according to SEQ ID NOs. 1391-1433, optionally (d.) a poly(A) sequence or a poly(A) signal, optionally (e.) a poly(C) sequence, and optionally (f.) at least one 3'UTR element, preferably derived from a gene providing a stable mRNA, e.g., which comprises or consists of a nucleic acid sequence which is derived from the 3'UTR of an albumin gene or an α-globin gene, such as a sequence selected from the group consisting of SEQ ID NOs: 1369, 1371, and 1434 or a variant thereof as described herein.

Preferably, the sequence of elements of the artificial nucleic acid molecule in 5'-to-3'-direction is 5'-[at least one 5'UTR]-[ORF]-[optional at least one 3'UTR]-[optional poly(A) sequence]-[optional poly(C) sequence]-[at least one histone stem-loop]-3'.

In a particularly preferred embodiment, the artificial nucleic acid moleucle comprises (a.) at least one 5'UTR element which comprises or consists of a nucleic acid sequence which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit Vic gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit VIc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cytochrome c oxidase subunit VIc gene (COX6C), or a mammalian N-acylsphingosine amidohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit VIc gene (COX6C), or a human N-acylsphingosine amidohydrolase (acid ceramidase) 1 gene (ASAH1) or from a variant thereof, wherein preferably the 5'UTR element does not comprise the 5'TOP of said gene, such as the sequence according to SEQ ID NO: 1368 or SEQ ID NOs 1452-1460 or a variant thereof, (b.) at least one open reading frame, (c.) at least one histone stem-loop, such as at least one histone stem-loop according to SEQ ID NOs. 1391-1433, optionally (d.) a poly(A) sequence and/or a poly(A) signal, optionally (e.) a poly(C) sequence, and optionally (f.) at least one 3'UTR element which comprises or consists of a nucleic acid sequence which is derived from an albumin gene or an $\alpha$-globin gene, such as a sequence selected from the group consisting of SEQ ID NOs: 1369, 1371, and 1434 or a variant thereof as described herein.

In a particularly preferred embodiment, the artificial nucleic acid molecule disclosed herein comprises:

(a.) at least one 5'UTR element which comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 1368 or SEQ ID NOs. 1452-1460, or a corresponding RNA sequence,

(b.) at least one open reading frame,

(c.) at least one histone stem-loop as described herein, such as a histone stem-loop sequence according to any one of SEQ ID NOs. 1391-1433, preferably a histone stem-loop sequence having a sequence identity of at least about 75%, preferably of at least about 80%, preferably at least about 85%, more preferably at least about 90%, even more preferably at least about 95% to the sequence according to SEQ ID NO. 1433 or a corresponding RNA sequence, wherein preferably positions 6, 13 and 20 of the sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence are conserved, i.e. are identical to the nucleotides at positions 6, 13 and 20 of SEQ ID NO. 1433,

(d.) optionally, a poly(A) sequence or a poly(A) signal as described herein,

(e.) optionally, a poly(C) sequence, and

(f.) optionally, a 3'UTR element, preferably a 3'UTR element which is derived from a gene providing a stable mRNA, such as a 3'UTR element which comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about

95%, even more preferably of at least about 99%, most preferably of 100% to the nucleic acid sequence according to SEQ ID No. 1369, 1371, or 1434 or a corresponding RNA sequence.

Thus, in a particularly preferred embodiment, the present disclosure provides an artificial nucleic acid molecule comprising a 5'UTR element which comprises or consists of a nucleic acid sequence which has an identity of at least about 90% to the nucleic acid sequence according to SEQ ID No. 1368 or SEQ ID NOs. 1452-1460, or a corresponding RNA sequence, a histone stem-loop comprising a sequence which has an identity of at least about 90% to the sequence according to SEQ ID NO. 1434 or a corresponding RNA sequence, optionally a poly(A) sequence and/or a poly(A) signal as described herein, optionally a poly(C) sequence, and optionally a 3'UTR element which comprises or consists of a nucleic acid sequence which has an identity of at least about 90% to the nucleic acid sequence according to SEQ ID No. 1369, 1371 or 1434.

Preferably, the artificial nucleic acid molecule disclosed herein does not contain one or two or at least one or all but one or all of the components of the group consisting of: a sequence encoding a ribozyme (preferably a self-splicing ribozyme), a viral nucleic acid sequence, a histone stem-loop processing signal, in particular a histone stem-loop processing sequence derived from mouse histon H2A614 gene, a Neo gene, an inactivated promoter sequence and an inactivated enhancer sequence. Even more preferably, the nucleic acid disclosed herein does not contain a ribozyme, preferably a self-splicing ribozyme, and one of the group consisting of: a Neo gene, an inactivated promotor sequence, an inactivated enhancer sequence, a histon stem-loop processing signal, in particular a histon-stem loop processing sequence derived from mouse histon H2A614 gene. Accordingly, the nucleic acid may in a preferred mode neither contain a ribozyme, preferably a self-splicing ribozyme, nor a Neo gene or, alternatively, neither a ribozyme, preferably a self-splicing ribozyme, nor any resistance gene (e.g. usually applied for selection). In an other preferred mode, the nucleic acid molecule disclosed herein may neither contain a ribozyme, preferably a self-splicing ribozyme, nor a histone stem-loop processing signal, in particular a histone stem-loop processing sequence derived from mouse histone H2A614 gene.

Furthermore, it is preferred that the artificial nucleic acid molecule disclosed herein does not comprise an intron.

The artificial nucleic acid molecule disclosed herein may be RNA, such as mRNA, DNA, such as a DNA vector, or may be a modified RNA or DNA molecule. It may be provided as a double-stranded molecule having a sense strand and an anti-sense strand, for example, as a DNA molecule having a sense strand and an anti-sense strand.

The present disclosure also provides an artificial nucleic acid molecule which is an mRNA molecule comprising a, 5'UTR element, an open reading frame, a histone stem-loop as described herein, an optional 3'UTR element as described herein and an optional poly(A) sequence.

The artificial nucleic acid molecule disclosed herein may further comprise a 5'-cap. The optional 5'-cap is preferably attached to the 5'-side of the 5'UTR element.

The present disclosure provides an artificial nucleic acid molecule which may be a template for an RNA molecule, preferably for an mRNA molecule, which is stabilised and optimized with respect to translation efficiency. In other words, the artificial nucleic acid molecule may be a DNA or RNA which may be used for production of an mRNA. The obtainable mRNA, may, in turn, be translated for production of a desired peptide or protein encoded by the open reading frame. If the artificial nucleic acid molecule is a DNA, it may, for example, be used as a double-stranded storage form for continued and repetitive *in vitro* or *in vivo* production of mRNA.

Potential transcription systems are *in vitro* transcription systems or cellular transcription systems etc. Accordingly, transcription of an artificial nucleic acid molecule disclosed herein, e.g. transcription of an artificial nucleic acid molecule comprising a 5'UTR element, an open reading frame, a histone stem-loop, a 3'UTR element, and a polyadenylation-signal, may result in an mRNA molecule comprising a 5'UTR element, an open reading frame, a histone stem-loop, a 3'UTR element and a poly(A) sequence.

For example, the artificial nucleic acid molecule disclosed herein may comprise a nucleic acid sequence corresponding to the DNA sequence

CATCACATTT AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG TCTAAAAAAC
ATAAATTTCT TTAATCATTT TGCCTCTTTT CTCTGTGCTT CAATTAATAA AAAATGGAAA
GAATCTAGAT CTAAAAAAAA AAAAAAAAAA AAAAAAAAAA AAAAAAAAAA AAAAAAAAAA
AAAAAAAAAA AAAAAA (SEQ ID No. 1377).

Transcription of such a sequence may result in an artificial nucleic acid molecule comprising a corresponding RNA sequence.

Such artificial RNA molecule may also be obtainable *in vitro* by common methods of chemical synthesis without

being necessarily transcribed from a DNA progenitor.

In a particularly preferred embodiment, the artificial nucleic acid molecule disclosed herein is an RNA molecule, preferably an mRNA molecule comprising in 5'-to-3'-direction a 5'UTR element as described above, an open reading frame, an optional 3'UTR element as described above, an optional poly(A) sequence, an optional poly(C) sequence, and a histone stem-loop as described herein.

In some embodiments, the artificial nucleic acid molecule comprises further elements such as an IRES-motif. An internal ribosome entry side (IRES) sequence or IRES-motif may separate several open reading frames, for example if the artificial nucleic acid molecule encodes for two or more peptides or proteins. An IRES-sequence may be particularly helpful if the mRNA is a bi- or multicistronic RNA.

Furthermore, the artificial nucleic acid molecule may comprise additional 5'-elements such as a promoter or enhancer sequence. The promoter may drive and or regulate transcription of the artificial nucleic acid molecule disclosed herein, for example of an artificial DNA molecule disclosed herein.

In preferred embodiments, the present disclosure provides artificial nucleic acid molecules, preferably mRNA molecules, comprising in 5'-to-3'-direction at least one of the following structures:

5'-cap - 5'UTR element - ORF - 3'UTR element - histone stem-loop - poly(A) sequence

5'-cap - 5'UTR element - ORF - 3'UTR element - poly(A) sequence - histone stem-loop

5'-cap - 5'UTR element - ORF - IRES - ORF - 3'UTR element - histone stem-loop - poly(A) sequence

5'-cap - 5'UTR element - ORF - IRES - ORF - 3'UTR element - poly(A) sequence - histone stem-loop

5'-cap - 5'UTR element - ORF - 3'UTR element - poly(A) sequence - poly(C) sequence - histone stem-loop

5'-cap - 5'UTR element - ORF - IRES - ORF - 3'UTR element - poly(A) sequence - poly(C) sequence - histone stem-loop

5'-cap - 5'UTR element - ORF - IRES - ORF - 3'UTR element - histone stem-loop - poly(A) sequence - poly(C) sequence

More preferably, the artificial nucleic acid molecule disclosed herein comprises or codes for (a.) a 5'UTR-element; (b.) an open reading frame, preferably encoding a peptide or protein; (c.) at least one histone stem-loop, optionally (d.) a poly(A) sequence and/or polyadenylation signal; (e.) optionally a poly(C) sequence; and (f.) optionally a 3'UTR element, preferably for increasing the expression level of an encoded protein, wherein the encoded protein is preferably no histone protein, no reporter protein and/or no marker or selection protein, as defined above. The elements (c.) to (f.) of the artificial nucleic acid molecule disclosed herein may occur in the artificial nucleic acid molecule disclosed herein in any sequence, i.e. the elements (a.), (b.), (c.), (d.), (e.) and (f.) may, for example, occur in the sequence (a.), (b.), (c.), (d.), (e.) and (f.), or (a.), (b.), (d.), (c.), (e.) and (f.), or (a.), (b.), (c.), (d.), (f.) and (e.), or (a.), (b.), (d.), (c.), (f.) and (e.), or (a.), (b.), (e.), (d.), (c.) and (f.), or (a.), (b.), (e.), (d.), (f.) and (c.), or (a.), (b.), (c.), (f.), (e.) and (d.) etc., wherein further elements as described herein, may also be contained, such as a 5'-CAP structure, stabilization sequences, IRES sequences, etc. Each of the elements (a.) to (f.) of the artificial nucleic acid molecule disclosed herein, particularly b), may occur in di- or multicistronic constructs and/or each of the elements (a.), (c.) and (f.) may also be repeated at least once, preferably twice or more in the artificial nucleic acid molecule disclosed herein. As an example, the artificial nucleic acid molecule disclosed herein may comprise its sequence elements (a.), (b.), (c.) and optionally (d.) in e.g. the following order. In all cases the artificial nucleic acid molecule may additionally comprise one or more optional 3'UTR element(s) and/or a poly(C) sequence as defined herein:

5'UTR - ORF - histone stem-loop - 3'; or
5'UTR - ORF - ORF - histone stem-loop - 3'; or
5' UTR - ORF - IRES - ORF - histone stem-loop - 3'; or
5' UTR - ORF - histone stem-loop - poly(A) sequence - 3'; or
5' UTR - ORF - histone stem-loop - polyadenylation signal - 3'; or
5' UTR - ORF - ORF - histone stem-loop - polyadenylation signal - 3'; or
5' UTR - ORF - histone stem-loop - histone stem-loop - 3'; or
5' UTR - ORF - histone stem-loop - histone stem-loop - poly(A) sequence - 3'; or
5' UTR - ORF - histone stem-loop - histone stem-loop - polyadenylation signal- 3'; or
5' UTR - ORF - histone stem-loop - poly(A) sequence - histone stem-loop - 3'; or

5’ UTR - ORF - poly(A) sequence - histone stem-loop - 3’; or
5’ UTR - ORF - poly(A) sequence - histone stem-loop - histone stem-loop - 3’;etc.

It is preferred that the above sequences comprise a poly(C) sequence. Preferably, this poly(C) sequence is located 5’ to the histone stem-loop, preferably between the poly(A) sequence and the histone stem-loop sequence.

In this context, it is particularly preferred that the artificial nucleic acid molecule disclosed herein comprises or codes for a) a 5’UTR element, b) an open reading frame, preferably encoding a peptide or protein; c) at least one histone stem-loop, and d) a poly(A) sequence or polyadenylation sequence; preferably for increasing the expression level of an encoded protein, wherein the encoded protein is preferably no histone protein, no reporter protein (e.g. Luciferase, GFP, EGFP, β-Galactosidase, particularly EGFP) and/or no marker or selection protein (e.g. alpha-Globin, Galactokinase and Xanthine:Guanine phosphoribosyl transferase (GPT)).

The open reading frame of the artificial nucleic acid molecule is not particularly limited. For example, the open reading frame may encode a protein or peptide that may be used for therapy of a disease. The particular choice of the protein or peptide depends on the disease to be treated and is not the subject of the invention. Accordingly, the artificial nucleic acid molecule may be for use in treatment of a disease that is treatable with the protein or peptide that is encoded by the open reading frame. The open reading frame may also encode a protein or peptide that may be used as an antigen for vaccination. Again, the particular choice of the protein or peptide depends on the disease or infection to be prevented. Accordingly, the artificial nucleic acid molecule may be for use in prevention of a disease by inducing a specific immune response.

However, the encoded protein is preferably no histone protein. In the context of the present disclosure, such a histone protein is typically a strongly alkaline protein found in eukaryotic cell nuclei, which package and order the DNA into structural units called nucleosomes. Histone proteins are the chief protein components of chromatin, act as spools around which DNA winds, and play a role in gene regulation. Without histones, the unwound DNA in chromosomes would be very long (a length to width ratio of more than 10 million to one in human DNA). For example, each human cell has about 1.8 meters of DNA, but wound on the histones it has about 90 millimeters of chromatin, which, when duplicated and condensed during mitosis, result in about 120 micrometers of chromosomes. More preferably, in the context of the present disclosure, such a histone protein is typically defined as a highly conserved protein selected from one of the following five major classes of histones: H1/H5, H2A, H2B, H3, and H4", preferably selected from mammalian histone, more preferably from human histones or histone proteins. Such histones or histone proteins are typically organised into two super-classes defined as core histones, comprising histones H2A, H2B, H3 and H4, and linker histones, comprising histones H1 and H5.

In this context, linker histones, are preferably excluded from the scope of protection of the pending disclosure, preferably mammalian linker histones, more preferably human linker histones, are typically selected from H1, including H1F, particularly including H1F0, H1FNT, H1FOO, H1FX, and H1H1, particularly including HIST1H1A, HIST1H1B, HIST1H1C, HIST1H1D, HIST1H1E, HIST1H1T.

Furthermore, in some embodiments, core histones which are preferably excluded from the scope of protection of the pending disclosure, preferably mammalian core histones, more preferably human core histones, are typically selected from H2A, including H2AF, particularly including H2AFB1, H2AFB2, H2AFB3, H2AFJ, H2AFV, H2AFX, H2AFY, H2AFY2, H2AFZ, and H2A1, particularly including HIST1H2AA, HIST1H2AB, HIST1H2AC, HIST1H2AD, HIST1H2AE, HIST1 H2AG, HIST1 H2AI, HIST1H2AJ, HIST1H2AK, HIST1H2AL, HIST1H2AM, and H2A2, particularly including HIST2H2AA3, HIST2H2AC; H2B, including H2BF, particularly including H2BFM, H2BFO, H2BFS, H2BFWT H2B1, particularly including HIST1H2BA, HIST1H2BB, HIST1H2BC, HIST1H2BD, HIST1H2BE, HIST1H2BF, HIST1H2BG, HIST1H2BH, HIST1H2BI, HIST1H2BJ, HIST1H2BI<, HIST1H2BL, HIST1H2BM, HIST1H2BN, HIST1H2BO, and H2B2, particularly including HIST2H2BE; H3, including H3A1, particularly including HIST1H3A, HIST1H3B, HIST1H3C, HIST1H3D, HIST1H3E, HIST1H3F, HIST1H3G, HIST1H3H, HIST1H3I, HIST1H3J, and H3A2, particularly including HIST2H3C, and H3A3, particularly including HIST3H3; H4, including H41, particularly including HIST1H4A, HIST1H4B, HIST1H4C, HIST1H4D, HIST1H4F, HIST1H4F, HIST1H4G, HIST1H4H, HIST1H4I, HIST1H4J, HIST1H4K, HIST1H4L, and H44, particularly including HIST4H4, and H5.

Preferably, the protein encoded by the open reading frame is no reporter protein (e.g. Luciferase, Green Fluorescent Protein (GFP), Enhanced Green Fluorescent Protein (EGFP), β-Galactosidase) and no marker or selection protein (e.g. alpha-globin, Galactokinase and Xanthine:guanine phosphoribosyl transferase (GPT)). Preferably, the artificial nucleic acid molecule disclosed herein does not contain a (bacterial) Neo gene sequence (Neomycin resistance gene).

Preferably, the ORF does not code for a protein selected from the group consisting of albumin proteins, α-globin proteins, β-globin proteins, tyrosine hydroxylase proteins, lipoxygenase proteins, and collagen alpha proteins.

In a preferred embodiment, the open reading frame does not code for human albumin, provided that the 3’UTR element is identical to the 3’UTR of human albumin. In some further embodiment, it is preferred that the open reading frame does not code for human albumin according to GenBank Accession number NM_000477.5 provided that the

3'UTR element is identical to the 3'UTR of human albumin. In some further embodiments, it is preferred that the open reading frame does not code for human albumin or variants thereof provided that the 3'UTR element is a sequence which is identical to SEQ ID No. 1369 or to a corresponding RNA sequence.

Furthermore, in some embodiments, it is preferred that the open reading frame does not code for a reporter protein selected from the group consisting of globin proteins, luciferase proteins, GFP proteins or variants thereof, for example, variants exhibiting at least 70% sequence identity to a globin protein, a luciferase protein, or a GFP protein.

Preferably, the artificial nucleic acid molecule, preferably the open reading frame, is at least partially G/C modified. Thus, the artificial nucleic acid molecule disclosed herein may be thermodynamically stabilized by modifying the G (guanosine)/C (cytidine) content of the molecule. The G/C content of the open reading frame of an artificial nucleic acid molecule disclosed herein may be increased compared to the G/C content of the open reading frame of a corresponding wild type sequence, preferably by using the degeneration of the genetic code. Thus, the encoded amino acid sequence of the nucleic acid molecule is preferably not modified by the G/C modification compared to the coded amino acid sequence of the particular wild type sequence. The codons of a coding sequence or a whole nucleic acid molecule, e.g. an mRNA, may therefore be varied compared to the wild type coding sequence, such that they include an increased amount of G/C nucleotides while the translated amino acid sequence is maintained. In respect to the fact that several codons code for one and the same amino acid (so-called degeneration of the genetic code), the most favourable codons for the stability can be determined (so-called alternative codon usage).

Depending on the amino acid to be encoded by the coding region of the nucleic acid molecule as defined herein, there are various possibilities for modification of the nucleic acid sequence, e.g. the open reading frame, compared to its wild type coding region. In the case of amino acids which are encoded by codons which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U/T is present.

In contrast, codons which contain A and/or U/T nucleotides may be modified by substitution of other codons which code for the same amino acids but contain no A and/or U/T. For example

the codons for Pro can be modified from CC(U/T) or CCA to CCC or CCG;
the codons for Arg can be modified from CG(U/T) or CGA or AGA or AGG to CGC or CGG;
the codons for Ala can be modified from GC(U/T) or GCA to GCC or GCG;
the codons for Gly can be modified from GG(U/T) or GGA to GGC or GGG.

In other cases, although A or (U/T) nucleotides cannot be eliminated from the codons, it is however possible to decrease the A and (U/T) content by using codons which contain a lower content of A and/or (U/T) nucleotides. Examples of these are:
The codons for Phe can be modified from (U/T)(U!T)(U!T) to (U/T) (U/T)C;

the codons for Leu can be modified from (U/T) (U/T)A, (U/T) (U/T)G, C(U/T) (U/T) or C(U/T)A to C(U/T)C or C(U/T)G;
the codons for Ser can be modified from (U/T)C(U/T) or (U/T)CA or AG(U/T) to (U/T)CC, (U/T)CG or AGC;
the codon for Tyr can be modified from (U/T)A(U/T) to (U/T)AC;
the codon for Cys can be modified from (U/T)G(U/T) to (U/T)GC;
the codon for His can be modified from CA(U/T) to CAC;
the codon for Gln can be modified from CAA to CAG;
the codons for Ile can be modified from A(U/T)(U/T) or A(U/T)A to A(U/T)C;
the codons for Thr can be modified from AC(U/T) or ACA to ACC or ACG;
the codon for Asn can be modified from AA(U/T) to AAC;
the codon for Lys can be modified from AAA to AAG;
the codons for Val can be modified from G(U/T)(U/T) or G(U/T)A to G(U/T)C or G(U/T)G;
the codon for Asp can be modified from GA(U/T) to GAC;
the codon for Glu can be modified from GAA to GAG;
the stop codon (U/T)AA can be modified to (UZT)AG or (U!T)GA.

In the case of the codons for Met (A(U/T)G) and Trp ((U/T)GG), on the other hand, there is no possibility of sequence modification without altering the encoded amino acid sequence.

The substitutions listed above can be used either individually or in all possible combinations to increase the G/C content of the open reading frame of the nucleic acid sequence as defined herein, compared to its particular wild type open reading frame (i.e. the original sequence). Thus, for example, all codons for Thr occurring in the wild type sequence can be modified to ACC (or ACG).

Preferably, the G/C content of the open reading frame of the artificial nucleic acid molecule as defined herein is increased by at least 7%, more preferably by at least 15%, particularly preferably by at least 20%, compared to the

G/C content of the wild type coding region. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the open reading frame of the artificial nucleic acid molecule disclosed herein or a fragment, variant or derivative thereof are substituted, thereby increasing the G/C content of said open reading frame.

In this context, it is particularly preferable to increase the G/C content of the open reading frame of the nucleic acid sequence as defined herein, to the maximum (i.e. 100% of the substitutable codons), compared to the wild type open reading frame.

Furthermore, the open reading frame is preferably at least partially codon-optimized. Codon-optimization is based on the finding that the translation efficiency may be determined by a different frequency in the occurrence of transfer RNAs (tRNAs) in cells. Thus, if so-called "rare codons" are present in the coding region of the artificial nucleic acid molecule as defined herein, to an increased extent, the translation of the corresponding modified nucleic acid sequence is less efficient than in the case where codons coding for relatively "frequent" tRNAs are present.

Thus, the open reading frame of the nucleic acid sequence disclosed herein is preferably modified compared to the corresponding wild type coding region such that at least one codon of the wild type sequence which codes for a tRNA which is relatively rare in the cell is exchanged for a codon which codes for a tRNA which is comparably frequent in the cell and carries the same amino acid as the relatively rare tRNA. By this modification, the open reading frame of the artificial nucleic acid molecule as defined herein, is modified such that codons for which frequently occurring tRNAs are available may replace codons which correspond to rare tRNAs. In other words, according to the present disclosure, by such a modification all codons of the wild type open reading frame which code for a rare tRNA may be exchanged for a codon which codes for a tRNA which is more frequent in the cell and which carries the same amino acid as the rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. Accordingly, preferably, the open reading frame is codon-optimized, preferably with respect to the system in which the nucleic acid molecule disclosed herein is to be expressed, preferably with respect to the system in which the nucleic acid molecule disclosed herein is to be translated. Preferably, the codon usage of the open reading frame is codon-optimized according to mammalian codon usage, more preferably according to human codon usage. Preferably, the open reading frame is codon-optimized and G/C-content modified.

For further improving degradation resistance, e.g. resistance to *in vivo* degradation by an exo- or endonuclease, and/or for further improving protein production from the artificial nucleic acid molecule disclosed herein, the artificial nucleic acid molecule may further comprise modifications, such as backbone modifications, sugar modifications and/or base modifications, e.g., lipid-modifications or the like. Preferably, the transcription and/or the translation of the artificial nucleic acid molecule disclosed herein is not significantly impaired by said modifications.

Nucleotide analogues/modifications that may be used in the context of the present disclosure may be selected, for example, from 2-amino-6-chloropurineriboside-5'-triphosphate, 2-aminoadenosine-5'-triphosphate, 2-thiocytidine-5'-triphosphate, 2-thiouridine-5'-triphosphate, 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-iodocytidine-5'-triphosphate, 5-iodouridine-5'-triphosphate, 5-methylcytidine-5'-triphosphate, 5-methyluridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

Further, lipid-modified artificial nucleic acid molecules may typically comprise at least one linker which is covalently linked with the artificial nucleic acid molecule, and at least one lipid which is covalently linked with this linker. Alternatively, a lipid-modified artificial nucleic acid molecule may comprise at least one artificial nucleic acid molecule as defined herein and at least one, preferably bifunctional lipid which is covalently linked, preferably without a linker, with that artificial nucleic acid molecule. According to a third alternative, a lipid-modified artificial nucleic acid molecule may comprise an artificial nucleic acid molecule as defined herein, at least one linker which is covalently linked with that artificial nucleic acid molecule, at least one lipid which is covalently linked with this linker, and additionally at least one, preferably bifunctional lipid which is covalently linked, preferably without a linker, with the artificial nucleic acid molecule.

In a further aspect, the present invention provides a vector comprising

a. at least one 5'-untranslated region element (5'UTR element) which comprises or consists of a nucleic acid sequence which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4),

an androgen-induced 1 gene (AIG1), cytochrome c oxidase subunit VIc gene (COX6C), or a N-acylsphingosine amidohydrolase (acid ceramidase) 1 gene (ASAH1) or from a functional fragment or a functional variant thereof, wherein the functional fragment or variant fulfils at least one function of the naturally occuring 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit VIc gene (COX6C), or a N-acylsphingosine amidohydrolase (acid ceramidase) 1 gene (ASAH1), wherein the at least one function is stabilizing the mRNA and/or stabilizing and/or prolonging protein production from an mRNA; and

b. at least one open reading frame (ORF) and/or at least one cloning site,

wherein the 5'UTR element and the open reading frame are heterologous.

Further disclosed herein is also a vector comprising

(a.) at least one 5'-untranslated region element (5'UTR element) which comprises or consists of a nucleic acid sequence which is derived from the 5'UTR of a TOP gene or which is derived from a variant of the 5'UTR of a TOP gene;

(b.) at least one open reading frame and/or at least one cloning site; and

(c.) optionally, at least one histone stem-loop.

The cloning site may be suitable for accepting an open reading frame, i.e. an open reading frame coding for a protein or peptide to be expressed may be cloned into the vector via the cloning site.

The at least one 5'UTR element, the at least one ORF, and the at least one optional histone stem-loop are as described herein for the artificial nucleic acid molecule disclosed herein. The cloning site may be any sequence that is suitable for introducing an open reading frame or a sequence comprising an open reading frame, such as one or more restriction sites.

Thus, the vector comprising a cloning site is preferably suitable for inserting an open reading frame into the vector. Preferably, it may be suitable for inserting an open reading frame between the 5'UTR element and a desired 3' structure such as a histone stem loop, a polyl(A) sequence, a polyadenylation signal and/or a 3'UTR element, more preferably it is suitable for insertion 5' to the 3' structure and 3' to the 5'UTR element. For example the 3' structure may comprise a histone stem-loop, a poly(A) sequence or a polyadenylation signal and/or a 3'UTR element as described above. Thereby the histone stem loop, the poly(A) sequence and/or the polyadenylation signal and the 3'UTR element may occur in any order that may be desired. Preferably, the cloning site or the ORF is located 5' to the 3'UTR structure, preferably in close proximity to the 5'-end of the histone stem-loop, poly(A) sequence, polyadenylation signal and/or a 3'UTR element as described above. For example, the cloning site or the ORF may be directly connected to the 5'-end of the histone stem-loop, poly(A) sequence, polyadenylation signal and/or a 3'UTR element or they may be connected via a stretch of nucleotides, such as by a stretch of 2, 4, 6, 8, 10, 20 etc. nucleotides as described above for the artificial nucleic acid molecule disclosed herein. Preferably, the cloning site or the ORF is located 3' to the 5'UTR element, preferably in close proximity to the 3'-end of the 5'UTR element. For example, the cloning site or the ORF may be directly connected to the 3'-end of the 5'UTR element or they may be connected via a stretch of nucleotides, such as by a stretch of 2, 4, 6, 8, 10, 20 etc. nucleotides as described above for the artificial nucleic acid molecule disclosed herein.

Preferably, the vector disclosed herein is suitable for producing the artificial nucleic acid molecule disclosed herein, preferably for producing an artificial mRNA disclosed herein, for example, by optionally inserting an open reading frame or a sequence comprising an open reading frame into the vector and transcribing the vector. Thus, preferably, the vector comprises elements needed for transcription, such as a promoter, e.g. an RNA polymerase promoter. Preferably, the vector is suitable for transcription using eukaryotic, prokaryotic, viral or phage transcription systems, such as eukaryotic cells, prokaryotic cells, or eukaryotic, prokaryotic, viral or phage *in vitro* transcription systems. Thus, for example, the vector may comprise a promoter sequence, which is recognizes by a polymerase, such as by an RNA polymerase, e.g. by a eukaryotic, prokaryotic, viral, or phage RNA polymerase. In a preferred embodiment, the vector comprises a phage RNA polymerase promoter such as an SP6 or T7, preferably a T7 promoter. Preferably, the vector is suitable for *in vitro* transcription using a phage based *in vitro* transcription system, such as a T7 RNA polymerase based *in vitro* transcription system.

The vector may further comprise a poly(A) sequence and/or a polyadenylation signal and/or a poly(C) sequence as described above for the artificial nucleic acid molecule disclosed herein.

The vector may be an RNA vector or a DNA vector. Preferably, the vector is a DNA vector. The vector may be any vector known to the skilled person, such as a viral vector or a plasmid vector. Preferably, the vector is a plasmid vector, preferably a DNA plasmid vector.

in a preferred embodiment, the vector disclosed herein comprises or codes for the artificial nucleic acid molecule disclosed herein.

Preferably, a vector disclosed herein comprises a sequence according to SEQ ID NOs. 1-1363, SEQ ID NO. 1435, SEQ ID NO. 1461, SEQ ID NO. 1462, or a sequence according to SEQ ID NOs. 1368 or 1452-1460, a fragment thereof as described above, or a corresponding RNA sequence, or a sequence having an identity of at least about 40%, preferably of at least about 50%, preferably of at least about 60%, preferably of at least about 70%, more preferably of at least about 80%, more preferably of at least about 90%, even more preferably of at least about 95%; even more preferably of at least about 99% to a sequence according to any one of SEQ ID NOs. 1-1363, SEQ ID NO. 1435, SEQ ID NO. 1461, SEQ ID NO. 1462, or a sequence according to SEQ ID NOs. 1368 or 1452-1460, a fragment thereof as described above, preferably a functional fragment thereof, or a corresponding RNA sequence.

Preferably, a vector disclosed herein comprises a sequence according to any one of SEQ ID Nos. 1369-1390 and 1434, a fragment thereof as described above or a corresponding RNA sequence, or a sequence having an identity of at least about 40%, preferably of at least about 50%, preferably of at least about 60%, preferably of at least about 70%, more preferably of at least about 80%, more preferably of at least about 90%, even more preferably of at least about 95%; even more preferably of at least about 99% to a sequence according to any one of SEQ ID Nos. 1369-1390 and 1434 or a fragment thereof as described above, preferably a functional fragment thereof, or a corresponding RNA sequence.

Preferably, a vector disclosed herein comprises a sequence according to any one of SEQ ID Nos. 1391-1433 or a corresponding RNA sequence, or a sequence having an identity of at least about 75%, preferably of at least about 80%, more preferably of at least about 85%, even more preferably of at least about 90%; even more preferably of at least about 95% to a sequence according to SEQ ID Nos. 1433 as described above or a corresponding RNA sequence.

Preferably, the vector is a circular molecule. Preferably, the vector is a double-stranded molecule, such as a double stranded DNA molecule. Such circular, preferably double stranded DNA molecule may be used conveniently as a storage form for the artificial nucleic acid molecule disclosed herein. Furthermore, it may be used for transfection of cells, for example, cultured cells. Also it may be used for *in vitro* transcription for obtaining an artificial RNA molecule disclosed herein.

Preferably, the vector, preferably the circular vector, is linearizable, for example, by restriction enzyme digestion. In a preferred embodiment, the vector comprises a cleavage site, such as a restriction site, preferably a unique cleavage site, located immediately 3' to the open reading frame or - if present - to the histone stem-loop, or - if present - to the poly(A) sequence or the polyadenylation signal, or - if present - to the 3'UTR element, or - if present - to the poly(C) sequence. Thus, preferably, the product obtained by linearizing the vector terminates at the 3'end with the 3'-end of the open reading frame, or - if present - with the 3'-end of the histone stem loop, or - if present - with the 3'-end of the poly(A) sequence or the 3'-end of the polyadenylation signal, or - if present - with the 3'-end of a 3'UTR element, plus some optional nucleotides, e.g. remaining from the restriction site after cleavage.

In a further aspect, the present invention relates to a cell as defined in the claims. Further provided is also a cell comprising the artificial nucleic acid molecule disclosed herein or the vector disclosed herein. The cell may be any cell, such as a bacterial cell, insect cell, plant cell, vertebrate cell, e.g. a mammalian cell. Such cell may be, e.g., used for replication of the vector disclosed herein, for example, in a bacterial cell. Furthermore, the cell may be used for transcribing the artificial nucleic acid molecule or the vector disclosed herein and/or translating the open reading frame of the artificial nucleic acid molecule or the vector disclosed herein. For example, the cell may be used for recombinant protein production.

The cells disclosed herein are, for example, obtainable by standard nucleic acid transfer methods, such as standard transfection methods. For example, the artificial nucleic acid molecule or the vector disclosed herein may be transferred into the cell by electroporation, lipofection, e.g. based on cationic lipids and/or liposomes, calcium phosphate precipitation, nanoparticle based transfection, virus based transfection, or based on cationic polymers, such as DEAE-dextran or polyethylenimine etc.

Preferably, the cell is a mammalian cell, such as a cell of a human subject, a domestic animal, a laboratory animal, such as a mouse or rat cell. Preferably the cell is a human cell. The cell may be a cell of an established cell line, such as a CHO, BHK, 293T, COS-7, HELA, HEK etc. cell, or the cell may be a primary cell, such as a HDF cell, preferably a cell isolated from an organism. In a preferred embodiment, the cell is an isolated cell of a mammalian subject, preferably of a human subject. For example, the cell may be an immune cell, such as a dendritic cell, a cancer or tumor cell, or any somatic cell etc., preferably of a mammalian subject, preferably of a human subject.

In a further aspect, the present invention provides a pharmaceutical composition as defined in the claims. Further disclosed is also a pharmaceutical composition comprising the artificial nucleic acid molecule disclosed herein, the vector disclosed herein, or the cell disclosed herein. The pharmaceutical composition disclosed herein may be used, e.g., as a vaccine, for example, for genetic vaccination. Thus, the ORF may, e.g., encode an antigen to be administered to a patient for vaccination. Thus, in a preferred embodiment, the pharmaceutical composition disclosed herein is a vaccine. Furthermore, the pharmaceutical composition disclosed herein may be used, e.g., for gene therapy.

Preferably, the pharmaceutical composition further comprises one or more pharmaceutically acceptable excipients, vehicles, fillers and/or diluents. In the context of the present disclosure, a pharmaceutically acceptable vehicle typically includes a liquid or non-liquid basis for the pharmaceutical composition disclosed herein. In one embodiment, the pharmaceutical composition is provided in liquid form. In this context, preferably, the vehicle is based on water, such as pyrogen-free water, isotonic saline or buffered (aqueous) solutions, e.g phosphate, citrate etc. buffered solutions. The buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of mammalian cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

One or more compatible solid or liquid fillers or diluents or encapsulating compounds suitable for administration to a patient may be used as well for the pharmaceutical composition disclosed herein. The term "compatible" as used herein preferably means that these components of the pharmaceutical composition disclosed herein are capable of being mixed with the nucleic acid, vector or cells as defined herein in such a manner that no interaction occurs which would substantially reduce the pharmaceutical effectiveness of the pharmaceutical composition disclosed herein under typical use conditions.

The pharmaceutical composition disclosed herein may optionally further comprise one or more additional pharmaceutically active components. A pharmaceutically active component in this context is a compound that exhibits a therapeutic effect to heal, ameliorate or prevent a particular indication or disease. Such compounds include, without implying any limitation, peptides or proteins, nucleic acids, (therapeutically active) low molecular weight organic or inorganic compounds (molecular weight less than 5000, preferably less than 1000), sugars, antigens or antibodies, therapeutic agents already known in the prior art, antigenic cells, antigenic cellular fragments, cellular fractions, cell wall components (e.g. polysaccharides), modified, attenuated or de-activated (e.g. chemically or by irradiation) pathogens (virus, bacteria etc.).

Furthermore, the pharmaceutical composition disclosed herein may comprise a carrier for the artificial nucleic acid molecule or the vector. Such a carrier may be suitable for mediating dissolution in physiological acceptable liquids, transport and cellular uptake of the pharmaceutical active artificial nucleic acid molecule or the vector. Accordingly, such a carrier may be a component which may be suitable for depot and delivery of an artificial nucleic acid molecule or vector disclosed herein. Such components may be, for example, cationic or polycationic carriers or compounds which may serve as transfection or complexation agent.

Particularly preferred transfection or complexation agents in this context are cationic or polycationic compounds, including protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*), pAntp, pIsl, FGF, Lactoferrin, Transportan, Buforin-2, Bac715-24, SynB, SynB(1), pVEC, hCT-derived peptides, SAP, or histones.

Furthermore, such cationic or polycationic compounds or carriers may be cationic or polycationic peptides or proteins, which preferably comprise or are additionally modified to comprise at least one -SH moiety. Preferably, a cationic or polycationic carrier is selected from cationic peptides having the following sum formula (III):

$$\{(Arg)_l;(Lys)_m;(His)_n;(Orn)_o;(Xaa)_x\}; \qquad \text{formula (III)}$$

wherein l + m + n + o+ x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred.

Further, the cationic or polycationic peptide or protein, when defined according to formula $\{(Arg)_l;(Lys)_m;(His)_n;(Orn)_o;(Xaa)_x\}$ (formula (III)) as shown above and which comprise or are additionally modified to comprise at least one -SH moeity, may be, without being restricted thereto, selected from subformula (IIIa):

$\{(Arg)_l;(Lys)_m;(His)_n;(Orn)_o;(Xaa')_x(Cys)_y\}$ subformula (IIIa)

wherein $(Arg)_l;(Lys)_m;(His)_n;(Orn)_o$; and x are as defined herein, Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide. Further, the cationic or polycationic peptide may be selected from subformula (IIIb):

Cys, $\{(Arg)_l;(Lys)_m;(His)_n;(Orn)_o;(Xaa)_x\}$ $Cys_2$ subformula (IIIb)

wherein empirical formula $\{(Arg)_l;(Lys)_m;(His)_n;(Orn)_o;(Xaa)_x\}$ (formula (III)) is as defined herein and forms a core of an amino acid sequence according to (semiempirical) formula (III) and wherein Cys, and $Cys_2$ are Cysteines proximal to, or terminal to $(Arg)_l;(Lys)_m;(His)_n;(Orn)_o;(Xaa)_x$.

Further preferred cationic or polycationic compounds, which can be used as transfection or complexation agent may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-($\alpha$-trimethylammonio-acetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as $\beta$-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g polyethyleneglycole); etc.

In this context, it is particularly preferred that the artificial nucleic acid molecule or the vector disclosed herein is complexed at least partially with a cationic or polycationic compound, preferably cationic proteins or peptides. Partially means that only a part of the artificial nucleic acid molecule disclosed herein or the vector disclosed herein is complexed with a cationic or polycationic compound and that the rest of the artificial nucleic acid molecule disclosed herein or the vector disclosed herein is in uncomplexed form ("free"). Preferably the ratio of complexed nucleic acid to: free nucleic acid is selected from a range. of about 5:1 (w/w) to about 1:10 (w/w), more preferably from a range of about 4:1 (w/w) to about 1:8 (w/w), even more preferably from a range of about 3:1 (w/w) to about 1:5 (w/w) or 1:3 (w/w), and most preferably the ratio of complexed nucleic acid to free nucleic acid is selected from a ratio of about 1:1 (w/w).

The pharmaceutical composition disclosed herein may optionally further comprise one or more adjuvants, for example, adjuvants for stimulating the innate immune system or for enhancing cellular uptake of the artificial nucleic acid molecule or vector. In this context, an adjuvant may be understood as any compound, which is suitable to initiate or increase an immune response of the innate immune system, i.e. a non-specific immune response. In other words, when administered, the pharmaceutical composition disclosed herein preferably elicits an innate immune response due to the adjuvant, optionally contained therein. Preferably, such an adjuvant may be an adjuvant supporting the induction of an innate immune response in a mammal. Such an adjuvant may be, for example, an immunostimulatory nucleic acid, i.e. a nucleic acid that may bind to a Toll-like-receptor or the like, preferably an immunostimulatory RNA.

Such adjuvants, preferably such immunostimulatory nucleic acids, may induce an innate, i.e. unspecific, immune response which may support a specific, i.e. adaptive, immune response to the peptide or protein, i.e. the antigen, encoded by the artificial nucleic acid molecule of the pharmaceutical composition, preferably the vaccine.

The pharmaceutical composition disclosed herein may also additionally comprise any further compound, which is known to be immunostimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Further additives which may be included in the pharmaceutical composition disclosed herein are, e.g., emulsifiers, such as, for example, Tween®; wetting agents, such as, for example, sodium lauryl sulfate; colouring agents; taste-imparting agents, pharmaceutical carriers; tablet-forming agents; stabilizers; antioxidants; preservatives etc.

The pharmaceutical composition disclosed herein preferably comprises a "safe and effective amount" of the components of the pharmaceutical composition, particularly of the nucleic acid sequence, the vector and/or the cells as defined herein. As used herein, a "safe and effective amount" means an amount sufficient to significantly induce a positive modification of a disease or disorder as defined herein. At the same time, however, a "safe and effective amount" preferably avoids serious side-effects and permits a sensible relationship between advantage and risk. The determination of these limits typically lies within the scope of sensible medical judgment.

In a further aspect, the present invention provides the artificial nucleic acid as defined in the claims, the vector as defined in the claims, the cell as defined in the claims or the pharmaceutical composition as defined in the claims for use as a medicament, preferably for use as a vaccine or for use in gene therapy. Further disclosed herein is also the artificial nucleic acid molecule disclosed herein, the vector disclosed herein, the cell disclosed herein, or the pharmaceutical composition disclosed herein for use as a medicament, for example, as vaccine (in genetic vaccination) or in gene therapy.

The artificial nucleic acid molecule disclosed herein, the vector disclosed herein, the cell disclosed herein, or the pharmaceutical composition disclosed herein are particularly suitable for any medical application which makes use of the therapeutic action or effect of peptides, polypeptides or proteins, or where supplementation of a particular peptide or protein is needed. Thus, the present disclosure provides the artificial nucleic acid molecule disclosed herein, the vector disclosed herein, the cell disclosed herein, or the pharmaceutical composition disclosed herein for use in the treatment or prevention of diseases or disorders amenable to treatment by the therapeutic action or effect of peptides, polypeptides or proteins or amenable to treatment by supplementation of a particular peptide, polypeptide or protein.

For example, the artificial nucleic acid molecule disclosed herein, the vector disclosed herein, the cell disclosed herein, or the pharmaceutical composition disclosed herein may be used for the treatment or prevention of genetic diseases, autoimmune diseases, cancerous or tumour-related diseases, infectious diseases, chronic diseases or the like, e.g., by genetic vaccination or gene therapy.

In particular, such therapeutic treatments which benefit from a stable, prolonged and/or increased presence of therapeutic peptides, polypeptides or proteins in a subject to be treated are especially suitable as medical application in the context of the present disclosure, since the 5'UTR element in particular in combination with a histone stem-loop provides for increased protein expression from the ORF of the nucleic acid molecule disclosed herein. Thus, a particularly suitable medical application for the artificial nucleic acid molecule disclosed herein, the vector disclosed herein, the cell disclosed herein, or the pharmaceutical composition disclosed herein is vaccination, for example against infections or tumours. Thus, the present disclosure provides the artificial nucleic acid molecule disclosed herein, the vector disclosed herein, the cell disclosed herein, or the pharmaceutical composition disclosed herein for vaccination of a subject, preferably a mammalian subject, more preferably a human subject. Preferred vaccination treatments are vaccination against infectious diseases, such as bacterial, protozoal or viral infections, and anti-tumour-vaccination. Such vaccination treatments may be prophylactic or therapeutic.

Depending on the disease to be treated or prevented, the ORF may be selected. For example, the open reading frame may code for a protein that has to be supplied to a patient suffering from total lack or at least partial loss of function of a protein, such as a patient suffering from a genetic disease. Additionally, the open reading frame may be chosen from an ORF coding for a peptide or protein which beneficially influences a disease or the condition of a subject. Furthermore, the open reading frame may code for a peptide or protein which effects down-regulation of a pathological overproduction of a natural peptide or protein or elimination of cells expressing pathologically a protein or peptide. Such lack, loss of function or overproduction may, e.g., occur in the context of tumour and neoplasia, autoimmune diseases, allergies, infections, chronic diseases or the like. Furthermore, the open reading frame may code for an antigen or immunogen, e.g. for an epitope of a pathogen or for a tumour antigen. Thus, in preferred embodiments, the artificial nucleic acid molecule or the vector disclosed herein comprises an ORF encoding an amino acid sequence comprising or consisting of an antigen or immunogen, e.g. an epitope of a pathogen or a tumour-associated antigen, a 5'UTR element as described above, preferably a histone stem-loop as described herein, and optional further components, such as a 3'UTR element and/or a poly(A) sequence and/or a poly(C) sequence etc. as described herein.

In the context of medical application, in particular, in the context of vaccination, it is preferred that the artificial nucleic acid molecule disclosed herein is RNA, preferably mRNA, since DNA harbours the risk of eliciting an anti-DNA immune response and tends to insert into genomic DNA. However, in some embodiments, for example, if a viral delivery vehicle, such as an adenoviral delivery vehicle is used for delivery of the artificial nucleic acid molecule or the vector disclosed herein, e.g., in the context of gene therapeutic treatments, it may be desirable that the artificial nucleic acid molecule or the vector is a DNA molecule.

The artificial nucleic acid molecule disclosed herein, the vector disclosed herein, the cell disclosed herein, or the pharmaceutical composition disclosed herein may be administered orally, parenterally, by inhalation spray, topically,

rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques.

Preferably, the artificial nucleic acid molecule disclosed herein, the vector disclosed herein, the cell disclosed herein, or the pharmaceutical composition disclosed herein is administered parenterally, e.g. by parenteral injection, more preferably by subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, sublingual injection or via infusion techniques. Particularly preferred is intradermal and intramuscular injection. Sterile injectable forms of the pharmaceutical composition disclosed herein may be aqueous or oleaginous suspension. These suspensions may be formulated according to techniques known in the art using suitable dispersing or wetting agents and suspending agents.

The artificial nucleic acid molecule disclosed herein, the vector disclosed herein, the cell disclosed herein, or the pharmaceutical composition disclosed herein may also be administered orally in any orally acceptable dosage form including, but not limited to, capsules, tablets, aqueous suspensions or solutions.

The artificial nucleic acid molecule disclosed herein, the vector disclosed herein, the cell disclosed herein, or the pharmaceutical composition disclosed herein may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the artificial nucleic acid molecule disclosed herein, the vector disclosed herein, the cell disclosed herein, or the pharmaceutical composition disclosed herein may be formulated in a suitable ointment suspended or dissolved in one or more carriers.

In one embodiment, the use as a medicament comprises the step of transfection of mammalian cells, preferably *in vitro* transfection of mammalian cells, more preferably *in vitro* transfection of isolated cells of a subject to be treated by the medicament. If the use comprises the *in vitro* transfection of isolated cells, the use as a medicament may further comprise the (re)administration of the transfected cells to the patient. The use of the artificial nucleic acid molecules or the vector disclosed herein as a medicament may further comprise the step of selection of successfully transfected isolated cells. Thus, it may be beneficial if the vector further comprises a selection marker. Also, the use as a medicament may comprise *in vitro* transfection of isolated cells and purification of an expression-product, i.e. the encoded peptide or protein from these cells. This purified peptide or protein may subsequently be administered to a subject in need thereof.

The present disclosure also provides a method for treating or preventing a disease or disorder as described above comprising administering the artificial nucleic acid molecule disclosed herein, the vector disclosed herein, the cell disclosed herein, or the pharmaceutical composition disclosed herein to a subject in need thereof.

As described above, the 5'UTR element, preferably, the histone stem-loop, and optionally the poly(A)sequence and/or the 3'UTR element are capable of increasing protein production from an artificial nucleic acid molecule, such as an mRNA or vector, comprising these elements and an ORF, preferably in an at least additive, preferably in a synergistic manner. Thus, in a further aspect, the present invention relates to an *in vitro* method for increasing protein production from an artificial nucleic acid molecule, comprising the step of providing the artificial nucleic acid molecule with

i. at least one 5'-untranslated region element (5'UTR element) which comprises or consists of a nucleic acid sequence, which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (1 7-beta) dehydrogenase 4 gene 15 (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a functional fragment or a functional variant thereof, wherein the functional fragment or variant fulfils at least one function of the naturally occuring 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD1 7B4), an androgen-induced 1 gene (AIG1), cytochrome c oxidase subunit VIc gene (COX6C), or a N-acylsphingosine amidohydrolase (acid ceramidase) 1 gene (ASAH1), wherein the at least one function is stabilizing the mRNA and/or stabilizing and/or prolonging protein production from an mRNA,

ii. preferably at least one histone stem-loop; and

iii. optionally, a poly(A) sequence and/or a polyadenylation signal.

Further disclosed herein is also a method for increasing protein production from an artificial nucleic acid molecule comprising the step of associating the artificial nucleic acid molecule, preferably an ORF contained within the artificial nucleic acid molecule, with (i) at least one 5'-untranslated region element (5'UTR element) which comprises or consists

of a nucleic acid sequence which is derived from the 5'UTR of a TOP gene or which is derived from a variant of the 5'UTR of a TOP gene as described above, preferably (ii) at least one histone stem-loop as described herein, and optionally one or more futher elements, such as a poly(A)sequence and/or polyadenylation signal, and/or a poly(C) sequence, and/or a 3'UTR element, which comprises or consists of a nucleic acid sequence derived from the 3'UTR of a chordate gene, preferably a vertebrate gene, more preferably a mammalian gene, most preferably a human gene, or from a variant of the 3'UTR of a chordate gene, preferably a vertebrate gene, more preferably a mammalian gene, most preferably a human gene as described above.

Associating the artificial nucleic acid molecule or the vector with a 5 'UTR element and preferably a histone stem-loop as well as optional further elements in the context of the present disclosure preferably means functionally associating or functionally combining an artificial nucleic acid molecule, e.g. comprising an ORF, such as an mRNA or a vector, with the 5'UTR element and optionally the histone stem-loop and/or the poly(A) sequence and/or the 3'UTR element. This means that the artificial nucleic acid molecule, preferably the ORF contained within the artificial nucleic acid molecule, the 5'UTR element and preferably the histone stem-loop and the optional further elements, such as the poly(A)sequence and/or the 3'UTR element as described above, are associated or coupled such that the function of the 5'UTR element and the histone stem-loop and the optional further elements, e.g. protein production increasing function, is exerted. Typically, this means that the 5'UTR element and the histone stem-loop and optionally the poly(A)sequence and/or the 3'UTR element are integrated into the artificial nucleic acid molecule, preferably into the mRNA molecule or the vector, such that the open reading frame is positioned between the 5'UTR element and the optional histone stem-loop and the optional poly(A)sequence and/or the optional 3'UTR element.

The product of said method is preferably the artificial nucleic acid molecule disclosed herein or the vector disclosed herein. Thus, e.g. the nature and sequence of the elements, such as the 5'UTR element, the histone stem-loop, the poly(A) sequence, the polyadenylation signal, the poly(C) sequence, and the 3'UTR element are as described above for the artificial nucleic acid molecule disclosed herein or the vector disclosed herein.

In a further aspect, the present invention provides the in vitro use of a 5'UTR element, which comprises or consists of a nucleic acid sequence, which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit Vic gene (COX6C), or a N-acylsphingosine amidohydrolase (acid ceramidase) 1 gene (ASAH1) or from a functional fragment or a functional variant thereof, wherein the functional fragment or variant fulfils at least one function of the naturally occuring 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (1 7-beta) dehydrogenase 4 gene 15 (HSD1 7B4), an androgen-induced 1 gene (AIG1), cytochrome c oxidase subunit VIc gene (COX6C), or a N-acylsphingosine amidohydrolase (acid ceramidase) 1 gene (ASAH1), wherein the at least one function is stabilizing the mRNA and/or stabilizing and/or prolonging protein production from an mRNA, and preferably further comprises at least one histone stem-loop for increasing protein production from a nucleic acid molecule.

Further disclosed herein is also the use of preferably at least one histone stem-loop, and optionally further elements, such as a poly(A)sequence and/or a polyadenylation signal, and/or a poly(C) signal), and/or a 3'UTR element which comprises or consists of a nucleic acid sequence derived from the 3'UTR of a chordate gene, preferably a vertebrate gene, more preferably a mammalian gene, most preferably a human gene, or from a variant of the 3'UTR of a chordate gene, preferably a vertebrate gene, more preferably a mammalian gene, most preferably a human gene as described above for increasing protein production from an artificial nucleic acid molecule, such as an mRNA or a vector.

The use disclosed herein preferably comprises associating the artificial nucleic acid molecule with the 5'UTR element, preferably the histone stem-loop and optional further elements, such as a poly(A)sequence or 3'UTR element etc., as described above.

The compounds and ingredients of the pharmaceutical composition disclosed herein may also be manufactured and traded separately of each other. Thus, the invention relates further to a kit or kit of parts as defined in the claims, the kit or kit of parts comprising an artificial nucleic acid molecule as defined in the claims, the vector as defined in the claims, the cell as defined in the claims, and/or the pharmaceutical composition as defined in the claims, the kit or kit of parts preferably further comprising instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector or the pharmaceutical composition.

Further disclosed herein is also a kit or kit of parts comprising an artificial nucleic acid molecule disclosed herein, a vector disclosed herein, a cell disclosed herein, and/or a pharmaceutical composition disclosed herein. Preferably, such kit or kit of parts may, additionally, comprise instructions for use, cells for transfection, an adjuvant, a means for administration of the pharmaceutical composition, a pharmaceutically acceptable carrier and/or an pharmaceutically acceptable solution for dissolution or dilution of the artificial nucleic acid molecule, the vector, the cells or the pharmaceutical composition.

The following Figures, Sequences and Examples are intended to illustrate the invention further. They are not intended to limit the subject-matter of the invention thereto.

Figure 1: shows the histone stem-loop consensus sequence generated from metazoan and protozoan stem-loop sequences (as reported by Dávila López, M., & Samuelsson, T. (2008), RNA (New York, N.Y.), 14(1), 1-10. doi:10.1261/rna.782308). 4001 histone stem-loop sequences from metazoa and protozoa were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

Figure 2: shows the histone stem-loop consensus sequence generated from protozoan stem-loop sequences (as reported by Dávila López, M., & Samuelsson, T. (2008), RNA (New York, N.Y.), 14(1), 1-10. doi:10.1261/rna.782308). 131 histone stem-loop sequences from protozoa were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

Figure 3: shows the histone stem-loop consensus sequence generated from metazoan stem-loop sequences (as reported by Dávila López, M., & Samuelsson, T. (2008), RNA (New York, N.Y.), 14(1), 1-10. doi:10.1261/rna.782308). 3870 histone stem-loop sequences from metazoa were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

Figure 4: shows the histone stem-loop consensus sequence generated from vertebrate stem-loop sequences (as reported by Dávila López, M., & Samuelsson, T. (2008), RNA (New York, N.Y.), 14(1), 1-10. doi:10.1261/rna.782308). 1333 histone stem-loop sequences from vertebrates were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

Figure 5: shows the histone stem-loop consensus sequence generated from human (Homo sapiens) stem-loop sequences (as reported by Dávila López, M., & Samuelsson, T. (2008), RNA (New York, N.Y.), 14(1), 1-10. doi:10.1261/rna.782308). 84 histone stem-loop sequences from humans were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

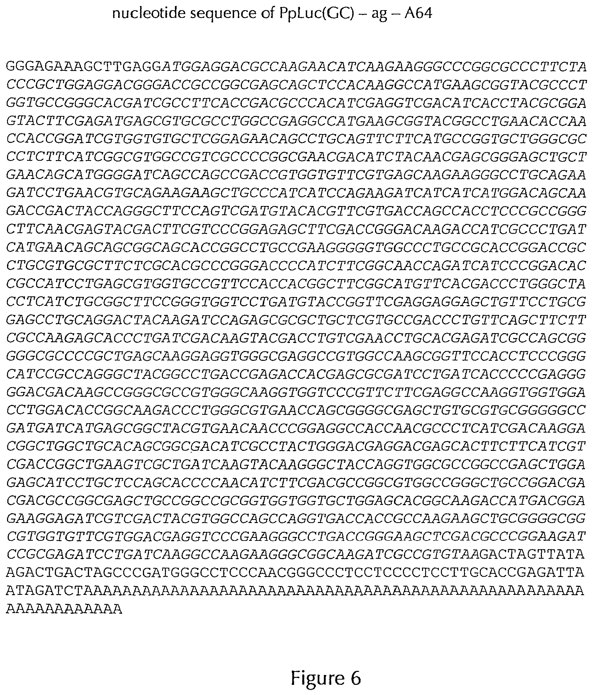

Figure 6 shows the nucleotide sequence of a *Photinus pyralis* luciferase encoding nucleic acid molecule PpLuc(GC) - ag - A64. This artificial construct does not comprise a 5'UTR element or a histone stem loop. The coding region for PpLuc(GC) is depicted in italics. The sequence depicted in Figure 6 corresponds to SEQ ID No. 1364.

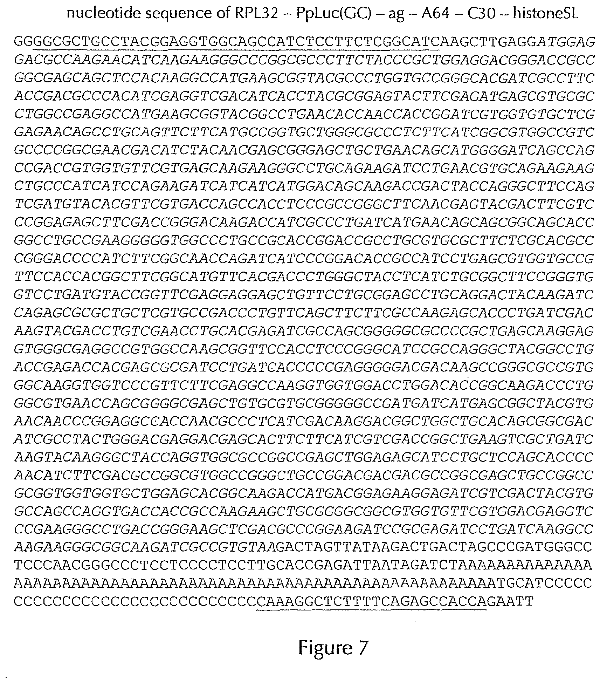

Figure 7 shows the nucleotide sequence of RPL32 - PpLuc(GC) - ag - A64 - C30 - histoneSL. The 5'UTR of human ribosomal protein Large 32 lacking the 5' terminal oligopyrimidine tract was inserted 5' of the ORF. A histoneSL was appended 3' of A64 poly(A). The coding region for PpLuc(GC) is depicted in italics. The 5'UTR element sequence and the histone stem-loop sequence are underlined. The sequence depicted in Figure 7 corresponds to SEQ ID No. 1365.

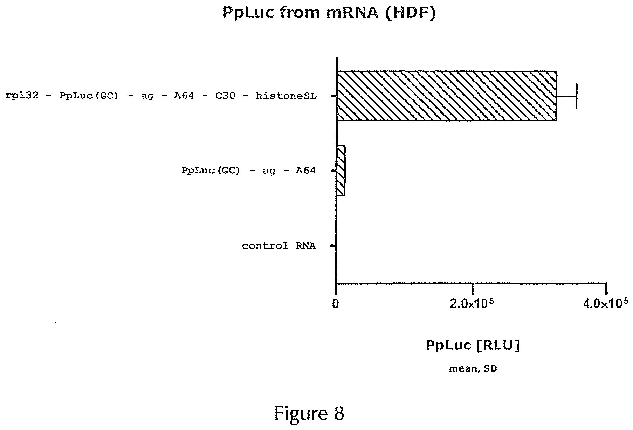

Figure 8 shows that the combination of the 5'UTR element derived from the 5'UTR of the TOP gene RPL32 and a histone stem-loop increases protein production from mRNA strongly. The effect of the combination of the 5'UTR element and the histone stem-loop on luciferase expression from mRNA was examined. To this

end, different mRNAs were transfected into human dermal fibroblasts (HDF) by lipofection. Luciferase levels were measured at 24 hours after transfection. Luciferase was clearly expressed from mRNA having neither 5'UTR element nor histoneSL. Strikingly however, the combination of 5'UTR element and histoneSL strongly increased the luciferase level. The magnitude of the rise in luciferase level due to combining 5'UTR element and histoneSL in the same mRNA indicates that they are acting synergistically. Data are graphed as mean RLU ± SD (relative light units ± standard deviation) for duplicate transfections. RLU are summarized in Example 5.1.

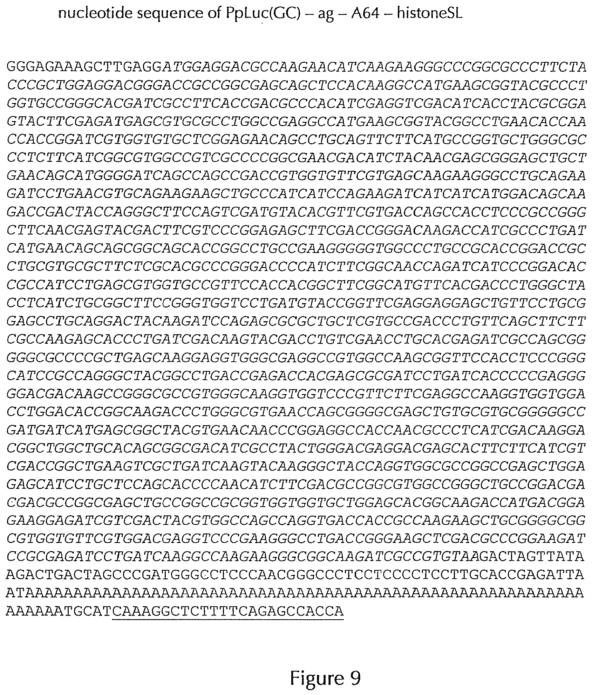

Figure 9 shows the nucleotide sequence of PpLuc(GC) - ag - A64 - histoneSL. A histoneSL was appended 3' of A64 poly(A). The coding region for PpLuc(GC) is depicted in italics. The histone stem-loop sequence is underlined. The sequence depicted in Figure 9 corresponds to SEQ ID No. 1464.

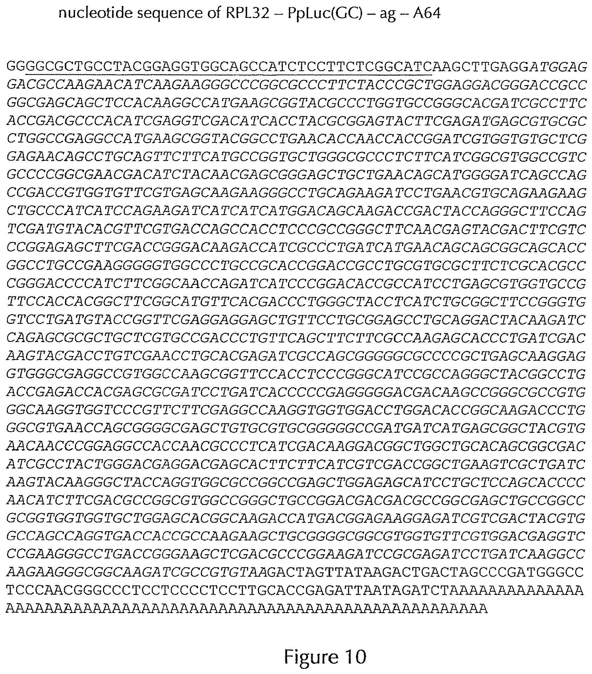

Figure 10 shows the nucleotide sequence of rpl32 - PpLuc(GC) - ag - A64. The 5'UTR of human ribosomal protein Large 32 lacking the 5' terminal oligopyrimidine tract was inserted 5' of the ORF. The coding region for PpLuc(GC) is depicted in italics. The 5'UTR element sequence is underlined. The sequence depicted in Figure 10 corresponds to SEQ ID No. 1463.

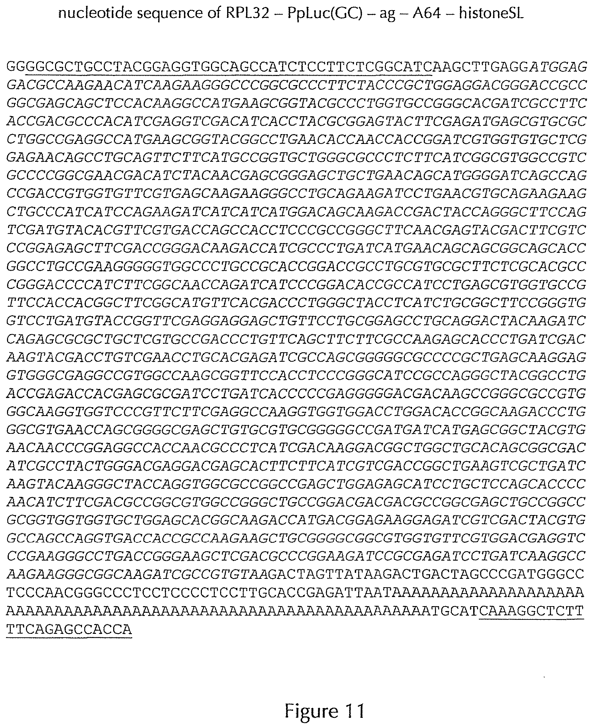

Figure 11 shows the nucleotide sequence of rpl32 - PpLuc(GC) - ag - A64 - histoneSL. The 5'UTR of human ribosomal protein Large 32 lacking the 5' terminal oligopyrimidine tract was inserted 5' of the ORF. A histoneSL was appended 3' of A64 poly(A). The coding region for PpLuc(GC) is depicted in italics. The 5'UTR element sequence and the histone stem-loop sequence are underlined. The sequence depicted in Figure 11 corresponds to SEQ ID No. 1480.

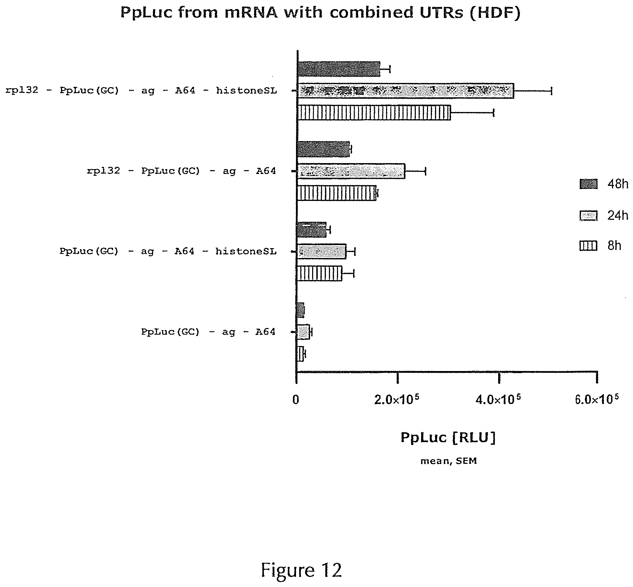

Figure 12 is a graphical representation of the effect of the 5'UTR element derived from the 5'UTR of the TOP gene RPL32, the histone stem-loop, and the combination of the 5'UTR element and the histone stem-loop on luciferase expression from mRNA. A variety of mRNAs were transfected into human dermal fibroblasts (HDF) by lipofection. Luciferase levels were measured at 8, 24, and 48 hours after transfection. Both, either the histone stem-loop or the 5'UTR element increase luciferase levels compared to mRNA lacking both these elements. Strikingly, the combination of 5'UTR element and histone stem-loop further strongly increases the luciferase level, much above the level observed with either of the individual elements, thus acting synergistically. Data are graphed as mean RLU ± SEM (relative light units ± standard error) for triplicate transfections. RLU are summarized in Example 5.2.

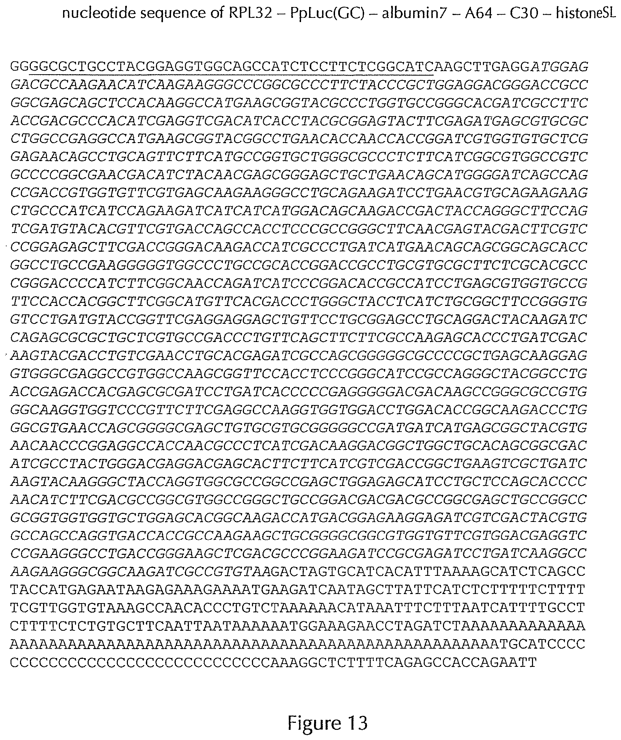

Figure 13 shows the nucleotide sequence of rpl32 - PpLuc(GC) - albumin7 - A64 - C30 - histoneSL. The albumin7 3'UTR element replaced the alpha-globin 3'UTR element in the construct shown in Figure 7 (which contains the rpl32 5'UTR element). The 5'UTR element sequence is underlined. The sequence depicted in Figure 13 corresponds to SEQ ID No. 1481.

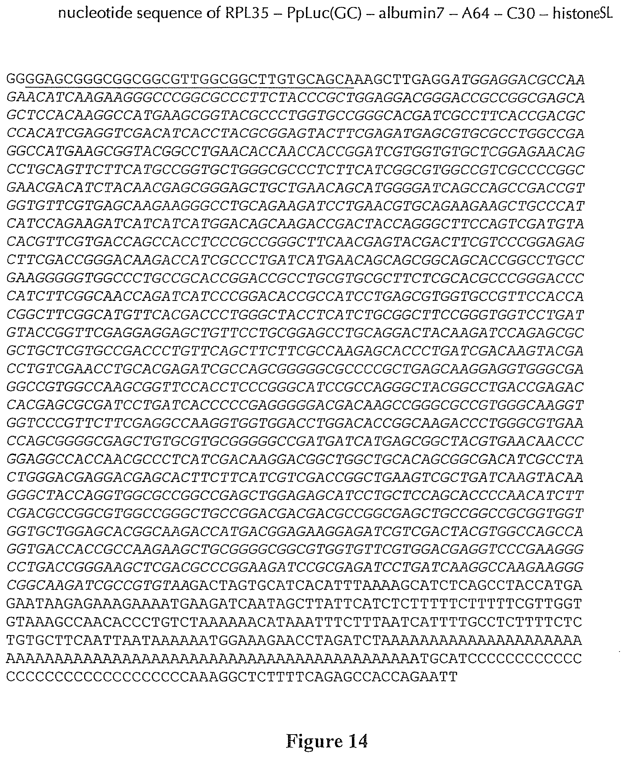

Figure 14 shows the nucleotide sequence of rpl35 - PpLuc(GC) - albumin7 - A64 - C30 - histoneSL. The 5'UTR of human ribosomal protein Large 35 lacking the 5' terminal oligopyrimidine tract replaced the rpl32 5'UTR element in the construct shown in Figure 13. The 5'UTR element sequence is underlined. The sequence depicted in Figure 14 corresponds to SEQ ID No. 1436.

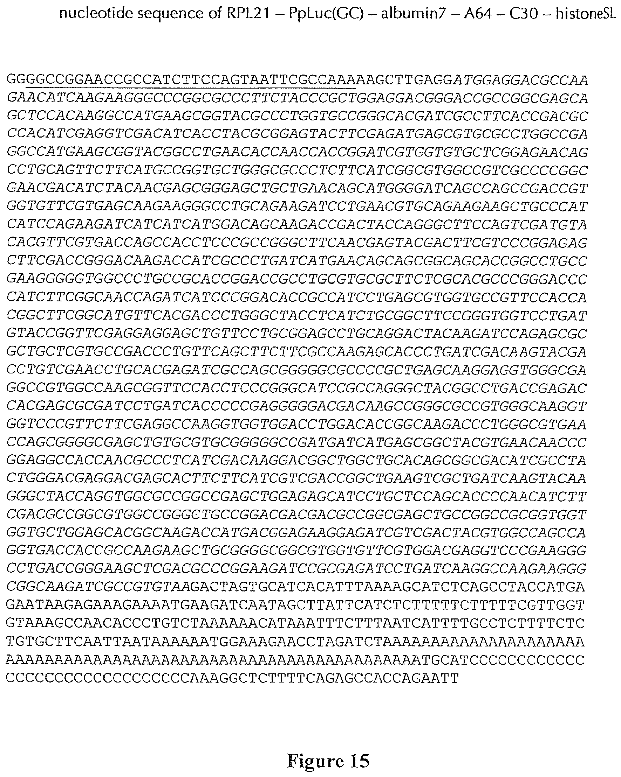

Figure 15 shows the nucleotide sequence of rpl21 - PpLuc(GC) - albumin7 - A64 - C30 - histoneSL. The 5'UTR of human ribosomal protein Large 21 lacking the 5' terminal oligopyrimidine tract replaced the rpl32 5'UTR element in the construct shown in Figure 13. The 5'UTR element sequence is underlined. The sequence depicted in Figure 15 corresponds to SEQ ID No. 1437.

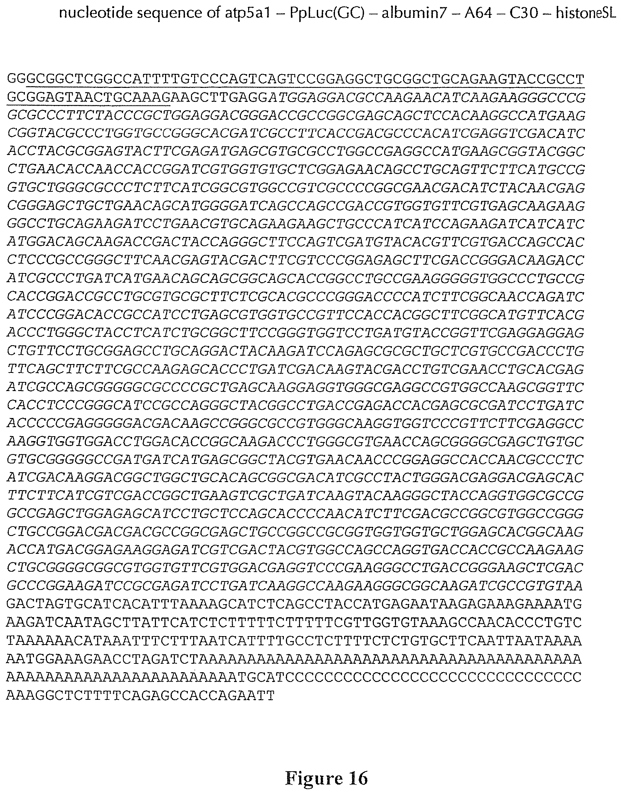

Figure 16 shows the nucleotide sequence of atp5a1 - PpLuc(GC) - albumin7 - A64 - C30 - histoneSL. The 5'UTR of human ATP synthase, H+ transporting, mitochondrial F1 complex, alpha subunit 1 lacking the 5' terminal oligopyrimidine tract replaced the rpl32 5'UTR element in the construct shown in Figure 13. The 5'UTR element sequence is underlined. The sequence depicted in Figure 16 corresponds to SEQ ID No. 1438.

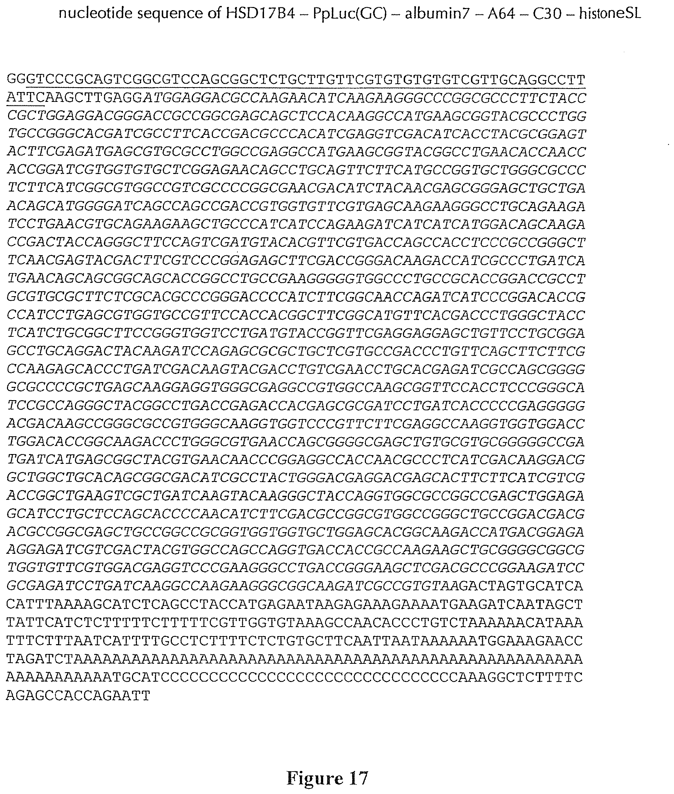

Figure 17 shows the nucleotide sequence of HSD17B4 - PpLuc(GC) - albumin7 - A64 - C30 - histoneSL. The 5'UTR of human hydroxysteroid (17-beta) dehydrogenase 4 lacking the 5' terminal oligopyrimidine tract replaced the rpl32 5'UTR element in the construct shown in Figure 13. The 5'UTR element sequence is underlined. The sequence depicted in Figure 17 corresponds to SEQ ID No. 1439.

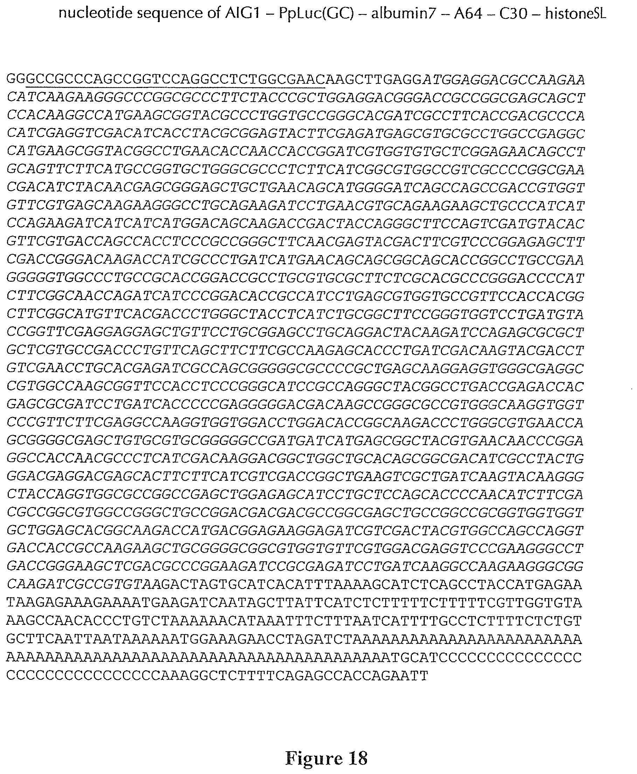

Figure 18 shows the nucleotide sequence of AIG1 - PpLuc(GC) - albumin7 - A64 - C30 - histoneSL. The 5'UTR of human androgen-induced 1 lacking the 5' terminal oligopyrimidine tract replaced the rpl32 5'UTR element in the construct shown in Figure 13. The 5'UTR element sequence is underlined. The sequence depicted in Figure 18 corresponds to SEQ ID No. 1440.

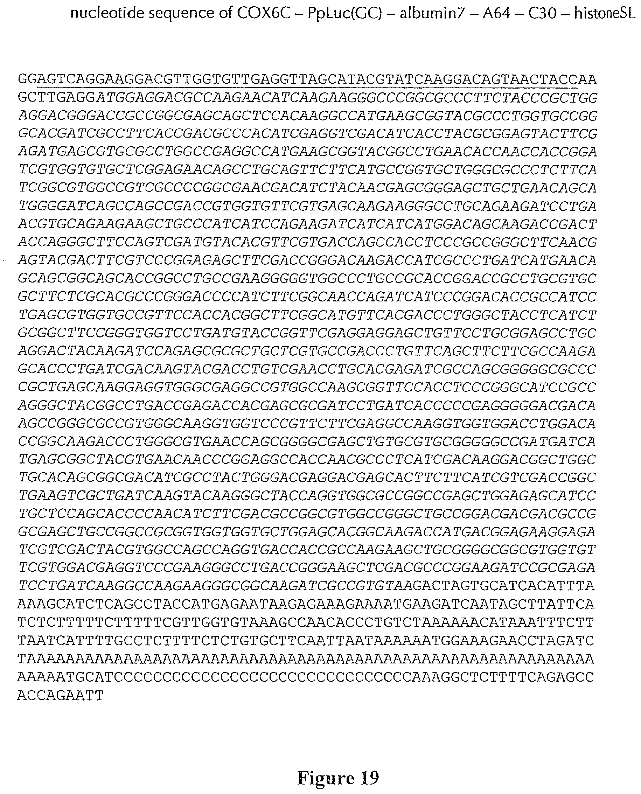

Figure 19 shows the nucleotide sequence of COX6C - PpLuc(GC) - albumin7 - A64 - C30 - histoneSL. The 5'UTR of human cytochrome c oxidase subunit VIc lacking the 5' terminal oligopyrimidine tract replaced the rpl32 5'UTR element in the construct shown in Figure 13. The 5'UTR element sequence is underlined. The sequence depicted in Figure 19 corresponds to SEQ ID No. 1441.

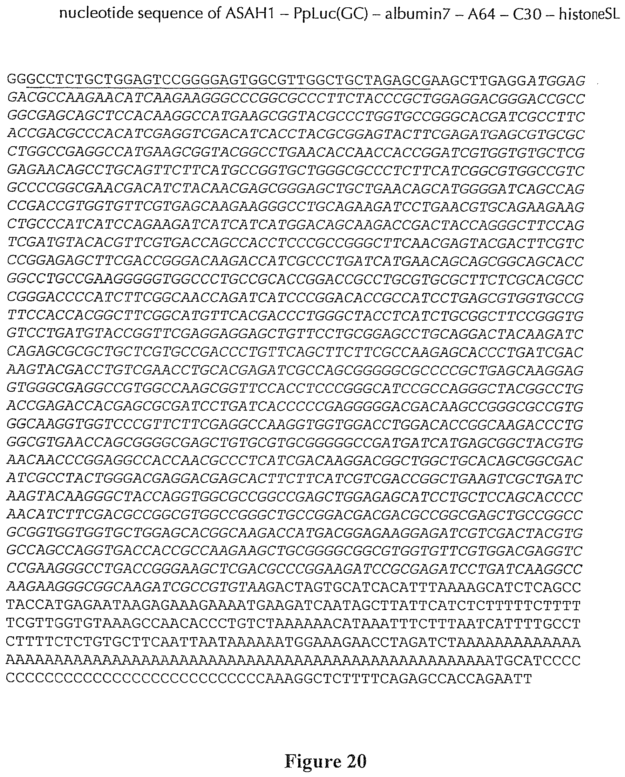

Figure 20 shows the nucleotide sequence of ASAH 1 - PpLuc(GC) - albumin7 - A64 - C30 - histoneSL. The 5'UTR of human N-acylsphingosine amidohydrolase (acid ceramidase) 1 lacking the 5' terminal oligopyrimidine tract replaced the rpl32 5'UTR element in the construct shown in Figure 13. The 5'UTR element sequence is underlined. The sequence depicted in Figure 20 corresponds to SEQ ID No. 1442.

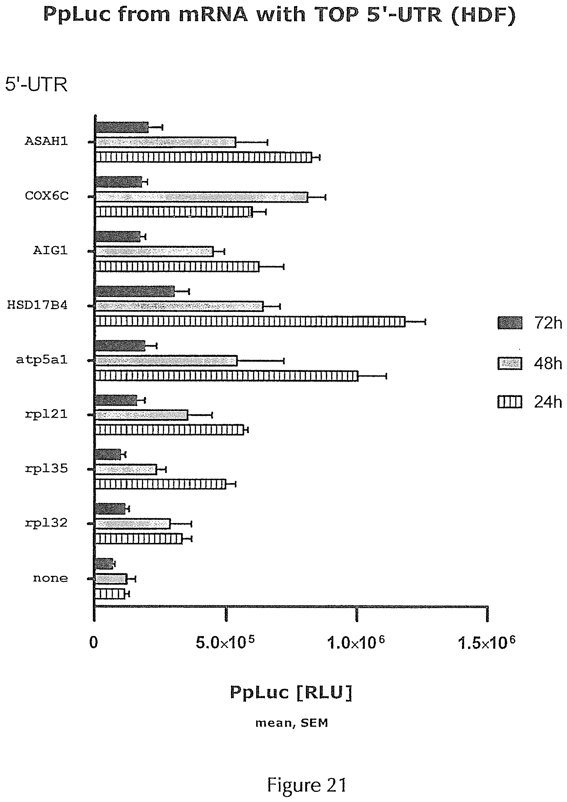

Figure 21 is a graphical representation of the effect of the 5'UTR element derived from the TOP genes RPL32, RPL35, RPL21, ATP5A1, HSD17B4, AIG1, COX6C and ASAH1 on luciferase expression from mRNA. The mRNAs were transfected into human dermal fibroblasts (HDF) by lipofection. Luciferase levels were measured at 24, 48, and 72 hours after transfection. The 5'UTR elements strongly increase luciferase levels compared to mRNA lacking a 5'UTR element. Data are graphed as mean RLU ± SEM (relative light units ± standard error) for triplicate transfections. RLU are summarized in Example 5.3.

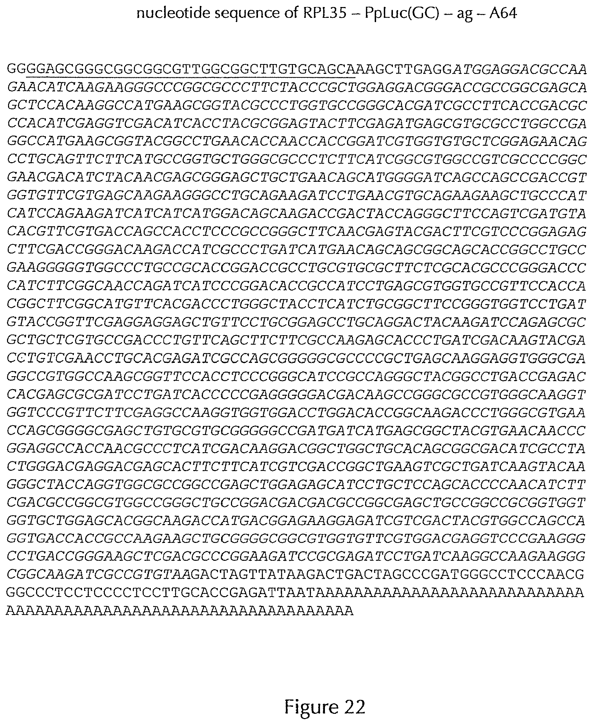

Figure 22 shows the nucleotide sequence of rpl35 - PpLuc(GC) - ag - A64. The 5'UTR of human ribosomal protein Large 35 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 22 corresponds to SEQ ID No. 1466.

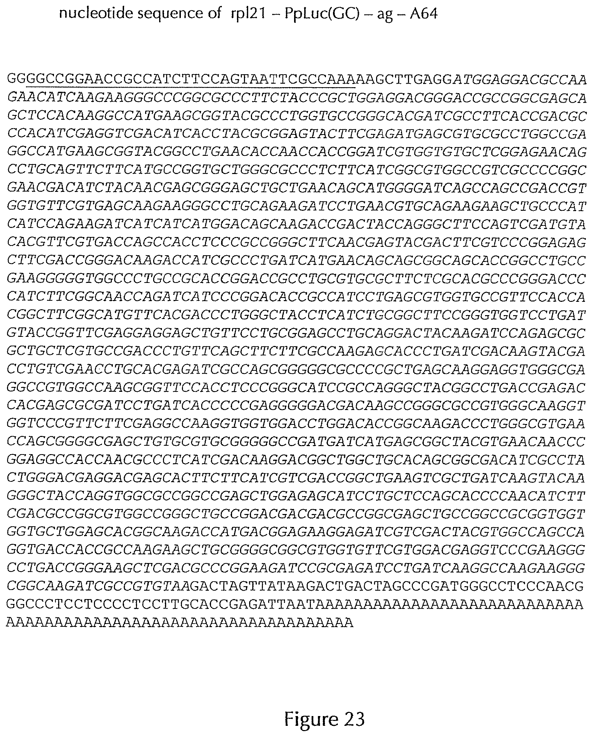

Figure 23 shows the nucleotide sequence of rpl21 - PpLuc(GC) - ag - A64. The 5'UTR of human ribosomal protein Large 21 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 23 corresponds to SEQ ID No. 1467.

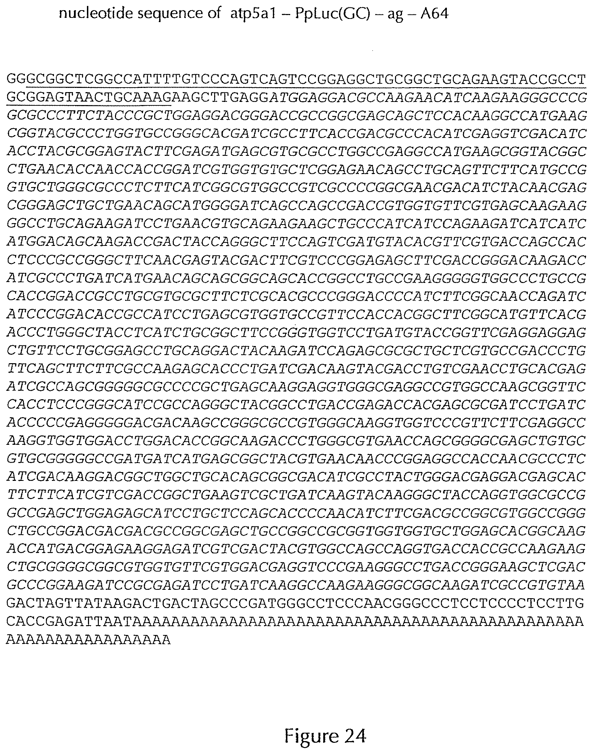

Figure 24 shows the nucleotide sequence of atp5a1 - PpLuc(GC) - ag - A64. The 5'UTR of human ATP synthase, H+ transporting, mitochondrial F1 complex, alpha subunit 1 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 24 corresponds to SEQ ID No. 1468.

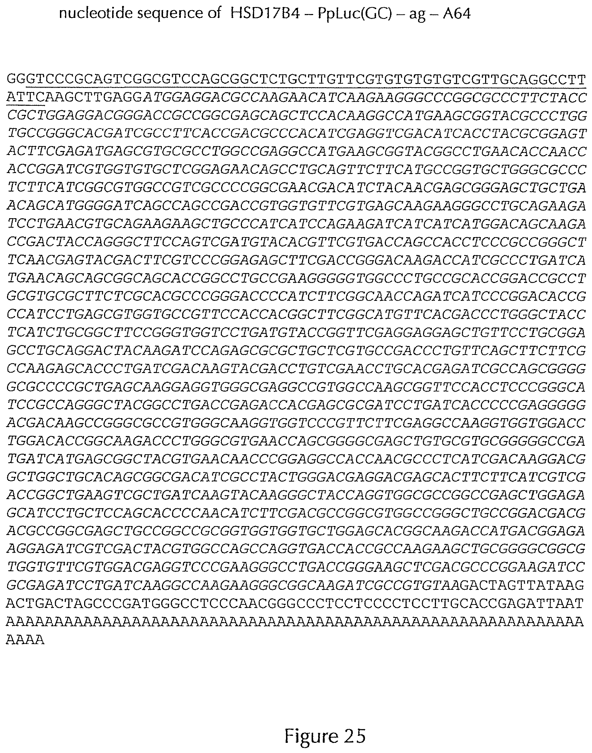

Figure 25 shows the nucleotide sequence of HSD17B4 - PpLuc(GC) - ag - A64. The 5'UTR of human hydroxysteroid (17-beta) dehydrogenase 4 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 25 corresponds to SEQ ID No. 1469.

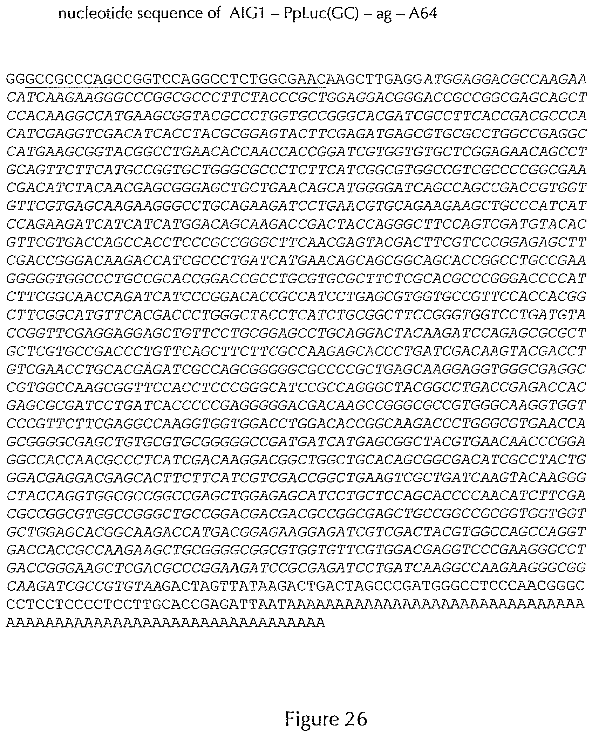

Figure 26 shows the nucleotide sequence of AIG1 - PpLuc(GC) - ag - A64. The 5'UTR of human androgen-induced 1 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 26 corresponds to SEQ ID No. 1470.

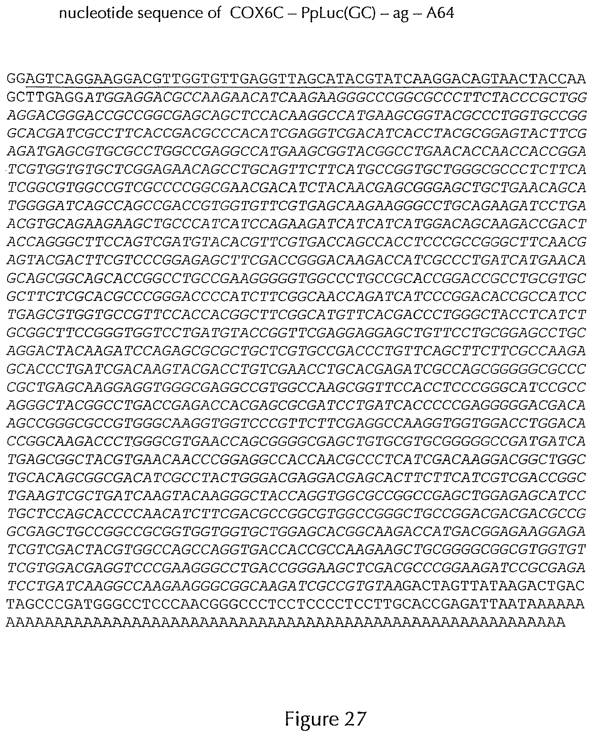

Figure 27 shows the nucleotide sequence of COX6C - PpLuc(GC) - ag - A64. The 5'UTR of human cytochrome c oxidase subunit VIc lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 27 corresponds to SEQ ID No. 1471.

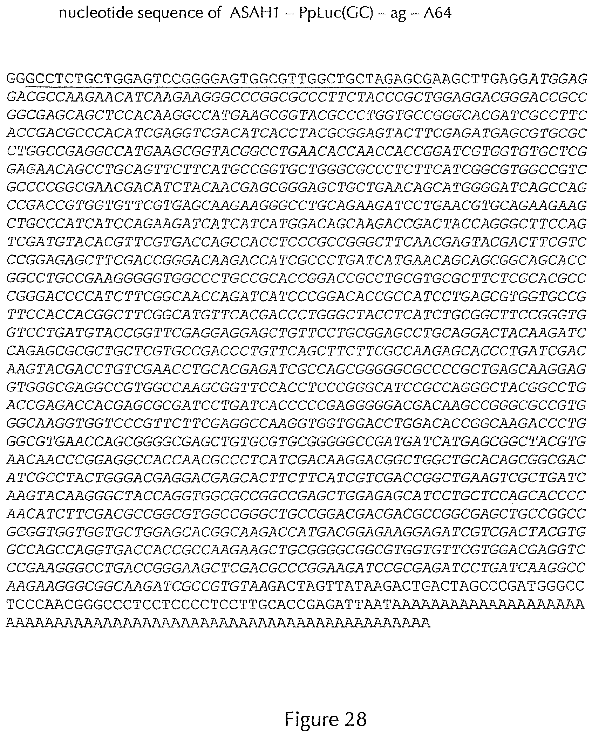

Figure 28 shows the nucleotide sequence of ASAH1 - PpLuc(GC) - ag - A64. The 5'UTR of human N-acylsphingosine amidohydrolase (acid ceramidase) 1 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 28 corresponds to SEQ ID No. 1472.

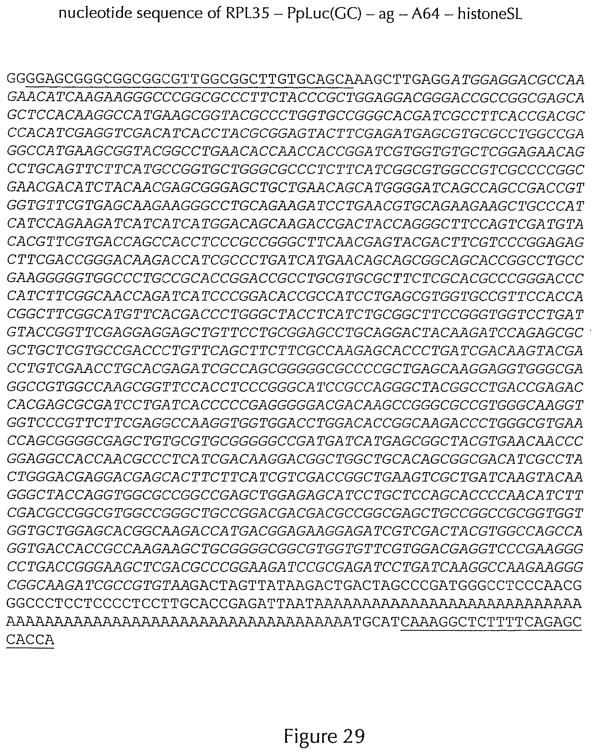

Figure 29 shows the nucleotide sequence of rpl35 - PpLuc(GC) - ag - A64 - histoneSL. The 5'UTR of human ribosomal protein Large 35 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 29 corresponds to SEQ ID No. 1473.

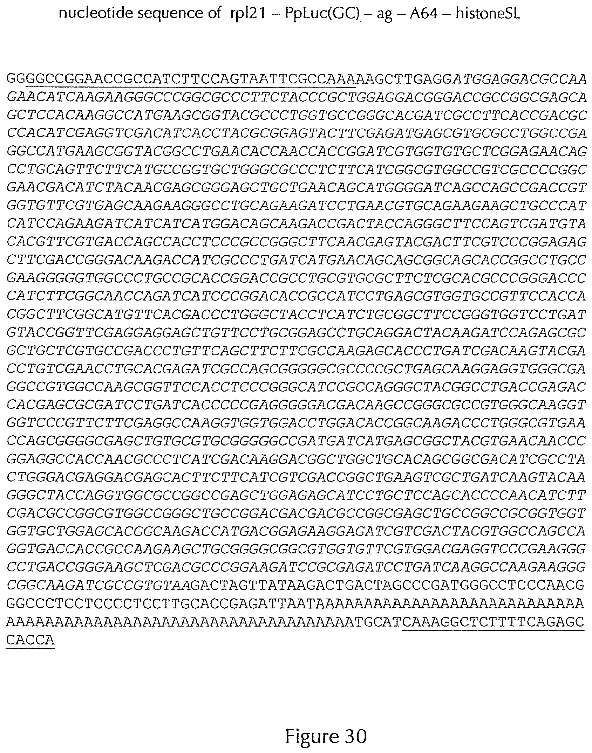

Figure 30 shows the nucleotide sequence of rpl21 - PpLuc(GC) - ag - A64 - histoneSL. The 5'UTR of human ribosomal protein Large 21 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 30 corresponds to SEQ ID No. 1474.

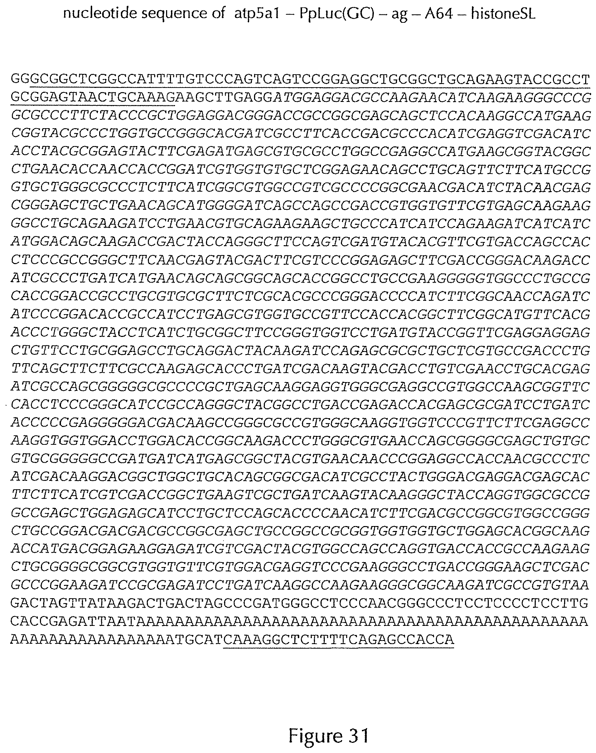

Figure 31 shows the nucleotide sequence of atp5a1 - PpLuc(GC) - ag - A64 - histoneSL. The 5'UTR of human ATP synthase, H+ transporting, mitochondrial F1 complex, alpha subunit 1 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 31 corresponds to SEQ ID No. 1475.

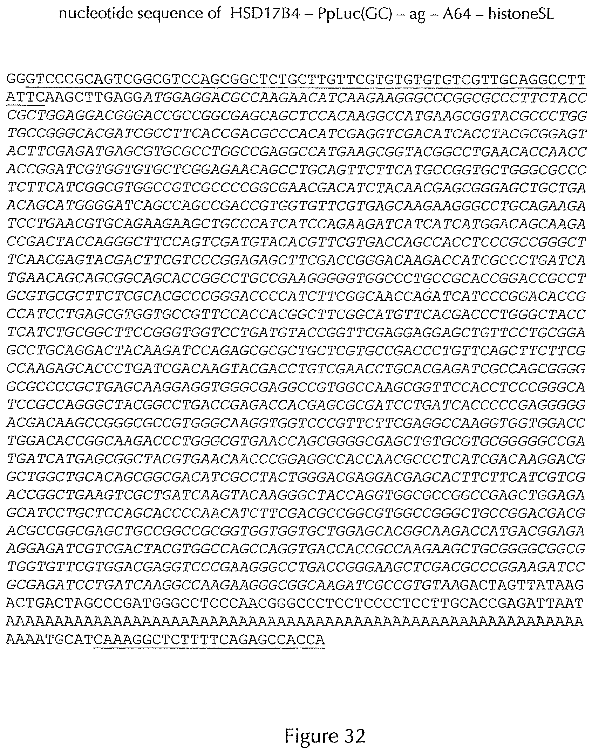

Figure 32 shows the nucleotide sequence of HSD17B4 - PpLuc(GC) - ag - A64 - histoneSL. The 5'UTR of human hydroxysteroid (17-beta) dehydrogenase 4 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 32 corresponds to SEQ ID No. 1476.

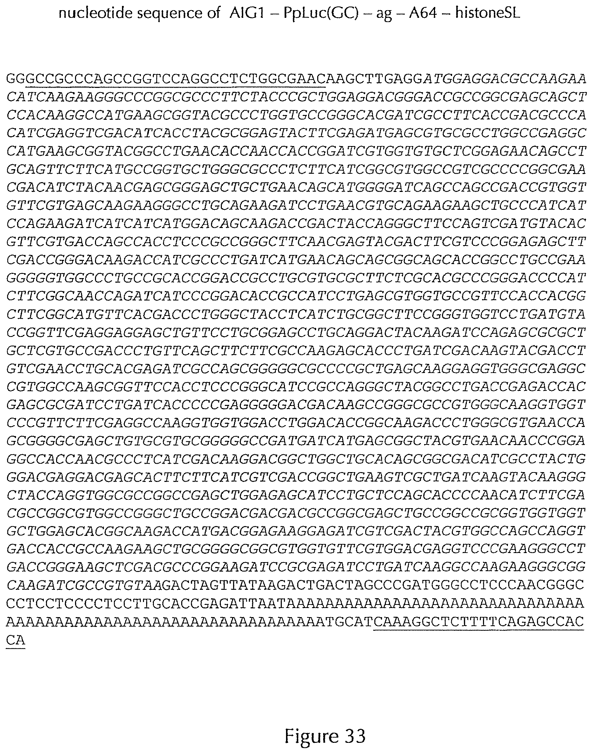

Figure 33 shows the nucleotide sequence of AIG1 - PpLuc(GC) - ag - A64 - histoneSL. The 5'UTR of human androgen-induced 1 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 33 corresponds to SEQ ID No. 1477.

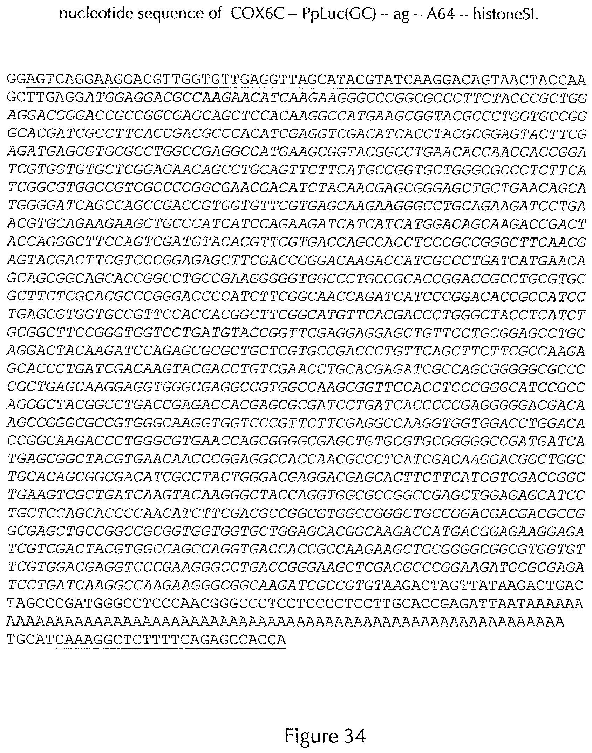

Figure 34 shows the nucleotide sequence of COX6C - PpLuc(GC) - ag - A64 - histoneSL. The 5'UTR of human cytochrome c oxidase subunit Vlc lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 34 corresponds to SEQ ID No. 1478.

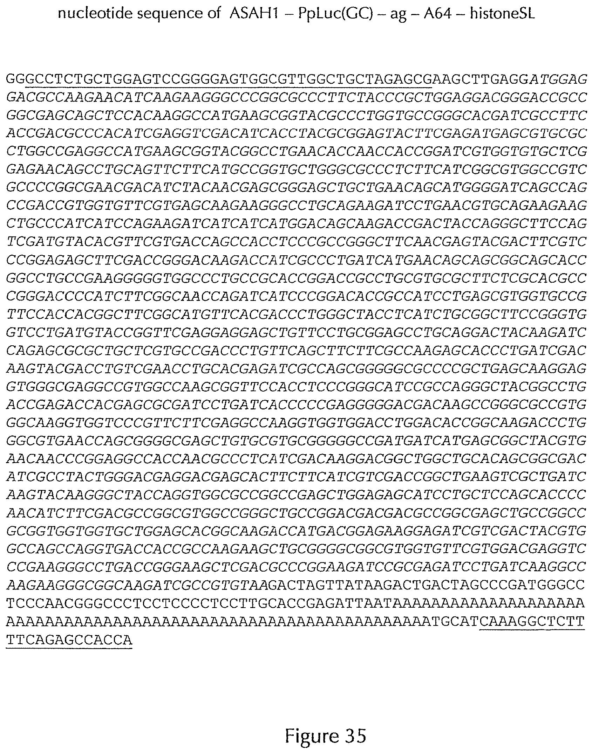

Figure 35 shows the nucleotide sequence of ASAH 1 - PpLuc(GC) - ag - A64 - histoneSL. The 5'UTR of human N-acylsphingosine amidohydrolase (acid ceramidase) 1 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 35 corresponds to SEQ ID No. 1479.

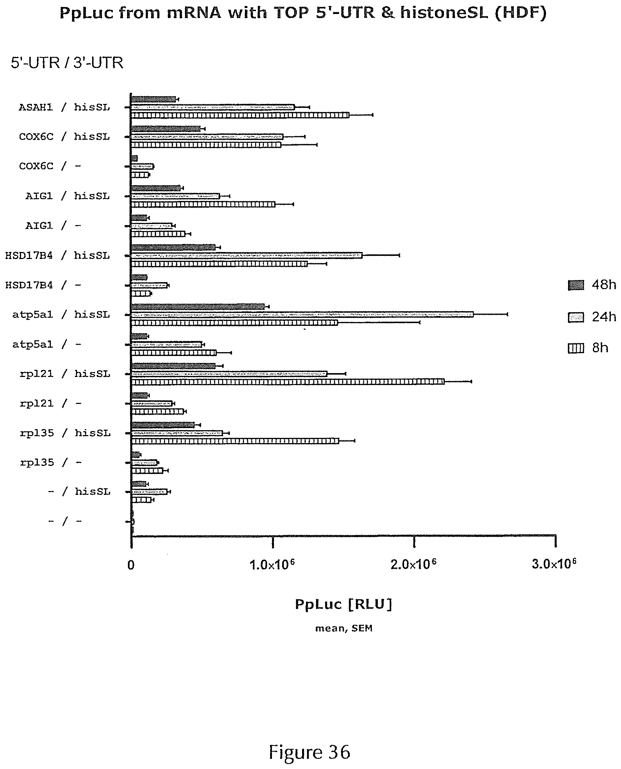

Figure 36 is a graphical representation of the effect of 5'UTR elements derived from 5'UTRs of the TOP genes RPL35, RPL21, ATP5A1, HSD17B4, AIG1, COX6C and ASAH1, the histone stem-loop, and the combination of 5'UTR elements and histone stem-loop on luciferase expression from mRNA. The different mRNAs were transfected into human dermal fibroblasts (HDF) by lipofection. Luciferase levels were measured at 8, 24, and 48 hours after transfection. Both, either the histone stem-loop or the 5'UTR elements increase luciferase levels compared to mRNA lacking both these elements. Strikingly, the combination of 5'UTR elements and histone stem-loop further strongly increases the luciferase level, much above the level observed with either of the individual elements, thus acting synergistically. Data are graphed as mean RLU $\pm$ SEM (relative light units $\pm$ standard error) for triplicate transfections. The synergy between 5'UTR elements and histone stem-loop is summarized in example 5.4.

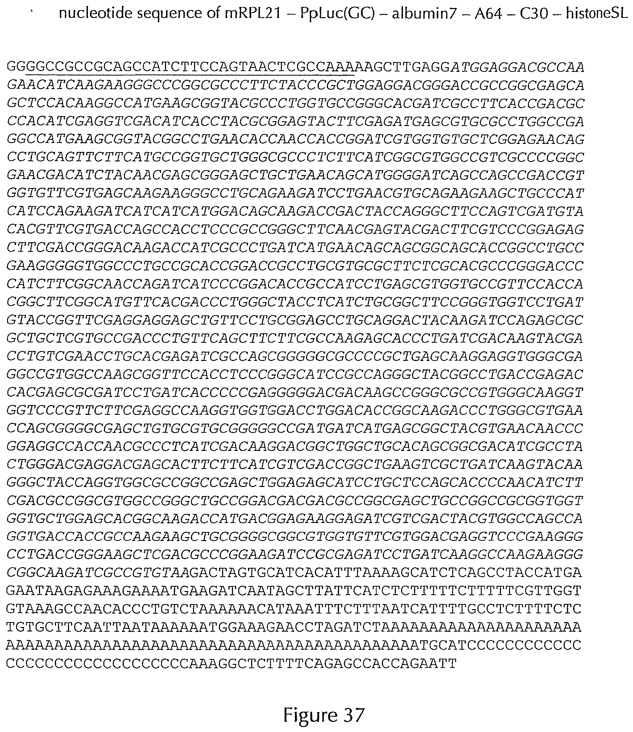

Figure 37 shows the nucleotide sequence of mrpl21 - PpLuc(GC) - albumin7 - A64 - C30 - histoneSL. The 5'UTR of murine ribosomal protein Large 21 lacking the 5' terminal oligopyrimidine tract replaced the rpl32 5'UTR element in the construct shown in Figure 13. The 5'UTR element sequence is underlined. The sequence depicted in Figure 36 corresponds to SEQ ID No. 1443.

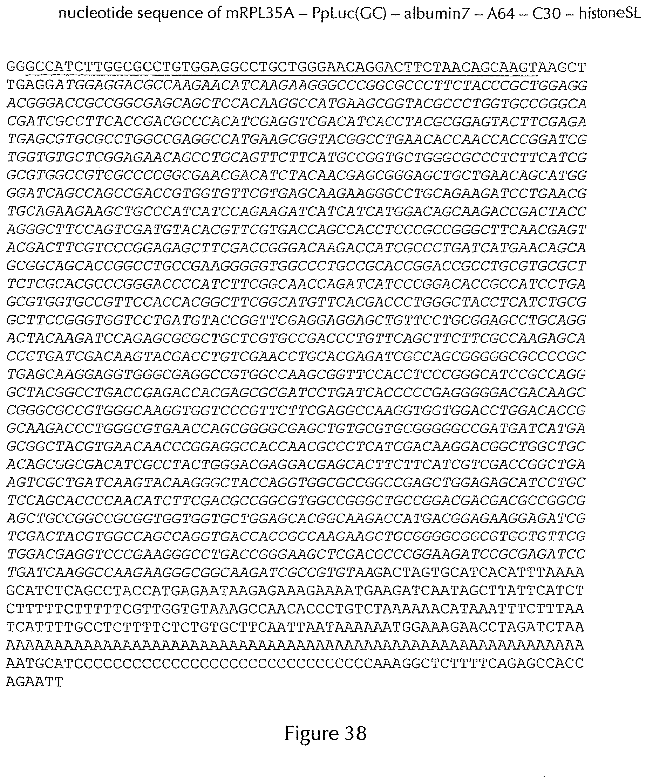

Figure 38 shows the nucleotide sequence of mrpl35A - PpLuc(GC) - albumin7 - A64 - C30 - histoneSL. The 5'UTR of murine ribosomal protein Large 35A lacking the 5' terminal oligopyrimidine tract replaced the rpl32 5'UTR element in the construct shown in Figure 13. The 5'UTR element sequence is underlined. The sequence depicted in Figure 37 corresponds to SEQ ID No. 1444.

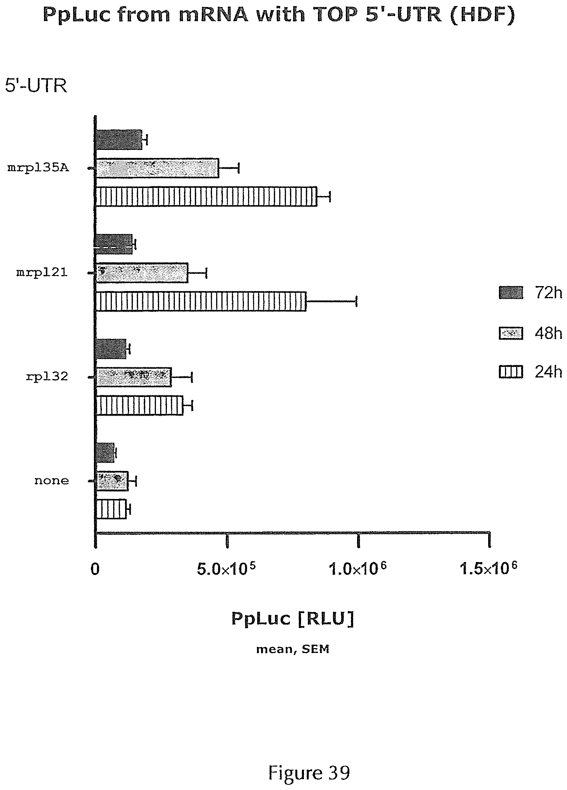

Figure 39 is a graphical representation of the effect of the 5'UTR elements derived from 5'UTRs of mouse TOP genes on luciferase expression from mRNA. mRNAs containing either a mouse or a human 5'UTR element were transfected into human dermal fibroblasts (HDF) by lipofection. Luciferase levels were measured at 24, 48, and 72 hours after transfection. Mouse 5'UTR elements strongly increase luciferase levels compared to mRNA lacking a 5'UTR element, similarly as the human 5'UTR element. Data are graphed as mean RLU $\pm$ SEM (relative light units $\pm$ standard error) for triplicate transfections. RLU are summarized in Example 5.5.

| | |
|---|---|
| SEQ ID No. 1 -1363. 1435, and 1461-1462 | sequences comprising 5'UTRs of TOP genes |
| SEQ ID No. 1364 | PpLuc(GC) - ag - A64 (Fig. 6) |
| SEQ ID No. 1365 | RPL32 - PpLuc(GC) - ag - A64 - C30 - histoneSL (Fig. 7) |
| SEQ ID No. 1366 | fragment of the 5'UTR of human ribosomal protein Large 32 |
| SEQ ID No. 1367 | fragment of the 5'UTR of human ribosomal protein Large 32 |
| SEQ ID No. 1368 | 5'UTR of human ribosomal protein Large 32 lacking the 5' terminal oligopyrimidine tract |
| SEQ ID No. 1369 | Human albumin 3'UTR |
| SEQ ID No. 1370 | 3'UTR of Homo sapiens hemoglobin, alpha 1 (HBA1) |
| SEQ ID No. 1371 | 3'UTR of Homo sapiens hemoglobin, alpha 2 (HBA2) |
| SEQ ID No. 1372 | 3'UTR of Homo sapiens hemoglobin, beta (HBB) |
| SEQ ID No. 1373 | 3'UTR of Homo sapiens tyrosine hydroxylase (TH) |
| SEQ ID No. 1374 | 3'UTR of Homo sapiens arachidonate 15-lipoxygenase (ALOX15) |
| SEQ ID No. 1375 | 3'UTR of Homo sapiens collagen, type 1, alpha 1 (COL1A1) |
| SEQ ID No. 1376 | albumin7 3'UTR |
| SEQ ID No. 1377 | Human albumin 3'UTR + poly(A) sequence |
| SEQ ID No. 1378 | Human albumin 3'UTR fragment 1 |
| SEQ ID No. 1379 | Human albumin 3'UTR fragment 2 |
| SEQ ID No. 1380 | Human albumin 3'UTR fragment 3 |
| SEQ ID No. 1381 | Human albumin 3'UTR fragment 4 |
| SEQ ID No. 1382 | Human albumin 3'UTR fragment 5 |
| SEQ ID No. 1383 | Human albumin 3'UTR fragment 6 |
| SEQ ID No. 1384 | Human albumin 3'UTR fragment 7 |
| SEQ ID No. 1385 | Human albumin 3'UTR fragment 8 |
| SEQ ID No. 1386 | Human albumin 3'UTR fragment 9 |
| SEQ ID No. 1387 | Human albumin 3'UTR fragment 10 |
| SEQ ID No. 1388 | Human albumin 3'UTR fragment 11 |
| SEQ ID No. 1389 | Human albumin 3'UTR fragment 12 |
| SEQ ID No. 1390 | Human albumin 3'UTR fragment 13 |
| SEQ ID NO. 1391 | Sequence according to formula (Ic) |
| SEQ ID NO. 1392 | Sequence according to formula (IIc): |
| SEQ ID NO. 1393 | Sequence according to formula (Id): |
| SEQ ID NO. 1394 | Sequence according to formula (IId) |
| SEQ ID NO. 1395 | Sequence according to formula (Ie) |
| SEQ ID NO. 1396 | Sequence according to formula (IIe) |
| SEQ ID NO. 1397 | Sequence according to formula (If) |
| SEQ ID NO. 1398 | Sequence according to formula (IIf) |
| SEQ ID NO. 1399 | Sequence according to formula (Ig) |
| SEQ ID NO. 1400 | Sequence according to formula (IIg) |
| SEQ ID NO. 1401 | Sequence according to formula (Ih) |

(continued)

| | |
|---|---|
| SEQ ID NO. 1402 | Sequence according to formula (IIh) |
| SEQ ID NO. 1403 | Sequence according to formula (Ic) |
| SEQ ID NO. 1404 | Sequence according to formula (Ic) |
| SEQ ID NO. 1405 | Sequence according to formula (Ic) |
| SEQ ID NO. 1406 | Sequence according to formula (le) |
| SEQ ID NO. 1407 | Sequence according to formula (le) |
| SEQ ID NO. 1408 | Sequence according to formula (le) |
| SEQ ID NO. 1409 | Sequence according to formula (If) |
| SEQ ID NO. 1410 | Sequence according to formula (If) |
| SEQ ID NO. 1411 | Sequence according to formula (if) |
| SEQ ID NO. 1412 | Sequence according to formula (Ig) |
| SEQ ID NO. 1413 | Sequence according to formula (Ig) |
| SEQ ID NO. 1414 | Sequence according to formula (Ig) |
| SEQ ID NO. 1415 | Sequence according to formula (Ih) |
| SEQ ID NO. 1416 | Sequence according to formula (Ih) |
| SEQ ID NO. 1417 | Sequence according to formula (Ih) |
| SEQ ID NO. 1418 | Sequence according to formula (IIc) |
| SEQ ID NO. 1419 | Sequence according to formula (IIc) |
| SEQ ID NO. 1420 | Sequence according to formula (IIc) |
| SEQ ID NO. 1421 | Sequence according to formula (IIe) |
| SEQ ID NO. 1422 | Sequence according to formula (IIe) |
| SEQ ID NO. 1423 | Sequence according to formula (IIe) |
| SEQ ID NO. 1424 | Sequence according to formula (IIf) |
| SEQ ID NO. 1425 | Sequence according to formula (IIf) |
| SEQ ID NO. 1426 | Sequence according to formula (IIf) |
| SEQ ID NO. 1427 | Sequence according to formula (IIg) |
| SEQ ID NO. 1428 | Sequence according to formula (IIg) |
| SEQ ID NO. 1429 | Sequence according to formula (IIg) |
| SEQ ID NO. 1430 | Sequence according to formula (IIh) |
| SEQ ID NO. 1431 | Sequence according to formula (IIh) |
| SEQ ID NO. 1432 | Sequence according to formula (IIh) |
| SEQ ID NO. 1433 | Example histone stem-loop sequence |
| SEQ ID NO. 1434 | Center, $\alpha$-complex-binding portion of the 3'UTR of an $\alpha$-globin gene |
| SEQ ID NO. 1435 | ATP synthase lipid-binding protein, mitochondrial (atp5g2) |
| SEQ ID NO. 1436 | RPL35 - PpLuc(GC) - albumin7 - A64 - C30 - histoneSL (Fig. 14) |
| SEQ ID NO. 1437 | RPL21 - PpLuc(GC) - albumin7 - A64 - C30 - histoneSL (Fig. 15) |
| SEQ ID NO. 1438 | ATP5A1 - PpLuc(GC) - albumin7 - A64 - C30 - histoneSL (Fig. 16) |
| SEQ ID NO. 1439 | HSD1 7B4 - PpLuc(GC) - albumin7 - A64 - C30 - histoneSL (Fig. 17) |
| SEQ ID NO. 1440 | AIG1 - PpLuc(GC) - albumin7 - A64 - C30 - histoneSL (Fig. 18) |
| SEQ ID NO. 1441 | COX6C - PpLuc(GC) - albumin7 - A64 - C30 - histoneSL (Fig. 19) |
| SEQ ID NO. 1442 | ASAH1 - PpLuc(GC) - albumin7 - A64 -- C30 - histoneSL (Fig. 20) |
| SEQ ID NO. 1443 | mRPL21 - PpLuc(GC) - albumin7 - A64 - C30 - histoneSL (Fig. 37) |
| SEQ ID NO. 1444 | mRPL35A - PpLuc(GC) - albumin7 - A64 - C30 - histoneSL (Fig. 38) |
| SEQ ID NO. 1445 | RPL35 - PpLuc(GC) - A64 - C30 - histoneSL |
| SEQ ID NO. 1446 | RPL21 - PpLuc(GC) - A64 - C30 - histoneSL |
| SEQ ID NO. 1447 | ATP5A1 - PpLuc(GC) - A64 - C30 - histoneSL |
| SEQ ID NO. 1448 | HSD1 7B4 - PpLuc(GC) - A64 - C30 - histoneSL |
| SEQ ID NO. 1449 | AIG1 - PpLuc(GC) - A64 - C30 - histoneSL |
| SEQ ID NO. 1450 | COX6C - PpLuc(GC) - A64 - C30 - histoneSL |
| SEQ ID NO. 1451 | ASAH1 - PpLuc(GC) - A64 - C30 - histoneSL |

(continued)

| | |
|---|---|
| SEQ ID NO. 1452 | 5'UTR of human ribosomal protein Large 35 (RPL35) lacking the 5' terminal oligopyrimidine tract |
| SEQ ID NO. 1453 | 5'UTR of human ribosomal protein Large 21 (RPL21) lacking the 5' terminal oligopyrimidine tract |
| SEQ ID NO. 1454 | 5'UTR of human ATP synthase, H+ transporting, mitochondrial F1 complex, alpha subunit 1, cardiac muscle (ATP5A1) lacking the 5' terminal oligopyrimidine tract |
| SEQ ID NO. 1455 | 5'UTR of human hydroxysteroid (17-beta) dehydrogenase 4 (HSD17B4) lacking the 5' terminal oligopyrimidine tract |
| SEQ ID NO. 1456 | 5'UTR of human androgen-induced 1 (AIG1) lacking the 5' terminal oligopyrimidine tract |
| SEQ ID NO. 1457 | 5'UTR of human cytochrome c oxidase subunit Vic (COX6C) lacking the 5' terminal oligopyrimidine tract |
| SEQ ID NO. 1458 | 5'UTR of human N-acylsphingosine amidohydrolase (acid ceramidase) 1 (ASAH1) lacking the 5' terminal oligopyrimidine tract |
| SEQ ID NO. 1459 | 5'UTR of mouse ribosomal protein Large 21 (mRPL21) lacking the 5' terminal oligopyrimidine tract |
| SEQ ID NO. 1460 | 5'UTR of mouse ribosomal protein large 35A (mRPL35A) lacking the 5' terminal oligopyrimidine tract |
| SEQ ID NO. 1461 | Mouse ribosomal protein Large 21 (mRPL21) |
| SEQ ID NO. 1462 | Mouse ribosomal protein large 35A (mRPL35A) |
| SEQ ID NO. 1463 | RPL32 - PpLuc(GC) - ag - A64 (Fig. 10) |
| SEQ ID NO. 1464 | PpLuc(GC) - ag - A64 - histoneSL (Fig. 9) |
| SEQ ID NO. 1465 | PpLuc(GC) - albumin7 - A64 - C30 - histoneSL |
| SEQ ID NO. 1466 | RPL35 - PpLuc(GC) - ag - A64 (Fig. 22) |
| SEQ ID NO. 1467 | RPL21 - PpLuc(GC) - ag - A64 (Fig. 23) |
| SEQ ID NO. 1468 | atp5a1 - PpLuc(GC) - ag - A64 (Fig. 24) |
| SEQ ID NO. 1469 | HSD1 7B4 - PpLuc(GC) - ag - A64 (Fig. 25) |
| SEQ ID NO. 1470 | AIG1 - PpLuc(GC) - ag - A64 (Fig. 26) |
| SEQ ID NO. 1471 | COX6C - PpLuc(GC) - ag - A64 (Fig. 27) |
| SEQ ID NO. 1472 | ASAH1 - PpLuc(GC) - ag - A64 (Fig. 28) |
| SEQ ID NO. 1473 | RPL35 - PpLuc(GC) - ag - A64 - histoneSL (Fig. 29) |
| SEQ ID NO. 1474 | RPL21 - PpLuc(GC) - ag - A64 - histoneSL (Fig. 30) |
| SEQ ID NO. 1475 | atp5a1 - PpLuc(GC) - ag - A64 - histoneSL (Fig. 31) |
| SEQ ID NO. 1476 | HSD1 7B4 - PpLuc(GC) - ag - A64 - histoneSL (Fig. 32) |
| SEQ ID NO. 1477 | AIG1 - PpLuc(GC) - ag - A64 - histoneSL (Fig. 33) |
| SEQ ID NO. 1478 | COX6C - PpLuc(GC) - ag - A64 - histoneSL (Fig. 34) |
| SEQ ID NO. 1479 | ASAH1 - PpLuc(GC) - ag - A64 - histoneSL (Fig. 35) |
| SEQ ID NO. 1480 | RPL32 - PpLuc(GC) - ag - A64 - histoneSL (Fig. 11) |
| SEQ ID NO. 1481 | RPL32 - PpLuc(GC) - albumin7 - A64 - C30 - histoneSL (Fig. 13) |

Examples

**1. Preparation of DNA-templates**

A vector for *in vitro* transcription was constructed containing a T7 promoter followed by a GC-enriched sequence coding for *Photinus pyralis* luciferase (PpLuc(GC)) and an A64 poly(A) sequence. The poly(A) sequence was followed by a restriction site used for linearization of the vector before *in vitro* transcription. mRNA obtained from this vector accordingly by *in vitro* transcription is designated as "PpLuc(GC) - A64".

This vector was modified to include untranslated sequences 5' or 3' of the open reading frame. In summary, vectors comprising the following mRNA encoding sequences have been generated:

SEQ ID No. 1364 PpLuc(GC) - ag - A64 (Fig. 6)

(continued)

| | |
|---|---|
| SEQ ID No. 1365 | RPL32 - PpLuc(GC) - ag - A64 - C30 - histoneSL (Fig. 7): |
| SEQ ID NO. 1436 | RPL35 - PpLuc(GC) - albumin7 - A64 - C30 - histoneSL (Fig. 14) |
| SEQ ID NO. 1437 | RPL21 - PpLuc(GC) - albumin7 - A64 - C30 - histoneSL (Fig. 15) |
| SEQ ID NO. 1438 | ATP5A1 - PpLuc(GC) - albumin7 - A64 - C30 - histoneSL (Fig. 16) |
| SEQ ID NO. 1439 | HSD1 7B4 - PpLuc(GC) - albumin7 - A64 - C30 - histoneSL (Fig. 17) |
| SEQ ID NO. 1440 | AIG1 - PpLuc(GC) - albumin7 - A64 - C30 - histoneSL (Fig. 18) |
| SEQ ID NO. 1441 | COX6C - PpLuc(GC) - albumin7 - A64 - C30 - histoneSL (Fig. 19) |
| SEQ ID NO. 1442 | ASAH1 - PpLuc(GC) - albumin7 - A64 - C30 - histoneSL (Fig. 20) |
| SEQ ID NO. 1443 | mRPL21 - PpLuc(GC) - albumin7 - A64 - C30 - histoneSL (Fig. 37) |
| SEQ ID NO. 1444 | mRPL35A - PpLuc(GC) - albumin7 - A64 - C30 - histoneSL (Fig. 38) |
| SEQ ID NO. 1445 | RPL35 - PpLuc(GC) - A64 - C30 - histoneSL |
| SEQ ID NO. 1446 | RPL21 - PpLuc(GC) - A64 - C30 - histoneSL |
| SEQ ID NO. 1447 | ATP5A1 - PpLuc(GC) - A64 - C30 - histoneSL |
| SEQ ID NO. 1448 | HSD1 7B4 - PpLuc(GC) - A64 - C30 - histoneSL |
| SEQ ID NO. 1449 | AIG1 - PpLuc(GC) - A64 - C30 - histoneSL |
| SEQ ID NO. 1450 | COX6C - PpLuc(GC) - A64 - C30 - histoneSL |
| SEQ ID NO. 1451 | ASAH1 - PpLuc(GC) - A64 - C30 - histoneSL |
| SEQ ID NO. 1463 | RPL32 - PpLuc(GC) - ag - A64 (Fig. 10) |
| SEQ ID NO. 1464 | PpLuc(GC) - ag - A64 - histoneSL (Fig. 9) |
| SEQ ID NO. 1465 | PpLuc(GC) - albumin7 - A64 - C30 - histoneSL |
| SEQ ID NO. 1466 | RPL35 - PpLuc(GC) - ag - A64 (Fig. 22) |
| SEQ ID NO. 1467 | RPL21 - PpLuc(GC) - ag - A64 (Fig. 23) |
| SEQ ID NO. 1468 | atp5a1 - PpLuc(GC) - ag - A64 (Fig. 24) |
| SEQ ID NO. 1469 | HSD17B4 - PpLuc(GC) - ag - A64 (Fig. 25) |
| SEQ ID NO. 1470 | AIG1 - PpLuc(GC) - ag - A64 (Fig. 26) |
| SEQ ID NO. 1471 | COX6C - PpLuc(GC) - ag - A64 (Fig. 27) |
| SEQ ID NO. 1472 | ASAH1 - PpLuc(GC) - ag - A64 (Fig. 28) |
| SEQ ID NO. 1473 | RPL35 - PpLuc(GC) - ag - A64 - histoneSL (Fig. 29) |
| SEQ ID NO. 1474 | RPL21 - PpLuc(GC) - ag - A64 - histoneSL (Fig. 30) |
| SEQ ID NO. 1475 | atp5a1 - PpLuc(GC) - ag - A64 - histoneSL (Fig. 31) |
| SEQ ID NO. 1476 | HSD17B4 - PpLuc(GC) - ag - A64 - histoneSL (Fig. 32) |
| SEQ ID NO. 1477 | AIG1 - PpLuc(GC) - ag - A64 - histoneSL (Fig. 33) |
| SEQ ID NO. 1478 | COX6C - PpLuc(GC) - ag - A64 - histoneSL (Fig. 34) |
| SEQ ID NO. 1479 | ASAH 1 - PpLuc(GC) - ag - A64 - histoneSL (Fig. 35) |
| SEQ ID NO. 1480 | RPL32 - PpLuc(GC) - ag - A64 - histoneSL (Fig. 11) |
| SEQ ID NO. 1481 | RPL32 - PpLuc(GC) - albumin7 - A64 - C30 - histoneSL (Fig. 13) |

2. *In vitro* transcription

The DNA-template according to Example 1 was linearized and transcribed *in vitro* using T7-Polymerase. The DNA-template was then digested by DNase-treatment. mRNA transcripts contained a 5'-CAP structure obtained by adding an excess of N7-Methyl-Guanosine-5'-Triphosphate-5'-Guanosine to the transcription reaction. mRNA thus obtained was purified and resuspended in water.

3. Luciferase expression by mRNA lipofection

Human dermal fibroblasts (HDF) were seeded in 24 well plates at a density of $5\times10^4$ cells per well. The following day, cells were washed in opti-MEM and then transfected with 50 ng per well of Lipofectamine2000-complexed PpLuc-encoding mRNA in opti-MEM. As a control, mRNA not coding for PpLuc was lipofected separately. mRNA coding for *Renilla reniformis* luciferase (RrLuc) was transfected together with PpLuc mRNA to control for transfection efficiency (20 ng of RrLuc mRNA per well). 90 minutes after start of transfection, opti-MEM was exchanged for medium. 24, 48, 72 hours after transfection, medium was aspirated and cells were lysed in 200 µl of lysis buffer (25 mM Tris, pH 7.5

(HCl), 2 mM EDTA, 10% glycerol, 1% Triton X-100, 2 mM DTT, 1 mM PMSF). Lysates were stored at -20°C until luciferase activity was measured. Alternatively, HDF were seeded in 96 well plates one to three days before transfection at a density of 104 cells per well. Immediately before lipofection, cells were washed in opti-MEM. Cells were lipofected with 25 ng of PpLuc-encoding mRNA per well complexed with Lipofectamine2000. In some experiments, mRNA coding for Renilla reniformis luciferase (RrLuc) was transfected together with PpLuc mRNA to control for transfection efficiency (2.5 ng of RrLuc mRNA per well). 90 minutes after start of transfection, opti-MEM was exchanged for medium. At various time points post transfection, medium was aspirated and cells were lysed in 100 μl of lysis buffer (Passive Lysis Buffer, Promega). Lysates were stored at -80°C until luciferase activity was measured.

**4. Luciferase measurement**

Luciferase activity was measured as relative light units (RLU) in a BioTek SynergyHT plate reader. PpLuc activity was measured at 15 seconds measuring time using 50 μl of lysate and 200 μl of luciferin buffer (75 μM luciferin, 25 mM Glycylglycin, pH 7.8 (NaOH), 15 mM $MgSO_4$, 2 mM ATP). RrLuc activity was measured at 15 seconds measuring time using 50 μl of lysate and 200 μl of coelenterazin buffer (40 μM coelenterazin in phosphate buffered saline adjusted to 500 mM NaCl).

Alternatively, luciferase activity was measured as relative light units (RLU) in a Hidex Chameleon plate reader. PpLuc activity was measured at 2 seconds measuring time using 20 μl of lysate and 50 μl of luciferin buffer (Beetle-Juice, PJK GmbH). RrLuc activity was measured at 2 seconds measuring time using 20 μl of lysate and 50 μl of coelenterazin buffer (Renilla-Juice, PJK GmbH).

**Results**

5.1 The combination of 5'UTR elements derived from 5'UTRs of TOP genes and histone stem-loop increases protein expression strongly.

To investigate the effect of the combination of a 5'UTR element derived from a 5'UTR of a TOP gene and a histone stem-loop (histoneSL) on protein expression from mRNA, mRNAs with different UTRs were synthesized: mRNAs either lacked both 5'UTR element and histoneSL, or contained both 5'UTR element and histoneSL. Luciferase-encoding mRNAs or control mRNA were transfected into human dermal fibroblasts (HDF). Luciferase levels were measured at 24 hours after transfection (see following Table 1 and Figure 8).

Table 1:

| mRNA | RLU at 24 hours |
|---|---|
| control RNA | 588 |
| PpLuc(GC) - ag - A64 | 12246 |
| RPL32 - PpLuc(GC) - ag - A64 - C30 - histoneSL | 319840 |

Luciferase was clearly expressed from mRNA having neither 5'UTR element nor histoneSL. Strikingly however, the combination of 5'UTR element and histoneSL strongly increased the luciferase level. The magnitude of the rise in luciferase level due to combining 5'UTR element and histoneSL in the same mRNA indicates that they are acting synergistically.

5.2 The combination of 5'UTR elements derived from 5'UTRs of TOP genes and histone stem-loop increases protein expression from mRNA in a synergistic manner.

To investigate the effect of the combination of a 5'UTR element derived from a 5'UTR of a TOP gene and histone stem-loop on protein expression from mRNA, mRNAs with different UTRs were synthesized: mRNAs either lacked both 5'UTR element and histone stem-loop, or contained either a 5'UTR element or a histone stem-loop, or both 5'UTR element and histone stem-loop. Luciferase-encoding mRNAs were transfected into human dermal fibroblasts (HDF). Luciferase levels were measured at 8, 24, and 48 hours after transfection (see following Table 2 and Figure 12).

Table 2:

| mRNA | RLU at 8 hours | RLU at 24 hours | RLU at 48 hours |
|---|---|---|---|
| PpLuc(GC)-ag-A64 | 13110 | 25861 | 14362 |
| PpLuc(GC)-ag-A64-histoneSL | 88640 | 97013 | 57026 |
| rpl32-PpLuc(GC)-ag-A64 | 155654 | 212245 | 102528 |
| rpl32-PpLuc(GC)-ag-A64-histoneSL | 301384 | 425825 | 161974 |

Luciferase was clearly expressed from mRNA having neither 5'UTR element nor histone stem-loop. Both, either the histone stem-loop or the 5'UTR element increased luciferase levels compared to mRNA lacking both these elements. Strikingly however, the combination of 5'UTR element and histone stem-loop further strongly increased the luciferase level, much above the level observed with either of the individual elements. The magnitude of the rise in luciferase level due to combining 5'UTR element and histone stem-loop in the same mRNA demonstrates that they are acting synergistically.

The synergy between 5'UTR element and histone stem-loop was quantified by dividing the signal from mRNA combining both elements by the sum of the signal from mRNA lacking both elements plus the rise in signal effected by the 5'UTR element plus the rise in signal effected by the histone stem-loop. This calculation was performed for the three time points individually and for total protein expressed from 0 to 48 hours calculated from the area under the curve (AUC) (see following Table 3).

Table 3:

| 8 h | | | | | |
|---|---|---|---|---|---|
| rpl32 | histoneSL | RLU | Δ RLU | RLU predicted (additive) | synergy |
| - | - | 13110 | | | |
| - | + | 88640 | 75530 | | |
| + | - | 155654 | 142544 | | |
| + | + | 301384 | | 231184 | 1,30 |
| 24 h | | | | | |
| rpl32 | histoneSL | RLU | Δ RLU | RLU predicted (additive) | synergy |
| - | - | 25861 | | | |
| - | + | 97013 | 71152 | | |
| + | - | 212245 | 186384 | | |
| + | + | 425825 | | 283397 | 1,50 |
| 48 h | | | | | |
| rpl32 | histoneSL | RLU | Δ RLU | RLU predicted (additive) | synergy |
| - | - | 14362 | | | |
| - | + | 57026 | 42664 | | |
| + | - | 102528 | 88166 | | |
| + | + | 161974 | | 145192 | 1,12 |
| AUC 0 - 48 hours | | | | | |
| rpl32 | histoneSL | RLU | Δ RLU | RLU predicted (additive) | synergy |
| - | - | 846881 | | | |
| - | + | 3688000 | 2841119 | | |
| + | - | 7343000 | 6496119 | | |
| + | + | 14080000 | | 10184119 | 1,38 |

The synergy thus calculated specifies how much higher the luciferase level from mRNA combining 5'UTR element and histone stem-loop is than would be expected if the effects of 5'UTR element and histone stem-loop were purely additive. This result confirms that the combination of 5'UTR element and histone stem-loop effects a markedly synergistic increase in protein expression.

5.3 5'UTR elements derived from 5'UTRs of TOP genes increase protein expression from mRNA.

To investigate the effect of 5'UTR elements derived from 5'UTRs of TOP genes on protein expression from mRNA, mRNAs with one of different 5'UTR elements were synthesized. In addition, mRNAs contained the albumin7 3'UTR element. Luciferase-encoding mRNAs were transfected into human dermal fibroblasts (HDF). Luciferase levels were measured at 24, 48, and 72 hours after transfection (see following Table 4 and Figure 21).

Table 4:

| 5'UTR | RLU at 24 hours | RLU at 48 hours | RLU at 72 hours |
|---|---|---|---|
| none | 114277 | 121852 | 68235 |
| rpl32 | 332236 | 286792 | 114148 |
| rpl35 | 495917 | 234070 | 96993 |
| rpl21 | 563314 | 352241 | 156605 |
| atp5a1 | 1000253 | 538287 | 187159 |
| HSD17B4 | 1179847 | 636877 | 299337 |
| AIG1 | 620315 | 446621 | 167846 |
| COX6C | 592190 | 806065 | 173743 |
| ASAH1 | 820413 | 529901 | 198429 |

Luciferase was clearly expressed from mRNA lacking a 5'UTR element. Strikingly however, all 5'UTR elements strongly increased the luciferase level.

**5.4 The combination of 5'UTR elements derived from 5'UTRs of TOP genes and histone stem-loop increases protein expression from mRNA in a synergistic manner.**

To investigate the effect of the combination of 5'UTR elements derived from the 5'UTRs of TOP genes and histone stem-loop on protein expression from mRNA, mRNAs with different UTRs were synthesized: mRNAs either lacked both 5'UTR element and histone stem-loop, or contained a histone stem-loop, or contained one of different 5'UTR elements derived from 5'UTRs of TOP genes, or contained both one of different 5'UTR elements and a histone stem-loop. In addition, mRNAs contained the alpha-globin 3'UTR element. Luciferase-encoding mRNAs were transfected into human dermal fibroblasts (HDF). Luciferase levels were measured at 8, 24, and 48 hours after transfection (see Figure 36). Luciferase was clearly expressed from mRNA having neither 5'UTR element nor histone stem-loop. The histone stem-loop increased the luciferase level. All 5'UTR elements also increased the luciferase level. Strikingly however, the combinations of 5'UTR element and histone stem-loop further strongly increased the luciferase level, much above the level observed with either of the individual elements. The magnitude of the rise in luciferase level due to combining 5'UTR element and histone stem-loop in the same mRNA demonstrates that they are acting synergistically.

The synergy between 5'UTR element and histone stem-loop was quantified by dividing the signal from mRNA combining both elements by the sum of the signal from mRNA lacking both elements plus the rise in signal effected by the 5'UTR element plus the rise in signal effected by the histone stem-loop. This calculation was performed for total protein expressed from 0 to 48 hours calculated from the area under the curve (AUC) (see following Table 5).

Table 5:

| TOP 5'UTR | Synergy with histone stem-loop |
|---|---|
| 35L | 2,50 |
| 21L | 3,25 |
| atp5a1 | 3,00 |

(continued)

| TOP 5'UTR | Synergy with histone stem-loop |
|---|---|
| HSD17B4 | 3,55 |
| AIG1 | 1,52 |
| COX6C | 3,19 |

The synergy thus calculated specifies how much higher the luciferase level from mRNA combining 5'UTR element and histone stem-loop is than would be expected if the effects of 5'UTR element and histone stem-loop were purely additive. The luciferase level from mRNA combining 5'UTR element and histone stem-loop was up to more than three times higher than if their effects were purely additive. This result confirms that the combination of 5'UTR element and histone stem-loop effects a markedly synergistic increase in protein expression.

**5.5 5'UTR elements derived from 5'UTRs of mouse TOP genes increase protein expression from mRNA.**

To investigate the effect of TOP 5'UTR elements derived from 5'UTRs of mouse TOP genes on protein expression from mRNA, mRNAs with two different mouse 5'UTR elements were synthesized. In addition, mRNAs contained the albumin7 3'UTR element. Luciferase-encoding mRNAs were transfected into human dermal fibroblasts (HDF). For comparison, mRNA containing the human rpl32 5'UTR element was transfected. Luciferase levels were measured at 24, 48, and 72 hours after transfection (see following Table 6 and Figure 39).

Table 6:

| 5'UTR | RLU at 24 hours | RLU at 48 hours | RLU at 72 hours |
|---|---|---|---|
| none | 114277 | 121852 | 68235 |
| rpl32 | 332236 | 286792 | 114148 |
| mrpl21 | 798233 | 351894 | 139249 |
| mrpl35A | 838609 | 466236 | 174949 |

Luciferase was clearly expressed from mRNA lacking a 5'UTR element. Both mouse 5'UTR elements strongly increased the luciferase level, similarly as the human 5'UTR element.

**Sequences:**

Homo sapiens alpha-2-macroglobulin (A2M):
gctccttctttctgcaacatg (Seq ID No: 1)

Homo sapiens acyl-CoA dehydrogenase, C-4 to C-12 straight ch ain (ACADM):

ggctctctttccgcgctgcggtcagcctcggcgtcccacagagagggccagaggtggaaa
cgcagaaaaccaaaccaggactatcagagattgcccggagaggggatg
(Seq ID No: 2)

Homo sapiens arylsulfatase E (chondrodysplasia punctata 1) (ARSE):

cttcctcttcttgatcggggattcaggaaggagcccaggagcagaggaagtagagagaga
gacaacatg (Seq ID No: 3)

Homo sapiens Bruton agammaglobulinemia tyrosine kinase (BTK) :

tgtccttcctctctggactgtaagaatatgtctccagggccagtgtctgctgcgatcgag

tcccaccttccaagtcctggcatctcaatgcatctgggaagctacctgcattaagtcagg
actgagcacacaggtgaactccagaaagaagaagctatg (Seq ID No: 4)

Homo sapiens complement component 2 (C2):
tgacctttccctcccgcggctctctacctctcgccgcccctagggaggacaccatg
(Seq ID No: 5)

Homo sapiens cyclin-dependent kinase 4 (CDK4):

gggcctctctagcttgcggcctgtgtctatggtcgggccctctgcgtccagctgctccgg
accgagctcgggtgtatggggccgtaggaaccggctccggggccccgataacgggccgcc
cccacagcaccccgggctggcgtgagggtctcccttgatctgagaatg
(Seq ID No: 6)

Homo sapiens cytochrome P450, family 17, subfamily A, polype ptide 1 (CYP17A1):
agctcttctactccactgctgtctatcttgcctgccggcacccagccaccatg
(Seq ID No: 7)

Homo sapiens endoglin (ENG):

cttcctctacccggttggcaggcggcctggcccagccccttctctaaggaagcgcatttc
ctgcctccctgggccggccgggctggatg (Seq ID No: 8)

Homo sapiens excision repair cross-complementing rodent repa ir deficiency, complementation group 3 (ERCC3):
tcttctctctgctgctgtagctgccatg (Seq ID No: 9)

Homo sapiens excision repair cross-complementing rodent repa ir deficiency, complementation group 5 (ERCC5):

ctgtctttcttccgggaggcggtgacagctgctgagacgtgttgcagccagagtctctcc
gctttaatgcgctcccattagtgccgtcccccactggaaaaccgtggcttctgtattatt
tgccatctttgttgtgtaggagcagggagggcttcctcccggggtcctaggcggcggtgc
agtccgtcgtagaagaattagagtagaagttgtcggggtccgctcttaggacgcagccgc
ctcatg (Seq ID No: 10)

Homo sapiens ferritin, light polypeptide (FTL):

cgtcccctcgcagttcggcggtcccgcgggtctgtctcttgcttcaacagtgtttggacg
gaacagatccggggactctcttccagcctccgaccgccctccgatttcctctccgcttgc
aacctccgggaccatcttctcggccatctcctgcttctgggacctgccagcaccgtttttt
gtggttagctccttcttgccaaccaaccatg (Seq ID No: 11)

Homo sapiens galactosylceramidase (GALC):
ccgcctccctgggcgccggagtcatgtgacccacacaatg (Seq ID No: 12)

Homo sapiens gap junction protein, alpha 1, 43kDa (GJAl):

ttttctttcattaggggaaggcgtgaggaaagtaccaaacagcagcggagttttaaact
ttaaatagacaggtctgagtgcctgaacttgccttttcattttacttcatcctccaagga
gttcaatcacttggcgtgacttcactactttaagcaaaagagtggtgcccaggcaacat
g (Seq ID No: 13)

Homo sapiens gap junction protein, beta 1, 32kDa (GJB1):

cattctctgggaaagggcagcagcagccaggtgtggcagtgacagggaggtgtgaatgag
gcaggatg (Seq ID No: 14)

Homo sapiens glucose-6-phosphate isomerase (GPI):
cgctccttcctcctcggctcgcgtctcactcagtgtaccttctagtcccgccatg
(Seq ID No: 15)

Homo sapiens hydroxyacyl-CoA dehydrogenase/3-ketoacyl-CoA th iolase/enoyl-CoA hydratase (trifunctional protein), alpha subunit (HADHA):
ctgtcctcttcagctcaagatg (Seq ID No: 16)

Homo sapiens hydroxyacyl-CoA dehydrogenase/3-ketoacyl-CoA th iolase/enoyl-CoA hydratase (trifunctional protein), beta subunit (HADHB):

gggccctttctgggcaggacccgccccttggtcccgcagagccttggtacttggacctga
accttgctccgagagggagtcctcgcggacgtcagccaagattccagaatg
(Seq ID No: 17)

Homo sapiens complement factor H (CFH):

cttccttttgcagcaagttctttcctgcactaatcacaattcttggaagaggagaactgg
acgttgtgaacagagttagctggtaaatgtcctcttaaaagatccaaaaaatg
(Seq ID No: 18)

Homo sapiens sarcoglycan, gamma (35kDa dystrophin-associated glycoprotein) (SGCG):

agccctttctccagggacagttgctgaagcttcatcctttgctctcattctgtaagtcat
agaaaagtttgaaacattctgtctgtggtagagctcgggccagctgtagttcattcgcca
gtgtgcttttcttaatatctaagatg (Seq ID No: 19)

Homo sapiens lipase A, lysosomal acid, cholesterol esterase (LIPA) :

ggtcccctatccgcaccccggcccctgagagctggcactgcgactcgagacagcggcccg
gcaggacagctccagaatg (Seq ID No: 20)

Homo sapiens lipoprotein lipase (LPL):

ccccctcttcctcctcctcaagggaaagctgcccacttctagctgccctgccatcccctt
taaagggcgacttgctcagcgccaaaccgcggctccagccctctccagcctccggctcag
ccggctcatcagtcggtccgcgccttgcagctcctccagagggacgcgccccgagatg
(Seq ID No: 21)

Homo sapiens mutL homolog 1, colon cancer, nonpolyposis type 2 (E. coli) (MLH1): ggctcttctggcgccaaaatg (Seq ID No: 22)

Homo sapiens Niemann-Pick disease, type C1 (NPC1):

cttccttcctgaccggcgcgcgcagcctgctgccgcggtcagcgcctgctcctgctcctc
cgctcctcctgcgcggggtgctgaaacagcccggggaagtagagccgcctccggggagcc
caaccagccgaacgccgccggcgtcagcagccttgcgcggccacagcatg
(Seq ID No: 23)

Homo sapiens peroxisomal biogenesis factor 12 (PEX12):

gcgcctctcttccgccaggcatcccagaggtcctggtggtttcatttccgggtgcggctt
ctgtcataaagcggagacctcccttcaaacgtggcgtcgtgggttgtttgcgcctcgcct
ggggtcagcgagcaaggacgggcgcgggcggggatactcaaagccaacagctggagtcag
cccttgtgtcccgggctcacagtggcacgactgaatcctcagagtcggctggcttttgag
ctctcacgattggggaggagggggcgtttctggttcgcagctccagaggattgcgttcct
tcccccatacctgtcccccacagtcacgctctgccctgacgtgcagcatttgacaagtta
ccccctcgccacatactacttccacccacgtccgagttaactttgttcttaaccttcttg
agactaccctcggcctccaggtctttttttcccagttcatttttgcccataagattgagt
ttcgagtttcagatatcatgcagaaagtttacctttaagactgagcacccatctgatact
cttcctcccgaaaaagttcatgctcacgagagagtttgtgggaaaagtgaaagccagtac
acgcaggaaactatg (Seq ID No: 24)

Homo sapiens peroxisomal biogenesis factor 6 (PEX6):
cgctccttcaccctcctcgttggtgtcctgtcaccatg (Seq ID No: 25)

Homo sapiens phosphofructokinase, muscle (PFKM):

gagccttcttgtcagcatctgttagtggaggttgggaagcctctcctccttcccctccc
tctttgcctccacctggctcctccccatgttcgtccatcacccctccccctttcccaag
gacaatctgcaagaaagcagcggcggaggagagctaagactaaaagagtggatcatg
(Seq ID No: 26)

Homo sapiens serpin peptidase inhibitor, clade A (alpha-1 antiproteinase, antitrypsin), member 1 (SERPINA1):
ctgtctcctcagcttcaggcaccaccactgacctgggacagtgaatcgacaatg
(Seq ID No: 27)

Homo sapiens phosphatase and tensin homolog (PTEN):

agttctctcctctcggaagctgcagccatgatggaagtttgagagttgagccgctgtgag
gcgaggccgggctcaggcgagggagatgagagacggcggcggccgcggcccggagcccct
ctcagcgcctgtgagcagccgcgggggcagcgccctcggggagccggccggcctgcggcg
gcggcagcggcggcgtttctcgcctcctcttcgtcttttctaaccgtgcagcctcttcct
cggcttctcctgaaagggaaggtggaagccgtgggctcgggcgggagccggctgaggcgc
ggcggcggcggcggcacctcccgctcctggagcggggggagaagcggcggcggcggcgg
ccgcggcggctgcagctccagggagggggtctgagtcgcctgtcaccatttccagggctg
ggaacgccggagagttggtctctccccttctactgcctccaacacggcggcggcggcggc
ggcacatccagggacccgggccggttttaaacctcccgtccgccgccgccgcacccccccg
tggcccgggctccggaggccgccggcggaggcagccgttcggaggattattcgtcttctc
cccattccgctgccgccgctgccaggcctctggctgctgaggagaagcaggcccagtcgc
tgcaaccatccagcagccgccgcagcagccattacccggctgcggtccagagccaagcgg
cggcagagcgagggcatcagctaccgccaagtccagagccatttccatcctgcagaaga
agccccgccaccagcagcttctgccatctctctcctccttttcttcagccacaggctcc
cagacatg (Seq ID No: 28)

Homo sapiens solute carrier family 3 (cystine, dibasic and neutral amino acid transporters, activ ator of cystine, dibasic and neutral amino acid transport), member 1 (SLC3AI):
cctcccttactgcaggaaggcactccgaagacataagtcggtgagacatg
(Seq ID No: 29)

Homo sapiens aldehyde dehydrogenase 3 family, member A2 (ALDH3A2) :
ccgcctcccactccccagcgcccccggaccgtgcagttctctgcaggaccaggccatg
(Seq ID No: 30)

Homo sapiens bleomycin hydrolase (BLMH):

gtttctcccagcctcagcctccccgccgccgccgccgccgccgagccggtttcc
tttttccggcgctccgggtgcgagagacaggtcgggccccctaggcagcgagccgcagcg
caatcccggcgctcgcccaaggaccctggaagctaccgttaccccgccgggcagcgtggg
cgccatg (Seq ID No: 31)

Homo sapiens cathepsin K (CTSK):

cctcctcctcttacccaaattttccagccgatcactggagctgacttccgcaatcccgat
ggaataaatctagcacccctgatggtgtgcccacactttgctgccgaaacgaagccagac
aacagatttccatcagcaggatg (Seq ID No: 32)

Homo sapiens GM2 ganglioside activator (GM2A):

gcttctttgcgtaaccaatactggaaggcatttaaaggcacctctgccgccacagacctt
gcagttaactccgccctgacccacccttcccgatg (Seq ID No: 33)

Homo sapiens hydroxysteroid (17-beta) dehydrogenase 4 (HSD17B4):

ccgcctcctcctgtcccgcagtcggcgtccagcggctctgcttgttcgtgtgtgtgtcgt
tgcaggccttattcatg (Seq ID No: 34)

Homo sapiens neutrophil cytosolic factor 2 (NCF2):

ctctctctgcttctttccttttctctctcatggtagggttatgagtcagttgccaaaagg tggggacatttcctgatgcatttgcaacactgagaagttatcttaagggaggctgggccc cattctactcatctggcccagaaagtgaacaccttggggccactaaggcagccctgcta ggggagacgctccaacctgtcttctctctgtctcctggcagctctcttggcctcctagtt tctacctaatcatg (Seq ID No: 35)

Homo sapiens 3-oxoacid CoA transferase 1 (OXCT1): cagcctcctcctgcctcaccgcccgaagatg (Seq ID No: 36)

Homo sapiens sulfite oxidase (SUOX):

ccgccccttctcgagaactcgcagagctgggctggtaaaattgcagtgctgaagacactg gacccgcaaaaggctgtccctcccaaacctgggattctgggctcactgagttcacctgcg agtcagccctacctgcactgctctggtctagtacaaacaggctgctggcattgagggacg gagtctccaactcctggcctctagcagtcctcctgtgtaggtctcccaaagtgctagtgt gtccggaattggtgggttcttggtctcactgacttcaagaatgaagccgcggaccctcgc agtctgctacaatg (Seq ID No: 37)

Homo sapiens albumin (ALB): ttttctcttctgtcaaccccacacgcctttggcacaatg (Seq ID No: 38)

Homo sapiens arylsulfatase A (ARSA):

ctccctctagcgccttccccccggcccgactccgctggtcagcgccaagtgacttacgcc cccgaccctgagcccggaccgctaggcgaggaggatcagatctccgctcgagaatctgaa ggtgccctggtcctggaggagttccgtcccagcccgcggtctcccggtactgtcgggccc cggccctctggagcttcaggaggcggccgtcagggtcggggagtatttgggtccggggtc tcagggaagggcggcgcctgggtctgcggtatcggaaagagcctgctggagccaagtagc cctccctctcttgggacagacccctcggtcccatg (Seq ID No: 39)

Homo sapiens elastin (ELN):

ctccctccctctttccctcacagccgacgaggcaacaattaggctttggggataaaacga ggtgcggagagcgggctggggcatttctccccgagatg (Seq ID No: 40)

Homo sapiens hemoglobin, alpha 2 (HBA2): cactcttctggtccccacagactcagagagaacccaccatg (Seq ID No: 41)

Homo sapiens hexosaminidase B (beta polypeptide) (HEXB):

cttcctctgatccgggccgggcgggaagtcgggtcccgaggctccggctcggcagaccgg gcggaaagcagccgagcggccatg (Seq ID No: 42)

Homo sapiens mannosidase, alpha, class 2B, member 1 (MAN2B1): cggcctttccagggccggggaaccccaggaggaagctgctgagccatg (Seq ID No: 43)

Homo sapiens recombination activating gene 2 (RAG2):

cactctctttacagtcagccttctgcttgccacagtcatagtgggcagtcagtgaatctt ccccaagtgctgacaattaatacctggtttagcggcaaagattcagagaggcgtgagcag cccctctggccttcagacaaaaatctacgtaccatcagaaactatg (Seq ID No: 44)

Homo sapiens CD53 molecule (CD53):

tctcctttacacaaatagccccggatatctgtgttaccagccttgtctcggccacctca aggataatcactaaattctgccgaaaggactgaggaacggtgcctggaaaagggcaagaa tatcacggcatg (Seq ID No: 45)

Homo sapiens Fc fragment of IgG, low affinity IIIa, receptor (CD16a) (FCGR3A): tggtccctttagggctccggatatctttggtgacttgtccactccagtgtggcatcatg (Seq ID No: 46)

Homo sapiens interleukin 1, beta (IL1B):

aaacctcttcgaggcacaaggcacaacaggctgctctgggattctcttcagccaatcttc attgctcaagtgtctgaagcagccatg (Seq ID No: 47)

Homo sapiens CD4 molecule (CD4):

ctgtctctcttcatttaagcacgactctgcagaaggaacaaagcaccctccccactgggc tcctggttgcagagctccaagtcctcacacagatacgcctgtttgagaagcagcgggcaa gaaagacgcaagcccagaggccctgccatttctgtgggctcaggtccctactggctcagg cccctgcctccctcggcaaggccacaatg (Seq ID No: 48)

Homo sapiens serpin peptidase inhibitor, clade A (alpha-1 antiproteinase, antitrypsin), member 5 (SERPINA5):

agccctctgccctttctgagcccgagggactgccacctccactgtgtgcacactcagcta cgggacacatttcaggtatccaaggcagcagaggtgagtgggtcccccgagctctgtgac cttatgctccacactaactctggcagagcctccgtttcctcatagaacaaagaacagcca ccatg (Seq ID No: 49)

Homo sapiens vitronectin (VTN):

tgccctccttccctgtctctgcctctccctcccttcctcaggcatcagagcggagacttc agggagaccagagcccagcttgccaggcactgagctagaagccctgccatg (Seq ID No: 50)

Homo sapiens aldehyde dehydrogenase 9 family, member A1 (ALDH9A1) :

ccgcccctcccgcggccccgcccctcccgcggcccgtcagcctctgccgcggagctgcgt ccgccactcatg (Seq ID No: 51)

Homo sapiens annexin A1 (ANXA1):

cttcctttaaaatcctataaaatcagaagcccaagtctccactgccagtgtgaaatcttc agagaagaatttctctttagttctttgcaagaaggtagagataaagacactttttcaaaa atg (Seq ID No: 52)

Homo sapiens ATPase, Na+/K+ transporting, alpha 1 polypeptid e (ATP1A1):
ttttctctctgattctccagcgacaggacccggcgccgggcactgagcaccgccaccatg
(Seq ID No: 53)

Homo sapiens ATPase, Na+/K+ transporting, alpha 2 polypeptid e (ATP1A2):

ctttctctgtctgccagggtctccgactgtcccagacgggctggtgtgggcttgggatcc
tcctggtgacctctcccgctaaggtccctcagccactctgccccaagatg
(Seq ID No: 54)

Homo sapiens calcium channel, voltage-dependent, beta 3 subu nit (CACNB3):

ccctccttcgcgctctctcgctccctgccgccgcccgcagggctgcggggctcggtggca
tctcccgggcgcggcccgcagtccttgcccctgcctccgggccgctcccgcccccggcgc
cgctcgctcccccgacccggactcccccatg (Seq ID No: 55)

Homo sapiens cholinergic receptor, nicotinic, alpha 7 (neuronal) (CHRNA7):

gtgcctctgtggccgcaggcgcaggcccgggcgacagccgagacgtggagcgcgccggct
cgctgcagctccgggactcaacatg (Seq ID No: 56)

Homo sapiens cytochrome P450, family 51, subfamily A, polype ptide 1 (CYP51A1):

gcttctctcgttccgtcgattgggaggagcggtggcgacctcggccttcagtgtttccga
cggagtgaatg (Seq ID No: 57)

Homo sapiens glutamate decarboxylase 1 (brain, 67kDa) (GAD1) :

atctctctcttctcctggcgctcgcgtgcgagagggaactagcgagaacgaggaagcagc
tggaggtgacgccgggcagattacgcctgtcagggccgagccgagcggatcgctgggcgc
tgtgcagaggaaaggcgggagtgcccggctcgctgtcgcagagccgagcctgtttctgcg
ccggaccagtcgaggactctggacagtagaggccccgggacgaccgagctgatg
(Seq ID No: 58)

Homo sapiens gamma-glutamyl carboxylase (GGCX):

aattctcctggcggcctccgttcagacgcggcagctgtgacccacctgcctcctccgcag
agcaatg (Seq ID No: 59)

Homo sapiens glutamate receptor, metabotropic 3 (GRM3):

tcccctctttccccaacctcctccctctcttctactccacccctccgttttcccactccc
cactgactcggatgcctggatgttctgccaccgggcagtggtccagcgtgcagccgggag
ggggcaggggcaggggggcactgtgacaggaagctgcgcgcacaagttggccatttcgagg
gcaaaataagttctcccttggatttggaaaggacaaagccagtaagctacctcttttgtg
tcggatgaggaggaccaaccatgagccagagcccgggtgcaggctcaccgccgccgctgc
caccgcggtcagctccagttcctgccaggagttgtcggtgcgaggaatttgtgacaggc
tctgttagtctgttcctcccttatttgaaggacaggccaaagatccagtttggaaatgag
agaggactagcatgacacattggctccaccattgatatctcccagaggtacagaaacagg
attcatgaagatg (Seq ID No: 60)

Homo sapiens guanylate cyclase 1, soluble, alpha 3 (GUCY1A3) :

ggttcctttggggtgatcaaagagggagacacagacacagagagacaaaggcaaggagga
ctgtctgggagccacgcgggcgatacagtttccgaggcacgccgcgtcccgcctagcctg
ttgaacaggtagacatgagcgacccaagctgcggatttgcgaggcgcgccctggagctgc
tagagatccggaagcacagccccgaggtgtgcgaagccaccaagtcaagttcctaacgag
tcttcagaggaggcagcaggaagctcagagagctgcaaagcaaccgtgcccatctgtcaa
gacattcctgagaagaacatacaagaaagtcttcctcaaagaaaaaccagtcggagccga
gtctatcttcacactttggcagagagtatttgcaaactgattttcccagagtttgaacgg
ctgaatgttgcacttcagagaacattggcaaagcacaaaataaaagaaagcaggaaatct
ttggaaagagaagactttgaaaaaacaattgcagagcaagcagttgcagcaggagttcca
gtggaggttatcaaagaatctcttggtgaagaggtttttaaaatatgttacgaggaagat
gaaaacatccttggggtggttggaggcacccttaaagatttttaaacagcttcagtacc
cttctgaaacagagcagccattgccaagaagcaggaaaaggggcaggcttgaggacgcc
tccattctatgcctggataaggaggatgattttctacatgtttactacttcttccctaag
agaaccacctccctgattcttcccggcatcataaaggcagctgctcacgtattatatgaa
acggaagtggaagtgtcgttaatg (Seq ID No: 61)

Homo sapiens 3-hydroxy-3-methylglutaryl-CoA reductase (HMGCR) :

ggctccttccgctccgcgactgcgttaactggagccaggctgagcgtcggcgccggggtt
cggtggcctctagtgagatctggaggatccaaggattctgtagctacaatg
(Seq ID No: 62)

Homo sapiens IMP (inosine 5'-monophosphate) dehydrogenase 2 (IMPDH2) :
aggtctctgcggcgcggtcctcggagacacgcggcggtgtcctgtgttggccatg
(Seq ID No: 63)

Homo sapiens leukotriene A4 hydrolase (LTA4H):

acttcctttcccggcgtgcaccgcgaatccctcctcctcttctttacctctctccctcct
cctcaggttctctatcgacgagtctggtagctgagcgttgggctgtaggtcgctgtgctg
tgtgatcccccagagccatg (Seq ID No: 64)

Homo sapiens neuropeptide Y receptor Yl (NPY1R):

ccttctttaataagcaggagcgaaaaagacaaattccaaagaggattgttcagttcaagg
gaatgaagaattcagaataattttggtaaatggattccaatatggggaataagaataagc
tgaacagttgacctgctttgaagaaacatactgtccatttgtctaaaataatctataaca
accaaaccaatcaaaatg (Seq ID No: 65)

Homo sapiens pyruvate dehydrogenase (lipoamide) beta (PDHB):
cggcccctctgttgtcgtttggcagcggatagaggacacgaccaagatg
(Seq ID No: 66)

Homo sapiens ribosomal protein L36a-like (RPL36AL):

cttccctttcctgttaggcgagagctgcgaaaggcgagagctgcgaagggccaggtgtcg
ggcgctgtttctcgttttcatcatatagacaaaacagccctgctgcaaagatg
(Seq ID No: 67)

Homo sapiens ATPase, Ca++ transporting, type 2C, member 1 (ATP2C1) :

gcttcttctcacgccgggagcaggctcccgcctcgcaccgctgccccgcgagcagctcct
cttctcccgaggcgcgcggggcgcccccgcgagccccgcggctgagaccccgcagcctgg
aggagggctgtccggggctttggatgctgctgctaggggtggtgggagcagccgtgggac
gcgtggccgggagcgggggtgacagcctgggattccgggggcttctcttccttgtcctcc
tcctctcctctctattcccagtgtggccgtggctgacactaaagactttgtagccatcaa
cccgagtgcagtttcgatggaaaatg (Seq ID No: 68)

Homo sapiens UDP-glucose pyrophosphorylase 2 (UGP2):

ccgcctctttcattgaagaaatttaagttcgtgtggttttacctttccgggagtctcca
gctggccctcatttgtgtccggagctcaggagttcccaaaccgactcagtcgcaccaagt
ttccgtcttttggaattggggaaggagtttctttctttcttttctttttcttgagccag
ttttaatcgctttgaataaatactcccttaagtagttaaatataggaggagaaagaatac
atcggttgttaaagcaggagaggaagagagacctgccctgtagcgtgactcctctagaaa
aaaaaaaaaaaagccggagtattttactaagcccctaaaatg (Seq ID No: 69)

Homo sapiens ATPase, Na+/K+ transporting, beta 1 polypeptide (ATP1B1) :

cctcctcctgctcctgccttggctcctccgccgcgcgtctcgcactccgagagccgcagc
ggcagcggcgcgtcctgcctgcagagagccaggccggagaagccgagcggcgcagaggac
gccagggcgcgcgccgcagccacccaccctccggaccgcggcagctgctgacccgccatc
gccatg (Seq ID No: 70)

Homo sapiens glycoprotein M6B (GPM6B):

ctgtctttatggaccagtaggcagagcgaaattgacgctgacaagacttttgcatcttgg
aagggactgtaatctactgtagtgaagaacagagcctctcaatcagacgggtgtaaataa

gagacggaggggagtccaaaagaaaaggaagaggaggaaaaacaagtgtgtgttgggggg
aacaggggaaaagcatttttggtggatggtatg (Seq ID No: 71)

Homo sapiens wntless homolog (Drosophila) (WLS):

gctcctttaagcgtccacaggcggcggagcggccacaatcacagctccgggcattgggg
aacccgagccggctgcgccgggggaatccgtgcgggcgccttccgtcccggtcccatcct
cgccgcgctccagcacctctgaagttttgcagcgcccagaaaggaggcgaggaaggaggg
agtgtgtgagaggagggagcaaaaagctcaccctaaaacatttatttcaaggagaaaaga
aaaagggggggcgcaaaaatg (Seq ID No: 72)

Homo sapiens flavin containing monooxygenase 3 (FMO3):

ttttctctttcaaactgcccagacggttggacaggacgtagacacacagaagaaaagaag
acaaagaacgggtaggaaaattaaaaaggttaccatg (Seq ID No: 73)

Homo sapiens multiple C2 domains, transmembrane 1 (MCTP1):

cagcctcttttgccggtattcagtgaagaaagcaagtctaaatatgcagttctctcactg
gagtgaaagatgttttgttcatttctaatcaactatg (Seq ID No: 74)

Homo sapiens structural maintenance of chromosomes 4 (SMC4):

ccgcctctcggcgagcccgccctcttctgaagaggcgtttctggaccactgagccccgcc
tcccactgtgagcggaaccctaccgtttttaaaaaatctttttcaaacttgccaggtt
gtctttccaaatatttttaataatagtgctgctgctgtagaccacagagaaaagaatccc
tcgctcttccttttcacttagtagaaacttctaccgcgtaggtcccgccaggagttcgcg
catgcgcaggagcgacaataagatggcggtgataatcgccgcactttttttcaaattagt
ggatcccagaaatcattgcgcgcatttgtaacgaatttccgttcgagtttgtattttagg
cgccattttcgagtgaaggacccggagccgaaacaccggtaggagcggggaggtgggtac
tacacaaccgtctccagccttggtctgagtggactgtcctgcagcgaccatg
(Seq ID No: 75)

Homo sapiens GLE1 RNA export mediator homolog (yeast) (GLE1):

tggccttcccggcggctgattcgagggcttgtttggtcagaagggggcgtcagagaagc
tgccccttagccaaccatg (Seq ID No: 76)

Homo sapiens tripartite motif containing 6 (TRIM6):

gagtctttcggcctgggtggaggacgcggctgcttcaagtccttggctctgatccaggcc
acagattccaggattctacaggcaggaaacatcttagaaatcagggttgggcaggcagga
gccaggagagtagctacaatg (Seq ID No: 77)

Homo sapiens ecotropic viral integration site 2A (EVI2A):

tatcctttttttactgcagatttactttaaggctcatattctccaagtctattctgcttta
aaaagaagacaagaaaagaagtggtttatcaaaatcacgttataatcagattttgaccaa
gcattttgtaagtatacaaatgtcagccaatgacatataacaaccatttcttataaaacc
ttgatgttcaaaagcctgactagcagtggcatccatg (Seq ID No: 78)

Homo sapiens heterogeneous nuclear ribonucleoprotein L (HNRNPL):

tgctcttttcgatccgggacggccggtcaggctcgccgccgagctggagaactacgatga
cccgcacaaaacccctgcctccccagttgtccacatcaggggcctgattgacggtgtggt

ggaagcagaccttgtggaggccttgcaggagtttggacccatcagctatgtggtggtaat
g (Seq ID No: 79)

Homo sapiens mitochondrial translational initiation factor 2 (MTIF2):

cattcttccgggtccagaaggtgatctccgcccgtgctcagaatccaggggcccggggct
gtagattccttgacaaggatatcctagcggcgaaacaacaccgtactgggagtcagaacg
tctgggttctagtcttgactgccattaactagcggtatgacattggagaagctttttga
cccttctggatttccgtttccttttctgtaaaatgaggagcttggaagatccggaaaatg
aggcccataggaaacaagtgacttgctgagtccagataacactgactgtcagagagaaac
atg (Seq ID No: 80)

Homo sapiens nuclear factor of kappa light polypeptide gene enhancer in B-cells inhibitor, zeta (NFKBIZ):

tggcctcctcttgccacgaggtcagacggcgagttcttagagaaaaaggctgcttagctg
ctgcttatcatgtaacctcaaaaggaaactgatcgtctttctcatgctgtcacgtacttg
ggttattatcgctgattacagctggaaacaattgatttgctcttacgtatttgtgtgact
tgactcttcaaacacaaaggttaacaggaagatctcgagggccctggctgaacttcacct
tttggctttcttggcctgatgctgaactctcgaggttgagccccatatg
(Seq ID No: 81)

Homo sapiens v-erb-b2 erythroblastic leukemia viral oncogene homolog 3 (avian) (ERBB3):

atccctccccggactccggctccggctccgattgcaatttgcaacctccgctgccgtcgc
cgcagcagccaccaattcgccagcggttcaggtggctcttgcctcgatgtcctagcctag
gggcccccgggccggacttggctgggctcccttcaccctctgcggagtcatg
(Seq ID No: 82)

Homo sapiens podoplanin (PDPN): ccgcctcctcgggagagataaatg (Seq ID No: 83)

Homo sapiens ribonucleotide reductase MI (RRM1):

gcgcccctttgtgcgtcacgggtggcgggcgcgggaaggggatttggattgttgcgcctc
tgctctgaagaaagtgctgtctggctccaactccagttctttcccctgagcagcgcctgg
aacctaacccttcccactctgtcaccttctcgatcccgccggcgctttagagccgcagtc
cagtcttggatccttcagagcctcagccactagctgcgatg (Seq ID No: 84)

Homo sapiens solute carrier family 2 (facilitated glucose transporter), member 4 (SLC2A4):

gcgtcttttcccccagccccgctccaccagatccgcgggagccccactgctctccgggtc
cttggcttgtggctgtgggtcccatcgggcccgccctcgcacgtcactccgggacccccg
cggcctccgcaggttctgcgctccaggccggagtcagagactccaggatcggttctttca
tcttcgccgcccctgcgcgtccagctcttctaagacgagatg (Seq ID No: 85)

Homo sapiens steroid-5-alpha-reductase, alpha polypeptide 1 (3-oxo-5 alpha-steroid delta 4-dehydrogenase alpha 1) (SRD5A1):

aaccctttctgcagagtcccggcagtgcgggactccggtagccgcccctccggtagccgc
ccctcctgcccccgcgccgccgccctatatgttgcccgccgcggcctctggggcatggag
cacgctgcccagccctggcgatg (Seq ID No: 86)

Homo sapiens thromboxane A synthase 1 (platelet) (TBXAS1):

gttccctttctacctgcagagcacggttcccataagggcggcgagatcagcctcctgtc
tcatctggaagaccaccactctggggtctcagaggaatg (Seq ID No: 87)

Homo sapiens transketolase (TKT):
ctatctctgtgtgtccgcgtgtgcgcccggtccccgcctgccgcaccatg
(Seq ID No: 88)

Homo sapiens tumor necrosis factor receptor superfamily, mem ber 1A (TNFRSF1A):

cctcctcctccagctcttcctgtcccgctgttgcaacactgcctcactcttcccctccca ccttctctcccctcctctctgctttaattttctcagaattctctggactgaggctccagt tctggcctttggggttcaagatcactgggaccaggccgtgatctctatgcccgagtctca accctcaactgtcaccccaaggcacttgggacgtcctggacagaccgagtcccgggaagc cccagcactgccgctgccacactgccctgagcccaaatgggggagtgagaggccatagct gtctggcatg (Seq ID No: 89)

Homo sapiens tubulin, beta 2A class IIa (TUBB2A):

aggtctctgcgcagcccagcccgccggtccacgccgcgcaccgctccgagggccagcgcc acccgctccgcagccggcaccatg (Seq ID No: 90)

Homo sapiens actin, beta (ACTB):
tcgcctttgccgatccgccgcccgtccacacccgccgccagctcaccatg
(Seq ID No: 91)

Homo sapiens adenylosuccinate synthase (ADSS):

ggctccttcttcctctgcatgtggctggcggccgcagagcagttcagttcgctcactcct cgccggccgcctctccttcgggctctcctcgcgtcactggagccatg
(Seq ID No: 92)

Homo sapiens alanyl (membrane) aminopeptidase (ANPEP):
cgttctctgcctggcctgaggctccctgagccgcctccccaccatcaccatg
(Seq ID No: 93)

Homo sapiens beaded filament structural protein 1, filensin (BFSP1):

gcctcctttctttctcagcccagacctggccctctggagagggttttggagtcctgggta ggcagggtacctcaggcagcaggcagcacaccttggatgtgagctgaatggattttcaaa tttcacagaaggagcctccatgctggagaaagtatgtatg (Seq ID No: 94)

Homo sapiens basic transcription factor 3 (BTF3):

cggcctccctttagctgccatcttgcgtccccgcgtgtgtgcgcctaatctcaggtggtc cacccgagaccccttgagcaccaaccctagtcccccgcgcggccccttattcgctccgac aagatg (Seq ID No: 95)

Homo sapiens complement component 1, q subcomponent binding protein (C1QBP): ttgtcctttgcatctgcacgtgttcgcagtcgtttccgcgatg (Seq ID No: 96)

Homo sapiens calsequestrin 1 (fast-twitch, skeletal muscle) (CASQ1):

tttcctttcttaatatggcgatgagctcttaggccagtgtggggaccggggctgaggtgc
cctggacactggaggagggggagggaaggagcccctgggagcctggggtagaagtgtagg
aggtgggaggattccggcccgcatggagctgtcctggcctcagaaggttatccgtctctc
ctgccaaccatggagacatatttagacaggaccaggtggggactgaggggtgccaatttc
agggggcagctccggttccctccccgcccccctgctcctattcctccacctgacccttttt
cccttggctctgtcggcagtttctccaggacccagcagtgccctctgtccactgctctgg
gccattccccaatccccccctcccacttgagcccctaactcagaatctgggacccaggggc
ccctccctaccccagctaacctcttctggaccaggagagccaacccagatcccactacct
ccatg (Seq ID No: 97)

Homo sapiens caveolin 3 (CAV3):

gtctctctgcccctctctgccccaagtatttcagccccagccggccacacagctcggat
ctcctcctgtggatccccccagctctgcgatg (Seq ID No: 98)

Homo sapiens serpin peptidase inhibitor, clade H (heat shock protein 47), member 1, (collagen binding protein 1) (SERPINH1):

aggtctttggctttttttggcggagctggggcgccctccggaagcgtttccaactttcca
gaagtttctcgggacgggcaggagggggtggggactgccatatatagatcccgggagcag
gggagcgggctaagagtagaatcgtgtcgcggctcgagagcgagagtcacgtcccggcgc
tagcccagcccgacccaggcccaccgtggtgcacgcaaaccacttcctggccatg
(Seq ID No: 99)

Homo sapiens CD68 molecule (CD68):

tttcctcctttccaagagagggctgagggagcagggttgagcaactggtgcagacagcct
agctggactttgggtgaggcggttcagccatg (Seq ID No: 100)

Homo sapiens cell division cycle 20 homolog (S. cerevisiae) (CDC20):

gggtccctttctgtcccctgagcaccgtcgcctcctttcctccagggctccgtaggcacc
aactgcaaggacccctccccctgcgggcgctcccatg (Seq ID No: 101)

Homo sapiens cadherin 13, H-cadherin (heart) (CDH13):

gagcctctcctcaaagcctggctcccacggaaaatatgctcagtgcagccgcgtgcatga
atgaaaacgccgccgggcgcttctagtcggacaaaatg (Seq ID No: 102)

Homo sapiens regulator of chromosome condensation (RCCI) and BTB (POZ) domain containing protein 2 (RCBTB2):

cgctcccttcgtttccgtctcggccgggcacccgagcgcatcccgccgaggccgggccgt
ttcagggggaggcgccaactcatcgcggcgccgggcccctgaccgtgcagtaaccgctac
ccaggaggcggagcggacaaggctccggcctgcgaggagtcacattaactttgctctaga
agacaactttacaaggatctaaaaggaacaggattaaagatgactgaatactgggttcca
gaaatttaaaacaatcagcttagcaaatcatatattcttctgtggagctgagaattgatg
tccgctcttccccgtgatttggaactttccaatcccagagaaaagttgacaaagggactg
cccaggactgagtccatatg (Seq ID No: 103)

Homo sapiens cold inducible RNA binding protein (CIRBP):

cccccccctcactcgcgcgttaggaggctcgggtcgttgtggtgcgctgtcttcccgcttg
cgtcagggacctgcccgactcagtggccgccatg (Seq ID No: 104)

Homo sapiens LIM domain binding 2 (LDB2):

cctcctctcctctccctctcctctcctgctatagagggctccgacagcagttcccagcca
gcgtgttcagcctgcctgcctgcctgcctctgtgtgtgtgtgagcgtgtgtgcgtgcgtc
tactttgtactgggaagaacacagcccatgtgctctgcatggacgttactgatactctgt
ttagcttgattttcgaaaagcaggcaagatg (Seq ID No: 105)

Homo sapiens chloride channel, nucleotide-sensitive, 1A (CLNS1A):

ctgcctcttccagggcgggcggtgtggtgcacgcattgctgtgctccaactccctcaggg
cctgtgttgccgcactctgctgctatg (Seq ID No: 106)

Homo sapiens collapsin response mediator protein 1 (CRMP1):

cctcctccttctcccgccctcctcgccgatccgggcggtgctggcagccggagcggcggc
gggcgggccgagcagccggggcagccgcgcgtgggcatccacgggcgccgagcctccgtc
cgtgtctctatccctcccgggcctttgtcagcgcgcccgctgggagcggggccgagagcg
ccggttccagtcagacagccccgcaggtcagcggccgggccgagggcgccagagggggcc
atg (Seq ID No: 107)

Homo sapiens catenin (cadherin-associated protein), delta 1 (CTNND1):

ttgcctttggctgggtgcaacttccattttaggtgttggatctgaggggaaaaaaaaga
gagagggagagagagagaaagaagagcaggaaagatcccgaaaggaggaagaggtggcga
aaaatcaactgccctgctggatttgtctttctcagcaccttggcgaagccttgggtttct
ttcttaaaggactgattttagaactccacatttgaggtgtgtggcttttgaagaaaatg
tatgtactgacgggaaaaggaggataagcaagtcgaattttgtcttacgctctctcctt
cctgcttcctccttgctgtggtggctgggatgcttcttccatgatttttgaatctagac
tgggctgttctctgtgttaaaccaatcagttgcgaccttctcttaacagtgtgaagtgag
ggggtctctctccctccttctccttcctctgtgattcaccttcctttttaccctgccctg
cggcggctccgccccttaccttcatg (Seq ID No: 108)

Homo sapiens diacylglycerol kinase, alpha 80kDa (DGKA):

ccgtcccctccagcccagctcgggctccagctccagcgccggcgcttcagctgcgaccgc
gagccctctcaagcaagatataacttccccaagtcacacagtggtatcagagctaagaat
gggacccagatatgactgatctagttctgttccaaaaccgtgctgtattatattaacgcc
taccctctgaagaggtccaagcaacggaagtactactacgaagctgcctttctggccatc
cttgagaaaaatagacagatgagttcctgccagtgagtccctaggcctccatctctctcc
cttgctgtaccaccttcaccaccatccatgcgaccccaagagccttaatgactctagaag
agactccaggcaggggaagctgaaaggacctttcactccctacttttggccagggccttc
tgtgccacctgccaagaccagcaggcctaccctctgaagaggtccaagcaacggaagtac
tactacgaagctgcctttctggccatccttgagaaaaatagacagatg
(Seq ID No: 109)

Homo sapiens aspartyl-tRNA synthetase (DARS):

cgatctttctggagccgcacctccacgcggagtccgagcgcgtgtgctgagaccccaggg

tcgggagggcggagactgggagggagggagaagcccctttggcctgccttacggaagcct
gcgagggagggtggtgtccactgcccagttccgtgtcccgatg (Seq ID No: 110)

Homo sapiens dynein, cytoplasmic 1, intermediate chain 2 (DYNC1I2):

agttcttctcgatcgtgtcagtttgtaaggcgagggcggaagttggattcctggcctgag
aatattaggcgtagttttccagtttttggcaaagcggaaatacttaaggcccctgggttg
actgggttctttgttttatctaccggcttctgctttacgacaggtcacaaacatg
(Seq ID No: 111)

Homo sapiens dedicator of cytokinesis 1 (DOCK1):

tttcctccccatcctgtcgcggctcgaaaggaatggaaaatggcggcctagacgcggagt
ttcctgcccgacccgcggcggctccggcggcgccatg (Seq ID No: 112)

Homo sapiens dihydropyrimidinase-like 2 (DPYSL2):

ctctctctttttttccgccctagctggggctgtgttggaggagaggaagaaagagagac
agaggattgcattcatccgttacgttcttgaaatttcctaatagcaagaccagcgaagcg
gttgcacccttttcaatcttgcaaaggaaaaaaacaaaacaaaacaaaaaaaacccaagt
ccccttcccggcagtttttgccttaaagctgccctcttgaaattaatttttcccaggag
agagatg (Seq ID No: 113)

Homo sapiens developmentally regulated GTP binding protein 2 (DRG2):

tgttctctttggcttccgggcgcacgctactctgtcgccgccgtcagaccggaattgccg
gtgccgccgccaccgctgtctgtgcgcccacctctgctgctaccatg
(Seq ID No: 114)

Homo sapiens eukaryotic translation elongation factor 1 alph a 1 (EEF1A1):

cgttctttttcgcaacgggtttgccgccagaacacaggtgtcgtgaaaactacccctaaa
agccaaaatg (Seq ID No: 115)

Homo sapiens eukaryotic translation elongation factor 1 gamm a (EEF1G):

tctcctctttcccccctcccttctctcccgggcggcttactttgcggcagcgccgagaacc
ccacccccctttctttgcggaatcaccatg (Seq ID No: 116)

Homo sapiens eukaryotic translation initiation factor 2, sub unit 3 gamma, 52kDa (EIF2S3): atttccttcctcttttggcaacatggcgggc (Seq ID No: 117)

Homo sapiens eukaryotic translation initiation factor 4B (EIF4B): gggtcttttgcgttctctttccctctcccaacatg (Seq ID No: 118)

Homo sapiens eukaryotic translation initiation factor 4 gamm a, 2 (EIF4G2): tattcttttgaagattcttcgttgtcaagccgccaaagtg

(Seq ID No: 119)

Homo sapiens epithelial membrane protein 1 (EMP1):

cttcccctcagtgcggtcacatacttccagaagagcggaccagggctgctgccagcacct gccactcagagcgcctctgtcgctgggacccttcagaactctctttgctcacaagttacc aaaaaaaaaagagccaacatg (Seq ID No: 120)

Homo sapiens fibrillarin (FBL):

cgctcttttccacgtgcgaaagccccggactcgtggagttgtgaacgccgcggactccgg agccgcacaaaccagggctcgccatg (Seq ID No: 121)

Homo sapiens exostoses (multiple)-like 2 (EXTL2):

ctgtcccttgctccaggcgctcactttgcgggcggcacttttttccaggttgttaatccag ctaatggagaaggatagatgcacgctacttggtttagaaaaaaaaacaaaaatgagcaaa cgagacgccccttccgttttatgataactaagctgcagggaaataaatcggctggcccta ctgcaatctactgcactcgagaaacatcacagaaaattctttgatttatcttaatagtga caagtgagcctgcttctgtcaattactgaagctataaggagatttttttaaaaattaaact tcaacacaatg (Seq ID No: 122)

Homo sapiens solute carrier family 37 (glucose-6-phosphate transporter), member 4 (SLC37A4):

ccgcctctgttcaggacactgggtccccttggagcctccccaggcttaatgattgtccag aaggcggctataaagggagcctgggaggctgggtggaggagggagcagaaaaaacccaac tcagcagatctgggaactgtgagagcggcaagcaggaactgtggtcagaggctgtgcgtc ttggctggtagggcctgctcttttctaccatg (Seq ID No: 123)

Homo sapiens GDP dissociation inhibitor 2 (GDI2):

agccctcccctcctcgctccctcccctcctctccccgcccagttcttctcttcccgtctg aggtggcggtcggtctcgccttgtcgccagctccatttcctctctttctcttcccccttt ccttcgcgcccaagagcgcctcccagcctcgtagggtggtcacggagcccctgcgccttt tccttgctcgggtcctgcgtccgcgcctgccccgccatg (Seq ID No: 124)

Homo sapiens UDP-Gal:betaGlcNAc beta 1,4- galactosyltransfer ase, polypeptide 1 (B4GALT1): caccccttcttaaagcg-gcggcgggaagatg (Seq ID No: 125)

Homo sapiens GDP-mannose 4,6-dehydratase (GMDS):

ggccctccctgcacggcctcccgtgcgcccctgtcagactgtggcggccggtcgcgcggt gcgctctccctccctgcccgcagcctggagaggcgcttcgtgctgcacacccccgcgttc ctgccggcaccgcgcctgccctctgccgcgctccgccctgccgccgaccgcacgcccgcc gcgggacatg (Seq ID No: 126)

Homo sapiens histone deacetylase 2 (HDAC2):

gggcccctcctcgcgagttggtgccgctgccacctccgattccgagctttcggcacctct
gccgggtggtaccgagccttcccggcgcccctcctctcctcccaccggcctgcccttcc
ccgcgggactatcgcccccacgtttccctcagcccttttctctcccggccgagccgcggc
ggcagcagcagcagcagcagcagcaggaggaggagcccggtggcggcggtggccggggag
cccatg (Seq ID No: 127)

Homo sapiens protein arginine methyltransferase 2 (PRMT2):

gggccttcccggctgacggcctgcgtgcactgcgcttgcgcgggttgagggcggtggctc
aggctcctggaaaggaccgtccacccctccgcgctggcggtgtggacgcggaactcagcg

gagaaacgcgattgagagcagtgtgtggattacactatcactggaaaaatacgaattgag
aagaaggaaaagactggaagatgcagaccttggttcctgttagtggaaacactgtaaggt
cccagaaatggaaaagaaaatgaaataaatcagcagttatgaggcagagcctaagagaac
tatg (Seq ID No: 128)

Homo sapiens immunoglobulin (CD79A) binding protein 1 (IGBP1): gttcctctctccccaagatg (Seq ID No: 129)

Homo sapiens eukaryotic translation initiation factor 3, sub unit E (EIF3E): actcccttttctttggcaagatg (Seq ID No: 130)

Homo sapiens activated leukocyte cell adhesion molecule (ALCAM):

gtccctctactcagagcagcccggagaccgctgccgccgctgccgctgctaccaccgctg
ccacctgaggagacccgccgccccccgtcgccgcctcctgcgagtccttcttagcacct
ggcgtttcatgcacattgccactgccattattattatcattccaatacaaggaaaataaa
agaagataccagcgaaaagaaccgcttacacctttccgaattactcaagtgtctcctgga
aacagagggtcgttgtccccggaggagcagccgaagggcccgtgggctggtgttgaccgg
gagggaggaggagttgggggcattgcgtggtggaaagttgcgtgcggcagagaaccgaag
gtgcagcgccacagcccaggggacggtgtgtctgggagaagacgctgcccctgcgtcggg
acccgccagcgcgcgggcaccgcggggcccgggacgacgcccccctcctgcggcgtggact
ccgtcagtggcccaccaagaaggaggaggaatatg (Seq ID No: 131)

Homo sapiens acyloxyacyl hydrolase (neutrophil) (AOAH):

ttttctttatcctgcagtctttacctcagcagaaccgcacaccacagactccctccagct
ctttgtgtgtggctctctcagggtccaacaagagcaagctgtgggtctgtgagtgtttat
gtgtgctttttattcacttcacacttattgaaaagtgtgtatgtgagagggtggggtgtgt
gtgtcaaagagagtgaggaagagaaggagagagagatcaattgattctgcagcctcagct
ccagcatccctcagttgggagcttccaaagccgggtgatcacttggggtgcatagctcgg
agatg (Seq ID No: 132)

Homo sapiens ADP-ribosylation factor 1 (ARF1):

ccgccccttacccggcgtgccccgcgcccggaggcgctgacgtggccgccgtcagagccg
ccatcttgtgggagcaaaaccaacgcctggctcggagcagcagcctctgaggtgtccctg
gccagtgtccttccacctgtccacaagcatg (Seq ID No: 133)

Homo sapiens ADP-ribosylation factor 6 (ARF6):

gcgccttttccggcagcggcggcggcagaactgggaggaggagttggaggccggagggag
cccgcgctcggggcggcggctggaggcagcgcaccgagttcccgcgaggatccatgacct
gacggggccccggagccgcgctgcctctcgggtgtcctgggtcggtggggagcccagtgc
tcgcaggccggcgggcgggccggagggctgcagtctccctcgcggtgagaggaaggcgga
ggagcgggaaccgcggcggcgctcgcgcggcgcctgcgggggggaagggcagttccgggcc
gggccgcgcctcagcagggcggcggctcccagcgcagtctcagggcccgggtggcggcgg
cgactggagaaatcaagttgtgcggtcggtgatgcccgagtgagcggggggcctgggcct
ctgcccttaggaggcaactcccacgcaggccgcaaaggcgctctcgcggccgagaggctt
cgtttcggtttcgcggcggcggcggcgttgttggctgaggggacccgggacacctgaatg
cccccggccccggctcctccgacgcgatg (Seq ID No: 134)

Homo sapiens ras homolog family member A (RHOA):

cgccctcccgccgccgcccgccctcgctctctcgcgctaccctcccgccgcccgcggtcc

tccgtcggttctctcgttagtccacggtctggtcttcagctacccgccttcgtctccgag
tttgcgactcgcggaccggcgtccccggcgcgaagaggctggactcggattcgttgcctg
agcaatg (Seq ID No: 135)

Homo sapiens ras homolog family member G (RHOG):

cggcctcccgctctcacttccttctcgagcccggagccgctgccgccgcccccagctccc
ccgcctcggggagggcaccaggtcactgcagccagaggggtccagaagagagaggaggca
ctgcctccactacagcaactgcacccacgatg (Seq ID No: 136)

Homo sapiens ATP synthase, H+ transporting, mitochondrial F1 complex, O subunit (ATP50):
ctctcttcccactcgggtttgacctacagccgcccgggagaagatg
(Seq ID No: 137)

Homo sapiens B lymphoid tyrosine kinase (BLK):

ccacctctgtctgctgccggcagaaagccacaagccatgaaaactgattgagatgagaag
aattcatctgggactggcttttgctttaggatggtgttggaagttgctcgttgtcgctag
gagcctgctccactgtaagggtgtcaggatctgaagagctatggtgaaacaccactgaag
cattgccaaggatg (Seq ID No: 138)

Homo sapiens B-cell translocation gene 1, anti-proliferative (BTG1):

gcatctcttcgcctctcggagctggaaatgcagctattgagatcttcgaatgctgcggag
ctggaggcggaggcagctggggaggtccgagcgatgtgaccaggccgccatcgctcgtct
cttcctctctcctgccgcctcctgtctcgaaaataactttttttagtctaaagaaagaaag
acaaaagtagtcgtccgcccctcacgccctctcttcctctcagccttccgcccggtgagg
aagcccggggtggctgctccgccgtcggggccgcgccgccgagccccagccgccccgggc
cgcccccgcacgccgcccccatg (Seq ID No: 139)

Homo sapiens calcium modulating ligand (CAMLG):

cggcctctagtcatcgccctcgcagcggcggccaacatcaccgccactgccacccctccc
agactgtggacgggaggatg (Seq ID No: 140)

Homo sapiens calnexin (CANX):

aggcctcttggttctgcggcacgtgacggtcgggccgcctccgcctctctctttactgcg gcgcggggcaaggtgtgcgggcgggaaggggcacgggcacccccgcggtccccgggaggc tagagatcatg (Seq ID No: 141)

Homo sapiens calpain 2, (m/II) large subunit (CAPN2): cgacctttctctgcgcagtacggccgccgggaccgcagcatg (Seq ID No: 142)

Homo sapiens caveolin 1, caveolae protein, 22kDa (CAV1):

gcgcctttttttcccccatacaatacaagatcttccttcctcagttcccttaaagcaca gcccagggaaacctcctcacagttttcatccagccacgggccagcatg (Seq ID No: 143)

Homo sapiens CDld molecule (CD1D):

cgacctctttgcagctcgcacagctaagggcgagggcgcccttcggcagaagcagcaaac cgccggcaagcccagcgaggagggctgccggggtctgggcttgggaattggctggcaccc

agcggaaagggacgtgagctgagcggcgggggagaagagtgcgcaggtcagaggcggcg cgcagcggcgctccgcgaggtccccacgccgggcgatatg (Seq ID No: 144)

Homo sapiens CD22 molecule (CD22):

tctcctttgctctcagatgctgccagggtccctgaagagggaagacacgcggaaacagg cttgcacccagacacgacaccatg (Seq ID No: 145)

Homo sapiens CD37 molecule (CD37):

cttcctcttttggggttcttcctttctctctcagctctccgtctctctttctctctcagc ctctttctttctccctgtctcccccactgtcagcacctcttctgtgtggtgagtggaccg cttaccccactaggtgaagatg (Seq ID No: 146)

Homo sapiens CD38 molecule (CD38):

gcctctctcttgctgcctagcctcctgccggcctcatcttcgcccagccaaccccgcctg gagccctatg (Seq ID No: 147)

Homo sapiens CD48 molecule (CD48):

cggccttttctagccaggctctcaactgtctcctgcgttgctgggaagttctggaagga agcatg (Seq ID No: 148)

Homo sapiens chromogranin B (secretogranin 1) (CHGB): cttcctttccgcacaggggccgccgagcggggccatg (Seq ID No: 149)

Homo sapiens chloride channel, voltage-sensitive 3 (CLCN3):

ttccccttccgtgggtcagggccggtccggtccggaacctgcagcccctttcccagtgtt
ctagttcgcccgtgacccggaataatgagcaaggagggtgtggtgggttgaaagccatcc
tactttactcccgagttagagcatggattcagttttagtcttaaggggggaagtgagattg
gagattttatttttaattttgggcagaagcaggttgactctagggatctccagagcgag
aggatttaacttcatgttgctcccgtgtttgaaggaggacaataaaagtcccaccgggca
aaattttcgtaacctctgcggtagaaaacgtcaggtatcttttaaatcgcgatagttttc
gctgtgtcaggctttcttcggtggagctccgagggtagctaggttctaggtttgaaacag
atgcagaatccaaaggcagcgcaaaaaacagccaccgattttgctatgtctctgagctgc
gagataatcagacagctaaatg (Seq ID No: 150)

Homo sapiens colipase, pancreatic (CLPS):

ttccccttccgtgggtcagggccggtccggtccggaacctgcagcccctttcccagtgtt
ctagttcgcccgtgacccggaataatgagcaaggagggtgtggtgggttgaaagccatcc
tactttactcccgagttagagcatggattcagttttagtcttaaggggggaagtgagattg
gagattttatttttaattttgggcagaagcaggttgactctagggatctccagagcgag
aggatttaacttcatgttgctcccgtgtttgaaggaggacaataaaagtcccaccgggca
aaattttcgtaacctctgcggtagaaaacgtcaggtatcttttaaatcgcgatagttttc
gctgtgtcaggctttcttcggtggagctccgagggtagctaggttctaggtttgaaacag
atgcagaatccaaaggcagcgcaaaaaacagccaccgattttgctatgtctctgagctgc
gagataatcagacagctaaatg (Seq ID No: 151)

Homo sapiens cytochrome c oxidase subunit IV isoform 1 (COX4I1):

ctacccttttccgctccacggtgacctccgtgcggccgggtgcgggcggagtcttcctcg
atcccgtggtgctccgcggcgcggccttgctctcttccggtcgcgggacaccgggtgtag
agggcggtcgcggcgggcagtggcggcagaatg (Seq ID No: 152)

Homo sapiens cytochrome c oxidase subunit VIIc (COX7C):

ctttcttttcagtccttgcgcaccggggaacaaggtcgtgaaaaaaaaggtcttggtgag
gtgccgccatttcatctgtcctcattctctgcgcctttcgcagagcttccagcagcggta
tg (Seq ID No: 153)

Homo sapiens activating transcription factor 2 (ATF2):

cagccttttcctccaggggtgctttgtaaacacggctgtgctcagggctcgcgggtgacc
gaaaggatcatgaactagtgacctggaaagggtactagatggaaacttgagaaaggactg
cttattgataacagctaaggtattcctggaagcagagtaaataaagctcatggcccacca
gctagaaagtattcttgccatgagaaaaagaatgtgataagttattcaacttatg
(Seq ID No: 154)

Homo sapiens casein kinase 1, alpha 1 (CSNK1A1):

agatccctttcccagagtgctctgcgccgtgaagaagcggctcccggggactgggggcat tttgtgttggctggagctggagtaacaagatggcgtcgtccgcggagtgacaggggtccc tctgggccggagccggcggcagtggtggcagcggtatcgccgccctagctcaccgcgccc cttttccagcccgcgacgtcgccgcgcaagcgaggcagcggcggccgccgagaaacaagt ggcccagcctggtaaccgccgagaagcccttcacaaactgcggcctggcaaaaagaaacc tgactgagcggcggtgatcaggttcccctctgctgattctgggccccgaaccccggtaaa ggcctccgtgttccgtttcctgccgccctcctccgtagccttgcctagtgtaggagcccc gaggcctccgtcctcttcccagaggtgtcggggcttggccccagcctccatcttcgtctc tcaggatg (Seq ID No: 155)

Homo sapiens catenin (cadherin-associated protein), beta 1, 88kDa (CTNNB1):

aagcctctcggtctgtggcagcagcgttggcccggccccgggagcggagagcgaggggag gcggagacggaggaaggtctgaggagcagcttcagtccccgccgagccgccaccgcaggt cgaggacggtcggactcccgcggcgggaggagcctgttcccctgagggtatttgaagtat accatacaactgttttgaaaatccagcgtggacaatg (Seq ID No: 156)

Homo sapiens dCMP deaminase (DCTD):

ccgcctcctcccccgacttccttccctgagcacggcggcggcggggacgagcaccggcct gcgcgcggagccggcaccggatgacccaacatg (Seq ID No: 157)

Homo sapiens damage-specific DNA binding protein 1, 127kDa (DDB1):

ctgtcttttcgcttgtgtccctctttctagtgtcgcgctcgagtcccgacgggccgctcc aagcctcgacatg (Seq ID No: 158)

Homo sapiens desmin (DES):

ctgtctcccctcgccgcatccactctccggccggccgcctgcccgccgcctcctccgtgc gcccgccagcctcgcccgcgccgtcaccatg (Seq ID No: 159)

Homo sapiens deoxyhypusine synthase (DHPS):

cgttccctacttcctgtgctcttgcggagacgcgcgcgtcggggtttaacgcgtttctgg gccgccgtaagcccggcctaggggcagctttgactcgagagccggctataggcgcatg (Seq ID No: 160)

Homo sapiens dihydrolipoamide S-acetyltransferase (DLAT):

caccctttcggatgcctcccctagaaccctaccactttccacccctttccgtctgttatt

tctcccaaacttgcgcccgcacaggcccctctggaacactcctgccccgtagtgcccctc gtccccgctccgtagagaaagagcgtgcgtgccgcgcatttctggcctggggagcgggtg gagtaaacctgcgggaaccattttacgacaacgtgcggctgtgcggtgtggctgacggca acgccgctgctcttggagaggtcactccggagacggcgttggttttggggtgtgggggt tggtggcactatg (Seq ID No: 161)

Homo sapiens down-regulator of transcription 1, TBP-binding (negative cofactor 2) (DR1):

ccttccctggcatctggagggaccaccgttgccgcgtcttcggcttccacgatctgcgtt
cgggctacgcggccacggcggcagccactgcgactcccactgtgcctggctctgtccata
ttagttcccaggcggccgtcgccgttccagcagcggcagcggcagcggcagcggcggaca
tgttgtgaggcggcggcgcgggtgtctgaaggatggtttggccgaggcggcggcaacggc
tgctggcggcggcggcagcggcagcggggcctcgggctctatagagccgagcccgctggg
tacccgcccggtaccgcggcgaggccagtgcccctggatcttgcctctgctccgacgccg
ttggggaccagttaggcgacagcgcccgcccctctgaggagacacgaaggtggttcccca
gccgctcaaatttccggaccaccgcgctttcccctcctcagcctgggctgtgctctctct
agaatcctcgggcccccactttcttcccaaactcatcctaaatctctcacacacgcgagt
gttcccagccctcaagccagctgctcctccgttcatttctgcaccctcttcgcaaagca
ccccccgggatcactctccgagggcgacttttgagaaatctcggtggagtagtggacca
gagctggggagtttttaaaagccggggcgcgagaaacaggaaggtactatg
(Seq ID No: 162)

Homo sapiens endothelin receptor type A (EDNRA):

ttttctttttcgtgcgagccctcgcgcgcgcgtacagtcatcccgctggtctgacgattg
tggagaggcggtggagaggcttcatccatcccacccggtcgtcgccggggattggggtcc
cagcgagacctccccgggagaagcagtgcccaggaggttttctgaagccggggaagctgt
gcagccgaagccgccgccgcgccggagcccgggacaccggccaccctccgcgccacccac
cctcgccggctccggcttcctctggcccaggcgccgcgcggacccggcagctgtctgcgc
acgccgagctccacggtgaaaaaaaagtgaaggtgtaaaagcagcacaagtgcaataaga
gatatttcctcaaatttgcctcaagatg (Seq ID No: 163)

Homo sapiens eukaryotic translation elongation factor 1 alph a 2 (EEF1A2):

cagtccctctggctgagacctcggctccggaatcactgcagccccccctcgccctgagcca
gagcaccccgggtcccgccagcccctcacactcccagcaaaatg
(Seq ID No: 164)

Homo sapiens eukaryotic translation elongation factor 2 (EEF2):

cgttctcttccgccgtcgtcgccgccatcctcggcgcgactcgcttctttcggttctacc
tgggagaatccaccgccatccgccaccatg (Seq ID No: 165)

Homo sapiens eukaryotic translation initiation factor 4A2 (EIF4A2): ctgtcttttcagtcgggcgctgagtggtttttcggatcatg (Seq ID No: 166)

Homo sapiens egf-like module containing, mucin-like, hormone receptor-like 1 (EMR1): gtttcttttctttgaatgacagaactacagcataatg (Seq ID No: 167)

Homo sapiens enolase 2 (gamma, neuronal) (ENO2):

gcgcctcctccgcccgccgcccgggagccgcagccgccgccgccactgccactcccgctc
tctcagcgccgccgtcgccaccgccaccgccaccgccactaccaccgtctgagtctgcag
tcccgagatcccagccatcatg (Seq ID No: 168)

Homo sapiens esterase D (ESD):

ccgccttttacttcggcccgcttcttctggtcactccgccaccgtagaatcgcctaccat ttggtgcaagcaaaaagcaatcagcaattggacaggaaaagaatg (Seq ID No: 169)

Homo sapiens Finkel-Biskis-Reilly murine sarcoma virus (FBR-MuSV) ubiquitously expressed (FAU): cttcctctttctcgactccatcttcgcggtagctgggaccgccgttcagtcgccaatatg (Seq ID No: 170)

Homo sapiens Friend leukemia virus integration 1 (FLI1):

ctgtctctttcgctccgctacaacaacaaacgtgcacaggggagtgagggcagggcgctc gcagggggcacgcagggagggcccagggcgccagggaggccgcgccgggctaatccgaag gggctgcgaggtcaggctgtaaccgggtcaatgtgtggaatattgggggctcggctgca gacttggccaaatg (Seq ID No: 171)

Homo sapiens fibromodulin (FMOD):

gccccttttcacaatatttgattaggaatttggggcgggaccctggtctggcacaggcac gcacactctcagtagactctttcactcctctctcttcctctctcacacgttctccaac ccaaggaggccagacagagggacgtggtcactctctgaaaagttcaacttgagagacaaa atg (Seq ID No: 172)

Homo sapiens ferritin, heavy polypeptide 1 (FTH1):

cgttcttcgccgagagtcgtcggggtttcctgcttcaacagtgcttggacggaacccggc gctcgttccccaccccggccggccgcccatagccagccctccgtcacctcttcaccgcac cctcggactgccccaaggcccccgccgccgctccagcgccgcgcagccaccgccgccgcc gccgcctctccttagtcgccgccatg (Seq ID No: 173)

Homo sapiens glyceraldehyde-3-phosphate dehydrogenase (GAPDH):

cgctctctgctcctcctgttcgacagtcagccgcatcttcttttgcgtcgccagccgagc cacatcgctcagacaccatg (Seq ID No: 174)

Homo sapiens glycyl-tRNA synthetase (GARS): caccctctctggacagcccagggccgcaggctcatg (Seq ID No: 175)

Homo sapiens glutamic-oxaloacetic transaminase 2, mitochondr ial (aspartate aminotransferase 2) (GOT2):

ctgtccttaccttcagcaggagccggttccctgtgtgtgtgtccgctcgccctctgctcc gtcctgcggctgcccactgccctcctacggtccaccatg (Seq ID No: 176)

Homo sapiens general transcription factor IIF, polypeptide 1, 74kDa (GTF2F1):

gcgcctcttccggttacctttcccagcgccagaggcgcctagggttggggtcctcgctc aggcacagagacccgacaccgagcggcggcttccccgggatcgagggacgcgcacgccag

aggagacgaaaggaacccgggtcggaccagatcggaaccactgaccattgcccatg (Seq ID No: 177)

Homo sapiens glycogen synthase 1 (muscle) (GYS1):

cggcctccttctgcctaggtcccaacgcttcggggcaggggtgcggtcttgcaataggaa
gccgagcgtcttgcaagcttcccgtcgggcaccagctactcggccccgcaccctacctgg
tgcattccctagacacctccggggtccctacctggagatccccggagccccccttcctgc
gccagccatg (Seq ID No: 178)

Homo sapiens major histocompatibility complex, class I, C (HLA-C): cattctccccagaggccgagatg (Seq ID No: 179)

Homo sapiens major histocompatibility complex, class II, DP beta 1 (HLA-DPB1):

gctccctttagcgagtccttcttttcctgactgcagctcttttcattttgccatcctttt
ccagctccatg (Seq ID No: 180)

Homo sapiens 3-hydroxy-3-methylglutaryl-CoA synthase 1 (soluble) (HMGCS1):

ctgtcctttcgtggctcactccctttcctctgctgccgctcggtcacgcttgctctttca
ccatg (Seq ID No: 181)

Homo sapiens hippocalcin (HPCA):

ccgccttccctgcgcagtcggtgtctccgcgtcgctgggtgggacttggctcggcggcca
tg (Seq ID No: 182)

Homo sapiens hydroxysteroid (17-beta) dehydrogenase 2 (HSD17B2):

ctcccttcttgactctctgttcacagaactcaggctgcctccagccagcctttgcccgct
agactcactggccctgagcacttgaaggtgcagcaagtcactgagaatg
(Seq ID No: 183)

Homo sapiens heat shock 60kDa protein 1 (chaperonin) (HSPD1):

ctgtccctcactcgccgccgacgacctgtctcgccgagcgcacgccttgccgccgccccg
cagaaatg (Seq ID No: 184)

Homo sapiens intercellular adhesion molecule 3 (ICAM3):

ccgccttttcccctgcctgcccttcgggcacctcaggaaggcaccttcctctgtcagaat
g (Seq ID No: 185)

Homo sapiens inositol polyphosphate-1-phosphatase (INPP1):

cgtcctctggccgcgcctgcggccgcacgcccagcgcccctcgcctaacctcgcgcccgg
gccgcgcctcctcctcctcctgctccccgccgcttccgtttctcgagggaaaggctgctg
cctcctgctctgtcctcatccccggcttagctgacggcccagagggtgggtgccaattcc
accagcagctgcaactgaaaagcaaggttcagaaatg (Seq ID No: 186)

Homo sapiens interferon regulatory factor 2 (IRF2):

gtttcctctccttgttttgctttcgatctggactgttctcaggcaagccggggagtaact
tttagttttgctcctgcgattattcaactgacgggctttcatttccatttcacataccct

agcaacacttataccttgcggaattgtattggtagcgtgaaaaagcacactgagagggc
accatg (Seq ID No: 187)

Homo sapiens inter-alpha-trypsin inhibitor heavy chain 2 (ITIH2):

ttttcttctttttcttctttcttaaagcgaactgtactcctctgctgttcctttgaact
tggttcagtaggaagaagtgatatcctccccagaccatctgctttggggagcttggcaaa
actgtccagcaaaatg (Seq ID No: 188)

Homo sapiens karyopherin (importin) beta 1 (KPNB1):

ccgccttcctccctccctcgctccctccctgcgcgccgcctctcactcacagcctccctt
ccttctttctccctccgcctcccgagcaccagcgcgctctgagctgcccccagggtccct
cccccgccgccagcagcccatttggagggaggaagtaagggaagaggagaggaaggggag
ccggaccgactacccagacagagccggtgaatgggtttgtggtgacccccgcccccacc
ccaccctcccttcccacccgacccccaacccccatccccagttcgagccgccgcccgaaa
ggccgggccgtcgtcttaggaggagtcgccgccgccgccacctccgccatg
(Seq ID No: 189)

Homo sapiens karyopherin alpha 3 (importin alpha 4) (KPNA3):

ctctccccctcctccccctcccgctccaagattcgccgccgccgccgccgcagccgcagg
agtagccgccgccggagccgcgcagccatg (Seq ID No: 190)

Homo sapiens keratin 19 (KRT19):

gctcctcccgcgaatcgcagcttctgagaccagggttgctccgtccgtgctccgcctcgc
catg (Seq ID No: 191)

Homo sapiens laminin, beta 1 (LAMB1):

attcccttctttgggctcgggggctcccggagcagggcgagagctcgcgtcgccggaaag
gaagacgggaagaaagggcaggcggctcggcgggcgtcttctccactcctctgccgcgtc
cccgtggctgcagggagccggcatg (Seq ID No: 192)

Homo sapiens ribosomal protein SA (RPSA):

ctgtcttttccgtgctacctgcagaggggtccatacggcgttgttctggattcccgtcgt
aacttaaagggaaattttcacaatg (Seq ID No: 193)

Homo sapiens lymphocyte cytosolic protein 1 (L-plastin) (LCP1) :

ttttctttcctggctgatgatttgtcattctagtcacttcctgccttgtgaccacacacc
caggcttgacaaagctgttctgcagatcagaaagaaggggttcctggtcatacaccagta
ctaccaaggacagctttttcctgcaagatctgttacctaaagcaataaaaatg
(Seq ID No: 194)

Homo sapiens lectin, galactoside-binding, soluble, 1 (LGALS1) :

ccatctctctcgggtggagtcttctgacagctggtgcgcctgcccgggaacatcctcctg
gactcaatcatg (Seq ID No: 195)

Homo sapiens SH2 domain containing 1A (SH2D1A):

ttctctctttttgcacatctggctgaactgggagtcaggtggttgacttgtgcctggct

gcagtagcagcggcatctcccttgcacagttctcctcctcggcctgcccaagagtccacc
aggccatg (Seq ID No: 196)

Homo sapiens mannosidase, alpha, class 2A, member 1 (MAN2A1) :

tgttcctttcccctccgcttctctgacctagctgcgcggccccggcccgggagctgccga
acccgcgcctcccctgggtgaggaggacacgcctgccctcgtcgagaaaacttttcctgc
cgactcagttggggcggcggtggcaggaagtgcgggcagcgacctctcctccgcctgccc
cgcgcgccctgccggaggtcggcgctgagcttgcgatcaagtttgtgggggcccccttc
ccagttgccggcgagtctcgcctcgagaggggcgcccgaccccggggagggcggcaggcc
agggcgaaggccaagggcgtgtggtggcgccggagactaggtgcggagcaaggcggggac
tcgcacccgcatccgagagcgcggaggtcgcgcagcccgggagaagggagcctccggcgg
ctgcttcctagagtccacagtgcgctgtctcctttggctgaggagagtgtcctggccccg
agtctatcgaggaaaatg (Seq ID No: 197)

Homo sapiens myelin basic protein (MBP):

ccgcctcttttcccgagatgccccggggagggaggacaacaccttcaaagacaggccctc
tgagtccgacgagctccagaccatccaagaagacagtgcagccacctccgagagcctgga
tgtgatg (Seq ID No: 198)

Homo sapiens melanocortin 1 receptor (alpha melanocyte stimulating hormone receptor) (MC1R):

cattcttcccaggacctcagcgcagccctggcccaggaaggcaggagacagaggccagga
cggtccagaggtgtcgaaatgtcctggggacctgagcagcagccaccagggaagaggcag
ggagggagctgaggaccaggcttggttgtgagaatccctgagcccaggcggtagatgcca
ggaggtgtctggactggctgggccatgcctgggctgacctgtccagccagggagagggtg
tgagggcagatctgggggtgcccagatggaaggaggcaggcatgggggacacccaaggcc
ccctggcagcaccatgaactaagcaggacacctggaggggaagaactgtggggacctgga
ggcctccaacgactccttcctgcttcctggacaggactatg (Seq ID No: 199)

Homo sapiens malic enzyme 1, NADP(+)-dependent, cytosolic (ME1) :

gggcctttcccagtgcggccgccgccgccacagctgcagtcagcaccgtcaccccagcag
catccgccgcctgcaccgcgcgtgcggcccgccccggcctgaccccgccgccgaacccgg
cgccagccatg (Seq ID No: 200)

Homo sapiens myocyte enhancer factor 2C (MEF2C):

agctctctgctcgctctgctcgcagtcacagacacttgagcacacgcgtacacccagaca
tcttcggctgctattggattgactttgaaggttctgtgtgggtcgccgtggctgcatgt
ttgaatcaggtggagaagcacttcaacgctggacgaagtaaagattattgttgttatttt
tttttctctctctctctcttaagaaaggaaaatatcccaaggactaatctgatcggg
tcttccttcatcaggaacgaatgcaggaatttgggaactgagctgtgcaagtgctgaaga
aggagatttgtttggaggaaacaggaaagagaaagaaaaggaaggaaaaaatacataatt
tcagggacgagagagagaagaaaaacggggactatg (Seq ID No: 201)

Homo sapiens mannosyl (alpha-1,3-)-glycoprotein beta-1,2-N-acetylglucosaminyltrans ferase (MGAT1):

agcccttcttggggaagtcagctacccagcagcctgtagtcctcggctacccaccctcac

cgcctggggtcccatggtgagacagctgggtgggcatcaggcttctgcagagggccaggc
cggagggagctgggcgagggagtggggctggctcctggcttgcaccggcctcgtggaatc
caggcctcagacctgatcgctggcgaaactggctctgtgcgctggagcccctggtcttct
gcgtctgtcctcctcccggccagactttactcctggctcagcgacaggtatttgctatgg
aagagctgtccctccctcccctcggtgggcctgggtccacctccacctcctcttcaggtc
cgcaccttcctcccctttaaacaccagccgggcgcagacccgttctaggcttttccatg
gtgcttccgccaaagcttgtgaccgagtccttcccgcctagggctggtgggcctcccctg
ctggtaggtctctcttcgctttctttactcagaactgaagctctcattccccacccacca
aggaaaaacaaaagggaagaagccacagctggccccggcttgctttggcacaggtgtttc
cccccggcccccccgtcgggcaccctggttcctgttctgtccctgccccacgcgaccctgg
ggctcccacccgggctcctcagcctcccctgggttggggtgggggactggctcccagcc
cttggcctagggtttggtgaacgcctttcctggactgcgggcccacttcaggcgcggctc
caggctgggcagctgcgctggagggccgagggcaggggtggggtcgggcgtccaccctca
gggttgcgccagggagccggaaagccgactcccgaagttggggtcctgggaaaacttggg
tcctgggttgactgagaagcggcggggaaaggaggcgggccaggaggagggggcctggcg
gacgccggccgggggcggggcgcggcggggctgtcggtcacgcccctcagtccgccccg
ccccgccccgcctgccggggaagggccacgttgcccgcccggccgtccggccccggcgcg
ccgcagaaagggctggcgagtcgaaaggcgaggcggccgcggcagcgcttgggacgcgcc
tgggcaccgggctcgctccctgcgccccggagcaggccaagttcggggccaggacgtcgg
gaggacctggtgcatggctgcctcctaatcccatagtccagaggaggcatccctaggact
gcgggcaagggagccgggcaagcccagggcagccttgaaccgtcccctggcctgccctcc
ccggtgggggccaggatg (Seq ID No: 202)

Homo sapiens mitogen-activated protein kinase kinase kinase 11 (MAP3K11):

ctgcctcccgcccccggggccaaagtacaaaggaggaggaagaagggagcggggtcgga
gccgtcggggccaaaggagacggggccaggaacaggcagtctcggcccaactgcggacgc
tccctccacccccctgcgcaaaaagacccaaccggagttgaggcgctgcccctgaaggccc
caccttacacttggcgggggccggagccaggctcccaggactgctccagaaccgagggaa
gctcgggtccctccaagctagccatggtgaggcgccggaggccccggggccccaccccccc
cggcctgaccacactgccctgggtgccctcctccagaagcccgagatgcgggggccggg
agacaacactcctggctccccagagaggcgtgggtctggggctgagggccagggcccgga
tgcccaggttccgggactagggccttggcagccagcgggggtggggaccacgggcaccca
gagaaggtcctccacacatcccagcgccggctcccggccatg (Seq ID No: 203)

Homo sapiens membrane protein, palmitoylated 1, 55kDa (MPP1) :

ccgccttctccgcagccccgcaggccccgggccctgtcattcccagcgctgccctgtctt
gcgttccagtgttccagcttctgcgagatg (Seq ID No: 204)

Homo sapiens v-myc myelocytomatosis viral oncogene homolog (avian) (MYC):

ggccctttataatgcgagggtctggacggctgaggacccccgagctgtgctgctcgcggc
cgccaccgccgggccccggccgtccctggctcccctcctgcctcgagaagggcagggctt
ctcagaggcttggcgggaaaaagaacggagggagggatcgcgctgagtataaaagccggt
tttcggggctttatctaactcgctgtagtaattccagcgagaggcagagggagcgagcgg
gcggccggctagggtggaagagccgggcgagcagagctgcgctgcgggcgtcctgggaag
ggagatccggagcgaataggggcttcgcctctggcccagccctcccgctgatcccccag
ccagcggtccgcaacccttgccgcatccacgaaactttgcccatagcagcgggcgggcac

tttgcactggaacttacaacacccgagcaaggacgcgactctcccgacgcggggaggcta
ttctgcccatttggggacacttccccgccgctgccaggacccgcttctctgaaaggctct
ccttgcagctgcttagacgctg (Seq ID No: 205)

Homo sapiens nuclear cap binding protein subunit 1, 80kDa (NCBP1): tggcctctcggttccgcggcgcaccggagggcagcatg (Seq ID No: 206)

Homo sapiens necdin homolog (mouse) (NDN):

cttcctctccaggaatccgcggagggagcgcaggctcgaagagctcctggacgcagaggc
cctgcccttgccagacggcgcagacatg (Seq ID No: 207)

Homo sapiens NADH dehydrogenase (ubiquinone) 1 beta subcomplex, 5, 16kDa (NDUFB5): ccttcttcctcctgcccgtagtagccatg (Seq ID No: 208)

Homo sapiens NADH dehydrogenase (ubiquinone) Fe-S protein 4, 18kDa (NADH-coenzyme Q reductase) (NDUFS4): ccgtcctttcatcctggcgtttgcctgcagcaagatg (Seq ID No: 209)

Homo sapiens nuclear factor of kappa light polypeptide gene enhancer in B-cells 2 (p49/p100) (NFKB2):

tgccccttccccggccaagcccaactccggatctcgctctccaccggatctcacccgcca
cacccggacaggcggctggaggaggcgggcgtctaaaattctgggaagcagaacctggcc
ggagccactagacagagccgggcctagcccagagacatg (Seq ID No: 210)

Homo sapiens non-metastatic cells 2, protein (NM23B) expressed in (NME2):

gcccctcctccgccgccggctcccgggtgtggtggtcgcaccagctctctgctctcccag
cgcagcgccgccgcccggcccctccagcttcccggaccatg (Seq ID No: 211)

Homo sapiens nucleophosmin (nucleolar phosphoprotein B23, numatrin) (NPM1):

gcgtcctttccctggtgtgattccgtcctgcgcggttgttctctggagcagcgttctttt
atctccgtccgccttctctcctacctaagtgcgtgccgccacccgatg
(Seq ID No: 212)

Homo sapiens 5'-nucleotidase, ecto (CD73) (NT5E):

cattccttttgtagaaaaacccgtgcctcgaatgaggcgagactcagagaggacccaggc
gcggggcggacccctccaattccttcctcgcgcccccgaaagagcggcgcaccagcagcc
gaactgccggcgcccaggctccctggtccggccgggatgcggccggtacccgctccccgc
cgggaacaacctctccactcttcctgcagggagctggtgccagccgacagccgcgccagg
gccgctccgggtaccagggtcggatcgggtgacgtcgcgaacttgcgcctggccgccaag
ccggcctccaggctgaagaaggacccgccccggccttgacccgggccccgcccctccagc
cggggcaccgagccccggccctagctgctcgcccctactcgccggcactcgcccggctcg
cccgctttcgcacccagttcacgcgccacagctatg (Seq ID No: 213)

Homo sapiens phosphatidylethanolamine binding protein 1 (PEBP1) :

gcgtcttcccgagccagtgtgctgagctctccgcgtcgcctctgtcgcccgcgcctggcc
taccgcggcactcccggctgcacgctctgcttggcctcgccatg
(Seq ID No: 214)

Homo sapiens poly(A) binding protein, cytoplasmic 1 (PABPC1):

gcttccccttctccccggcggttagtgctgagagtgcggagtgtgtgctccgggctcgga
acacacatttattattaaaaaatccaaaaaaaatctaaaaaaatcttttaaaaaacccca
aaaaaatttacaaaaaatccgcgtctcccccgccggagacttttattttttttcttcctc
ttttataaaataacccggtgaagcagccgagaccgacccgcccgcccgcggccccgcagc
agctccaagaaggaaccaagagaccgaggccttcccgctgcccggacccgacaccgccac
cctcgctccccgccggcagccggcagccagcggcagtggatcgaccccgttctgcggccg
ttgagtagttttcaattccggttgatttttgtccctctgcgcttgctccccgctcccctc
cccccggctccggcccccagccccggcactcgctctcctcctctcacggaaaggtcgcgg
cctgtggccctgcgggcagccgtgccgagatg (Seq ID No: 215)

Homo sapiens proprotein convertase subtilisin/kexin type 2 (PCSK2):

cgctctttctctccggtacacacagctccccacattcgcacccctgcccgcgcgccgggc
cgcctgactgcacggcttcccctccagccagatgctggagaacacacactgattcgctgc
tttccaagaccctgttcagtctctttctctatacaaagatttttttaaaaactatatata
agaattctttatttgcaccctccctccgagtcccctgctccgccagcctgcgcgcctcct
agcaccacttttcactcccaaagaaggatg (Seq ID No: 216)

Homo sapiens phosphoqluconate dehydrogenase (PGD):

gggtctttccctcactcgtcctccgcgcgtcgccgctcttcggttctgctctgtccgccg
ccatg (Seq ID No: 217)

Homo sapiens phosphoglucomutase 1 (PGM1):

cgctcccctttcccctcccgccggacctgccaggaggtgggctggcgcggagggagggcc
ctgtcccctgtccctttaaggaggagggccaaacgccggcctagagtgcggcgtagcccc
cacccgccgtgccctcaccccagagcagctgcagcctcagccggccgcccctccgccagc
caagtccgccgctctgacccccggcagcaagtcgccaccatg (Seq ID No: 218)

Homo sapiens solute carrier family 25 (mitochondrial carrier; phosphate carrier), member 3 (SLC25A3):

cggcctctgtgagccgcaacctttccaagggagtggttgtgtgatcgccatcttagggag
tgagtgtggccgggccttctcctgtggcgggtgtggggagcggagcccagagctcctgtg
gggccgctgctttggcggtgggcccagccgggagcagcctctttcgaaggccgccgtgac
ctcttcaagggcgtggagacgggaaggaaaaggccccggttggggttccagggcgccggt
aacgttaaccggcgccttgcctgtcctctaaccgtcgctccctcctcccctagaaagatg
(Seq ID No: 219)

Homo sapiens pim-1 oncogene (PIM1):

cctcccctttactcctggctgcggggcgagccgggcgtctgctgcagcggccgcggtggc
tgaggaggcccgagaggagtcggtggcagcggcggcggcgggaccggcagcagcagcagc
agcagcagcagcagcaaccactagcctcctgccccgcggcgctgccgcacgagccccacg
agccgctcaccccgccgttctcagcgctgcccgaccccgctggcgcgccctcccgccgcc

agtcccggcagcgccctcagttgtcctccgactcgccctcggccttccgcgccagccgca
gccacagccgcaacgccacccgcagccacagccacagccacagccccaggcatagccttc
ggcacagccccggctccggctcctgcggcagctcctctgggcaccgtccctgcgccgaca
tcctggaggttgggatg (Seq ID No: 220)

Homo sapiens pyruvate kinase, muscle (PKM2):

ggatctcttcgtctttgcagcgtagcccgagtcggtcagcgccggaggtgagcggtgcag
gaggctacgccatcagtccccaccaagggccagtcgcccggctagtgcggaatcccggcg
cgccggccggccccgggcacgcaggcagggcggcgcaggatccagggcgtctgggatgca
gtggagctcagagagaggagaacggctcctcacgcctggggcctgctcttcagaagtccc
cagcgccgttccttccagatcaggacctcagcagccatg (Seq ID No: 221)

Homo sapiens pleiomorphic adenoma gene-like 1 (PLAGL1):

cggcctcctcggcgcagccatcctcttggctgccgcgggcggcaaagcccacggcatctg
ccatttgtcattcagcccgtcggtaccgccccgagccttgatttagacacggctggggcg
tgctctggcctcactctccgggcgggtgctggacggacggacggacggggcagccgtgct
cacagctcagcagcgcggggccttggcgcgcggggcgcttccccgggtcgccgtcatggc
cgcggaggtggcacgcccgagcggcctcgcctgagctccgggggtcgtcgccccgcaggg
attgctgtcacgtctaatgtggctgctgcctcgtgtcacatctgaaactcatctgtacct
cacttagaaagtggttctgattagacaagacttttcgttgcagtcgacagaaacctaatg
ggaccattgaagaattccaaacaggtatttgcataggaatcagaggagttaatcttgtct
cttctcacaggtttgaatcttcagacaaacttctgggaggactcggtccctgcctcgcag
cagatgttccctgtcactcagtaggcatatg (Seq ID No: 222)

Homo sapiens phospholipase D2 (PLD2):
tgctctcttggctccggaacccccgcgggcgctggctccgtctgccagggatg
(Seq ID No: 223)

Homo sapiens proteolipid protein 2 (colonic epithelium-enriched) (PLP2):

ccccccttcccggccagacggcgggcaagacagctgggtgtacagcgtcctcgaaaccacg
agcaagtgagcagatcctccgaggcaccagggactccagcccatgccatg
(Seq ID No: 224)

Homo sapiens pinin, desmosome associated protein (PNN):

cagtcctttcgcgcctcggcggcgcggcatagcccggctcggcctgtaaagcagtctcaa gcctgccgcagggagaagatg (Seq ID No: 225)

Homo sapiens phosphoribosyl pyrophosphate amidotransferase (PPAT): ggtccttccacgtgctttcggcggcgacatg (Seq ID No: 226)

Homo sapiens protein phosphatase 1, catalytic subunit, gamma isozyme (PPP1CC):

tgttcttctcgtggttccagtggggagagaaggaggaagtagggagcggggtggcagggg ggggacccgccgcggctgctgccaccgccgccaccaccgcctctgctcgtggcgtgggaa aggaggtgtgagtcccgggcgcgagccggcggcggcgccgctgcgggagggtcggcggtg ggaaggcgatg (Seq ID No: 227)

Homo sapiens protein phosphatase 1, regulatory subunit 8 (PPP1R8):

cggtcttccagtttcccggcgtgcttagggcgcgccaaatgggagggggagacgcaagat g (Seq ID No: 228)

Homo sapiens protein phosphatase 6, catalytic subunit (PPP6C):

cggcctccgccgctgccgccgccgctgctacagccgccgccgccgctgttgccgcggctt gttattcttaaaatg (Seq ID No: 229)

Homo sapiens protein kinase C substrate 80K-H (PRKCSH):

ctttctttctgcagcaggaaccgcggctgctggacaagaggggtgcggtggatactgacc tttgctccggcctcgtcgtgaagacacagcgcatctccccgctgtaggcttcctcccaca gaacccgtttcgggcctcagagcgtctggtgagatg (Seq ID No: 230)

Homo sapiens mitogen-activated protein kinase 6 (MAPK6):

cgccccctcttcctcgccctctctcgcgggtcggggttacatggcggcgactgcggcaaa gcgagagcctcggagacgccgctgccgccagcacagccggagacctgagccgacactggg ggcagtccgcgagccccgcactctctcgatgagtcggagaagtcccgttgtatcagagta agatggacggtagctttgattgtgattgtggtgagctggagccacctgatcactaacaaa agacatcttctgttaaccaacagccgccagggcttcctgttgaaataaatatatagcaac aaaggaaaaaaagaagcaaaacggaaatagtgcttaccagcaccttagaatgatgctgct caggaccagtccaacactgaatgtatctgcactgtgaggagaatgttcatagaagcctgt tgtgtgcatatttattcacattttgttaaatgttaaatcgtttagcacggtaatctgag tgcacagtatgtcatttcattccgtttgagtttcttgttttcgttaaatgtctgcagagt tgctgcccctttcttgaactatgagtactgcaatctttttaattctcaatatgaatagag ctttttgagctttaaatctaagggaactcgacaggcctgtttggcatatgcaatgaaca tcaagaaaccatcttgctgtggaagcataattatttttcttctccctttttgaaagatct ttccttttgatgccagtttcttccttgtttacacaagttcaatttgaaaggaaaaggca atagtaagggtttcaaaatg (Seq ID No: 231)

Homo sapiens phosphoribosyl pyrophosphate synthetase 2 (PRPS2):

cctccccttccctacatctagccgccgcgctttcccgctcccgcagcagcagcctcccgc
gtcgctgtcgctgttgcctccgccacctcctccgccgccgcgcgcccctcggagttccgc
gccccaccatg (Seq ID No: 232)

Homo sapiens phosphoribosyl pyrophosphate synthetase-associa ted protein 1 (PRPSAP1):

ttgcctctggctctgaggcggcggcgccgggcgctgcgaaggctcggccgctgtagtcag
tggtgtggggtgcgcaagggcacggacctcggagctctccccgcttgcgccgagtttctc
agcgccttccccacccaaaccggggtctcgcagtcggaagcactcagagtgcagccccgc
gcggggccggtcgtaaccgcgccgcgggccggacgatg (Seq ID No: 233)

Homo sapiens proteasome (prosome, macropain) subunit, beta type, 5 (PSMB5):
agttctttctgcccacactagacatg (Seq ID No: 234)

Homo sapiens proteasome (prosome, macropain) 26S subunit, non-ATPase, 13 (PSMD13):
tgttcttctgtgccgggggtcttcctgctgtcatg (Seq ID No: 235)

Homo sapiens protein tyrosine phosphatase, receptor type, N (PTPRN):

cagcccctctggcaggctcccgccagcgtcgctgcggctccggcccgggagcgagcgccc
ggagctcggaaagatg (Seq ID No: 236)

Homo sapiens RAB3A, member RAS oncogene family (RAB3A):

ctccctttgcaggacgtcacggaggactgcaggggcctgagccgctgctgccgccgccgc
cgcgcagccccacatcaacgcaccggggtcctgtcaccgccaccgccaaaaaagtcaccg
ccgctagggtcgccgttgcatcggtgcagggcaagatg (Seq ID No: 237)

Homo sapiens RNA binding motif, single stranded interacting protein 2 (RBMS2):
ctctctctctctctctctcgctcgttccctaacattaaagagaaaatg
(Seq ID No: 238)

Homo sapiens reticulocalbin 1, EF-hand calcium binding domai n (RCN1):

gcgcccctctgctccggctcggggcgggcactggcggagggactggccagtcccctcctc
cgcgccggccccaaccctgtcgctgccgccgcgctccgagtccccattcccgagctgccg
ctgttgtcgctcgctcagcgtctccctctcggccgccctctcctcgggacgatg
(Seq ID No: 239)

Homo sapiens radixin (RDX):

ccgccttttcccgcggaggcgccgagcggccatattgcggagctgtctgcggtggcggcg
gcgcctctcgtctcccgcggcccagcgctcgcaccaccgcttctccctccctgtcgcagc
cgcgccgccgcgcagcgccccagccacacgccggcgggcagaagccgcccgctctccgga
aagtgataacagaattcattgaagtggagaattttaaagaaggtaacaaaaagagaaag
aaaatg (Seq ID No: 240)

Homo sapiens replication factor C (activator 1) 1, 145kDa (RFC1):

tcgccttcttgcacttcgcgggagaagttgttggcgcgaatggatcctgagcctcgataa
cagattcctcaaccggcccacccgccagccagccagcgccttcatcctggggctgcgatg
(Seq ID No: 241)

Homo sapiens ring finger protein 4 (RNF4):

gcatctttctcgaggagctctcctgggcggctgaagaaggagcttcttctccggagtgcg
ccggcggtggcgcctgcggacctaactagctccaggttaggccgagctttgcgggaaagc
agcggacttgaaaatactggaaatctgtccggatccaaattattttgcaagccagatgag
taaccagagggcatgaaaggttgagaacatttgacttccctgcaaaccttggtatagatc
acttccttttctgtaggaaaggaaaggcaccaaagagcacaatg
(Seq ID No: 242)

Homo sapiens ribophorin I (RPN1): tgctcttcccggtcatg (Seq ID No: 243)

Homo sapiens ribosomal protein S27a (RPS27A):
cgttcttccttttcgatccgccatctgcggtggagccgccaccaaaatg
(Seq ID No: 244)

Homo sapiens secreted and transmembrane 1 (SECTM1):

cttcctttagcgtgaaccgcgggtgcggtgcctcccgtgaaaataataaattcaccgtca
cgcttgttgtgaacgcgggtggttcccgaaacttggaggcttcccgtaaacccagctcct
tcctcatctgggaggtgggtcccgcgcgggtccgccgcctcctccctggcccctccctct
cgtgtctttcattttcctggggctccggggcgcggagaagctgcatcccagaggagcgcg
tccaggagcggacccgggagtgtttcaagagccagtgacaaggaccaggggcccaagtcc
caccagccatg (Seq ID No: 245)

Homo sapiens small glutamine-rich tetratricopeptide repeat (TPR)-containing, alpha (SGTA):

ctttcttttgcgcaggcgtcgcgccctggggccggggccgggcggcaccgcggtgcgcaa
gcgcaaccgtcggtgggtcggggatcggtcgcctgagaggtatcacctcttctgggctca
agatg (Seq ID No: 246)

Homo sapiens SH3 domain binding glutamic acid-rich protein l ike (SH3BGRL):

agttctccttccaccttcccccacccttctctgccaaccgctgtttcagcccctagctgg
attccagccattgctgcagctgctccacagcccttttcaggacccaaacaaccgcagccg
ctgttcccaggatg (Seq ID No: 247)

Homo sapiens solute carrier family 1 (glutamate/neutral amino acid transporter), member 4 (SLC1A4):

cgccctcctacttccccgtctgcgtccgcgttcgcggctcccgtttgcatcatccccgtc
tgcgtccgcgttcgcggctcccgtttgcatcatctccagccggcggctgctccagggagg
ctgggcgcgatcctctccgcccgcggctccaacccgcactctgcgcctctcctcgccttt
ctcgcacctgctcctgcgccaggcccggagacccccggggcggcttcccagaacctgcgg
agcacaactggccgaccgacccattcattgggaaccccgtcttttgccagagcccacgtc
ccctgccacctctagctcggagcggcgtgtagcgccatg (Seq ID No: 248)

Homo sapiens small nuclear RNA activating complex, polypepti de 2, 45kDa (SNAPC2): ctgcctctttctgagcggcatg

(Seq ID No: 249)

Homo sapiens sorting nexin 1 (SNX1):
ctatctctcgataaagttgttgttgcggcttccgccgcgggtggaagaagatg
(Seq ID No: 250)

Homo sapiens signal recognition article 54kDa (SRP54):

ctatctctcatctttccgctcttagctgggagtgctccgcctagtcacttttcttaaggt
ggctcgtcgaggcctgacttcttccccgaaatcacgtccctagacagcctcctattttac
cactaactttactcctgcagttattcagcggtaggaaactgaaaccaaaaaccagtgtaa
gcaagtaaacatctaaactgtttcaggagccgcgtagaaggaacgcggcggtgtgccccg
gaagcggaagtagattctcctatagaaaggctggactacgcggagtggtgacgtttcctc
attgggcggaaggttcgctggcactccgttggtcttccagctggtgggagttgacgacgt
ggtgctgggcgttgggaccctactttatctagttcgggaagttgggttgtggggtcatac
ctgtctgtctgctcccagctttcttgggtttcttccgacggcgtggggcctcgctaagga
attcccggcccctcagggccacggctttagcggtgtcttttgcgagttcttcgtaagtac
atcttaaagctgtcaagatg (Seq ID No: 251)

Homo sapiens signal sequence receptor, beta (translocon-associated protein beta) (SSR2):

cggtctttcggatgctgacgctctcttcctgtctttgtggctccggaaaggcgtttggga
tgccaacgatg (Seq ID No: 252)

Homo sapiens signal transducer and activator of transcriptio n 6, interleukin-4 induced (STAT6):

ttttcttttggtggtggtggtggaagggggaggtgctagcagggccagccttgaactc
gctggacagagctacagacctatggggcctggaagtgcccgctgagaaagggagaagaca
gcagaggggttgccgaggcaacctccaagtcccagatcatg (Seq ID No: 253)

Homo sapiens suppressor of Ty 4 homolog 1 (S. cerevisiae) (SUPT4H1): tgttcttcccatcggcgaagatg (Seq ID No: 254)

Homo sapiens transcription factor 7 (T-cell specific, HMG-box) (TCF7):

ggtccttcccctaaaacttggcactgccgatactcccagcccgttccttcccaagtcagg
aacttgcaggggaccccttggcaattctttttctctcaagagcagacagccttcagtccc
agccgctgccagggctggtgtgtctgacccagctgtggtttttccaggcctgaaggcccc
ggagtgcaccagcggcatg (Seq ID No: 255)

Homo sapiens TEA domain family member 4 (TEAD4):

cagtctcctccccgaggtgccggtggccccgccgccactccctccggctccctccctccc
gccgcggcgcgcatctcattccagccctcattccgcgcattccagcgtcctcctcgcaca
ctcgaggccagggggcgggagggccgcagctccggcgccgccgcgtcccgccaggtgaga
ggcgcccgcgcccgccgcacccgccggcgccctcacgggccgcgcgccccacgccgccgc
agccgaccgctcgcgccgcgtgctcggctgctcttttctttccgccgcccgcgttcccgc
cttggacctctgcgctccgacgcgctccgtcccgacctctggcttccctccgcgctccgg
cgctgctcgctgcccctctcccgcttccctcctgtccgccccgcgctcccctcctcgctc
ccggttgactcactcctccaggaatagggatccccgtgttttcccgtcagtcccattctg
ggaaaactcctccctccgcgcgctccgctccgctccgctgggcgcaccggggccggtcgg
cgcggggtgggcttggccccgcggccccgccttcactgcgccgcccgtcggccccggccg
gagcccggctctgcgcgctgacgccctgtcgtccccgcagaacgatcgccgcggccggaa
gagttggcgctcggggcggactccttggaactggcttagcgcacccatcccaccttcccg
caccctgggaccggtcggaacgagctgattgcccgctacatcaagctccggacagggaag
acccgcaccaggaagcaggtctccagccacatccaggtgctggctcgtcgcaaagctcgc
gagatccaggccaagctaaaggaccaggcagctaaggacaaggccctgcagagcatg
(Seq ID No: 256)

Homo sapiens G protein-coupled receptor 137B (GPR137B):

ttttctttcctccagtctcggggctgcaggctgagcgcgatgcgcggagacccccgcggg
ggcggcggcggccgtgagccccgatg (Seq ID No: 257)

Homo sapiens tumor protein, translationally-controlled 1 (TPT1):

cggcctttccgcccgctccccccctcccccgagcgccgctccggctgcaccgcgctcgc
tccgagtttcaggctcgtgctaagctagcgccgtcgtcgtctcccttcagtcgccatcat
g (Seq ID No: 258)

Homo sapiens ubiquitin A-52 residue ribosomal protein fusion product 1 (UBA52):
ctatcttctttttcttcagcgaggcggccgagctgacgcaaacatg
(Seq ID No: 259)

Homo sapiens ubiquinol-cytochrome c reductase core protein I I (UQCRC2):

cggcctccgccaccatcttgctttcctttaatccggcagtgaccgtgtgtcagaacaatc
ttgaatcatg (Seq ID No: 260)

Homo sapiens ubiquitin specific peptidase 1 (USP1):

ctgcctttcgtgtctctgcagcgtggagactggaaccggcaatttcaaaggacgccacgt
tcaatcgcagcgctggcgcgggcggaggctaaaacacgggggtcctgagactgaggaaaa
cgcgccaagttcccctcggtggcggagtgctaaagaccctagcggttcaggcgttcggcg
agcggggccgctgcttgttgcgctcctggctctcccggggcgggcgcagatgggcgccgc
tcccgggatgtagttggtgttggtgcaagacgggagcgagcggcggtcggggttcccgct
cttgggagcggatggtcactcccccgcggggagggcgagccgaccagattttcctggggc
cggggacccggcgggctcggggcagggactcacctgtcgcacccacactcattcgggttg
gacttgccggcgtcaccgccgcggacttcgctttgggccatgaccagatataattggtga
ttacaactttcctctataaattaactcttgacactccttgggatttgaagaaaaaaatg
(Seq ID No: 261)

Homo sapiens voltage-dependent anion channel 2 (VDAC2):

gtgtctccttcacttcgccctccagctgctggagctgcagcccgaccgcgagcgtgccaa
gcggcttcagcagctagcggagcggtggcggcggccccctcaggacaccaccagattcc
cctcttcccgcggcctcgccatg (Seq ID No: 262)

Homo sapiens vimentin (VIM):

gcctcttctccgggagccagtccgcgccaccgccgccgcccaggccatcgccaccctccg
cagccatg (Seq ID No: 263)

Homo sapiens very low density lipoprotein receptor (VLDLR):

ccccctccccgctgctcaccccgctctccggccgccgccggtgcgggtgctccgctaccg
gctcctctccgttctgtgctctcttctgctctcggctccccacccctctcccttccctc
ctctccccttgcctcccctcctctgcagcgcctgcattattttctgcccgcaggctcggc
ttgcactgctgctgcagcccggggaggtggctgggtgggtggggaggagactgtgcaagt
tgtaggggagggggtgccctcttcttccccgctcccttcccccgccaactccttcccctc
cttctccccctttcccctccccgcccccaccttcttcctcctttcggaaggactggtaac
ttgtcgtgcggagcgaacggcggcggcggcggcggcggcggcaccatccaggcgggcacc
atg (Seq ID No: 264)

Homo sapiens wingless-type MMTV integration site family, mem ber 10B (WNT10B):

agtcctttgctcgccggcttgctagctctctcgatcactccctcccttcctccctccctt
cctcccggcggccgcggcggcgctggggaagcggtgaagaggagtggcccggccctggaa
gaatgcggctctgacaaggggacagaacccagcgcagtctccccacggtttaagcagcac
tagtgaagcccaggcaacccaaccgtgcctgtctcggaccccgcacccaaaccactggag
gtcctgatcgatctgcccaccggagcctccgggcttcgacatg (Seq ID No: 265)

Homo sapiens CCHC-type zinc finger, nucleic acid binding pro tein (CNBP):

cagcctctaccttgcgagccgtcttccccaggcctgcgtccgagtctccgccgctgcggg
cccgctccgacgcggaagatctgactgcagccatg (Seq ID No: 266)

Homo sapiens zinc finger protein 43 (ZNF43):

gggcctttgtctctggctgcagttggagctctgcgtctcgtcttcgttcttctgtgtcct
ctgctgctagaggtccagcctctgtggctctgtgacctgcgggtattgggggatccacag
ctaagacgccaggacccccggaagcctagaaatg (Seq ID No: 267)

Homo sapiens zinc finger protein 74 (ZNF74):

cagtccttttgtgggagtccggtctgtccacttgccggtccctcagaccgtcggcggtct
ctgtccgcttcgggacctgtccgctggtcgctccgcgtccgatggctcctggccgcggaa
ccttaggcctggccctggtctccgagcgcgggttcgccgggaggagcgtgtggcggggt
gtgccggggcgtgagtgcgccgagcatggggctgagcctggtgtggggagtgggtatctg
cggagccggcctgaaccccacctcagccgggcgcggggaggggctccgtgcgtgtgatc
gtgcagctgtgagcgcgtggccgccccgcggggctccgctgcaggcccctcagccccagg
agcagtactcgctcttcagggcctgccctggatcctggaggctacacagctgcccactcc
tcctggggaggctgccgtggaggccatg (Seq ID No: 268)

Homo sapiens zinc finger protein 85 (ZNF85):

gggcctttgtctctcgctgcagcctgagctctaggtcttgttttccctgctttgtgtttt
ctgctcgtggacgcccagcctctgtggccctgtggcctgcaggtattggagatccacag
ctaagacgccgggacccccctggaagcctagaaatg (Seq ID No: 269)

Homo sapiens zinc finger protein 91 (ZNF91):

gggcctttgtctctcgctgccgccggagtttccaggtctcgacttcactgctctgtgtcc
tctgctccaggaggcccagcctgtgtggccctgtgacctgcaggtattggagagccacag
ctaagatg (Seq ID No: 270)

Homo sapiens zinc finger protein 141 (ZNF141):

gggtctttgcgtctggctactaccagaccgcgggttaggggcttcatctctctgcgttct
cagttgtgggaggccttggtgattcggccacagcctcagcctccgtcgctctgtgacctg
cgggtattggatgattggtagctaagactcccgaatacttcagaagtggggaaatg
(Seq ID No: 271)

Homo sapiens zinc finger protein 205 (ZNF205):
tgttctttctagctctgaaatagaaaatg (Seq ID No: 272)

Homo sapiens transmembrane protein 187 (TMEM187):

ctccctttttcggagatttgaatttcccccagcgaggcgagtgaggcgaaatacccgtatg
gtgatagctggccttttcgcgccaatactgaaaaaggcagaacgttcctccgctggcgcc
agccaatcagcaggactcctgccttccttcggggcaaggtcgcagcatctgcctcggaaa
tcacgaaatcacggggcttctttctgctggctcagccgggaggcccagagtgttctgcag
aggctgcgtattgaaggctgctctctgaagctccctgccccaggtcacgccgccggttcc
agatg (Seq ID No: 273)

Homo sapiens histone cluster 2, H2be (HIST2H2BE):
acttcttttcttggctaagccgcgtttgtactgtgtcttaccatg
(Seq ID No: 274)

Homo sapiens solute carrier family 25 (mitochondrial carrier; oxoglutarate carrier), member 11 (SLC25A11):

ccgcctttgcgctgcgcgcctgcgcccgcgccggcttccagcgggtgtcggacctgagag
ctggaggggcgtgcgcgcgccctcgctctgttgcgcgcgcggtgtcaccttgggcgcgag
cggggccgcgcgcgcacgggacccggagccgagggccattgagtggcgatg
(Seq ID No: 275)

Homo sapiens tyrosylprotein sulfotransferase 2 (TPST2):

cctccccttccccggctggggcggctggagagccgggagtcgctgggtgcgtggggctg
cctcgccgcgtctcgccacgggctctgccagcagacagccttggcacacaggcacaaggg
ctggagcccagagatgagagtgcccaaggagatgtgagcctggcgggctgcccgctaac
ctgtcgctgaagccccagaagcgggccctcaggccaggcctaccctgcctccggcccagc
atg (Seq ID No: 276)

Homo sapiens sorbin and SH3 domain containing 2 (SORBS2):

aagcctcttttatacatctcttcagggaagagagaagcaatgggcatgttagtatacaat
gatcacagccacgcaggcctgcaagctgccttttggacaggctgttgactgccgttccaa
ttagctgattggagaatgtggaatgcagagtgataatgctgcatatctgctatcaggcag
cagcaaaggtttttgtcttgggaaggcaagctttccctgcaatattatctcagcagctcc
ctagctgcttaccctgaaaacgagggatccaaacggagggtgttgcactctgctaacgct
ggtcctgtgcgtggctgtggcatatgagcggcaggtctgaaaaagcaggtgtgtgctggg
acgggcactggactggaacgcaggcggacgctctcgggtttacctgcttcctgttaacag
attgtgggctcccagggcatatgtctgcacgctgaggccgaggcggagaaggggcttcct
gagcgtcccagtacactgacagagacacttggattggacttaatcttaaacctctggagt
tcaagaccttttaaaaagggctaaataaacaatctctacatgtaaaaggccactgactcc
tacttcctctgtatagagcaactgttgaactcagctgcctgtaggaaaactgaagacttt
aataacaaactctccaaggtgaaaatg (Seq ID No: 277)

Homo sapiens G protein-coupled receptor 65 (GPR65):

gtttctcttcttgacttgatgcaggcacagatttatcaagctcctcagtcaacaaacaca
tcaccggaagaaatatggaaggaaaggaattttaaaaggaaataccaatctctgtgcaaa
caaagccttgtatattcatgtttgcaccaatctactgtgagatttatgaagaaaaacaaa
ttgcggacaactctctatgtacacttacaaatgcctcagttgatgcttgtggctgtttg
tcagcgttctgtgataatgaacacatggacttctgtttattaaattcagttgacccttt
agccaattgccaggagcctggattttacttccaactgctgatatctgtgtaaaaattga
tctacatccaccctttaaaagcattgatgaattaattagaactttagacaacaaagaaaa
attgaaaaagaattctcagtaaaagcgaattcgatgttcaaaacaaactacaaagagaca
agacttctctgtttactttctaagaactaatataattgctaccttaaaaaggaaaaaatg
(Seq ID No: 278)

Homo sapiens nipsnap homolog 1 (C. elegans) (NIPSNAP1): gggccttcctgcaacctttgcggctccaacatg (Seq ID No: 279)

Homo sapiens inhibitor of kappa light polypeptide gene enhan cer in B-cells, kinase complex-associated protein (IKBKAP):

gcttctttgcagcgcttcagcgttttcccctggagggcgcctccatccttggaggcctag
tgccgtcggagagagagcgggagccgcggacagagacgcgtgcgcaattcggagccgact
ctgggtgcggactgtgggagctgactctgggtagccggctgcgcgtggctggggaggcga

ggccggacgcacctctgtttgggggtcctcagagattaatgattcatcaaggatagttg
tacttgtctcgtgggaatcacttcatcatg (Seq ID No: 280)

Homo sapiens COP9 constitutive photomorphogenic homolog subu nit 3 (Arabidopsis) (COPS3): ctgccttcgccgctcgggccgcccgggggaaaacatg (Seq ID No: 281)

Homo sapiens pirin (iron-binding nuclear protein) (PIR):

ccgcctcctctaggccgccggccgcgaagcgctgagtcacggtgaggctactggacccac
actctcttaacctgccctccctgcactcgctcccggcggctcttcgcgtcacccccgccg
ctaaggctccaggtgccgctaccgcagcgtgagtacctggggctcctgcaggggtccact
agccctccatcctctacagctcagcatcagaacactctcttttttagactccgatatg
(Seq ID No: 282)

Homo sapiens THO complex 5 (THOC5):

ccttccttacttccggttctctatggtgcgcgggcaagctttgctccgcctccggcagtg
gcttactcccggtgccaggttcttggagctgtgaggaggaacaaccatg
(Seq ID No: 283)

Homo sapiens RuvB-like 1 (E. coli) (RUVBL1):

gggcctttgcaaaattgccctagtaacggccgcatggtaactcaggcgccgggcgcactg
tcctagctgctggttttccacgctggttttagctcccggcgtctgcaaaatg
(Seq ID No: 284)

Homo sapiens Kruppel-like factor 7 (ubiquitous) (KLF7):

tttccttttagttgactgaaacaaaacaaaacaaaagggccactggatgtctgccttct
tggggggtgagccagacagactgacaaacaaacagccccaactgtgttcgggggagggtt
tcgcctcccgttttgcccggcagcagcagcatg (Seq ID No: 285)

Homo sapiens USO1 vesicle docking protein homolog (yeast) (USO1):

gctccccttttgccttcaaccttcgagccgccacgtaatgccacgtccccgcgcatgcgc
atcttggccgctgctggcggctgtttccgggcttagagggctggagtggccgccgagttg
gaggcggtggtggcagcagtaggagtgtgtagagtgcgggattgggggccaggccctgcg
gagggcgggggaagttgtcttcttttttttccggaggggccggtaaacctggtggctgaa
cggcaagatg (Seq ID No: 286)

Homo sapiens unc-5 homolog C (C. elegans) (UNC5C):

ccccctttttggcccctgcctttggagaaagtggagtgtggcgcttggttgtcgttatttc
ttcggactgcttcgcggtgcacggattcagcttctgcccagtggggctttcagctgtttg
cgcgtctctctgtccccctcccctccccccggcacacctctgtctacgatg
(Seq ID No: 287)

Homo sapiens RNA terminal phosphate cyclase domain 1 (RTCD1):

gcttcttccgctttctcgtcaggctcctgcgccccaggcatgaaccaaggtttctgaact
actgggcgggagccaacgtctcttctttctcccgctctggcggaggctttgtcgctgcgg
gctgggccccagggtgtcccccatg (Seq ID No: 288)

Homo sapiens eukaryotic translation initiation factor 3, sub unit A (EIF3A):

ggctccttcctttccgtctctggccggctgggcgcgggcgactgctggcgaggcgcgtgg
gaccttacgctggttccccttcgtctcctctcccggcccgggccactagagagttcgctg
acgccgggtgagctgagcctgccgccaagatg (Seq ID No: 289)

Homo sapiens eukaryotic translation initiation factor 3, sub unit D (EIF3D):

gtttcctcttttcctggtttctcaagagtgctgctgctaacgcggtccccggcacgcacc
atctgttgccatcccggccggccgaggccattgcagattttggaagatg
(Seq ID No: 290)

Homo sapiens eukaryotic translation initiation factor 3, sub unit F (EIF3F): ccgcctccttctttctcgacaagatg (Seq ID No: 291)

Homo sapiens eukaryotic translation initiation factor 3, sub unit G (EIF3G):
cgctctctggccgggcttgggctgcgtggagaatactttttgcgatg
(Seq ID No: 292)

Homo sapiens eukaryotic translation initiation factor 3, sub unit H (EIF3H): gtttctctttcttcctgtctgcttggaaagatg (Seq ID No: 293)

Homo sapiens eukaryotic translation initiation factor 3, sub unit I (EIF3I):
aaaccttttccggtcttactcacgttgcggccttcctcgcgtcacagccgggatg
(Seq ID No: 294)

Homo sapiens eukaryotic translation initiation factor 3, sub unit J (EIF3J): ctccctctcacacacgctcacacccggctcgagatg (Seq ID No: 295)

Homo sapiens poly(A) binding protein, cytoplasmic 4 (inducible form) (PABPC4):

ccgcctctctccgccccgggtcgctgccgcctccgccgctttcgggcttcgcagcctgag
gaaaaaaagagaaaaagataaaaaaatctgaaaacgcttcaaaatcctgaaaaaaaaa
aggaaaagaaaaaacgaatcctcggagaacccgcggggaagtcactttcgtacgcttccg
gcctgccccgcgcccgccgccgcagcgcttggcgtccgtcggtctccgtccgtcggtccg
ggggtgagccgcccgcccggcccgccgtgccctcccccgctcggccccgagccccgcg
ccccgcgcctgccccggcgcaccacgtgtccgtgctgcccttcgccgcccgcccggggct
cgccgagtcggcgcccacaaagatttggtttccctctgccccggcggttgtaatcttaaa
ccgccggagcccgaggcctatatttatagagaaacgcgtgtccccgaggccgccgtgggc
agcgtccggtcgcctcttaaaggatttttacccttcggaaggggattccccgtttaattt
ttttcctactttgatttttttgaaatttggagcttcgcaccaggaccgcggagaagtgcaa
agtcgcggggagggccgtattgtgcggagagccttttgtctgcggtgctgcggccgtggg
agccggcccccgcctcccgtttccgtcccgtctccaagcccgccgactccagctcgtcct
cgccgcgccggtgccacctgtgagccgcggcgcgggcccgggctccgaaggcgcccctttt
gtcctgcggcgggcccgataagaagtcctcctggcggggctcggggtggtgggggcggg
gagatg (Seq ID No: 296)

Homo sapiens receptor-interacting serine-threonine kinase 2 (RIPK2):

agctctttcgcggcgctacggcgttggcaccagtctctagaaaagaagtcagctctggtt
cggagaagcagcggctggcgtgggccatccggggaatgggcgccctcgtgacctagtgtt
gcggggcaaaaagggtcttgccggcctcgctcgtgcaggggcgtatctgggcgcctgagc
gcggcgtgggagccttgggagccgccgcagcaggggcacacccggaaccggcctgagcg
cccgggaccatg (Seq ID No: 297)

Homo sapiens neuropilin 1 (NRP1):

ctttcttttctccaagacgggctgaggattgtacagctctaggcggagttggggctcttc
ggatcgcttagattctcctctttgctgcatttcccccacgtcctcgttctcccgcgtct
gcctgcggacccggagaagggagaatg (Seq ID No: 298)

Homo sapiens guanine monphosphate synthetase (GMPS):

tggtcttctctcccgcggcgctggggcccgcgctccgctgctgttgctccattcggcgct
tttctggcggctggctcctctccgctgccggctgctcctcgaccaggcctccttctcaac
ctcagcccgcggcgccgacccttccggcaccctcccgccccgtctcgtactgtcgccgtc
accgccgcggctccggccctggccccgatg (Seq ID No: 299)

Homo sapiens far upstream element (FUSE) binding protein 1 (FUBP1):

ttttctttctttcttagctgttagctgagaggaagtctctgaacaggcggcagcggctct
tatagtgcaaccatg (Seq ID No: 300)

Homo sapiens eukaryotic translation initiation factor 2B, su bunit 5 epsilon, 82kDa (EIF2B5):

gatccttttgtcccctactgcgtgcggtggcagcttccttgcggaagtggtgaccgtga
gagaagaagatg (Seq ID No: 301)

Homo sapiens eukaryotic translation initiation factor 2, sub unit 2 beta, 38kDa (EIF2S2):

gtttcctttcgctgatgcaagagcctagtgcggtggtgggagaggtatcggcaggggcag
cgctgccgccggggcctggggctgacccgtctgacttcccgtccgtgccgagcccactcg
agccgcagccatg (Seq ID No: 302)

Homo sapiens adaptor-related protein complex 1, sigma 2 subu nit (AP1S2):

cctcccctctccgcctaagcctgccctatgccagccgggtgtcctccccacagcaccacg
gcttctcttcctcagcacggcgacaggggcttccccttcgccgccgccgccgccgccggc
caagctccgccgcgcccgcggcccgcggccgccatg (Seq ID No: 303)

Homo sapiens suppression of tumorigenicity 13 (colon carcinoma) (Hsp70 interacting protein) (ST13):

cgccccccttctgcgcggtcacgccgagccagcgcctgggcctggaaccgggccgtagccc
ccccagtttcgcccaccacctccctaccatg (Seq ID No: 304)

Homo sapiens solute carrier family 7 (cationic amino acid transporter, y+ system), member 7 (SLC7A7):

ctccctttcttaaatgcttggggtgagagagaagagaggctagggtggggcatggaggac

acagagagagagagagtgctgtgtattccttccccgctactgtcctgtcctcagctaacttg ctctgggacagcttccccagggctacagatactgcactcagctgactgtcctttcttctg ggcccctggtcccagagcagagctgacaaaggagattcctgagagagcaccttcttatca cagaaagtgctgagccaagagctcctagctgcccctttgcagatgtgaagggccagtga accttggacccagatggttgcttaatactcctttccccctccctcactccttcctttgcg ggctgcctcacctcctccacccttcttgcttaaatccataggcatttgtctggccttccc ttttactgctggctgggaaggaggagcatcagaccacagatcctggaaggcacttctctc cctgactgctgctcacactgccgtgagaacctgcttatatccaggaccaaggaggcaatg ccaggaagctggtgaagggtttcctctcctccaccatg (Seq ID No: 305)

Homo sapiens paired box 2 (PAX2):

ctccctttctcctcaagtcctgaagttgagtttgagaggcgacacggcggcggcggccg cgctgctcccgctcctctgcctccccatg (Seq ID No: 306)

Homo sapiens 5-aminoimidazole-4-carboxamide ribonucleotide f ormyltransferase/IMP cyclohydrolase (ATIC):

agccctcctacctgcgcacgtggtgccgccgctgctgcctcccgctcgccctgaacccag tgcctgcagccatg (Seq ID No: 307)

Homo sapiens ATP synthase, H+ transporting, mitochondrial F1 complex, alpha subunit 1, cardiac muscle (ATP5A1):

ccttctttgcggctcggccatttgtcccagtcagtccggaggctgcggctgcagaagta ccgcctgcggagtaactgcaaagatg (Seq ID No: 308)

Homo sapiens cyclin G1 (CCNG1):

cggccccttcggctccgagctgaccctgatcagggccgagttgtctcggcggcgctgccg aggcctccacccaggacagtcccccctccccgggcctctctcctcttgcctacgagtcccc tctcctcgtaggcctctcggatctgatatcgtggggtgaggtgagcaggcccggggaggg tggttaccgctgaggagctgcagtctctgtcaagatg (Seq ID No: 309)

Homo sapiens cadherin 16, KSP-cadherin (CDH16):

agctctcttcttgcttggcagctggaccaagggagccagtcttgggcgctggagggcctg tcctgaccatg (Seq ID No: 310)

Homo sapiens cyclin-dependent kinase inhibitor 1B (p27, Kip1) (CDKN1B):

ttttcttcttcgtcagcctcccttccaccgccatattgggccactaaaaaaagggggctc gtcttttcggggtgtttttctcccctcccctgtccccgcttgctcacggctctgcgact ccgacgccggcaaggtttggagagcggctgggttcgcgggacccgcgggcttgcacccgc ccagactcggacgggctttgccaccctctccgcttgcctggtcccctctcctctccgccc tcccgctcgccagtccatttgatcagcggagactcggcggccgggccggggcttccccgc agcccctgcgcgctcctagagctcgggccgtggctcgtcggggtctgtgtcttttggctc cgagggcagtcgctgggcttccgagaggggttcggctgcgtaggggcgctttgttttgt tcggttttgtttttttgagagtgcgagagaggcggtcgtgcagacccgggagaaagatg (Seq ID No: 311)

Homo sapiens chimerin (chimaerin) 2 (CHN2):

tctcctcttcttcctttgtgtgtgcgcgagcggagttggggcggagggagaagggggagg tcgctctgtctgtccgtctcccgccgcctctgcccggtctactcgaagtgcggcgggaga ggcgggagcccaggagagggtgcgggagctggcggggcggctcggagctgccaggacgcc

ctggtcccagccgcgcacaggggagcgtggacggcagaggggctcggcgggagccgagat ccgcccgtcccggctgcccctcggcctccctctgctcccacctacccctgacacccata gaaaagcgtgcaaaggcgcggagcgggacggaaaccacaaataaatagcggcggcggcag cgcgtcatctggtggagcaggaagtgcaggcagagtccggaggctggtgctttctgcgcg tccccaggactttgccatgggctggggggccgcggaggctgcgagcggccgggcgagggca gcggcggcggcgtccgcaccggggctgagcgagcagcgacgcgaggggcgcgcggagatg (Seq ID No: 312)

Homo sapiens citrate synthase (CS):

gggcctccttgaggaccccgggctgggcgccgccgccggttcgtctactctttccttcag ccgcctcctttcaaccttgtcaacccgtcggcgcggcctctggtgcagcggcggcggctc ctgttcctgccgcagctctctccctttcttacctccccaccagatcccggagatcgcccg ccatggctttacttactgcggccgcccggctcttgggaaccaaggcacccagtggcaagt actagctgagcatttgggagatgcttgtcttacttggctgttgcttctcctgctgctggg gaaaaggaatgcatcttgtcttgttcttgcagcccggcatgccagtgcttcctccacgaa tttgaaagacatattggctgacctgatacctaaggagcaggccagaattaagactttcag gcagcaacatggcaagacggtggtgggccaaatcactgtggacatg (Seq ID No: 313)

Homo sapiens cathepsin S (CTSS):

atttcttttcaagtcaattgaactgaaatctccttgttgctttgaaatcttagaagagag cccactaattcaaggactcttactgtgggagcaactgctggttctatcacaatg (Seq ID No: 314)

Homo sapiens deoxynucleotidyltransferase, terminal (DNTT):

cagtctccctcccttctggagacaccaccagatgggccagccagaggcagcagcagcctc ttcccatg (Seq ID No: 315)

Homo sapiens dual specificity phosphatase 3 (DUSP3): cgctctccgcctcgcttgctcctgccgggcgtgcagggccccgccgccgccatg (Seq ID No: 316)

Homo sapiens coagulation factor II (thrombin) receptor-like 2 (F2RL2):

catcctttccctgcggaggaccagggcaagtttcctgcctgcacggcacaggagagcaaa cttctacagacagaccaaggcttccatttgctgctgacacatggaactgaggtgaaattg tgctccatgattttacagatttcataacgtttaagagacgggactcaggtcatcaaaatg (Seq ID No: 317)

Homo sapiens Fc fragment of IgG, receptor, transporter, alph a (FCGRT): cgtcctctcagcatg (Seq ID No: 318)

Homo sapiens guanylate binding protein 2, interferon-inducib le (GBP2):

ttacctctttttcttgtctctcgtcaggtctctgacattgacagagcctggacgttggag gaagccccaggacgttggaggggtaaagtaaaagtccacagttaccgtgagagaaaaaag agggagaaagcagtgcagccaaactcggaagaaaagagaggaggaaaaggactcgacttt cacattggaacaaccttctttccagtgctaaaggatctctgatctggggaacaacaccct ggacatg (Seq ID No: 319)

Homo sapiens G protein pathway suppressor 1 (GPS1):

cgctctttctcccttcagcagccagccagctctgtgtcagggtcgggggtgcagaaagt caggacagaatg (Seq ID No: 320)

Homo sapiens general transcription factor IIF, polypeptide 2 , 30kDa (GTF2F2):

gttcctcttttcctcggttcccagtgttctggcaggtaaggaacgccggctcttcgcctc tcagcgcggcttgtcctttgttccggacgcccgctcctcagccctgcggctcctggggtc gctgctgcatcccgcacgcctccaccggctgcagacccatg (Seq ID No: 321)

Homo sapiens glycogenin 1 (GYG1):

cgctccctcccggtgccggcttctctgagtcaccaacctgaggctgccccggccgcctgc gcacccggcagcaccatg (Seq ID No: 322)

Homo sapiens heat shock 70kDa protein 9 (mortalin) (HSPA9): agctctttgccgtcggagcgcttgtttgctgcctcgtactcctccatttatccgccatg (Seq ID No: 323)

Homo sapiens iron-responsive element binding protein 2 (IREB2) :

cttccttctttcctcccttgccagtccgcctgtcttcctccccgtcttccctgcccggcc tccccttcttcccccgctggccccctccccggagggataatatggtctccggcgatg (Seq ID No: 324)

Homo sapiens origin recognition complex, subunit 1 (ORC1):

ccaccttcttttcatttctagtgagacacacgctttggtcctggctttcggcccgtagtt gtagaaggagccctgctggtgcaggttagaggtgccgcatcccccggagctctcgaagtg gaggcggtaggaaacggagggcttgcggctagccggaggaagctttggagccggaagcca tg (Seq ID No: 325)

Homo sapiens RAB1A, member RAS oncogene family (RAB1A):

cattcctttctttcgattacccgtggcgcggagagtcagggcggcggctgcggcagcaag ggcggcggtggcggcggcggcagctgcagtgacatg (Seq ID No: 326)

Homo sapiens cytohesin 2 (CYTH2):

gagtcttttcagcgctgaggactggcgctgaggaggcggcggtggctcccggggcgtttg agcgggctcacccgagcccgcgggccaacgcggatccaggcccgactggcgggaccgccc cggattccccgcgggccttcctagccgccatg (Seq ID No: 327)

Homo sapiens COP9 constitutive photomorphogenic homolog subu nit 2 (Arabidopsis) (COPS2): atttctcctccccctcccggccaagatg (Seq ID No: 328)

Homo sapiens solute carrier family 9 (sodium/hydrogen exchanger), member 3 regulator 1 (SLC9A3R1) :

ggtcctctctcggctcctcgcggctcgcggcggccgacggttcctgggacacctgcttgc
ttggcccgtccggcggctcagggcttctctgctgcgctcccggttcgctggacgggaaga
agggctgggccgtcccgtcccgtccccatcggaaccccaagtcgcgccgctgacccgtcg
cagggcgagatg (Seq ID No: 329)

Homo sapiens peptidase (mitochondrial processing) beta (PMPCB): ctaccttccttctagcagaaatg (Seq ID No: 330)

Homo sapiens RAB3D, member RAS oncogene family (RAB3D):

cggcccttcctccgccttctgggcggagcccgcgcgggatccgggtggctgcaggctgct
ggcttctgcggctgcggggtcggggtcgcggccagggccaagccgcagcgagttcacagg
cggaacccctgcaggcggcgcccccctacgcgaggtcacccctgggaaggagcgcagccca
cccggcccctccgcatccgagcaggacgcccgtctcctctccctgaggatttcaggtctc
cctgtcccaggaggcttgtgccaagatg (Seq ID No: 331)

Homo sapiens ATP-binding cassette, sub-family B (MDR/TAP): tcttctctcggttcctctttcctcgctcaagatg (Seq ID No: 332)

Homo sapiens N-acylsphingosine amidohydrolase (acid ceramidase) 1 (ASAH1): ggctcttctttgcctctgctggagtccggggagtggcgttggctgctagagcgatg (Seq ID No: 333)

Homo sapiens cytochrome c oxidase subunit VIc (COX6C):

ttttcctttagtcaggaaggacgttggtgttgaggttagcatacgtatcaaggacagtaa
ctaccatg (Seq ID No: 334)

Homo sapiens COX15 homolog, cytochrome c oxidase assembly pr otein (yeast) (COX15):

gcttctcttttccttggcggaggagggagaccacagagccctgggttgtggaagaggtgg
ctgttccctgtcatcagtatg (Seq ID No: 335)

Homo sapiens c-src tyrosine kinase (CSK):

ccccccttcccccgcctttcttccctccgcgacccgggccgtgcgtccgtccccctgcctc
tgcctggcggtccctcctcccctctccttgcacccatacctctttgtaccgcacccccctg
gggacccctgcgcccctcccctccccccctgaccgcatggaccgtcccgcaggccgctgat
gccgcccgcggcgaggtggcccggaccgcagtgccccaagagagctctaatggtaccaag
tgacaggttggctttactgtgactcggggacgccagagctcctgagaagatg
(Seq ID No: 336)

Homo sapiens versican (VCAN):

gagcctttctggggaagaactccaggcgtgcggacgcaacagccgagaacattaggtgtt
gtggacaggagctgggaccaagatcttcggccagccccgcatcctcccgcatcttccagc
accgtcccgcaccctccgcatccttccccgggccaccacgcttcctatgtgacccgcctg
ggcaacgccgaacccagtcgcgcagcgctgcagtgaatttccccccaaactgcaataag
ccgccttccaaggccaagatg (Seq ID No: 337)

Homo sapiens dystroglycan 1 (dystrophin-associated glycoprotein 1) (DAG1):

gcgcctcttaggcttggcggtggcggcggcggcagcttcgcgccgaatccccggggagcg
gcggtggcggcgtcctggggccaggaggagcgaacacctgccgcggtcctcccgccggcg
ctgggctctgtgtgctccgggatggagcaggtgtgcagagggtgagaacccagctctggg
accaagtcacttgcttccttacttagcaagactatcgacttgagcaaacttggacctggg
atg (Seq ID No: 338)

Homo sapiens DEAD (Asp-Glu-Ala-Asp) box helicase 5 (DDX5):

cccccctcttttggttacagacgtgagggctctttggagacgtaaacatctccgagtggcg
agggtgggcggggctgggcttgggaaagggcggggtggcttgcttgaggtgtggaaagac
cagaagaaggtgaggtcaagagagtgcagaatgaggcattccaatggtgggtgggccctg
acctgagagagtggcgcggggaggggtgaaagcgcggcgatcctggaacgccagcgggcg
ttgcggcctatgcgcgaggggcggggcgattaggtcatagagcggctcccagcgttccct
gcggcgtaggaggcggtccagactataaaagcggctgccggaaagcggccggcacctcat
tcatttctaccggtctctagtagtgcagcttcggctggtgtcatcggtgtccttcctccg
ctgccgcccccgcaaggcttcgccgtcatcgaggccatttccagcgacttgtcgcacgct
tttctatatacttcgttccccgccaaccgcaaccattgacgccatg
(Seq ID No: 339)

Homo sapiens desmoplakin (DSP):

gctcctctgcgcccttgccgccctccgagccacagctttcctcccgctcctgcccccggc
ccgtcgccgtctccgcgctcgcagcggcctcgggagggcccaggtagcgagcagcgacct
cgcgagccttccgcactcccgcccggttccccggccgtccgcctatccttggccccctcc
gctttctccgcgccggcccgcctcgcttatgcctcggcgctgagccgctctcccgattgc
ccgccgacatg (Seq ID No: 340)

Homo sapiens glutamyl-prolyl-tRNA synthetase (EPRS):

cttcctttcgcggggtcctccgtagttctggcacgagccaggcgtactgacaggtggacc
agcggactggtggagatg (Seq ID No: 341)

Homo sapiens acyl-CoA synthetase long-chain family member 4 (ACSL4) :

gctcctcctcgtcccagcgctagcgggcacgcggttcctttttgcgagctttccgagtgc
caggcgccggccggctgcgaagacgcggtgggccgcccctccgattgaaatcacagaaga
tattcgtgttcttcttaagagaaaaagaggacattttagctttctcagttgaaggcgtac
tttattgtcggcttccaaagattactaacttttatctgtatcactaagattgaactgcct
tggctgtactgctattcttactgctgcttctattattgccttcttcagcacaataaggct
ttcaaaagccaaagaataacaagaaataagcaccattttagaagcctttccactatg
(Seq ID No: 342)

Homo sapiens fibroblast activation protein, alpha (FAP):

tggtccttttcaacggttttcacagatccagtgacccacgctctgaagacagaattagct
aactttcaaaaacatctggaaaaatg (Seq ID No: 343)

Homo sapiens UDP-N-acetyl-alpha-D-galactosamine:polypeptide N-acetyl_galactosaminyltransferase 3 (GalNAc-

T3) (GALNT3):

ctgcctctccaggcaacgcgggaggcccagcgggaaggcaggaggcggcggcggaggagg
agctctactgagccgcaactgtggcgacagcaaccggagtcgcagccgccgccacctgca
cctggcgcctagcccacgtccagcgcctgcccggccgccgcttcccgccaccctgccctg
cccacccgccaggtactaccattaaagataccttcttctcagcaaatctatgataaaaaa
tataagtaacagaagaagaaataactgttatttgtcaagtgacaagcttttaatgtcaga
atg (Seq ID No: 344)

Homo sapiens glypican 3 (GPC3):

acgtctcttgctcctcagggccactgccaggcttgccgagtcctgggactgctctcgctc

cggctgccactctcccgcgctctcctagctccctgcgaagcaggatg
(Seq ID No: 345)

Homo sapiens interleukin enhancer binding factor 2, 45kDa (ILF2) :

acgcctcttcagttgtctgctactcagaggaaggggcggttggtgcggcctccattgttc
gtgttttaaggcgccatg (Seq ID No: 346)

Homo sapiens nucleosome assembly protein 1-like 1 (NAP1L1):

gggtctttttagcgccatctgctcgcggcgccgcctcctgctcctcccgctgctgctgc
cgctgccgccctgagtcactgcctgcgcagctccggccgcctggctccccatactagtcg
ccgatatttggagttcttacaacatg (Seq ID No: 347)

Homo sapiens asparaginyl-tRNA synthetase (NARS):

cgctctctgatgcaacgccggaatcgcggaaaccgccggtgcacgttggagtcataagac
ggcgtcggtgttgcagtctgtgtccttggaggtgaccagggccactgcaggcatg
(Seq ID No: 348)

Homo sapiens NADH dehydrogenase (ubiquinone) 1 alpha subcomplex, 10, 42kDa (NDUFA10): cgtccccttgggtccttgatcctgagctgaccgggtagccatg (Seq ID No: 349)

Homo sapiens NADH dehydrogenase (ubiquinone) Fe-S protein 2, 49kDa (NADH-coenzyme Q reductase) (NDUFS2):
ttctccttcccgcagtctgcagccggagtaagatg (Seq ID No: 350)

Homo sapiens NADH dehydrogenase (ubiquinone) Fe-S protein 5, 15kDa (NADH-coenzyme Q reductase) (NDUFS5):

catcctttacggcaggcgtccgcgtcgctagctagtcgttctgaagcggcggccagagaa
gagtcaagggcacgagcatcgggtagccatg (Seq ID No: 351)

Homo sapiens phosphoenolpyruvate carboxykinase 2 (mitochondrial) (PCK2):

ccctccttttaagcgcctcccgccagcctctgctgtggctcgcttcgccgcgctccctc cttccccgccttccatacctccccggctccgctcggttcctggccaccccgcagcccctg cccaggtgccatg (Seq ID No: 352)

Homo sapiens serpin peptidase inhibitor, clade B (ovalbumin), member 6 (SERPINB6):

ctcccttcgcgctccggacgggcgacggtagctcgagacccgggactccgcccgcctccc cgcgagtatttgaggtccggggcggctccggcgcctctgcccgccgttctgctcgctcgc tccccgctctggagtctgccatcatg (Seq ID No: 353)

Homo sapiens Rab geranylgeranyltransferase, alpha subunit (RABGGTA) :

ttctctcctcagacttcaagggctaccactggacccttcccctgtcttgaaccctgagcc ggcaccatg (Seq ID No: 354)

Homo sapiens Rab geranylgeranyltransferase, beta subunit (RABGGTB): ctctctcctttccctgttagacatg (Seq ID No: 355)

Homo sapiens small nuclear ribonucleoprotein polypeptide A (SNRPA) :

agttctctccgcacgcgggctggagaagcgggtcctacgcacgctttgttgtcgcgcttt gcctccgtccttgcccctactcccgccttacctgacttccttttcggaggaagatccttg agcagccgacgttgggacaaaggatttggagaaacccagggctaaagtcacgtttttcct cctttaagacttacctcaacacttcactccatg (Seq ID No: 356)

Homo sapiens sterol regulatory element binding transcription factor 2 (SREBF2):

cgccctttctgtgcggcgcccgggcgcaacgcaaacatggcggcgggtggcacccgtcgg tgaggcggtgccgggcgggggttgtcgggtgtcatgggcggtggcgacggcaccgccccc gcgtctccctgagcgggacggcaggggggcttctgcgctgagccgggcgatg (Seq ID No: 357)

Homo sapiens translin (TSN):

ctgccctttggacgcgcgcctcggttccgaacgcagcggacggcgcctcaggcagcgcgg cggacagcccgtcctccggcgcgccgcgagcctcggaggaccctagcgacggtcgtggcg taagaccggggggacgcggcggtagcggcggccgttgcgattgattgcgctggttgcctg cggcgtccacttccttggccgcccttgctacactggctgattgttgtgcagccggcgcca tg (Seq ID No: 358)

Homo sapiens Fanconi anemia, complementation group G (FANCG) :

ccaccctttctcgaggctgtggcctccgcgagagccgagcgggccgcaccgccggccgtg cgactgccccagtcagacacgaccccggcttctagcccgcctaagcctgtttggggttgc tgactcgtttcctccccgagtttcccgcgggaactaactcttcaagaggaccaaccgcag cccagagcttcgcagacccggccaaccagaggcgaggttgagagcccggcggccgcggg gagagagcgtcccatctgtcctggaaagcctgggcgggtggattgggaccccgagagaag caggggagctcggcggggtgcagaagtgcccaggcccctccccgctggggttgggagctt gggcaggccagcttcacccttcctaagtccgcttctggtctccgggcccagcctcggcca ccatg (Seq ID No: 359)

Homo sapiens DEAD (Asp-Glu-Ala-Asp) box polypeptide 39B (DDX39B) :

ttccctccttcgtcgctgttgctgccgccatacgcgctctccctgtttagctcttctgtt
agaaatagtatctttgttttcctttgctgttcctcaatcccctactcttcaccccttgtt
ttcacctattttgcgagaacccatccagatccccccttcccttcttcccctgccggcccag
ttatg (Seq ID No: 360)

Homo sapiens RAB11A, member RAS oncogene family (RAB11A): ccgccctttcgctcctcggccgcgcaatg (Seq ID No: 361)

Homo sapiens SPARC-like 1 (hevin) (SPARCL1):

agctctttcccttttggtttgcaagcactgcctgtaaagccctcgcatgagaggccagcc
tgctagggaaatccaggaatctgcaacaaaaacgatgacagtctgaaatactctctggtg
ccaacctccaaattctcgtctgtcacttcagacccccactagttgacagagcagcagaat
ttcaactccagtagacttgaatatgcctctgggcaaagaagcagagctaacgaggaaagg

gatttaaagagtttttcttgggtgtttgtcaaacttttattccctgtctgtgtgcagagg
ggattcaacttcaatttttctgcagtggctctgggtccagccccttacttaaagatctgg
aaagcatg (Seq ID No: 362)

Homo sapiens cyclin B2 (CCNB2):

ctccctttcagtccgcgtccctccctgggccgggctggcactcttgccttccccgtccc
tcatg (Seq ID No: 363)

Homo sapiens cytochrome c oxidase subunit VIIa polypeptide 2 like (COX7A2L):

ggtccttctctggggcggtcgcgttggcagcggatgcgggaagccggactctgggcgtca
tg (Seq ID No: 364)

Homo sapiens lysophosphatidic acid receptor 2 (LPAR2):

cgccctctcagcaacccgcacagggcgcacccggacgctctaccgctcccgccgcagtcg
ccgggccatgggcctcgagcccgccccgaacccccgcgagcccgccttgtctgcggcgtg
actggaggcccagatg (Seq ID No: 365)

Homo sapiens adaptor-related protein complex 4, mu 1 subunit (AP4M1):

cgttcttttgttccggggccgcagggcggggcaggcccgactttcgccgtcttcttgtct
actctccagaacggccatg (Seq ID No: 366)

Homo sapiens budding uninhibited by benzimidazoles 3 homolog (yeast) (BUB3):

cttcctctccgcctccttcgcctagcctgcgagtgttctgagggaagcaaggaggcggcg
gcggccgcagcgagtggcgagtagtggaaacgttgcttctgaggggagcccaagatg
(Seq ID No: 367)

Homo sapiens DEAD (Asp-Glu-Ala-Asp) box helicase 21 (DDX21):

ctacctcttcctctccacgcggttgagaagaccggtcggcctgggcaacctgcgctgaag atg (Seq ID No: 368)

Homo sapiens solute carrier family 33 (acetyl-CoA transporter), member 1 (SLC33A1):

tgctctctgccgcattgatagcagcgagagctggaggtgttgggtcgggagaccagccgt tcgatcccgccgcaggtaggagctggtttccatcctggcaccacggcacacacctccagc ctcgagcccggcgctgctgcccggggtctccttcaggctctttgacgccgttccagggg gcacctatccaggcatcctctgggcctctagccagaggactggctcccggcttcagcact ccgggctgcagtaagaagtgcccttatcgctctgagccctgccaccatcccgtgaaccac cgaaaccctggtccagcgcgacagccttggacctgggactggacggatccaaaacgctca gcctcggcccccacagacggggctctgcatcgtctctgatatg (Seq ID No: 369)

Homo sapiens G protein-coupled receptor 37 like 1 (GPR37L1): tgctcttcctgggctggctgtctcctgctcatccagccatg (Seq ID No: 370)

Homo sapiens neuronal regeneration related protein homolog (rat) (NREP):

ctgtctttctagcatgttgccctttttcaaccacatttgtgtttcaggtgtagagaggag

agagagtgaacagggagcggggcttttgtctgttggtctccctggactgaagagagggag aatagaagcccaagactaagattctcaaaatg (Seq ID No: 371)

Homo sapiens vesicle-associated membrane protein 3 (cellubrevin) (VAMP3): gcttctctgctgaccctctctcgtcgccgctgccgccgccgcagctgccaaaatg (Seq ID No: 372)

Homo sapiens synaptosomal-associated protein, 29kDa (SNAP29): cctccttctgtttcccagaccgagagccgcgccggcaccatg (Seq ID No: 373)

Homo sapiens lon peptidase 1, mitochondrial (LONP1):

cccccctcttctccgcgtaggcccagctccctgaagcggctgtttcgagccacgcgcccat cgggtaccgaggcacgcgccgggcgtcacgtgcgtttcgcggcgagcggaaatgacgcga gttgtgtgagccgccagtatggccgggctatg (Seq ID No: 374)

Homo sapiens kinesin family member 3B (KIF3B):

ctgtctctccccatccggggcagcggggaatggctgagccaggggttcgccgcccccgcc gccgccgccgccgccgccgccgccgccgccgcccgctttcggctcgggcctcaggaccgt agcatcctgagacattttgaattgacacttctcaagatttgactggatcagagttcatca tg (Seq ID No: 375)

Homo sapiens transmembrane 9 superfamily member 2 (TM9SF2):

cttcctttatctctggcggccttgtagtcgtctccgagactccccacccctccttccctc ttgacccccctaggtttgattgccctttccccgaaacaactatcatg (Seq ID No: 376)

Homo sapiens cytosolic iron-sulfur protein assembly 1 (CIAO1) :

gagcctctgtcggccgcggaagcctggagtgggcggtacgcagacgcgcgcggtgagacc
cgctgtctgctcagcggactctgcccgcccccacctccccctgcgtcgggccgacatg
(Seq ID No: 377)

Homo sapiens GRB2-related adaptor protein 2 (GRAP2):

caccctctttcagagtggtacatggaagacagcacaaagtggatccatactctgaaatgc
agtaactctgatgcttgaatttgtctcccttcttgccagaaaggattctaataactcggt
gtcaaagccaagacataaactcaacccctctcttccaaaagcttcacgttacagcatg
(Seq ID No: 378)

Homo sapiens leupaxin (LPXN):

gtacctttctcggggtgtctgcgtaactgcccagacttgccttggtttggtcagatgaca
cctcctctgggactggctagccagcgttcatg (Seq ID No: 379)

Homo sapiens SH3-domain binding protein 5 (BTK-associated) (SH3BP5) :

tttcctctgctccgccgcggccggaggtatccgcatcggcgagctgcgtctcccgggtgt
cggccccggcggctccccgaccgtgcccggctgtggcgaggcggctccagcccagcctgt
ggcagccgcgacccccggggcgctccggagcccactgcgcggcgcgcgtgccggctgcct
gcatg (Seq ID No: 380)

Homo sapiens phosphatidylinositol glycan anchor biosynthesis , class B (PIGB): ctttcttccgccttaggaaggtggcggccagggatg (Seq ID No: 381)

Homo sapiens lipopolysaccharide-induced TNF factor (LITAF):

cggccctttctcggggcgcccgagaggccagctcagacctcccggctcgacaggcggcg
cgggcggcggtgagtgcggcgcggggacgccggggcgcggggaccagcgggagacagcgg
ggggccggtggcgccagcacctgctgggggccccgggcactgagcccttggctggggcct
cctgggatgccagggggcgcgggtcgggtcgcgggcatcgaggcgcggcggagggcgtgg
gggcccggccggggcggggtccggcctcccagcgctggtcccggccgcgtctccggttgg
gttcagctcctgcgtcccagagtggcccgatcgcgcgtggcggggtcgtccggcccccac
ccgaacgagcgcccttcgcggcccgccgcgtccccctccccggagaggacggcccctggg
ctttttagaaaaaggcgcgattctctctagtgactcaggttgagatttccagaaatatcc
cccgggggttcagaaacaaaaccaaaacaaacaaaaaaaccccaacgaattcccaaatgc
tatttgccaaacatttgacttctaggggcgcgggtacccgcgtttctctccctgcccccg
cgacttcgcgcaagatccgggaaggacacccgaggcccctgggagaccctggggaggtga
aaatcagagagcgaagcgggccgtggcccctaggcctgacccctccccgcggggtaaggc
gggcaccccgcgagcgcaggggtcctcttactgctgatggcacccagctctgggcccaga
cgccgctcaccgtccaccgccggtgctgggtaaaatg (Seq ID No: 382)

Homo sapiens etoposide induced 2.4 mRNA (EI24):

ccaccccttcggctctgggccccgcctcgtggtgccggctggttcttcgcgctcgcccga cttcccagcggccccgtgcggcccgggcatgcccagtgcgggcgcagcggccccggccct ggaagcgccccggcggagctggcctgcggtgggctaggggcagggccggagccgcggcgg cggagctgtggatccttcatgatgagagatttggggacacttctctctcctgtgtgtagt tgatagtttggtggtgaagagatg (Seq ID No: 383)

Homo sapiens chromosome 14 open reading frame 2 (C14orf2):

tgacctttccgagttggctgcagatttgtggtgcgttctgagccgtctgtcctgcgccaa gatg (Seq ID No: 384)

Homo sapiens peroxiredoxin 6 (PRDX6):

attcctccgcgcgctgggacaggctgcttcttcgccagaaccaaccggttgcttgctgtc ccagcggcgccccctcatcaccgtcgccatg (Seq ID No: 385)

Homo sapiens solute carrier family 29 (nucleoside transporters), member 1 (SLC29A1):

ctctcttccgcccggcggcccacaccggtcaggcccggcgcgggctgcgctctccagctg tggctatggccccagccccgagatgaggagggagagaactaggggcccgcaggcctggga atttccgtcccccaccaagtccggatgctcactccaaagtctcagcaggcccctgaggga gggagctgtcagccagggaaaaccgagaacaccatcaccatg (Seq ID No: 386)

Homo sapiens heterogeneous nuclear ribonucleoprotein F (HNRNPF) :

cgaccttcctgccgggccgggcggtccgaggctgctggagtgccgtgagcaggccgcggg aacgtcgccgtcaccttgtctcggggcctcggcgctgcttcccgccaaaacacgtttacc gcgcgcccgggcctcccaccttgcggaagggaccccaccaccacttggatttctgttgca ggttgagaacaaaaacatgcacctggagtttccccggagccctctgcgtggttgagcttc ggtggaatttcggggctcttggctgccagccgcgcttgcctggtagcaacagaaaccagt

cctgctcgcctccgtggacatttcattaccatccagaagtgtctcccactgaaggcatcc gtggttgtttttaagccacaaaaaagccacacccaagatcacctgacacccaccctgaca agtgtccatg (Seq ID No: 387)

Homo sapiens islet cell autoantigen 1, 69kDa (ICA1):

ccgcccctttccctcgccttcggctgacgctgacgtcggatgagtgatccggagggacgc tccgaccgcggccgggaggctcctgggggccggggctccgaggttataatataacttatc ctctcatgctttttttcctgccccttctccccaaatcatcaacaatagaagaagaagaaaa catg (Seq ID No: 388)

Homo sapiens PWP2 periodic tryptophan protein homolog (yeast) (PWP2):
gtgtctctgtgggcggccgccgggttgagctgcggcacacgtgcgacggccgtgatg (Seq ID No: 389)

Homo sapiens glutaminyl-tRNA synthetase (QARS):
gtttcttttagtttccggtgtctctgcaatg (Seq ID No: 390)

Homo sapiens stearoyl-CoA desaturase (delta-9-desaturase) (SCD) :

cggcctctgtctcctcccccctcccgcccttacctccacgcgggaccgcccgcgccagtca actcctcgcactttgcccctgcttggcagcggataaaaggggctgaggaaataccggac acggtcacccgttgccagctctagcctttaaattcccggctcggggacctccacgcaccg cggctagcgccgacaaccagctagcgtgcaaggcgccgcggctcagcgcgtaccggcggg cttcgaaaccgcagtcctccggcgaccccgaactccgctccggagcctcagcccccctgga aagtgatcccggcatccgagagccaagatg (Seq ID No: 391)

Homo sapiens fragile X mental retardation, autosomal homolog 1 (FXR1): cggcctttgcggttccaacatg (Seq ID No: 392)

Homo sapiens musculin (MSC):

tagcctttcaaaaggcgcagcttaccgcggtgcgcgcggattctggacttgggcgccaa ctcgtagtccacgctccccggggtcagcagaggggcgctcacgctctcgccacccacctc gctttctcaccccgcgcttcccggcctgggtttttagtcttccttggagcgctctctggc ctccgcctccgccagggagcggaaggcggagacagcgagactggccaggggggaggaaag aggacgcgtgtgggcaaggggacaacgggatg (Seq ID No: 393)

Homo sapiens RNA binding motif protein 8A (RBM8A):

cgacctttcccctctgcgacagtttcccgaggtacctagtgtctgagcggcacagacgag atctcgatcgaaggcgagatg (Seq ID No: 394)

Homo sapiens heparan sulfate (glucosamine) 3-O-sulfotransferase 1 (HS3ST1):

ggtcctctgcgccctggcagccaggagtcgccgccacgaccgccgggtctcagtgggtgc ctgcgccttctccccgcccgcctgccccgggccatccagaaacttgctctacccgccgcg ggtgctcggcagtgctgcccatggcccagcccaggagcctatttagggcgccggacgggc tggacagaggcgcggctcagtaattgaaggcctgaaacgcccatgtgccactgactagga ggcttccctgctgcggcacttcatgacccagcggcgcgcggcccagtgaagccaccgtgg tgtccagcatg (Seq ID No: 395)

Homo sapiens solute carrier family 12 (potassium/chloride transporters), member 6 (SLC12A6):

ctgtctcttgtaggcagggatcacagtctgaaacgacagcaaggaagaggtaggcaggga aaactaactggaaggaagtttaaatacagaaagagcaaagtattatctaactataacaat g (Seq ID No: 396)

Homo sapiens apelin receptor (APLNR):

cttcctccagggtctggagaacccagaggcagctcctcctgagtgctgggaaggactctg ggcatcttcagcccttcttactctctgaggctcaagccagaaattcaggctgcttgcaga gtgggtgacagagccacggagctggtgtccctgggaccctctgcccgtcttctctccact ccccagcatg (Seq ID No: 397)

Homo sapiens calpain 1, (mu/l) large subunit (CAPN1):

cgctcttcctggttgggccctgccctgagctgccaccgggaagccagcctcagggactgc agcgacccccaaacacccctcccccaggatg (Seq ID No: 398)

Homo sapiens cyclin C (CCNC):

cttcctttcgccgtcgccgccgcggagcggagtcgagccgagctgatttgatcgaggagc
gcggttaccggacgggctgggtctatggtcgctccgcgggccgctccgccggctggtgct
tttttatcagggcaagctgtgttccatg (Seq ID No: 399)

Homo sapiens glutamate dehydrogenase 1 (GLUD1):

cttcctccctagtcgcggggagtctgagaaagcgcgcctgtttcgcgaccatcacgcacc
tcccctccgcttgtggccatg (Seq ID No: 400)

Homo sapiens guanine nucleotide binding protein-like 1 (GNL1) :

cctccttcctcgccgccggggcgccctctcggtgccactggctctcacgtgccagtagcc
caccccgcatcatcctctcgcctcgctcctggagggaagtgactatatctcccccgtccg
ccttccatcgccgccgcggcggtaattctgtcgggcccgcccgctgacgtcacctgctag
ccccgcctcctctagggtcccgggcccctgcggcgggggctgccccgggggcagtcagt
tgaggcggcgggagctcggcggagggcgggccaggtgactggtccgggccatg
(Seq ID No: 401)

Homo sapiens lysophosphatidic acid receptor 4 (LPAR4):

aggcctttttgtgtcctgtttgctaaaggcatgcgggctacagcattcaagagagggagt
cgttaacaaagggaaagagataaatgtaaataagctcacatttacagaatgagcggtttg
cagtaaaaagctgcggcagcccagagtctgctactttaggctgggctaacctttccctgt
aaaaaaaaaaaaaaaaaaaaaaaaaaaaaaatggataaaaatatgcacttccaaagggcg
agttgcccatttacatgtttattagctaattatctacaggcatcagcacattctctcatc
tagcacactctttcttggggaggaaaatatttcctaccggtccatagtgtcagagtggtg
aaccccctgcagccagcaggcctcctgaaaaaaaagtccatg (Seq ID No: 402)

Homo sapiens G protein-coupled receptor kinase 5 (GRK5):

gctcctctttgcagagggggaaactcttgggctgagagcaggaataatgcggtaggcaag
gcgggctgctggctcccccggctccggcagcagcggcggcagcccgagcagcggcagcag
cagcggcagcaccccaggcgctgacagccccgccggccggctccgttgctgaccgccgac
tgtcaatg (Seq ID No: 403)

Homo sapiens glutamic-pyruvate transaminase (alanine aminotransferase) (GPT):

agccctttctgtccctcccagtgaggccagctgcggtgaagagggtgctctcttgcctgg
agttccctctgctacggctgccccctcccagccctggcccactaagccagacccagctgt
cgccattcccacttctggtcctgccacctcctgagctgccttcccgcctggtctgggtag
agtcatg (Seq ID No: 404)

Homo sapiens hydroxyacyl-CoA dehydrogenase (HADH): gggtctcctcgctgtcgccgccgctgccacaccatg (Seq ID No: 405)

Homo sapiens high density lipoprotein binding protein (HDLBP) :

tcttctcctttaccaagatggcggcttgtccctgtttcgccacagttcctaccttatgag ctcggttttcttatgcttataagagtggaacagcaaaagctggcaggctgacagaggcgg cctcaggacggaccttctggctactgaccgttttgctgtggttttcccggattgtgtgta ggtgtgagatcaaccatg (Seq ID No: 406)

Homo sapiens histidine triad nucleotide binding protein 1 (HINT1) : gttcctcccttcttccgagcctctcctctggccgccgcgcgggagagaggccgagatg (Seq ID No: 407)

Homo sapiens heat shock 70kDa protein 1A (HSPA1A):

ctaccttttcgagagtgactcccgttgtcccaaggcttcccagagcgaacctgtgcggc tgcaggcaccggcgcgtcgagtttccggcgtccggaaggaccgagctcttctcgcggatc cagtgttccgtttccagcccccaatctcagagcggagccgacagagagcagggaaccggc atg (Seq ID No: 408)

Homo sapiens nucleolin (NCL):

cagtctttcgcctcagtctcgagctctcgctggccttcgggtgtacgtgctccgggatct tcagcacccgcggccgccatcgccgtcgcttggcttcttctggactcatctgcgccactt gtccgcttcacactccgccgccatcatg (Seq ID No: 409)

Homo sapiens nuclear factor, interleukin 3 regulated (NFIL3) :

ccgcccctttctttctcctcgccggcccgagagcaggaacacgataacgaaggaggccca acttcattcaataaggagcctgacggatttatcccagacggtagaacaaaaggaagaata ttgatggattttaaaccagagtttttaaagagcttgagaatacggggaaattaatttgtt ctcctacacacatagatagggtaaggttgtttctgatg (Seq ID No: 410)

Homo sapiens protein phosphatase 1, regulatory subunit 3C (PPP1R3C) :

cagtctctcccagcgaccgccgcgggggcaaggcctggagctgtggttcgaatttgtgca ggcagcgggtgctggcttttagggtccgccgcctctctgcctaatg (Seq ID No: 411)

Homo sapiens protein tyrosine phosphatase, non-receptor type 14 (PTPN14):

agttctttccaactttttctcggcggagtgagcgcagcgggcgcagactcgggggcaggt tgctgtgcttctccgggctcagccgcctgctctcctggctcaggtcctcggggagcccta

gacagacatcaagtggccactggcgctccttcccctcccagctgagccatcctccccggc ctcctcgggcgggacagccccgtgcttaggtttttctcctttttctcccccggtgcgcctc tgctcggactctcgcgccgggatcgcggcggaaacctccctcccctttcgcctcctgcgg ctccttcccttcgcccctcctccgccagtcactggaatcaattccgtggggaatcggctc cgccgccgcgaaggacagcctttccgcgcgggactccggggcgccacgggggccatgtaa gcagctatcttccagagggccacactgggcatggacacccttttccctgcctggaggagc acaggtgatagtgtaattttccagtcacgaaactgctaaggccatctcaggggcgtgtgc gccaggataggcgggcggcgtccgaggaccacatagccatg (Seq ID No: 412)

Homo sapiens selenoprotein P, plasma, 1 (SEPP1):

ctttcttttaagttgataacaatcagctcaggggtttgctctgcttgcaaggtcactgca agaatgaacattgaactttggactatacctgaggggtgaggtaaacaacaggactataaa tatcagagtgtgctgctgtggctttgtggagctgccagagtaaagcaaagagaaaggaag caggcccgttggaagtggttgtgacaaccccagcaatg (Seq ID No: 413)

Homo sapiens serine hydroxymethyltransferase 2 (mitochondrial) (SHMT2):

agctcttctcgcgcatgcgttctccgaacggtcttcttccgacagcttgctgccctagac cagagttggtggctggacctcctgcgacttccgagttgcgatg (Seq ID No: 414)

Homo sapiens tyrosine kinase with immunoglobulin-like and EG F-like domains 1 (TIE1):

tttcctcttcctccccagcaccgacccacactgaccaacacaggctgagcagtcaggccc acagcatctgaccccaggcccagctcgtcctggctggcctgggtcggcctctggagtatg (Seq ID No: 415)

Homo sapiens coiled-coil domain containing 6 (CCDC6): cctcctttccccagcccgccgcggccatg (Seq ID No: 416)

Homo sapiens nuclear receptor coactivator 4 (NCOA4):

ggacctttcgcactcgggtcaggggtaaagcagcctgtcgcttgccgggcagctggtgag tcggtgacctggcctgtgaggagcagtgaggagaatg (Seq ID No: 417)

Homo sapiens chromatin assembly factor 1, subunit B (p60) (CHAF1B) :

gtgcctctgactgtccgggtccctccagcattttgcagctttctcctgtcttgaagaagt agaacggtgcccgagaaacgttttccccttcgagactcaggaggatgaaagtcatcact tgtgaaatagcctggcacaacaaggagcccgtgtacagcctggacttccagcatg (Seq ID No: 418)

Homo sapiens 3'-phosphoadenosine 5'-phosphosulfate synthase 1 (PAPSS1):

agccccgccccgctcgctggcctgccctcctcttgctaccctcccggcgcagagaacccc ggctgctcagcgcgctccgcggtcatg (Seq ID No: 419)

Homo sapiens Fas apoptotic inhibitory molecule 3 (FAIM3):

tgccctcctcttgctaccctcccggcgcagagaaccccggctgctcagcgcgctccgcgg tcatg (Seq ID No: 420)

Homo sapiens N-acetylated alpha-linked acidic dipeptidase 2 (NAALAD2):

cagcctcctgccagcgcgctctctgtttctctgcagccccgaagctcgcgaatgtagcag gcgccccaagctcggtcctcaagaagccatggcggaatccaggggccgtctgtacctttg gatgtgcttggctgctgcgctggcatctttcctgatgggatttatggtgggtaagt (Seq ID No: 421)

Homo sapiens abl-interactor 1 (ABI1):
ctgtctctttaacgcgagaggaagcgatgcagaggggtggaaaatg
(Seq ID No: 422)

Homo sapiens potassium voltage-gated channel, Isk-related fa mily, member 3 (KCNE3):

cttccttttctgccttctctcctgctttctagctctgggctttcccagctccgaagtcaa tactgagatcccagatgtgtccagagacatcctgaagaggctcggggtggaggagcctt agtgtgtccacaaagggactcctgaaactgactgagagccagt (Seq ID No: 423)

Homo sapiens target of myb1 (chicken)-like 1 (TOM1L1): ggccctctggcgctaccatg (Seq ID No: 424)

Homo sapiens ubiquitin-like modifier activating enzyme 2 (UBA2) :

cgcccttcccccacccgcttccggccgcggctcggttctcccgcctccgcctccgccgcg gctcgtggttgtcccgccatg (Seq ID No: 425)

Homo sapiens scavenger receptor class B, member 2 (SCARB2):

ctccctccttgcagttggatccctggcgggtgcggcccggcccggcccgtgagcggcgca cagaatg (Seq ID No: 426)

Homo sapiens insulin induced gene 1 (INSIG1):

actcctcctttcccccgccccgcctccgttcggagagccggcgggcgggcgcctctcggc caggaagcgcctcttggacgcgtgtgaccgatg (Seq ID No: 427)

Homo sapiens kinesin family member C3 (KIFC3):

aggcctcttctgaggctctaggtgccccagtagcagggccttctgcagcaaggccgggaa ctgctgcaccattggtgtgttttaccttaagggactccaggcagcttccttgctgggaag atattcatttgctggggtggggctgggggtgcagaggtaggaagtgctgtggctagaagg cggcctggccagcgagtaggtggtggagcgagtgagagcgtgtgcgctgtaaacagtgtg agtgcatg (Seq ID No: 428)

Homo sapiens LIM domain kinase 2 (LIMK2):

aggcctcttctgaggctctaggtgccccagtagcagggccttctgcagcaaggccgggaa ctgctgcaccattggtgtgttttaccttaagggactccaggcagcttccttgctgggaag atattcatttgctggggtggggctgggggtgcagaggtaggaagtgctgtggctagaagg cggcctggccagcgagtaggtggtggagcgagtgagagcgtgtgcgctgtaaacagtgtg agtgcatgtgcgccagcgcgtgcaaggacacggtaagggatgtacatgtattgtctcgtg agtaagagcttgtgtgtgtgttgggatgggaagacacgtactggtatgagagcccgcgtg agaagtgtatgtgtgagtactcgcgtggaagttttgcactcgggtttgaggctgtgcaaa agtacgcatggctcaccaggtgtggggctgtgtggctgcctcgtgtgtgccagcccgtg tgcaggcctgttttgtgagagccttcagggaacgcatgagcacgtgtgccagtgcgagtg

cgggacgcggggaggcgggagagaccgagtgggaggccccgcgaaggagtgggagtggga gtgggagtgccggcgggagacctgcggggcgcgcccgggctgacgcgtgcgcgccagtg cgcgtgagtgcgggcgcgcgccgccgccccccgccggggtcggagccggttgccatggga acgcgccgcggcccgagttaatcatttcctgtggaaagtgtgcgggaggggcgcgagcgg gctggccgaggaggaggcggcggcgtggagctgcctcctgccggcgggccgggccgggcc gagccccgggcgctgcggcgacgcctggatcctgcctccgccaggccggctgcctggtgc cccgaggaggctgctgagccccaggccatg (Seq ID No: 429)

Homo sapiens lectin, mannose-binding, 1 (LMAN1):
cctcctccgcgttccagaatccaagatg (Seq ID No: 430)

Homo sapiens MRE11 meiotic recombination 11 homolog A (S. cerevisiae) (MRE11A):

cgttctctcccgcggaattcaggtttacggccctgcgggttctcagaggcaagttcagac cgtgttgttttcttttcacggatcctgccctttcttcccgaaaagaagacagccttgggt cgcgattgtggggcttcgaagagtccagcagtgggaatttctagaatttggaatcgagtg cattttctgacatttgagtacagtacccaggggttcttggagaagaacctggtcccagag gagcttgactgaccataaaaatg (Seq ID No: 431)

Homo sapiens nascent polypeptide-associated complex alpha su bunit (NACA):

cttccttctgcaacaggcgtgggtcacgctctcgctcggtctttctgccgccatcttggt tccgcgttccctgcacagtaagtactttctgtgccgctactgtctatccgcagccatccg cctttctttcgggctaagccgccccggggactgagagttaaggagagttggaggctttac tgggccacagggttcctactcgcccctgggcctccggacaaaatggggtctgcggttggt gtcctggcaaaagcagggtagaagggctgcggggcgggcccagaatccgagcctgcagag atgggagcagttgcagtgttgagggcggaagaggagtgcgtcttgttttgggaactgctt cacaggatccagaaaaggaaatg (Seq ID No: 432)

Homo sapiens claudin 11 (CLDN11):

cgcccttcgccgctgagctcgcagcctccggcgcccacctccacctccagtgtcccgcct cgggccgtcgccctccagcggctcgcgagcgtgggagacgtacctgggcaggcactgtcc agcccaggcccaggcacagccgtgaggggcgaggcacggggacatcctggcggccaccat g (Seq ID No: 433)

Homo sapiens retinoblastoma binding protein 4 (RBBP4): ccgcccctcccgcaacgctcgaccccaggattcccccggctcgcctgcccgccatg (Seq ID No: 434)

Homo sapiens acyl-CoA synthetase medium-chain family member 3 (ACSM3):

ccctcttctttagactgccacgaggaaaaagcagatgtgagaactcaaggttcagggctg ctcttctaagaaacaagtctgccataatctccatctgtgttggaatctgttaactaatga actggtctctgtgcaaatcctgagtgctaaagcttccaacaagactgatg
(Seq ID No: 435)

Homo sapiens syndecan binding protein (syntenin) (SDCBP):

cgctctcttacactcgggcctcagaagtccgtgccagtgaccggaggcggcggcggcgag cggttccttgtgggctagaagaatcctgcaaaaatg (Seq ID No: 436)

Homo sapiens serum/glucocorticoid regulated kinase 1 (SGK1):

agtccttctcattccttgcccccgcccaaggctctcttcaccttccccgcggggtcctc
tcgttttctgtctcccaaatgctggcttcccgcctttcctcccccgcttatttacttaat
taaggccctggggctgcaccccaccggcagctccttcgggggtgtggccgaagagctccg
agggcggggctgaccgagccatattcgggcgtggccggtggtgattggtgagggcggggc
ctgccgcagggggcggggcctgcaggtttggcccccgcagggagcgcagctggcgccgct
gggagctggtggcgcggcgcaggtcccggccgagtgtggcgcagcagtggcggcgcttcc
cattcgccatgcgccgggggtgggtgcccgaaggttgcatgatggaatttgaacattact
tcaagaggttttgtattttggattagttaattgggtttgtcctctgctgactgtttcttc
ggatgcatttttggtgtgctcttgagggattaaatg (Seq ID No: 437)

Homo sapiens Wolf-Hirschhorn syndrome candidate 2 (WHSC2):

cgtccttccggctctcggctttgccacaaagcttcccgaagacgcggccgctacccggag
acgcggtcgccacccagaagcgctctcccgggaagccccgctcgtgggaccgcgccacct
gcgccgcctctgcggcccgcagcccgacgggcgccgccatgttggggtcctagcgaggga
cgcgtaggtgtcttcataagatg (Seq ID No: 438)

Homo sapiens nuclear receptor subfamily 1, group H, member 3 (NR1H3) :

cagtccttttgcaagagctgctaagagcgctgggtaaggagaggaaggggagagacatgg
aacttggctggtctgcagggaaatgccactgttttggccgggagtagggggcgggagtgg
cgggagaggggtggccggctggggaggagccagcctggtggagaagctgccctgtgggc
gggggtgaggagggggagggctgtggtcaccaggcaggaaggaggggtggcctgacccctc
ggcagtccctcccctcagcctttccccaaattgctacttctctggggctccaggtcctgc
ttgtgctcagctccagctcactggctggccaccgagacttctggacaggaaactgcacca
tcctcttctcccagcaaggggggctccagagactgcccacccaggaagtctggtggcctgg
ggatttggtgggtctgctccttag (Seq ID No: 439)

Homo sapiens glypican 6 (GPC6):

cctcctttctccttccctcttgcctccagtgactgtctccaggatttctctcttcctatt
tcaggaggactctcacaggctcccacagcctgtgttaagctgaggtttcccctagatctc
gtatatccccaacacatacctccacgcacacacatccccaagaacctcgagctcacacca
acagacacacgcgcgcatacacactcgctctcgcttgtccatctccctcccgggggagcc
ggcgcgcgctcccacctttgccgcacactccggcgagccgagcccgcagcgctccaggat
tctgcggctcggaactcggattgcagctctgaacccccatggtggtttttaaacacttc
ttttccttctcttcctcgttttgattgcaccgtttccatctgggggctagaggagcaagg
cagcagccttcccagccagcccttgttggcttgccatcgtccatctggcttataaaagtt
tgctgagcgcagtccagagggctgcgctgctcgtcccctcggctggcagaaggggtgac
gctgggcagcggcgaggagcgcgccgctgcctctggcgggctttcggcttgagggcaag
gtgaagagcgcaccggccgtggggtttaccgagctggatttgtatgttgcaccatg
(Seq ID No: 440)

Homo sapiens peptidylprolyl isomerase F (PPIF):

cggccttctgggcgcgcgcgacgtcagtttgagttctgtgttctccccgcccgtgtcccg
cccgacccgcgcccgcgatg (Seq ID No: 441)

Homo sapiens ARP1 actin-related protein 1 homolog A, centrac tin alpha (yeast) (ACTR1A): agttccttccccagaaggagagattcctctgccatg (Seq ID No: 442)

Homo sapiens tripartite motif containing 28 (TRIM28):

ggctctttctgcgagcgggcgcgcgggcgagcggttgtgcttgtgcttgtggcgcgtggt
gcgggtttcggcggcggctgaggaagaagcgcgggcggcgccttcgggaggcgagcaggc
agcagttggccgtgccgtagcagcgtcccgcgcgcggcgggcagcggcccaggaggcgcg
tggcggcgctcggcctcgcggcggcggcggcggcagcggcccagcagttggcggcgagcg
cgtctgcgcctgcgcggcgggccccgcgcccctcctcccccccctgggcgcccccggcggc
gtgtgaatg (Seq ID No: 443)

Homo sapiens aminoadipate-semialdehyde synthase (AASS):

cggccttccatcccagtttcttctaggaattcggagcctcccctgcagcgactcggaaga
ttcgaggcggcgggggacaagtcggcgccccagagcggacgagtcaccaggtgtcaagat
g (Seq ID No: 444)

Homo sapiens cornichon homolog (Drosophila) (CNIH): ccgcctttctccgctggcaacggcgccgctccccgctcctcctccccagccatg (Seq ID No: 445)

Homo sapiens M-phase phosphoprotein 10 (U3 small nucleolar ribonucleoprotein) (MPHOSPH10): ctcccttcccttgcatgctgcattgtgtcgggagttgctgacagccatg
(Seq ID No: 446)

Homo sapiens ubiquitin specific peptidase like 1 (USPL1):

ccgccttcctagtggagacgcgagtgggggaggagcagtccgaggggaacgtgggttgaa
cgttgcaactagggtggagatcaagctggaacaggagttccgatcgacccggtaccaaga
aggggagtgcccgcggcagggttcattgaaaaaatccttagtgatattgacatgtctcaa
gtgacataaattagccaatgactcggaatg (Seq ID No: 447)

Homo sapiens solute carrier family 23 (nucleobase transporters), member 1 (SLC23A1):

tggcctttgtcaagtcatcccctcttctcctcaggaactgctcaaacctgtgccccaaag
atg (Seq ID No: 448)

Homo sapiens splicing factor 3b, subunit 4, 49kDa (SF3B4): ggatctctttcgccatg (Seq ID No: 449)

Homo sapiens DnaJ (Hsp40) homolog, subfamily A, member 2 (DNAJA2) :

ctgtctccctcggcctgtgccgccgccgacgccgcttgtgggcccgactccgctctgtct
gcttcgccaccttctccccgagcactgcccggccggccgccatg
(Seq ID No: 450)

Homo sapiens calicin (CCIN):

catcctctcttccaccctctcttctccctggtcaaccgctctgcaaacaaccatcaatct
gatcccacaggcctgagaaagtctgctctccagtacctgctgctgatctgtttcagccga
caagaggcaccatg (Seq ID No: 451)

Homo sapiens mannosidase, beta A, lysosomal (MANBA): ctgcctttcgatctctccacatctcggtggcgcgggatctcaagatg

(Seq ID No: 452)

Homo sapiens microtubule-associated protein 1B (MAP1B):

aatcctttctcctgccgcagtggagaggagcggccggagcgagacacttcgccgaggcac agcagccggcaggatg (Seq ID No: 453)

Homo sapiens malate dehydrogenase 1, NAD (soluble) (MDH1):

gagccttttctcgctaacaccgctcgccctctccgagtcagttccgcggtagaggtgacc tgactctctgaggctcattttgcagttgttgaaattgtccccgcagttttcaatcatg (Seq ID No: 454)

Homo sapiens microfibrillar-associated protein 1 (MFAP1):

gtttctctatcagtcgcgcagctgtgttcgcggactcaggtggaaggaatttcttctctt cgttgacgttgctggtgttcactgtttggaattagtcaagtttcgggaatcaccgtcgct gccatcaacatg (Seq ID No: 455)

Homo sapiens chaperonin containing TCP1, subunit 3 (gamma) (CCT3) :

ggttctctctctccagaaggttctgccggttcccccagctctgggtacccggctctgcat cgcgtcgccatg (Seq ID No: 456)

Homo sapiens tubulin, alpha 1a (TUBA1A):
caacctctcctcttcgtctccgccatcagctcggcagtcgcgaagcagcaaccatg
(Seq ID No: 457)

Homo sapiens CD164 molecule, sialomucin (CD164):
ctttctcccgaacgccagcgctgaggacacgatg (Seq ID No: 458)

Homo sapiens cysteine-rich secretory protein 3 (CRISP3): ctctctctgcaccttccttctgtcaatagatg (Seq ID No: 459)

Homo sapiens SMYD family member 5 (SMYD5):

cggcctccatgtgcgacgtgttctccttctgcgtgggcgtggcgggccgcgcgcgggtct ccgtggaagtccgtttcgtgagcagcgccaaggtgaggtcggggcgggtcctgccgggag cctctccccagtccggccatg (Seq ID No: 460)

Homo sapiens kelch repeat and BTB (POZ) domain containing 10 (KBTBD10) :

ctgccttttacagctagacctgtgtgctgcaaggagctaaggccttcagtgtccccttc cttacccaggtttctcacagaatg (Seq ID No: 461)

Homo sapiens aldo-keto reductase family 1, member A1 (aldehyde reductase) (AKR1A1):

ccgccccttgcaccgcccacgtggccagcgccacctgcctcattgtgcccaggagttctc
caaacccgcgctgcggagtgagtgaccaagttccggccagttcgacctcgaggatccaga
ggtggagacggtactacctcccagctctgttttccatccccttcaggtccttcctcggga
ggcggcgaaggcggtccaccctgcgcgtgatcctttatgcccggcccctgcccctccctc
cgggtggaacttccccctcaccgccagacttaagctgaggatcgttggatctctggcggg
gtgcagaactgagcccaggccacagtaccctattcacgctctgtgcttgtgccaaggttt
caagtgatcctcccgcctcagcctgcccaggtgctgagattacatgtatgagccactgca
cctggaaaggagccagaaatgtgaagtgctagctgaaggatgagcagcagctagccaggc
aaaggggcaatg (Seq ID No: 462)

Homo sapiens TRK-fused gene (TFG):

tgttcttcccccacctgccacgtacagagcccaagttctcgctaggcttgttgggtcagc
gcgattggccggggcccgcgcgagcctgcgagcgaggtgcggcggtcgcgaagggcaacc
gaggggccgtgaccaccgcctccccgcgacgccccagtccagtggcctcgcgtccgccc
attcagcggagacctgcggagaggcggcggccgcggcctccgcaagccgtctttctctag
agttgtatatatagaacatcctggagtccaccatg (Seq ID No: 463)

Homo sapiens 3'(2'), 5'-bisphosphate nucleotidase 1 (BPNT1):

catccttctcaaaagacttattgacagtgccaaagctcggtactggacacaacgagggac
ctgggtctacgataacgcgcttttgctcctcctgaagtgtctttggtccaacgttgttcc
agagtgtaccatg (Seq ID No: 464)

Homo sapiens guanine nucleotide binding protein (G protein):

ttttctctctctctttcactgcaaggcggcggcaggagaggttgtggtgctagtttctct
aagccatccagtgccatcctcgtcgctgcagcgacacacgctctcgccgccgccatg
(Seq ID No: 465)

Homo sapiens major histocompatibility complex, class II, DM alpha (HLA-DMA):

caccctctcggggagggagttggggaagctgggttggctgggttggtagctcctacctac
tgtgtggcaagaaggtatg (Seq ID No: 466)

Homo sapiens transmembrane protein 50B (TMEM50B):

tctccttcctgcgcgcgcgcctgaagtcggcgtgggcgtttgaggaagctgggatacagc
atttaatgaaaaatttatgcttaagaagtaaaaatg (Seq ID No: 467)

Homo sapiens lactoperoxidase (LPO):

cagtctttcctgctaagcctcagcgtctcctccaagccacatcaaaatctttccttctgg
gcctttcccagaagtgaattcttgctggaaggtataaaagaccagctcctccaagcagag
caactccctggctgccgtgaaaagacaaggcactgggcagtgatg
(Seq ID No: 468)

Homo sapiens NEL-like 2 (chicken) (NELL2):
ctgcctttacaacagagggagacgatggactgagctgatccgcaccatg
(Seq ID No: 469)

Homo sapiens nucleobindin 1 (NUCB1):
cgccctctgcggtgaaggagagaccacactgccatg (Seq ID No: 470)

Homo sapiens paired box 9 (PAX9):

aagcctctttcatcggggcacagacttccttttacttcttccttttgccctctcgcctcc
tcctcctgggaagaagcggaggcgccggcggtcggccgggatagcaacaggccgggccac
tgaggcggtgcggaaagtttctgtctgggagtgcggaactggggccgggttggtgtactg
ctcggagcaatg (Seq ID No: 471)

Homo sapiens cyclin-dependent kinase 16 (CDK16):

cgccctttattcttgctcggcctcgccacagagagcaaatcagattggctgggcgacaac
ctcaaagggcggggctgcacacgttcactacgggaatgaggtagcggtggagggggcagt
tgggcggggataggccgtcctagctaaggtggtaaaggccaataactcttcaggctgcct
ctcctcgaaaagtcatcttctcgcgaacctttaaaatgccttcctccccaagcacctcaa

gggactagaactgagtgcttcatttgtctttttcctccttgcaaaagtcccgtttgcca
ccatggggatgtaccaagtgagaccgagtaggggaacgagtggtgattgacgcgccagg
ttactggccactgctcacctaggcgctagcaaacttctgccaagatcggaactgagtact
aaacagcctccacagttctccctggtgccgtctccggcttggcgccgcatcctcctctgg
gctcgcgatggccgcgtcccctcccgctgcggacgggtcctttggtacatg
(Seq ID No: 472)

Homo sapiens serpin peptidase inhibitor, clade E (nexin, plasminogen activator inhibitor type 1), member 2 (SERPINE2) :

ctgcctctttccggctgtgaccctcctcgccgccgccgcttggctgcgtcctccgactcc
ccgcgccgccgagaccaggctcccgctccggttgcggccgcaccgccctccgcggccgcc
ccctggggatccagcgagcgcggtcgtccttggtggaaggaaccatg
(Seq ID No: 473)

Homo sapiens pancreatic lipase-related protein 1 (PNLIPRP1):

aactcctttccccctgctgtgacgtacaggtgaggtaaacagtactgaagtccagggcgt
cggtgctcactgctctggcaatgcccggtgagactgaattatgtttaaatttattgtaga
tg (Seq ID No: 474)

Homo sapiens peripherin (PRPH):

ggctccttcccagcccccggcctagctctgcgaacggtgactgcccatccttggccgcaa
tg (Seq ID No: 475)

Homo sapiens RAD21 homolog (S. pombe) (RAD21):

gacccttttcccctccccgggccacccagcccgcccaactcccagcggagagcaaggttt
tcttctgttttcatagccagccagaacaatg (Seq ID No: 476)

Homo sapiens signal sequence receptor, delta (SSR4): ttttcttttcctctaggcagagaagaggcgatg (Seq ID No: 477)

Homo sapiens tissue factor pathway inhibitor

(lipoprotein-associated coagulation inhibitor) (TFPI):
ctccctctttgctctaacagacagcagcgactttaggctggataatagtcaaattcttac
ctcgctctttcactgctagtaagatcagattgcgtttctttcagttactcttcaatcgcc
agtttcttgatctgcttctaaaagaagaagtagagaagataaatcctgtcttcaatacct
ggaaggaaaaacaaaataacctcaactccgttttgaaaaaaacattccaagaactttcat
cagagattttacttagatg (Seq ID No: 478)

Homo sapiens ubiquinol-cytochrome c reductase binding protei n (UQCRB): gcttctctttctggtcaaaatg (Seq ID No: 479)

Homo sapiens mitogen-activated protein kinase kinase kinase 12 (MAP3K12):

ccgccttttgtgctgcggccgcggagcccccgagggcccagtgttcaccatcataccagg
ggccagaggcgatg (Seq ID No: 480)

Homo sapiens sushi-repeat containing protein, X-linked (SRPX) :

tggtctcttcggtctcctgccgcccccgggaagcgcgctgcgctgccgaggcgagctaag
cgcccgctcgccatg (Seq ID No: 481)

Homo sapiens aminopeptidase puromycin sensitive (NPEPPS):

cccccctctccctccctccttgcgggccctcctccccttccctcccctccgccccccttccc
cgtaggcagcccgcccgccagtccgcccgcaccgcctccttcccagcccctagcgctccg
gctgggtctctcccccgccccccaggctcccccggtcgctctcctccggcggtcgcccgc
gctcggtggatg (Seq ID No: 482)

Homo sapiens fibulin 5 (FBLN5):

tcgccttctgcccgggcgctcgcagccgagcgcggccggggaagggctctcctcccagcg
ccgagcactgggccctggcagacgccccaagattgttgtgaggagtctagccagttggtg
agcgctgtaatctgaaccagctgtgtccagactgaggccccatttgcattgtttaacata
cttagaaaatgaagtgttcatttttaacattcctcctccaattggtttaatgctgaatta
ctgaagagggctaagcaaaaccaggtgcttgcgctgagggctctgcagtggctgggagga
ccccggcgctctccccgtgtcctctccacgactcgctcggcccctctggaataaaacacc
cgcgagccccgagggcccagaggaggccgacgtgcccgagctcctccgggggtcccgccc
gcgagctttcttctcgccttcgcatctcctcctcgcgcgtcttggacatg
(Seq ID No: 483)

Homo sapiens lysophospholipase I (LYPLA1):
cgctcttccttccgcttgcgctgtgagctgaggcggtgtatg (Seq ID No: 484)

Homo sapiens high mobility group nucleosomal binding domain 4 (HMGN4):

tcgtcttctctgtcttagggctggtgctggccctgcccacgcctagggctccggcgcgtc
acgggcctcagctgggattcccgcgcccctcggacggccacgagactcggacatctttcc
aggaacagcgtgaggaggacagaagcacccaacaggactgctcaagccacctgcgaacac
tgctgctaccatg (Seq ID No: 485)

Homo sapiens eukaryotic translation initiation factor 3, sub unit M (EIF3M):

agttccctttccggtcggcgtggtcttgcgagtggagtgtccgctgtgcccgggcctgc accatg (Seq ID No: 486)

Homo sapiens Sec23 homolog A (S. cerevisiae) (SEC23A): cctcctcttgacgtggcagaggcggcgccagccatg (Seq ID No: 487)

Homo sapiens cartilage associated protein (CRTAP):

cgtcctctttcctttccttctccctccccttttcccttccttcgtcccttccttccttcc tttcgccgggcgcgatg (Seq ID No: 488)

Homo sapiens vesicle amine transport protein 1 homolog (T. californica) (VAT1):

ccgcccctcccgctggatcccgcagccgcggctcttcccgacgcgttccgccttccccag ctgtgcactctccatccagctgtgcgctctcgtcgggagtcccagccatg (Seq ID No: 489)

Homo sapiens importin 7 (IPO7):

gcttctctttcctttcgcgccggttgccgctgcggagcgcggcgggtccatgtgcgcagt gagtggcgctattcctggcccagtagcacccgagccccgggtttgaccgagtccgcgctg cgatg (Seq ID No: 490)

Homo sapiens ATG7 autophagy related 7 homolog (S. cerevisiae) (ATG7):

gctcctttgcgcacgcgcgccgcttcccagtggcaagcgcgggcaggaccgcgttgcgtc atcggggcgcgcgcctcagagagagctgtggttgccggaagttgagcggcggtaagtgag ccgcggcgggcgagggtgtagtggggtcttgctgggccggttttggaggcctggagtcaa ggggcgagctcgccagggagggcgagggtcacagcaagtctcaggatcctcctctgccag tttctgggtggtccttcctcctccagggactcactgattccggctggcgcccttcgtctg tagccgcgtcccctcagactggttcagtccggggtcttctgacttggaagctcgtgctga tttcctaagtcagcccctcctgtcctcttggtaggcagtgctcagaatcttcagtgttgg aacacgggagatgggacatttggattcccagcctggctgtgtctggatttgctgtctctg gcacgttccttccccatctaagctgcttttccatctgcaaaatggaatgataatccgcc atttgtttaagtgaggaggttaaataagtttactttctgagaaagaagattctcgattcc ttggttacagggttagaaactaatg (Seq ID No: 491)

Homo sapiens dynactin 2 (p50) (DCTN2):

cgctccctttgccgccgccttagcccgggacccgaacccagcctctcccctacccgaaca ccggccccggctccaccgaggcccgggtcccccagcccgtctcgccgccgccatg (Seq ID No: 492)

Homo sapiens acidic (leucine-rich) nuclear phosphoprotein 32 family, member B (ANP32B):

agccccctttccctccatggtttctctccgctcccgtgagtaacttggctccgggggct ccgctcgcctgcccgcacgccgcccgccacccaggaccgcgccgccggcctccgccgcta gcaaacccttccgacggccctcgctgcgcaagccgggacgcctctccccctccgccccc gccgcggaaagttaagtttgaagaggggggaagaggggaacatg (Seq ID No: 493)

Homo sapiens protein C receptor, endothelial (PROCR):

acttctcttttccctagactgcagccagcggagcccgcagccggcccgagccaggaaccc aggtccggagcctcaacttcaggatg (Seq ID No: 494)

Homo sapiens actin related protein 2/3 complex, subunit 1A, 41kDa (ARPC1A):

cgctccctctgggcttccgtcctccgcccgcgcccgacggagcctgttcgcgtcgactgc ccagagtccgcgaatcctccgctccgagcccgtccggactcccccgatcccagctttctc tcctttgaaaacactaagaataatg (Seq ID No: 495)

Homo sapiens chaperonin containing TCP1, subunit 4 (delta) (CCT4) :

aggccccttctccgcctccgcctcctcccgacgccggcgccgctttctggaaggttcgt gaaggcagtgagggcttaccgttattacactgcggccggccagaatccgggtccatccgt ccttcccgagccaacccagacacagcggagtttgccatg (Seq ID No: 496)

Homo sapiens Niemann-Pick disease, type C2 (NPC2):

gcttctttcccgagcttggaacttcgttatccgcgatg (Seq ID No: 497)

Homo sapiens phosphoribosylaminoimidazole carboxylase, phosp horibosylaminoimidazole succinocarboxamide synthetase (PAICS) :

acccctcttttctagagttctgcctcgcttcccggcgcggtcgcagccctcagcccactt aggataatg (Seq ID No: 498)

Homo sapiens ST6 (alpha-N-acetyl-neuraminyl-2,3-beta-galactosyl-1,3)-N-acetyl galactosaminide alpha-2,6-sialyltransferase 2 (ST6GALNAC2): ctcccttctgcctgggacgtcagcggacggggcgctcgcgggccggggctgtatg (Seq ID No: 499)

Homo sapiens polymerase (RNA) III (DNA directed) polypeptide C (62kD) (POLR3C):

aagccctttccgaggatggcaaaggatctgggaatgcttctccaaagatatgtggatgga
cgaaataggtctctggtgatactgaggcggggtggggacggggaggcaaagacttggctt
cttaggaattggaagaaataagtaaacaatgtttggtagcaatttgtaataaggaagtaa
tcataaaattaactacgtccgtttctgattgtgtcaactttgtcaaggagtagaagttta
agaattgaatactgtcctgcaaacaacgtaacctcatctcctgtttgacacaccctgttg
agaagcagtcctttacctcctaaatttctttttcgaaattatcatttcctttatggactg
agaataacactgcctgttcactcccaccgagctgtgaacagtgaccttaattcttccaag
cagggaagtgtagaaactaaggtctgtgacagaccgcaaaatcatctcccaatctttaag
gaaaatcagaatcacgcataatcccatagagataaatttgatgcatagtcttttcctatg
catacatttttcctttttttttacaataattgaattttttatatttttttcagcttgcttct
gtcacttaatatattatgagtaatttttttttggtttttttttgttttggagacagaatctc
gcactgtcgcccgggttggagtgcagtggcgcgatctcggctcactgcaacctctgcctc
ccggcttcaagcgattctcctgtctcagcctccctagtagctgggattacaggcacccgc
caccacgcccagctaatttttttgtgtgtttttagtagagaaggggtttcactatattgg
ccaggctggtctcaaactcctgacctcatgatacgcccacctcggtctcccaaagtgcta
ggattacaggcctgagccaccgcgccagcctattatgaataattttctacatgaatacgc
atcgtactaaataactttaaatgttggtgtagtatgccattgtatgggtatggcatcatt
tattgttagacgttagattgtttccactaagtcggtattataaagagaactaatgacttc
attattattagctttttctttctttggacacaatatccaaaaagaaattgttgtttcaaa
gatatgcaagattttttaaggcttttgatatgtattgtcaaattgccctccagaaagaat
acatgaatttacactcagcagctctgcttccagcgtgaaagactttctattgtaccattt
tggtgtttttttccctagctctcagactccccagtacaatg (Seq ID No: 500)

Homo sapiens influenza virus NS1A binding protein (IVNS1ABP) :

gtgtctcccggtcgcgcgtggaggtcggtcgctcagagctgctgggcgcagtttctccgc
ctgctgcttcggcgcggctgtatcggcgagcgagcgagttcccgcgagttctcggtggcg
ctccccccttcctttcagtctccacggactggcccctcgtccttctacttgaccgctcccg
tcttccgccgccttctggcgctttccgttgggccgattcccgcccgcttcctcctgcttc
ccatcgaagctctagaaatgaatgtttccatctcttcagagatgaaccagattatgatgc
atcattatcacagaagaaattcgtgtctatagcttttaaggacttgattacatcattttc
aagcctgatagttttggaatcaccattagagcttaagacacacctgccttcatttcaacc
acctgtcttcataccctgacgaagtgcaccttttaacactcctttgtccttggattactt
aagagttcccagaaatacatttgccaccaacagagtagccaaatttataaggaaaaatg
(Seq ID No: 501)

Homo sapiens thioredoxin interacting protein (TXNIP):

acccctctttttctccaaaggagtgcttgtggagatcggatcttttctccagcaattggg
ggaaagaaggctttttctctgaattcgcttagtgtaaccagcggcgtatatttttttaggc
gccttttcgaaaacctagtagttaatattcatttgtttaaatcttattttatttttaagc
tcaaactgcttaagaataccttaattccttaaagtgaaataatttttttgcaaaggggttt
cctcgatttggagctttttttttcttccaccgtcatttctaactcttaaaaccaactcag
ttccatcatg (Seq ID No: 502)

Homo sapiens ecotropic viral integration site 2B (EVI2B):

ttttcctttcttagccaaatcaccaaaatgtccagttagaacaagaatttagcattctgc
aaaagaagttaacagctgagataacgaggaaatattctgaaatg
(Seq ID No: 503)

Homo sapiens guanine nucleotide binding protein (G protein), alpha inhibiting activity polypeptide 3 (GNAI3):

ggttcttctgggcgctaagggagctgacggagagggccaccgcccagcaatagacggtgc
ctcagcctgccgagccgcagtttccgtggtgtgagtgagtccgggcccgtgtcccctctc
ccgccgccgccatg (Seq ID No: 504)

Homo sapiens polymerase (DNA directed), eta (POLH):

cggcccttcgcagcgggcgcgctgtcagacctcagtctggcggctgcattgctgggcgcg
ccgctctcgtctgatccctgctggggacggttgcccgggcaggatcctttacgatccctt
ctcggtttctccgtcgtcacagggaataaatctcgctcgaaactcactggaccgctccta
gaaaggcgaaaagatattcaggagcccttccatttccttccagtaggcaccgaacccag
cattttcggcaaccgctgctggcagttttgccaggtgtttgttaccttgaaaaatg
(Seq ID No: 505)

Homo sapiens solute carrier family 2 (facilitated glucose transporter), member 1 (SLC2A1):

cgctctctggcaagaggcaagaggtagcaacagcgagcgtgccggtcgctagtcgcgggt
ccccgagtgagcacgccagggagcaggagaccaaacgacggggtcggagtcagagtcgc
agtgggagtccccggaccggagcacgagcctgagcgggagagcgccgctcgcacgcccgt
cgccacccgcgtacccggcgcagccagagccaccagcgcagcgctgccatg
(Seq ID No: 506)

Homo sapiens zinc finger protein 138 (ZNF138):

gggtctttgtctcgctgcagcgggtgctgcaggtctggccttcacttttctgcgtcctct
tactcctagaggcccagcctctgtggcgctgtgatctggttattgggagattcacagcta
agacgccaggatcccccggaagcctagaaatg (Seq ID No: 507)

Homo sapiens ubiquitin specific peptidase 3 (USP3):

ctttctttgacgcaagggctcgagacgcagccgccgtcggccgagcgcccggctagaagc
gacaccagacggagcctccggagttcctccgcccccacctcgccgggtcctggagccgca
gtcctcccagctgccctcctcgtggccatg (Seq ID No: 508)

Homo sapiens calcium channel, voltage-dependent, gamma subun it 3 (CACNG3):

ctgtcttttctccagtttgagcgggggtgtcgggagcaggcggagagctttcctgcgagg
ctgtggaagcagtgaacactcttctcagcggctcgcctcccagcagtgctatttttttgcc

atccgccctcacccccagcacacgcgctcgcacacacacgcacgcacgcacacacacaca
cacacacactcacacagagacctctctgggtttctttgccttgagtctcccggggctgtg
agaagccaggcgcatctcaaaccgagctggcagctccaggctccggagccatgccctgca
cggaccctcgtctttaccacgctcctgaggaatgaaaggaacccagggaccctcagaagg
cagcagtgatgcggaccaaccccccggagcctgcacccttccgagggccataggcgaccc
agggaactggagagagctccagaaaggaaatcccagctttcccaaagtccctgtggatgc
tgacaaaaggagacctgaattttggaagagcctgtactaggttacccggctgcagagtg
atttcccctccggcactgactctccccctccaacccccagccgtccagagtaccatgaa
gaattatg (Seq ID No: 509)

Homo sapiens guanine nucleotide binding protein (G protein), beta 5 (GNB5): ttccctctccgctgcgtccccgcgcgaagatg (Seq ID No: 510)

Homo sapiens chaperonin containing TCP1, subunit 8 (theta) (CCT8) : cttcctccgcggtcttccgagcggtcgcgtgaactgcttcctgcaggctggccatg (Seq ID No: 511)

Homo sapiens prostaglandin E synthase 3 (cytosolic) (PTGES3) :

cgctctttccgcgcggtgcattctggggcccgaggtcgagcccgccgctgccgccgtcgc
ctgagggaagcgagaagaggccgcgaccggagagaaaaagcggagtcgccaccggagaga
agtcgactccctagcagcagccgccgccagagaggcccgcccaccagttcgcccgtcccc
ctgccccgttcacaatg (Seq ID No: 512)

Homo sapiens zinc finger protein 266 (ZNF266):

ttttcttcctggtggcgtttgggcttaatacagctttggcgaggtcggatgacgggtggg
agccagcggtggaaggggtggcgaaagtaccggtttgccccaggccgccgaggggcctcc
ttagagagaccttgcctgctccgctcgcgtccgccggggccgcgcgggtcctcctggcgc
cgccaggttcaaaaagccactcgagttgtcactgcgacggccctgggccaggagccgttt
cgggatctgtcaaacaacgagttttcgtcgttcgaatcaggttgactggtccttcatccc
cccaatctcccgtacctggcgagtccagctcgtcgcggcaatgctaagaaaagagtgata
tgcaagctgagaccaaaaatatggtatgatttagccatactgaaggggaaggaaataaga
gctgggcaaagcattctgtgaattggctgactccacttctatggtgagagagaggagtgc
atcaaagattactcccagtagagatggtttcagcatgttggccagtctggtctcagactc
ctgacctcaagtgatccacccacctcggcctcccaaaatgctgggattacaggtataagc
cactgtgcctggccaaagataccgttaaccctggataaagagaatggaggttacctctgt
ccgtgtagattcctaagctgtcctggagtgatccttggagtaaaggaaaggtgctttgaa
gcacattcagccatcagccctgtgggatggcagccactgatttgtcctatggtctttaca
gggacccagtctgccttcaagaaaagacagaagtagaaagggtggtggctgactgtctga
caaattgttatcaggtatgcaggaagtatatccttctccaaaatatcatacttgcatcac
caggtagacacatttccttctacacagaattatcttcagagcttcttaaagcaaataaag
cctgcttcaaggactgagtccctagtcgaattcccggaaggagtggagcctgtcatattg
tgtttatctagcatctgctcaagagtgtgctgcagtggagggaaatcagatgacctccca
gtctggttgtgttacatacaatcatgtgtaagaagtgccattcaagccgtgtcactggag
gggactgacagtgagattcagtgacttttgatgatctggctgtggacttcaccccagaag
aatggactttactggacccaactcagagaaacctctacagagatgtgatg
(Seq ID No: 513)

Homo sapiens methylenetetrahydrofolate dehydrogenase (NADP+ dependent) 2, methenyltetrahydrofolate cyclohydrolase (MTHFD2): gcttccctcccggcgcagtcaccggcgcggtctatg
(Seq ID No: 514)

Homo sapiens chemokine (C-C motif) receptor 9 (CCR9):

cttcctttctcgtgttgttatcgggtagctgcctgctcagaacccacaaagcctgcccct
catcccaggcagagagcaacccagctctttccccagacactgagagctggtggtgcctgc
tgtcccagggagagttgcatcgccctccacagagcaggcttgcatctgactgacccacca
tg (Seq ID No: 515)

Homo sapiens heat shock 105kDa/110kDa protein 1 (HSPH1):

cctccccttttgggtcggtagttcagcgccggcgccggtgtgcgagccgcggcagagtga
ggcaggcaacccgaggtgcggagcgacctgcggaggctgagccccgctttctcccagggt
ttcttatcagccagccgccgctgtccccgggggagtaggaggctcctgacaggccgcggc
tgtctgtgtgtccttctgagtgtcagaggaacggccagaccccgcgggccggagcagaac
gcggccagggcagaaagcggcggcaggagaagcaggcaggggggccggaggacgcagaccg
agacccgaggcggaggcggaccgcgagccggccatg (Seq ID No: 516)

Homo sapiens StAR-related lipid transfer (START) domain containing 10 (STARD10):

tggtcctttcttttatgattcacaaggaatgaccctcttcatcgcctctcctaattcagt
cctcacaacagtcctttttacaaatgggacaacaggttagaggaagtcaggcagatttcca
gcatcatagagagtaaaggaccagggaaggatcaggattcaaggactgcacccaggctct
gcttccagcttgctgtgtgactttgggtaatttgttcccttagggaactgagctttctc
atttgtaaatgcaaacaggctgttgggaggatcaaatgagatccaggggtgaaaacagct
tagtttactttcaggaatttacccacgcggtatataaaggcaaaatattattatagtcag
gtgattgtagattgaggaacccatttcctcattctgcaaattgcaaacctgagggcccaa
agagggacaggggcttgccccaggtctcagcaggctgtgagcaagagctaaagcctaatc
ctcctgcctttgggcctggagcccttccttgtaccccaggggtcagtgtctttgttggat
acaggcttagattgactgactgtaccctgagaacctaggggagtccctgttcccaattct
tctcctacccccaccttggcctgatggaggaagaccctgctgtgttgagatgagcaccag
agccaagaagctgaggaggatctggagaattctggaggaagaggagagtgttgctggagc
tgtacagaccctgcttctcaggtcccaggaaggtggcgtcagcatctgcagccgcgtcga
cgttgtcggagcctccgcggaggacccaggagagccggactaggaccagggccctgggcc
tccccacactccccatg (Seq ID No: 517)

Homo sapiens UTP14, U3 small nucleolar ribonucleoprotein, ho molog A (yeast) (UTP14A):
ctttccttcggcttccgttcttggtccatgtgagagaagctggctgctgaaatg
(Seq ID No: 518)

Homo sapiens SUB1 homolog (S. cerevisiae) (SUB1):

ggttctctgtcagtcgcgagcgaacgaccaagagggtgttcgactgctagagccgagcga
agcgatg (Seq ID No: 519)

Homo sapiens minichromosome maintenance complex component 5 (MCM5) :

ccgcctcttgtttttcccgcgaaactcggcggctgagcgtggaggttcttgtctcccctg
gtttgtgaagtgcggaaaaccagaggcgcagtcatg (Seq ID No: 520)

Homo sapiens RNA binding motif (RNP1, RRM) protein 3 (RBM3):

tactctttatcaatcgtcttccggcgcagccccgtccctgtttttgtgctcctccgagc
tcgctgttcgtccgggtttttacgttttaatttccaggacttgaactgccatg
(Seq ID No: 521)

Homo sapiens KDEL (Lys-Asp-Glu-Leu) endoplasmic reticulum protein retention re ceptor 1 (KDELR1):

ctccccctctcgctctcctccctcttcccggctccagctccgccgccagctccagccttt
gctccccctcccaaagtcccctccccggagcggagcgcacctagggtccctcttccgtcc
ccccagcccagctacccgttcagaccagcagcctcgggggcacccccccgccagcctgc
ctccctcccgctcagccctgccagggttccccagccatg (Seq ID No: 522)

Homo sapiens StAR-related lipid transfer (START) domain containing 3 (STARD3):

agatcttcttccgctctgaggcgctactgaggccgcggagccggactgcggttggggcgg
gaagagccggggccgtggctgacatggagcagccctgctgctgaggccgcgccctccccg
ccctgaggtgggggcccaccaggatg (Seq ID No: 523)

Homo sapiens heterogeneous nuclear ribonucleoprotein A0 (HNRNPA0) :

cggcctctttgtgtggtgcccagataggggagcggaggtggcggcggcggtagcggt
ggccttggttgtcttccagtctcctcggctcgccctttagccggcaccgctccccttccc
tccccccttcctctcttccttccttccctccccttccctttttcccttccccgtcggtgag
cggcgggggtggctccagcaacggctgggcccaagctgtgtagaggccttaaccaacgat
aacggcggcgacggcgaaacctcggagctcgcagggcgggggcaaggcccgggccttgga
gatg (Seq ID No: 524)

Homo sapiens chromobox homolog 1 (CBX1):

ggctcttttgttcggctgaggggagggccgttggccggggcctgcggtacgccgcttcag
tgagggacgccactgcggccacccggcttgctgccttcctgggcgccactcccccaggcg
acccgacgcgacgcgccagcagcgcagcaccgattcctctcggctcttgggcgctgctc
tgaggtgaggagcccgctggaggcgggagagctgggggagggggcgcggcggcggcggcg
gcgggagccctgcgtgagggaacgcgctttcgaggcggaggttaggagcggggagcgcgc
ccgggtccagcgtcctgcttctccgcttcccgcgctgagctcttcgcctgtcgctgaggc
gtcggtgccagctgcgtgaaggatggagagggcggggcgcgaatcctgagccagagactg
agtgcttgggggtgggccgagcacttgggggccgctcttcggggcccgggtggtctggaa
caatgttgcttggctgggcggctgcgggatagggcggaaggggacaggcttgaggcttgg
ataggcgtgaggaggcgcatacgaccgcacaacccgaggtttgtaactgtattcggaaga
cgccgggtccggctgggactgccagaggaacctggctttgcaggactacggaggagtaac
gtcgagtgaattggaagagggcccagggccgcacaagcagcgtcaccctttacaccagaa
agctggcgggcactatg (Seq ID No: 525)

Homo sapiens myeloid/lymphoid or mixed-lineage leukemia (trithorax homolog, Drosophila); translocated to, 11 (MLLT11) :

cgcccttcttaggaggggctgcattgcaggggagagtgaactgacagactcagtcactg
aagagggaaaaggagtgagaagacaaagccgtcaaagccccaacagctttgtatttctcc
agcccggcgcagaccccggagctcccgaggcactccctccatctttggaacacgccagta
attgattgataacaggaagctatg (Seq ID No: 526)

Homo sapiens interferon-induced protein 44-like (IFI44L):

ttttctttctttcctagagtctctgaagccacagatctcttaagaactttctgtctccaa
accgtggctgctcgataaatcagacagaacagttaatcctcaatttaagcctgatctaac
ccctagaaacagatatagaacaatg (Seq ID No: 527)

Homo sapiens cyclin I (CCNI):

acttcttcctcccttcccctctcttcccctccctccccagccttccccgcgagcggacgc
ggcagcgcctctgtctcgctttttcttatttttccccctttcccctttctttttttttt
tttcttttcttttctcccctcccccctttcaccatttcccctcggaggcgctttccccg
ggcaggggcagagccggtctcacccccgcctctccccggcccccgccgccctatggcga
gagggagccccctcccaacccgggctcgagcggcggcggcctcaggccgggggtcatcat
ggaactaattcgctgaccgacccagcggccgcagccgtgcgtcccgctcgagcgccagcg
cccgcgcccgcgcccccgatccgcttcccctttctccctcctcagttggccgagtcgtc
ccgcgcgcaccgcctccgcgcgcctatgagaatgaggtggtaacgggcccccggatgacc
ccgcgtcaccactgtgaggcctacagctctgccggggaggaggaggaggaggaagaggag
gagaaggtagctacagcaagctgggtagcaggcagatccaaaggatatcatg
(Seq ID No: 528)

Homo sapiens methionyl aminopeptidase 2 (METAP2):
cattccctcgcgctctctcgggcaacatg (Seq ID No: 529)

Homo sapiens leukocyte immunoglobulin-like receptor, subfami ly B (with TM and ITIM domains), member 4 (LILRB4):

gtctctttgtcctgccggcactgaggactcatccatctgcacagctggggcccctgggag
gagacgccatg (Seq ID No: 530)

Homo sapiens destrin (actin depolymerizing factor) (DSTN):

gggtctctcggtcccgcagccgtgaggaggacggtctgcatactcgctgcccgccggctc
cctcccccgcgtccctgcgaccgccgcggcgaagatg (Seq ID No: 531)

Homo sapiens eukaryotic translation initiation factor 2D (EIF2D) :

gggccctttcgcggccgggccccagcatggctgcccccacggctgagggcctggcagct
gctgcgccctcgctttcttgacattccctggcttctgtgctctcttccccaggccacccc
agcagacatg (Seq ID No: 532)

Homo sapiens histamine N-methyltransferase (HNMT):
ctgtctttctcagaaaaccaaatatg (Seq ID No: 533)

Homo sapiens ras-related C3 botulinum toxin substrate 1 (rho family, small GTP binding protein Rac1) (RAC1):

gtttctctgcagttttcctcagctttgggtggtggccgctgccgggcatcggcttccagt
ccgcggagggcgaggcggcgtggacagcggccccggcacccagcgccccgccgcccgcaa
gccgcgcgcccgtccgccgcgccccgagcccgccgcttcctatctcagcgccctgccgcc
gccgccgcggcccagcgagcggccctgatgcaggccatcaagtgtgtggtggtgggagac

ggaaacaagaatctcagtgtaacccgagcaaaatcgcgcgtctcagcgttgcttgtatag
agctgtaggtaaaacttgcctactgatcagttacacaaccaatgcatttcctggagaata
tatccctactgtctttgacaattattctgccaatgttatg (Seq ID No: 534)

Homo sapiens signal recognition particle 72kDa (SRP72):
tcgtctcctccaagatg (Seq ID No: 535)

Homo sapiens zinc finger protein 33B (ZNF33B):

ccgcctttcctttgtttgtctcacgttttgcgtgggaggcggtcccgggatttcagggg
tctaccggctctcttatggcgaatgcaacccgaagagagagtgagctgtatcttcagagt
tgtctccgtctttccaagaacagaacaaaatg (Seq ID No: 536)

Homo sapiens zinc finger protein 16 (ZNF16):
gcctcctttccaagcgcgacccgttgaggtccttgtcatg (Seq ID No: 537)

Homo sapiens zinc finger protein 33A (ZNF33A):

ccgcctttcctttgtttttctcaggttttgcgtgggaggcggtcccgggatttcaaggg
tctacgcgcttttctatggcgaatgcaacccgacgagggagtgggctgtatcttcagagt
tgtctccgtctttccaagaacagaacaaaatg (Seq ID No: 538)

Homo sapiens butyrophilin, subfamily 3, member A3 (BTN3A3):

ctttctttttcctttcttcggaatgagagactcaaccataatagaaagaatggagaacta
ttaaccaccattcttcagtggctgtgattttcagaggggaatactaagaaatggttttc
catactggaacccaaaggtaaagacactcaaggacagacatttttggcagagctgctcac
tccttgctcagctcagttttctgtgcttggaccctctgggcccatcctggccatg
(Seq ID No: 539)

Homo sapiens butyrophilin, subfamily 2, member A2 (BTN2A2):

ctctttgggatgctttgttgtctggtggtgactgtgcccatgggtgagttgtatcggaaa
atcgtcatgtgaggatcagaggggaaaagaaaacagaggcctctggtctctgcctgccct
gggtgctcatg (Seq ID No: 540)

Homo sapiens nudix (nucleoside diphosphate linked moiety X)-type motif 21 (NUDT21) :

acgcctcctcttgcgctgtcctgttaatggcgggcagtagccgctgaggggattgcagat
aaccgcttcccgcacggggaaagtctaccctgcctgccactttctgctcgccgtcagcgc
cggagctcgccagcatg (Seq ID No: 541)

Homo sapiens stathmin-like 2 (STMN2):

tgctctttctctagcacggtcccactctgcagactcagtgccttattcagtcttctctct
cgctctctccgctgctgtagccggaccctttgccttcgccactgctcagcgtctgcacat
ccctacaatg (Seq ID No: 542)

Homo sapiens katanin p60 (ATPase containing) subunit A 1 (KATNA1) :

caccctcttccgccgctcccgcccagcgacctcgctcccggggcgacgccccgcgtgcgc
cagagtcgccgaggtcgtccccggcaccggaagtgaccctggcgggtttgtcttcaaatt
ctcggcgagcaggagccgcgccggcaggtggtgttgacgattgaactgggcagtactggg
gccgtgagcggagagcaaagtgggctggactgggtcaggccctccttcctcgctgccggg

atctccactccgccaatcccctgtgcctggcgttgggcggtttcccgaggagcttgggcc
gccgcagcttacagttgaacatg (Seq ID No: 543)

Homo sapiens butyrophilin, subfamily 3, member A2 (BTN3A2):

ctttctctttttcctttcttccggatgagaggctaagccataatagaaagaatggagaat
tattgattgaccgtctttattctgtggctctgattctccaatgggaataccaaggatg
gttttccatactggaacccaaaggtaaagacactcaaggacagacatttttggcagagca
tagatg (Seq ID No: 544)

Homo sapiens CLK4-associating serine/arginine rich protein (CLASRP) :

cggcctttcatttccgcttccggtgcgggccgcgcgcgagcgcagcggtgggaggcggcg
accagccggttgaggccccaggcttggcctcaccacaatg (Seq ID No: 545)

Homo sapiens clathrin, light chain A (CLTA):

ctccctcctggcgcttgtcctcctctcccagtcggcaccacagcggtggctgccgggcgt
ggtgtcggtgggtcggttggtttttgtctcaccgttggtgtccgtgccgttcagttgccc
gccatg (Seq ID No: 546)

Homo sapiens NADH dehydrogenase (ubiquinone) flavoprotein 1, 51kDa (NDUFV1):

gcgtctctatcgcgccagttcctcagcctcagtgctatgaaggtgacagcgtgaggtgac
ccatctggcccgccgcgatg (Seq ID No: 547)

Homo sapiens signal sequence receptor, gamma (translocon-associated protein gamma) (SSR3):

gggcctttgcccgccttggcggccggctctacgttccctgttctcgcctgcagctccgcc
atg (Seq ID No: 548)

Homo sapiens valosin containing protein (VCP):

gcttcccttccgatgattcggctcttctcggctcagtctcagcgaagcgtctgcgaccgt
cgtttgagtcgtcgctgccgctgccgctgccactgccactgccacctcgcggatcaggag
ccagcgttgttcgcccgacgcctcgctgccggtgggaggaagcgagagggaagccgcttg
cgggtttgtcgccgctgctcgcccaccgcctggaagagccgagccccggcccagtcggtc
gcttgccaccgctcgtagccgttacccgcgggccgccacagccgccggccgggagaggcg
cgcgccatg (Seq ID No: 549)

Homo sapiens zinc finger protein 195 (ZNF195):

gggcctttgtcccgacagagctccacttcctgtccccgcggctctgtgtcccctgctagc
cgtaggtcgtgtgacccgcaggcaccgggagatccagaagtgaaacgccaggctctctgg
aggccaggagatg (Seq ID No: 550)

Homo sapiens testis-specific kinase 2 (TESK2):

cagtctttcgcggcccgggagctcagcagagctaccagctgccctgttggcttcgctggt
cggatcgtcctcctggccccgccaaacaggcgggggggagcggccccgactgtggggccat
ggcagtagtctcctcgttcgccgccgccgctagcctagctgagtcgccggcttctgcgct
aggggctcccaccgcctccgcaggctaaggagccgctgccaccaacgagctgtgagggtt
actatgctccctctttgccgccgtctcctcctcttgcccgcgcaggcacccctctggctg
ctcagtcctgcctcagtgtcaaaccagaagagaagtaaaattcaacaaaaatttatgtgt

ggagttccttcttaaaagaagaaaaaagtgattatttagactatg
(Seq ID No: 551)

Homo sapiens family with sequence similarity 107, member A (FAM107A) :

agccctccttgctagtctgggacttcccggtggagtgaggaacccagcaacacgctcctg
acttcccttcccaaggactcgacctgagaaggacacagcagtctctgaatttcatgctct
cctctttgatgtgaagaaaatgaaaagctgaacagttgtggaactgtggatagagttaga
caataaggccgccatg (Seq ID No: 552)

Homo sapiens serine/threonine kinase receptor associated pro tein (STRAP):

ccctccctccctttccctccctcgtcgactgttgcttgctggtcgcagactccctgaccc
ctccctcacccctccctaacctcggtgccaccggattgcccttcttttcctgttgcccag
cccagccctagtgtcagggcgggggcctggagcagcccgaggcactgcagcagaagagag
aaaagacaacgacgaccctcagctcgccagtccggtcgctggcttcgccgccgccatg
(Seq ID No: 553)

Homo sapiens mitochondrial ribosomal protein L3 (MRPL3):

ctttctttccgtcgcagagagcatcggccggcgaccgttccggcggccattgcgaaaact
tccccacggctactgcgtccacgtggcggtggcgtggggactccctgaaagcagagcggc
agggcgcccggaagtcgtgagtcgagtcttcccgggctaatccatg
(Seq ID No: 554)

Homo sapiens zinc fingers and homeoboxes 1 (ZHX1):

ctcccttcccccctccgcccccggacggccgctggggcgcgcgcctctcctcgcaccccca
ccctgagtccccacactccgcggggccaccgagctgctgaggcccctttgcgggcccgcc
gagcggttccgggtttagggttcacaggtcagagttgactccctgaaaagtgcagccggt
ttgaaatgcaagatggcggcggcgtggcgctgagaggcgcggcggcccctgcaggagaag
acagactgctgctttggacctgttggtaatgatggcctgagctaaacatctaactagaag
ggataccccttccatttcaaagaacagaatgctaaggaagctgtggcaagtgattggagtt
gtgcttcaaaaatttcagaaattcagcagtattttatctgccaacaataagctctttact
tgattgcaccatgagaaagctgctaatgagacttgttgagcacaaaaatggacttgaaga
accaaaagccattgttttcaaatgaagaacactgaacagttttaagcctcgatgcttttt
aatcaccactgagcttttcctcataacatcagaatg (Seq ID No: 555)

Homo sapiens calcium binding protein P22 (CHP):

ccttccttccctccctccttccctcctgtcgccgtctcttctggcgccgctgctcccgga
ggagctcccggcacggcgatg (Seq ID No: 556)

Homo sapiens ecdysoneless homolog (Drosophila) (ECD):

ctttctctcaggatttccgctggcttcaggttccggtcaggcgtcgggacagagcctgat
ccaggcttcggcggccggtggcagctctcgatcagctctcgcagtcggagaggcggctaa
ggaaaggtgccacagcagagacgcgaaggagaggccctagaacctttcaaagaagaatg
(Seq ID No: 557)

Homo sapiens V-set and immunoglobulin domain containing 4 (VSIG4) : gagcctctttggtagcaggaggctggaagaaaggacagaagtagctctggctgtgatg (Seq ID No: 558)

Homo sapiens prohibitin 2 (PHB2):

tgccctttctttcgccagccttacgggcccgaaccctcgtgtgaagggtgcagtacctaa
gccggagcggggtagaggcgggccggcacccccttctgacctccagtgccgccggcctca
agatcagacatg (Seq ID No: 559)

Homo sapiens signal transducer and activator of transcriptio n 1, 91kDa (STAT1):

ctgcctttctcctgccgggtagtttcgctttcctgcgcagagtctgcggaggggctcgg
ctgcaccgggggatcgcgcctggcagaccccagaccgagcagaggcgacccagcgcgct
cgggagaggctgcaccgccgcgcccccgcctagcccttccggatcctgcgcgcagaaaag
tttcatttgctgtatgccatcctcgagagctgtctaggttaacgttcgcactctgtgtat
ataacctcgacagtcttggcacctaacgtgctgtgcgtagctgctcctttggttgaatcc
ccaggcccttgttggggcacaaggtggcaggatg (Seq ID No: 560)

Homo sapiens heat shock protein 90kDa alpha (cytosolic), class B member 1 (HSP90AB1):

agctctctcgagtcactccggcgcagtgttgggactgtctgggtatcggaaagcaagcct
acgttgctcactattacgtataatccttttcttttcaagatg (Seq ID No: 561)

Homo sapiens cancer susceptibility candidate 3 (CASC3): cgttctccgtaagatg (Seq ID No: 562)

Homo sapiens nuclear cap binding protein subunit 2, 20kDa (NCBP2): gcttctctgcactatg (Seq ID No: 563)

Homo sapiens non-POU domain containing, octamer-binding (NONO) :

cgctcttttctcgggacgggagaggccgtgtagcgtcgccgttactccgaggagatacca
gtcggtagaggagaagtcgaggttagagggaactgggaggcactttgctgtctgcaatcg
aagttgagggtgcaaaaatg (Seq ID No: 564)

Homo sapiens lectin, galactoside-binding, soluble, 9 (LGALS9) :

atttctttgttaagtcgttccctctacaaaggacttcctagtgggtgtgaaaggcagcgg
tggccacagaggcggcggagagatg (Seq ID No: 565)

Homo sapiens chaperonin containing TCP1, subunit 5 (epsilon) (CCT5): cggtctccgccggttgggggaagtaattccggttgtt-gcaccatg (Seq ID No: 566)

Homo sapiens haloacid dehalogenase-like hydrolase domain con taining 1 (HDHD1): cttcctcctcgcccccacccagacccagaaggcgccaccatg (Seq ID No: 567)

Homo sapiens glutamate dehydrogenase 2 (GLUD2):

cttccttcctagtcgcggggagtctgagaaagcgcacctgttccgcgaccgtcacgcacc cctcctccgcctgccgcgatg (Seq ID No: 568)

Homo sapiens general transcription factor IIIC, polypeptide 3, 102kDa (GTF3C3):
ggttctctgtcccggttcctggggttgcacagacagaccctgtaaacatg
(Seq ID No: 569)

Homo sapiens general transcription factor IIIC, polypeptide 5, 63kDa (GTF3C5):

gggtccctcgctggctagtaggagagactggtgcttgccccgcccggtggactaactcgc ttaattttaaataaaaagtcgaggacacggcggtcgttttcccgaagacatgggccctcc catgggccatttgctccctggaggccctcgcgtcttgctgagcccggggagttaggatga cgcgagcggtgagggagcccggaacgattccttcgcggaacaattgaggcgaggcctttg ggagtactttgtgggacggaccctggcgggccctgccagacgcacagggatg (Seq ID No: 570)

Homo sapiens ancient ubiquitous protein 1 (AUP1):

ccgccttcccaagagcccctgcggccgggcgcgaaaatggcggcggcggcgacggccggg cgctcctgaagcagcagttatg (Seq ID No: 571)

Homo sapiens coatomer protein complex, subunit gamma 2 (COPG2) :

cggccttcctgcagcctcttccgctcgccggctgcggcgcctgggacggttgcggtgggt ctgggcgctgggaagtcgtccaagatg (Seq ID No: 572)

Homo sapiens apoptosis antagonizing transcription factor (AATF) :

cggtctctggcggagtcggggaatcggatcaaggcgagaggatccggcagggaaggagct tcggggccgggggttgggccgcacatttacgtgcgcgaagcggagtggaccgggagctgg tgacgatg (Seq ID No: 573)

Homo sapiens integrator complex subunit 6 (INTS6):
tctcctctttctccaccacctcgggccccggtgtccccggccagcactatg
(Seq ID No: 574)

Homo sapiens F-box and leucine-rich repeat protein 4 (FBXL4):

tcttccttccgggtcgcgctaggccgggcttgcggcggttgtgccgcatctagagagtcg gggagccgcccccgcacccaggccttctcgcgctgcctggtcgctggtgaagcccgcggc gcgcgcctctcccggaccctgcagggtaaaagaatgtcacatgtcagcatttgtacctga agtcagcatgcaaagttcagggtacctggatgaatgccaacttttgcatttcccatgtgt atcctgtgaccattctatctgggaacatccttcaaagagttcatgcatcttactgaggac acctgaccttttgaagcttcataattcacatctagatg (Seq ID No: 575)

Homo sapiens guanine nucleotide binding protein (G protein), gamma 3 (GNG3):

gctccttctagcatccttcatccttcaggtaccagccatccagacagtgcttgagctgca gaaactgagaccagacctctggcctggccctccccaggggcctcctttcgtatagtcact gcttctgcatcagatactttcagctgcaactccctactgggtggggcacccatttcaggc agaaggttttggtaccctccactgaccctacacccagggctgctactgccgcttgtggct tcaggatg (Seq ID No: 576)

Homo sapiens histidyl-tRNA synthetase 2, mitochondrial (putative) (HARS2): aggccttttgttcctgtcccggaaagccggcgtcctgccgcgcgatg (Seq ID No: 577)

Homo sapiens interleukin enhancer binding factor 3, 90kDa (ILF3) :

cctcctcctcctcttctcgccattgcagttggacccagcagcccggcgcgcaccgcgtgg cttttggggggcagaccccggcgggctgtggcaggagggcggcggcggcggctgcggtcga agaaggggacgccgacaagagttgaagtattgataacaccaaggaactctatcacaattt gaaaagataagcaaaagtttgatttccagacactacagaagaagtaaaaatg (Seq ID No: 578)

Homo sapiens polymerase I and transcript release factor (PTRF) :

gtttcctctgctctccgctctcgcccgctagctctcctcccttccgctcctgcttctctc cgggtctcccgctccagctccagccccacccggccggtcccgcacggctccgggtagcca tg (Seq ID No: 579)

Homo sapiens 5'-3' exoribonuclease 2 (XRN2):

tgccctctgccgctgctcccgtctctttggttacgctcgtcagccggtcggccgccgcct ccagccgtgtgccgctatg (Seq ID No: 580)

Homo sapiens 2-hydroxyacyl-CoA lyase 1 (HACL1):

ccgcctcttccttcccgttgtttaaggcagttggttgccctcctgtccgtcagaggtgca gtaccagaggtggcgtgctgccgatttcgcgtttgccttgctggatgattccgcttgttt gccggctgcgtgagtgcttagagcttttcggtggaagatg (Seq ID No: 581)

Homo sapiens zinc finger protein 346 (ZNF346): ggctctctaccggtgagggtttgcggggaagatg (Seq ID No: 582)

Homo sapiens microtubule-associated protein, RP/EB family, m ember 3 (MAPRE3):

cagtctctgtgcgttgaagccggagaccgcggcggcctcagcgaggaccctccgccccgg agccgccggccggagccgcagcctctgccgcagcgcccccgccacctgtcccctccccct ccgcctccgccggagccgcctcgtgcactctggggtatg (Seq ID No: 583)

Homo sapiens splicing factor 3b, subunit 3, 130kDa (SF3B3):

gtgcctttttccgccgcgcgccaccagaatgtccctgtcttgaggtctaatggcggacgc cagtatgttggagttggtggtggcttaagttttgaagggaggtagcatccgttggatatc cacaccatccttctcgctgcaggctttcttggactccgtactgttggtgtaaccaaggcc tggaggtctgggtggctcaggtttcctgcagccatg (Seq ID No: 584)

Homo sapiens spondin 2, extracellular matrix protein (SPON2):

ctgcctctcgctggaggccaggccgtgcagcatcgaagacaggaggaactggagcctcat tggccggcccggggcgccggcctcgggcttaaataggagctccgggctctggctgggacc cgaccgctgccggccgcgctcccgctgctcctgccgggtgatg (Seq ID No: 585)

Homo sapiens solute carrier family 13 sodiumsulfate symporters), member 4 (SLC13A4):

ttttcttttctgctttgcaggcccaggctcaaggcaaattataagtagggaaccaatttg agggaaagacatgtgaacagagttaaggtaccacgtcctgggagcgaccagcagccccac ctgaagtccgcatgcaactctgacaagctcaggtgcttgttttaaggaaaggggctacta gagtcttaccaacagcgagcccaggtgggagatgaaacaggtactccccaaaataggtca tccgagggaggaaaactgatggagagcacaatgtgctctgagcgtttttaatgtttttaa gcttttaaatgatttcttcaaggccgagcagcagcagcaaaggtgtggcttaaaggatta aggggggtttctgctgacacctagaatgaagttactctattactaatcaagccgagaggag gcccactatgcccccgtttatcatcctttcccagttcctttttgctggtcacaaaacgat gctcatcaatcccacctaaagcaggaggccaggagcccagcctcttgtagaaacagcgag ggtataactgccctcccgttctgcccccaagacgaaggaggactctcggaagccaagaaa ggtttaagaagtctttctggatagagagcagtgcccaggcaggaagcctttcgccggcag agcggggtccaaggacgagctggagaggacagaggcgcgatg (Seq ID No: 586)

Homo sapiens PRP6 pre-mRNA processing factor 6 homolog (S. cerevisiae) (PRPF6): attcctttccttcctagccttggtcgtcgccgccaccatg (Seq ID No: 587)

Homo sapiens eukaryotic translation initiation factor 3, sub unit K (EIF3K):

ccacctcttcctgttcccgtccttgaggacgccgtgccgggtcagtgttagcctccagcc ctggttgtggaaggcgacagaagtcatg (Seq ID No: 588)

Homo sapiens ataxin 10 (ATXN10):

ccccctcccccgcggcgccgtctcctcctcccgcctgaggcgagtctgggctcagcctag agctctccggcggcggcgcagcttcagggcagcgcgggctgcagcggcggcggcggttag ggctgtgtagggcgaggcctccccccttcctcctcgccatcctactcctccctcctcgtca tcctccccccttcgtcctcctcgccttcctcctcctcgtcaggctcgacccagctgtgagc ggcaagatg (Seq ID No: 589)

Homo sapiens secretogranin III (SCG3):

cttccttcctcacttcctctgcaggagggagcgagagtaaagctacgccctggcgcgcag tctccgcgtcacaggaacttcagcacccacagggcggacagcgctcccctctacctggag acttgactcccgcgcgccccaaccctgcttatcccttgaccgtcgagtgtcagagatcct gcagccgcccagtcccggcccctctcccgccccacacccaccctcctggctcttcctgtt tttactcctccttttcattcataacaaaagctacagctccaggagcccagcgccgggctg tgacccaagccgagcgtggaagaatg (Seq ID No: 590)

Homo sapiens polymerase (DNA directed), mu (POLM):

cttccttccgtctcgctcggagtttccctctgcgttcgctccgcgctgctggaggctgtc gtcccaatg (Seq ID No: 591)

Homo sapiens epsin 1 (EPN1):

cctccttctgttgcttcccgtctcctcggcggctcccctcccccgcccggctctccgcgc
cccttctgggcggcggggcggcggagccgtcggcgtgcggccctccttgcgttcgtgcgt
gcgcccgtggcccggcgcacgtcccgcgacaccgaggccgagcggggcaggggctgacc
gccatgacccccagagcccggcgtgaggggccgagatgcggtgacctgccagcacctg
ccgcagccttcgtccgggagtcgccccatctctccacgcatcggggccctgtgccccttg
ctgctgcagccgggcaccatg (Seq ID No: 592)

Homo sapiens Sec61 alpha 1 subunit (S. cerevisiae) (SEC61A1) :

gtgtctctcggcggagctgctgtgcagtggaacgcgctgggccgcgggcagcgtcgcctc
acgcggagcagagctgagctgaagcgggacccggagcccgagcagccgccgccatg
(Seq ID No: 593)

Homo sapiens Obg-like ATPase 1 (OLA1):

cgttctctcctccttcctccccgcctccagctgccggcaggacctttctctcgctgccgc
tgggaccccgtgtcatcgcccaggccgagcacgatg (Seq ID No: 594)

Homo sapiens sorting nexin 12 (SNX12):
aggcctctgtcccccacccccttcccggtcccaggctctccttcggaaagatg (Seq ID No: 595)

Homo sapiens LAG1 longevity assurance homolog 2 (S. cerevisiae) (LASS2):

cggcctttttttcccggctgggctcgggctcagctcgactgggctcggcgggcggcggcg
gcggcgccggcggctggcggaggagggagggcgagggcgggcgcgggccggcgggcgggc
ggaagagggaggagaggcgcggggagccaggcctcggggcctcggagcaaccacccgagc
agacggagtacacggagcagcggccccggccccgccaacgctgccgccggctactccctc
ttgatgccctcccctttgcccctcactcaggatg (Seq ID No: 596)

Homo sapiens cytohesin 4 (CYTH4):
tcatcttttccccagaggcgtcggaatg (Seq ID No: 597)

Homo sapiens transportin 2 (TNPO2):

aattctctctctttggctccctccttccgcgcgagtctctggagaagccgcagcgcgagt
tgccgccgctgctgcccggggccgggtaagtgggcctcactcagagcccgaccctcttgg
ccccggcttgcgtcgacccccgccgggcaccgagcctgcgccgcgcgcggcccgggcgtc
ggggccgcgcccgaccgggaaaggccgggaagccggttgggcccgatcctcctggcagct
agaacgggccgggcgggggaggggggaaccgagcagagcttaggggggtggggcctcggag
ccaggccatgtcggggctcctcaagaagagggccagtgggactgctggggtcggctgga
ggggatctgattgggggaagcgtctggggactgcttggggcctgattgggggacgtcgcg
aggatcggcttgccttgcgccatg (Seq ID No: 598)

Homo sapiens makorin ring finger protein 1 (MKRN1):
gggcctttgctgtgtgggataaacagtaatg (Seq ID No: 599)

Homo sapiens vinculin (VCL):

ctgtctcttcgccggttcccggccccgtggatcctacttctctgtcgcccgcggttcgcc gccccgctcgccgccgcgatg (Seq ID No: 600)

Homo sapiens DEAH (Asp-Glu-Ala-His) box polypeptide 38 (DHX38) :

cctcctttcctgcccccagactagaggcgggatgtagtctcttaggctaagagtgattg gtcacaaggagactcggaagtgtctgatcagagccccagaggaggccttgagagcctgtt ggcgtaccgttccacacttggatccaggaatcgggcgtgttccaggctgctctctatggt agctttgggcggatagaggggcgcgcaaagtattaagggacaataatggccgctttcaa ggtgtggattttggctccttgagcctgtctgagcgaggggtggcagcgccggcgccccag aatccgggacagaagggtcccaagagtcgcgcttggtgagagaaatcccagatcctgtga tg (Seq ID No: 601)

Homo sapiens osteoglycin (OGN):

catcctctaagcttttaaatattgcttcgatggtctgaattttatttccagggaaaaag agagttttgtcccacagtcagcaggccactagtttattaacttccagtcaccttgatttt tgctaaaatg (Seq ID No: 602)

Homo sapiens NIN1/RPN12 binding protein 1 homolog (S. cerevisiae) (NOB1): gctcccctctcacgcagccaacatg (Seq ID No: 603)

Homo sapiens nudix (nucleoside diphosphate linked moiety X)-type motif 5 (NUDT5) :

catcctttagcaccgcgagaggcgccggtgtttcgagccgtggcaccggcatcggctga cactgctgcctccagctagttatttcgtcctcttccgttcttcaccctacaccttggag gtgaacttctcacctgagggctgtaaagactcgtttgaaaatg (Seq ID No: 604)

Homo sapiens WD repeat domain 91 (WDR91):
cgtccctcaccgcaccacccctaaagacgctagcgctgcgatg (Seq ID No: 605)

Homo sapiens nuclear transcription factor Y, gamma (NFYC):

gggcctctgcattgcccgactccgtaggagcgcgggggcggctcctgctcttcctggact cctgagcagagttgtcgagatg (Seq ID No: 606)

Homo sapiens protein phosphatase 2, regulatory subunit A, al pha (PPP2R1A):

ccgcccttccttcttctcccagcattgcccccccacgtttcagcacagcgctggccgca gtctgacaggaaagggacggagccaagatg (Seq ID No: 607)

Homo sapiens vesicle-associated membrane protein 2 (synaptobrevin 2) (VAMP2):

ccatctttccgtcccgggcagccagcgccagtcggagccagcgcgagccgccgccgccat cactgccgctgccaagtcctccacccgctgcccccgccatg (Seq ID No: 608)

Homo sapiens transmembrane protein 5 (TMEM5): gattctctttccgcccgctccatggcggtggatgcctgactggaagcccgagtgggatg (Seq ID No: 609)

Homo sapiens UDP-GlcNAc:betaGal beta-1,3-N-acetylglucosaminy ltransferase 3 (B3GNT3):

aactctttcttcggctcgcgagctgagaggagcaggtagaggggcagaggcgggactgtc
gtctgggggagccgcccaggaggctcctcaggccgaccccagaccctggctggccaggat
g (Seq ID No: 610)

Homo sapiens SEC11 homolog A (S. cerevisiae) (SEC11A): gcgccctttcccctgccggtgtcctgctcgccgtccccgccatg (Seq ID No: 611)

Homo sapiens RUN and SH3 domain containing 1 (RUSC1):

ctccctccccgcgccccgtcctctcccgccctacaggccctagcagggcaggcgggaggt

gagcgcggccatcccgctcccggagttccgggatcctggagtccgtagttcgtggtcctt
cgccggtgtccccggagcccagcggctgtggatg (Seq ID No: 612)

Homo sapiens aryl hydrocarbon receptor interacting protein-1 ike 1 (AIPL1): cctccctttctcctgcagccatg (Seq ID No: 613)

Homo sapiens tumor necrosis factor, alpha-induced protein 8 (TNFAIP8) :

cctccttttctcccgccggctctaacccgcgcttggctaaggtccgcgggaacccgtgag
ccaccgagagagcagagaactcggcgccgccaaacagcccagctcgcgcttcagcgtccc
ggcgccgtcgcgccactcctccgatg (Seq ID No: 614)

Homo sapiens staphylococcal nuclease and tudor domain contai ning 1 (SND1):

gcgtctctttcgctccgtgtcccgctgctgctcctgtgagcgcccggcgagtccgtcccg
tccaccgtccgcagctggtagccagcctgcccctcgcctcgactccctttcaccaacacc
gacacccacattgacacctccagtccggccagccgctccactcgttgcctttgcatctcc
acacatg (Seq ID No: 615)

Homo sapiens DNA segment on chromosome 4 (unique) 234 expressed sequence (D4S234E):

cgccctcttttggtcgccccctccccaacccagcactaaggagcaccctgctctggtctc
cgccaccacccagcgcctcctggacccatccccccaaacccttgaacgtcctcaggaccc
ccaggtgagcgcggcgcgctgcgggcggggaccctctctgcacctccccgcaccccctggg
ggtcgctctgtccctacggtccccgcctcccctttctcctttctaagcgcctcgcgccca
ggccgccgcccggggtggcgcagcccgcagccctcccgctccgggcgccctccgccgctc
cgagacccccctgggggcgcgtcctctcccgctcccctgttccctcccccggctcagggcg
ggcgcgtggtcccaggggaggctcccgcccagccccgcactcctttgtgcggccgggcgg
gcgctgcgtcaaggtggaggcgcggccacacgcgcgcacccacccgcgcgcacccagccc
ccgggagaggcaggaagggaggcggcggcgcgaggaggagggagcggccgtggagcccaa
tcgttcgctccccttcccgggtccgcgcgcggcgccgcctccgccattgctgcgagcagg
agcaggagacgcggagctcggagcgctcagctgacctgccggagccgggcgtgggctgca
gcctcggagctcccggaacgatg (Seq ID No: 616)

Homo sapiens growth hormone inducible transmembrane protein (GHITM) :

acgtcctttcgatgttgcgtcatgcagtgcgccggaggaactgtgctctttgaggccgac
gctaggggcccggaagggaaactgcgaggcgaaggtgaccggggaccgagcatttcagat
ctgctcggtagacctggtgcaccaccaccatg (Seq ID No: 617)

Homo sapiens stress-associated endoplasmic reticulum protein 1 (SERP1):

tttccttcctctttcactccgcgctcacggcggcggccaaagcggcggcgacggcggcgc
gagaacgacccggcggccagttctcttcctcctgcgcacctgccccgctcggtcagtcag
tcggcggccggcgcccggcttgtgctcagacctcgcgcttgcggcgcccaggcccagcgg
ccgtagctagcgtctggcctgagaacctcggcgctccggcggcgcgggcaccacgagccg
agcctcgcagcggctccagaggaggcaggcgagtgagcgagtccgaggggtggccggggc
aggtggtggcgccgcgaagatg (Seq ID No: 618)

Homo sapiens ADP-ribosylation factor interacting protein 1 (ARFIP1) :

cggtctcctcacttccggcttcgctgctcttggttctggttctggaggctgggttgagag
gtcgccggtccgactgtcctcggcggttggtcagtgtgaatttgtgacagctgcagttgc
tccccgcccccgagcagccgaggagtctaccatg (Seq ID No: 619)

Homo sapiens tumor necrosis factor receptor superfamily, mem ber 21 (TNFRSF21):

ccgccccttcggcgccaccacgtgtgtccctgcgcccggtggccaccgactcagtccctc
gccgaccagtctgggcagcggaggagggtggttggcagtggctggaagcttcgctatggg
aagttgttcctttgctctctcgcgcccagtcctcctccctggttctcctcagccgctgtc
ggaggagagcacccggagacgcgggctgcagtcgcggcggcttctccccgcctgggcggc
cgcgccgctgggcaggtgctgagcgcccctagagcctcccttgccgcctccctcctctgc
ccggccgcagcagtgcacatggggtgttggaggtagatgggctcccggcccgggaggcgg
cggtggatgcggcgctgggcagaagcagccgccgattccagctgccccgcgcgccccggg
cgcccctgcgagtccccggttcagccatg (Seq ID No: 620)

Homo sapiens sushi-repeat containing protein, X-linked 2 (SRPX2):

ccccctcttctgcagcagacggactgagttcctctaatccctgtgttccttctcccccat
ctttctaaacccttctctgagagaggaataactatagcttcagggataatatagcttta
aggaaacttttggcagatgtggacgtcgtaacatctgggcagtgttaacagaatcccgga
ggccgggacagaccaggagccactcgttctaggaatgttaaagtagaaggtttttccaa
ttgatgagaggagcagagaggaaggagaaagaggaggagagagaaaaagggcacaaaata
ccataaaacagatcccatatttctgcttcccctcacttttagaagttaattgatggctga
cttctgaaagtcactttcctttgccctggtacttcaggccatatacatcttttcttgtct
ccataatcctccctttcaaggatg (Seq ID No: 621)

Homo sapiens HIV-1 Tat specific factor 1 (HTATSF1):

acctccctttctctgctcagctccagcgtcatttcggcctcttagttcttctgaaccctg
ctcctgagctaggtaggaaacatg (Seq ID No: 622)

Homo sapiens trafficking protein particle complex 2 (TRAPPC2) :

gggtctcttccgcggaaactgacattgcgtttccgttgtcggcctcccactgcaggagcc
atatattgaagaccatg (Seq ID No: 623)

Homo sapiens UDP-N-acetyl-alpha-D-galactosamine:polypeptide N-acetylgalactosaminyltransferase 5 (GalNAc-T5) (GALNT5):

ccacctttcttgggcttgtaggaaggtggacatgggctcccggagacaagacaagtgat
atgttgaactgttcggtggctggaatcaactgctcctggagtgacctaaggccagtgttt
atcagaacttagccagggccagccaagcaggcacagatgctctgctatgaaatgccacgc
aggcagagactgacaagcggtaggaactgagctttccccttggactgctgcttcctgctg
tgttcaggggaggggtcactttctggcaactctgctgctgctgctgctgctgctgctac
ttcagcttcctctccactcaaggtaagcaggctaagggagggcaggctgctagggaaagc
tttgtaccatg (Seq ID No: 624)

Homo sapiens transmembrane protein 97 (TMEM97):
tggcccctcttctcacatcagcgggtccaggcccaaccgacagactatg (Seq ID No: 625)

Homo sapiens EH-domain containing 2 (EHD2):

cgtcctccccgctccgggccccacccggctcagacggctccggacgggaccgcgagcaca
ggccgctccgcgggcgcttcggatcctcgcgggaccccaccctctcccagcctgcccagc
ccgctgcagccgccagcgcgccccgtcggcagctctccatctgcacgtctctccgtgaac
cccgtgagcggtgtgcagccaccatg (Seq ID No: 626)

Homo sapiens tubulin tyrosine ligase-like family, member 4 (TTLL4) :

cgccctcttcttccagactctcggtctgtccgctgggggcgcgcgcggtgtgtggcaggc
ggcagcggcgctggcggccgagtgcgcttgtcacgcgtggcggtgcgtggttgctagggg
cgcctgaggctgccgggtagcccagcaggccgagggaggaagtagcgtggagccggtgcc
gagccggggcgaagctggatcccctagatagactgtcttcaagctcactgatattttcct
ctgcttgatccattgtgctgttgagagcctctagtaaatttttcagactgacagacttca
aggatgcagctgctactaccggaggtgtgtggcaccttacctcagcaaggccatgagacc
gtgtggccatgatgtgggcccctcatg (Seq ID No: 627)

Homo sapiens basic leucine zipper and W2 domains 1 (BZW1):

acctctccctcctcctggcgttagttccggtcgcagaggagacaccgccgcagttgccgg
tacatcggggatttctggctctttcctcttcgccttaaattcgggtgtcttttatg
(Seq ID No: 628)

Homo sapiens centrosomal protein 57kDa (CEP57):

ttgccctttctgtgtaagctgtgagcgtaggcggccctgaggggggtgtgttgcaggggtt
tccaagcccagcaccagcaccccttgcccttttccatcaggggttcagcctagggtccccg
ctggtgggcggctcccgagtcttggagaagagcacgagaacctagaccgcccccgaagtg
cggagacccccctgggcaggctgaaagatg (Seq ID No: 629)

Homo sapiens family with sequence similarity 115, member A (FAM115A) :

ctgccctttgcctcctgggcggagaagctgcttcctcctgggaacaaccgcctcccgctc
ctagcaggttgctactgccccgaacccgcgctgcagggaacagcggggcaaacagtgagt
ggggttcagcgtagactctggaccaggagaggcccgcggtgaccgaggcctgggccccgg
aaaccaatagagccatg (Seq ID No: 630)

Homo sapiens ATG13 autophagy related 13 homolog (S. cerevisiae) (ATG13):

agccctctttcacccccccccccccggccattaccgaagcggatgaaaacaaacactaacg
atggcggcgccgggaagcgaccggctgctgggcttaaggcgggagtgaccgcttaaccag
tgagggaagcactgaagagcgccagtcgacgtgggtgcgacaactcgcggagtcttagga
gcaaaacgtctggggcctgcgagccaggacccttctgaagccttaggtgtctatcggcga
cgtgtacggtcactgcagctccggagcgcggaaccctcagccaggaggcgcggctggtcg
gtcccaggtcccggcctccgtaatgagagcccggaaccactctttgtgccgcagcttcgc
agcatcttggactcaagtgattctcctgcctcagcctcctgagtagctgggactacagat
tcctataggcaatg (Seq ID No: 631)

Homo sapiens sorting nexin 17 (SNX17):

ccgccttcccacatcggatcgcagggctcccaaaatggcgagtgaggctgcggggactcg
ctgagcagcggagggggagcgtgcagagccgctgcggccctcacagtccggagcccggcc
gtgccgtgccgtagggaacatg (Seq ID No: 632)

Homo sapiens phytanoyl-CoA 2-hydroxylase interacting protein (PHYHIP) :

cgttctttctcccttctctgcctctctctcctccacgctgctttgatttcgctcttgcct
ctcttcttgcgctgctcagctgggaacatcgtctcaccaggggcagcagcgacgcgctgc
acagccagacaggagctggctgcggggcatggaagcagcctccttggcagccgggagagg
agcaagcgcacgccactgcccgtgacccaggcgtccggctgctgtcccctgccggggagc
tcatccacgcagaggtctctccctgtcctccctgcgagcttttcctctgcagagcccagt
ggagccagtccccacaggagacaaccctgacgggagcatg (Seq ID No: 633)

Homo sapiens translocase of outer mitochondrial membrane 20 homolog (yeast) (TOMM20):

cggcctttctgtgttcctggcccgcggccgtcgggtgtgagctgcgccgaccgctctgag
ggttcgtggcccaccgctccttcgcggtccctgccgccaccgtccacgctcagcgttgta
gagaagatg (Seq ID No: 634)

Homo sapiens KIAA0141 (KIAA0141):

cggcctttctagccgctgtcccaagggttggtctcgcgctttcggctgcgagctctctgt
ggtgctggcagcgacatg (Seq ID No: 635)

Homo sapiens janus kinase and microtubule interacting protei n 2 (JAKMIP2):

ctccctcctttaaacagcttctccgggtctcagcatgggcttccagggcagcgattgagg
agaccttaccaaggagcaccacacagtagatgctgagacatcgtactccaggataagaaa
cagtaacatggcagcacctgcttgaaagaaattaaaaaccaacagactccatttagaaag
gaacaatg (Seq ID No: 636)

Homo sapiens EPM2A (laforin) interacting protein 1 (EPM2AIP1):

cctcctctccccttgcggcctttctaacgttggccctgctcttgtggcctcccgcagaat
g (Seq ID No: 637)

Homo sapiens centrosomal protein 170kDa (CEP170):

cggtctttgccgttaccgctatgtgtggggcgtgtgtggaataacgttattgcccagcgg
agctgagggccccggagctcgaccgcagcggcagcgacgacaacagcggcgacgacgacg
acgacgaggtgggggaggacggcgtgcgagagactcacgggacgcgacgcgccccgcct
cccccgtccggtccctctctccacggtaaggggatgacgtagctttgccaaagacttaga
agctaagcagaaaatg (Seq ID No: 638)

Homo sapiens suppressor of Ty 7 (S. cerevisiae)-like (SUPT7L) :

aggcctctcgaggtccagacagccgcccagcccgctctgcgacgcagcagtgaatagtgt
ggtacctccttgtctcggttcaggtccagacctccccgtcttccggctgccctgaacgtc
aggcgacctcaggaccctgtgattggcgcctgcgccggcggaccgtgaccgaggaaaccc
ctggagggacttgggcattccttgggctccgtgcctgttcttcgtgctcctttcgggcaa

ggatctcacattatcagtctttgaccgacacagaatgcctggcatttgataaatgtttgt
tgaacttgaagagacatatggacaatg (Seq ID No: 639)

Homo sapiens non-SMC condensin I complex, subunit D2 (NCAPD2) :

ttttccttttcatttcagcctgactgccggaatcagagccgcgggtgagatccccagccc
tgtgagcctgtaggagtagaatg (Seq ID No: 640)

Homo sapiens ring finer protein 10 (RNF10):

ggttctttgagatgctgtttggcgactcgtcgccattcccggagcaggtcggcctcggcc
caggggcgagtatccgttgctgtgtcggagacactagtccccgacaccgagacagccagc
cctctcccctgcctcgcggcgggagagcgtgtccggccggccggccggcggggctcgcgc
aacctccctcgcctccccttcccccgcagcctccgccccgccaggcccggcccggactcc
cgagccccggcctcctcgtcctcggtcgccgctgccgccgggcttaacagccccgtccgc
cgcttctcttcctagtttgagaagccaaggaaggaaacagggaaaaatgtcgccatgaag
gccgagaaccgctgccgccgccgacccccgccggccctgaacgccatgagcctgggtccc
cgccgcgcccgctccgctccgactgccgtcgccgccgaggcccccgttgatg
(Seq ID No: 641)

Homo sapiens PAN2 poly(A) specific ribonuclease subunit homo log (S. cerevisiae) (PAN2):

agcccttcttgattggaagaagcgcctcggaccccggtccttggcgccgtagtggttagg
ttgagccctaggcgtggggggagaactggggaaactggaatttcccgcggagctgacagcg
cttgcgctccccctactcgttctaattccacgcgctccaaaatatccgccatggagaaat
cttggccaggatgtccattctaggcccatcggtgctgtcttgctgaaggttgggtcaggc
atctaaaggactgtggtaagggagggtgtgacacaggtgtaagctgccatcgtcatcat
g (Seq ID No: 642)

Homo sapiens CD302 molecule (CD302):
gctcctctccggccgcgcagccgctgccgcccacccgcacccgccgtcatg (Seq ID No: 643)

Homo sapiens NSA2 ribosome biogenesis homolog (S. cerevisiae) (NSA2):

gactctttcctgtcccggcctgcgtggtgtgggcttgtgggtctttgagacccgaaaatt
gagagcgttttcgcactccagcggctgctcctggcggctctgcggccgtcaccatg
(Seq ID No: 644)

Homo sapiens DIS3 mitotic control homolog (S. cerevisiae) (DIS3) : acgccttttgctggaagagcgctgctggggttaggattctgcgcggcgaggcaagatg (Seq ID No: 645)

Homo sapiens caspase recruitment domain family, member 8 (CARDS):

cctcctctgcgagcgttatttcaaaagaagttgagaaccagagaaaccgacctaaggga
ttctcccatttggcccgtcctaccctaaagtcaccacctgctgcttttctggagcgctta
ccagtgaccaagaggaacagaacacagagcagcctggcagtgtccaagcaacaagcctcc
gctcctccttcctgcaccctggggctcctgaaactcacatgggtaaaaaagatacagtaa
agacataaataccacatttgacaaatg (Seq ID No: 646)

Homo sapiens epsin 2 (EPN2):

ccgcctctcgagcgctgccggtggccgcagcggcgcacccacgccggcccggaggagcag
agtgttcatttctgtgtcgggcacagtgctaagtgctgggtgctcactggtgatgaggca
gatgaaggttaccaaacttgtggacaggagcctcatatcagagacgtggacctcactgta
gcctggtcatggcttccagcttttcgaatctgaggctccaaaggaggaaatgaccattca
gggatcttactccagcttgattacggagactgaaccttcatagggtgcgcacttaccaag
gacaggaaggtttctctgtttgaagggctttaaacttataacaaagaaaataaaaatg
(Seq ID No: 647)

Homo sapiens pyridoxal-dependent decarboxylase domain containing 1 (PDXDC1):

ccgcctctcaaccatcaggttcggcagcccgcggcgccgcctggcagctcctcctcttct
ccgccccgccggccgcgggcgcgggggacgtcagcgctgccagcgtggaaggagctgcgg
ggcgcgggaggaggaagtagagcccgggaccgccaggccaccaccggccgcctcagccat
g (Seq ID No: 648)

Homo sapiens nicotinamide nucleotide adenylyltransferase 2 (NMNAT2) :

ccttcctttctccctctgcagacacaacgagacacaaaaagagaggcaacccctagacca
ccgcgaaggacccatctgcaccatg (Seq ID No: 649)

Homo sapiens mitochondrial ribosomal protein S27 (MRPS27): tgttccttttggtacgctccaagatg (Seq ID No: 650)

Homo sapiens leucine-rich repeats and calponin homology (CH) domain containing 1 (LRCH1):

tcccctccttccagcgcctttcggtggagcactgcggcactcagcccgagctgccgtttt
cccctcgcggggaacgctgtgaccccccgcaggagcggcggggcggggtgggggggccc
gggagaagatg (Seq ID No: 651)

Homo sapiens PAS domain containing serine/threonine kinase (PASK) :

gctcctttccgtggtgtgtagccggcttggcgtgaccctcgcctgatccagttgttagag
ttggaagcttggcagttggcctcccttcttcccatg (Seq ID No: 652)

Homo sapiens megalencephalic leukoencephalopathy with subcor tical cysts 1 (MLC1):

cttcctttcctagttgggttctgacagctccgaggcagtggtttacacaaccaacacgaa acatttctacgatccacccgattcctcccctcattgatattcaggaagcagctctccttc ccctgccttcagctcaagtttgctgagcttttgtttcatttgtgaatacttcttgctgga agtccctcacccagagaccagtgctcccaacggcagagcagcgggggagataaagaactg gtgacacgtggctgtacattcagcacagctgtggtgtccccaagtgccatg (Seq ID No: 653)

Homo sapiens RRS1 ribosome biogenesis regulator homolog (S. cerevisiae) (RRS1):

ctttcttttccggattgggcatcccggcatctgcacgtggttatgctgccggagtttggg ccgccactgtaggaaaagtaacttcagctgcagccccaaagcgagtgagccgagccggag ccatg (Seq ID No: 654)

Homo sapiens formin binding protein 4 (FNBP4):
cgctctctgctcgcgcttgggctcgcgatg (Seq ID No: 655)

Homo sapiens peptidylprolyl isomerase domain and WD repeat containing 1 (PPWD1): gcgccttttctgacgatgcgaacaacatg (Seq ID No: 656)

Homo sapiens sorting and assembly machinery component 50 horn olog (S. cerevisiae) (SAMM50):

ccgccttctgccctcagcagcagacgctctgtcccgcccgggcagctctgcgaggcagcg gctggagagggaaccatg (Seq ID No: 657)

Homo sapiens Yip1 domain family, member 3 (YIPF3):

gcttctccttttgtgttccggccgatcccacctctcctcgaccctggacgtctaccttc cggaggcccacatcttgcccactccgcgcgcggggctagcgcgggtttcagcgacgggag ccctcaaggacatg (Seq ID No: 658)

Homo sapiens tectonin beta-propeller repeat containing 1 (TECPR1) :

caccctcttgcccggtccccgggagggccggtccgctcctcccggacgccgaggacctac caccgcgacttcgccccgcccggcgcgggcccaggaccctgatgtcgcttttgaacagcc cctgcacctggcagccagcgagctactgtagtaggcattgccgactgtttgcataccgga tgggagtgacagtgtaatagaaaaacaagcaagaaacctttaggtaggactcctaaggc tcagaggaagttacctccagccgctgccatg (Seq ID No: 659)

Homo sapiens DDB1 and CUL4 associated factor 12 (DCAF12):

ccttccctttcccggctcaagtccttcctctctctttcctttctttccgcctatcttttt tctgctgccgctccgggtccgggccattttccgggccgggcgcactaaggtgcgcggccc cggggcccagtatatgacccgccgtcctgctatccttcgcttcccccgccccatgtggct gcggggccgcggcggcgctgcccactatg (Seq ID No: 660)

Homo sapiens chromosome 3 open reading frame 17 (C3orf17) : ccgcctttcgtaagtccccccgcctcgcatg (Seq ID No: 661)

Homo sapiens LETM1 domain containing 1 (LETMD1): caacctcttctctcccgcttctctcgctgtgaagatg (Seq ID No: 662)

Homo sapiens chordin-like 2 (CHRDL2):

ctcccttctgctggaccttccttcgtctctccatctctccctcctttccccgcgttctct
ttccacctttctcttcttcccaccttagacctcccttcctgccctcctttcctgcccacc
gctgcttcctggcccttctccgaccccgctctagcagcagacctcctggggtctgtgggt
tgatctgtggcccctgtgcctccgtgtccttttcgtctcccttcctcccgactccgctcc
cggaccagcggcctgaccctggggaaaggatg (Seq ID No: 663)

Homo sapiens CCR4-NOT transcription complex, subunit 10 (CNOT10) :

actcctctagccggaacctgggggcccggagccggggtaggcacagagttgtcctcggag
gtccaggacagcggccagcccggcggcgggagtcagggccacgccacctgcagggaagaa
cccgagtcgaagcgggaagatg (Seq ID No: 664)

Homo sapiens THUMP domain containing 3 (THUMPD3):

cttcctcttgcagttgaggccggcgccgagccggacttcaggcggatctcgtggcggagc
ccatcttgctccctctcccaggcctttacccgctccctaggattcccgggccctgtaggt
gggagttgggagacgacagtactgcttttaaagagacagtgttagggatcttggaagcac
agccaacatg (Seq ID No: 665)

Homo sapiens nipsnap homolog 3A (C. elegans) (NIPSNAP3A):

gctcctttccactcgggaaaccttcagaggagtctcagaaaggacacggctggctgcttt
tctcagcgccgaagccgcgccatg (Seq ID No: 666)

Homo sapiens CAP-GLY domain containing linker protein 3 (CLIP3) :

gcccctccctctccgcccccacccccctgtcggcgtctgggcctcgtccccttctctctgt
ctcccttgcctcccccatcacgtcccctgacaccgacaccccattgctcccacagtctcc
ccagtctccactttggtccccagcgctgtctgcccgaggatttgcctgaaggctgccccc
aactctgcacccgccccccgagggccaccgaggaccatg (Seq ID No: 667)

Homo sapiens ring finger protein 167 (RNF167):

caccccttcccgaagtttttctgtcacctgtgttaggctccgtcccctttccgcgttttat
ccccgtaccagaaaaggatacatttagtgcctcccacccagctccactaaacgggttgga
tatctcattctttgagttggtgttccttccccggcgcccccatgtagctgggaagtggga
cctgggggtggttggacccctgggatcctaaaggagggggcagggagggcgcagaactccg
cttctgctccttgctaccaggacgcgcggcctcctcagcctctttcctcccgctgccatg
(Seq ID No: 668)

Homo sapiens polymerase (RNA) II (DNA directed) polypeptide M (POLR2M):

cgttcttccgggaaaatggcgactcccgctcgtgccccggagtcaccgccgtccgcggat
ccggcgctagtagcggggcctgccgaggaagccgagtgcccgccgccgcgccagcctcag
cccgcgcagaatg (Seq ID No: 669)

Homo sapiens dihydroxyacetone kinase 2 homolog (S. cerevisiae) (DAK):

tcgcctctttccgccagcgcccgcaggacccggatgagagcgcacgcttcggggtctccg ggaagtcgcggcgccttcggatgtggcggatgcggccgtgagccggcgggggaggtgctg ctgctgcctccactgtactcagacccaggtagcacaggattgtccatcctccagcagctc agtgcaacggtgtgaactcagcctgtttcagagcctccacaccatg (Seq ID No: 670)

Homo sapiens RNA polymerase II associated protein 1 (RPAP1):

cgatctctgcggggcaagatggcggcgcccagacaggcctggagcacggatgaataagag ggaacccccacacggagacactgctggagagagtcgtactggggaggcagctggagcagc aagatg (Seq ID No: 671)

Homo sapiens torsin A interacting protein 1 (TOR1AIP1):

cctcctctttggtgcctccagccaggaggcgggagcgatccacagcagctgacccagctc aggcactgcctctctcacagccctcaagacacaccatgggcccagaggcaggtttgctac acagcagcgacgacgcaggcggcggccccagcgactcgcaactgcctccctgaccacagc ggccaccgcccaacacccccgagaagccatcgccaccaccggcaggagaacctagggtcc

ataaagccatcttcgcgatcgactaaagctacgtcaacaactatg (Seq ID No: 672)

Homo sapiens SERPINE1 mRNA binding protein 1 (SERBP1):

cccccctctctcggcccggccatcttgtgggaagagctgaagcaggcgctcttggctcggc gcggcccgctgcaatccgtggaggaacgcgccgccgagccaccatcatg (Seq ID No: 673)

Homo sapiens N-acetyltransferase 9 (GCN5-related, putative) (NAT9): cacccttctgcgggggacgatttcgtcggtggtaggct-gctaccatg (Seq ID No: 674)

Homo sapiens ribosomal L1 domain containing 1 (RSL1D1): gcgcctcttcacgaggtggaaacaagatg (Seq ID No: 675)

Homo sapiens SH3 domain containing, Ysc84-like 1 (S. cerevisiae) (SH3YL1): cttcctcttcctgggcagcctcgggacggggcgccgcggccgggcgggcagcatg (Seq ID No: 676)

Homo sapiens methylmalonic aciduria (cobalamin deficiency) cblD type, with homocystinuria (MMADHC) :

acttcctttgcctgctcaccgccagcgtaggtgctaccaccgctgccgtcgccgccgcca ttttgatggcaggaagagtccggttctgggacagctggagacagtggtggtgactgaaat aactttaccaaaggaaagctattttgcgaactatcttctccagcggagatg (Seq ID No: 677)

Homo sapiens glioma tumor suppressor candidate region gene 2 (GLTSCR2): agttcttcctttgacaagatg (Seq ID No: 678)

Homo sapiens DDB1 and CUL4 associated factor 8 (DCAF8):

cagtcttctcgagcacatcgtcgcaaacggggccggaaagcgtggcagcgcaggcgcaag cgcagagagcggaggcggtggtggtggcggccgctggccagttccttcagtgaatctaca gacctattttctcaggagctcagcctggccttacttcagtgataaaaggaggaaaggctg gctacagcaaacatcattcaagatg (Seq ID No: 679)

Homo sapiens UBX domain protein 1 (UBXN1):

ctttcttctcgtcggtgttcccggctgctatagagccgggtgagagagcgagcgcccgtc
ggcgggtgtcgagggcgggttgcctcgcgctgacccttcccgccctccttctcgtcacac
accaggtccccgcggaagccgcggtgtcggcgccatg (Seq ID No: 680)

Homo sapiens antizyme inhibitor 1 (AZIN1):

ccgccttctcacactttcaggctctgatcgcggccgcagttttcctttttcttctgcc
gtcgccttctctgcctcttctcatcctttctcgctctgctgctctgcagtgtgacgagtc
cgaatcctcttcccacccagcccgcgcctttcttcttttgcctgcgctgttctatttctc
cttcggccgccgccgccactgctgcacacagctggtgtcggtgccgcgcttttacccca
agtcgttcccgcagcctatggcccaggccgccttgggtatttctgctcaaggtaaccaca
tccctctttaaaaattccgccgaaaaagagaagacgctttacccgactctttggccgtt
atctcacggcgaactttctgaccaagtatacaactacccagagggcctaggagaagtgct

gtatagagagcagttcgacttcaacgctgagccaccttgggaacctagctgatgataggg
gggttccatctcccaacttgtccatggaggtcttcacttcagaaatccaagactcatatt
catccagcttggtgtcaagtgggctgttgctgccagaattatcttgtgattatttgagag
atgtatcagtttcttctgaagtacaatcaactgtagaagcctttgtagcagtttgttgca
tattctaaggacccagacataggcttggtggcccgtctcttgtctttcctggtttatgac
tttcggctttgtggaatacggctgagatg (Seq ID No: 681)

Homo sapiens cell division cycle 40 homolog (S. cerevisiae) (CDC40) :
gcctcttcttcttccgccctggcagggtctccgcagaagatttgttgccgtcatg (Seq ID No: 682)

Homo sapiens stathmin-like 3 (STMN3):
gcgcctctccagcctccgcaggcccaaccgccgccagcaccatg
(Seq ID No: 683)

Homo sapiens nudix (nucleoside diphosphate linked moiety X)-type motif 13 (NUDT13) :

tttcctcttttgtgctgattcctgaggactaggaaggtgccccgaaaagaattcagagac
ctgacaatg (Seq ID No: 684)

Homo sapiens calcium homeostasis modulator 2 (CALHM2):

ctctcttttctggagttagattagtctgaagccgccaccagccccaggcccccgtgcaga
agaaaagcgggagggaacggcggaggccgccgctgccctgcaccgccctcctggaggcca
cttggagagtccggccccgaggaggccatggccacaagtgcccacagctggccccaggtt
gccagcgtcgctacagcccagaccaaggcagaataatctccggatgagctggtggcaccg
ctgagcctttggtctcaccagggcttcctgttgctggcaggcggggtggagcggagctgc
tgggaggctgctggataggagaggggtcacggctgcggaagaggaggttcttcgggacac
ccgtggatggacacggcaaggaaacaccaggccaaccacagctggggataaaatagcaca
accacaccctgccgtccagcgcctcccagcctgtgccccttcctagtaccaccagcaacc
atcaatcccgtctcctcctgcctcctctcctgcaatccaccccgccacgactatcgccat
g (Seq ID No: 685)

Homo sapiens NMD3 homolog (S. cerevisiae) (NMD3):

tcttctctgtggcggagacagccaggttggcagctgacgggacagccggggtctatttg ttgcgggttttcagcaaatccagggctggtctggaggcgcgaaaacttaaggcatacaga acgatg (Seq ID No: 686)

Homo sapiens ATPase, H+ transporting, lysosomal 50/57kDa, V1 subunit H (ATP6V1H):

gcgcctctgtcattctactgcggccgccctggcttccttctacctgtgcggccctcaacg tctccttggtgcgggacccgcttcactttcggctcccggagtctccctccactgctcaga cctctggacctgacaggagacgcctacttggctctgacgcggcgccccagcccggctgtg tccccggcgccccggaccaccctccctgccggctttgggtgcgttgtggggtcccgagga ttcgcgagatttgttgaaagacattcaagattacgaagtttagatg (Seq ID No: 687)

Homo sapiens DPHS homolog (S. cerevisiae) (DPH5):

gggccttttctctgcacggagccggcgcttttgcagttgcttctgcggaaaggtggtagt

taagaatttgtaaaggccagagaactacctacgattctctcagcggtctctcttctcctc aagtttgaaatg (Seq ID No: 688)

Homo sapiens polymerase (RNA) I polypeptide D, 16kDa (POLR1D) :

cctcctccctccttccgtcctccgcgccttccgtcggtcggtccttgcttcctgcttcgc ctccgcgcctcgcgctatgggacagagcccccgatccgccagcaccacctgaggatccag aaaccgccccagcgatg (Seq ID No: 689)

Homo sapiens HMP19 protein (HMP19):

ctgtcctttcagcaccacaagctcgggctgaggagggaggactcctggccgtcctcctcc tcttcaaattggcttgaatcttctctgaccccccacgagtgcagcacagtctgggaagaa aggcgtaaggatg (Seq ID No: 690)

Homo sapiens adiponectin receptor 1 (ADIPOR1):

gcgccccttccggcgcggggagggcgctgaagatcggggccgctcggccgcaggccgcct ccagcgccgcgggatgtagcgcgggggaccgcggcccccagcagagcccgcctgcccggc ttgtctaccatcagagggagatctctgccccctggggctgagagaccccaacctttcccc aagctgaagctgcagggtattgaggtaccagccagatg (Seq ID No: 691)

Homo sapiens SH3-domain GRB2-like endophilin B1 (SH3GLB1):

ttttcccttgggacccgggtccacacggcggggtcgcccgtccatctccggctcgcccgc ggggcccatcgtcgacgttagcggccgttctccgagccgactgacccatccttggcgctg ccgccgcgcgcttgttctcctccctcgccccgccttcatcctccccgttcacggaaacga cagctgcggctgcggggctggcgccgcctccctccacctaccacgtctgccctcgccgct ctagccctgcgccccagcccggccgcggcacctccgcctcgccgccgctaggtcggccgg ctccgcccggctgccgcctaggatg (Seq ID No: 692)

Homo sapiens anterior pharynx defective 1 homolog A (C. elegans) (APH1A):

gtcccctcttcggcttccgtagaggaagtggcgcggaccttcatttggggtttcggttcc ccccttccccttccccggggtctgggggtgacattgcaccgcgcccctcgtggggtcgc gttgccaccccacgcggactccccagctggcgcgcccctcccatttgcctgtcctggtca ggcccccacccccccttcccacctgaccagccatg (Seq ID No: 693)

Homo sapiens RNA binding motif protein, X-linked 2 (RBMX2): ctgcctttcccgggcgctgattcctgagtgctgagcgcgaacccgaggagatg (Seq ID No: 694)

Homo sapiens family with sequence similarity 82, member B (FAM82B) :

atctcctttagccccgcccgcctccgtagctgcctgaagtagtgcagggtcagcccgcaa gttgcaggtcatg (Seq ID No: 695)

Homo sapiens UTP11-like, U3 small nucleolar ribonucleoprotein, (yeast) (UTP11L): tgatcttttccaaggctgtacagacatg (Seq ID No: 696)

Homo sapiens chromosome 14 open reading frame 166 (C14orf166) :

cgccctctcgccgcgtcgccggtgcctgcgcctcccgctccacctcgcttcttctctccc ggccgaggcccgggggaccagagcgagaagcggggaccatg (Seq ID No: 697)

Homo sapiens transmembrane emp24 protein transport domain co ntaining 5 (TMED5):

gcttctctttcggagggagtgttcgccgccgccgcggccgccacctggagtttcttcaga ctccagatttccctgtcaaccacgaggagtccagagaggaaacgcggagcggagacaaca gtacctgacgcctctttcagcccgggatcgccccagcagggatg (Seq ID No: 698)

Homo sapiens coatomer protein complex, subunit zeta 1 (COPZ1): gtttcttttgcggctccacgtcggcaccagctgcggggcaagat (Seq ID No: 699)

Homo sapiens mitochondrial ribosomal protein S16 (MRPS16):

ggttctttctgtgtttgttctctgccctgccaaggccgtagagctggtgcgtgcgggtag cggggctctccgaggagccgcacgccggcggcaccatg (Seq ID No: 700)

Homo sapiens charged multivesicular body protein 3 (CHMP3):

ctacctccttttccgcgggccccgcccaggcggctgcccgtgacctgcctgggcgcgggg aactgaaagccggaaggggcaagacgggttcagttcgtcatggggctgtttggaaagacc caggagaagccgcccaaagaactgatatccaaagagaagaagaaaaagtgaaacgatctg tgaaagatgctgccaagaagggccagaaggatgtctgcatagttctggccaaggagatg (Seq ID No: 701)

Homo sapiens RNA binding motif protein 7 (RBM7): cgaccttttggccaggttagggagggggcgacgctgagatg (Seq ID No: 702)

Homo sapiens eukaryotic translation initiation factor 3, sub unit L (EIF3L): cgctctttccggcggtgctcgcaagcgaggcagccatg (Seq ID No: 703)

Homo sapiens zinc finger protein 706 (ZNF706):

ccttcctttccctccggcgtcctctcccggccctctcgcgctgcactgtctctccgacgc
aagactgtcccggcccggatatg (Seq ID No: 704)

Homo sapiens androgen-induced 1 (AIG1): cgccctccttgccgcccagccggtccaggcctctggcgaacatg (Seq ID No: 705)

Homo sapiens interleukin-1 receptor-associated kinase 4 (IRAK4):

cgccccttcgcggcgcttcctagttcggctggttcttctgtcgccggcttcagcagcccg
cgcccgggcaggaatagaagatg (Seq ID No: 706)

Homo sapiens transmembrane protein 66 (TMEM66):

cgttccttcgccgccgccaggggtagcggtgtagctgcgcagcgtcgcgcgcgctaccgc
acccaggttcggcccgtaggcgtctggcagcccggcgccatcttcatcgagcgccatg
(Seq ID No: 707)

Homo sapiens carboxypeptidase Q (CPQ):

ccgcctctcggccccgcggcctggccggcaagcagggctgcagtcacggggcggcgcgga
gggccccagcccagtcaggggtgtggccgccgccaccgtaaggctaggccgcgagcttag
tcctgggagccgcctccgtcgccgccgtcagagccgccctatcagattatcttaacaaga
aaaccaactggaaaaaaaaatg (Seq ID No: 708)

Homo sapiens hydroxysteroid (17-beta) dehydrogenase 12 (HSD17B12): cgctcttttcattcacgaaggtagtgaggcctagtggaaagccatg (Seq ID No: 709)

Homo sapiens protein phosphatase methylesterase 1 (PPME1): cctcccctcgatg (Seq ID No: 710)

Homo sapiens HemK methyltransferase family member 1 (HEMK1):

cccccttttccggcaggctactgggctccgcccacacacctcccggcctggttcctaaacg
ccagctcggagcaatccccttgggctggagccaaatccctgctgtgattttaaggaagac
cggcaggtccgggcccccaagggtcaaccccacacacatccccgcactttcctgtatgca
ggcctgcgagcgtagagggagtggaattcacagcctccccacccatccgcaggggtctcc
tgggaggaacccaccagcgataggaacactgaagctgggctacggcgtccgcccgagcct
tttcttaaaggcgccgaccccggaagcggggcgtccgagggagcgcgcgacgggccacgc
acgtccgggcgtccagttcggggcagcttctccggctggtgggtgggtggggcagccttt
caggcagggtggcaaccaactatatctgaggaccagagccattttggggcaccagagctt
gtgacctctccatctccacccagctgggtccaggggccactctcagcactcacctcagca
gctgacatcataaagcagacttgggaacctggaagcactctggagaacctttccctgaga
catg (Seq ID No: 711)

Homo sapiens N(alpha)-acetyltransferase 38, NatC auxiliary subunit (NAA38):

cgccctttcagttctgcttgctgtcggcaccgctgcgttacccggaaccgccgggccgaa
cagcatg (Seq ID No: 712)

Homo sapiens cleavage and polyadenylation specific factor 3, 73kDa (CPSF3):

ggttcttcctttttatttaccggtggctgtgcttccaatttaggaagaccccggcgacc tgttcctcacccccgcttcgccctcacactttcgggatg (Seq ID No: 713)

Homo sapiens dynactin 4 (p62) (DCTN4):
tcgcctcctccctccccaagatg (Seq ID No: 714)

Homo sapiens hydroxysteroid (17-beta) dehydrogenase 11 (HSD17B11) :

gttcctccttgctctcgcccctactctttctggtgttagatcgagctaccctctaaaagc agtttagagtggtaaaaaaaaaaaaaacacaccaaacgctcgcagccacaaaagggatg (Seq ID No: 715)

Homo sapiens YTH domain family, member 2 (YTHDF2):

tagtctttccaggtgttagtcgaaacctcgtggtgcgaccctggtcgtcccaaacccccct aggccttaatcctggggcggtgggggcggggaggccgtgagcacggcttccgctcctcca atccgccagaggcgcagcggccggcctctcccttcccggggttcttcgcgccggcccc ttccgcgtgggtgagtgaatgtgagagtcagcgctcgcgccgcgcgcgccgcccgcctcc

gctgttcggcgctctgctttaggcggtgggggcgggcgcgcgcgtaaaagcatagagac gggcattgagctcttgggctagagcgtcgccgagtcggagccggagcctgagccgcgcgc tgtgtctccgctgcgtccgccgaggcccccgagtgtcagggacaaaagcctccgcctgct cccgcagccggggctcatctgccgccgccgccgcgctgaggagagttcgccgccgtcgcc gcccgtgaggatctgagagccatg (Seq ID No: 716)

Homo sapiens tubulin, epsilon 1 (TUBE1):

agctctctagcagagcgccgttgctgggggaatgcagaagcggccgcgggctagcaagct cccggagccggcggcgcaccaccatg (Seq ID No: 717)

Homo sapiens ubiquitin interaction motif containing 1 (UIMC1) :

cctcctttttcttcctcagcgggtccgcggcccgctactctccgggaggggcgcttcccga cgccaaggtaggcctctcccgacgccggggcggcccttcctgatgccggggtgtgtctct cgcgacgcgggggtgggctccggacgccggggctggccttgccgaagtcgggggtgggtc cctccggacgccgaagtgggctcgggatgcggggctgggaccctcccgattccggggcgg attccggacgccgggaccggccattactggtgccgggttgggcttctccagatgccgggg ctgggtccttcccaaggttgagacaaaaggatg (Seq ID No: 718)

Homo sapiens TNF receptor-associated protein 1 (TRAP1):

ccgccccttcccatcgtgtacggtcccgcgtggctgcgcgcggcgctctgggagtacgac atg (Seq ID No: 719)

Homo sapiens cereblon (CRBN): cagcctcctttgcgggtaaacagacatg (Seq ID No: 720)

Homo sapiens ribosomal L24 domain containing 1 (RSL24D1): cttcctctcaagcttggcgtttgtttggtggggttacacgcgggttcaacatg (Seq ID No: 721)

Homo sapiens leucine carboxyl methyltransferase 1 (LCMT1):

taccctcttctgttgctttctccctgtggctcgcgccgtcccccgccgcccgtcgacccc gcttccatgtccctggcggacacagctcccaggaacctccacgcccatggccactaggca gagggaatcctctatcacctcctgctgttccacctcgagctgcgacgcagacgacgaggg cgtgcgcggcacctgcgaagatg (Seq ID No: 722)

Homo sapiens RAB14, member RAS oncogene family (RAB14):

ccccttcttttgtggtccggcccattgcgagggtgacaggaaaccctgtgcagggagcg ccgccatcttggaccagcccgaggaagatactgagggagcacaggagcagtcaccgctgc cactgctactgccgctactgctgccggcgcgtctgcacctctcggcctgccagtgtacct gccggcgcctcggtcgaccgcccccgccccctctcccgctgcgtccgcactcctgttcct ggtcctgacgccccccctcccgcccggaaagctgcccagccaccagcaacccccccagtgcc accatg (Seq ID No: 723)

Homo sapiens Enah/Vasp-like (EVL):

cttcctttttcctgtttggtttttaagtaggctataaaaatcaagttgctgtcttcagaggg tctgtggtcctctgatcaacataggctggtgggagtacaggactcgcctcctcagggttc cctgtgctgccacttttcagccatg (Seq ID No: 724)

Homo sapiens LIM domain and actin binding 1 (LIMA1):

ctctcttcccctctccctctccctctgccgggtggatgctttctccatgtggcaaggctg taactgttcacagctgtctgaaacagcagtggaccaggagcagcttggagttttaacttt cattttacaaagaacaacatgtttgaatgtttcagcaggcaagttataactggcatctac ttcttgttcttctagaacaccgaaaatctctcccagcactttagaaaggggaccctgact gtgttaaagaagaagtgggagaacccagggctgggagcagagtctcacacagactctcta cggaacagcagcactgagattaggcacagagcagaccatcctcctgctgaagtgacaagc cacgctgcttctggagccaaagctgaccaagaagaacaaatccaccccagatctagactc aggtcacctcctgaagccctcgttcagggtcgatatccccacatcaaggacggtgaggat cttaaagaccactcaacagaaagtaaaaaaatg (Seq ID No: 725)

Homo sapiens ubiquitin-fold modifier conjugating enzyme 1 (UFC1): gtttctcttgcgccctggtccaagatg (Seq ID No: 726)

Homo sapiens coatomer protein complex, subunit beta 1 (COPB1) :

cacccccttccacgtcagccaaggactctggagccgccgccgccgctgctgcggttcata gccggagtagacggagccgcagtagacggatccgcggctgcaccaaaccactgcccctcg gagcctggtagtgggccacaagcccccagtcccagaggcgtggtgggtcgggcagagtcg gaagaactggctttctagctggaagatgcggaaggggagcgactaggccgcttgcgtctg ggcctggcagaagggaccggattttctggcatccttaaatcttgtgtcaaggattggtta taatataaccagaaaccatg (Seq ID No: 727)

Homo sapiens transmembrane protein 9 (TMEM9):

gggtcttttgcggctgcagcgggcttgtaggtgtccggctttgctggcccagcaagcctg ataagcatg (Seq ID No: 728)

Homo sapiens shisa homolog 5 (Xenopus laevis) (SHISA5):

ctttcttttctccaaaaggggaggaaattgaaactgagtggcccacgatgggaagaggg gaagcccaggggtacaggaggcctctgggtgaaggcagaggctaacatg (Seq ID No: 729)

Homo sapiens transmembrane protein 69 (TMEM69):

gtgcctttccagtggacctggctgttgttgcggttgttttccttctctccgtgcaacgc tggcaagtctcaaagtcgccacagaaacatgcccctgattcagtgcctctgcttagctgt aacatgttaatcagaactacctggcatcttcctgaacaagactttcaataggggccagta tg (Seq ID No: 730)

Homo sapiens kelch repeat and BTB (POZ) domain containing 4 (KBTBD4) : agatcttcttccgggcggacgtggagccggaagcggaggttccgggctccgggatg (Seq ID No: 731)

Homo sapiens pipecolic acid oxidase (PIPOX):

cgtcctttagccgggagcctgtctttgcttgcctttgcctttgaggctctgtggctgtgg ggctgagtggcatcatg (Seq ID No: 732)

Homo sapiens blocked early in transport 1 homolog (S. cerevisiae)-like (BET1L):

agctctttccccgcgactgcgccacgtctgaggcggctgtggccgcgtcggtgtccgcgt cgaggagccggggcagggcacgatg (Seq ID No: 733)

Homo sapiens zinc finger protein 581 (ZNF581):

ttctctctttcggccggcgccgccagttcctggggcacacccagaggtccccttctcgcc gccgcctgcaactgcgagggtagcccggggccgcttggagtcgcccggacctgagaggct gctgcactgggcctcagccagccctccggatg (Seq ID No: 734)

Homo sapiens armadillo repeat containing, X-linked 1 (ARMCX1) :

cgtccttctaatcctagtcttcgtttggtccggttgcactcttcctatagcccagagggc gagagggcctgtggcctggggaaggaggacgaggttctgcctggatcccagcagtagga cgctgtgccatttgggaacaaaggaatagtctgcctggaatccctgcagatcttggggcc ggaggccagtccaacccttggagcaggaagaaacgcaaagttgtcaagaaccaagtcgag ctgcctcagagccggcccgcagtagctgcagactccgcccgcgacgtgtgcgcgcttctc tgggccagagcgagcctgttttgtgctcgggttaagagatttgtcccagctataccatg (Seq ID No: 735)

Homo sapiens spastic paraplegia 21 (autosomal recessive, Mast syndrome) (SPG21):

cggcctcccgcacgcaccgcgcagcctgctgtgcccgtgggtcccgagtgctccgccgcc cgccccgacccgggcccagccgcctccacggcccgcgctcgtactggagcgaagagcggc ctcctgaaggaggggaagggacgtgggggcggccacggcaggattaacctccatttcagc taatcatg (Seq ID No: 736)

Homo sapiens staufen, RNA binding protein, homolog 1 (Drosophila) (STAU1):

tctcccttttttccttcttccttcccctcctcgccgccaccgcccaggaccgccggccgg
gggacgagctcggagcagcagccagagtttattaaccacttaacctctcagaactgaaca
aagacaacattgttcctggaacgccctctttttaaaaaagaaagcataacccctactgta
gaactaaatgcactgtgcatg (Seq ID No: 737)

Homo sapiens adducin 2 (beta) (ADD2):

cggccttttgtcagcgcgcagggccaggagagctctcatttcctcccagcctcgtgcggg
aaatggctttaattctgacggcagggctgtgagggactagcgggaacccgagccttttgt
caaggaactgcggcgtcggtggccagtcatccccgccgccgcggagccgctgcactgctg
ggggatctcccagcagctctgacgagcgcgggctgcagcatgggcagaaaacgctgccct
gcagattagctgggtggatttttaagcgcaccccacccccccaaacccataaaataacaa
aaccaacccgcagtggccgaccggagatagctaagatgccgcgcaggagtttccacctgg
atgtttgaggttgtgtagatgtggccggcacccttgagagtggagctaggggggtgcagac
tgagcagtgaacagaaggagccttggacagggctgggccagcctcccgagttccaggagc
gaattgcaaacccaccgggaaaatg (Seq ID No: 738)

Homo sapiens WD repeat domain 1 (WDR1):

ccgccttccggctccagtccccgggctcggcctcggcgaggtgtaattcgcagcgcgggc
cggccccggaggctctcggcgagcgcggcgcggtaacaagtgggcgaggatg
(Seq ID No: 739)

Homo sapiens family with sequence similarity 20, member A (FAM20A) :

cgacctctacttccacctctggccccaagtacagcgccagctgcggcctcgggagcgccc
gcgggggtgcccgtgcaccggccgcgcctcctccctggcgcgggactcggccgcagctgc
ctcggaccccggcacgatcgtgcacaacttttcccgaaccgagccccggactgaaccggc
tggcggcagccacagcgggtcgagctccaagttgcaggccctcttcgcccacccgctgta
caacgtcccggaggagccgcctctcctgggagccgaggactcgctcctggccagccagga
ggcgctgcggtattaccggaggaaggtggcccgctggaacaggcctcagttcctgctttt
gaaaggaagagggggagtctgtgacccctgaggcctccttgcaactctgttttccaagct
ttgcacatcttccgaatttcttcttcaaagtctaccctaatgaaatatcagacaattttc
caagtgtgcttcatgaacttctgggaggtgcttcacagtttctgcaaatgattgattgaa
ttttcactttgaaaaaatatactttaaggcgacacaagatg (Seq ID No: 740)

Homo sapiens kelch domain containing 4 (KLHDC4):
ttttctttcctggtgtcccgtcgcggcttgggacccggcaagatg
(Seq ID No: 741)

Homo sapiens calcium channel flower domain containing 1 (CACFD1) :

tgctccctctcccacaaggcagcgcgccggctcggacgcggccggctaccgagccctttg
tgagggctgtgagctgcgcctgacggtggcaccatg (Seq ID No: 742)

Homo sapiens zinc finger, CCHC domain containing 8 (ZCCHC8): gaatcttttccacagcccaaaatg (Seq ID No: 743)

Homo sapiens kelch-like 24 (Drosophila) (KLHL24):

gtttcctttgttgtgagctgcggcagagactggtggctggaggagacgccggcgctggag agtgcgctgcgccgcccgccgctgagggaccgcggggttagccactgctggctgcttcca gtgttcgccgagaggtaccggggtgacagctccgggaccggccgaaaggcgaggaaccg gtgtggaaattaaaagaacacacatattttgactggggctttgatcaaccaaatgctaaa aagccacataaagaagatccctaatagtcatttctcaacaattatatagtcaactgatgt aacaatg (Seq ID No: 744)

Homo sapiens FtsJ homolog 3 (E. coli) (FTSJ3):
ctcccctttccaccatg (Seq ID No: 745)

Homo sapiens dymeclin (DYM):

gcttccctcttctctcgccgcctcctggcctccgcaccgacgcggcccgggctggagccg agccggggccgagctgcaggccggaccggagccggatctgtacccgctgagacgtggaaa catggaggcctgagccggtgtgcgccacctgggctgcggcggcgacagcgacttctcctg acccctctgccaccctcccatccgtccgcgggtccgtggagctggagcagatcccccagc cggctgagacaggttgtcttttggaaatgcaggtttaaggacaaattatctgcttaagct agaagatg (Seq ID No: 746)

Homo sapiens zinc finger protein 280D (ZNF280D):

cctcctctttctcctcctcctcagggctccagtcaggccgatccgctccgctcacggaag gaaaacagaaataacttgctggcttgtctggagtcacatgtacttaggtgacaatttaca gaaagtcatctctgcagcttgatg (Seq ID No: 747)

Homo sapiens ankyrin repeat domain 10 (ANKRD10):

cgttcctttgtgctgcggcggcggcttctcgagtcctccccgacgcgtcctctaggccag cgagccccgcgctctccggtgacggaccatg (Seq ID No: 748)

Homo sapiens SWT1 RNA endoribonuclease homolog (S. cerevisiae) (SWT1):

ctctcctttggcttggggctccggagttgccactgccgccggcgctggtaagcttttcag gatg (Seq ID No: 749)

Homo sapiens leucine rich repeat containing 49 (LRRC49):

tgacctctttcgggtctctttgaatctccgctgtagcgtcacctggaaggcagatctaac agagaacctggactgtctcctatcatg (Seq ID No: 750)

Homo sapiens F-box and leucine-rich repeat protein 12 (FBXL12) :

ccgccttctggacttggtcttagttcccagtcgcggccaaatcacgcctcagccacctcc cgcaagcctctcactgcctcagccacgctttccaggctggtttctggtccccatccgcgg ctggtccggccctgggaccgaatcacttcccagcgagaggaaggtcaaatttctcgaccg gctacgggaaggtcgcggccgccgccctgtcagccgcctcggcgcccccaggacccctcg ggtctctttaaccggaagcggaagtgcgtgtcggcgggatcatg
(Seq ID No: 751)

Homo sapiens WD repeat domain 55 (WDR55): cagtccttctcagcatg (Seq ID No: 752)

Homo sapiens zinc finger protein 3 (ZNF3):

cgttctttgttctgtccccggtgtgtgggtctgtgacagggtccaacagggcctggtccg tgtccggtcccccaaatctgtcgtccctgcccccaggcattggcatcaacaaaagtcaga attcccgggaacttgaacagaggctgctaaattcccagtaattgctcctttggccttcta gggactgacttcaaagaaggaaggaaagaatcaggcagtgcttcctcattctcttttaaa acccgcttcccgctgagtctgcacccaggagaccagagagcaccttgcccttccatg (Seq ID No: 753)

Homo sapiens tetratricopeptide repeat domain 27 (TTC27):

ggttcttctcctaggcggaagccagaccagagagcgtgcgtgttttcccagggtgcccc gcgctgctgttatggccgcctccttgaggtagtatccgcacatggaattctagggccgca ggtgtatttacggtaactgtcgccactagatttcagcgcctttggactctcctgttttca ctttcttttgttgactcccgtgtggccctcgtgggagcctgttttggctgcagcggtgtc tggggtgatg (Seq ID No: 754)

Homo sapiens THUMP domain containing 1 (THUMPD1): gtttctctttcctctcagtttgcgcacaccatg (Seq ID No: 755)

Homo sapiens ankyrin repeat and KH domain containing 1 (ANKHD1) :

tgctcttctcgttcccgagatcagcggcggcggtgaccgcgagtgggtcggcaccgtctc cggctccgggtgcgaacaatg (Seq ID No: 756)

Homo sapiens syntabulin (syntaxin-interacting) (SYBU): cctcctcctggacggcggcagcggcggcgcgaggagccggcgggcagcggcgcgatg (Seq ID No: 757)

Homo sapiens coiled-coil-helix-coiled-coil-helix domain cont aining 3 (CHCHD3):

gcgccttctccttgcttctggggtcgtggccttgctcccgctgtgcgggaaaagaatcc aggcccttccacgcgcgtgtgggtgcgggggccccgaagtgctcgtggttccccgctagg tctccgctggggcaggaaccggaatcatg (Seq ID No: 758)

Homo sapiens HAUS augmin-like complex, subunit 4 (HAUS4):

cctccttcgtcgcggcctctagtgcactttcggctccttccccttcccgggcctttcagc ttggtctttccgggcctcgcttcccccagcccctgcgcccggcccgaacgagaggttccg gagccccggcgcgggcgggttctggggtgtagacgctgctggccagcccgccccagccga ggttctcggcaccgccttgagagcttcagctgccccaggattagaatcccaagaaaatca aatg (Seq ID No: 759)

Homo sapiens solute carrier family 41 member 3 (SLC41A3):

ccgcctctttcccgccgccgcctgggaggggacccgggctgccaggcgcccagctgtgcc cagatg (Seq ID No: 760)

Homo sapiens phosphatidylinositol glycan anchor biosynthesis , class V (PIGV):

cttcctttccagcctcccgccctcgtctgcttccggccctgtggcctggtggggctctgc
aggctccctcgggagtggtccttgggccgtggcccctctgggaggcctgagggagctcaa
tcctggtagcaacacccctgaattcctggtggtgaaaggatg (Seq ID No: 761)

Homo sapiens poly (ADP-ribose) polymerase family, member 16 (PARP16) :

agttcctttatccctgggcccaacctccccgccgacccgcggtccaggcctcggtctctc
tcttcggcggcgagccgcggcccagaccccggcagaggacacttgtcggcacgttctcac
ccctgtcatctcagcccccctgcctagctccaccccaggcttgggaacccggcccctgacg
gcccattgtccgcgggcccagcccccgcgctgaacgcacgctcgcccttgcccctaacca
gcgcgtctaccccggcaacgcgcagtgacctgggatg (Seq ID No: 762)

Homo sapiens thioredoxin-like 4B (TXNL4B):

gtttcttttctgcgcttgtgcgttttctgttcggtttccttcccgctagcggggccacga
gggttgctaggcaacagcccctgggtgacttggtcttagggtcctgtccggcttggggct
gatgaaaggagctgtccgcgcccgggctcttccgagaagtggttgctgacagccacaaag
tgaaagggagtgaggcggcgtggacgagtaaggagtgacagtgaggattcacatttgggt
tatttcaagatg (Seq ID No: 763)

Homo sapiens slingshot homolog 3 (Drosophila) (SSH3):

cgtccttcctggtcctgcgggtccaggactgtccgcggggttgagggaagggccgtgcc
cggtgccagcccaggtgctcgcggcctggctccatg (Seq ID No: 764)

Homo sapiens zinc finger protein 692 (ZNF692):

ctccctctggggcgcgggcctcagttccgggctacagcagccgacgccgagaggcaccgt
ttcttcttaaaagagaaacgctgcgcgcgcgaggtgggcccctgtcttccagcagctccg
ggcctgctcgctaggcccgggaggcgcaggcgcaggcgcagtgggggtgagggcgcgtgg
gggcgcacagcctctggtgcacatg (Seq ID No: 765)

Homo sapiens tRNA-histidine guanylyltransferase 1-like (S. cerevisiae) (THG1L): tggccctttcctttccgcgtgtagaatg (Seq ID No: 766)

Homo sapiens solute carrier family 25, member 38 (SLC25A38):

tctccccttctacagagttcctccggcgcttcctccaccccgggatacacagaacctcat
ctcctacggtgctgaagcctgcagcagggcaggatgggcaggagagcagagccgcggagt
ctgcggcgcgggtgaagagcggcgcgtaattcccgcagcaagattgttccgcgcccgcag
cccctggactagcaggatccgaaccccggcggctgcgtgcttataggcgcagacgtcaga
gagcccgcggcttaaagcgcgtcgcctggctagcgccacccccctagccttcttcaaggcc
tccagggctgggcccaagcgcccgtcgacggcaccctgggcccagaggactcgcgggcct
catctccaatg (Seq ID No: 767)

Homo sapiens WD repeat domain 13 (WDR13):

agttctttctgatagcaggcagccatcttgcctggagcctgagaaagggaggagagacag
aaggaaccggcgacagtggtctcagggccgctccgggggcctcaagaaccggaggcagc
cccggaggtggtccccgatcccgggctatgctcttggatctgagaagggaaggcggaggg
cggcggggacaagatgggtggagaatgtcaagcaaggaatgctaggcgggggaggggcgt
tgctatggcgactggggaggggcggtgtctgttctgaatcgctgtgtgtcacccgggcgc
tgcccaggaagggcagggctggggtgatgaccatggtaacacccgggggggagttcgtga
catctccggcgcggagggactcgatgtctatggcaatggtcgcctggtggaaggacgga
actagatcccttcgctcgggacgctcacattccaggcccttgtcctgcaggctgccgcgg
gcggacacgccagaggaggaggccggggaatg (Seq ID No: 768)

Homo sapiens chromosome 1 open reading frame 123 (C1orf123):

ccgccttttacgacgcgccggaaagcaacggcaagggcggcagccagcaccgggcggaga
gggctaccatg (Seq ID No: 769)

Homo sapiens chromosome 20 open reading frame 11 (C20orf11):

ctgcctccttctactcgggcgccccggcggccgccacctctccccagcccaggagaggct
gcggagccgcagccgcccagaccgcgcagcgcgggaggcaggttccgcacgaaataaatc
agaatg (Seq ID No: 770)

Homo sapiens zinc finger protein 446 (ZNF446):

ttccccttttggggacagatcccgaagttcgagcatccctcggataggccgggtgtcagg
cctggtctctcaggcccgtccaggcccatcttgacgattccaagaccacccccttgagca
agaatg (Seq ID No: 771)

Homo sapiens mitofusin 1 (MFN1):

ccgccctttgccactcccccctgcctcctctccgcctttaacttctcgggaagatgaggca
gtttggcatctgtggccgagttgctgttgccgggtgatagttggagcggagacttagcat
aatg (Seq ID No: 772)

Homo sapiens phosphotyrosine interaction domain containing 1 (PID1) :

agtcctctcgcagctgcgccaggacagccggcgcgcggccgtgcccacaagttgccggca
gctgagcgccgcgcctcctcctgctcgcagccccctacgcccacccggcggcggtggcca
gcgccaggacgcacatcccgcggacaccgaccccagatgtaaagcgggaccccagcccct
cgccccccggcgcgatcgacagtctcgccagcgtctcctctgccaaaacccaggctgga
agatgtggcagccggccacggagcgcctgcaggagagatttgcagacacagaagcggcac
agagaaggccattgtgaagatcaaggcagaaaccggagttatggcatcataagccaagga
atg (Seq ID No: 773)

Homo sapiens pleckstrin homology domain interacting protein (PHIP) :

tttcctcctcctcctcctccgcctccgccgccgttgcttgaatggtggagccgaagctcg
gctcgtgaacacacactgacagctatagggcaggcggcggcaccgtccccgcttcccctc
ggcggcggggtgtcccgtcggcggccctgaagtgacccataaacatg
(Seq ID No: 774)

Homo sapiens LIM and senescent cell antigen-like domains 2 (LIMS2) :

tggcctttttgggcgtctccctgctccgcggcccgggctggcgggcgggcgctcggctg gcggctgcagcagcagagggagacccgcggcaaccccggcaacccagggctcggcgtcgc tgccaccatg (Seq ID No: 775)

Homo sapiens SCY1-like 2 (S. cerevisiae) (SCYL2):

aggtctttagtctttttccccctcccttactcttcgtccccggtccctcccctccccac ccctttccttctagctccgacgtttgcggccgcgggggcggcggaggatatggagtaaag ccagagtcagtggccaggcacgaaggcagagcaggaacagccaggaggcgtttattaggg gggcgggggggaaagagccccagcaccgcccctcctggaagaaggaagaggtaagtgaccg gccgccggcaccgaccgacctccctcaccggcggctctctcgcctgggctcccggagccg gcgaggagggaatggaggactcgcgcccgggttaggcctcccagggccgctcaggctggt gggtgttgcctggtgacgggcctgccggcggccggccgggcgatcggcggtcggcgcccg cgcaaagcggggctggacgagcagcgagctccggggagcggatccgagagggccgagtcc tcgaaagaggccttgaggcgacgggagacccgggatcgaagtcagctgccggagggagag ccccccatgccggctcgagagctcgggtttcggtggtggagaacgtagtacctttcgggg acattggacactactctaggaccgggtaactataactacccaatattgcagccatg (Seq ID No: 776)

Homo sapiens ring finger protein 31 (RNF31):

caccctctctcctagtacttcctgttctcggctaaccctggcgctgggccgggggctgga gagtgaccgtggtctgagtgacctggggcggctgcgtgggccggggtgggcctcaaagcc gggcaccagacgggaggggcggcgctcgggccgcgcgctgcccgcgccgggtcctggcgg gcggcgaggctggggctgactcctgcctcaggatg (Seq ID No: 777)

Homo sapiens mediator complex subunit 9 (MED9):
cgacctctggctaacctacccccggagccatg (Seq ID No: 778)

Homo sapiens ATP5S-like (ATP5SL):
cggccccttccggttacgaaaccttagcaagatg (Seq ID No: 779)

Homo sapiens GPN-loop GTPase 2 (GPN2):

tctccttttgcgcgacacggtctcagctgttccgcctgaggcgagtgacgctggccgcca acgaggtatacgtactgggaccctcgccctcagtctcgtctccggcgcggctacctgccc cgttttccctgtgagttgacctgctccgggccgcgggccgccaatg (Seq ID No: 780)

Homo sapiens transmembrane protein 48 (TMEM48):
cggtctcctgtacgccctagactaggggccgccatctccatg (Seq ID No: 781)

Homo sapiens ankyrin repeat and zinc finger domain containig 1 (ANKZF1):

ttgtcctcttcgctgctccgtagtgacggggattgttgtgttgcagaaatccggcaatcg acctgaggacttgcgagccgctcagctcccgggacgtttggagctgctgctaaataattt ctgctcagccatg (Seq ID No: 782)

Homo sapiens notchless homolog 1 (Drosophila) (NLE1):
ggctctttctcctccacgtggggacgcaggatg (Seq ID No: 783)

Homo sapiens cell division cycle associated 8 (CDCA8):

cgctctctctcactggcacagcgaggttttgctcagcccttgtctcgggaccgcagcctc cgccgagcgccatg (Seq ID No: 784)

Homo sapiens polymerase (RNA) III (DNA directed) polypeptide E (80kD) (POLR3E):

cgctccccccacgtgtccgccggagtttctccaccagcaacatggccgccgcctgagag gagagccgggccgccgccgtctctgcagcccgcgggtaactgggccgttgccgccgtccg cgctcggcccccgcggagagatcgagctgaaggactgcgcggctggctctcctctagtat g (Seq ID No: 785)

Homo sapiens armadillo repeat containing 1 (ARMC1):

gagcctttgcccgccagcgccttcgctctttggctccctgagttagtccggttgcttgcg atcgccgcggccggggctgcgaaccgaagggctcgctccgcgccgcctgggtctctacct catccgtaggtgtggccctgatggtgtggcaggctctggactcctaaagctctggagcga atttaagattttattcatgtgcatggcatagaagatg (Seq ID No: 786)

Homo sapiens transmembrane protein 33 (TMEM33):

ccgtctttctggaaacaccgctttgatctcggcggtgcgggacaggtacctcccggctgc tgcgggtgccctggatccagtcggctgcaccaggcgagcgagacccttccctggtggagg ctcagagttccggcagggtgcatccggcctgtgtgtggcgcgaggcagggaagccggtac ccgggtcctggccccagcgctgacgttttctctcccctttcttctctcttcgcggttgcg gcgtcgcagacgctagtgtgagcccccatg (Seq ID No: 787)

Homo sapiens pyridoxamine 5'-phosphate oxidase (PNPO):

ccttccttccccggggtagaagtccagggtgagaaattggttccgaactcaaaggaaccc agtgccgggccacagccgggtcacgtggccggcggccccccatg (Seq ID No: 788)

Homo sapiens golgi phosphoprotein 3-like (GOLPH3L):

attccttctctgcatcgaaggatcaggaagtttgtgctctctgcgtggctaagtttttca cctactaggacgggggtggggtggggagaacaggtgtccttctaaaatacagcacaagct acagcctgcgtccagccataacccaggagtaacatcagaaacaggtgagaatg (Seq ID No: 789)

Homo sapiens regulator of chromosome condensation (RCC1) and BTB (POZ) domain containing protein 1 (RCBTB1):

cgctcctcctcttcgctgccggtgggcaccgccgctcgctcgcacttctgcgcccattgg agcttcggagatccctgcggtcccgcgggacggcgcggcagcagctgacctcgcagacag gatcttgctctcttgcccagactggaatacagtggtgtgaacacggctcactgcagcctc aacctcctggactcagagatgtcggcttatttataggaattgcttgaagccagagtcatg (Seq ID No: 790)

Homo sapiens leprecan-like 1 (LEPREL1):

cgtccctttaagagcggctggccaggcacggcctccgcctctcagtacgcggagcgccgg
cggtcacctggggctcgcggagcggccagatcgcggcggagtcggcgcgcttccccgagg
gaaggtgggagaggggacccggacgcgaggtgccccgaagccctctcgagcgtaaccgtc
ccgcgcctctctgaggcggaggatg (Seq ID No: 791)

Homo sapiens hedgehog acyltransferase (HHAT):

ctgtctcttggctcaggcttggaggcctccgagcagcaacatcgtcccaattataccccg
ttggagcatcttcagatcttccactcttttcacaacgcaatcaaaatcttcgtacccatt
ttgcagtagtgatctctgtaagttgctttacaattcataaagtttattctatttgatctt
cactctaatttacaaagaaaagcagggaagtctatttctgttttacagaggtgtacaggg
aggctcacaggggctaagttcacacagtaagccctcgaagctgccagggctgcaaagccc
accctctttccaccgcaccgaactacctcctttcgcctacaaaacgtaggtggggaccac
tggtgttggaatgacggcccacctcgagtttcaggtgacttccactctgcaattaacttg
caggcagccccagacctgcaatgaacacacgggtgggggagagatatgcacgccagggtc
agtgggaaccaacagccgaggggtgagcggggctaggggccccgggccgccggcggggca
aacgcggttcagaaacgcaggccgcgctctggcccgccccctgcagcagcacggcctgct
cgccatcgcccggagagcgccgcgggttcccgagtccgggcgcggagggcgcgcgggcac
ggcggcaggggcgtgctcggaggacgcgcgctgcgctgctcctccaaagggcagctccgg
gggaaagagggtggcgtcccggggaagcccgcagccgccgccgatgtcgctgggactcgg
aagtgccgaaagaggggtgttgggaactcgcggcgcgcgtgaacgttgccgtcgccgccg
cccgggacagcccggagaaactctcagcgtaggcatcgggaaccttcgtgccaaggagcc
atg (Seq ID No: 792)

Homo sapiens chromosome 11 open reading frame 57 (C11orf57) :

cctccttttctcccaaaccacttcttccccctaccccccgccacgcgaggctgcggcg
cacggtatgggtgtgtttgtgtgtatttgtgtggggagggcgtttggagggaaggttacc
gggagctccgaggccgctggggaacagggatcccggtgacaaagatggggatatttcctc
tgtcttccacttggaaacctcaaccccgcttcaggctccctagatactttctggggccc
aaccgaaggccgtagccatccaaagcgttcccagcctttctggggagtgaaacttacccc
cggggttcgtcctagaggagcgtgagcggggaatgcccaggtcaaccggctgtccgaat
tccgccccggctcagcctccggcctcagtccgggagagagatctgcctgtcggtctgggc
tggggggaaacgcggcagtggcctgggccacaggtgagggcagagtaaccagtgggaaggc
tgcgttttcacgaaggactcgggtgaagctgcagagctgcctttgagccctgactccttg
gcttcctgggtcggaggagatcttgtaatggagtggttcttcgtctcactagcaagatgc
ctgatttcctcaggatcaagggattgaagaatg (Seq ID No: 793)

Homo sapiens high mobility group 20A (HMG20A):

agtccttcgccgcattggggcaaaataatcccttcatttttgtgaaggtaccgtggaaaa
tatttcatttttcttctcaccggagcaattgtaaatgctatgcggtaagaggagttacct
gtggaaaggtggttaagagattaggtaaagaaaaggaaaggacaccaaaataaagtgctg
cggaagaattttgtccagctgtgagacgacgagtgcgtgaagtgaaggcgattgagagg
ggctgagggaattgtcctctgtggaagggactttcttttggccctaggccccttcctgcc
cctgtcgtcagcagagtctctacaaggaagataacggactgtaaaattctataaagcaaa
gctacacatcacttgacaccatacaccatcttggttacataatgaagagagatg
(Seq ID No: 794)

Homo sapiens checkpoint with forkhead and ring finger domain s, E3 ubiquitin protein ligase (CHFR):

atgtctcttgacagcggcggcggcgcagccggttccgggttcggcgcggggcggggatgt
gaatcccgatg (Seq ID No: 795)

Homo sapiens nucleoporin 133kDa (NUP133):

ccatctcttcccttaggtgtttaagttccgcgcgcaggccaggctgcaacctgacggcca
gatccctcgctgtcctagtcgctgctccttggagtcatg (Seq ID No: 796)

Homo sapiens CNDP dipeptidase 2 (metallopeptidase M20 family) (CNDP2):
cttccttccaagaaccttcgagatctgcggtctggggtctggttgaaagatg
(Seq ID No: 797)

Homo sapiens oxoglutarate dehydrogenase-like (OGDHL):

gcacccccttccgcgcagccccctgacctgcagcctccggacctcgctgcagcgcggaccc
ggcccgcccgcccgaatg (Seq ID No: 798)

Homo sapiens transmembrane protein 30A (TMEM30A):

ccgcctcttccgctctacagcggaggtggctgtggcggtggcgctggtggctgcggcggc
ggcggcggcagcggcgctcgagcggttcctgtcagggtcagccggcgggccccctgggtg
gtccacctgcaaatcgcggagcggcgccccagggatcgatg (Seq ID No: 799)

Homo sapiens elongation protein 2 homolog (S. cerevisiae) (ELP2): gcgtctcttgtttgtgcggctgaccagttggcgacatg
(Seq ID No: 800)

Homo sapiens WD repeat domain 12 (WDR12):

cgttcttttctttgtatttccgcctctcgcctctctctaaaagccgcagttagaggcgag
atttaggaaaaacctctgccgagtgagcctctggttgggaatatgtatgagaaaaaaaaa
ctggcaaggcgttagtcaagcaaagctgaaggcagaggaaatttgatatctggctggagt
ctagaggatttaatgcaaataagatactctgagggcagcgtggcaaaaaaagactacaat
tcccggtggtcacagcgtttgagaagcgatgctttctgagacttgtagtaactaggagct
gtgtttgaactatccaggctcaggacagcctcttgaaaaaaaatttttattaataaagc
ggatttgagtgggatctttttcctaatcgattacgggcccacacgtatgggaagaattct
aacaatgattaaagggacatgctacctttacgactatccttttctaatcgatgactccta
aatctaggagtaggtagtcgatgtttgtggtctgggcgtctgtagaagggcaacctcgtg
ctttctgcagaggagaccggagggcagaaggcagagtccaggcttagactgcagttcctc
gcttacctgtgcagtctaattttgagctgcctctttgtagtcttaaaggcaggagcttc
gtgttgtgggtctgctaacccgtacgtttccgtgggcaagtcgtgtgtactcctcgccat
g (Seq ID No: 801)

Homo sapiens tetratricopeptide repeat domain 17 (TTC17):

cgacctcttcaagatggcgggcgccggagactagcttccgcttccggtgtgagcggcccg
gccgggggggcaagatg (Seq ID No: 802)

Homo sapiens proline rich 11 (PRR11):

ttttctttatggcgtgggagaggccacagcccggactccatcgactcccccggctcttag actaaaatcatg (Seq ID No: 803)

Homo sapiens TBC1 domain family, member 23 (TBC1D23):

ctccctctttcttcccctctggggaagctcagtgctggacttccgaagaccttttacgac

attgagtctcggagttggtctcagcgccggatccacttttcggcaaagtgacgtggacgt caacagcaatg (Seq ID No: 804)

Homo sapiens leucine rich repeat neuronal 3 (LRRN3):

gctcctctctggggagtggagggtgttcagttattaatgaccgctgagcaggcagcacca tgtcagtgtgacaactgatcgggtgaacgatgcaccactaaccaccatggaaacaaggaa aaataaagccagctcacaggatctctcttcactggattgagagcctcagcctgccgactg agaaaaagagttccaggaaaaagaaggaatcccggctgcagcctcctgccttcctttata ttttaaaatagagagataagattgcgtgcatgtgtgcatatctatagtatatatttgta cactttgttacacagacacacaaatgcacctatttataccgggcaagaacacaaccatgt gattatctcaaccaaggaactgaggaatccagcacgcaaggacatcggaggtgggctagc actgaaactgcttttcaagcatcatgctgctattcctgcaaatactgaagaagcatggga tttaaatattttacttctaaataaatgaattactcaatctcctatgaccatctatacata ctccaccttcaaaaagtacatcaatattatatcattaaggaaatagtaaccttctcttct ccaatatgcatgacattttggacaatgcaattgtggcactggcacttatttcagtgaag aaaaactttgtggttctatggcattcatcatttgacaaatgcaagcatcttccttatcaa tcagctcctattgaacttactagcactgactgtggaatccttaagggcccattacatttc tgaagaagaaagctaagatg (Seq ID No: 805)

Homo sapiens MIS18 binding protein 1 (MIS18BP1):

ggccctctctccgcgcggagccgagccggaactgcggcagtctctccctgccaggctctt catccaaggtttctgtggatcccttctgaagttctatctgaaaattgcgcttaagtgaat tttctgttagaagaacttggttgctactttcttgtcaagatg (Seq ID No: 806)

Homo sapiens LMBR1 domain containing 1 (LMBRD1):

ccgcccctttaacctttagggtgcgcgggtgcagtatatctcgcgctctctcccctttcc ccctcccctttccccaccccgggcgctcaggttggtctggaccggaagcgaagatg (Seq ID No: 807)

Homo sapiens ST6 (alpha-N-acetyl-neuraminyl-2,3-beta-galactosyl-1,3)-N-acetyl galactosaminide alpha-2,6-sialyltransferase 1 (ST6GALNAC1): cttcctctagaacccgacccaccaccatg (Seq ID No: 808)

Homo sapiens spermatogenesis associated 7 (SPATA7):

gctcctcttttccagtcctccactgccggggctgggcccggccgcgggaaggaccgaagg ggatacagcgtgtccctgcggcggctgcaagaggactaagcatg (Seq ID No: 809)

Homo sapiens docking protein 5 (DOK5):

cctcctccttcctcctcctcctccttcttctcctccttctcggccgggaggaggcag
ggctggatccctcagccgccgccgctcctcctcctggcaggccggccgcggagtcagctg
acgccggcgctccagcctcgcctccccgcgccgcgctctgcgctccccgaaagtggctgc
aagccggccgcccactgtcagggttgggggacagagaaagtgatgtgcgccttctaaag
cctcgcccagcgccgccgaagcagcttcacctctccaactttctcccaccgactgcttgt
cttgaccctgccctccaccctccccagagccacttcgggtgcgcgctcttgggtaaaggg
ggggtcaccggctgtctgggatg (Seq ID No: 810)

Homo sapiens glycosyltransferase 8 domain containing 1 (GLT8D1) :

tctcctccatcgcctgcagtaagggcggccgcggcgagcctttgaggggaacgacttgtc
ggagccctaaccaggggtatctctgagcctggtgggatccccggagcgtcacatcacttt
ccgatcacttcaaagtggttaaaaactaatatttatatgacagaagaaaaagatg
(Seq ID No: 811)

Homo sapiens cullin-associated and neddylation-dissociated 1 (CAND1) :

tggccttttgccctagggagcgagtgcggagcgagtgggagcgagacggccctgagtgga
agtgtctggctccccgtagaggcccttctgtacgccccgccgcccatgagctcgttctca
cgcgaacagcgccgtcgttaggctggctctgtagcctcggcttaccccgggacaggccca
cgcctcgccagggagggggcagcccgtcgaggcgcctccctagtcagcgtcggcgtcgcg
ctgcgaccctggaagcgggagccgccgcgagcgagaggaggagctccagtggcggcggcg
gcggcggcagcggcagcgggcagcagctccagcagcgccagcaggcgggatcgaggccgt
caacatg (Seq ID No: 812)

Homo sapiens BRICK1, SCAR/WAVE actin-nucleating complex subunit (BRK1): cgctcttcctcaggcggcggccatg (Seq ID No: 813)

Homo sapiens zinc finger CCCH-type containing 15 (ZC3H15):

cggtcttcctcctcgtcctgccgcagggccagaacccctgacggtattcagctgcgcgta
agtctggccggtgccatctgtctccgcaatg (Seq ID No: 814)

Homo sapiens polo-like kinase 1 substrate 1 (PLK1S1):

cggtctccttcggcaaccccggccgaacggccacccagaggctgtgctgagctggcgcag
cggcagcagcatg (Seq ID No: 815)

Homo sapiens dysbindin (dystrobrevin binding protein 1) domain containing 2 (DBNDD2) :

gtttctttcctacgcagccgctcctgccgccgtggtcgctggagctttgcctctctaggc
cggcagcgcctctcctccatggtcctgtctgtcagcgctgttttgggagcccgccggtga
ggccgggccacgctcagacacttcgatcgtcgagtctgtcactgggcatg
(Seq ID No: 816)

Homo sapiens KIAA1704 (KIAA1704):

gattctttttggatagggttgacgttcgtggatagactcatatctgtgaccagtgtccgc
caccgcggatg (Seq ID No: 817)

Homo sapiens solute carrier family 25, member 37 (SLC25A37): ccccctccctgcccacctcctgcagcctcctgcgccccgccgagctggcggatg (Seq ID No: 818)

Homo sapiens myoneurin (MYNN):

cgtcctcccaagatggcggagacagagtgaagaaactgtgttccccccttgggttgctat
cgatcaagggtaaaattccattctgatatcaaaatg (Seq ID No: 819)

Homo sapiens vacuolar protein sorting 33 homolog B (yeast) (VPS33B) :

gcttcttttctggtagaaggcggggttctcctcgtacgctgcggagtctctgcggggtg
tagaccggaatcctgctgacgggcagagtggatcagggagggagggtcgagacacggtgg

ctgcaggtctgagacaaggctgctccgaggtagtagctctcttgcctggaggtggccatt
cattcctggagtgctgctgaggagcgagggcccatctggggtctctggaagtcggtgccc
aggcctgaaggatagccccccttgcgcttccctgggctgcggccggccttctcagaacga
agggcgtccttccaccccgcggcgcaggtgaccgctgccatg (Seq ID No: 820)

Homo sapiens zinc finger, C4H2 domain containing (ZC4H2):

aggcctctccaagcccctaccgcacaggctcatagccccaagcccggaggaggtggctac
attgtgtctattgtatcccttggctggtgtatttgtacatctctcgggacgtgaaattga
cagtgaaaagtatg (Seq ID No: 821)

Homo sapiens BAII-associated protein 2-like 1 (BAIAP2L1):

cttcctctggcggcgtccggccgcttctcctctgctcctcgaagaaggccagggcggcgc
tgccgcaagttttgacattttcgcagcggagacgcgcgcgggcactctcgggccgacggc
tgcggcggcggccgaccctccagagccccttagtcgcgccccggccctcccgctgcccgg
agtccggcggccacgaggcccagccgcgtcctcccgcgcttgctcgcccggcggccgcag
ccatg (Seq ID No: 822)

Homo sapiens solute carrier family 25, member 40 (SLC25A40):

cgtccttctcgcgcctcgctctggccctgcaggttgtgtttccgcctctaccccgcctcc
attccgttgctctctcagtctcagacccgggctctcggtccgccgcttcaggtcttggcg
cagcctcagagagttggcgcggctctgtgttgaccaaacctagtggatgcagttagcgcc
ggagcccggccccgcccgtcaccagggttattcccgccttctaggtttgccaggactgcc
ggccctgcagctgccttctgccccaggtttttggctactgatgttacaaacaataaaata
ttggagcatagagttgaagaacagactcaaaccaggtttttatttaattagttaaaaata
tg (Seq ID No: 823)

Homo sapiens protocadherin alpha subfamily C, 2 (PCDHAC2):

tttcctttccctcccccctggagctgtagcggcagcagcagcaggaagccgagccgggtt gagcgactcggaggcgagcggaggagctggaatatggggagtcagcgaggacggtggggc caggagcccttgggagggcctacggagggagcggccccaggcgctttctagagcgtgagc ggtgggggagcaggcgcagggtggcacgagcggaggcggggcccgggcgtggggcacggc tggggaagctgccgcctccggccctgcccggctgcctccgccgcggccagtggctatg (Seq ID No: 824)

Homo sapiens chondroitin polymerizing factor 2 (CHPF2):

gttccttttgggttagctttggcagtattgagttttacttcctcctctttttagtggaa gacagaccataatcccagtgtgagtgaaattgattgtttcatttattaccgttttggctg ggggttagttccgacaccttcacagttgaagagcaggcagaaggagttgtgaagacagga caatcttcttggggatgctggtcctggaagccagcgggcctcgctctgtctttggcctca ttgaccccaggttctctggttaaaactgaaagcctactactggcctggtgcccatcaatc cattgatccttgaggctgtgcccctggggcacccacctggcagggcctaccaccatg (Seq ID No: 825)

Homo sapiens thioredoxin-related transmembrane protein 3 (TMX3) :

gcttctcttccgctccgggtcggctccgtttccctttccgggcgggcaggcggcggaccc cagtgtctttatccctcttttgcacagtcagcttctgcagctctcccgggctagcatg (Seq ID No: 826)

Homo sapiens ras homolog family member F (in filopodia) (RHOF) :

cgacctcttggctccgctagtgcccggcgcgccgccgccagtgctgcgggctccgggcaa tg (Seq ID No: 827)

Homo sapiens amyloid beta (A4) precursor protein-binding, family B, member 1 interacti ng protein (APBB1IP):

ctttctctcaggaaactccactcccaactgacaggtgctatttccagccagtcctatgct gttgcaaatagtgagtccatgaatgccctctgccgtgtgcattacttattttcatcagca gatcttcgtaacacactcctggaagtgggatgacggggtcaaaaggcgaatccatacata agttaaatagatattgctcaattctcttccacggggttcagaccattttggatttctacg agcaatgaagacagtgctattcctctacaccctggccggccaactgagcgtggttaaacg tggggagggaggagggtgaggttaccaacctgatggttgagaaagggcctccgcccagcg cgcccttcctccacccccacccgagagacagctgaactccggccgggacgcgcgtgttgc cagtccagccctgcaccgcgtcccctgagggcgggctgcaggcggccgggaagccttgca caaccggcccaaaagaggaagcccagaaagtgctgaagtaaacactttgggagaccgttg caacataaagcggcctctcagtctttggtggaaccatcactaggccccaatcccttagtc cctcttgcgtcgaggctgcaaaatggttccattcgccaggagacgctcctgagagaaggg cgcgcgcggcacaggggccttccttgcacctcggagcaaagcagctcggatagcgccaca cgtctgcgcgctgcgtgggaagggcagggctgacagcacttcctccccggggcagcgacc tggagcccgggtgcggcagtctgcaccgcgcgtcgctttcccggccggagtctcgccgcc ttcccgcgccccgcagcgccccgcagagcagtcgagatg (Seq ID No: 828)

Homo sapiens roundabout, axon guidance receptor, homolog 4 (Drosophila) (ROBO4):

ccttccctcttcactgtgagctcagagcagcaggacaaagtgctcgggacaaggacatag ggctgagagtagccatg (Seq ID No: 829)

Homo sapiens translocase of outer mitochondrial membrane 7 h omolog (yeast) (TOMM7):

acctcctttccctttcggattcccgacgctgtggttgctgtaaggggtcctccctgcgcc acacggccgtcgccatg (Seq ID No: 830)

Homo sapiens major histocompatibility complex, class II, DR alpha (HLA-DRA):

ttttcttttattcttgtctgttctgcctcactcccgagctctactgactcccaacagagc gcccaagaagaaaatg (Seq ID No: 831)

Homo sapiens protein arginine methyltransferase 8 (PRMT8): cctcctctactatctcggtatcaccaaacccttgccggctcttatg (Seq ID No: 832)

Homo sapiens adducin 3 (gamma) (ADD3):

ctgcctcttatgaagcaatactagagaggaaaaacaaaacccattcctttaagaaagatt ccgcctcctctcataagcaagcgcctaatggtaattgtagagtttactaagtcaaacact tactactcagcattgagagaagctgctgctgctaatgctgctgctgctgctgccgccgcc gccgctgctgctgctgctgttggtctgaggctgcagtaggtttctgtgcagcattgcaga atccacacctagagaacagaagacacagacacgtacgtctactacccttgttagaaggaa

gctttggatcttcggtggataacaagagtaatccacagacttaaaacatg (Seq ID No: 833)

Homo sapiens BarH-like homeobox 1 (BARHL1):

agccctttggatctaatgcgcagaggaggttggcccagagctcccgggctcccccaagg ctgaactccgtccaaggtgcccgcaggctccctgcccgccttccccatgccagcccgcag ctaggggcaggggcagcggcggctggggttgggggtgggtggggagcttttggggaggac aggtcgcagcttggctatg (Seq ID No: 834)

Homo sapiens intraflagellar transport 46 homolog (Chlamydomonas) (IFT46):

ttatcttttgcctagcgactgacaacaggctggttgcttggcgtggaatcctaaagtgg cctggctttgagactggagtgagaccccagccctaggctggggttctttccattatagag gagacggattcagaagggctacagaccaaggttgttgaaaaccagacatatgatgagcgt ctagagattaacgactccgaagaggttgcaagtatttatactccaaccccaagacaccaa ggacttcctcgttctgcccatcttcctaacaaggctatg (Seq ID No: 835)

Homo sapiens carbonic anhydrase X (CA10):

cccccttttcgggaggagggaggcagggacttgcaggcaagagttgcacctggtctagga
acctgcagagaaaagaactctggggtaagtagtgttctggcactggcacggaaaggggta
aagggtgggggcatgagagggacgaaatggagagggcagggaatgaattatgcaaaaaa
atctccaatatttcgcagcggagggagagcacagcacagcactcccaggatgagtcctgc
ctgggtctcccgcgccgaacccgcagcacgaagttctttttaagaagagaaactcgaaaa
tcctggagggtaacagaggcagccagggcggggcggagtgcggaggcggctgccagggac
tggggccgaggcggcggccaaggtggcctgaagctgtgacacccagcctcctcctcctcc
tcctcatggccgcgctcagcctcacctccccgcccgggcctcctgcctccgcccccgggt
gccgggctgcggagctgacgctgggacgcccggcggcggcgaggacgctcacctggccaa
gcctccttctcctcctcccccctcccgcccccacctgtcctcctcctctctgagttgggaa
gcgtagggatccgtaggcgaggaaataacgacccctgcagttgtattgcggaaaatctcg
acagcggcgctagttgcgggcgatggaagccaggcaactggggggttctggggagttcagg
aaaatagcagaggagcaggaagggcgcgcgcgacctggagagtctgtgtgcccccaccgc
gccccagtccccggggcccagcccttcccctcggcgccctggacgcactgccggaacccg
gctgagaggctgcaggctgcgcgcggacctggggagcagggagggtcggcggaggctgcc
ggcggctggcggtttcgggcaataatccctgcctctctttctctgtgtgtctgctgtgtc
tgctccttccccgcccccccggaagcaggagaagaactgccccggagcgcagcagccaccc
tccgaccatgccccggtgagggggcggacttcgagggcaacttgccgcggactgcctgg
gcttagccagcgagctacgcgctcccgggagcccggaattgcacggcgcagcccggcggg
gggctatcgtctatgtcttcttggggcgccagacgaatcggggtctcgtttttgctggaa
gagcccagtgttggtggcttcaggtggctgctgccgccgccgccgccgccgccgctgcta
gtgcggtttccgccgctggtgcgaagagaagagacacgcgagcggggagacctccaaggc
agcgaggcatcggacatgtgtcagcacatctggggcgcacatccgtcgagcccgaggga
gatttgccggaacaattcaaactgcgatattgatcttggggtgactgtccctggccggc
tgtcgggtgggagtgcgagtgtgcactcgctcggaagtgtgtgcgagtgtgtatgtgtgt
gtgccgtgtcgggctccccccttccccccgttttcccgtcgagtgatgcacttggaatga
gaatcagaggatg (Seq ID No: 836)

Homo sapiens dual specificity phosphatase 22 (DUSP22):

cctcctccctgtaacatgccatagtgcgcctgcgaccacacggccggggcgctagcgttc
gccttcagccaccatg (Seq ID No: 837)

Homo sapiens olfactomedin-like 3 (OLFML3):
gttccttctactctggcaccactctccaggctgccatg (Seq ID No: 838)

Homo sapiens phosphoribosyl transferase domain containing 1 (PRTFDC1): ccgtcttcccttcccgcgttccccgggagaaacatg (Seq ID No: 839)

Homo sapiens translocase of outer mitochondrial membrane 22 homolog (yeast) (TOMM22):
cctcctttccgcttccggtgtcccctacagtcatg (Seq ID No: 840)

Homo sapiens arrestin, beta 1 (ARRB1):

gctcctcctgctggctggggattttccagcctgggcgctgacgccgcggacctccctgcg
accgtcgcggaccatg (Seq ID No: 841)

Homo sapiens cytokine induced apoptosis inhibitor 1 (CIAPIN1) :

cctcctctcgcgagaggcgcaaggcgtggagtcgacggctggagagaagccgggagcgag cccaggcggcagtcttgattccctttggccagcagtttttaggtctgtcagtactgcac tgcaagaatg (Seq ID No: 842)

Homo sapiens leucine zipper transcription factor-like 1 (LZTFL1) :

taccctccttccccatttctgtggtccaactaccctcggcgatcccaggcttggcgggg caccgcctggcctctcccgttcctttaggctgccgccgctgcctgccgccatg (Seq ID No: 843)

Homo sapiens phospholipid scramblase 4 (PLSCR4):

agccctcccttccgcgcgcttactttgtttataacttgaaaaatcctctccgtctccctt ccctgcctcctttcctttccctttcctctgccagtacaactagacccggcgtctggcgtc cccggtgcccagcattctgcggggcaggcggattaattggaattcttcaaaatg (Seq ID No: 844)

Homo sapiens ectonucleoside triphosphate diphosphohydrolase 7 (ENTPD7):

cctccttccggctgggcaaggggccgcggggagcagctcgggactgaaccgagaggtgcc gaaggaaccggcgggccgcttgatcccgctgcagacgtaggagatgcctgggacaaggag gccaccttctcagggcaaaagaaaaagaaggtgacaggcgttgagaccaccgaagggaac ccatg (Seq ID No: 845)

Homo sapiens fascin homolog 3, actin-bundling protein, testicular (Strongylocentrotus purpuratus) (FSCN3):

agttctctctgggaacatctggtgggtactacaggccctattccaggccctatggcctgt ggaacctcaccacgggggggagggctgggccagacggagacatcacctgtggtgtcagcc ccatg (Seq ID No: 846)

Homo sapiens X-prolyl aminopeptidase (aminopeptidase P) 1, soluble (XPNPEP1):

cctccttcgcgccggcccttccgcgggtgatcagctggtctgcgctcccctgacgtgggc tggggcacgtcaccgccgaatg (Seq ID No: 847)

Homo sapiens REX4, RNA exonuclease 4 homolog (S. cerevisiae) (REXO4) :

gggtctcttccggagtcttttcctggacggggtccctgcggtgggtgtgtttcggcctgg cctgggcaggcgcttgtgctgccagggcgccgggcccggggaggccggggtctcgggtgg ccgccggcccaggcgctggacggcagcaggatg (Seq ID No: 848)

Homo sapiens LYR motif containing 4 (LYRM4):
ttttctttccaaaatg (Seq ID No: 849)

Homo sapiens DEAD (Asp-Glu-Ala-Asp) box polypeptide 24 (DDX24):
ggttcttcactcgcgactgacggagctgcggtggcgtctccacacgcaaccatg (Seq ID No: 850)

Homo sapiens transmembrane protein 159 (TMEM159):

ccttcttcctcttgttcctcctcctgcctctcttcgcttcgcctgcaaacgcggtgggggctgctcggcggtcaggagcaggttaccctccgtctgcatgcccaccatcaaggtatgaggatggtagaagctctcgtcgaaccagatggatgaagaccactaacggcttttgtttcctctggtaacagcaagagacagagcgacatgagagattggaccgcgggctgcactggagaatttactggtaggataattcatccctaaagagattgaagtgagcttcagaatg (Seq ID No: 851)

Homo sapiens NDRG family member 4 (NDRG4):

cggcctccgcccctgcagccgcgggcacgcggaggggctcctggctgcccgcacctgcacccgcgcgtcggcggcgccgaagccccgctccccgcctgcgcgtctgtctcgtccgcatctccgcggcctcctgctccacgacgtgaccatg (Seq ID No: 852)

Homo sapiens pre-B-cell leukemia homeobox interacting protei n 1 (PBXIP1):

ttttcttctcgggctgcaaacaaagggaagcctgcaacaagttaagctgaagaccgaagcaagagctggttcaggtggcagccacagcagcctcagggacctcagcaactatg (Seq ID No: 853)

Homo sapiens twisted gastrulation homolog 1 (Drosophila) (TWSG1) :

ctgtctctttaaggtgcccgaggctcgcgggcgctgcgctgaggggacggcgggaggcgcggcctggcctcgcactcaaagccgccgcagcgcgccccgggctcggccgacccggcggggatctaggggtgggcgacttcgcgggaccgtggcgcatgtttcctgggagttactgatcatcttctttgaagaaacatg (Seq ID No: 854)

Homo sapiens zinc finger protein 286A (ZNF286A):

gtcccctttgtgaggcccgggatgggaggtgcccggttcccccagggacagcttcaagcggtagggacagacatctgaggacccagcctcagggatgctgtccccgggcttccaggctccagcgccgtaggactgaggcagactccacggtgagaaagagacccgatctaacccaggccttcatcagagcccaggagggaaggcaggaagtgggaccacgaggcccgggggcttctaactcgtctggccagggagatctgaattggggtgaagagcagaatctccagaacaaggaggaggtggtgatcatg (Seq ID No: 855)

Homo sapiens S100 calcium binding protein A14 (S100A14) :

gctcctcctgtcttgtctcagcggctgccaacagatcatgagccatcagctcctctgggg

ccagctataggacaacagaactctcaccaaaggaccagacacagtgggcaccatg (Seq ID No: 856)

Homo sapiens ANKHD1-EIF4EBP3 readthrough (ANKHD1-EIF4EBP3):

tgctcttctcgttcccgagatcagcggcggcggtgaccgcgagtgggtcggcaccgtctccggctccgggtgcgaacaatg (Seq ID No: 857)

Homo sapiens KIAA1143 (KIAA1143): ctgtctttacccagagctaccatg (Seq ID No: 858)

Homo sapiens neuroligin 4, X-linked (NLGN4X):

ctctcttttcttgcagaaccgtctctctcccttctctgtctcttagcacagagctctta
ttcagccactagcttggcccttcctgcttcaattgtaatgcttgttctgcccgtccacag
actattggcggcagaaacaacgaatttcctccaaactaggcggtgttggtggctcttgca
ttcctctggatgaggaaatctagttgggggttccagaaggggaaggctcctgggctttc
aatacatcctcctgaatcatacctcgtttcgggttccctagaaaaatctggacgtgtaaa
aagaactcttaacggccgatgcagctcttccaaagctaaggctgccttggagttttcata
agaaattgtccctggaggtgttggatgatcacagcttccttggagcattgcagttgctgg
aatccagtttcaggattaagggagggctgcctccttgcaatgggctgccaagaaaacggc
tgtgcttgttcttaacctcaggctctgtctgtgatcagtctgagagtctctcccaggtct
actgctccctggaaagccctatctctctgcaggctcgcctctgggctttgtctccttgga
gccacatcactgggacagctgtggatgtggatgcagatttgaaccatg
(Seq ID No: 859)

Homo sapiens mitochondrial antiviral signaling protein (MAVS) :

ccgcctcctcgctgcgggaagggtcctgggccccgggcggcggtcgccaggtctcagggc
cggggggtacccgagtctcgtttcctctcagtccatccacccttcatggggccagagccct
ctctccagaatctgagcagcaatg (Seq ID No: 860)

Homo sapiens serine incorporator 1 (SERINC1):
ctgtctccatcttgtctgtatccgctgctcttgtgacgttgtggagatg (Seq ID No: 861)

Homo sapiens KIAA1324 (KIAA1324):

cctcccctttttttccgccttctgccagcagaagcagcagccgcagcacctgagccgcta
ctgccgctcactcaggacaacgctatg (Seq ID No: 862)

Homo sapiens synaptotagmin IV (SYT4):

ggacctccctctttgcctcctccctgttccaggagctggtgccctgggctctgcgctgtt
gttttcagcgctccgaaagccggcgcttgagatccaggcaagtgaatccagccaggcagt
tttcccttcagcacctcggacagaacacgcagtaaaaaatg (Seq ID No: 863)

Homo sapiens pyruvate dehyrogenase phosphatase catalytic sub unit 2 (PDP2):

cttccttctggagctgggtcctgactagggaccgcctgggtgaggtgaggacctggtggc
cgcagttgtggcactgtgcgcaggcgctgaactgaccggacggagcgggcggctgtggcc
tcgccagctggtttaaaaatatcctttttgctgaaggaacacatttgctggtatagttt
cagaatg (Seq ID No: 864)

Homo sapiens gephyrin (GPHN):

ctatcctttcctctcagtcctgccatctagctgccttgggtctcgcgctccgcagagcgt
tccgacactctccggcctcgttctgccgcctccgcgcgctctccccgtgcggccaccgcg
ccccccaagcttgcctccttcttgccggacttggggccgcgcgccctgactccttcccct
cccgcggacccgcgcactcccggcgcggcctctcccccacgcaggccaccgtgcactctg
tggcctccccctccttccccgctctcctcgcgcttctctggctccctagctgtcgcgctc
tcctcggcgagcgcgctcccggcccgcgcgctccgggctccggtttctcccggctcctgt
cagtgcggtgactgcgctgggaaacatg (Seq ID No: 865)

Homo sapiens deltex homolog 2 (Drosophila) (DTX2):

ccttctcctgagagtcggagccacagccagagccctgcccaggccgagccggagctgcag
cccgagcgcggtggtgccctcagccccgtcctcttgtcctcctcagcctcggtgccttgg
aatttgtgtcgctgagtcagcaagcctttcagatttgcccggtttttgttgtttgtggtt
tgtatcaagatgggaactcaaacaagtcattcctcctaaggagctggtgtcttcatccag
aagggacagtttgtgccagctctccagagagaaaaggatctggtactgttctggagtggc
ctgtagcagacactgaaccaccagccagctgcatttgttgtcctggaagtcattgccaac
tctgccagtcacactggggtccccagagaagtcaagatctgccggaggcgctgggcaatg
accccgggactccaggccagaggggtctgaagctgtttgggaaagcagcgggactccttg
ggaagatg (Seq ID No: 866)

Homo sapiens melanoma antigen family E, 1 (MAGEE1):

ctgcctttttcaccacctctaatttcagcttcagcagttgcttggaactttggttctggc
agcagcagcaacatcattaccgctagcggcagttttgtgccgaggcacctacacacctcc
cgtcctctctgccagatcgcgggcctgtcggtgtctgctcctacacgccaacgccggtgg
gcaggaccatg (Seq ID No: 867)

Homo sapiens G protein-coupled receptor 107 (GPR107):

cgccctttcaccccggacgtgggcgggagaggaagcggctggtgatgctggaacaaacat
g (Seq ID No: 868)

Homo sapiens PDZ and LIM domain 1 (PDLIM1):

cgctctttctccgacagctgccggggtgccctgcaagctgttccgcgcgtcctgcccgt
ctgtccccgcgggtcgtcgcccgccacagccgcgccatg (Seq ID No: 869)

Homo sapiens thymosin beta 10 (TMSB10):

cgctcttttgtttcttgctgcagcaacgcgagtgggagcaccaggatctcgggctcggaa
cgagactgcacggattgttttaagaaaatg (Seq ID No: 870)

Homo sapiens phospholipid scramblase 1 (PLSCR1):

agaccctttttcagaccctttttccggctgacttctgagaaggttgcgcagcagctgtgccc
ggcagtctagaggcgcagaagaggaagccatcgcctggccccggctctctggaccttgtc
tcgctcgggagcggaaacagcggcagccagagaactgttttaatcatg
(Seq ID No: 871)

Homo sapiens eukaryotic translation elongation factor 1 beta 2 (EEF1B2):

gggtcctttttcctctcttcagcgtggggcgcccacaatttgcgcgctctctttctgctg ctccccagctctcggatacagccgacaccatg (Seq ID No: 872)

Homo sapiens pyrophosphatase (inorganic) 1 (PPA1):

ggctctctccttgtcagtcggcgccgcgtgcgggctggtggctctgtggcagcggcggcg gcaggactccggcactatg (Seq ID No: 873)

Homo sapiens X-ray repair complementing defective repair in Chinese hamster cells 5 (double-strand-break rejoining) (XRCC5) :

ggctctttccgctatctgccgcttgtccaccggaagcgagttgcgacacggcaggttccc gcccggaagaagcgaccaaagcgcctgaggaccggcaacatg (Seq ID No: 874)

Homo sapiens GATA zinc finger domain containing 1 (GATAD1):

gatccctttcccagtcctgcttcccagtgcctcgggccagggaatcctggcctccgcctg cggagccggcggaacccgcttcccgcctccacggggcagcgccagcggcctggtcctttc accggcagctccgtgccgacgctctcaccgctcttcctatcgccgggagtggcgggccga ccagggggcggccgggctaccgtccgccattcccgtgtctctgcgcccgcgggggccgcc cgagccggccaccatg (Seq ID No: 875)

Homo sapiens enolase-phosphatase 1 (ENOPH1):

ccgccttttccagttccaggtgtgcagaagtgtcctctccccacgcgcggcgggctgcac ttggtcgctggctccgagatcgcgcggggccgccggaagcccaagacggtaccgggggcc gcagccgcagccggcgccgccctccgccctccccaacagcaggccgagtcccgtagcatc cggtagggaaatg (Seq ID No: 876)

Homo sapiens regulation of nuclear pre-mRNA domain containing 1B (RPRD1B):

agctctttccgggggcccggggaactactctccttgcctcgctctgtctccttcgaagtg ctctgcgcgaggttcagagcggccgccgcctccaaagggacggttttctagagctccgac gcctctcggtgcccctctgctccggcccttgccctttgacctcgctctcgcggcagggtg agaggtcgggtggccatcttgtggcggcggcgcgggcggctgttactgcggagacccatc ccctccccttctcgcacccctggcagtctgtcagtcggtaaaaagtcccgcagcctgtc aggtgaggccccggcctcgtgccgtcgctcttcccgccgcactgggcggcccaggccgct ccctgccgggcctcactgccgccaccatg (Seq ID No: 877)

Homo sapiens family with sequence similarity 60, member A (FAM60A) :

ctatctttctagacaaggcagttgaggaggagggagcgcttgaggggactggcctggcg tgcactccgcacctcggggacattattgcgcgtggaacggctgcttttggaaggcacaac ttcctgaatggaccatgactcccaccaaagatccctgtctctgattcaccaaacagcttc aaccctgaaaccaggacgagaagttgacaacatctgagtggacagctaattgacctaaga cttcagaccagactattgcccagaagaaaagatg (Seq ID No: 878)

Homo sapiens MID1 interacting protein 1 (MID1IP1):

gggcctttatctcggtgctgccgggggaggcgggaggaggagacaccaggggtggccct gagcgccggcgacacctttcctggactataaattgagcacctgggatgggtagggggcca acgcagtcaccgccgtccgcagtcacagtccagccactgaccgcagcagcgcccttgcgt agcagccgcttgcagcgagaacactgaattgccaacgagcaggagagtctcaaggcgcaa gaggaggccagggctcgacccacagagcaccctcagccatcgcgagtttccgggcgccaa agccaggagaagccgcccatcccgcagggccggtctgccagcgagacgagagttggcgag ggcggaggagtgccgggaatcccgccacaccggctatagccaggcccccagcgcgggcct

tggagagcgcgtgaaggcgggcatccccttgacccggccgaccatccccgtgcccctgcg tccctgcgctccaacgtccgcgcggccaccatg (Seq ID No: 879)

Homo sapiens transmembrane protein 35 (TMEM35):

ctctccctttgtcattctagctgcctgctgcctccgcagcgtccccccagctctccctgt gctaactgcctgcaccttggacagagcgggtgcgcaaatcagaaggattagttgggacct gccttggcgaccccatg (Seq ID No: 880)

Homo sapiens Fc fragment of IgG, low affinity IIa, receptor (CD32) (FCGR2A):
cttcctcttttctaagcttgtctcttaaaacccactggacgttggcacagtgctgggatg (Seq ID No: 881)

Homo sapiens tribbles homolog 2 (Drosophila) (TRIB2):

ctttctctttttgtttggcttctaacgcgttgggactgagtcgccgccgtgagctccccg aagactgcacaaactaccgcgggctcctccgccccgtctgcgattcggaagccggcctgg gggtcgcgtcgggagccctggcgctgcagctccgcaccttagcagcccgggtactcatcc agatccacgccggggacacacacacagagtaactaaaagtgcggcgattctgcacatcgc cgactgctttggggtaacaaaaagacccgagttgcctgccgaccgaggacccccgggagc cgggctcggagcagacgaggtatccggcggcgcccatttggggggcttctaactctttctc cacgcagcccctcttctgtcccctcccctctcgctccctttttaaaatcagtggcaccgag gcgcctgcagccgcactcgccagcgactcatctctccagcgggtttttttttgtttgtcg tgtgcgatcctcacactcatg (Seq ID No: 882)

Homo sapiens family with sequence similarity 3, member A (FAM3A) :

cgtcctctccgggggcggagcgggtcggcgggcctgacagggaacctccctgaccgagcc cacgtctccccacggccagagaaatctccggcccggcccgcatcgccagcccccaggccc ggaggaacggcccgagcccaggagaaccacatcttcgtcccagccccggaggctcctgtg ggcaagatcgtgagccaacgggttcctgaggcccctcctggccaggcagggtttccccgc gcgtttccgaggagccctgcctggccgggcggctggacaaacaggtcgtagcaccgatcg cgcccgcccccagcaggggtcccgcacaggcttgcccctgacccccacccaaacctgtcc ttccgctttgcccccaaacagtgcacttgccggcggtcccaacccagcaggagaagtgga catg (Seq ID No: 883)

Homo sapiens exocyst complex component 4 (EXOC4):
ggctctccccgcgtccaagatg (Seq ID No: 884)

Homo sapiens ELOVL fatty acid elongase 5 (ELOVL5):

gcgccttcctcttcccatcgcgcgggtcctagccaccggtgtctccttctacatccgcct
ctgcgccggctgccacccgcgctccctccgccgccgccgccttgctgctgctcaaagctg
ctgccgccccttgggctaaaaggttttcaaatg (Seq ID No: 885)

Homo sapiens apolipoprotein B mRNA editing enzyme, catalytic polypeptide-like 3G (APOBEC3G):

ctttctctttccctttgcaattgccttgggtcctgccgcacagagcggcctgtctttatc
agaggtccctctgccaggggggagggccccagagaaaaccagaaagagggtgagagactga
ggaagataaagcgtcccagggcctcctacaccagcgcctgagcaggaagcgggaggggcc
atgactacgaggccctgggaggtcactttagggagggctgtcctaaaaccagaagcttgg

agcagaaagtgaaaccctggtgctccagacaaagatcttagtcgggactagccggccaag
gatg (Seq ID No: 886)

Homo sapiens gamma-aminobutyric acid (GABA) B receptor, 1 (GABBR1) :

gctcctcctcctcccctccgtcggtcagtcagtccgcgaggagagtccgcggtggcggcg
acggtggcgagagccgcgggggccgtaggaagccaaccttccctgcttctccggggccct
cgccccctcctccccacaaaatcagggatggaggcgcctccccggcaccctcttagcagc
cctccccaggaaaagtgtccccccctgagctcctaacgctccccaacagctacccctgccc
cccacgccatg (Seq ID No: 887)

Homo sapiens cofilin 2 (muscle) (CFL2):

cctccttctcctcccagtgccacagagccgaagcccgagctgccgccgcagccacagccg
agggcactatg (Seq ID No: 888)

Homo sapiens DEAH (Asp-Glu-Ala-His) box polypeptide 35 (DHX35): tgaccttttaccccaacatg (Seq ID No: 889)

Homo sapiens resistance to inhibitors of cholinesterase 8 ho molog A (C. elegans) (RIC8A): ccgccttccccggcgcgccatg (Seq ID No: 890)

Homo sapiens FK506 binding protein 10, 65 kDa (FKBP10):

agttctttgtagtgcctccctcagactctaacacactcagcctggccccctcctcctatt
gcaacccccctcccccgctcctcccggccaggccagctcagtcttcccagcccccattcca
cgtggaccagccagggcgggggtagggaaagaggacaggaagagggggagccagttctgg
gaggcgggggggaaggaggttggtggcgactccctcgctcgccctcactgccggcggtccc
aactccaggcaccatg (Seq ID No: 891)

Homo sapiens small ArfGAP 1 (SMAP1):

cctcctcccgttccagctgccgctgccgcttcctgggctgagtccgcccgcggtcccggc
ggcgccaggtgcgttcactctgcccggctccagccagcgtccgccgccgccgtagctgcc
ccaggctccccgccccgctgccgagatg (Seq ID No: 892)

Homo sapiens chromosome 14 open reading frame 93 (C14orf93):

cctccttttgcacacacacgaatacaaagagccatacgaccttcggatgccggaaggtc
cttctgaatcccttccctgttccttaggttgcactagtcggggggttccatgctgggggc
agaaggaatgctctctaccgtctgaaaccgttcatcaggaaggccttgatttgtgatgtg
ctaggagagcacaggatctgcaaatagaaggcacctgtctcccttctgcaggccgaggag
aggccgccatggactgtgtgcttcttcatggcttgtttactcttctttcacagaccctac
agcttggggcctgggctcctctgaccatcctcattgagaaaggaaagtgagtccagagaa
gttgatgcttcctacctgttggagcggcccagcagtgtaagcgtggttgttactgcccca
tccgccatg (Seq ID No: 893)

Homo sapiens brevican (BCAN):

cgccctcttccgaatgtcctgcggccccagcctctcctcacgctcgcgcagtctccgccg
cagtctcagctgcagctgcaggactgagccgtgcacccggaggagacccccggaggaggc
gacaaacttcgcagtgccgcgacccaaccccagccctgggtagcctgcagcatg
(Seq ID No: 894)

Homo sapiens H2.0-like homeobox (HLX):

cggcctctcttcctcagtgcgggcggagaagcgaaagcggatcgtcctcggctgccgccg
ccttctccgggactcgcgcgcccctccccgcgcgcccacccacccagtccggctggactg
cggcagccgcgcggctcaccccggcaggatg (Seq ID No: 895)

Homo sapiens v-rel reticuloendotheliosis viral oncogene homo log A (avian) (RELA):

ccgcctctggcgaatggctcgtctgtagtgcacgccgcgggcccagctgcgaccccggcc
ccgcccccgggaccccggccatg (Seq ID No: 896)

Homo sapiens zinc finger protein 277 (ZNF277):
cctccctttcttttctgccgggtaatg (Seq ID No: 897)

Homo sapiens globoside alpha-1,3-N-acetylgalactosaminyltrans ferase 1 (GBGT1):

cttcctcttttctgtctggcccgcggccccgctgcctgccctgctccaggctccacctgc
gccgccgatcgcccgggtatcgcgggggcccaggccagctgagtccgttttccgcgccgg
ggtggcgcccctccaaccgtcctaacgccgggccggcagcaaggagtgttcctgggacct
cagagaccaggctcagagcctgacatccctgcgaggggacagcctcatccgcccaggcca
gtggggtctctacaagtgcccaggctcaggtgcagcccccagcaatg
(Seq ID No: 898)

Homo sapiens FXYD domain containing ion transport regulator 6 (FXYD6): ggtcctcctgggagtctcggaggggaccggctgtgcagacgccatg (Seq ID No: 899)

Homo sapiens nuclear RNA export factor 3 (NXF3):

tcctctctatgcttggggaaggaacttcctgtaagcaaggcttgaggcttgctctcgcct
tcgtcagcagccctcctcaatcttctccaaactcccgtccccaggccacacagattctcc
tcaagagagccctataaggacattggtaaaatg (Seq ID No: 900)

Homo sapiens chromosome 14 open reading frame 133 (C14orf133) :

attcccttccgccccttctctaagctgcacagcctgaatagaagggctggtccagcggc
ggcggaggctggcgctgtcctgagagggagggctctgtgcggaagagtcagggcgaccct
tgggcgctggagtacgcttgggactggggctgcgagtgagcaccagcgattggttcggaa
gcggacatttggttcagaacgagcatttaactctgccagggatccgctgggctctgacga
ctgcggtagatccatggcttcctggacgttcacccgtagagtcatcctagcttaactctt
gttccctggtctcagttcacaagcctcacctgtatcttcctggctcggaagataattgaa
accaagtctgacttctcaatg (Seq ID No: 901)

Homo sapiens X-prolyl aminopeptidase (aminopeptidase P) 3, putative (XPNPEP3): ctttctcttcccgacgcgtgagttaggccgtaatg (Seq ID No: 902)

Homo sapiens death inducer-obliterator 1 (DIDO1) :

ggccctctggcaagatggctgctgcggaggcgttggagcgcggaaatctggaaccgggat
ggcgacgtctacactgagtcggaggcgaaggagcttactccacgggaacagcctctagat
aatctgagttgttgaaaatacgaagcctgttactcgtgaacagtggctgacaacagtgtt
gttgtgagcctggctgtctgcttggacccagaggtttcgtctgccagggttttggttgt

atttaggatttcagggaaaagtgtccaagctttcagtgttggagcaggtatg
(Seq ID No: 903)

Homo sapiens PERP, TP53 apoptosis effector (PERP):

cggcctcttcgcttttgtggcggcgcccgcgctcgcaggccactctctgctgtcgcccgt
cccgcgcgctcctccgacccgctccgctccgctccgctcggccccgcgccgcccgtcaac
atg (Seq ID No: 904)

Homo sapiens tubulointerstitial nephritis antigen-like 1 (TINAGL1) :

tcctctcttgactttgagcgtccggcggtcgcagagccaggaggcggaggcgcgcgggcc
agcctgggccccagcccacaccttcaccagggcccaggagccaccatg
(Seq ID No: 905)

Homo sapiens eukaryotic translation initiation factor 4H (EIF4H): ggttcctctcggagcggagacggcaaatg (Seq ID No: 906)

Homo sapiens non-SMC condensin I complex, subunit G (NCAPG):

ccccctctcgcgggaattatttgaacgttcgagcggtaaatactccctggggctgtcata
gaagactactcggagagcgctgcctctgggttggcgggctggcaggctgtagccgagcgc
gggcaggactcgtcccggcagggttccagagccatg (Seq ID No: 907)

Homo sapiens MMS19 nucleotide excision repair homolog (S. cerevisiae) (MMS19): tatcccctcccacggtctctagttcgcgttatg (Seq ID No: 908)

Homo sapiens DnaJ (Hsp40) homolog, subfamily C, member 1 (DNAJC1) :

ctgcctctacagctgtgtgtaggcctgggggcgagggtcttcggaacgtagcgctggctg
cggccccgcccgcctacccacccgcccgtccggcagccggctcccgccgcctccgcgctc
tgtctggggccagccacctggcgggccgctccggtgcgcctgcccgcgcttttcactgac
aggcgctgttccccacagccagcgccgcccgccacgtcccagctctcggccaacggagct
gcgcggcgggtgacctttccgagcccagcgcgatg (Seq ID No: 909)

Homo sapiens stimulated by retinoic acid gene 6 homolog (mouse) (STRA6) :

ctaccctttcatctctgcaactccttcctccctgggcctcccttctggtgtgtctgtggg
tctgtctaggtgggcttgggaaaggggaaggaaggggcgtctctttaggcagctcagact
ggacaagccttctttgaaaatggtcctttgaacacacgcctgctggtggttggtcagaca
gatgcgccagcgggagccccggggccccaaggggacagctatctctgcaggaccagtgcg
atg (Seq ID No: 910)

Homo sapiens 5-azacytidine induced 2 (AZI2):

cagccccttttccggctgagagctcatccacacttccaatcactttccggagtgcttccc
ctccctccggcccgtgctggtcccgacggcgggcctgggtctcgcgcgcgtattgctggg
taacgggccttctctcgcgtcggcccggcccctcctgcctcggctcgtccctccttccag
aacgtcccgggctcctgccgagtcagaagaaatgggactccctccgcgacgtgcccggag
cagctcccttcgctgtggaagcggcggtgtcttcgaagaaaccggaagcccgtggtgacc
cctggcgacccggtttgttttcggtccgtttccaaacactaaggaatcgaaactcggcgg
ccttgggggcggccctacgtagcctggcttctggttgtcatg (Seq ID No: 911)

Homo sapiens polymerase (RNA) I polypeptide E, 53kDa (POLR1E) :

acgccttttccggcccgcagcgcggcctgggctcccgcgtgtttaaaagtgcgcttgtgg
ctgctgctgtcttaactcctgtgcttggcggacagacaggcgagatg
(Seq ID No: 912)

Homo sapiens mitochondrial ribosomal protein S25 (MRPS25):

agtcctttctcgtcgctgctcggctcgcggcccgtggggtcggccccgccaccgttgccg
ccatg (Seq ID No: 913)

Homo sapiens TRM2 tRNA methyltransferase 2 homolog A (S. cerevisiae) (TRMT2A):

cggcctccgccgcacgcgctggcggactaagagtggctggcgaagcgagcggccggcgcg
ggcccctggcgggcgggcggtacagccccaagcctgagacccggacctgagcatcgcagg
ttcgagtcccgccccgcctggggcgaagccgggggtggcggcgacctcgcggcgttgcac
cggctctgtgagcacctcccctctgagcacttcccttgtgacaggccacttcccttgtga
caggcccaggacgaggtggccaggcggcccccatggcgtccctggtctaggcggagaacc
gcctgggcgatg (Seq ID No: 914)

Homo sapiens lipid phosphate phosphatase-related protein typ e 2 (LPPR2):

ccctccctccacctcggagtctgcgcggcgcggccaggcccggccgaccgcgtctcggtc ttcgcgtctgccagcctggctggcagtccgtctgtccatcccgccgcgccggggcagtct aggcggagcggggctcaggcggcggcggcctcgacgcgagtgagtgtcgtggttggggt gctggacccagagtgcctaccctcgcctgcctgggcctcagtttccacatctgcacaatg gggtgaccatccctgccctgctggctgccaggagcggctgtgagtcttcaggcgtggat gcagcctgggggaagccatagggcgctttcacaggcctggccttcaccatg (Seq ID No: 915)

Homo sapiens chromosome 11 open reading frame 1 (C11orf1): gaaccttttttcacctcgtctgaaatg (Seq ID No: 916)

Homo sapiens microtubule associated monoxygenase, calponin a nd LIM domain containing 1 (MICAL1):

cgccctcccacccgctcagacctggttgccagcccaacaggaagcggcccctcccggctt cggagccgccgccactcatctctgcccagctgctgccctccccaggaggcctccatg (Seq ID No: 917)

Homo sapiens kinesin light chain 2 (KLC2):

gctcctttaaggcagcgaacgggccaagagaagcgtgtttcgccccctccgacgccaccg aggtagcggcttcacctttaaggcggcgcgggggctgctgggaaggccggcgggatggag gcggcgggaccggctcgcgggtgcgggtccgggtgaagcgggaggcagccagagtcggag ccgggcccgagcaccaggcgcaggcccggcgcccgcctgcccgcaccctcgtcctcacag acgccacagccatg (Seq ID No: 918)

Homo sapiens DNA cross-link repair 1B (DCLRE1B):

acttccttttctgcccactctggtaacttattgctctgctgggctctttcccttagggt ctctggccctgttcttgccccagcatgacttttatcgggacgccgttgtggaagcctcac gcaggagccctgcccccgtggagaagatcccactggtgactccaaccctaccaccatg (Seq ID No: 919)

Homo sapiens armadillo repeat containing, X-linked 5 (ARMCX5) :

gctcctcccactgccgttgtgggtaacgcggacgtggaagaacctcgtctgcggaggaaa aggtagatgttaaatggtaactacgcgcgaggttctgaggagccctgggaacaggaagga gaaaagaataccaaaagtgacaacagtttgccaatcgcagtctttaatctgataaagcgg ttatctcgtcttgagtcccaggtgccgagtcaatccccatacacagccgccgccattgcc tcgagtccttgtgtctgactgtctgttcctgctgctgtatgacacagcacctcgaggcaa ggaaataagaaaactgcctctgatccaagcagagaaggtctgcctgtagatctgctgtag ggcttgtcaccattggaagcaaggtcctacttcagtggcagatctggtggccttggagtg gctgaagaccaccaccctccacagggctgggcccatgcacagccatccttccctaccttg agtgagcttcctctgcatgttttctatatcactggcagagcctgtagttggaaaggggac agagtgactactggactttgtgtgaaaacaccaaccgggacaaaacttcagtcaaggctg agacgggtggggtatataacttgtccttacgttaaacttggaacatg (Seq ID No: 920)

Homo sapiens chromosome 12 open reading frame 43 (C12orf43): aatcctttgcggtggttcaagatg (Seq ID No: 921)

Homo sapiens vacuolar protein sorting 33 homolog A (S. cerevisiae) (VPS33A):

ggtcctcccgtaggaaccggcggactcggttggcgttgtggggcaggggtggtggagca
agatg (Seq ID No: 922)

Homo sapiens arginine/serine-rich coiled-coil 2 (RSRC2):

gggcctcctcgcctttgtgccatccgggtctctcgcgcgagcgatttagtctgaggcgaa
gcttcggagcggccggtactgttgaaagcgacaagtggaggcgccgctctagcggccggg
actctgaactatggcggctagtgatacagagcgagatggactagccccagaaaagacatc
accagatagagataagaaaaaagagcagtcagaagtatctgtttctcctagagcttcaaa
acatcattattcaagatcacgatcaaggtcaagagaaagaaaacgaaagtcagataatga
aggaagaaaacacaggagccggagcagaagcaaagagcgtgcttatgcgcgaagagactg
aactgaagacgctgcagactcagatagcaaaataataagcctacttcatgataagggaag
aagacatgaatccaaagataaatcctctaagaaacataagtctgaggaacataatgacaa
agaacattcttctgataaaggaagagagcgactaaattcatctgaaaatggtgaggacag
gcacaaacgcaaagaaagaaagtcatcaagaggcagaagtcactcaagatctaggtctcg
tgaaagacgccatcgtagtagaagcagggagcggaagaagtctcgatccaggagtaggga
gcggaagaaatcgagatccagaagcagagagaggaagaaatcgagatccagaagcaggga
aagaaaacggcggatcaggtctcgttcccgctcaagatcaagacacaggcataggactag
aagcaggagtaggacaaggagtaggagtcgagatagaaagaagagaattgaaaagccgag
aagatttagcagaagtttaagccggactccaagtccacctcccttcagaggcagaaacac
agcaatg (Seq ID No: 923)

Homo sapiens integrator complex subunit 3 (INTS3):

ccgccttcccacccccgcccttccactatggccgcttctgtgtggtgtggggagacgct
ggtcctccccgtcctcccatagcgcttattgcctcaccctcacccccctaggggccggatc
caaaggcgctgcactccccaagccttggggcatcagccaggaaggtttcctacctcctaa
ttcaggggcaggactcctcttttcccccacggggaaaagaggcagaaacttaggggttt
ccctcctttcttagggtcagacgctcttagggtccacttcttcaggggcggaagcctctc
ctacccttcccataggggcacaggcctttaccccactgtacttcggagccaacgcctttc
cctcagcactgccaccccagagtcaggacccagaggactgtgccttcgcccccaacgcag

gcgcggccttttggagaggagggaggagtggagaggacaggggcccttgctctcccctcc
ccaacttgttcctcttgcccccagtccctggcaatccagagatcccgatatctaggact
gtccatccatccactccctgacctttcccggctcctggctgcagccatg
(Seq ID No: 924)

Homo sapiens spermatogenesis associated, serine-rich 2 (SPATS2) :

tctcctttcctcttctcagacccgggagcgtccgggacgcggagcccggagctggggcga
cgaggcgattgcgggggcctgggctagctgctggctaccaatattctactttctgtctct
atgaatgtgactaccctggttacctcatataatctccctggaaaaggagacatgaatgtc
tgcaatgatacttcctgacaagaagttgatacaagaaaaggaaaggagattaacagctag
tgagcagaatttcgaacagcaggatttcgtatttttttgcttccaactgcacacttccgtt
gcccactttaaatcagagatacctacactcaaaacccagacaaggcaaaaggatacttt
tcttgtatatttttttgagatcgaagaaacgacaatg (Seq ID No: 925)

Homo sapiens fibroblast growth factor receptor 1 (FGFR1):

ccgcccctttcacctcctggctccctcccgggcgatccgcgccccttgggtctcccctcc cttccctccgtccgcgtctcctgcgccccctccctgcgctcgtcccgccgctcttcccgc cgcccaacttttcctccaactcgcgctcgggagctggcgaggcggcggcggctcctcagg tcagtttgaaaaggaggatcgagctcactgtggagtatccatggagatgtggagccttgt caccaacctctaactgcagaactgggatg (Seq ID No: 926)

Homo sapiens FUN14 domain containing 2 (FUNDC2): ctccctcttccgctgccgccgtgggaatg (Seq ID No: 927)

Homo sapiens ganglioside induced differentiation associated protein 1-like 1 (GDAP1L1): cctccttctttcctgcctctgattccgggctgtcatg (Seq ID No: 928)

Homo sapiens chromosome 19 open reading frame 43 (C19orf43): agtcctttgcgcggcacctggcgacaaaatg (Seq ID No: 929)

Homo sapiens MIS12, MIND kinetochore complex component, homo log (S. pombe) (MIS12):

ccctctcttctccaccagccaacgtccgggaaaaacgagtaagtacaggttccttctgcc aatccccgccggccacagctaactttcccgcccggcccctttctgtcataattgaggtgt ccacaaccagccaatcaggaacgcgagagtatcccgcgtttgctttcgctcgccgaggcg cgtatcagtcggaattttggggagccaaccgcgccgtctgtccctggcaagccagcggcg gtttaaaggaggtggcgggaagcctgtgtgtgcttcaaatcgtcaccctcatggtcgctc cggtaagtgctgcggggcagcattttctctgaggaggagcggggacgggcgagactggca taagcgtcttcgcgagggagcaaggcggcctgtgggtcggcctcaccccggcctccgacc tgaagatcccagcatgcagcgcgggcgcggggcccgacggaagccgggagccggccggaa gcagttcctgcgctctggcttctgggtcctgtcctgcgcgatcgcggggtcttagacagc tcaactcgccgagatgacctgggcacctctgcgttgaatcggcaaatactgatcaagccg catttattctgctctcaggaactctaagtctagcagagaagatgaggcggtagaagttca tcaatggcttggctggaggacaagcaaattgaggacattggcaacggagtgatcaaaatg atagatcatgaggcctaaaatgaataaggaaagaagagaagtggcagaggctgagaacag aaagagagggtggaggggctgtaaatcttgaagattagggtataatatgagtatatgggt aagaattggaagaattgtgtaggaggcagtagtcaaaaagtagaagcagtttggaagagt

agttacaaatatcaagagccaggtggctaaaaggtggagctataggtcattgaagctcaa gaaactgagtctctagggcattggttaagtcatctgtctagacttcaaagttgtctagga tgataattcagaagactgatctgtgccaaagtcacaggtttttcacgactgaaaacaaca tagcaaaataagccaagatg (Seq ID No: 930)

Homo sapiens DEAD (Asp-Glu-Ala-Asp) box polypeptide 50 (DDX50) :

cttcctttcacgctgtcgctgcccgtaggtggttgtggccactgtgcccggagggaggcg gcggtggccagtaatg (Seq ID No: 931)

Homo sapiens chromosome 7 open reading frame 25 (C7orf25):

cggcctctgcgtgcacgcgcctgcgtgctcgcgctcgcggttctggcgctgccggaataa tgctgacagcatg (Seq ID No: 932)

Homo sapiens KxDL motif containing 1 (KXD1):

ccgccctttcctgtcgtgacttaacgcacgcaagcggctccagggtacgtccccgccacg
cgcgctcgcaggatcggtgcgtggtgacgtttcgccggcgcgggcgccatcccggaagcg
cgagcaaggccgccagatgtgcaggcagcggaggaggagaaagagatg
(Seq ID No: 933)

Homo sapiens defective in sister chromatid cohesion 1 homolo g (S. cerevisiae) (DSCC1):

acttctttcttgcccgccaagcccgcagccacccgggcgcggcgggactcctagacccgg
cgctgcgatg (Seq ID No: 934)

Homo sapiens zinc finger protein 426 (ZNF426):

cgttccttttgtgacgccggctgtgagcgcctgagagtctttttgcctttcagagttaag
gcctcactggcctgggaaaataattgctgccttttgcatccgcgttggctccgtccccag
gatcttcccggttcagggacctggcgatttctgagtgttccggaatcccaataaccctgt
ttaaagaggaatggagattgccactgtccatttagattaatgaggtgtcctgaagtgatg
gtgacatcaatgaaaggagggttctgacacgttctcacctcgcgggatg
(Seq ID No: 935)

Homo sapiens TATA box binding protein (TBP)-associated factor, RNA polymerase I, D, 41kDa (TAF1D):

caaccctttcttccgcacggttggaggaggtcggctggttatcgggagttggagggctg
aggtcgggagggtggtgtgtacagagctctaggacaccaggccagtcgcgggttttgggc
cgaggcctgggttacaagcagcaagtgcgcggttggggccactgcgaggccgttttagaa
aactgtttaaaacaaagagcaattgatg (Seq ID No: 936)

Homo sapiens PHD finger protein 1 (PHF1):

ccgcctcctcctcctgccgctgccgctgctttggctgctgcgtcatacgccccagagccg
ccgggacggaggggctgggcctggggacccccggcctccgcctgcacgccccccacgc
ccggacgtgccctctccgcgcgggggactcgcctaggtctcctacgtctgcccctgcccg
gctcccggcggccccagctgtcaccggccccccaggatgcaatg
(Seq ID No: 937)

Homo sapiens family with sequence similarity 134, member A (FAM134A) :

ccccctccgcctgacgcgcccccggcggcggccgcgcagccctggctcctcgcgggctc
gggcggcggctgcggcggggctatg (Seq ID No: 938)

Homo sapiens membrane bound O-acyltransferase domain contain ing 7 (MBOAT7):

ccgcctcctttccggagcccgtctgttccccttcgggtccaaagcttttggctcctcctt
gttccgagcccgaaggcccgccccttcacgtactcggagctcggatcccagtgtggacct
ggactcgaatcccgttgccgactcgcgctctcggcttctgctccggggcttcttccctgc
ccgcccggggccctgaccgtggcttcttccccggcctgatctgcgcagcccggcgggcgc
ccagaaggagcaggcggcgcgggggcgcgctgggcgggggaggcgtggccggagctgcgg
cggcaagcgggctgggactgctcggccgcctcctgcccggcgagcagctcagaccatg
(Seq ID No: 939)

Homo sapiens major facilitator superfamily domain containing 11 (MFSD11):

acgcccctttttgctcagccgtcagccccgtctccgtctgaagagtgcttctgccctca
tttgcctctccctgtgaccccggccccctcagactccgctgcgtcgtctctcggccccgt
ccagccgttcctgactgctcttcgccggagtccgcttcccaacccccttcgccagagcc
cgagagctccgtcggctctgcgtcctggcggattgtcagtggcttcgccccgaggagagc
tgactgccctggctgctgcctccggcagagctgagccaaaatg
(Seq ID No: 940)

Homo sapiens thiamine triphosphatase (THTPA):

ctcccttccccctctgtgggtcccgcgaggagactctcgggctttgaggtgagacctgaa
gttccgctggccggtagtgtagcaggaaagggcaggtcctcccgggtcgtgagccagtag
cctcctggggtggcaaggtgtagagagggggcgttgaaaggacacccgctacccggcct
gctttctaggggtctctttggattgaggacatcagcagcagtggaagggattttactgga
gacctgtcactgtcagagccttaaaatatcaccgacggggccttaatgtcaccgaggtag
agagaaaagggcagtagccctagagactattgcgacacagtgtgcccctcataagtttt
ccagggaggggttctgtactgagttgacgccccaggagctgagcaccaggctttgcatcc
ttgggaactcagcaaacgtttgttcagccaattgcaggtagcatg
(Seq ID No: 941)

Homo sapiens acyl-CoA synthetase short-chain family member 3 (ACSS3) :

tactcccttccctcaggccccaggaagttgcaagagtaccatttgtcgcacactcgggga
ccgcgggtggccggaggagatg (Seq ID No: 942)

Homo sapiens chromosome 6 open reading frame 211 (C6orf211):

gctcctccttcgcggcggtaccgcctctgtttctgcggcgattgaacagccgagctttgc
ggccgggatcgcggaaagtgatg (Seq ID No: 943)

Homo sapiens transmembrane protein 204 (TMEM204):

atttcctctctgctgagagccagggaaggcgagctctgcgcacacgggcgtccctgcagc
agccactctgctttccaggaccggccaactgccctggaggcatccacacaggggcccagg
cagcacagaggagctgtgaacccgctccacaccggccaccctgcccggagcctggcactc
acagcaggccggtgctaaggagtgtggcgcgggctcgactcccactgctgccggcctccc
gagtgactctgttttccactgctgcaggcgagaagaggcacgcgcggcacaggccggcct
ccgcttcccgggaagacggcgcactcctggccctgggttcttgctgctgcccaccctctg

ctccctgggatgggccccgaggcgagcagcttcagcacaggcctggccctgctccaggtg
caggaaggaggataaggccgggccgagaggcggcacacctggaccatcccatgggcctcc
gcccgcgccgccccgaggatgagtggtgatgtcctctagccacccctagcagcgtcggct
ctccctggacgtgcggccgcggactgggacttggctttctccggataagcggcggcaccg
gcgtcagcgatg (Seq ID No: 944)

Homo sapiens DEAH (Asp-Glu-Ala-His) box polypeptide 40 (DHX40) :

tcgtctttcccctcccatctcctcagatcggtggacgtgctcgcctccactcggggccag gtctatg (Seq ID No: 945)

Homo sapiens importin 4 (IPO4): cctccccttttcggcccagtagcggcggctcagttgctgccatg (Seq ID No: 946)

Homo sapiens N-acetyltransferase 10 (GCN5-related) (NAT10):

ccttctctttcggagttgttccgtgctcccacgtgcttccccttctccactggctgggat cccccgggctcggggcgcagtaataatttttcaccatg (Seq ID No: 947)

Homo sapiens lin-28 homolog A (C. elegans) (LIN28A):

aaccctttgccttcggacttctccggggccagcagccgcccgaccaggggcccggggcca cgggctcagccgacgaccatg (Seq ID No: 948)

Homo sapiens CAP-GLY domain containing linker protein family , member 4 (CLIP4):

cggcctttcctccgcgcccccgcgtccccagccggccgctccgagaggacccggaggagg caggtggctttctagaagatg (Seq ID No: 949)

Homo sapiens zinc finger, AN1-type domain 1 (ZFAND1): ccgccccttacggcgccggagagatg (Seq ID No: 950)

Homo sapiens GTPase, IMAP family member 6 (GIMAP6):

cctccctttttctacttccgaggctgcaaagtgcaacagcagactcttctgactcaggaa ggccggtgctcctacccacttcctgttcctccatctccagcggacactgctctttcaagg gcaggtctccagcccagctctctgaaaacatttgctgaaaatataagcaaacatcggcc ttgtcctccttgtgttcatacactgtggaagcttttctctgcctcctccgtgagagtgcg tggccgggagaccagaaacgtggtcctttctcttgcctgtgagctggtgcagagatg (Seq ID No: 951)

Homo sapiens thioredoxin domain containing 15 (TXNDC15):

cttcctccggctggcagcacgactcgcgtagccgtgcgccgattgcctctcggcctgggc aatg (Seq ID No: 952)

Homo sapiens asparagine-linked glycosylation 9, alpha-1,2-ma nnosyltransferase homolog (S. cerevisiae) (ALG9): aattcttttttccccaggcttgccatg (Seq ID No: 953)

Homo sapiens glutathione S-transferase, C-terminal domain co ntaining (GSTCD):

acttccctttttccggtccgccggattatgaatgacggccggcgcgagtattttccacat

aaggtggctgtcgtttttctcctggcgtctgtggaggcgagtggtctgcgggcagcagct
cccagaggcagccttggaattccagctcggactgggcgggaaggcgcaggcggcccaggt
cgccgacacgctcacgcaccctccctgcctggccgcgcctctgcgaccaggtgacccaat
gaaagaagaaaatg (Seq ID No: 954)

Homo sapiens CXADR-like membrane protein (CLMP):

actcctttttctttccaaacagggaaaagtgttccacgaagcggtagcgcctttccgcct
cgcgtttcctccctgaccctggtcccggctcccgtccgggcgccagctggtggggcgag
cgccgggagcccatctgcccccaggggcacggggcgcggggccggctcccgcccggcaca
tggctgcagccacctcgcgcgcaccccgaggcgccgcgcccagctcgcccgaggtccgtc
ggaggcgcccggccgccccggagccaagcagcagctgagcggggaagcgcccgcgtccgg
ggatcgggatg (Seq ID No: 955)

Homo sapiens nonhomologous end-joining factor 1 (NHEJ1):

cctcctcttgcggtgggggaaagcggcctcttactctaggcctttcggtttgcgcgagc
gggcaggaaagcgtgcgtgcggctaagagagtgggcgctctcgcggccgctgacgatg
(Seq ID No: 956)

Homo sapiens gametogenetin binding protein 2 (GGNBP2):

cctccttcttccactccccgcggcgcgagcggctgactgcccgtagaggaaacgacattc
ggagctgcgctcccgcccaggccggccctgacgcgggcctcgtcagccagtaacagggag
cagaggtgggagttagcgaggcgaccacgaaaacggtgaaggtcggaaccgacagcctcc
tccgagaagggcaggagctgggaggaggcggcagcggcggcggcagaaacagcagcggcg
gcggcggcggcagctgggaggaggtggtgacggtggcaacggcagcgtcggggacgatg
(Seq ID No: 957)

Homo sapiens zinc finger protein 672 (ZNF672):

ctttctcttttagccccgcctgcttcccggctccagctggggccggagaggctgagtggt
tggtacgctgctcgctggcctcccagtcttcccagcaaccggtgacactgcccgcgccag
actgaccactagccgacgcgggcgagagggacaggagcgtgacctccccatcccgagggg
ccggacgctcgggcgcctccccgctccccccactcggaggccgcgcgcgccgttagcccc
ttcctcgctcccccgccccagtcccgcagtccgggaggcgggggtcggcagccggctgag
tgggaaccgcgcggtgtctgaggaggcagtcggcgaccggtttccacttcaagcgtgacc
cttttgcctgtgggatgagctccagcatggggtgaggtacagaagagagacttgaagagc
gtgccttgggactcaagcgccaaacctgtaccctagcgagtgtcctactccgcatccgta
atggaaggaaatgcacatcttactccagaggcacaagaggaggacatcccatgcggctac
tcctgcccagcgtggtggggcagcagaagctccagagcccagacttgcaggctcacggtg
cagggtgaacctggccacagctcaccctggaacagccacaatgtctgccccttagagaag
aaccctgaaatcagaccagtttttgcggcctcccccttcctctctgttacagtgccctt
tccaggccttaagagaagtaaaacttagctgcagcgccaggaggtggaccccagagtgtg
agtggcacgcttccctgtgaacccgtcctcaccatg (Seq ID No: 958)

Homo sapiens N(alpha)-acetyltransferase 60, NatF catalytic subunit (NAA60) :

ccgcctccgtcccggctgcggcccctgccggttacataactcgttgcggctccgcgcgg tcccacttcccggctcccttcgcctccaggatgcgctgagccctacaacacccccagcgg ccgccggctcccccacgaggtgtgaatg (Seq ID No: 959)

Homo sapiens transcription elongation factor A (SII)-like 4 (TCEAL4) :

tgccctctgtccccgcggctgggtctcgtctgctccggttcctgggctcctaattcttgg tccagcttcttccaggtcagtgtgcgggccttccacgctgccagcggaacactggaatgg cggaaggggaacgggtctgcgcgtctgttgttcccagcgctctgcgaagcctgaaaagga ggagcaacctgtccagaatccccgcaggacaggaaaaggaggggaaatctcgacatg (Seq ID No: 960)

Homo sapiens progestin and adipoQ receptor family member VI (PAQR6) :

tcccctttgtctccccactccccgcccaggcctggcccgcctgcctggccactcttcctc catcagcctggctggcagcagccttggactccgcccgtggagccctgggcctgttgaccc accagcttaggagcacccaccaagctctgggtaaggaagctcaccttctggggctcttct gggaaaatagaggtcaacgtggaggtaccaggccaccatgctcagtctcaagctgcccca acttcttcaagtccaccaggtcccccgggtgttctgggaagatggcatcatgtctggcta ccgccgccccaccagctcggctttggactgtgtcctcagctccttccagatgaccaacga gacggtcaacatctggactcacttcctgcccacctggtgaggggaggctctgccccaggc cgcggccttgagctcagaggggtacccaggcgggcagggaccgtccaggcccacgggct gcagcggcagtcgcgggggtccgcggcggcctgagcacgcgcccgccgcaggtacttcct gtggcggctcctggcgctggcgggcggccccggcttccgtgcggagccgtaccactggcc gctgctggtcttcctgctgcccgcctgcctctaccccttcgcgtcgtgctgcgcgcacac cttcagctccatg (Seq ID No: 961)

Homo sapiens DENN/MADD domain containing 2D (DENND2D):

catccttcttgctcaaccactgggtgcacaggatggaaacttctattccctctctggaag acagcgcgtggcttggcttcacagagttgtggctggagaccgaagcagcccctttctcag gcttactgtcaccagtctgtctgtgttaggggagaggggagtccgctctgtcctgaaggc ccagagatg (Seq ID No: 962)

Homo sapiens family with sequence similarity 188, member A (FAM188A) :

ccttcttctttcctgcctcaccttccaattcgtttgccgccgccgtcccgcagctgctgt ttccggagttgccccttccccatgttccggggcaggagtccgcaaagcgaagatccgccc gccggttcctcatcatg (Seq ID No: 963)

Homo sapiens neurensin 2 (NRSN2):

ccgcctttgctcggcggagacagcaggcagagagatgaggaaactgagacccagaaaggt ggaagcacttgtctaaggtcacgcctccaggaagcagtgtgtccacgactccagtccaag tggtcaggctccagagcccacagtcccaggggtccatg (Seq ID No: 964)

Homo sapiens tripartite motif containing 46 (TRIM46):

agccctcctcacacccccactgggctcctgcattaagcccggggttcgcagccgcagccg ggatcgggcacccaggggcgggcggcacggtagggccatg (Seq ID No: 965)

Homo sapiens target of EGR1, member 1 (nuclear) (TOE1):

catcctctctgggaatttaccgatgcccagaacgcccttctttcccccacacgaccctct
cctagtctaactcctgggcgtgctttaagctcagctcaggcagcgtcaccttctctggaa
agcccaaacccagccaccccactacccgctacccgcggcccacgctgatgaagacagcag
aacacggaggccccgcgttcccgccgcgagagcaggagagaaagattacctcccgcgagc

tctagcgcgcccggctttccggcgcactccagggggcgtggctcgggtccacccgggctg
cgagccggcagcacaggccaataggcaattagcgcgcgccaggctgccttccccgcgccg
gacccgggacgtctgaacggaagttcgacccatcggcgacccgacggcgagaccccgccc
catccccgactgcctgaaccgcgccaggagacggaccgcaagtccagcgtacccacagac
gactcaggcgggagacgagcggtgtcatg (Seq ID No: 966)

Homo sapiens DBF4 homolog B (S. cerevisiae) (DBF4B):

cgttcttttaggggtggagccggcaggaaatttaaactgaagccgcggccgaaaacgcca
agagattgatgctgtagctgccctgagataaccaggactgtggaatcggaagagctcat
ggagctcgcgaatgtaatacggaggcctctgaggaaggagtacggaggccgagaaggagc
cggcatttgatg (Seq ID No: 967)

Homo sapiens myc target 1 (MYCT1): atttccttttatg (Seq ID No: 968)

Homo sapiens myosin XIX (MYO19):

ggttcctttcctcactgcacgctcttgcccctcctcttttctctcctgcccgtgttcttc
ccgccgcctgacctggcccgcccgcctttccagtctggccgggcgggggcctgaagcacg
gcggctcgggccgtgggaccgtgttcacaccctttccagaaattcttggctggtaaccgc
gaaaccgactggagcaggagctgggagaactggagaaaactgctctaatctcacttgact
ccagctaggagctgatgctgcatcgtaataacatttgcagagcgctttcacaggcgctgg
agtgacttgtctgagattcctccagaactgagccctttgttggaaccataccccagccca
tggtcccatgactaggtggatagtactccttgtacctcctgcaacccagaaccctggctg
accactttgaaggaggatg (Seq ID No: 969)

Homo sapiens KIAA0226-like (KIAA0226L):

cctcccctttctgctgttaccgggagcgcggtggccacggaacgctgcccggagccgcgc
gagggaggacccgacgcgcggcgtttacccagcgcagcgttccaccgctcgggtttggct
ggataaaataaaaaatggggatattgacctcctgtcactactgcatggactttgatggtt
tccaatcattactttctcctctgtgtcaatctgcctcttcgagaaattcatactcctgaa
tagctctccagaccccccagctggccatgtggtgagttcagggcccaaatcaagtagtacc
agcaatcagggaactcctatctgttttgaatggattcacaccagccacaagcctggaaag
atg (Seq ID No: 970)

Homo sapiens MUS81 endonuclease homolog (S. cerevisiae) (MUS81) :

ctccctcttcccccgccccgccctgggccaggtgttcgaatcccgactccagaactggcg
gcgtcccagtcccgcgggcgtggagcgccggaggacccgccctcgggctcatg
(Seq ID No: 971)

Homo sapiens zinc finger protein 430 (ZNF430):

gggcctttgtccctcgctgtggcctgagctccaggtctcgtcttcagcgctctgtgtcct ctgctcctagaggtccaggctctgtggccctgtgacccgcaggtattgggagatctacag ctaagacgccaggaacccctggaagcctagaaatg (Seq ID No: 972)

Homo sapiens mutS homolog 5 (E. coli) (MSH5):

gctcctttgcaggctcgtggcggtcggtcagcggggcgttctcccacctgtagcgactc aggttactgaaaaggcgggaaaacgctgcgatggcggcagctgggggaggaggaagataa gcgcgtgaggctggggtcctggcgcgtggttggcagaggcagagacataagacgtgcacg

actcgccccacagggccctcagaccccttccttccaaaggagcctccaagctcatg (Seq ID No: 973)

Homo sapiens proline rich 3 (PRR3):
gccccttcctcactaccctccaaatcccgctgcagccattgccgcagacacgatg (Seq ID No: 974)

Homo sapiens sirtuin 2 (SIRT2):

cgccctttaccaacatggctgctgacgccacgccttctgggactcgtagtccggtcctcg cgcgctttcttacctaactggggcgctctgggtgttgtacgaaagcgcgtctgcggccgc aatgtctgctgagagttgtagttctgtgccctatcacggccactcccatttctggtgccg tcacgggacagagcagtcggtgacaggacagagcagtcggtgacgggacacagtggttgg tgacgggacagagcggtcggtgacagcctcaagggcttcagcaccgcgcccatggcagag ccagaccgactcagattcagactctgagggaggagccgctggtggagaagcagacatg (Seq ID No: 975)

Homo sapiens KIAA1715 (KIAA1715):

ttgtctctctgtcagtggcggctgctgcctgctctggaggcaggctgggcggtggcggcc gagactggcgggggtggacgcccgggccgggctgcgcccgcttcttgcagctgtgaattc ctttggacaattgatgatatttatcattgtgcccagtttctacaaataaaagatg (Seq ID No: 976)

Homo sapiens proline-rich transmembrane protein 1 (PRRT1):

ctgccttcatctctccatctctgcgctgctgccggctgcgccatccagcacccagactcc agcaccggccgaggacccccactccggctgcagggaccctgtcccagcgagaccgcaggc atg (Seq ID No: 977)

Homo sapiens t-complex 1 (TCP1):

ccgccccttccccggagcctcacttccgtcacagtcctgtttctctccctgttgtccctg cctctttttccttcccgccgtgccccgcggccgggccggggcagccgggaagcgggtggg gtggtgtgttacccagtagctcctgggacatcgctcgggtacgctccacgccgtcgcagc cactgctgtggtcgccggtcggccgagggccgcgatactggttgcccgcggtgtaagca gaattcgacgtgtatcgctgccgtcaagatg (Seq ID No: 978)

Homo sapiens Yip1 domain family, member 5 (YIPF5):

cgttctttggccctgtgacacgtagcaacggggctggttcagggtctgaaacagagtttg
ggggttgtttgggattagtgaagctactgcctttgccgccagcgcagcctcagagtttga
ttatttgcaatg (Seq ID No: 979)

Homo sapiens glucose-fructose oxidoreductase domain contain! ng 2 (GFOD2):

cctccctttccagagcccccagttccttagaaaccaggcggcgcgttcccggtggcggcg
ccctggactcccgggcccgcgcatccccgccagccttccttaaggcggatgggtggcccc
cgagaccccgtcggacccatggtttccagtgcagcgcggagtgggcgatgccagcgtgcc
aggagccatgtctgaccaggacgtttggaagatcatatccatgccagaggctcttgtgag
gagatgagttggtaaagagagaggctgggatg (Seq ID No: 980)

Homo sapiens apolipoprotein L, 2 (APOL2):

ttccctttcgaattccagggtatatctgggaggccggaggacgtgtctggttattacaca
gatgcacagctggacgtgggatccacacagctcagaacagttggatcttgctcagtctct

gtcagaggaagatcccttggacaagaggaccctgccttggtgtgagagtgagggaagagg
aagctggaacgagggttaaggaaaaccttccagtctggacagtgactggagagctccaag
gaaagcccctcggtaacccagccgctggcaccatg (Seq ID No: 981)

Homo sapiens microtubule-associated protein 4 (MAP4):

ccgcctccctgcgccccgcccctccggctagctcgctggctcccggctcctcccgacgtc
tcctacctcctcacggctcttcccggcgctctcctggctcccttctgccccagctccgtc
tcggcggcggcgggcagttgcagtggtgcagaatg (Seq ID No: 982)

Homo sapiens exonuclease NEF-sp (LOC81691):

cttccttctttgccaggcagacgcccgttgtagccgttggggaaccgttgagaatccgcc
atg (Seq ID No: 983)

Homo sapiens ST6 (alpha-N-acetyl-neuraminyl-2,3-beta-galactosyl-1,3)-N-acetyl galactosaminide alpha-2,6-sialyltransferase 5 (ST6GALNAC5):

ctgtctctaatctctgcaacagccgcgcttcccgggtcccgcggctcccgcgcgcgatct
gccgcggccggctgctgggcaaaaatcagagccgcctccgccccattacccatcatggaa
accctccaggaaaaagtggccccggacgcgcgagcctgaggattctgcacaaaagaggtg
cccaaaatg (Seq ID No: 984)

Homo sapiens heterogeneous nuclear ribonucleoprotein A1 (HNRNPA1) :

tgctcctttctgcccgtggacgccgccgaagaagcatcgttaaagtctctcttcaccctg
ccgtcatg (Seq ID No: 985)

Homo sapiens zinc finger protein 93 (ZNF93):

gggtcctttgtctctcggtgcagccggagctccaggtctcctcttcactactctgtgtcc tgtgctcctacaggcccagcctctgtggccctgtgacctgcaggtattgggagatccaca gctaagacaccaggacccctggaagcctagaaatg (Seq ID No: 986)

Homo sapiens N-terminal EF-hand calcium binding protein 3 (NECAB3) :

cggcctctagccacaccgagtccgccgcggcgtccagggtcggcagcaaccgcagccgag cccgagcgggtggcggcgccatg (Seq ID No: 987)

Homo sapiens splicing factor 3b, subunit 5, 10kDa (SF3B5):

cattcttctgcgacggcgcggacctggagcttccgcgcggtggcttcactctcctgtaaa acgctagagcggcgagttgttacctgcgtcctctgacctgagagcgaaggggaaagcggc gagatg (Seq ID No: 988)

Homo sapiens INO80 complex subunit B (INO80B):
gtcccctttcctcgcaggacctcatg (Seq ID No: 989)

Homo sapiens polyamine modulated factor 1 binding protein 1 (PMFBP1) :

ctttcttcctcttggcttatattagggataggggatgtggtttgttacaaaggatgagta ttttgatagcttctcattccttgaactattctgcaggtttataacaaagctcagaaaata ctaaaggttaaaggagaattgagagctgccaaggaaatg (Seq ID No: 990)

Homo sapiens pseudouridylate synthase 3 (PUSS):

cttcctttctcggaaacgcggcgcggccggctgccggaaaacagggcagacctgtatggt tcgtttattcctggggttgtcatatcatg (Seq ID No: 991)

Homo sapiens heterogeneous nuclear ribonucleoprotein D (AU-rich element RNA binding protein 1, 37kDa) (HN-RNPD):

tattctttttagtgcagcgggagagagcgggagtgtgcgccgcgcgagagtgggaggcg aagggggcaggccagggagaggcgcaggagcctttgcagccacgcgcgcgccttccctgt cttgtgtgcttcgcgaggtagagcgggcgcgcggcagcggcggggattactttgctgcta gtttcggttcgcggcagcggcgggtgtagtctcggcggcagcggcggagacactagcact atg (Seq ID No: 992)

Homo sapiens GABA(A) receptor-associated protein like 1 (GABARAPL1) :

atttctccatctggctctcctctacctccaggcaggctcacccgagatccccgccccgaa cccccccctgcacactcggcccagcgctgttgcccccggagcggacgtttctgcagctatt ctgagcacaccttgacgtcggctgagggagcgggacagggtcagcggcgaaggaggcagg ccccgcgcggggatctcggaagccctgcggtgcatcatg (Seq ID No: 993)

Homo sapiens chromosome 22 open reading frame 13 (C22orf13):

ccttcctttccccagtgttgagcgcggtctcgcctccgcttcctcctcactccgcctgcc
ggctgggaaactagggcaccagtacgatagttccggcaccggaaaagagggctgatgact
gggcccgggggccgccgcaacgaccttggggccggcaaagagccagagagggtgctcac
acttccaagcaccccacaccaaggacaggctggacggcaaggcggagacgcggggcttgg
gccctcagaccggggacagcaggaggttgggccaagggccaggacttcccgtcacaattt
catttgttgatcccggcaccgccaggtaaggggggccctgagtgaggctaggtatctggt
acggataaagttaggtatagagtagagcggctgcccgctcagggttatccctaaagacag
ttggaggagagttgcttggggcctcggggatgcactgggcgggatcagggcttacaccta
ggactggcaaaagagcgggacccggcagaggcggggcttgccgaagggacgagcctctat
tcaggaaatgcacgagctttggggcggggctcaaagaaaggggcggggcttccgggggccc
gcgtcctggtgagctgcgcgtctgcgcgaggattgggcgagagggtggggccactcaacg
ctgaggcggcgaatggccggagcagacttaaatcaagaggctggggacctctaagatcaa
agtttggggcggggcctaaggagggggcggggcctccagattcgagacctggaaggctg
gggcggcgcttggggcggccctgccgccgcctcccgttctcccctccgcagcggcggcgg
tggcggagaaggaactcgacacgcaccgaccgccctcccgccccagccgaagcggaagct
gtagcccgctctgggccggggccatgggcgccccgcgccgcccgggtcatg
(Seq ID No: 994)

Homo sapiens lon peptidase 2, peroxisomal (LONP2): ggctcttttgacagcccccagtgcgaaaggctgccagcatg (Seq ID No: 995)

Homo sapiens RNA binding motif protein 4B (RBM4B): ggttctctctgacgtgggagccgccgtcgctgccgccacccggaggctcttgtcaggatg (Seq ID No: 996)

Homo sapiens protocadherin alpha 3 (PCDHA3):

aggtctttctccacaaaagaaataacagcgtgcattacgtattcagatactgctttgctt
catcctctctaaaatttaacaccgaggagtttaagaaatgaagataaggaactcgaatta

tttttaaactttggatcaatgtaaaggcaatctaatatttggaaaatacttgcaatg
(Seq ID No: 997)

Homo sapiens RAB34, member RAS oncogene family (RAB34):

gcctctccttgggccccttctctccccctttcccctccctgctggttcctggcatcgcca
gatgctgcgcagcagtctccgattccccatcaccaattcggctggcgtctccgagaccgc
ggactcccgtagggtccccgtggccccgagttgtagtcgggacaccccggccgcgggtga
tcgtcgggtctccacgcgcccgggtcgctgacgcggatccggcctcggcgccttctcagg
gcgccctgcaaggccgcaggcaggatg (Seq ID No: 998)

Homo sapiens cell division cycle associated 7 (CDCA7):

gctcctcctgctgtgggaccgctgaccgcgcggctgctccgctctccccgctccaagcgc
cgatctgggcacccgccaccagcatg (Seq ID No: 999)

Homo sapiens ArfGAP with GTPase domain, ankyrin repeat and P H domain 3 (AGAP3):

gggtcttttaggagagcactgctgcagccggcagtggagagcctgggcagggagacaggg
agaaaactccggcagcagggtggtctctagggctgacctcggagcctggggacaggggag
cctatgccgcactgaaggcgggacgctgtaagcgaggagcagctgggcctgggcggactc
ctcggccaatcagcctcggtcagcagcaccctcaggcgcagggcactgtttgggcattgc
ctagagatccgacaccccgcccagatcagcgcagggaggcgaaagcgacagccgggcgcg
ggaggagaccagggcagctgtcccctccgcgagggtggccctcgaggcaatgcgggtggg
ggctggtgaggaggcggaagggccgaggctgagtgggagggccggggcgccagggctgg
agcgcgcggctcgggggtggaggctgcagagccagcgagcgagcgaggggcgggggcgcc
cgggccggcgcgcaggagggggcgggggcggcgggggaggggggctcgggctgcgtgtgccg
gagccggcgggggcggcggtgcgtgcgcatgacgcgggggggaggcctgggccgcgcgct
cccggtcccgttgttgttgccgctggaggctgctccgaggcagcgggatcacggcgctgg
gaagcgctcggcagcggcggccacagcgtgcgcggcggcgcctcctggcctcggcctccg
gcccccggcccccggctccatgcgctagccccgcgccgccagcccagtagtcccggcccc
gccagccccgcgctcccgctcgccgctgccgccgccgccgccgccgccgcctccgccgcg
ccgccccgggcccgcctcgggccccacggctccgaagccatg (Seq ID No: 1000)

Homo sapiens potassium channel tetramerisation domain containing 10 (KCTD10):
ctgcctctctcagtccgggtttggagactcctgcgtcctccgacttttcatg
(Seq ID No: 1001)

Homo sapiens cyclin B1 (CCNB1):

cattctctgcgaccggcagccgccaatgggaagggagtgagtgccacgaacaggccaata
aggagggagcagtgcggggtttaaatctgaggctaggctggctcttctcggcgtgctgcg
gcggaacggctgttggtttctgctgggtgtaggtccttggctggtcgggcctccggtgtt
ctgcttctccccgctgagctgctgcctggtgaagaggaagccatg
(Seq ID No: 1002)

Homo sapiens eukaryotic translation initiation factor 2A, 65 kDa (EIF2A): gtttctctttccgggacaacatg (Seq ID No: 1003)

Homo sapiens protocadherin gamma subfamily B, 7 (PCDHGB7):

cagcctctagcctgggattccctgcgcagccaacaacagaaaagaaaaccagctcccaca
cagaggctcccggctgcgcagaccttgcccagcacaccagattgccagctccgagacccg

ggactcctcctgtcctggccgaatgctcttttagcgcggtagagtgcactttctccaac
tggaaaagcggggacccagcgagaacccgagcgaacgatg (Seq ID No: 1004)

Homo sapiens acyl-CoA dehydrogenase family, member 11 (ACAD11) :

ggctctttcggcttccttcctcgctgggccggctaaacccggccgcagcagcaccggggt
gataagtgtccagggcaggaggccagcgatgttgccttgctaaccgggtatctaagagaa
acagggtctttttattcttaggctcgacagtctgacggccctttttctgaacgggaccct
gcaggtcttccgcctgctgttgcattaaatttggggggtggaagaggcttctgcgttgttc
cttacccgcaacgatgaccatggctttgccttctttaaaattgaggcctccaactctgac
gctgactggagaattgaaacccgaacacacattgggctcttttggcacttgactagagct
aaaacctcgggattcagcgggcaagcgttgctcagcaacggcgcgtaggctgtgtgcggt
tggctggagccagaccccaccccggcctcggcccatgctctagaggggacgttgccccaa
tcctgaaggacttcggcactcgagacctgtggatgccgcgttgctgtggcctgcgggggt
gatcatg (Seq ID No: 1005)

Homo sapiens zinc finger, CCHC domain containing 7 (ZCCHC7):

ccgtccctctacgcgttttggttcccggttggtgcttcctgttcgcagctgcggcacttc aaggttactgactttttatg (Seq ID No: 1006)

Homo sapiens zinc finger, MYND-type containing 12 (ZMYND12):

gggcctttctggacttggactccttgggagtcgtttctcggccatttgacccgtgggact tgtgggttttgtgctgctttttctttctttcttcccctttccaacttcagcaatacacc cagatgttagtcgagtcacgtcccgccgccctctgcccttgaaatgctggcaagtacgca gccccgcgatcgtcacgtgacgccggggttcagcgtatccttgctgggcaaccgtcttag agaccagcactgctggctgcaccatg (Seq ID No: 1007)

Homo sapiens forty-two-three domain containing 1 (FYTTD1):

cgctccctcggtgcggcgggctgcgtgcgcgagtgggaggtggcaggcctgcgactccgg ccttgtccgcgcccgctctcggcgcgacgtctccagccatg (Seq ID No: 1008)

Homo sapiens SH3-domain GRB2-like (endophilin) interacting protein 1 (SGIP1):

ctccctttctctcagcatcttcttggtagcctgcctgtaggtgaagaagcaccagcagca tccatggcctgtcttttggcttaacacttatctcctttggctttgacagcggacggaata gacctcagcagcggcgtggtgaggacttagctgggacctggaatcgtatcctcctgtgtt ttttcagactccttggaaattaaggaatgcaattctgccaccatg (Seq ID No: 1009)

Homo sapiens GTPase activating Rap/RanGAP domain-like 3 (GARNL3): cagccctttttgcaaatg (Seq ID No: 1010)

Homo sapiens DCN1, defective in cullin neddylation 1, domain containing 5 (S. cerevisiae) (DCUN1D5):

gagcctcttgcttgctgtgactggtggagctgccgcgctgtccgcgttatctcctcccgg tgagaacgaaccgcagtgtccaccggcgaggagccagccctgtcccggtcagagaaagac gacgaggatacctgggagcgggcggcggccgggctgggccgcgccggtgcgggctggcga ctctgctcctccgcttgctgctgtctctgggaactgggtgccagcgctgaggggcttcca gcggacagggaccccccttccccggctcccctgcccaccctgccggggagggcggaagatg (Seq ID No: 1011)

Homo sapiens alkB, alkylation repair homolog 7 (E. coli) (ALKBH7): tgccctctctcatgaccccgctccgggattatg (Seq ID No: 1012)

Homo sapiens nitric oxide associated 1 (NOA1): ccgcccctttggagctacttcctcatg (Seq ID No: 1013)

Homo sapiens BTB (POZ) domain containing 10 (BTBD10):

tcgcctcttcgcattgtgagctctcgcggtaagaggctgaggagccggcctgcaacctgc cggggcggctccgctacgcgcagccgcctcagtggcttcctccacagccacctccggagg gatctggctgaggaggaagtggaggtgtcactggccccggcctttgccccaatcttgtgt gggcactgaagggggactacaggttcgagagttatgggtgctacatgtgtgctttcagag cagtagtgtgaggaagcttggagtgggatg (Seq ID No: 1014)

Homo sapiens zinc finger protein 397 (ZNF397):

cggtctttgtggcttgcagctcggggtgggtggctcatttcctggccgctcctgggcttc
gcggaaagaagagattactcacactccttcgcaagcacagaaccagttgtactgagcttt
ttgctaagctgtttcagccaagaatg (Seq ID No: 1015)

Homo sapiens mitochondrial ribosomal protein L45 (MRPL45): gctcccttcccggcggcctttgcgggaacaagatg (Seq ID No: 1016)

Homo sapiens AKT1 substrate 1 (proline-rich) (AKT1S1):

cttccttctccatattgtatactggaattgaagccaaggaggtaccattttgctcgaggg
catggcctaagccggtcagctaaggccatgttaatacggggctgtcccatctctctgcgg
ggcgcgacagctggaagagccgaacggataagagaagaggaggtgagaggagctgtacac
cacaagaggcactgagggactcaggataacgggatgaagccgtcagtgcccccagaaacg
aagcggccccggacgaatttctgagtcaccgtcgcgagaaagcgggctgagccgccattt
tgaagcctggcaaaccgaagcaagaaatgctgccgtgttggatctttgccagccttcgtg
ccgaatgggagcaggttggagggagggagagccaatatacactatgggctgattaagccc
ggttggctgccatgttgttaacgagcaccgatttcctctactttgtcgaagaagtttat
tgtgggtcagggacgtcaggtcgcttgccttcgtttactgtggtcatgattgagcatatg
aggacggccattattgttgggggcaaatggaaatgctctaggcggggccatttttcttag
gggcaagctgtcgtcacccttgtcaactggttcggatgaagcccctgtggccgccatctt
gatctcgggcggccccgataagggaggcggagtgtgcggagaggaggcggggcaactgcg
cggacgtgacgcaaggcgccgccatgtcttttgagggcggtgacggcgccgggccggcca
tgctggctacgggcacggcgcggatg (Seq ID No: 1017)

Homo sapiens transmembrane protein 101 (TMEM101):
ctgccctttcccaagatg (Seq ID No: 1018)

Homo sapiens eukaryotic translation elongation factor 1 delt a (guanine nucleotide exchange protein) (EEF1D):

ggccctccctttcatcagtcttcccgcgtccgccgattcctcctccttggtcgccgcgtc
cttggctggcgttagagacagggtttcaacgtgttagccaggatggtctcagtctccaga
ccctgtgatccgcccgcctcggcctcccaaagtgttgggattacaggtgtgagccaccgt
gcctggccgaggctccttcttttatg (Seq ID No: 1019)

Homo sapiens ADP-ribosylation factor GTPase activating prote in 2 (ARFGAP2):

cgccctccccgccgtggattggcccgcggcgggacccgtcagccgcggttgtgtctggga
aggagagaaaatg (Seq ID No: 1020)

Homo sapiens junctophilin 4 (JPH4):

atttctctcctccctggggggtctcagtgcatctccttctcctctctgcctgcctcctccc
tcaccgaagggttagcggacacccatccttttctgcttggggaccccaccaccacccgca
acactgccgctgtctcttcttcaccgtatccttctctacccaccctcttctctcttctct
tctccctgcccctttaaatctgcctggcccagcctcccccgtgatgctgggatggagcaa
acattgatttgtgctgggatggaatcggaattttgatttatttttcctctcccaaccata
agaagaaaaaaataataaaaacaccccctcttgagagcccccctcccccctttgcatccagc
tcccagctcttcttccctatctccatccaaggcagatttttccccctacactattctcat
cttcccccacccttgccactacctcgccccccccacccagcctgctcctccagctggggag
agaggggactctccggactcccccacctttcctctctgggttggagcagtctctccggaa
ggggagggggcttggcttgtccgggcgaggtgggagtggaggtatcctgccatggatgct
gtgccggggaggcagcctgagccccagcccacatgagacgccgaagaaccggggcagagg
ggtcctgacagcagccagggaaacgggtgccctacgattctgcccagcccccctctcagga
cccccaaactgccatccacactcgacacttcggggttctagccactcaggatgagggtcc
ggccctgcctgccctcgctggggcccccccgcccggccccggtctaactgcccccgcccc
gaggcctcgcccggctccaaggcccccagcaggctctccagtcccaggatgcgctgagcc
gccggggggctgaggccgcgccaactacatgcatg (Seq ID No: 1021)

Homo sapiens embryonal Fyn-associated substrate (EFS):

ttttctttctcctcctccaaccttggcggaggccacgactcaggcgccacagctgggggc
tagaggccgcggaccatggtgcggggcagccaccgctgaagtcagcaaaaccgagcctgg
cctgaggcaggctgcgcgggaggccaaagccatg (Seq ID No: 1022)

Homo sapiens GH3 domain containing (GHDC):

cgctccttctttctggccggatgtgtgctgagacccagagtcacccaggggtctccgtca
cgtgccaggagtaggcagaagtgggctgtgacagatcaggaaacagagctcagtgcagcc
cactaaattgctcagggccctacagctaacaagcggcagaggcaggatctgcactcagga
gctgcttggagatg (Seq ID No: 1023)

Homo sapiens acrosin binding protein (ACRBP):

ggctctctctgcggcttggcccgttagaggcggcttgtgtccacgggacgcgggcggatc
ttctccggccatg (Seq ID No: 1024)

Homo sapiens jagunal homolog 1 (Drosophila) (JAGN1):

agttctcttcacggagccgcgcggctgcgggggcgcaaatagggtcagtgggccgcttgg
cggtgtcgttgcggtaccaggtccgcgtgaggggttcgggggttctgggcaggcacaatg
(Seq ID No: 1025)

Homo sapiens ligand of numb-protein X 1, E3 ubiquitin protei n ligase (LNX1):

gttcctttcctgggcatcagcttgcctgctctcagcctaagctctctcgccaaccgtggt
ggctccttgcgttcctacatcctctcatctgagaatcagagagcataatcttcttacggg
cccgtgatttattaacgtggcttaatctgaaggttctcagtcaaattctttgtgatctac
tgattgtgggggcatggcaaggtttgcttaaaggagcttggctggtttgggcccttgtag
ctgacagaaggtggccagggagaaggcagcacactgctcggagaatg
(Seq ID No: 1026)

Homo sapiens cyclin-dependent kinase 2 interacting protein (CINP): tctccttctacggatatctgtggaccttatg (Seq ID No: 1027)

Homo sapiens splA/ryanodine receptor domain and SOCS box con taining 2 (SPSB2):

gcttctttccgcccggctccttcagaggcccggcgacctccagggctgggaagtcaaccg
agctcccttccaggtcaatccaaactggagctcaactttcagaagagaaagacgccccag
caagcctctttcggggagtcctctagctcctcacctccatg (Seq ID No: 1028)

Homo sapiens Berardinelli-Seip congenital lipodystrophy 2 (seipin) (BSCL2):

cctcctcctttcctccctctactctgacacagcacttagcacctgaatcttcgtttctct
cccagggaccctccattttccatatccaggaaaatgtgatgcgccacaggtatcagcgtc
tggatcgccacttcacgttttagccacaagtgactcagtggaagatccagagtcaacaga
ggctcgtcaggaagatg (Seq ID No: 1029)

Homo sapiens tubulin, alpha 1c (TUBA1C):

caccctttcactacttctcccccggactccttggtagtctgttagtgggagatccttgtt
gccgtcccttcgcctccttcaccgccgcagaccccttcaagttctagtcatg
(Seq ID No: 1030)

Homo sapiens 1-acylglycerol-3-phosphate O-acyltransferase 9 (AGPAT9) :

tttccttcctctcttcccttcgcagaggtgagtgccgggctcggcgctctgctcctggag
ctcccgcgggactgcctggggacagggactgctgtggcgctcggccctccactgcggacc
tctcctgagtgggtgcgccgagtcatg (Seq ID No: 1031)

Homo sapiens 1-acylglycerol-3-phosphate O-acyltransferase 1 (lysophosphatidic acid acyltransferase, alpha) (AGPAT1):

gcccctttctttccttcgcttcctcttttagagaatgtccggattgctattggactttgg
agcgtatggctccaaatcaactcattggctaaaacttgacggaaaatggtggttaggtgg
ccagaatg (Seq ID No: 1032)

Homo sapiens abhydrolase domain containing 14B (ABHD14B):

cggcctcttcccagcgttcctcctccggccccaggtcaccgccagcacgcgcctgcttcc
cgtctgcgcgagtccacgcagctccccagatcaagaagctgaggccccaggttacacact
aaagtaaatggcagaggcagaaataacacctatgtcctcctgaccccaaggcatgttctt
aaagttctggaaacctcctggaggcttccttgctgctcctctgggactgccaccctgggc
agggtgttctgtggcccctcatcatcgtggttttgaaccacaggcccttcaccagcacag
cagcagcaggcatg (Seq ID No: 1033)

Homo sapiens protein tyrosine phosphatase, non-receptor type 5 (striatum-enriched) (PTPN5):

catcctcccgccagcctgcccgcctgctcgccggcgcccggagcccgctctggccgcttg
ctttttgctgagaaagcttcctgccctggaagatggcacccttccccatccagacacctt
gggaatg (Seq ID No: 1034)

Homo sapiens carbonyl reductase 4 (CBR4):

cttcctccttttcacggcgtcttgcattactattgtgcggctgcaggaggtgtcgagcgg
cgttattttttttgcggtttgcctttttttttcttttttttttttttggaaccgcggtt

gtttaaaagcctgagggaacctggagaggggctcccactccctaccctctttcctccgag
tttgtgactccgagatg (Seq ID No: 1035)

Homo sapiens zinc finger CCCH-type containing 10 (ZC3H10):

ggctctttgtcgaagctagaggaccggcaggcggcagcagcaactacggcggcggcggca
gaacccagcagcgatgtggaggtggagacccacaggagccccggacttcacctgagctac
ctcagtggtcaccaagagtggcaagataaagaaaaccctgagttgggcgggaccaggatg
(Seq ID No: 1036)

Homo sapiens poly (ADP-ribose) polymerase family, member 10 (PARP10) :

ccgtctttcagtttcacttttgttttcctgctcccagcagggttaggcttgctgaggggc
aggcacaggagtcctggctgagctcatggcctgaggctgcctagcggccacggggaatg
(Seq ID No: 1037)

Homo sapiens RNA pseudouridylate synthase domain containing 4 (RPUSD4): ccgcccttccttgtaagatg (Seq ID No: 1038)

Homo sapiens family with sequence similarity 73, member B (FAM73B) :

ctgcccttccgcagcgatggcatcccgggtgagtatcggccccggccgagcccccaaggc
gggcgggcagcgcggcagggccgggacttgagcggaggaccgagtaggcgcaggtgtccg
ggcccaacaggaccaggaaggtgtcggggttggaatgagtgggtacccgggccggggacg
gtgcgagagggtgccttgcttgggagcggaacgagaaggtacttgggtcagggaggtgat
gcccgggcctggaacgtggcggggattggagcaggcgcgcaggtacccgatccgaggcgg
ggagagcacccgggatggaaggagcaggcgtgcgggccgtgagcggcgccagagggtacc
tggctctgtggaggggccctctggtatgtgtgtccctgtccttctggggcgtggatggtg
cctgggacccagctggcaaccagttgaagacgttctccttggaagctcttggccctgagg
actttgcctggggcattggccctgccatg (Seq ID No: 1039)

Homo sapiens protein phosphatase 1, regulatory subunit 15B (PPP1R15B) :

gcgtctcttccggcgtctagggggtgtcctgccggcgcgcgggccctgcggccattttg
ggcttcgcttccaccgcaccagccggcctacccagtccttccggtatcgcgttgctcagg
ggcttttcaaccctctgtcagtcggaaaaccatcgccgaggccgtggggggactcctatc
catggtgttgaagcgtcgagccgactagggaacctccttccccgccaggatggaagtcgc
atcagtcgccgcctattgcgcgggctgttcttccctgtgttctgccgcccgctgccgcat
tcgctgccctctgtggcttttctgctggctcgaagatcggcctggagcagcgacgccacc
gctgggcaaggccgagactctgtaggcttcctccgaatcccgtcgacctccagccgctga
gcgccgcggccctacctgagagactgtcaagaaaaaggagatg
(Seq ID No: 1040)

Homo sapiens family with sequence similarity 104, member A (FAM104A) :
ccctctcttcgcggagcggcgccgcgtagcttccatccgccagctgccatg (Seq ID No: 1041)

Homo sapiens PRP38 pre-mRNA processing factor 38 (yeast) domain containing A (PRPF38A):

agccctttacactacggtgtttccggcttcaagatggtcgcctaagctgtttagtgaaac

ttcttccacctttctccattcctctaggtgctttttctgaacctggatgtgaggcattaa
aggatccgacggaaatagaattgaaggcattctaaaatg (Seq ID No: 1042)

Homo sapiens synaptotagmin-like 1 (SYTL1):

cctcctccgtgtggggcagctgctggctgggctgcctgttgagtcagccttcttccctca
cggctcttctcccggtccctgaaactcggctgccaggggagctggagccacctgcgaagg
tgtcctcccatactggacccctacaggaagctccgtgtgcccagctggggcacagcccca
gctgatg (Seq ID No: 1043)

Homo sapiens ubiquitin associated and SH3 domain containing B (UBASH3B):

gctccttttcctttttgatccattcaaaaattactcattgcaaattcccggactgctagg
cgaggagagggaagggggcggaggagacagggctactgcaggcgcagagctgggggcagc
cgggggcccgagtggctgaggctggtcccgcagcggccgcttgccggcgttctggctcct
gtggcctcaccaggaagcgtcagagtcccgacactggggaagctcggagcgccgcctccg
ctgccgccgcctcctgcctggctctgggtccccgagcccccctcccctggcccagcccgac
tccctcctccttcccgaaccatccggctcgggctccttccctggcgatggctggccgctg
agccatg (Seq ID No: 1044)

Homo sapiens transmembrane protein 241 (TMEM241):

ccgtctctgggcggctgctgccgctgccgctgctgctgctgcgggggtcgggcggcggcc
aggggatttgggcaggcaccgtggatccccgagaaggggacgagttgacagatg
(Seq ID No: 1045)

Homo sapiens ataxia, cerebellar, Cayman type (ATCAY):

gagcctctgccagccctgagctgggaagaagcagctacctcggaggcagggcgcgcaggc
gggcggcgatgagagggggcgcagccgcagccccgcgctggggagcccaccgctaaccct
gcaccccacccacccctgcacaaaagagctggcgggcgctggccacgtcgccctgggtga
ccttcctcggatgcagaatccgcccctgcgagcatcctcttcctcctaggctctgaaggc
ccggggagcgtgagcgatgcccagctgcacccgggcagggctcgcctttgtttgccagta
aggaggagaggctgtctcagctgcagaggggtcatccctgcttcaagccagtgcctcttc
ccagctcccatg (Seq ID No: 1046)

Homo sapiens ELL associated factor 1 (EAF1):

attcctctctcaccccccacgcagaggagagaacttgcttctggacccgggtgggtgccgg
ctcggctctccttgtcttccagagcggtggcccggaagcacagtcctcccagacgccagc
gccagaagctcggatcgcggctgcaccgggagagcgccgatctgggtgcgaggcaggtgc
ggggccatg (Seq ID No: 1047)

Homo sapiens tripartite motif containing 5 (TRIMS):

gttcctctaggaaaattcctttgtgcagatcaggcccgtggattggtgagtgaatcctaa
ccacgtcttccctggcctgtcttcactcttctccccagaatcaccacttctgcactggtg
tctgaaggtgtattgagtgattttgtggagggcagaagtaggaagtctttgggacaaaac
tgtatttaccttgggatctgtgaacaagaggaacctcagcagccaggacaggcaggagca
gtggaatagctactatg (Seq ID No: 1048)

Homo sapiens wingless-type MMTV integration site family, mem ber 3A (WNT3A): cgccctctcgcgcggcgatg (Seq ID No: 1049)

Homo sapiens chromosome 16 open reading frame 45 (C16orf45):

ctccctccctgcagcccgcaacgggaatggagtaaagggagacccgtcgacctggccacg
gggatcagcgatg (Seq ID No: 1050)

Homo sapiens zinc finger protein 502 (ZNF502):

cattcttccggtttcagaagttaaggctggtgtcctggccccagtccacctctgggagcg
cctgcgccgctccgcggagagtccgtggatctcacagtgaaaaatgtttgctgacccttg
acattgacaaactgctgacagctcagatgatccatgattggaaggatgtggtcatcacca
agatgtctttctttctccggttcccagttttccagacctgaagtgttttccaatcaaagc
gaagagacgatctgtggatg (Seq ID No: 1051)

Homo sapiens armadillo repeat containing 6 (ARMC6):

ggctctcttgcgcaagcgcgctgtccgcttcttctgggcggacgctctggaggcaaaaca
tttccctgctgggggcggcgaccaccgtgagcgtcccggaaggggcggcaaagacgcctc
cgtcgcgcacgaggtggcctcgttggctttaccttggttcgcggtcgtccttggttatcg
tgagcgtccgcgagtctctgggaggccaagcctaggggcgccacagcgcctgcgcgcgta
cggcggccggaaggggctagaggcggctccctgggtgacaaccgcgcgccccacctttcc
ccacgtggccgcgaagaccggctcaggagcatctatcggctgcacgccaacatcaacaca
ggcgaagatg (Seq ID No: 1052)

Homo sapiens post-GPI attachment to proteins 3 (PGAP3): gctcctcccccggcggcgagccagggagaaaggatg (Seq ID No: 1053)

Homo sapiens histone cluster 3, H2a (HIST3H2A):

tgccctcttgtttttagtctcgcttttcggttgccgttgtctttttccttgactcggaa
atg (Seq ID No: 1054)

Homo sapiens ethanolaminephosphotransferase 1 (CDP-ethanolamine-specific) (EPT1):

ggctctcctaccttctcgggcagcccagtctttgccatccttgcccagccggtgtggtgc
ttgtgtgtcacagccttgtagccgggagtcgctgccgagtgggcgctcagttttcgggtc
gtcatg (Seq ID No: 1055)

Homo sapiens F-box and leucine-rich repeat protein 5 (FBXL5) :

ccgcctctgccccgcggcgagggtgtctatggagaggcggcggccgcggctgctgaggcg gaggctgaggcagtggcgatggcgccctttcctgaagaagtggacgtcttcaccgcccca cactggcggatgaagcagctggtggggctctactgcgacaagctttctaaaaccaatttt tccaacaacaacgatttccgtgctcttctgcagtctttgtatgctactttcaaggagttc aaaatgcatgagcagattgaaaatgaatacattattggtttgcttcaacaacgcagccag accatttataatgtacattctgacaataaactctccgagatgcttagcctctttgaaaag ggactgaagaatgttaagcctactactgttgactggaagccttaccaataacataaaaca atcgaataacaattatttcatgtattatatgtaaaatatatatactggattcttacagta agaatgaatatgaacagttaaattatgcaaaacaactgaaagagagattggaggcttta caagagattttcttcctcacatg (Seq ID No: 1056)

Homo sapiens major histocompatibility complex, class II, DP alpha 1 (HLA-DPA1):

ctgcctccactcggcctcagttcctcatcactgttcctgtgctcacagtcatcaattata gaccccacaacatg (Seq ID No: 1057)

Homo sapiens secretory carrier membrane protein 1 (SCAMP1): tcgtctctctctgcgcctgggtcgggtgggtgacgccga-gagccagagagatg (Seq ID No: 1058)

Homo sapiens chromosome 15 open reading frame 57 (C15orf57):

ccgcccctcccgatttcctccgggctacaggcgacagagctgagccaagcgtttactggg cagctgttacggtaagtgaggaggggctggggtgcccagcgttttggatctcccactctg gcccggccccggaataccacatagaggccttgggacctgattcatcccgtccagacagcc ctagagacctgagcgactgaggcctgggatctggacgccggaatttcctgcgtggttctg gacgccctgccctgggctcagattccaaatg (Seq ID No: 1059)

Homo sapiens WD repeat and FYVE domain containing 2 (WDFY2):

cctcctcttgtagtggcgccggcttgcatcccaggtcgtggcggttttggtgcctgaagc agggagcgcggagtcgttcccgagagaggcggccaggctatgctcgccggtttccggcgt tccgctccggccagccagagtctctgtctcaacctgtgtccgtgctccagcagtctcctc agcccggccccgcggcgcggttggcggcggcgccccaggcgcgccccctcctccgatg (Seq ID No: 1060)

Homo sapiens topoisomerase (DNA) I, mitochondrial (TOP1MT): cgctctttcccggaggctggcagatg (Seq ID No: 1061)

Homo sapiens intraflagellar transport 122 homolog (Chlamydomonas) (IFT122):

ctttccctttcggacatgcgcgctcggagcaaggcgccctcgcactcagcttaccgcgca tgtacgttgccaggggtaacgcaggtagccaaagtggcttgtggagtggcgaccgttagt gaggcggttgctgagacagacgctgaggcgggtaggaggagcccgagccgtaagggaagc cgtgatg (Seq ID No: 1062)

Homo sapiens mitochondrial ribosomal protein L53 (MRPL53): agttcttccggggcggaggtcaccatg (Seq ID No: 1063)

Homo sapiens T-cell activation RhoGTPase activating protein (TAGAP) :

ccgccccttcgcttataatgcagagcatgtgaagggagaccggctcggtctctctctc
ccagtggactagaaggagcagagagttatgctgtttctcccattctttacagctcaccgg
atgtaaaagaactctggctagagaccctccaaggacagaggcacagccacacgggagtga
aatccacccctggacagtcagccgcaatactgatgaagctgagaagcagccacaatgctt
caaaaacactaaacgccaataatatggagacactaatcgaatgtcaatcagagggtgata
tcaaggaacatcccctgttggcatcatgtgagagtgaagacagtatttgccagctcattg
gacattctcactattctatgccttaaaggcccttcaacggaagggatattcaggagagca
gccaacgagaaagcccgtaaggagctgaaggaggagctcaactctggggatgcggtggat
ctggagaggctccccgtgcacctcctcgctgtggtctttaaggacttcctcagaagtatc
ccccggaagctactttcaagcgacctctttgaggagtggatg (Seq ID No: 1064)

Homo sapiens phosphoserine aminotransferase 1 (PSAT1): ggtcctccttggctgactcaccgccctggccgccgcaccatg (Seq ID No: 1065)

Homo sapiens CD97 molecule (CD97):

ccccctccttcataaagtcctggcctcgggacagcctgcacagctgcctagcctgtggag

acgggacagccctgtcccactcactctttcccctgccgctcctgccggcagctccaacca
tg (Seq ID No: 1066)

Homo sapiens protein tyrosine phosphatase, non-receptor type 2 (PTPN2) :

cgctctccccggatcgtgcggggcctgagcctctccgccggcgcaggctctgctcgcgcc
agctcgctcccgcagccatg (Seq ID No: 1067)

Homo sapiens chromosome 20 open reading frame 112 (C20orf112) :

gcccctctccccgggcagccgcggcggcagcagcagcagcagcagctggagctgtggggc
tgtcaccgccgcccgccccgctcactcgcggatcccgaccgcccatctccgcctcgcttc
cagcccaggatgagacttctgtgagcagcgaggattttgatatg
(Seq ID No: 1068)

Homo sapiens APEX nuclease (multifunctional DNA repair enzyme) 1 (APEX1):

caccccttctttgtgctcgggttaggaggagctaggctgccatcggccggtgcagatacg
gggttgctcttttgctcataagaggggcttcgctggcagtctgaacggcaagcttgagtc
aggacccttaattaagatcctcaattggctggagggcagatctcgcgagtagggcaacgc
ggtaaaaatattgcttcggtgggtgacgcggtacagctgcccaagggcgttcgtaacggg
aatg (Seq ID No: 1069)

Homo sapiens intermediate filament family orphan 1 (IFFO1):

tttcctcttgagccatcatgcacatctgactgcagccccagcgagcccttccttccttgt
ctgactgctcttcttctcgatttcttcttgttctgccttctcggtttgcagccctgaccc
ccgctgtgtgtctggcccttggtgactgtccgtgtttctgttcctgtcattgtaactgtg
acttttctctctgtctgcccccccttcctactggttcatgcttctcccccattcccaccc
tctctgcccggcctcccgctcccgccctttctcctcatgcacccggcctcgtctctgtag
tctctgcacttgtctcccattaaggtcccatccatg (Seq ID No: 1070)

Homo sapiens neuralized homolog 2 (Drosophila) (NEURL2):

cagtcttcctcccgccccttctttggtccctacggacctgggggcggtggcggtcaatg
ccgggtcaaggtccgcgggcctcgcagatcgtagcccgggcgcacgcgatcagatgatcc
tgttgtggacggctaagttgtaggcgggatggctgagaaagcggcgctaggaccccggg
cagaggctcggggaagggagtcagggggaaatgccttacaaggtcgccttgcggtcacc
atcattgcccgccgcccaaaatagcccccggcgccagctggcctgccctatggccgagag
atg (Seq ID No: 1071)

Homo sapiens drebrin 1 (DBN1):

ctccctctttccctccctcctcctccgtccgcccgtccgtccgcgcgtctgtccgttcgg
cccggtccggcccgaagcatg (Seq ID No: 1072)

Homo sapiens WW domain containing adaptor with coiled-coil (WAC) :

cagcctcccttatttagtccgcgatggcttccctcgcgccccaccgtcctcttccggaag
gcggctccctccctgcgcagcccggagcccctgagatcagcctcgagcaggcgcccgagc
gagactatccctaaacgggaacggcggtggccgactcgcgagtgaggaaaagaaggaaag
ggcagactggtcgcgaagagaagatccaggcctcagaggaggagaaaggccggagccagc
cgaggtttgccgagggcggtgttccggacccgcgcggtgcggggaggaaggccgagggtg

ggagaggagggcccggcggaaactgccgaggtttcccgaaggcggcagcgtccgagttg
cccggatgtagttggtggagcggcagcggcggcaccagcggcggcggcggcggcgggagg
aggaggaggagaagaaggaccaggcggcggcagcagcggcggcggcgggggaggaggggg
aggaggcggcggagcaggaggaggagaaggcggaggaggcagtcgctctccgcggggctg
agccggacgcgtcgtcttgcccccctcccccggttcgcggtgccgccgtgtagttggcg
ccgctgccccggctgagagtgagcgtggtgtcgacggagggagatggcccgggagcgccg
gcgccagtaactgggagctgatgagagtcgccgagggcgcgccgggcccaggtgccgggg
ctgcccgccgcccgccgccgccgccgcctgcgcgcccgcccgcctttcgcggccgctctc
ccccctccccgacacacactcacaggccgggcattgatg (Seq ID No: 1073)

Homo sapiens kelch-like 6 (Drosophila) (KLHL6):
cgctccttcagtctcgatg (Seq ID No: 1074)

Homo sapiens GTPase, IMAP family member 1 (GIMAP1):

cagccttctgcactcacagccgaagggaaagcagcaggttggggcttcttgtggccaact
tcagagcctgtcaccaggaaaggtaagcatg (Seq ID No: 1075)

Homo sapiens RAB24, member RAS oncogene family (RAB24):

cgccctctagccccctcccgcgggagtcgcggcgctgcgggtaggagccgggttgcggga
gaccccaggttcggttgggattcccagccagaacggagcttaagccgggcaggcgagcga
atgacggagtagcgagctgcacggcggcgtgctgcgctgttgaggacgctgtcccgcgcg
ctcccaggccgccccgaggcttggggtcttcgaaggataatcggcgcccggggccgaaca
gcgggggcacacggggcgctgccgaagtgcaaggccacggccagagctcgagcccgacgc
gctgtctggagtcgtaggaccctgacgtggctgaagcggccccgggagcatg
(Seq ID No: 1076)

Homo sapiens adaptor-related protein complex 2, alpha 1 subu nit (AP2A1):

agccctccccgcggccggctcggctccttggcgctgcctggggtcctttccgcccggtcc ccgcttgccagcccccgctgctctgtgccctgtccggccaggcctggagccgacaccacc gccatcatg (Seq ID No: 1077)

Homo sapiens copine IV (CPNE4):

ctccctcttttctcagtaccctcctctttactctccgagttaactgagagccgacctgac atctccaacattttcaccctcttccccaccccatcaccgagaatggagtcagggtttc cggagagaccgaactctgctctcagcacctttcccagccgctgttgctaaactgacctcg gaggacgagaggggaaggaggtgcgacgccccttacatcagtacataactaccacaccaa ccacctccacttcaaagccggattttgcatcctggggcgggacagacctcgtcccgggc tgaattctctctccactcttcgagattggcacacccagaatg (Seq ID No: 1078)

Homo sapiens synaptosomal-associated protein, 25kDa (SNAP25) :

ctgtctttccttccctccctgctcggcggctccaccacagttgcaacctgcagaggcccg gagaacacaaccctcccgagaagcccaggtccagagccaaacccgtcactgaccccccag cccaggcgcccagccactccccaccgctaccatg (Seq ID No: 1079)

Homo sapiens cAMP responsive element binding protein 3-like 4 (CREB3L4):

aggtctcttgactctttccgcctttgtttacaaccctgccatgatctccctcttgcaaaa gcgagggctacagaacaggcattcaggagtcctgtgctccagtcacagccttttctgttc

ttcagctaggagacaccaaaccctcaggaagatttactatagctaagagaaaactgcagc agaaagggcgcggctacctacttcttaaattccgtttgtggaccctcagactcttagtcc cctactcccagatacagcggccctaccgtggctcctggcaaggtggcatccacttttgta gtaagcatg (Seq ID No: 1080)

Homo sapiens leucine-rich pentatricopeptide repeat containin g (LRPPRC):
ctgtccttctggcggagcgtgcttcccgctgcggggacgttcgagcaatg
(Seq ID No: 1081)

Homo sapiens zinc finger protein 418 (ZNF418):

cgttctctggtagcgaccattttggttaatgttgggtgtgtttctgcggtttgtgaggtg agaggcgctggagctatgggtccgaaccgcggtgtctgaacccagaaggtgaagagtcct tcttgctgcacagaggcagatcttaggccccgtaacggcgcccgccgctcccggcagtgc tttccccgcgtactcgggatggcggcggccgcgctgaggctcccggctcaggcatcatct ggctgcaaagaagagaacacactgtgtttgagggaggaggaaggaggatcagagtttaaa ctcctgccataatg (Seq ID No: 1082)

Homo sapiens tetratricopeptide repeat domain 14 (TTC14):

gtttcttccgcttcctgtaccacccggctcaagtagcggacacggaacagggaactatca gcccgtcggcctccgggccctgcattctctagccatg (Seq ID No: 1083)

Homo sapiens BMP binding endothelial regulator (BMPER):

agcccttttcgactgtgagctgcggcagctgagcagaggcggcggcgcgggacctgcagt
cgccagggattccctccaggtgacgatg (Seq ID No: 1084)

Homo sapiens zinc finger protein 384 (ZNF384):

ccccctttcgtttccggcgctcccgccttctctccgcagagctcttctctgagcctgtt
gggggagggagggggcgtggaggaactggggttcgcgggagcacgagctgcagcacca
cttccgggtgagtgcaagggagggcagcaaggagggggccacccactacctcgcgcc
cccgccctgcgggtgtctcgcgcgcgttccgtgcgtgtgagtgtgtgggtctgtctcgct
ccagaagtgcgtgcccgcgcgctgcgccttgcgctttttcccctccctcgccccttcctg
gtcctcccaccctcctcggctccctcctttcccagcaaacgccgcccctcccgcgccctg
gctcaggctctggcgccgccgcagccgtcgccgcccgaaagttcaggagccctggaaagg
agaaggaataagacggcaggaggaagagagagagagggtagaatg
(Seq ID No: 1085)

Homo sapiens RAD51-like 3 (S. cerevisiae) (RAD51L3):

ctctcctttctcctccggcagccagcgcgcctgtgtcctctctaggaaggggtaggggag
gggcgtctggagaggaccccccgcgaatgcccacgtgacgtgcagtccccctggggctgt
tccggcctgcggggaacatg (Seq ID No: 1086)

Homo sapiens CD99 molecule-like 2 (CD99L2):

gctcctcctcccgctcctcctcggcctccccttcgggcgctctcgcgctaactgtgctcc
tccggggccctccgcctgctcccagccatg (Seq ID No: 1087)

Homo sapiens glucosamine-6-phosphate deaminase 2 (GNPDA2):

gcgcctttatctgcatccgggtccgtgggattcgcgctccactggtcagctggggtcgct
ctcgggtggttgggtgttgcttgttcccgctgttccagcgtcgaagaaccattgggtctg
ccggtttgaacttgttctggaagctgtgcgtcaccgtaatg (Seq ID No: 1088)

Homo sapiens methionyl-tRNA synthetase 2, mitochondrial (MARS2): ccgcctcctccgcttgcggccggtctgcaccatg (Seq ID No: 1089)

Homo sapiens chromosome 12 open reading frame 57 (C12orf57):

tttcctttccgctcccaggggcgttgggaacggttgtaggacgtggctctttattcgtga
gttttccatttacctccgctgaacctagagcttcagacgccctatg
(Seq ID No: 1090)

Homo sapiens tRNA-yW synthesizing protein 3 homolog (S. cerevisiae) (TYW3):

ggaccttttcggccaccgctcgcttcaatatggctgcccccagggagagacgaggctacc
atgaaggagccgagcgcagaccctgagtccgtcacccatg (Seq ID No: 1091)

Homo sapiens Sp1 transcription factor (SP1):

ctccctcctccttacccccccctccctgtccggtccgggttcgcttgcctcgtcagcgtc cgcgtttttcccggcccccccccaaccccccggacaggacccccttgagcttgtccctca gctgccaccatg (Seq ID No: 1092)

Homo sapiens histidine triad nucleotide binding protein 3 (HINT3) :

cgccctctagtggcagccggttttgaggccggcctccggctttgaagttcctcaccgcgt ctccttccctctccccaaagcctggatcaccgcccagcgtcaggcgaggggcgacgtctc gaggtaaaacggaggaggtgcgggacgcggagactgcgcgggcccggtagccctggagag gccgaggctctaggccgcgaggggcgggtgcaatg (Seq ID No: 1093)

Homo sapiens M-phase specific PLK1 interacting protein (MPLKIP) :

agttctctgcggagggccggttgatacagttccggtgggagaacgcggctgcgaggtttt cggctttggctcctgatatg (Seq ID No: 1094)

Homo sapiens palmitoyl-protein thioesterase 2 (PPT2):

caccccttcccccgccaccgtgggttccagacttgggataagtaaacagcgggtggagcg aggcctacggacccaggccaggtgggagtctgcactcttcaaggggcctgggctgctgct cacgggtattaaagaactccgcgttgttcatggctgaggcgatgcattaggaagatcctg gacctagagaacaagtcccccgaacgctgagttggaggcgggacttcgggtgcgcgttgg cgggagcatg (Seq ID No: 1095)

Homo sapiens BCL2-like 14 (apoptosis facilitator) (BCL2L14):

aagcctcttttcaggctgagtcctaaacctgaagaaagtttagagcctggggctctaaac tacctgagtctttccaaacgacaagccaagaagacctgttgaaagtttcctcttaagttt cgtggagagagactcaggtatagaaatatccttactgccacctgacctgaagcagaagaa atcacagacagcttccagaccaggcccaacatg (Seq ID No: 1096)

Homo sapiens galactose mutarotase (aldose 1-epimerase) (GALM) :

acgccccttctcctgtaaacttgggtcgcctctagcttagcgagcgctggagtttgaaga gcgggcagtggctgcacacgccaaactttccctatg (Seq ID No: 1097)

Homo sapiens carboxymethylenebutenolidase homolog (Pseudomonas) (CMBL):

cttccttcccttccccgactttgcagatttctcttcccccaggcctccctcctccacctc tccgcccccctccgggcttggctctcccaggaggctacgactggagccactggtcccgcag gatccccgcgtcctcggtcgccgcgtccacgtccctctcgcgtccccgcccggcgccacg ccgcctcctctgggttcggcctccgcgcggtgcagcgcagtctcaggccgcgggacaagc ccgacttaaatctctgcaatg (Seq ID No: 1098)

Homo sapiens chromosome 7 open reading frame 31 C7orf31:

cgtccttctcccgcccccgcccctgcctgccagctccaccgggccgtaggtgcggacgac
ctcaaaattcctcggcccgcgaaggccgccagctgcggggaggggaggggaggcgcggtc
ccgcagcgcccccaggctcatgtcccaggtatgtccagacccccgaggcaccgcttgcag
ggcagtgacagcccgtgaggctcggcctcgacccctggcacccttggtcccagctacgcc
ggctcctggccttcccccaagtccgagagagaggtgggattctccccgacgcagttggaa
accgggaatcccctttagggtcccgttcgtgctgcactactgactccaccatctgcaaag
ggattcttgtccagaatccccgaaggctttaggacagcgcttattttgttgaatgaagag
tctctaattttcggaaagaccacaggctaaaagtcaagttgtgcctttttagccaagaag
catg (Seq ID No: 1099)

Homo sapiens secretory carrier membrane protein 5 (SCAMP5):

cggcctttcggcagccgaacggccgcggcagttcaggacaaagaggtgtgggcaggccac
tgggccagctggtaacatcatg (Seq ID No: 1100)

Homo sapiens mitogen-activated protein kinase 10 (MAPK10):

tgctcctttcggttgccatagcaaccccattccccaagccctctgtccgtctcctctggt
aggttccacaatggtacaggcagcatcacgctgcacaatggtttccaggcagtgaaagag
ggtgattcagcaagccactcttcttctattttctttaacctccccttcacttttttatttt
tatgggggtgggtggtgcttgctatatgcttacctttttcttttctttttttcatttttac
aaatttcctttttttgtcctcacccctcaattcctaggggcttgagtgagtttaagattgg
gttttcttggaaatcacctgtccatcgttaattttaaacaatctccatatctccaaagaa
tctcttccatgttagtctggaatgtggttaatgaaaaacaagtagggaggatttctgggg
caaacactgccggatcaggatcgtagttctcaggcacggaatggctagtgtgagaaacac
caacagcaggcccatctcagatcttcactatggcaacttatgcaagaaactgttgaatta
gacccgtttcctatagatgagaaaccatacaagctgtggtatttatgagcctccatttct
tatactactgcagtgaaccaacattggatgtgaaaattgccttttgtcaggtgtgtgttc
cttacaggtaaaacaagggattcgataaacaagtggatgtgtcatatattgccaaacatt
acaacatg (Seq ID No: 1101)

Homo sapiens beta-site APP-cleaving enzyme 2 (BACE2):

cgtcctccccgccgccgccggtcccggtgcgcgcccatccctgcccgcagccccgcgcgc
cggccgagtcgctgagccgcggctgccggacggacggaccggctaggctgggcgcgcc
ccccgggccccgccgtgggcatg (Seq ID No: 1102)

Homo sapiens SWI/SNF related, matrix associated, actin depen dent regulator of chromatin, subfamily d, member 1 (SMARCD1) :

acgcctttttccgctagtcgccccgctctatcccatagtctcgctgccctgagcctcccgt
gccggccggccggccgggggaacaggcgggcgctcgggggcgctcgggggcgggggga

gttccggttccggttctttgtgcggctgcatcggcggctccgggaagatg
(Seq ID No: 1103)

Homo sapiens family with sequence similarity 175, member A (FAM175A): cgtcctcttgtgtagcctgaggcggcggtagcatg (Seq ID No: 1104)

Homo sapiens adenosine deaminase domain containing 1 (testis-specific) (ADAD1):

aggcctcttttgaaagatgcggccctgaccctgtgaacctcgcgcagagcggcctgaagc
gagaggttgaggctgggaggtgagaaaatg (Seq ID No: 1105)

Homo sapiens acyl-CoA synthetase short-chain family member 2 (ACSS2) :

gcccctctacggaggccccgcctctagttcggcctgttttctcagtcccggcacccgccg
cgaccgcaaaggcggccgcggttctaggaacttgacgtgatg (Seq ID No: 1106)

Homo sapiens multiple coagulation factor deficiency 2 (MCFD2) :

cttcccttactcaccggtgtccggaaaggtgaacgctgcgctcgggctgcctcgcctgtt
acctccgccgccgggcatg (Seq ID No: 1107)

Homo sapiens SPOC domain containing 1 (SPOCD1):

gctccttttcagctagtgggtggaaccccaggagggaaaactcagggaagcccaggccc
gtgttgtgcttttggcccaggtaggtggacagacatg (Seq ID No: 1108)

Homo sapiens LY6/PLAUR domain containing 1 (LYPD1):

agttccttcagtctcagccgccaactccggaggcgcggtgctcggcccgggagcgcgagc
gggaggagcagagacccgcagccgggagcccgagcgcgggcgatgcaggctccgcgagcg
gcacctgcggctcctctaagctacgaccgtcgtctccgcggcagcagcgcgggccccagc
agcctcggcagccacagccgctgcagccggggcagcctccgctgctgtcgcctcctctga
tgcgcttgccctctcccggccccgggactccgggagaatg (Seq ID No: 1109)

Homo sapiens cytochrome b5 domain containing 1 (CYB5D1):

cattctttcatactgcctcctcccttgtttttctgtctcagagagatagtctgtcctaaa
tatcccatgtagcccaggccactgaattaaaacggagcgtattcgttctctgccccaccc
cgcaactcctgaaagcggcgcaactcaattacttgatccttatatgccccacgcgggact
catactacgtttcccgtgaacacgtgcagtccaaaccccgcccctgatatttatctcagt
ggacggtggccggaaaaggacaatggtttccatgtcagcggataaacgctctcccctcgg
ctcccggacgcgacggaggtcgtagtagtagtgagtacgtgctgaggagcaaaggagtaa
ccaagagatccagtgaccgacagagcaagagccatg (Seq ID No: 1110)

Homo sapiens synaptoporin (SYNPR):

tctcctcctttgcttcataaaaagagggacaagtggctggtgctgtggacagagaagctt
tatttttagtatgagacaacctctatttctttcaggagagggaagttggattatcaatt
cttttgtaaatg (Seq ID No: 1111)

Homo sapiens heterogeneous nuclear ribonucleoprotein U-like 1 (HNRPUL1):

cccccccctttccccttcgcctcctgacaggaaaggtttaagggggacagagccctggga

ggccgggccgggctcgggggccaccccgggggcccgggccatg
(Seq ID No: 1112)

Homo sapiens schlafen family member 5 (SLFN5):

ggttctctgctctggacttgggaggctccgttgcctgctcccggagggagacgcgctgcc
gaggagaacccagcgggagaacatttcaggataggaataggccaagtgctgagaagatg
(Seq ID No: 1113)

Homo sapiens MAS-related GPR, member F (MRGPRF):

ccatctcttccagcaggagagggctctactctgagctcctattttccaaggctccgggcc
gcgctcggcgctggcctgctgccccggcgggtccgccggccggaggcgggagtcacagga
agagccctccacaaaaggaggcctcggcggatcaggacagctgcaggtgggtgtgcagac
tggtgagctgccagcaggggcccagacgcgccaggcctggagatg
(Seq ID No: 1114)

Homo sapiens ubiquitin-like domain containing CTD phosphatas e 1 (UBLCP1):

cggtctctcagcggccggtttctgcgtccgctgccgcaggttccaccgcgctccaggtat
ttttttttctgaaggaaagctgcttcctcatatgtttcaagaatg
(Seq ID No: 1115)

Homo sapiens Rab interacting lysosomal protein-like 2 (RILPL2) :

cctccttttccgttgtcccttcgcgccccaaaccacatcctggagcgcactctccagcgt
ggctggcagcggggacggtgcgccggggcgcaggcccaagagtcgcgtgcgcggcccctt
gcaccatcccccgggcccacccccgggccgcgctgattgggcaggtagggactctgccc
agcggaaagttttgggtgccgggaggaagtctaacctttgggagactccaagacagcagc
tccgaggtcggcgggggtctgggtggccatg (Seq ID No: 1116)

Homo sapiens zinc finger with UFM1-specific peptidase domain (ZUFSP) :

acttcttttccgtgggagtaaggaagtgcttttgaatgaggtactgagggccaaggtgtt
ggaagttcctaattctttcctcggttaactgtgaaactctgcgtattggaaggcctggc
ctcagtcatcaggccaggagaggtactggacgccgcgcacgcactcgtctgccagcgagg
cccaaaggggaagcctagcggagctcagtgtggcagctgctggcctctgggccgctactt
gtcaataccatg (Seq ID No: 1117)

Homo sapiens mitogen-activated protein kinase kinase 5 (MAP2K5) :

ccgccttcctcctcctcctctcgccgctaccgccgtcgccgccgccgcagccgccgccgg
tccgcgcggcctcgggtggccggagctcagcctgcgcgcgccgcgccctgtgtctccggg
tggggcagaagactcgccccttgaacctcccgcggggactctccgtggtgtggcggccct
ggggctctttcttaatagccccggactgagtcccctccagtcgaggaccctctcctagtc
cactgacgagcggtggacacctgccgctgtatctcccccaaaccgagtccttgccctgct
gcctcctcatacccacacggcggcagagaccttcaccatagcgttcgctcaactccagaa
ccttccgacctccgctagttcctgcgggcctttgcccgcttcccggtgcaccctccccgg
gagacacctcagacccccgacagcctgggcaggctcggtgcctgcgggtgcgttcctgat
cacccctcccctcttccctcccccctcatcctccattcccttgttttcaccctctgtcctc

tgcccgtcactccccttgtcacctcttggagcccccctcctaaccagcggccagtgggttt
cccataccccaggatgtgagcctctttaacctgtaatg (Seq ID No: 1118)

Homo sapiens solute carrier family 2 (facilitated glucose transporter), member 12 (SLC2A12):

cactcttctttagcatgctattatggggaaagtgaccactcctgggagcgggggtggtcg
gggcggtttggtggcggggaagcggctgtaacttctacgtgaccatg
(Seq ID No: 1119)

Homo sapiens mitochondrial ribosomal protein L30 (MRPL30):

cttcctctgctctgcttcccttcggaggaaaatttcaggctgaaggtttagcgggtgccg
cctctaaagagagcaatcactacacttatg (Seq ID No: 1120)

Homo sapiens tripartite motif containing 11 (TRIM11):

gctcctcttcctgccggcatccgggatccctacgtcccgcgtcccccgagcgctcggagc
ctacgcgcccagcgctaccgaaacccagagtcctgcgccctggagtccccgcgccccgga
gcccgagcacccgggagtcccgagcctcgcgccccggagtgcccgagcctgcgccgccgc
acccggataccccgcgtccccgcgagctgccgaggccgcccgccgccgccccgcggacag
taccgccttcctcccctctgtccgcgccatg (Seq ID No: 1121)

Homo sapiens proline-rich transmembrane protein 2 (PRRT2):

ctccctccctagctgacttgctccctcccgggctgcggctgctgcaaaagccagcagcgg
cagcgggagctgtccggaggccggcgtcgagggtttgccgctgtctctgctattccatcc
tccccataggggctctctcccctctcccatctcaagatg (Seq ID No: 1122)

Homo sapiens zinc finger protein 626 (ZNF626):

cggcctttgtctctcgctgcagtcagagctccaggtctggttcttctcctaaaggcccag
gctgtgtggccccgtgtcctgcaggtattgggagatccacagctaagacaccgggacctc
ctggaagccaaaaatg (Seq ID No: 1123)

Homo sapiens solute carrier family 25, member 43 (SLC25A43): cggtcttccgggcccgggtcggggctcgatg (Seq ID No: 1124)

Homo sapiens crystallin, zeta (quinone reductase)-like 1 (CRYZL1) :

ggctctctgacgaaggactggaaggtggcggtggtgaaggtgcaggccgttggggcggct cagaggcaggtgactatg (Seq ID No: 1125)

Homo sapiens mitogen-activated protein kinase kinase kinase 7 (MAP3K7):

ctgcctctacccccgccacggatcgccgggtagtaggactgcgcggctccaggctgaggg tcggtccggaggcgggtgggcgcgggtctcacccggattgtccgggtggcaccgttcccg gccccaccgggcgccgcgagggatcatg (Seq ID No: 1126)

Homo sapiens septin 6 (SEPT6):

ctttctctttgtcggaggagctcctctgtttcctgtgcagtagctcccgttgcggcggca cccgtggcagccctggcggacgcaggagcgatg (Seq ID No: 1127)

Homo sapiens myotrophin (MTPN):

ctgcctctcctcggccaggcggaacctctctgctgggcccggtggccgcaaaagaacttt

ctttctcccgcccgaacggtcgccgcggccaactgcctcgcccgcctggcagcctaaccc tccttctcttcttctcctctccggcttcgcgcggccctgcctccctctcgcccggcggca tccgcttgctgctgccaccgcctcctcatcttctgcccggccaaccggcctgccccgctg cagtgatg (Seq ID No: 1128)

Homo sapiens annexin A11 (ANXA11):

ccctcccttgcactgcctctggcacctggggcagccgcgcccgcggagttttccgcccgg cgctgacggctgctgcgcccgcggctccccagtgccccgagtgccccgcggccccgcga gcgggagtgggacccagcccctaggcagaacccaggcgccgcgcccgggacgcccgcgga gagagccactcccgcccacgtcccatttcgcccctcgcgtccggagtccccgtggccagg gattattggacctgcctggtttaaactattgtcttagttaattttgtgctgctctaacaa aatatcacagactgagtaatttataagcaatagtagcttatttggctcacagttctggag gctgagaagatcgtgaggctgcatctggcaagggccttcttgctgcttcataacatggca gaagacatcatgcgggtgtgtgtctggggaagagacttacagaagtggagttgctgagtc aaagatctaaccatg (Seq ID No: 1129)

Homo sapiens RNA binding protein, fox-1 homolog (C. elegans) 1 (RBFOX1):

ttttctttctttcctctcccggcgttgatgagtgcttggctcctgacagaaggatttgg ctcccagctttgtagttcggaagaagttgggtctatagatttcccctaactctccattg atgtgttgagcttcagagggaataataactctacgtaaagcatg
(Seq ID No: 1130)

Homo sapiens prefoldin subunit 5 (PFDN5):
cttcctcttcgttaagtcggccttcccaacatg (Seq ID No: 1131)

Homo sapiens high mobility group AT-hook 1 (HMGA1):

cgctcttttaagctcccctgagccggtgctgcgctcctctaattgggactccgagccgg ggctatttctggcgctggcgcggctccaagaaggcatccgcatttgctaccagcggcggc cgcggcggagccaggccggtcctcagcgcccagcaccgccgctcccggcaacccggagcg cgcaccgcaggccggcggccgagctcgcgcatcccagccatcactcttccacctgctcct tagagaagggaagatg (Seq ID No: 1132)

Homo sapiens zinc finger protein 323 (ZNF323):

cggcctttgcggttgatcggtcattggggtgctgcagccccgccacctgttccgtagctt gccggtgccccgaaggtgtcttctcctaaggaagattaaatcagaaaattttaaatcaca gttatccctttacttaaagccagagtaagccttccaaattaaccccaggaatg (Seq ID No: 1133)

Homo sapiens tumor protein p53 inducible protein 3 (TP53I3):

ctttctcttctcttagcagcacccagcttgcccacccatgctcaagatgggcgggatgcc agcctgttacataaatgtgccaaaagcctggccatgcctggaaaatggaccaatccgccc gccaagaggttgggtctcgttccctagagagaaggaagtttcctctccttgaagtgagag ctagaatcgcactttctgtcaagctgagagaaagactcttttccagaggctaaaaggaca agaaaatctgatttgcttgcttctaactttgcgttttaaaggggaaggaggaaaggaaa gaggggagggtggttctgcttagccccacccctccggctaccccaggtccagccgtcca ttccggtggaggcagaggcagtcctggggctctggggctcggggctttgtcaccggaccc gcaggagccagaaccactcggcgccgcctggtgcatgggaggggagccgggccaggaaca atatg (Seq ID No: 1134)

Homo sapiens ceramide synthase 5 (CERS5):

ccgcctccccgcgggttccgttggctgtggcggcagctgacgcttgtggcggcggtggct tcggggtgggcgtaagatg (Seq ID No: 1135)

Homo sapiens TRAF3 interacting protein 2 (TRAF3IP2):

tgttcttctacttacctgggcccggagaaggtggagggagacgagaagccgccgagagcc gactaccctccgggcccagtctgtctgtccgtggtggatctaagaaactagaatg (Seq ID No: 1136)

Homo sapiens Smith-Magenis syndrome chromosome region, candi date 7 (SMCR7):

ggtccttcacgttccattcccaggctggtctgagctccggggccgtggtcccgctgcctc ctccggtcgtcgtgcggaagctgcgacgcaggcagaccatg (Seq ID No: 1137)

Homo sapiens mitochondrial ribosomal protein L10 (MRPL10):

cattcttccggtggagatggctgcggccgtggcggggatgctgcgaggggtctcctgcc ccaggcgggctagagtgcagtggcatg (Seq ID No: 1138)

Homo sapiens proteasome (prosome, macropain) subunit, alpha type, 1 (PSMA1):

acttctctgtagatcgctgagcgatactttcggcagcacctccttgattctcagttttgc tggaggccgcaaccaggcccgcgccgccaccatg (Seq ID No: 1139)

Homo sapiens sorting nexin 5 (SNX5):

cggtctttctctagacgcgtcttgctgggagagtgtccgttgcttcccgtccgtgtcgcg gccctgcggttggcggcctcctcgtggagcggagcaaggccaggcggcccctgctcgagt cccgcgtcgccatg (Seq ID No: 1140)

Homo sapiens zinc finger protein 276 (ZNF276):

gggcccccctccgcgcgtactgcgggccccacgggtgttagtggcgggggcggcagagtcc gggtgggttgtcgcgacggagccgggcctcttcgccgtcttgagacggggctggcgagaa gggcccctcacggagttgccatgggcgtctaaccgcggcagccaggcccctctctacgtg agaccccggcccccctcccctttctgcagcccgcccgccacctgcgcgccgcgtggcctc cgccggcgcctgcccgccccgcgcctccgtctcccacggagcaggccgggctctcgccat g (Seq ID No: 1141)

Homo sapiens zinc finger protein 561 (ZNF561):

ccatcttttccggcgctggctcctctccgtcagtgcggtttcgcctttatggtggtggag tctgcccaggctgtggaccgcaaataaccctgtacaaagaggaatggagattgcctctat ccacctagattcataagctggcctgaggtgatcttggcatcaaggaagggatgcacatca tcacaccatcagcttcagagaatg (Seq ID No: 1142)

Homo sapiens mucin 7, secreted (MUC7):

ctttctcttcttttgcttctagttaccatcctcaaaggattggctaaaagcaagcaactg gattgaacaccctaagaagaaagattcacactgcaccaggagacatcagaaagaatg (Seq ID No: 1143)

Homo sapiens threonyl-tRNA synthetase (TARS):

gcgcctttcgattgcatcagctggtccagccgaggccaagtcccgggcgctagcccacct

cccacccgcctcttggctcctctcctctaggccgtcgctttcgggttctctcatcgcttc gtcgttcgccaatg (Seq ID No: 1144)

Homo sapiens ATPase, Na+/K+ transporting, alpha 3 polypeptid e (ATP1A3):

cagcctctgtgcggtgggaccaacggacggacggacggacgcgcgcacctaccgaggcgc gggcgctgcagaggctcccagcccaagcctgagcctgagcccgccccgaggtccccgccc cgcccgcctggctctctcgccgcggagccgccaagatg (Seq ID No: 1145)

Homo sapiens chromosome 11 open reading frame 46 (C11orf46):

cgtcctctcagtggtagcgcggggactggctgggaagcggtcggtcgagtgtggcctgtg
tggactcgcatcttgcccgaagccgggcggaggagagctcaagctaagggtgatcagccc
atgacctaaacctccagacaaaataaaacggaaaatttgctagaatcaagaatg
(Seq ID No: 1146)

Homo sapiens chromosome 17 open reading frame 45 (C17orf45):

tgaccttttcattcccgttgttatggaggtaggctctctaggaatctgggagtagtagct
gggggcaagagcaaataaagagctcgagcttctgtggtctctggggagatg
(Seq ID No: 1147)

Homo sapiens AHA1, activator of heat shock 90kDa protein ATP ase homolog 2 (yeast) (AHSA2):

gggccttctggcagtttctgggagctgcgaacgcgccgccccggggctcggcggccggaa
acgctggcttcggagccttaggcgccgcggcctttccttgttttccgcccagtccacgcc
gccatggccaagtggggccaggggaacccccactggatcgtggaggagcgggaggacggg
accaacgtgaacaactggcgctggcgcggctggcggcggcctccttccgggatctgggga
gggccgggccgcgggagccggggctgccctggggtctgtgcggggccgcggggccagggg
gtcagggggccgccccccctcagctgctggacgcagggctcggccttcgcctctcggctc
gggagagtccttgagtacggagaccggctaggagggttgcagctgcctctttttgaaagt
tgggttgggccccaagagtgacttccgacagacctttccactcccaccgtctgtggcctg
agggccttcccttctcctcccgcccacccctctggatgtttcggggagttagaagggagc
tggattgagagactgtgttaggggcgggggtatggaacgtagtggaaagggcagaaattt
ggatctcagttcgcgcccaccccgcaggcgcctcccgcgagccgggccctctgtgagtga
gacaagctccccttcctttacgcgcctcacctggcgcgtggggagaggtcggcagccctc
cgccgcagaacctccggaagggatgtcctctgccctgcgcctctggccggggctgtggtc
cctccaggccgtcgaggggatgctgaggccggtccccagaggagcatgacttggctggtc
cggaggagctctgagggcatgggcaatcttggctcgctgcaacctcagcttccagagttc
aagcgagtctcctgcttcagcctcatgagtagctgggactacagatgcgtgccactacgt
ccgtctgatgtttgtatttttagtagagacagggtttcaccatgttggtcaggctgctct
cgaactccagatctcgtgatccgcccgcctgggcctactaaagtgctgggattacaggcg
tgagctagatctgactttctagtgtcctagccttggcccgatggacatgtcatttctctc
agctcgtttctgtcccctaaagtgagaatattgcctgggaagattacattagacgatgta
tatgcgaagacacttgatagctggtattgtcatgattctgattagttcactactgctact
ttccctgtggcctaggctttgcctatttccagtgggcgagctagctagatcctcctccct
taaataagccagtgtttttaagacagaatactacttgcatagtggacaataatatcttaa
agaactgagcaggatgaaaagaatttgatagaaagcaggtttgaggagcacattggaggt
tggcaggtttcgaggctgcttgagaggacttgggccgatctgggctgggcttggacgtga
ccctggcacccaggcaggtggatcccagctggggcttccattcacgactttctggtccct
ggcaggacagagcgggatgccaccagcttgtccaaagggaagttccaggagctcctggtg

ggcatcgttgtggagaatgacgctggccgcggcgagatcaacgagttgaagcaggtggaa
ggggaggcttcgtgcagcagccgcaaaggaaagctgatttcttctatgagtggaacatc
aaactgggctggaaaggcatcgttaaagaatctggagtgaagcacaaggattgattgaa
atacccaatctttctgaggaaaatgaagtagatgacactgagaatttacaacggaatg
(Seq ID No: 1148)

Homo sapiens GrpE-like 2, mitochondrial (E. coli) (GRPEL2): ctgcctctcagcccaaattggaaacatg (Seq ID No: 1149)

Homo sapiens xyloside xylosyltransferase 1 (XXYLT1):

ccgcccctttcatggccgccgcctggcgccggggctaagtggccgccggcgtccgggtac ccgagggctctcccgcgttgctggcaccgctggcgccgcggtctcgtagcgcatg (Seq ID No: 1150)

Homo sapiens chromosome 7 open reading frame 60 (C7orf60):

cctcctctggctgctgcctccgcagctccctcctcctaccccacctcctccatctgggga gcgtctgcgggggcctgagggcggcggcggcggcggctgcgatatg (Seq ID No: 1151)

Homo sapiens tetratricopeptide repeat domain 39B (TTC39B):

ccctcctttgcgctgggctgagcccagagccgagagcaggggtcggctctgagttccctg cttggtttttgggtggcagcagccagaggaggaatatg (Seq ID No: 1152)

Homo sapiens motile sperm domain containing 2 (MOSPD2):

cacccttctctgtctacctctgggcgggactgccgggtgatgagatactcggtcggcgac ggtagaacgggcgacggcgacaaccgcaatcacatccacgacggtgatcatg (Seq ID No: 1153)

Homo sapiens major facilitator superfamily domain containing 6-like (MFSD6L):

ggcccctttcggtccaacggcaggacctgggggctgtggccgggggcggccgttgacctg gtgaccgcggcgccgccccagaccgggggcgcagtcccactcgctccgagccccggtccc ccaagcctccctcccgggtacctgggccgcgcccgccctgcgcccagctccgccctccg tcggcccaggcctgacagagcccggcagccatg (Seq ID No: 1154)

Homo sapiens consortin, connexin sorting protein (CNST):

cttcctctctagccgccagtgctctatgctccgcggtcgcgggccgccagcctccagccg gccagccgcgaggggtgcgcagagggaggcggggcggaaaggcgagaggtgtctcctcca ccggagccaggggagacccgagcaagctccgtgacagcacgtcggccgccatgtcgccga gtggggctggaaacagacccggcgcccagcggtagccctccttgcgcctccgattcccag acatggaaggtctttaatgtaactttaaatggttcaccaaaggatgctctaatg (Seq ID No: 1155)

Homo sapiens zinc finger protein 92 (ZNF92):

gggcctttgtctctcgctgcagccggcgctccacgtctagtcttcactgctctgcgtcct gtgctgataaaggctcgccgctgtgaccctgttacctgcaagaacttggaggttcacagc taagacgccaggacccccctggaagcctagaaatg (Seq ID No: 1156)

Homo sapiens DnaJ (Hsp40) homolog, subfamily C, member 18 (DNAJC18) :

cccccttctctttcagcctcgggcacgggggaggctcggcggacctgctgattgggaacc gatatg (Seq ID No: 1157)

Homo sapiens polymerase (RNA) I polypeptide D, 16kDa (POLR1D) :

cctcctccctccttccgtcctccgcgccttccgtcggtcggtccttgcttcctgcttcgc
ctccgcgcctcgcgctatgggacagagcccccgatccgccagcaccacctgaggatccag
aaaccgccccagcgatg (Seq ID No: 1158)

Homo sapiens ring finger protein 182 (RNF182):

acctccctcccctcccaggcgccgccgcagccggagcggctcccgggccctgggccgccg
ccggccaggaagaaatacttgtgttggctgcatttccagggatgctaccagagctcaagg
ctgtcacctggtcttgcccagaagagccgttcttagaggcaggacttgatgaaggctttc
ctgctgatggaataggtttgctagagctggccttggaattagaacccttcatgtggcctt
tataaatatgcgtttgagacagagttatatgcagaagttgaaaatgcctggaagatttct
ggtttctttcactacttatcctgcctttttgcatcgctgccagatttggatgatatgata
ttcagaggggcaccttaatcaaagccattcttcaacaagacccacctggcataagattgc
acacataattcaagatg (Seq ID No: 1159)

Homo sapiens transmembrane protein 18 (TMEM18):

cctcctctgtggattctggccaggccgggttcggcggttgctgtgagagcgggcttccca
acaccatg (Seq ID No: 1160)

Homo sapiens Hermansky-Pudlak syndrome 4 (HPS4):

aggcctctctgccgcgcgcgcaggtacggggcagaagtcgcaggtacccagctgctgccc
acatttctggtccagagtcccgaaccccgagcactgggatgcctggctactccgagccaa
ggcactgatgtttgaactggaaacttcaaaacgtttaataagagtcttcaggatgggttt
gaactagacaagctagaaatttctttagaacaccagctctagcatgcatctcccactttt
ggctttcctggagaggagcttgaagaggtggttctgcagacagccacagtgatacttagg
aaaccagaggaatggatttgacttttctgctaggattctctgttatagtttctccctgag
ttgtaagaggcatggaaatatacatgaaactgaagaacctgcaaggaagggaagtggaac
tttccatgctgagtgaaaactaaccaagtggcagttgtgactgaaaacactgaaacctac
cacgtccagattcactggattgggggatagaggaacggtcacagctagggagaaagaagt
gataccggaaaagaaaacctaaatgaagagaatgaggatgactgcacagtagatg
(Seq ID No: 1161)

Homo sapiens PTK7 protein tyrosine kinase 7 (PTK7): agctccttttcctgagcccgccgcgatg (Seq ID No: 1162)

Homo sapiens kelch repeat and BTB (POZ) domain containing 6 (KBTBD6) :

agttctcctgggcgcctagcattgtcgcccacgctgcagtagcggcttctgcggctccaa
gccagcgggtcctgtgaaggcgagcagacgcggagaaaggacgcgggagtgagagagggt
gagtcagccactgtctaaacgataacgggaggcggctctgcggggtagggttgaattcag
taaatgggctcgtgctgctgtctcttcggagacgctgctatcttagcgtcagcgagggaa
ggttgaggaggagccagagccgggtcctgcagcgtttctcgccatcagcgcccgtcgcca
tctccaccatg (Seq ID No: 1163)

Homo sapiens sperm antigen with calponin homology and coiled -coil domains 1 (SPECC1):

ctttctttgactggagcggacccgccggacgcaaccgcctcgccagccggagccagcgcg agctcggcacggtggacacccggtccgaggccggcaagccggctggtgcccgagtcggcc aagcatg (Seq ID No: 1164)

Homo sapiens ST6 (alpha-N-acetyl-neuraminyl-2,3-beta-galactosyl-1,3)-N-acetyl galactosaminide alpha-2,6-sialyltransferase 3 (ST6GALNAC3):

ggtccccttatttggatctgcgggaatgtgggctggagaggtcctgccgtggtaccagcc tccagcctgcccccaggactgcccctgacccaggcgcgcccgctgctcggtggcaggagg gccggcggagcgccatg (Seq ID No: 1165)

Homo sapiens transportin 1 (TNPO1):

gattctctttgttccgcagccatttcaggccccggacaggaggcagtgccgcttcggccg aaggcccgagcgcccgaggcgtctgggatg (Seq ID No: 1166)

Homo sapiens heat shock 70kDa protein 8 (HSPA8):
cttccttcgttattggagccaggcctacaccccagcaaccatg
(Seq ID No: 1167)

Homo sapiens hyaluronoglucosaminidase 1 (HYAL1):

ggctccttcctccaggagtctctggtgcagctggggtggaatctggccaggccctgctta ggcccccatcctggggtcaggaaatttggaggataaggcccttcagccccaaggacatcc tggctgccatacctgctcctgacttctcagggctggcagtcatcgactgggaggcatggc gcccacgctgggccttcaactgggacaccaaggacatttaccggcagcgctcacgggcac tggtacaggcacagcaccctgattggccagctcctcaggtggaggcagtagcccaggacc agttccagggagctgcacgggcctggatg (Seq ID No: 1168)

Homo sapiens STE20-related kinase adaptor alpha (STRADA):

agtcctcccggtcgccccactgcgcatggcacgttgcgtactcccctcccagcaaccggt ctggcggcggcgcggcagtaaaactgaggaggcggagccaagacggtcggggctgcttgc taactccaggaacaggtttaagtttttgaaactgaagtaggcctacacagtaggaactca tg (Seq ID No: 1169)

Homo sapiens transmembrane protein 161B (TMEM161B):

ccctctctttcgctgtttgagagtctctcggctcaaggaccgggaggtaagaggtttggg actgccccggcaactccagggtgtctggtccacgacctatcctaggcgccatg
(Seq ID No: 1170)

Homo sapiens Usher syndrome 1C (autosomal recessive, severe) (USH1C):

ggctctttccagctcctggcagccgggcacccgaaggaacgggtcgtgcaacgacgcagc tggacctggcccagccatg (Seq ID No: 1171)

Homo sapiens interleukin 12 receptor, beta 1 (IL12RB1):

cagtcttttctccttgctcagcttcaatgtgttccggagtggggacggggtggctgaacc tcgcaggtggcagagaggctcccctggggctgtggggctctacgtggatccgatg (Seq ID No: 1172)

Homo sapiens Meis homeobox 2 (MEIS2):

atcccttcctctcttttctgttcgccctcttctccctgctctttttccctttccaccccc ctcctctgttctccctcacctcctgcgcccctccccttcccgggttctgacagtacga tgagctgccccattacggcgggatg (Seq ID No: 1173)

Homo sapiens G elongation factor, mitochondrial 2 (GFM2):

ttttcttttcgtttagatacattgccttttgcctaggctggcgtcgagacttgaggccgt tgcagactttggcgcggctcgcgcctcctgcttcaagagcccagcggtgagagctggcct gcggcacgcggcctaatgccagacagtaacagtttggaggatcaagatg (Seq ID No: 1174)

Homo sapiens lamin A/C (LMNA):

gagcctttgccccggcgtcggtgactcagtgttcgcgggagcgccgcacctacaccagcc aacccagatcccgaggtccgacagcgcccggcccagatccccacgcctgccaggagcaag ccgagagccagccggccggcgcactccgactccgagcagtctctgtccttcgacccgagc cccgcgccctttccgggacccctgccccgcgggcagcgctgccaacctgccggccatg (Seq ID No: 1175)

Homo sapiens calcium/calmodulin-dependent protein kinase II delta (CAMK2D):

cgctctttctctcgccgcgccgtcttgaagccgcgcgggctcgtgagcagcgcgaggccg ccaaggtgcctcgcttcgccggagccgctgccgcccgccggagggaagccggcctcgggc gcgcacgctcgtcggagccccggcgcgccccgcgcctgagcctgctgacagcggccgctg ggctcaggctgtccgctctgggctccgcggcctcggccccgctgcactccacctccgccc cctcggactccctcccctctgcttctactcctcctgctccagtgcggatcgtttcgcaac tgcttgccactcgtcccgtgcctggctgtttttccatttcccggcccccctcttcttgagt actttacccccctgcatttggggacagggactggaaaaggggcgggtggagcgtccagtgg agaagaaggaagcgaggcccgcaggaggaggaggatcggcggactgtggggaggagaccc cacgccaccctttctggtcatctcccctcccgccccgcccctgcgcacactccctcgcgg gcgagctactttcggaccaggaaagtaagagcggccctgggtgacagcgccgcgggggcca gtcccggggttagccgcgcgtctgctcgcttctggtccgtcgcgctcccagccagggcac agcccggaccgaggatg (Seq ID No: 1176)

Homo sapiens calcium/calmodulin-dependent protein kinase II gamma (CAMK2G):

ccgtctcctcctcttgctccctcggccgggcggcggtgactgtgcaccgacgtcggcgcg ggctgcaccgccgcgtccgcccgcccgccagcatg (Seq ID No: 1177)

Homo sapiens interleukin 15 (IL15):

ttttcttttcgccaggggttgggactccgggtggcaggcgcccgggggaatcccagctga
ctcgctcactgccttcgaagtccggcgcccccgggagggaactgggtggccgcaccctc
ccggctgcggtggctgtcgccccccaccctgcagccaggactcgatggagaatccattcc
aatatatggccatgtggctctttggagcaatgttccatcatgttccatgctgctgacgtc
acatggagcacagaaatcaatgttagcagatagccagcccatacaagatcgttttcaact
agtggccccactgtgtccggaattgatgggttcttggtctcactgacttcaagaatgaag
ccgcggaccctcgcggtgagtgttacagctcttaaggtggcgcatctggagtttgttcct
tctgatgttcggatgtgttcggagtttcttccttctggtgggttcgtggtctcgctggct
caggagtgaagctacagaccttcgcggaggcattgtggatggatggctgctggaaacccc
ttgccatagccagctcttcttcaatacttaaggatttaccgtggctttgagtaatgagaa
tttcgaaaccacatttgagaagtatttccatccagtgctacttgtgtttacttctaaaca
gtcattttctaactgaagctggcattcatgtcttcattttgggatgcagctaatataccc

agttggcccaaagcacctaacctatagttatataatctgactctcagttcagttttactc
tactaatgccttcatg (Seq ID No: 1178)

Homo sapiens protein O-fucosyltransferase 1 (POFUT1): gtccctccttccctccccgactgtgcgccgcggctggctcgggttcccgggccgacatg (Seq ID No: 1179)

Homo sapiens calpain 3, (p94) (CAPN3):

cactctctttctctctccctctggcatgcatgctgctggtaggagacccccaagtcaaca
ttgcttcagaaatcctttagcactcatttctcaggagaacttatggcttcagaatcacag
ctcggttttaagatggacataacctgtacgaccttctgatgggctttcaactttgaact
ggatgtggacacttttctctcagatgacagaattactccaacttcccctttgcagttgct
tcctttccttgaaggtagctgtatcttatttctttaaaaagctttttcttccaaagcca
cttgccatg (Seq ID No: 1180)

Homo sapiens PTK2B protein tyrosine kinase 2 beta (PTK2B):

agccctttactcagccacagcctccggagccgttgcacacctacctgcccggccgactt
acctgtacttgccgccgtcccggctcacctggcggtgcccgaggagtagtcgctggagtc
cgcgcctccctgggactgcaatgtgccgatcttagctgctgcctgagaggatg
(Seq ID No: 1181)

Homo sapiens ST6 beta-galactosamide alpha-2,6-sialyltranfera se 1 (ST6GAL1):

cttccttccttctccagtcccttccactgtgcgtcttctgtcccccgttcttccccagcg
gacccctctttcgagactccctagtggggtccccagctcccgggcgatcctgcccttgcc
gagcgcgttttctggagtcacctgggggaggggagtcctgggcagggccgggctggggaa
gacgcctggggcactgcccggcgttaacaaagggagccgataccgaccggcgtgggcgcg
gagcgggcggccgccaccgagcgtgctgagcaaccgcagcctccgcggccgagagtgcag
cgagcaaggggagagccagttgcgcagagccctgcaaccagcagtccagggagaagtggt
gaatgtcatggagcccagctgaaatggactggccccttgagcctgtcccaagccctggt
gccaggtgtccatccccgtgctgagatgagttttgatcatcctgagaaaaatgggccttg
gcctgcagacccaataaaccttccctcccatggataatagtgctaattcctgaggacctg
aagggcctgccgcccctggggggattagccagaagcagatgatcatgacgcagtcctgagg
tttaatggggcacccacagccaacttccaacaagatgtgggcacaaaaactaccattcgc
ctgatg (Seq ID No: 1182)

Homo sapiens ubiquitin-conjugating enzyme E2Q family member 2 (UBE2Q2): ctccccttccgcgcccggctccccttccgcgcccctcccgccggagatgaggggaagatg (Seq ID No: 1183)

Homo sapiens membrane magnesium transporter 1 (MMGT1): gcttcttttgctgggctgctgctccttcggcatcatg (Seq ID No: 1184)

Homo sapiens PAP associated domain containing 4 (PAPD4):

cggtcttccgggtgtctttgacagggttttctacgccgcttttcggcgactttttgctc
ttccgctttttgccaccgcccccaaccttctatatccttgcagcccctacctttttcttgt
gttgctcctcccctggcagccgtgaggggggttagatctcagccggagccggagctgggc
ctagctgtcccacgggccaccactacctcctttggttcgggagaaagctacgaccaagta
cgcccagctcgggccttagaacttctgaacgggcagtgcgggtaggccctgcttagccct

tcccggaggacacctgaccaaaagaggaagatagtcttgggacccttgcatggtgtttca
aagggtggtgaagaactaaggtagaagaatacatgttcacttccagtgaacaagagcatg
(Seq ID No: 1185)

Homo sapiens chromosome 3 open reading frame 23 (C3orf23):

ctcccttctggtgtactgggtgggaggtggaactagtcggacaaagccctcgcgtcggac
ccttgccagaactcaattaatggatgcctcgaagttgacgtacatatatattcagaaatg
(Seq ID No: 1186)

Homo sapiens mucosa associated lymphoid tissue lymphoma tran slocation gene 1 (MALT1):

cgcccctttgcgcggctggcgcggccagccggccaggctcccctcggcaaacctgtctaa
ttggggcggggagcggagcttcctcctctgagggccgtgccgcgctgccagatttgttct
tccgcccctgcctccgcggctcggaggcgagcggaaggtgccccgggggccgaggcccgtg
acggggcgggcgggagccccggcagtccggggtcgccggcgagggccatg
(Seq ID No: 1187)

Homo sapiens UDP glycosyltransferase 3 family, polypeptide A 2 (UGT3A2):

ctacctctacccacagccagtgcctttggcgcactgaggtgcacagggtcccttagccgg
gcgcagggcgcgcagcccaggctgagatccgcggcttccgtagaagtgagcatg
(Seq ID No: 1188)

Homo sapiens sodium channel, voltage-gated, type IV, beta su bunit (SCN4B):

cctcctctcgctctctgcccgctaactttcccgagccccgaccggcggcgcagagctccg
gggtagctttgtggccgaacgccgacctcgggcggagagcgcggctgtgcccagtatccc
atccccgcgacccccgcgcgctccggagagaacaggactatg (Seq ID No: 1189)

Homo sapiens JAZF zinc finger 1 (JAZF1):

tcccctctgcctcccggtggctcctcgctctccttccatctctctcgccccctctccctc
cgtcccgtcctcgccgctcccctcaccccgcctctctccccctcccccagcccctcctct
cctcaccccacccggcctccctccctccctcgcccgcccggcgctcgcagagccgacacc
agggggctctcgatgtagcaccatg (Seq ID No: 1190)

Homo sapiens chromosome 15 open reading frame 55 (C15orf55):

ttcccttccttggatccctgtgcacctactggagccaggttactctgggtcctggacctg
actgcctcattctggaggcttccagacagccacagttagtgcccaaacctgagaggatg
(Seq ID No: 1191)

Homo sapiens ras homolog family member C (RHOC): cgccctctcttcctgcagcctgggaacttcagccggctggagccccaccatg (Seq ID No: 1192)

Homo sapiens CTP synthase II (CTPS2):

cattctctttccttttccttctctcctgagcgctcctgcagttcctggggcgtagtaggg
gatccacaagcgtttgtgaccagtgaagttctttacaagggtgagatctgcacgggagga
cccgagcgagggtctcggcttgccaggaagccggggttccccgggaagcgtggagttcac
ccgcgcactcgaagtgcctttgcaaaattatatctgggtgttggcacccagccactattc
tgccaatg (Seq ID No: 1193)

Homo sapiens PRP4 pre-mRNA processing factor 4 homolog B (yeast) (PRPF4B):

agctcttttccttcttcctccacttcccctaccctccaccgtccgggagccgccgccacc
gccgccgaggagtcaggaagttcaagatg (Seq ID No: 1194)

Homo sapiens molybdenum cofactor synthesis 2 (MOCS2):

gcgcctttgcggccgtgattcggtcccgctgtcctaggcgggatggtgccgctgtgccag
gtaagggtggcgggtgtgcgtgcgggcctgggtgcggagccctcctcgacgtgtctctcc
cgccctttccctccacatacccagccttggtcagtcggacctccccactagcccccaacc
tggccggcgtcttgggttcgggggcgcccccgcccccgcccccgggcccttcctgtctcc
gggctttactgcgactgccccagcagaagtcgggtcctctccgagaactcttgtcagctc
acggcagcaaggacggactcgttctgaaggcgcctccacctttatgaccacctctttcc
cagattattcgttttgatgaagctaaaatttaatctaaaaagaaatgcacctcatggag
aattcttgtgaagaactgtgcttcatctgtggatttctacacccttgatcatttgcaaac
ctgtaattatttcgtaaagagttgtttgcacggagtgacaggttgaagtattgtattttg
caaaaagtgctgaaataacaggagttcgttcagagaccatttctgtgcctcaagaaataa
aagcgttgcagctgtggaaggagatagaaactcgacatcctggattggctgatgttagaa
atcagataatatttgctgttcgtcaagaatatg (Seq ID No: 1195)

Homo sapiens cat eye syndrome chromosome region, candidate 1 (CECR1): tttccttttccggaggggagatg (Seq ID No: 1196)

Homo sapiens solute carrier family 13 (sodium-dependent citrate transporter), member 5 (SLC13A5): ctgcccctcactcgtctcgcccgccagtctccctcccgcgcgatg
(Seq ID No: 1197)

Homo sapiens armadillo repeat containing, X-linked 3 (ARMCX3) :

agtccttcttgtcctggtcgttgttcccgtctgagtaccagctccccactgccctgaggg
cgggccggcctgcggcggagggaaaaaggaagaggagaaggaaattgtcccgaatccctg
cagtgggtccaagcctctcccgggtggccagtctttctgtaggttgcggcacaacgccag
gcaaaagaagaggaaggaatttaatcctaatcggtggaggtcgatttgagggtctgctgt
agcaggtggctccgcttgaagcgagggaggaagtttcctccgatcagtagagattggaaa
gattgttgggagtggcacaccactagggaaaagaagaaggggcgaactgcttgtcttgag
gaggtcaacccccagaatcagctcttgtggccttgaagtggctgaagacgatcaccctcc
acaggcttgagcccagtcccacagccttcctcccccagcctgagtgactactctattcct
tggtccctgctattgtcggggacgattgcatg (Seq ID No: 1198)

Homo sapiens armadillo repeat containing, X-linked 2 (ARMCX2) :

cgtcctcctctgggtaccaactctattgcgcagctcgctgccgtgcgtttaacccaggcg
aggaggaggaggagaaaattcccccagattcgggcaggcccgcaccccacattccgtcct
gttttgagaggaggagggaagagaaataaacgtggcagcgcatagaaggccagcagggag
actgctttccagacacctccggcccacacagccgttcacccccgtcttttcagtcctgg
aaaaggaattcggtctgtccttaggatgaagctctaactgaactgaagtaaggagaaaca
gccttgaatctttggagggtctgtcttccttttgggctctgtgcaactgcagctacagtg
gaaaaaagcaaactgctcttgatcccaggccctgcctaagcctcagcagaacttgtaagc

ctaaactgaagagcctcacccggacgagcaggcatcccttaaccttaagcaatccagttc
cacgccctggatcagtgaataaccccagctgcaccatg (Seq ID No: 1199)

Homo sapiens UBA domain containing 2 (UBAC2):

cgccctctggggctccgagcccggcgggaccatgttcaccagcaccggctccagtgggct
ctgtgagtaccggcctccgccatcctggctgcccctacacgccaccctaggcacctctt
tgaggaggctggggcagcggggaccctcgggtttgccggaggtggtggggccgaccctcc
agacccgcgtccgaaccctgctagttcccggtcttgggggtcagcggaaaccgcccccat
ttcggcctggagggggcgaatggggacaaagccccgccgcccgccccgaccccacctggta
tccccaggtgctctgcccaggagtctcttggggccgctgcaagtgggcaggtgccctggt
gttctcgtgggccggccccaggccctttgcggagcgtgtgccgcgctgaaggaaggggcc
gtccccttaccatgccccattcttttaggcttgggggaccgaactaactccccccgccc
ccacttgcaaagttcagcctccgctttagaagctgacctctcagtttcacttggatg
(Seq ID No: 1200)

Homo sapiens cancer susceptibility candidate 4 (CASC4):

cctcctccctcggccggccctggggccgtgtccgccgggcaactccagccgaggcctggg
cttctgcctgcaggtgtctgcggcgaggcccctagggtacagcccgatttggccccatg
(Seq ID No: 1201)

Homo sapiens protein phosphatase, Mg2+/Mn2+ dependent, 1G (PPM1G) :

cgctccctcacagctcccgtcccgttaccgcctcctggccggcctcgcgcctttcaccgg
caccttgcgtcggtcgcgccgcggggcctgctcctgccgcgcgcacccccgggcttcgg
ctccggcacgggtcgcgcccagctttcctgcacctgaggccgccggccagccgccgccat
g (Seq ID No: 1202)

Homo sapiens StAR-related lipid transfer (START) domain containing 13 (STARD13):

ctttcttttaaaaatcgctgggtctgttgagctgtcctgggctgggtgccttgctcttt
gactgagactggagacagacggcaacagccacaggcagactgaggtggcaataggaaatc
tgccgagatg (Seq ID No: 1203)

Homo sapiens tubulin, beta class I (TUBB):

gattctcccgcctcccagccccggcgcacgcgcgccccgcccagcctgctttccctccgc
gccctcccctctcctttctccctctcagaaccttcctgccgtcgcgtttgcacctcgctg
ctccagcctctgggcgcattccaaccttccagcctgcgacctgcggagaaaaaaaatta
cttatttcttgccccatacataccttgaggcgagcaaaaaaattaaattttaaccatg
(Seq ID No: 1204)

Homo sapiens cytochrome P450, family 4, subfamily X, polypep tide 1 (CYP4X1):

tttccttcttcccgcgagtcagaagcttcgcgagggcccagagaggcggtggggtgggcg
accctacgccagctccgggcgggagaaagcccaccctctcccgcgccccaggaaaccgcc
ggcgttcggcgctgcgcagagccatg (Seq ID No: 1205)

Homo sapiens doublecortin (DCX):

ttttctttctctcagcatctccacccaaccagcagaaaaccggtgagtggggcttttaag
tgattttcaagaagaatgtaacagatgtcaaacgggaaaagcacaaggcaaagcctgctc
tctctgtctctctgtctcctcttctcctttttttgccttattctatccgatttttttccta

agcttctacctgggattttcctttggaaaagtctctgaggttccaccaaaatatg
(Seq ID No: 1206)

Homo sapiens protein phosphatase 2, regulatory subunit B', g amma (PPP2R5C):

ttgtctttttttttttaaactaaaatggaggctggtttcttgccttaaggagcccattgc
ctttcccgctgaagtctagatg (Seq ID No: 1207)

Homo sapiens solute carrier family 9, subfamily B (cation proton antiporter 2), member 2 (SLC9B2):

ccacctttccgggggaagccacgcgcaccaggcatcgcacgcggctctgcacccgcgccg
ccggacctgaaacccggcggagggcacacggggctgccgctgcggccccggaccaaccc
atgcttactccggagcctgtaccggcgccgacgggtcggacctccctgcgcggtgtcgcc
cagcgggttcgtgcgaaaggcggggccgactacacgcggtgccgcgccctgagaccgttt
atctgcagtcaacgcagcctcccggctcagcctgggaagatgcgcgaatcgggaacccca
gagcgcggtggctagaccgggctccgccgcctcccccacagcccctttcctaatcgttca
gacggagcctggtcgacttcgccggagactgccagatctcgttcctcttccctgtgtcat
cttcttaattataaataatg (Seq ID No: 1208)

Homo sapiens hypoxia inducible factor 1, alpha subunit (basic helix-loop-helix transcription factor) (HIF1A):

caccctcttcgtcgcttcggccagtgtgtcgggctgggccctgacaagccacctgaggag aggctcggagccgggcccggaccccggcgattgccgcccgcttctctctagtctcacgag gggtttcccgcctcgcacccccacctctggacttgcctttccttctcttctccgcgtgtg gagggagccagcgcttaggccggagcgagcctgggggccgcccgccgtgaagacatcgcg gggaccgattcaccatg (Seq ID No: 1209)

Homo sapiens interleukin 21 receptor (IL21R):

cctcctcttcctccccactctgcacatgcggctgggtggcagccagcggcctcagacaga cccactggcgtctctctgctgagtgaccgtaagctcggcgtctggccctctgcctgcctc tccctgagtgtggctgacagccacgcagctgtgtctgtctgtctgcggcccgtgcatccc tgctgcggccgcctggtaccttccttgccgtctctttcctctgtctgctgctctgtggga cacctgcctggaggcccagctgcccgtcatcagagtgacaggtcttatgacagcctgatt ggtgactcgggctgggtgtggattctcaccccaggcctctgcctgctttctcagaccctc atctgtcacccccacgctgaacccagctgccacccccagaagcccatcagactgcccccca gcacacggaatggatttctgagaaagaagccgaaacagaagatgaggcaatgaggctgcg agaggtagagtgattttccctcggtgactcaactgggacgtagcaggtcgggcagtcaag ccaggtgaccccatg (Seq ID No: 1210)

Homo sapiens DDB1 and CUL4 associated factor 4 (DCAF4):

tggtctttccgggtccttgcacgcttcgctccaactcctgcagagctgagccggaggga atccggaagggacacgctgaacaggtctgactcccgggcagcacagcccgctcacgattc cggccacggtgatgacgagtctccgtcaacctcgtctggcacagctgggacctcctctgt gccagagctacctgggttttactttgaccctgaaaagaaacgctacttccgcttgctccc tggacataacaactgcaacccccctgacgaaagagagcatccggcagaaggagatg (Seq ID No: 1211)

Homo sapiens oxidation resistance 1 (OXR1):

ccgcctcttgtgaggcgcgcggagccgcctcccctgggtcaggtctgatgggccggtggg

cgcgctagtggtggccgccaccgccgaaaccgtcgacctcctgggccccagttccgcgtc cagccccgcggcagcatg (Seq ID No: 1212)

Homo sapiens cut-like homeobox 1 (CUX1):
ccccctctctatcagccgctcactccgtctcaatatgtctcaagatg
(Seq ID No: 1213)

Homo sapiens atlastin GTPase 1 (ATL1):

ctccctttcctccccactccttcccaccagcgccacagcaacatcctcagagtctgagc gaactgcgcccagcgcgggcacggagcctcccaccgccagcaacctgcggccccggagaa ggcagcgagcgcagtgacagcgcctcaccgccaccagctcctggaccaccatg (Seq ID No: 1214)

Homo sapiens chemokine-like factor superfamily 5 (CKLFSF5):

ctgccttctctcccggggccctgtgggcaagcctcctgcttcactttcaggtttctcgaa gtgccttcttgctcctgtctgtttccccatcctgccagatttctgtttctcttgctgggc ttttggcagtagggggctgtgttggtgggccctacgaagatg (Seq ID No: 1215)

Homo sapiens transmembrane emp24 protein transport domain co ntaining 7 (TMED7):

aggccttttccgcttctcttttacctccccaggtccgcccgtctgcgcccctcacaggaa gccggagggtcgctctgatcccgaatctcccacaggcgtgaacctgctctgctgtgtatc tttgcggggtggcctgcgctgaggcctgccgcgcgcggtgagtccgcgcagacctgaccc tgcgtctcgcagctcggttgaggccgccgccgccttctcgggatg (Seq ID No: 1216)

Homo sapiens ubiquitin-conjugating enzyme E2D 3 (UBE2D3):

cttcctttaccttcctcccatggtctccttccggttctcgatgcttctctgagcctaagg gtttccgccactcgttcaccctccccccagctcatgatcctcctccctcccccgccctcc tggtccaatctccgatctgtttagtaagaaggtgctgttccgagaagaaggaaaagggct tgacacgtattcactcggccccggacgtgggaagcaagccgtctggcttcggcctcacat cggtcttgtgctcgggacggcggcgttggcggactgatccgcggcggtgaagagaggccg ggaagttaaacttgtagccaccacctccgctcttcccgtcaccctcgcccccacttcggg ccgaaagcacggtacagaggctgttggtggctttgccacgccaccccacccaccccggat cgcggctgtcttaagggacctggattcatcaggggctcttcggggcctgtgcgagtgctg atctgctccgtttttgcaaaaggcgcctgtgtctggcagagctggtgtgagacgagacaa tcctgccccgccgccgggataatcaagagttttggccggacctttgagcatacaccgaga gagtgaggagccagacgacaagcacacactatg (Seq ID No: 1217)

Homo sapiens zinc finger protein 595 (ZNF595):

tttcctctggctcctgcgagggcttggtttagggcttcagctctctgcgttctcggctcc gggaggcctcggtgattcagccacagcctctgcctcccgttgctctgtgacctgagggta ttggacaatttgtagctaagactcccggataccctgaagtcgggaaatg (Seq ID No: 1218)

Homo sapiens acyl-CoA synthetase medium-chain family member 2B (ACSM2B):

tgctctcttccaaggctgtaggagttctggagctgctggctggagaggagggtggacgaa gctctctccagaaagacatcctgagaggacttggcagcctgcagatggcctattgtggga ccttgtgatcatgcctgaacatg (Seq ID No: 1219)

Homo sapiens SRSF protein kinase 2 (SRPK2):

tttccctttatagcaccattgaatcccagtcctaacagaagtactgcgaatcttgtggcc tcattctgaacaaaagggattagagaagaaaaatctcttgatataaggcttgaaagcaag ggcaggcaatcttggttgtgaatattttctgattttccagaaatcaagcagaagattga gctgctgatg (Seq ID No: 1220)

Homo sapiens synaptophysin-like 1 (SYPL1):

tgcccttcctcgccaccgggctgctctggtctcgtcggtcccctcctccgccccgtcgtc
ctgactctctctccctcctttcctcagaggatg (Seq ID No: 1221)

Homo sapiens thioredoxin reductase 1 (TXNRD1):

aaccctttcacctcagttttcttcactccggcatttgcagcagagcgaaaggtggtcgag
tcctgaaggagggcctgatgtcttcatcattctcaaattcttgtaagctctgcgtcgggt
gaaaccagacaaagccgcgagcccagggatgggagcacgcgggggacggcctgccggcgg
ggacgacagcattgcgcctgggtgcagcagtgtgcgtctcggggaagggaagatattta
aggcgtgtctgagcagacggggaggcttttccaaacccaggcagcttcgtggcgtgtgcg
gtttcgacccggtcacacaaagcttcagcatgtcatgtggcttatcaggagggcagactt
caaaagctactaaaaatg (Seq ID No: 1222)

Homo sapiens minichromosome maintenance complex component 7 (MCM7) :

tgtccttccgcgcggcggccgcggagagagctgcggcccggggggcgtgcctgggatcc
ggagcttcgctcgggcccgggaaaggcggcagtgggctgggatcgcggtgtctctgggtg
tgatggccaatggctggactggctcccgccctgggcggaggaatcccgagctgtgaagcg
gctggaatccgggcccatgtgcttctttgtttactaagagcggaagcgatggcgggagcg
ggggtggggtgcggtggcggggtgcggtggcggaggtcccggtgaaatcaggggctaagg
ggacccaaagaaggcgggggatcataggggtggaaagaaagctgagaaccttgagaccgg
agtgtgaggggccaacggggaagggcgctagaattttaaactaaagtagggaccggaatt
cccctggggagatgttggatggccctgtgcactgccacgggctctttattcttcgctggt
tagaaacagacttgtgaaaaagagttatgcccactttggggagacttcgaaaaggttaag
aagttcttacaagagttctaccaggatgatgaactcgggaagaagcagttcaagtatggg
aaccagttggttcggctggctcatcgggaacaggtggctctgtatgtggacctggacgac
gtagccgaggatgaccccgagttggtggactcaatttgtgagaatgccaggcgctacgcg
aagctctttgctgatgccgtacaagagctgctgcctcagtacaaggagagggaagtggta
aataaagatgtcctggacgtttacattgagcatcggctaatgatggagcagcggagtcgg
gaccctgggatggtccgaagcccccagaaccagtaccctgctgaactcatgcgcagattg
tgagtggtctctgtcgggaaagatgtaggattggttctccaggatcttgtttgtgactg
ttttctccccttagtgagctgtattttcaaggccctagcagcaacaagcctcgtgtgatc
cgggaagtgcgggctgactctgtggggaagttggtaactgtgcgtggaatcgtcactcgt
gtctctgaagtcaaacccaagatg (Seq ID No: 1223)

Homo sapiens pre-B-cell colony enhancing factor 1 (PBEF1):

tttccccctctcccccctcctccgccgaccgagcagtgacttaagcaacggagcgcggtga
agctcatttttctccttcctcgcagccgcgccagggagctcgcggcgcgcggcccctgtc
ctccggcccgagatg (Seq ID No: 1224)

Homo sapiens cyclin B1 interacting protein 1, E3 ubiquitin p rotein ligase (CCNB1IP1):

ctttctttccctctccgttttggtgggctggttgaagatgaaatccactgaggagggaag

tccagcaccctgtgtgccagtccagaactggcccatctgtagacccccctgaaaatcatat
gggcttggatttggatattctcaacagaaagggttaaaggctgatggtacctaaagcctg
gtacttgaattttgatcaagataagctgccttaagttctcttcattacacaaatgatcct
agataattgatagatcctgtggttcaactggatttctagatagaagctggattcatgtga
tgccagaggagtaaaatttcaagagactgaaaccagatctgagtttcgctgttccagtct
ggacctctttggtgctgtaaatcctggatatactgtagatgagtactgcgtttttctttt
atggcctctcttcagcttctggagacctcactatcctattatg
(Seq ID No: 1225)

Homo sapiens STEAP family member 3, metalloreductase (STEAP3) :

ccgccttcgccgcggaccttcagctgccgcggtcgctccgagcggcgggccgcagagatg
acatttattcattttatgcatcctgggttctactggtcgtcccacctcagttcctgtagc
aaagagacttgagtctgagccactaattatcacccgtgaggtttcctccccgagcaggaa
gcagcaggccagagctgcgctctctcagtgcactctccaaccaagcatcagtcaccactc
ccggtccagcccctgtggccaagagctggcgtgcaggctgcgggaggcagctggctgtgc
aagaccctggcagggccctcgcctcctgagaaaccgagagtcagaaccaaagccaggctg
tcctggttggagactgagccagaaagggtggctcacctcacggtgaggctgtcgagtgac
ctgagagcctcagaccctcacgtcagccggatg (Seq ID No: 1226)

Homo sapiens nicotinamide nucleotide transhydrogenase (NNT):

tgttcttccgggttggaggcgcagcgccgcggggcccaagcccgggtctgccagcgcgac
gtcctctcgcggccctcagggcacagcccaaggctgtcagcctcccggcccagtgatttg
ccttcaaggaaactggggagtcagaaaattgggaactcatatcaacatg
(Seq ID No: 1227)

Homo sapiens SHC (Src homology 2 domain containing) transforming protein 1 (SHC1) :

gtccctctccctccccaggacttctgtgactcctgggccacagaggtccaaccaggctaa
gggcctggggatacccccctgcctggccccccttgcccaaactggcagggggggccaggctgg
gcagcagcccctctttcacctcaactatg (Seq ID No: 1228)

Homo sapiens bromodomain containing 8 (BRD8): cggcccttccagaccgtctctcctcagggttggagacttcggggccaagatg (Seq ID No: 1229)

Homo sapiens ring finger protein 13 (RNF13):

tcgcctctttagtaggtcgggtgagtgtagtgtgcagggaagagacgcgtcagcgccagg
gccaggcccgcccgggggcagcccggcagccgaatcttgggctactctgtcccaacagcc
ggagcagatcagaccgaccggccctgcccgctcggtcccgcgccctccagaccctacggt
ctccgtttctaggggcacatggttagcggcaggcgcccacagccaatccactttgccagc
ctgccccttcctctgccaagagcagcttcttcagccgcgctccagttccgcagacgcctg
ccccaccctgctcttcccttccagggaagacggatcacgcggccaagaacgagactcgca
aactgggcatttctccgagccgggctagagcaagtagcgagactccgcgtgagagtggga
aagagccttaacaggcaaccatgttgcccagtgggttttctgtgcctttgggtgcggacc
aatgaggcgcgtggggcgggacttccgcttcgcctaggtgttgtcgtccctgctagtact
ccgggctgtgggggtcggtgcggatattcagtcatgaaatcagggtagggacttctcccg
cagcgacgcggctggcaagactgtttgtgttgcgggggccggacttcaagagagaaagag

agagtgggcagacatcgaagccaaacagcagtatcccggaagcactcatgcaactttggt ggcggccactcagttttctctgccagtgtctggtgattttacaacgagatg (Seq ID No: 1230)

Homo sapiens aldolase A, fructose-bisphosphate (ALDOA):

ccgcctcctgcgccgccccttccgaggctaaatcggctgcgttcctctcggaacgcgccg cagaaggggtcctggtgacgagtcccgcgttctctccttgaatccactcgccagcccgcc gccctctgccgccgcaccctgcacacccgcccctctcctgtgccaggaacttgctactac cagcaccatg (Seq ID No: 1231)

Homo sapiens LY6/PLAUR domain containing 6 (LYPD6):

cgctccttccctgagctcccgggctccggcagcgcgctggcggggcgccgcattgcacac tctgggggcgccgcagtgttcgtgggatggggcagcgggctgcagctggcggccggaatc cgcgcgcagcccgggtgcaagttctctcctgttgccctgagtgcccactcccaggccctc tgtatgagtgacacttcagtctgccatg (Seq ID No: 1232)

Homo sapiens butyrophilin, subfamily 3, member A1 (BTN3A1):

cagtctctgctttctttttcctttcttccagaaggagatttaaccatagtagaaagaatg gagaactattaactgcctttcttctgtgggctgtgattttcagaggggaatgctaagagg tgattttcaatgttgggactcaaaggtgaagacactgaaggacagaattttggcagagg aaagatcttcttcggtcaccatacttgagttagctctagggaagtggaggtttccatttg gaattctatagcttcttccaggtcatagtgtctgccccccaccttccagtatctcctgat atgcagcatgaatg (Seq ID No: 1233)

Homo sapiens lipoic acid synthetase (LIAS):

ctgtcctttcccgggagttagcgatccctcaacccctgcactgcgctagtcctaaagagg aaatg (Seq ID No: 1234)

Homo sapiens C-type lectin domain family 7, member A (CLEC7A) :

gattctcttttgtccacagacagtcatctcaggagcagaaagaaaagagctcccaaatgc tatatctattcaggggctctcaagaacaatg (Seq ID No: 1235)

Homo sapiens CD247 molecule (CD247):

actccttttctcctaaccgtcccggccaccgctgcctcagcctctgcctcccagcctctt tctgagggaaaggacaagatg (Seq ID No: 1236)

Homo sapiens myeloid zinc finger 1 (MZF1):

aagcctttctccattttgcggtctaggaagtagcagaggccccttcctgtagggagttgc
catggagacgcggtggggcaccgacggagttctaatgacggccgtgattggtgcaggatc
ctgctaatctcaggaaggcccgtagagaagtgaggaaaacgtggtggggggcatgcgcga
tctggtaggcggtgctgccgtctgttgtacctgagaggcttgcgcatgccgacgcacgga
ttcgaggcggggagcatgggaagaagcggccaggagtatgacctgatcattgcgaccacc
gctaggggaagggaggagagggtgtagaaacggggacgagggtggggggaagggcaaggag
gcgctcgagctggtgcgcggagcatcctgggagacgtagtccagcgggaggggggaagtcg
aagactgcgcgtgctcaggagcgcggagcggcccgctgagcgcagaggggcagacactgg
cctcagatacctgacctggtaccctctatg (Seq ID No: 1237)

Homo sapiens E2F transcription factor 6 (E2F6):

cctcctctttttccgtctgcgtcgggagctcccgggcacgtgaggccgtgccgcgtttac

tggcgggcgggacggcctagccgggcggcgcctcggaggaagccgcggaccccttaggtg
ctgggcccttggaaatcggcgcgtgggggcggtgctcgagctgagcgcgagagggcggg
agagctcgtggggtgcgaggggagcaggacgcccggccgggcagcatg
(Seq ID No: 1238)

Homo sapiens purinergic receptor P2Y, G-protein coupled, 10 (P2RY10) :

cttcctctttcaacaacaaatgtgtcagttatcagcaggatccatgccgccagagtaaag
ctttctaccctttactccctgcaaagaaacaagagtgcttatcccagctaagctccaggg
taatgttatcatgacagcttcaactttagaccacaggcaaatgctttgttaaaactcta
tgctggtcattcccttcaggatttggcactcaccaacatacccttctttcaagtgaaaag
gcatctcttttaatggtcctgacctttggaataggaagcatgtaccctggacagagcact
tcaaactagaggaaccataaatccatg (Seq ID No: 1239)

Homo sapiens chromosome 9 open reading frame 85 (C9orf85): catccttttgcctgctcccggcgaggggtggctttgatttcggcgatg (Seq ID No: 1240)

Homo sapiens ERGIC and golgi 3 (ERGIC3):
cgtcccctttccggccggtccccatg (Seq ID No: 1241)

Homo sapiens ankyrin repeat domain 46 (ANKRD46):

ccctcccctccgcccgtcaccgcctccttgaagctgccgctgtcgctgctgctcgttcga
gtcgcagatccttgccagcacattacagaatattttgttgaaccttcttgagaattcag
agaaactgctgagtgaccactgaacgaaaagatctaatcttaaggcttacgcctcacttt
gatgcccaggctggagtgctgtggctcaatcacagctcatcgcaacctcgacctcccggg
ctcaagtgatcctctcacctcagcgtcccgaacaggcgtgttccatccaccacatcagaa
caatg (Seq ID No: 1242)

Homo sapiens Ras and Rab interactor-like (RINL):

tcctctctccacttcctgctactgcaggcctctcctccgagaacagaggccaggtcatga
ctcactggcttcctgcaacctgacgatggcccagccagaagacaaggcacctgaagtccc
cacagaggggggtgaggtgaacaaagcagacaggacccctctaggggtcctcagcacccta
gagccacttactcgcctgcagaggacatggggggtgtggcatgtgccagagctggatacc
caggatgcggaggcccttgtggggctgtggccactagggagtttcttggtcacaggacgt
gaccccagccaggccctggtgttgaggtcaggacctttaccaggagaagtcaatacctac
cagatccagaagattcccagaggtgtgtccctggaatcctccaacctctgcatg
(Seq ID No: 1243)

Homo sapiens embigin (EMB):

ccgcctttcttcagcgtcctacccgcggcactggctgcgagcgccgggccacctgcgag
tgtgcgcagggactctggacacccgcggcggcgagctgagggagcagtctccacgaggac
ccaggcggaccctctggcgccatg (Seq ID No: 1244)

Homo sapiens MMS22-like, DNA repair protein (MMS22L):

ccgcctttccggagcgcgggcgcgcggtggcgggaatttcgcctgtttgcggtttagacc
ccaaagattcctgttggtggtctgggtcacaggaggcaggtttcgggagctggaaatgtg
agcgggtacgacaggcaccgcgggtaaccgacgccccgggtccttgctgcagccgggtac
gcgggataccggcaccccgccttctccgcccgagtgctgccaggcgtgggcctggaatct

cttcacaccttctctttggagcccttaatgatacgacgaaccccaagtgtttcagaacat
gaagtaaacaatg (Seq ID No: 1245)

Homo sapiens chromosome 19 open reading frame 54 (C19orf54):

actcctttcctttttccagtggttatcgcggcgcccaccggcctctgatctctgagtctt
ctccaacccacagacgtttttttgttgctctggttccaggaccttctccacaactaggcca
ttttccctgccaggtgtcctttttgacctcttgacctctgactcaaaggcctgctcccc
ctcatgtcttcggcctggagaagagccagctcctgaaggaggcctttgataaggccggcc
cggtccccaagggcagagaagatgtgaagaggcttctgaaactacacaaggaccggttcc
gaggtgacctgcggtggatcctcttctgtgcagacctgccgtccctcatccaagaaggcc
ctcaatg (Seq ID No: 1246)

Homo sapiens zinc finger protein 621 (ZNF621):

cgcccttccggctcggcctttagttagtgaccagctcctcggcgttctgcagagcgtggg
tttcagcgagttctacgtgccaggtccgcccggtgccggcttcctcgctgcccctggcgg
ctcgtcagcccccactacccctgaacttggtcccaatggcggcccgcccctccttcaccc
ggaccgtgggcatctgggcctcgccgaagccgtcaaggtggctgctcggccttctagagc
ccgtgtccagccctttgccaccgaggcctgatcctcttttctgccctaaagaacttgccc
tgacagcctctggctcccgctcttgaggatcttgcttgtccaaacccagaagacagtgca
tgaagccaggggacatccgccatg (Seq ID No: 1247)

Homo sapiens family with sequence similarity 73, member A (FAM73A): ccgccttctccatg (Seq ID No: 1248)

Homo sapiens RNA binding motif protein 43 (RBM43):

ccgccctttcttcgtagcctccaagggagctggaacaaaaacgaaaccaaaacctgcc tgctcgctcctctccccatcgcctgcgttccgctggttgtgggctttctgcggccgctga gggcgcgtctcccctccgccatg (Seq ID No: 1249)

Homo sapiens spermatogenesis and centriole associated 1 (SPATC1) :

caccctccttcagcccaggcaaggcctggggccctgggcagcctccaggtgcagtgccct cccgtgggccgcacccttgccactgccccagggcatg (Seq ID No: 1250)

Homo sapiens carbonic anhydrase XIII (CA13):
ctttctcttccttccaccccgagggaccatg (Seq ID No: 1251)

Homo sapiens transglutaminase 2 (C polypeptide, protein-glutamine-gamma-glutamyltransferase) (TGM2) :

cgctctccgcctcggcagtgccagccgccagtggtcgcacttggagggtctcgccgccag tggaaggagccaccgcccccgcccgaccatg (Seq ID No: 1252)

Homo sapiens NOP2/Sun domain family, member 4 (NSUN4):

atttcctttccctttttcgctcgtgtcccgccgggtggcgctcaccacctccccggaac acgcgagtctcctgtcgcggttccggtcggaattaccccgtggagcacgccgatatg (Seq ID No: 1253)

Homo sapiens mitochondrial ribosome recycling factor (MRRF):

gagtctttccttagtaacctgggcgatagctgtggatgtttccaaggattgtcttcagtc atg (Seq ID No: 1254)

Homo sapiens PHD finger protein 17 (PHF17):

cttcctccataacaagccaaacgccagaccgagagtgcctccgtgcgcgagtgcccggtg tgtgcgcgccggcgagagcaggggcccgcccggctccccgcccgccgcggcccgaactca tgcagctccgagcgagcgagcggcgcccagcccagcgcctcggccgaacccctccgcagc aggctgcctgctgtttcccggggagatcatg (Seq ID No: 1255)

Homo sapiens prolylcarboxypeptidase (angiotensinase C) (PRCP): cctccttttcgccctcccacccgcactgcagtctccagcctgagccatg (Seq ID No: 1256)

Homo sapiens proteolipid protein 1 (PLP1):

aagcccttttcattgcaggagaagaggacaaagatactcagagagaaaaagtaaaagacc gaagaaggaggctggagagaccaggatccttccagctgaacaaagtcagccacaaagcag actagccagccggctacaattggagtcagagtcccaaagacatg (Seq ID No: 1257)

Homo sapiens coiled-coil domain containing 80 (CCDC80):

cagccttctcactcctcactgagtccactctgaacgtgctaaaatgggaaggaggcggtg
ttttgctgatctgttaaattcttagtgaagtttccttgatttccagtggctgctgttgtt
tgagtttggtttggagcaaaactgaggtagtcctaacatttctgggactgaatccaggca
agagaaagaagaaaaagaagaagaaaaagaggaggaaaaaggtagggagaaataaaggga
ggagagaagcacagtgaaagaaaaaaaaagtcccttttcgacatcacattcctgtgtttt
ccctcagcctggaaaacatattaatcccagtgcttttacgcccggaaacaaagagactaa
gccagactatgggggaaagggagataagaaggatcctggaactttaaagagggaaagagt
gagattcagaaatcgccaggactggactttaagggacgtcctgtgtcagcacaagggact
ggcacacacagacacacgagaccgaggagaaactgcagacaaatggagatacaaagactt
agaaggacagctcctttcacctcatcctacttgtccagaaggtaaaaagacacagccaga
aagaaaaggcatcggctcagctctcagatcaggacaggctgtggatctgtggcggtactc
tgaaagctggagctgcagcacaccccttttgtattgctcaccctcggtaaagagagagag
ggctgggaggaaaagtagttcatctaggaaactgtcctgggaaccaaacttctgatttct
tttgcaaccctctgcattccatctctatgagccaccattggattacacaatg
(Seq ID No: 1258)

Homo sapiens chromosome 20 open reading frame 44 (C20orf44):

cgacctctttgcgcctgcgccccccttgccagtctttcgccggcaaaaggaggacgtaga
aaaggggacaccggaaactcactcttcacccggaaatggttattgaggaacatg
(Seq ID No: 1259)

Homo sapiens tryptophanyl tRNA synthetase 2, mitochondrial (WARS2): cgcccttctcaagatg (Seq ID No: 1260)

Homo sapiens myotubularin related protein 2 (MTMR2):

ctttccctgtgctgcccctgccgcgcgatggagaagagctcgagctgcgagagtcttggc
tcccagccggcggcggctcggccgcccagcgtggactccttgtccagttaatgtgttaag
agccattgacatttgaagatcatcagaagtgaagataaaacatctcaaaaattataattg
cctccacttctcattcagagaattcagtgcatacaaaatcagcttctgttgtatcatcag

attccatttcaacttctgccgacaacttttctcctgatttgaggagggagtctcgctcta
tcccctaggctggagtgcattggcgccatctcggctcatttgcaacctctgtctcccggg
ttcaagcgattctcctgcctcagcttcccgaggagctgggattacaggtcctgagggagt
ctaacaagttagcagaaatg (Seq ID No: 1261)

Homo sapiens reticulon 3 (RTN3):

cgccctctagctgcgctcggctgagtcagtcagtctgtcggagtctgtcctcggagcagg
cggagtaaagggacttgagcgagccagttgccggattattctatttcccctccctctctc
ccgccccgtatctcttttcacccttctcccaccctcgctcgcgtagccatg
(Seq ID No: 1262)

Homo sapiens G protein-coupled receptor 56 (GPR56):

gtccctccctctccgcactagctgtctgccctgccctgccgtaggagatgggctgggagc ctcccacgctctccagctcactcggcaggcagcggggaccagggctggcaggttaagcct ctggggtggatcctgaaaggtggtccagccgcctggccctgcgtgggaccctccacctg gcagcagacagggtctcgctctgtcacacaggctggagtgcagtggtgtgatcttggctc atcgtaacctccacctcccgggttcaagtgattctcatgcctcagcctcccgagtagctg ggattacaggtggtgacttccaagagtgactccgtcggaggaaaatg (Seq ID No: 1263)

Homo sapiens immunoglobulin superfamily containing leucine-r ich repeat (ISLR):

gctcctccctgccgcctcctctcagtggatggttccaggcaccctgtctggggcagggag ggcacaggcctgcacatcgaaggtggggtgggaccaggctgcccctcgccccagcatcca agtcctcccttgggcgcccgtggccctgcagactctcagggctaaggtcctctgttgctt tttggttccaccttagaagaggctccgcttgactaagagtagcttgaaggaggcaccatg (Seq ID No: 1264)

Homo sapiens glycoprotein M6A (GPM6A):

atttcttttccccatttaaatgcaaagcaagacttgtgaatcatagtgtctctgctcct gggattcagaccaaatttccccccaaaattctcaggctatttgtttgaatacctgcttac agtggtacacaatggcagctttgagaagaaaaattgataatcttcacggaagagtaatt tgaatgaaattacacttgacagcctgtctccaagcaaacaagaggaacgagggagcctga gctaagctctgaggacttgcccaagccactgctgttggagcttcccaggaaaaaaaaaaa aaaaaaaaaaaaaaaaaaaaaaaaaaaacaccagtttttccaacatctaattgagcttt tgattaattccgtgtaccagattctactgaagaaaggtagccatg (Seq ID No: 1265)

Homo sapiens splicing factor 1 (SF1):

ctccctctttgtgcgtctcgcgccgccgccgcccgccgcgtgagaggacgggctccgcgc gctccggcagcgcattcgggtcccctccccccgggaggcttgcgaaggagaagccgccgc agaggaaaagcaggtgccggtgcctgtccccgggggcgccatg (Seq ID No: 1266)

Homo sapiens cell cycle associated protein 1 (CAPRIN1):

ccgcccctcgcgacccagagggctgctggctggctaagtccctcccgctcccggctctcg cctcactaggagcggctctcggtgcagcgggacagggcgaagcggcctgcgcccacggag cgcgcgacactgcccggaagggaccgccacccttgccccctcagctgcccactcgtgatt tccagcggcctccgcgcgcgcacgatg (Seq ID No: 1267)

Homo sapiens hypothetical protein FLJ90297 (LOC388152):

ctgccctcttgcgtgccccggccacccccgggcggcttgtagccggtgcgcggggtggct ggggctacgtgcagagctgtcgcggagccggaacagcagcggtgaagcccctcggctcgg ccgagaccgccgtgcccattgctcgcctcggttgccgccgctttagccgcagccgctgct gccgccgccgggggagaggcagcctattgtctttctccgcggcgaaggtgaggagctgtc tcggctcggcccgcgggggagccccgggagccgcacggagatggaggaggacatctggac agtgagcaggaggcgcctcggcccatg (Seq ID No: 1268)

Homo sapiens kelch-like ECH-associated protein 1 (KEAP1):

cgccctctccccgcctccttttcgggcgtcccgaggccgctccccaaccgacaaccaaga
ccccgcaggccacgcagccctggagccgaggcccccgacggcggaggcgcccgcgggtc
ccctacagccaaggtccctgagtgccagaggtggtggtgttgcttatcttctggaacccc
atg (Seq ID No: 1269)

Homo sapiens F-box protein 38 (FBXO38):

ctccctctcaaccacaataacaggcggagggtcggcgtaggtactttgaactcaagtaaa
caaaagggaagattttctcgttgatactggagactgcacaacaatg
(Seq ID No: 1270)

Homo sapiens musculoskeletal, embryonic nuclear protein 1 (MUSTN1) :

agatcttttccagcagctgctgcctgccagagaggcgccttcagagacccagcgcttaca
caatacccaccatg (Seq ID No: 1271)

Homo sapiens QKI, KH domain containing, RNA binding (QKI):

cctcctctccggcggcggcggcggcggcgggcggagtgagctgcggagcctggaata
tg (Seq ID No: 1272)

Homo sapiens protein phosphatase 1, catalytic subunit, beta isoform (PPP1CB):

gggcctctcttgtttatttatttattttccgtgggtgcctccgagtgtgcgcgcgctctc
gctacccggcggggaggggggtgggggagggcccgggaaaaggggggagttggagccgggg
tcgaaacgccgcgtgacttgtaggtgagagaacgccgagccgtcgccgcagcctccgccg
ccgagaagcccttgttcccgctgctgggaaggagagtctgtgccgacaagatg
(Seq ID No: 1273)

Homo sapiens methyltransferase like 21B (METTL21B):

cagcctctaccccgctccggatccgggatctgagcgccggccgcggtgcccaggcactcc
cttggcgggccggatg (Seq ID No: 1274)

Homo sapiens adaptor-related protein complex 3, mu 1 subunit (AP3M1) :

cggccttctcggcttctccagcttcggtaggagaggatccggcgccgaatcactgactgg
cacaggtgttgggatagtgtctcacttggtcacccaggctggagtgcagtggcgcaatct
tagctctctacagcgtcgatcttcctcctgggctcaagcaattctcctgcttcatcctcc
tgagtacctaggactacagaaaatg (Seq ID No: 1275)

Homo sapiens muscleblind-like splicing regulator 1 (MBNL1):

cagtcttttcactgcagctgaatgagttgtggcgcccacaatgctcccatgacaaggagc
tgacaagttccattttccgtcgcggcatcttggaatcatgactcccacaatgccttggg

cacttggtcgacagtggggccgcctctgaaaaaaaaatgtgagaggttggtactaagaag
tgcctttcctgacgtctctgctgcttggaaccgcttctagagcagtctctgcttttgcct
tgcttgctgccagctagactgtgacgacagcacatccaccctccacctctagcccagaca
cccccatttctacttataatcaagagaaaagctctaagtatctggcattgccctaggctg
ctttagtgttaaaagaaaagtttgctgaaaaagtaagatatcttctgccaggaaatcaag
gaggaaaaaaaaaatcatttctcgattttgctctaaactgctgcatctgtctatgccaa
actaatcaataccgattgcaccaccaaactccattgcaaattcagctgtgaggagattcc
ctttcagacaactttgctgaaagcagcttggaaattcggtgtcgaagggtctgccacgtt
ttcatgcttgcattttgggctccaaattggcactgggaaggggttactgagagcacaagg
ctgataccaggccctactttaaacgttcatctacttacaatcctagtatttctctaaaa
accaaaacctctttgaattaacagtttcatgctgtgaatttctagtgggagatcttttcc
ttgatattgacgacacaattttccatgtactttaaagcagggagtggggaaaagtattt
tgaggggacattttcatcatcagttcagcttttttttttggttgttgctctttttttggg
ggggttgggtttgttggtttcactgaaacatttaactacctgtaaaatctaaacatg
(Seq ID No: 1276)

Homo sapiens lipid phosphate phosphatase-related protein typ e 1 (LPPR1):

cagccttttgctctttcctttcattaaacaaacaggagatcctgaaacctggaccctgtg
caagctgcagcgccaggaggaggcagcggaggaagcagagcgcgggatgggcgcccagcg
gcatctgtgatcccgcgcacctccgccccacgggcgcgcgcacaaacacggacacacaca
tacacacactcgcgcacacactcgcacaaacacacactcgtacacgcccgcgccgctcgc
tcgccggcttgctctcccacgcaagcggaatgcagcagcgcctggagagcgtgtctcgga
ccgccgcctgaatgtacctcgctcccgggagccggacggcccagtagggcgcactggagg
acgctccgctgcgggagcctggacagtttttgacggtgcagtcttgctatatggtgtgag
aaatg (Seq ID No: 1277)

Homo sapiens muscleblind-like splicing regulator 2 (MBNL2):

ctgtctttgcttcatcatctgaaggtaaaattttccagatacggcagacggctttcagag
tacaataaacagggaatgagaactatttacatggaagtttctttctcatgatgcggtgga
gaagcctcggccacttggttctgccagatgttcctggggttactgtaaatggaaggaca
ggcagagctaaacaaggtttatcatttaaaagtgcctgtgtgaagtcacttttgctggaa
aactgcagcttgggagctttctttgtattcacatcccactcttctgtcaagtacacttta
ccctgaccttatgagtggatgaagatacctcagttgtctgactttgccaattgcttaatt
tcagaatttaaaaaggggaaagaaaaacatcctgctaaaatatgaacatctgagtgtctt
attttccaacatcgtcaatagctgtgagcgtcagcattaaatattctcccaaggagtgcc
atgatattgaagtcactttattaataacagctgtatctgcaaaacagtcaagagactcgg
acgttgaaagccagagatgacactgagcatgcttttattgcggcctaccatctttaagtg
ggacatattgattgatgagtgattgcctgtccatacactctctcatcatcctgttccttg
gattggacttcactaagcaatttatcactcaccttcagacttacatgtgggagttttcac
aacagtagttttggaatcattagaacttggattgatttcatcatttaacagaaacaaaca
gcccaaattactttatcaccatg (Seq ID No: 1278)

Homo sapiens chromosome 3 open reading frame 25 (C3orf25):

gcgcctttcgcacgacttggagttacggtttatctgataccgcggtacccctacgcaagc
aagcccacatcgacacacattcacacacgcccttcagcaccccctcccagcaccacgacc
atg (Seq ID No: 1279)

Homo sapiens testis expressed 19 (TEX19):

cctcctcctttccctgggtgcccacatgaacagagacaccaggatgctctcctgagacca
cagcaactgcagaagctgaagacatttccagaagttcaagcttccaccctctgcaggtcc
ccactgagctgggacccaggtcatccaccccaccccaaatccctggataggaaaccctt
tctcctcctgctccttgtccccttcatccctgccgcccagcatcctactggcctcagcac
ctgtggccagaccgtccaagatcctctgaaggcccagctcttgctgtccaccccggcagt
aggcaggcagcctggccatg (Seq ID No: 1280)

Homo sapiens protein kinase C, beta (PRKCB):

gcctccctcccccgcagctggggccagcggtgccaagcgcagctggacgagcggcagcag
ctgggcgagtgacagccccggctccgcgcgccgcggccgccagagccggcgcaggggaag
cgcccgcggccccgggtgcagcagcggccgccgcctcccgcgcctccccggcccgcagcc
cgcggtcccgcggccccggggccggcacctctcgggctccggctccccgcgcgcaagatg
(Seq ID No: 1281)

Homo sapiens protein kinase N1 (PKN1):
ccctccctccgcgcggggacccctggcgggcggcaggaggacatg
(Seq ID No: 1282)

Homo sapiens hemochromatosis type 2 (juvenile) (HFE2):

ccttctctggttccctgacctcagtgagacagcagccggcctggggacctggggagaca
cggaggacccctggctggagctgacccacagagtagggaatcatggctggagaattgga
tagcagagtaatgtttgacctctggaaacatcacttacagggcttccggtcaaaattcac
taggtaggagggtcatcagctgggaagaaccggcgcctgggaaacctggctggataggta
tg (Seq ID No: 1283)

Homo sapiens ribosomal protein L9 (RPL9):
cgttctttctttgctgcgtctactgcgagaatg (Seq ID No: 1284)

Homo sapiens ribosomal protein L3 (RPL3):
cggcctctaccggcgggatttgatggcgtgatg (Seq ID No: 1285)

Homo sapiens ribosomal protein L4 (RPL4):

acttccttttcctgtggcagcagccgggctgagaggagcgtggctgtctcctctctccgc
catg (Seq ID No: 1286)

Homo sapiens ribosomal protein L5 (RPL5):

tggccctttcccacccctagcgccgctgggcctgcaggtctctgtcgagcagcggacg
ccggtctctgttccgcaggatg (Seq ID No: 1287)

Homo sapiens ribosomal protein L6 (RPL6):
aattctctttcccatcttgcaagatg (Seq ID No: 1288)

Homo sapiens ribosomal protein L7 (RPL7):
cttcctctttttccggctggaaccatg (Seq ID No: 1289)

Homo sapiens ribosomal protein L7a (RPL7A):
ctttcctttctctctcctcccgccgcccaagatg (Seq ID No: 1290)

Homo sapiens ribosomal protein L11 (RPL11):
ctttctcttcctgctctccatcatg (Seq ID No: 1291)

Homo sapiens ribosomal protein L12 (RPL12):

cggcctctcggctttcggctcggaggaggccaaggtgcaacttccttcggtcgtcccgaa
tccgggttcatccgacaccagccgcctccaccatg (Seq ID No: 1292)

Homo sapiens ribosomal protein L13 (RPL13):
gcttcctttccgctcggctgttttcctgcgcaggagccgcagggccgtaggcagccatg (Seq ID No: 1293)

Homo sapiens ribosomal protein L23 (RPL23):
acttcctttttcttttttccggcgttcaagatg (Seq ID No: 1294)

Homo sapiens ribosomal protein L18 (RPL18):
cgttctctctttccggacctggccgagcaggaggcgccatcatg (Seq ID No: 1295)

Homo sapiens ribosomal protein L18a (RPL18A):
acttccttttgcgggtggcggcgaacgcggagagcacgccatg (Seq ID No: 1296)

Homo sapiens ribosomal protein L19 (RPL19):
agctctttcctttcgctgctgcggccgcagccatg (Seq ID No: 1297)

Homo sapiens ribosomal protein L21 (RPL21):
gcctctttcctttcggccggaaccgccatcttccagtaattcgccaaaatg (Seq ID No: 1298)

Homo sapiens ribosomal protein L22 (RPL22):
acctccctttctaactccgctgccgccatg (Seq ID No: 1299)

Homo sapiens ribosomal protein L23a (RPL23A):
agacccttttcacaagatg (Seq ID No: 1300)

Homo sapiens ribosomal protein L17 (RPL17):

cgctcttcctctttccctaagcagcctgagggttgactggattggtgaggcccgtgtggc
tacttctgtggaagcagtgctgtagttactggaagataaaagggaaagcaagcccttggt
gggggaaagtatggctgcgatgatggcatttcttaggacacctttggattaataatgaaa
acaactactctctgagcagctgttcgaatcatctgatatttatactgaatgagttactgt
aagtacgtattgacagaattacactgtactttcctctaggtgatctgtgaaaatg
(Seq ID No: 1301)

Homo sapiens ribosomal protein L24 (RPL24):
ttctctctttcttttcgccatcttttgtctttccgtggagctgtcgccatg (Seq ID No: 1302)

Homo sapiens ribosomal protein L26 (RPL26):
agttctcttcccttttgcggccatcaccgaagcgggagcggccaaaatg (Seq ID No: 1303)

Homo sapiens ribosomal protein L27 (RPL27):
ctttcctttttgctggtagggccgggtggttgctgccgaaatg (Seq ID No: 1304)

Homo sapiens ribosomal protein L30 (RPL30):

aagtctttcctttctcgttccccggccatcttagcggctgctgttggttggggggccgtcc cgctcctaaggcaggaagatg (Seq ID No: 1305)

Homo sapiens ribosomal protein L27a (RPL27A):
ccttcctttttcgtctgggctgccaacatg (Seq ID No: 1306)

Homo sapiens ribosomal protein L28 (RPL28):
cttcctctttccgtctcaggtcgccgctgcgaagggagccgccgccatg (Seq ID No: 1307)

Homo sapiens ribosomal protein L29 (RPL29):
cagcccctttctcttccggttctaggcgcttcgggagccgcggcttatggtgcagacatg (Seq ID No: 1308)

Homo sapiens ribosomal protein L31 (RPL31):
cgctcttcctttccaacttggacgctgcagaatg (Seq ID No: 1309)

Homo sapiens ribosomal protein L32 (RPL32):

ccgtcccttctctcttcctcggcgctgcctacggaggtggcagccatctccttctcggca tcatg (Seq ID No: 1310)

Homo sapiens ribosomal protein L35a (RPL35A):

cgtccttctcttaccgccatcttggctcctgtggaggcctgctgggaacgggacttctaa aaggaactatg (Seq ID No: 1311)

Homo sapiens ribosomal protein L37 (RPL37):
ccttctcttccggtctttctggtctcggccgcagaagcgagatg (Seq ID No: 1312)

Homo sapiens ribosomal protein L37a (RPL37A):
gcgtctcttcctttctgggctcggacctaggtcgcggcgacatg (Seq ID No: 1313)

Homo sapiens ribosomal protein L38 (RPL38):

cgttctttttcgtccttttccccggttgctgcttgctgtgagtgtctctagggtgatacg tgggtgagaaaggtcctggtccgcgccagagcccagcgcgcctcgtcgccatg (Seq ID No: 1314)

Homo sapiens ribosomal protein L39 (RPL39):
ccctcctcttcctttctccgccatcgtggtgtgttcttgactccgctgctcgccatg (Seq ID No: 1315)

Homo sapiens ribosomal protein, large, P0 (RPLP0):

aggcccttctctcgccaggcgtcctcgtggaagtgacatcgtctttaaaccctgcgtggc aatccctgacgcaccgccgtgatg (Seq ID No: 1316)

Homo sapiens ribosomal protein, large, P1 (RPLP1):

cggtccttccgaggaagctaaggctgcgttggggtgaggccctcacttcatccggcgact agcaccgcgtccggcagcgccagccctacactcgcccgcgccatg (Seq ID No: 1317)

Homo sapiens ribosomal protein, large, P2 (RPLP2):

ccttcctttcctccctgtcgccaccgaggtcgcacgcgtgagacttctccgccgcctcc gccgcagacgccgccgcgatg (Seq ID No: 1318)

Homo sapiens ribosomal protein S3 (RPS3):
acttcctttcctttcagcggagcgcggcggcaagatg (Seq ID No: 1319)

Homo sapiens ribosomal protein S3A (RPS3A):
ccgcccttttggctctctgaccagcaccatg (Seq ID No: 1320)

Homo sapiens ribosomal protein S4, X-linked (RPS4X):
ggtcctctttccttgcctaacgcagccatg (Seq ID No: 1321)

Homo sapiens ribosomal protein S4, Y-linked 1 (RPS4Y1):
gattctcttccgtcgcagagtttcgccatg (Seq ID No: 1322)

Homo sapiens ribosomal protein S5 (RPS5):

ttttcttcccagttaaaagtgttggcccgcggcgcgcggcctcttcctgtctgtaccagg gcggcgcgtggtctacgccgagtgacagagacgctcaggctgtgttctcaggatg (Seq ID No: 1323)

Homo sapiens ribosomal protein S6 (RPS6):
ggccctcttttccgtggcgcctcggaggcgttcagctgcttcaagatg (Seq ID No: 1324)

Homo sapiens ribosomal protein S7 (RPS7):
gggtctcttcctaagccggcgctcggcaagttctcccaggagaaagccatg (Seq ID No: 1325)

Homo sapiens ribosomal protein S8 (RPS8):
gtttctctttccagccagcgccgagcgatg (Seq ID No: 1326)

Homo sapiens ribosomal protein S9 (RPS9):

gcgcctctttctcagtgaccgggtggtttgcttaggcgcagacggggaagcggagccaac atg (Seq ID No: 1327)

Homo sapiens ribosomal protein S10 (RPS10): gctccttcctttccagccccggtaccggaccctgcagccgcagagatg (Seq ID No: 1328)

Homo sapiens ribosomal protein S11 (RPS11):
ctgcccctttcttttttcaggcggccgggaagatg (Seq ID No: 1329)

Homo sapiens ribosomal protein S12 (RPS12):

aggcctctttccctgccgccgccgagtcgcgcggaggcggaggcttgggtgcgttcaaga ttcaacttcacccgtaacccaccgccatg (Seq ID No: 1330)

Homo sapiens ribosomal protein S13 (RPS13):
cgctctcctttcgttgcctgatcgccgccatcatg (Seq ID No: 1331)

Homo sapiens ribosomal protein S15 (RPS15):
cgatctcttctgaggatccggcaagatg (Seq ID No: 1332)

Homo sapiens ribosomal protein S15a (RPS15A):

cgtcctctttccgccatctttccgcgccggtgagtagcactctctgagagctccaatttc atccgtctgccatcggcgccatcctgcaatctaagccacaatg (Seq ID No: 1333)

Homo sapiens ribosomal protein S16 (RPS16):

ctttccttttccggttgcggcgccgcgcggtgaggttgtctagtccacgctcggagccat g (Seq ID No: 1334)

Homo sapiens ribosomal protein S19 (RPS19):
cgttccctttcccctggctggcagcgcggaggccgcacgatg (Seq ID No: 1335)

Homo sapiens ribosomal protein S20 (RPS20):

ccacccctttctttttgaggaagacgcggtcgtaagggctgaggatttttggtccgcacg ctcctgctcctgactcaccgctgttcgctctcgccgaggaacaagtcggtcaggaagccc gcgcgcaacagccatg (Seq ID No: 1336)

Homo sapiens ribosomal protein S21 (RPS21):
gcttcctttctctctcgcgcgcggtgtggtggcagcaggcgcagcccagcctcgaaatg (Seq ID No: 1337)

Homo sapiens ribosomal protein S23 (RPS23):
gcttctctctttcgctcaggcccgtggcgccgacaggatg (Seq ID No: 1338)

Homo sapiens ribosomal protein S25 (RPS25):

gcttccttttgtccgacatcttgacgaggctgcggtgtctgctgctattctccgagctt cgcaatg (Seq ID No: 1339)

Homo sapiens ribosomal protein S26 (RPS26):
ccgtctcctctctccggtccgtgcctccaagatg (Seq ID No: 1340)

Homo sapiens ribosomal protein S27 (RPS27):
cgctcctttccggcggtgacgacctacgcacacgagaacatg (Seq ID No: 1341)

Homo sapiens ribosomal protein S28 (RPS28):
actcctctccgccagaccgccgccgcgccgccatcatg (Seq ID No: 1342)

Homo sapiens ribosomal protein S29 (RPS29):
gcttcttccttttacctcgttgcactgctgagagcaagatg (Seq ID No: 1343)

Homo sapiens ribosomal protein L15 (RPL15):
agctctttcctttccgtctggcggcagccatcaggtaagccaagatg (Seq ID No: 1344)

Homo sapiens ribosomal protein S2 (RPS2):
cgttcttcttttccgacaaaacaccaaatg (Seq ID No: 1345)

Homo sapiens ribosomal protein L14 (RPL14):
gggtcttcttccttctcgcctaacgccgccaacatg (Seq ID No: 1346)

Homo sapiens ribosomal protein S14 (RPS14):
ctctctttccggtgtggagtctggagacgacgtgcagaaatg (Seq ID No: 1347)

Homo sapiens ribosomal protein L10 (RPL10):

gcgcctctttcccttcggtgtgccactgaagatcctggtgtcgccatg (Seq ID No: 1348)

Homo sapiens ribosomal protein L10a (RPL10A):
tagtctcttttccggttagcgcggcgtgagaagccatg (Seq ID No: 1349)

Homo sapiens ribosomal protein L35 (RPL35):
tcctctttccctcggagcgggcggcggcgttggcggcttgtgcagcaatg (Seq ID No: 1350)

Homo sapiens ribosomal protein L13a (RPL13A):
cctcctccttttccaagcggctgccgaagatg (Seq ID No: 1351)

Homo sapiens ribosomal protein L36 (RPL36):
cagcccttccgccacggccgtctctggagagcagcagccatg (Seq ID No: 1352)

Homo sapiens ribosomal protein L36a (RPL36A):
gtttctttctttccgcgccgatagcgctcacgcaagcatg (Seq ID No: 1353)

Homo sapiens ribosomal protein L41 (RPL41):
tcgcc tttctctcggccttagcgccatttttttggaaacctctgcgccatg (Seq ID No: 1354)

Homo sapiens ribosomal protein S18 (RPS18):
cgctctctcttccacaggaggcctacacgccgccgcttgtgctgcagccatg (Seq ID No: 1355)

Homo sapiens ribosomal protein S24 (RPS24):
ggttctcttttcctccttggctgtctgaagatagatcgccatcatg (Seq ID No: 1356)

Homo sapiens ribosomal protein L8 (RPL8):
tttcctctttcggccgcgctggtgaacaggtaggtcatccttgcggccttgcggcatg (Seq ID No: 1357)

Homo sapiens ribosomal protein L34 (RPL34):
cttcctcttccggggacgttgtctgcaggtatg (Seq ID No: 1358)

Homo sapiens ribosomal protein S17 (RPS17):
gtttcctcttttaccaaggacccgccaacatg (Seq ID No: 1359)

Homo sapiens ribosomal protein SA (RPSA):

ctgtcttttccgtgctacctgcagaggggtccatacggcgttgttctggattcccgtcgt
aacttaaagggaaattttcacaatg (Seq ID No: 1360)

Homo sapiens eukaryotic translation initiation factor 3, sub unit C (EIF3C):

cttctctctcggcgtttccgctgtcagggccctgcggtgtgactcgcgggctcagctggt
ccggccgtagcacctccgcgccgtcgccatg (Seq ID No: 1361)

Homo sapiens poly(A) binding protein, cytoplasmic 1 (PABPC1) :

cgctctcctcctctcacggaaaggtcgcggcctgtggccctgcgggcagccgtgccgaga
tg (Seq ID No: 1362)

Homo sapiens tubulin, beta 1 class VI (TUBB1):

cactcccttccaaaagcatgacaggcagaaagcagagaagggccaggactggctgagggc ggggagctgggcctctggggtggacacacccttggtcacattgtgagggtagcttggttg gccagtcccaccactgcagtgaccacagttgtgttgggctcacaccagtgaaccgaagct ctggattctgagagtctgaggattccgtgaagatctcagacttgggctcagagcaaggat g (Seq ID No: 1363)

PpLuc(GC)-ag-A64

GGGAGAAAGCTTGAGG*ATGGAGGACGCCAAGAACATCAAGAAGGGCCCGGCGCCCTTCTA CCCGCTGGAGGACGGGACCGCCGGCGAGCAGCTCCACAAGGCCATGAAGCGGTACGCCCT GGTGCCGGGCACGATCGCCTTCACCGACGCCCACATCGAGGTCGACATCACCTACGCGGA GTACTTCGAGATGAGCGTGCGCCTGGCCGAGGCCATGAAGCGGTACGGCCTGAACACCAA CCACCGGATCGTGGTGTGCTCGGAGAACAGCCTGCAGTTCTTCATGCCGGTGCTGGGCGC CCTCTTCATCGGCGTGGCCGTCGCCCCGGCGAACGACATCTACAACGAGCGGGAGCTGCT GAACAGCATGGGGATCAGCCAGCCGACCGTGGTGTTCGTGAGCAAGAAGGGCCTGCAGAA GATCCTGAACGTGCAGAAGAAGCTGCCCATCATCCAGAAGATCATCATCATGGACAGCAA GACCGACTACCAGGGCTTCCAGTCGATGTACACGTTCGTGACCAGCCACCTCCCGCCGGG CTTCAACGAGTACGACTTCGTCCCGGAGAGCTTCGACCGGGACAAGACCATCGCCCTGAT CATGAACAGCAGCGGCAGCACCGGCCTGCCGAAGGGGGTGGCCCTGCCGCACCGGACCGC CTGCGTGCGCTTCTCGCACGCCCGGGACCCCATCTTCGGCAACCAGATCATCCCGGACAC CGCCATCCTGAGCGTGGTGCCGTTCCACCACGGCTTCGGCATGTTCACGACCCTGGGCTA CCTCATCTGCGGCTTCCGGGTGGTCCTGATGTACCGGTTCGAGGAGGAGCTGTTCCTGCG GAGCCTGCAGGACTACAAGATCCAGAGCGCGCTGCTCGTGCCGACCCTGTTCAGCTTCTT CGCCAAGAGCACCCTGATCGACAAGTACGACCTGTCGAACCTGCACGAGATCGCCAGCGG GGGCGCCCCGCTGAGCAAGGAGGTGGGCGAGGCCGTGGCCAAGCGGTTCCACCTCCCGGG CATCCGCCAGGGCTACGGCCTGACCGAGACCACGAGCGCGATCCTGATCACCCCCGAGGG GGACGACAAGCCGGGCGCCGTGGGCAAGGTGGTCCCGTTCTTCGAGGCCAAGGTGGTGGA CCTGGACACCGGCAAGACCCTGGGCGTGAACCAGCGGGGCGAGCTGTGCGTGCGGGGGCC GATGATCATGAGCGGCTACGTGAACAACCCGGAGGCCACCAACGCCCTCATCGACAAGGA CGGCTGGCTGCACAGCGGCGACATCGCCTACTGGGACGAGGACGAGCACTTCTTCATCGT*

*CGACCGGCTGAAGTCGCTGATCAAGTACAAGGGCTACCAGGTGGCGCCGGCCGAGCTGGA GAGCATCCTGCTCCAGCACCCCAACATCTTCGACGCCGGCGTGGCCGGGCTGCCGGACGA CGACGCCGGCGAGCTGCCGGCCGCGGTGGTGGTGCTGGAGCACGGCAAGACCATGACGGA GAAGGAGATCGTCGACTACGTGGCCAGCCAGGTGACCACCGCCAAGAAGCTGCGGGGCGG CGTGGTGTTCGTGGACGAGGTCCCGAAGGGCCTGACCGGGAAGCTCGACGCCCGGAAGAT CCGCGAGATCCTGATCAAGGCCAAGAAGGGCGGCAAGATCGCCGTGTAA*GACTAGTTATA AGACTGACTAGCCCGATGGGCCTCCCAACGGGCCCTCCTCCCCTCCTTGCACCGAGATTA ATAGATCTAAAAAAAAAAAAAAAAAAAAAAAAAAAAAAAAAAAAAAAAAAAAAAAAAAAA AAAAAAAAAAAA (SEQ ID No: 1364)

RPL32-PpLuc(GC)-ag-A64-C30-histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGCCCTCCTCCCCTCCTTGCACCGAGATTAATAGATCTAAAAAAAAAAAAAAA
AAAAAAAAAAAAAAAAAAAAAAAAAAAAAAAAAAAAAAAAAAAAAAAAAATGCATCCCCC
CCCCCCCCCCCCCCCCCCCCCCCCCCAAAGGCTCTTTTCAGAGCCACCAGAATT (SEQ ID No: 1365)

fragment of the 5'UTR of human ribosomal protein Large 32 ACGGAGGTGGCAGCCATCTCCTTCTCGGCATC (SEQ ID No: 1366)

fragment of the 5'UTR of human ribosomal protein Large 32 GGCGCTGCCTACGGAGGTGGCAGCCATCTCCT (SEQ ID No: 1367)

5'UTR of human ribosomal protein Large 32 lacking the 5' terminal oligopyrimidine tract GGCGCTGCCTACGGAGGTGGCAGCCATCTCCTTCTCGGCATC (SEQ ID No. 1368)

Human albumin 3'UTR

CATCACATTT AAAAGCATCT CAGCCTACCA TGAGAATAAG AGAAAGAAAA
TGAAGATCAA AAGCTTATTC ATCTGTTTTT CTTTTTCGTT GGTGTAAAGC
CAACACCCTG TCTAAAAAAC ATAAATTTCT TTAATCATTT TGCCTCTTTT
CTCTGTGCTT CAATTAATAA AAAATGGAAA GAATCT (SEQ ID No: 1369)

3'UTR of Homo sapiens hemoglobin, alpha 1 (HBA1)

gctggagcctcggtggccatgcttcttgccccttgggcctccccccagcccctcctcccc ttcctgcacccgtacccccgtggtctttgaataaagtctgagtgggcggc (SEQ ID No: 1370)

3'UTR of Homo sapiens hemoglobin, alpha 2 (HBA2)

gctggagcctcggtagccgttcctcctgcccgctgggcctcccaacgggccctcctcccctccttg caccggcccttcctggtctttgaataaagtctgagtgggcag (SEQ ID No: 1371)

3'UTR of Homo sapiens hemoglobin, beta (HBB)

Gctcgctttcttgctgtccaatttctattaaaggttcctttgttccctaagtccaactactaaact gggggatattatgaagggccttgagcatctggattctgcctaataaaaaacatttattttcattgc (SEQ ID No: 1372)

3'UTR of Homo sapiens tyrosine hydroxylase (TH)

gtgcacggcgtccctgagggcccttcccaacctcccctggtcctgcactgtcccggagct caggccctggtgaggggctgggtcccgggtgccccccatgccctccctgctgccaggctc ccactgcccctgcacctgcttctcagcgcaacagctgtgtgtgcccgtggtgaggttgtg ctgcctgtggtgaggtcctgtcctggctcccagggtcctgggggctgctgcactgccctc cgcccttccctgacactgtctgctgccccaatcaccgtcacaataaaagaaactgtggtc tcta (SEQ ID No: 1373)

3'UTR of Homo sapiens arachidonate 15-lipoxygenase (ALOX15)

gcgtcgccaccctttggttatttcagcccccatcacccaagccacaagctgaccccttcg tggttatagccctgccctcccaagtcccaccctcttcccatgtcccaccctccctagagg ggcaccttttcatggtctctgcacccagtgaacacattttactctagaggcatcacctgg gaccttactcctctttccttccttcctcctttcctatcttccttcctctctctcttcctc tttcttcattcagatctatatggcaaatagccacaattatataaatcatttcaagactag aatagggggatataatacatattactccacacctttatgaatcaaatatgatttttttg ttgttgttaagacagagtctcactttgacacccaggctggagtgcagtggtgccatcacc acggctcactgcagcctcagcgtcctgggctcaaatgatcctcccacctcagcctcctga gtagctgggactacaggctcatgccatcatgcccagctaatatttttttattttcgtgga gacggggcctcactatgttgcctaggctggaaataggatttgaacccaaattgagttta acaataataaaaagttgttttacgctaaagatggaaaagaactaggactgaactattta aataaaatattggc (SEQ ID No: 1374)

3'UTR of Homo sapiens collagen, type I, alpha 1 (COL1A1)

actccctccatcccaacctggctccctcccacccaaccaactttccccccaacccggaaa cagacaagcaacccaaactgaaccccctcaaaagccaaaaaatgggagacaatttcacat

ggactttggaaaatatttttttcctttgcattcatctctcaaacttagttttatctttg
accaaccgaacatgaccaaaaaccaaaagtgcattcaaccttaccaaaaaaaaaaaaaaa
aaaagaataaataaataacttttaaaaaaggaagcttggtccacttgcttgaagaccca
tgcgggggtaagtccctttctgcccgttgggcttatgaaaccccaatgctgccctttctg
ctcctttctccacaccccccttggggcctcccctccactccttcccaaatctgtctcccc
agaagacacaggaaacaatgtattgtctgcccagcaatcaaaggcaatgctcaaacaccc
aagtggcccccaccctcagcccgctcctgcccgcccagcacccccaggccctgggggacc
tggggttctcagactgccaaagaagccttgccatctggcgctcccatggctcttgcaaca
tctccccttcgtttttgagggggtcatgccgggggagccaccagcccctcactgggttcg
gaggagagtcaggaagggccacgacaaagcagaaacatcggatttggggaacgcgtgtca
atcccttgtgccgcagggctgggcgggagagactgttctgttccttgtgtaactgtgttg
ctgaaagactacctcgttcttgtcttgatgtgtcaccggggcaactgcctgggggcgggg
atgggggcagggtggaagcggctccccattttataccaaaggtgctacatctatgtgatg
ggtggggtggggagggaatcactggtgctatagaaattgagatgcccccccaggccagca
aatgttccttttttgttcaaagtctatttttattccttgatatttttctttttttttttt
ttttttgtggatggggacttgtgaatttttctaaaggtgctatttaacatgggaggagag
cgtgtgcggctccagcccagcccgctgctcactttccaccctctctccacctgcctctgg
cttctcaggcctctgctctccgacctctctcctctgaaaccctcctccacagctgcagcc
catcctcccggctccctcctagtctgtcctgcgtcctctgtccccgggtttcagagacaa
cttcccaaagcacaaagcagtttttccccctaggggtgggaggaagcaaaagactctgta
cctattttgtatgtgtataataatttgagatgtttttaattattttgattgctggaataa
agcatgtggaaatgacccaaacataa (SEQ ID No: 1375)

albumin7 3'UTR

CATCACATTTAAAAGCATCTCAGCCTACCATGAGAATAAGAGAAAGAAAATGAAGATCAA
TAGCTTATTCATCTCTTTTTCTTTTTCGTTGGTGTAAAGCCAACACCCTGTCTAAAAAAC
ATAAATTTCTTTAATCATTTTGCCTCTTTTCTCTGTGCTTCAATTAATAAAAAATGGAAA
GAACCT (SEQ ID No: 1376)

Human albumin 3'UTR + poly(A) sequence

CATCACATTT AAAAGCATCT CAGCCTACCA TGAGAATAAG AGAAAGAAAA
TGAAGATCAA AAGCTTATTC ATCTGTTTTT CTTTTTCGTT GGTGTAAAGC
CAACACCCTG TCTAAAAAAC ATAAATTTCT TTAATCATTT TGCCTCTTTT
CTCTGTGCTT CAATTAATAA AAAATGGAAA GAATCTAGAT CTAAAAAAAA
AAAAAAAAAA AAAAAAAAAA AAAAAAAAAA AAAAAAAAAA AAAAAAAAAA
AAAAAA (SEQ ID No: 1377)

Human albumin 3'UTR fragment 1

AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG
TCTAAAAAAC ATAAATTTCT TTAATCATTT TGCCTCTTTT CTCTGTGCTT CAATT
(SEQ ID No: 1378)

Human albumin 3'UTR fragment 2

CATCACATTT AAAAGCATCT CAGCCTACCA TGAGAATAAG AGAAAGAAAA
TGAAGATCAA AAGCTTATTC ATCTGTTTTT CTTTTTCGTT GGTGTAAAGC
CAACACCCTG (SEQ ID No: 1379)

Human albumin 3'UTR fragment 3

AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG
TCTAAAAAAC (SEQ ID No: 1380)

Human albumin 3'UTR fragment 4

CAGCCTACCA TGAGAATAAG AGAAAGAAAA TGAAGATCAA AAGCTTATTC
ATCTGTTTTT CTTTTTCGTT GGTGTAAAGC CAACACCCTG TCTAAAAAAC
ATAAATTTCT (SEQ ID No: 1381)

Human albumin 3'UTR fragment 5

TGAGAATAAG AGAAAGAAAA TGAAGATCAA AAGCTTATTC ATCTGTTTTT
CTTTTTCGTT GGTGTAAAGC CAACACCCTG TCTAAAAAAC ATAAATTTCT
TTAATCATTT (SEQ ID No: 1382)

Human albumin 3'UTR fragment 6

AGAAAGAAAA TGAAGATCAA AAGCTTATTC ATCTGTTTTT CTTTTTCGTT
GGTGTAAAGC CAACACCCTG TCTAAAAAAC ATAAATTTCT TTAATCATTT
TGCCTCTTTT (SEQ ID No: 1383)

Human albumin 3'UTR fragment 7

TGAAGATCAA AAGCTTATTC ATCTGTTTTT CTTTTTCGTT GGTGTAAAGC
CAACACCCTG TCTAAAAAAC ATAAATTTCT TTAATCATTT TGCCTCTTTT
CTCTGTGCTT (SEQ ID No: 1384)

Human albumin 3'UTR fragment 8

AAGCTTATTC ATCTGTTTTT CTTTTTCGTT GGTGTAAAGC CAACACCCTG
TCTAAAAAAC ATAAATTTCT TTAATCATTT TGCCTCTTTT CTCTGTGCTT
CAATTAATAA (SEQ ID No: 1385)

Human albumin 3'UTR fragment 9

ATCTGTTTTT CTTTTTCGTT GGTGTAAAGC CAACACCCTG TCTAAAAAAC
ATAAATTTCT TTAATCATTT TGCCTCTTTT CTCTGTGCTT CAATTAATAA
AAAATGGAAA (SEQ ID No: 1386)

Human albumin 3'UTR fragment 10

CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No: 1387)

Human albumin 3'UTR fragment 11

TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No: 1388)

Human albumin 3'UTR fragment 12

CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No: 1389)

Human albumin 3'UTR fragment 13

AAGCTTATTC ATCTGTTTTT CTTTTTCGTT GGTGTAAAGC CAACACCCTG TCTAAAAAAC (SEQ ID No: 1390)

Sequence according to formula (Ic)
NGNNNNNNUNNNNNNCN (SEQ ID NO: 1391)

Sequence according to formula (IIc):
N*N*NNNNGNNNNNNUNNNNNNCNNNN*N*N* (SEQ ID NO: 1392)

Sequence according to formula (Id):
NCNNNNNNUNNNNNNGN (SEQ ID NO: 1393)

Sequence according to formula (IId)
N*N*NNNNCNNNNNNUNNNNNNGNNNN*N*N* (SEQ ID NO: 1394)

Sequence according to formula (Ie)
DGNNNNNNUNNNNNNCH (SEQ ID NO: 1395)

Sequence according to formula (IIe)
N*N*NNNDGNNNNNNUNNNNNNCHNNN*N*N* (SEQ ID NO: 1396)

Sequence according to formula (If)
NGNBYYNNUNVNDNCN (SEQ ID NO: 1397)

Sequence according to formula (IIf)
N*N*NNNNGNBYYNNUNVNDNCNNNN*N*N* (SEQ ID NO: 1398)

Sequence according to formula (Ig)
NGHYYYDNUHABRDCN (SEQ ID NO: 1399)

Sequence according to formula (IIg)
N*N*HNNNGHYYYDNUHABRDCNNNN*N*H* (SEQ ID NO: 1400)

Sequence according to formula (Ih)
DGHYCUDYUHASRRCC (SEQ ID NO: 1401)

Sequence according to formula (IIh)
N*H*AAHDGHYCUDYUHASRRCCVHB*N*H* (SEQ ID NO: 1402)

Sequence according to formula (Ic)
VGYYYYHHTHRVVRCB (SEQ ID NO: 1403)

Sequence according to formula (Ic)
SGYYYTTYTMARRRCS (SEQ ID NO: 1404)

Sequence according to formula (Ic)
SGYYCTTTTMAGRRCS (SEQ ID NO: 1405)

Sequence according to formula (Ie)
DGNNNBNNTHVNNNCH (SEQ ID NO: 1406)

Sequence according to formula (Ie)
RGNNNYHBTHRDNNCY (SEQ ID NO: 1407)

Sequence according to formula (Ie)
RGNDBYHYTHRDHNCY (SEQ ID NO: 1408)

Sequence according to formula (If)
VGYYYTYHTHRVRRCB (SEQ ID NO: 1409)

Sequence according to formula (If)
SGYYCTTYTMAGRRCS (SEQ ID NO: 1410)

Sequence according to formula (If)
SGYYCTTTTMAGRRCS (SEQ ID NO: 1411)

Sequence according to formula (Ig)
GGYYCTTYTHAGRRCC (SEQ ID NO: 1412)

Sequence according to formula (Ig)
GGCYCTTYTMAGRGCC (SEQ ID NO: 1413)

Sequence according to formula (Ig)
GGCTCTTTTMAGRGCC (SEQ ID NO: 1414)

Sequence according to formula (Ih)
DGHYCTDYTHASRRCC (SEQ ID NO: 1415)

Sequence according to formula (Ih)
GGCYCTTTTHAGRGCC (SEQ ID NO: 1416)

Sequence according to formula (Ih)
GGCYCTTTTMAGRGCC (SEQ ID NO: 1417)

Sequence according to formula (IIc)
H*H*HHVVGYYYYHHTHRVVRCBVHH*N*N* (SEQ ID NO: 1418)

Sequence according to formula (IIc)
M*H*MHMSGYYYTTYTMARRRCSMCH*H*H* (SEQ ID NO: 1419)

Sequence according to formula (IIc)
M*M*MMMSGYYCTTTTMAGRRCSACH*M*H* (SEQ ID NO: 1420)

Sequence according to formula (IIe)

N*N*NNNDGNNNBNNTHVNNNCHNHN*N*N* (SEQ ID NO: 1421)

Sequence according to formula (IIe)
N*N*HHNRGNNNYHBTHRDNNCYDHH*N*N* (SEQ ID NO: 1422)

Sequence according to formula (IIe)
N*H*HHVRGNDBYHYTHRDHNCYRHH*H*H* (SEQ ID NO: 1423)

Sequence according to formula (IIf)
H*H*MHMVGYYYTYHTHRVRRCBVMH*H*N* (SEQ ID NO: 1424)

Sequence according to formula (IIf)
M*M*MMMSGYYCTTYTMAGRRCSMCH*H*H* (SEQ ID NO: 1425)

Sequence according to formula (IIf)
M*M*MMMSGYYCTTTTMAGRRCSACH*M*H* (SEQ ID NO: 1426)

Sequence according to formula (IIg)
H*H*MAMGGYYCTTYTHAGRRCCVHN*N*M* (SEQ ID NO: 1427)

Sequence according to formula (IIg)
H*H*AAMGGCYCTTYTMAGRGCCVCH*H*M* (SEQ ID NO: 1428)

Sequence according to formula (IIg)
M*M*AAMGGCTCTTTTMAGRGCCMCY*M*M* (SEQ ID NO: 1429)

Sequence according to formula (IIh)
N*H*AAHDGHYCTDYTHASRRCCVHB*N*H* (SEQ ID NO: 1430)

Sequence according to formula (IIh)
H*H*AAMGGCYCTTTTHAGRGCCVMY*N*M* (SEQ ID NO: 1431)

Sequence according to formula (IIh)
H*M*AAAGGCYCTTTTMAGRGCCRMY*H*M* (SEQ ID NO: 1432)

Specific histone stem-loop sequence
CAAAGGCTCTTTTCAGAGCCACCA (SEQ ID NO: 1433)

Center, α-complex-binding portion of the 3'UTR of an α-globin gene
GCCCGATGGGCCTCCCAACGGGCCCTCCTCCCCTCCTTGCACCG (SEQ ID NO: 1434)

ATP synthase lipid-binding protein, mitochondrial (atp5g2)

tagttt ctcctctcga acgccaggtg gagcaaccgg ccggataccg ccacagccct
ggcaggcggc gctgtgatg (SEQ ID NO: 1435)

RPL35 - PpLuc(GC) - albumin? - A64 - C30 - histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT

CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCCAAAGGCTCTTTTCAGAGCCACCAGAATT (SEQ ID NO: 1436)

RPL21 - PpLuc(GC) - albumin? - A64 - C30 - histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT

GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCCAAAGGCTCTTTTCAGAGCCACCAGAATT (SEQ ID NO: 1437)

ATP5A1 - PpLuc(GC) - albumin? - A64 - C30 - histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTGCATCACATTTAAAAGCATCTCAGCCTACCATGAGAATAAGAGAAAGAAAATG
AAGATCAATAGCTTATTCATCTCTTTTTCTTTTTCGTTGGTGTAAAGCCAACACCCTGTC
TAAAAAACATAAATTTCTTTAATCATTTTGCCTCTTTTCTCTGTGCTTCAATTAATAAAA
AATGGAAAGAACCTAGATCTAAAAAAAAAAAAAAAAAAAAAAAAAAAAAAAAAAAAAAAA
AAAAAAAAAAAAAAAAAAAAAAAATGCATCCCCCCCCCCCCCCCCCCCCCCCCCCCCCCC
AAAGGCTCTTTTCAGAGCCACCAGAATT (SEQ ID NO: 1438)

HSD17B4 - PpLuc(GC) - albumin? - A64 - C30 - histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG

TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTGCATCA
CATTTAAAAGCATCTCAGCCTACCATGAGAATAAGAGAAAGAAAATGAAGATCAATAGCT
TATTCATCTCTTTTTCTTTTTCGTTGGTGTAAAGCCAACACCCTGTCTAAAAAACATAAA
TTTCTTTAATCATTTTGCCTCTTTTCTCTGTGCTTCAATTAATAAAAAATGGAAAGAACC
TAGATCTAAAAAAAAAAAAAAAAAAAAAAAAAAAAAAAAAAAAAAAAAAAAAAAAAAAAA
AAAAAAAAAAATGCATCCCCCCCCCCCCCCCCCCCCCCCCCCCCCCAAAGGCTCTTTTC
AGAGCCACCAGAATT (SEQ ID NO: 1439)

AIG1 - PpLuc(GC) - albumin7 - A64 - C30 - histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC

GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTGCATCACATTTAAAAGCATCTCAGCCTACCATGAGAA
TAAGAGAAAGAAAATGAAGATCAATAGCTTATTCATCTCTTTTTCTTTTTCGTTGGTGTA
AAGCCAACACCCTGTCTAAAAAACATAAATTTCTTTAATCATTTTGCCTCTTTTCTCTGT
GCTTCAATTAATAAAAAATGGAAAGAACCTAGATCTAAAAAAAAAAAAAAAAAAAAAAAA
AAAAAAAAAAAAAAAAAAAAAAAAAAAAAAAAAAAAAAAAATGCATCCCCCCCCCCCCCC
CCCCCCCCCCCCCCCCAAAGGCTCTTTTCAGAGCCACCAGAATT (SEQ ID NO: 1440)

COX6C - PpLuc(GC) - albumin? - A64 - C30 - histoneSL

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTGCATCACATTTA
AAAGCATCTCAGCCTACCATGAGAATAAGAGAAAGAAAATGAAGATCAATAGCTTATTCA
TCTCTTTTTCTTTTTCGTTGGTGTAAAGCCAACACCCTGTCTAAAAAACATAAATTTCTT
TAATCATTTTGCCTCTTTTCTCTGTGCTTCAATTAATAAAAAATGGAAAGAACCTAGATC
TAAAAAAAAAAAAAAAAAAAAAAAAAAAAAAAAAAAAAAAAAAAAAAAAAAAAAAAAAAA
AAAAATGCATCCCCCCCCCCCCCCCCCCCCCCCCCCCCCCAAAGGCTCTTTTCAGAGCC

ACCAGAATT (SEQ ID NO: 1441)

ASAH1 - PpLuc(GC) - albumin? - A64 - C30 - histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCAAAGGCTCTTTTCAGAGCCACCAGAATT (SEQ ID NO: 1442)

mRPL21 - PpLuc(GC) - albumin? - A64 - C30 - histoneSL

GGGGCCGCCGCAGCCATCTTCCAGTAACTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC

GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCCAAAGGCTCTTTTCAGAGCCACCAGAATT (SEQ ID NO: 1443)

mRPL35A - PpLuc(GC) - albumin? - A64 - C30 - histoneSL

GGGCCATCTTGGCGCCTGTGGAGGCCTGCTGGGAACAGGACTTCTAACAGCAAGTAAGCT
TGAGGATGGAGGACGCCAAGAACATCAAGAAGGGCCCGGCGCCCTTCTACCCGCTGGAGG
ACGGGACCGCCGGCGAGCAGCTCCACAAGGCCATGAAGCGGTACGCCCTGGTGCCGGGCA
CGATCGCCTTCACCGACGCCCACATCGAGGTCGACATCACCTACGCGGAGTACTTCGAGA
TGAGCGTGCGCCTGGCCGAGGCCATGAAGCGGTACGGCCTGAACACCAACCACCGGATCG
TGGTGTGCTCGGAGAACAGCCTGCAGTTCTTCATGCCGGTGCTGGGCGCCCTCTTCATCG
GCGTGGCCGTCGCCCCGGCGAACGACATCTACAACGAGCGGGAGCTGCTGAACAGCATGG
GGATCAGCCAGCCGACCGTGGTGTTCGTGAGCAAGAAGGGCCTGCAGAAGATCCTGAACG
TGCAGAAGAAGCTGCCCATCATCCAGAAGATCATCATCATGGACAGCAAGACCGACTACC
AGGGCTTCCAGTCGATGTACACGTTCGTGACCAGCCACCTCCCGCCGGGCTTCAACGAGT
ACGACTTCGTCCCGGAGAGCTTCGACCGGGACAAGACCATCGCCCTGATCATGAACAGCA
GCGGCAGCACCGGCCTGCCGAAGGGGGTGGCCCTGCCGCACCGGACCGCCTGCGTGCGCT
TCTCGCACGCCCGGGACCCCATCTTCGGCAACCAGATCATCCCGGACACCGCCATCCTGA
GCGTGGTGCCGTTCCACCACGGCTTCGGCATGTTCACGACCCTGGGCTACCTCATCTGCG
GCTTCCGGGTGGTCCTGATGTACCGGTTCGAGGAGGAGCTGTTCCTGCGGAGCCTGCAGG
ACTACAAGATCCAGAGCGCGCTGCTCGTGCCGACCCTGTTCAGCTTCTTCGCCAAGAGCA
CCCTGATCGACAAGTACGACCTGTCGAACCTGCACGAGATCGCCAGCGGGGGCGCCCCGC
TGAGCAAGGAGGTGGGCGAGGCCGTGGCCAAGCGGTTCCACCTCCCGGGCATCCGCCAGG
GCTACGGCCTGACCGAGACCACGAGCGCGATCCTGATCACCCCCGAGGGGGACGACAAGC
CGGGCGCCGTGGGCAAGGTGGTCCCGTTCTTCGAGGCCAAGGTGGTGGACCTGGACACCG
GCAAGACCCTGGGCGTGAACCAGCGGGGCGAGCTGTGCGTGCGGGGGCCGATGATCATGA
GCGGCTACGTGAACAACCCGGAGGCCACCAACGCCCTCATCGACAAGGACGGCTGGCTGC
ACAGCGGCGACATCGCCTACTGGGACGAGGACGAGCACTTCTTCATCGTCGACCGGCTGA
AGTCGCTGATCAAGTACAAGGGCTACCAGGTGGCGCCGGCCGAGCTGGAGAGCATCCTGC
TCCAGCACCCCAACATCTTCGACGCCGGCGTGGCCGGGCTGCCGGACGACGACGCCGGCG
AGCTGCCGGCCGCGGTGGTGGTGCTGGAGCACGGCAAGACCATGACGGAGAAGGAGATCG
TCGACTACGTGGCCAGCCAGGTGACCACCGCCAAGAAGCTGCGGGGCGGCGTGGTGTTCG

TGGACGAGGTCCCGAAGGGCCTGACCGGGAAGCTCGACGCCCGGAAGATCCGCGAGATCC
TGATCAAGGCCAAGAAGGGCGGCAAGATCGCCGTGTAAGACTAGTGCATCACATTTAAAA
GCATCTCAGCCTACCATGAGAATAAGAGAAAGAAAATGAAGATCAATAGCTTATTCATCT
CTTTTTCTTTTTCGTTGGTGTAAAGCCAACACCCTGTCTAAAAAACATAAATTTCTTTAA
TCATTTTGCCTCTTTTCTCTGTGCTTCAATTAATAAAAAATGGAAAGAACCTAGATCTAA
AAAAAAAAAAAAAAAAAAAAAAAAAAAAAAAAAAAAAAAAAAAAAAAAAAAAAAAAAAAA
AATGCATCCCCCCCCCCCCCCCCCCCCCCCCCCCCCCCAAAGGCTCTTTTCAGAGCCACC
AGAATT (SEQ ID NO: 1444)

RPL35 - PpLuc(GC) - A64 - C30 - histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTAGATCTAAAAAAAAAAAAAAAAAAAAAAAAAAAA
AAAAAAAAAAAAAAAAAAAAAAAAAAAAAAAAAAAAATGCATCCCCCCCCCCCCCCCCCC
CCCCCCCCCCCCAAAGGCTCTTTTCAGAGCCACCAGAATT (SEQ ID NO: 1445)

RPL21 - PpLuc(GC) - A64 - C30 - histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT

CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTAGATCTAAAAAAAAAAAAAAAAAAAAAAAAAAAA
AAAAAAAAAAAAAAAAAAAAAAAAAAAAAAAAAAAAATGCATCCCCCCCCCCCCCCCCCC
CCCCCCCCCCCCAAAGGCTCTTTTCAGAGCCACCAGAATT (SEQ ID NO: 1446)

ATP5A1 - PpLuc(GC) - A64 - C30 - histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG

CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTAGATCTAAAAAAAAAAAAAAAAAAAAAAAAAAAAAAAAAAAAAAAAAAAAAAA
AAAAAAAAAAAAAAAAATGCATCCCCCCCCCCCCCCCCCCCCCCCCCCCCCCAAAGGCT
CTTTTCAGAGCCACCAGAATT (SEQ ID NO: 1447)

HSD17B4 - PpLuc(GC) - A64 - C30 - histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTAGATCT
AAAAAAAAAAAAAAAAAAAAAAAAAAAAAAAAAAAAAAAAAAAAAAAAAAAAAAAAAAAA
AAAATGCATCCCCCCCCCCCCCCCCCCCCCCCCCCCCCCAAAGGCTCTTTTCAGAGCCA
CCAGAATT (SEQ ID NO: 1448)

AIG1 - PpLuc(GC) - A64 - C30 - histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT

CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTAGATCTAAAAAAAAAAAAAAAAAAAAAAAAAAAAAAAA
AAAAAAAAAAAAAAAAAAAAAAAAAAAAAAAAAATGCATCCCCCCCCCCCCCCCCCCCCC
CCCCCCCCCAAAGGCTCTTTTCAGAGCCACCAGAATT (SEQ ID NO: 1449)

COX6C - PpLuc(GC) - A64 - C30 - histoneSL

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTAGATCTAAAAAA

AAAAAAAAAAAAAAAAAAAAAAAAAAAAAAAAAAAAAAAAAAAAAAAAAAAAAAAAAAAATG
CATCCCCCCCCCCCCCCCCCCCCCCCCCCCCCCAAAGGCTCTTTTCAGAGCCACCAGAA
TT (SEQ ID NO: 1450)

ASAH1 - PpLuc(GC) - A64 - C30 - histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTAGATCTAAAAAAAAAAAAAAAAAAAA
AAAAAAAAAAAAAAAAAAAAAAAAAAAAAAAAAAAAAAAAAAAAATGCATCCCCCCCCCCC
CCCCCCCCCCCCCCCCCCCCAAAGGCTCTTTTCAGAGCCACCAGAATT (SEQ ID NO: 1451)

5'UTR of human ribosomal protein Large 35 (RPL35) lacking the 5' terminal oligopyrimidine tract
GGAGCGGGCGGCGGCGTTGGCGGCTTGTGCAGCA (SEQ ID NO: 1452)

5'UTR of human ribosomal protein Large 21 (RPL21) lacking the 5' terminal oligopyrimidine tract
GGCCGGAACCGCCATCTTCCAGTAATTCGCCAAA (SEQ ID NO: 1453)

5'UTR of human ATP synthase, H+ transporting, mitochondrial F1 complex, alpha subunit 1, cardiac muscle (ATP5A1) lacking the 5' terminal oligopyrimidine tract

GCGGCTCGGCCATTTTGTCCCAGTCAGTCCGGAGGCTGCGGCTGCAGAAGTACCGCCTGCGGAGTA
ACTGCAAAG (SEQ ID NO: 1454)

5'UTR of human hydroxysteroid (17-beta) dehydrogenase 4 (HSD17B4) lacking the 5' terminal oligopyrimidine tract
GTCCCGCAGTCGGCGTCCAGCGGCTCTGCTTGTTCGTGTGTGTGTCGTTGCAGGCCTTATTC (SEQ ID NO: 1455)

5'UTR of human androgen-induced 1 (AIG1) lacking the 5' terminal oligopyrimidine tract
GCCGCCCAGCCGGTCCAGGCCTCTGGCGAAC (SEQ ID NO: 1456)

5'UTR of human cytochrome c oxidase subunit VIc (COX6C) lacking the 5' terminal oligopyrimidine tract
AGTCAGGAAGGACGTTGGTGTTGAGGTTAGCATACGTATCAAGGACAGTAACTACC (SEQ ID NO: 1457)

5'UTR of human N-acylsphingosine amidohydrolase (acid ceramidase) 1 (ASAH1) lacking the 5' terminal oligopyrimidine tract
GCCTCTGCTGGAGTCCGGGGAGTGGCGTTGGCTGCTAGAGCG (SEQ ID NO: 1458)

5'UTR of mouse ribosomal protein Large 21 (mRPL21) lacking the 5' terminal oligopyrimidine tract
GGCCGCCGCAGCCATCTTCCAGTAACTCGCCAAA (SEQ ID NO: 1459)

5'UTR of mouse ribosomal protein large 35A (mRPL35A) lacking the 5' terminal oligopyrimidine tract
GCCATCTTGGCGCCTGTGGAGGCCTGCTGGGAACAGGACTTCTAACAGCAAGT (SEQ ID NO: 1460)

Mouse ribosomal protein Large 21 (mRPL21)

TCCTCCTTTCGGCCGCCGCAGCCATCTTCCAGTAACTCGCCAAAATGCCATCTTCCAGTAACTCGC
CAAAATG (SEQ ID NO: 1461)

mouse ribosomal protein large 35A (mRPL35A)

CTTCCTCTTTCCGCCATCTTGGCGCCTGTGGAGGCCTGCTGGGAACAGGACTTCTAACAGCAAGTA
TG (SEQ ID NO: 1462)

RPL32 - PpLuc(GC) - ag - A64

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG

GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAGATCTAAAAAAAAAAAAAA
AAAAAAAAAAAAAAAAAAAAAAAAAAAAAAAAAAAAAAAAAAAAAAAAAA (SEQ ID NO: 1463)

PpLuc(GC) - ag - A64 - histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAAAATGCATCAAAGGCTCTT
TTCAGAGCCACCA (SEQ ID NO: 1464)

PpLuc(GC) - albumin? - A64 - C30 - histoneSL

GGGGCGCTGCCTACGGAGGTGGCAGCCATCTCCTTCTCGGCATCAAGCTTGAGGATGGAG

GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT (SEQ ID NO: 1465)

RPL35 - PpLuc(GC) - ag - A64

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA

CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAA (SEQ ID NO: 1466)

RPL21 - PpLuc(GC) - ag - A64

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAA (SEQ ID NO: 1467)

atp5a1 - PpLuc(GC) - ag - A64

GGGCGGCTCGGCCATTTTGTCCCAGTCAGTCCGGAGGCTGCGGCTGCAGAAGTACCGCCT

GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTTATAAGACTGACTAGCCCGATGGGCCTCCCAACGGGCCCTCCTCCCCTCCTTG
CACCGAGATTAATAAAAAAAAAAAAAAAAAAAAAAAAAAAAAAAAAAAAAAAAAAAAAAA
AAAAAAAAAAAAAAAA (SEQ ID NO: 1468)

HSD17B4 - PpLuc(GC) - ag - A64

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA

TCCGCCAGGGCTACGGCCTGACCGAGACCACGAGCGCGATCCTGATCACCCCCGAGGGGG ACGACAAGCCGGGCGCCGTGGGCAAGGTGGTCCCGTTCTTCGAGGCCAAGGTGGTGGACC TGGACACCGGCAAGACCCTGGGCGTGAACCAGCGGGGCGAGCTGTGCGTGCGGGGGCCGA TGATCATGAGCGGCTACGTGAACAACCCGGAGGCCACCAACGCCCTCATCGACAAGGACG GCTGGCTGCACAGCGGCGACATCGCCTACTGGGACGAGGACGAGCACTTCTTCATCGTCG ACCGGCTGAAGTCGCTGATCAAGTACAAGGGCTACCAGGTGGCGCCGGCCGAGCTGGAGA GCATCCTGCTCCAGCACCCCAACATCTTCGACGCCGGCGTGGCCGGGCTGCCGGACGACG ACGCCGGCGAGCTGCCGGCCGCGGTGGTGGTGCTGGAGCACGGCAAGACCATGACGGAGA AGGAGATCGTCGACTACGTGGCCAGCCAGGTGACCACCGCCAAGAAGCTGCGGGGCGGCG TGGTGTTCGTGGACGAGGTCCCGAAGGGCCTGACCGGGAAGCTCGACGCCCGGAAGATCC GCGAGATCCTGATCAAGGCCAAGAAGGGCGGCAAGATCGCCGTGTAAGACTAGTTATAAG ACTGACTAGCCCGATGGGCCTCCCAACGGGCCCTCCTCCCCTCCTTGCACCGAGATTAAT AAAAAAAAAAAAAAAAAAAAAAAAAAAAAAAAAAAAAAAAAAAAAAAAAAAAAAAAAAAA AAAA (SEQ ID NO: 1469)

AIG1 - PpLuc(GC) - ag - A64

GGGCCGCCCAGCCGGTCCAGGCCTCTGGCGAACAAGCTTGAGGATGGAGGACGCCAAGAA CATCAAGAAGGGCCCGGCGCCCTTCTACCCGCTGGAGGACGGGACCGCCGGCGAGCAGCT CCACAAGGCCATGAAGCGGTACGCCCTGGTGCCGGGCACGATCGCCTTCACCGACGCCCA CATCGAGGTCGACATCACCTACGCGGAGTACTTCGAGATGAGCGTGCGCCTGGCCGAGGC CATGAAGCGGTACGGCCTGAACACCAACCACCGGATCGTGGTGTGCTCGGAGAACAGCCT GCAGTTCTTCATGCCGGTGCTGGGCGCCCTCTTCATCGGCGTGGCCGTCGCCCCGGCGAA CGACATCTACAACGAGCGGGAGCTGCTGAACAGCATGGGGATCAGCCAGCCGACCGTGGT GTTCGTGAGCAAGAAGGGCCTGCAGAAGATCCTGAACGTGCAGAAGAAGCTGCCCATCAT CCAGAAGATCATCATCATGGACAGCAAGACCGACTACCAGGGCTTCCAGTCGATGTACAC GTTCGTGACCAGCCACCTCCCGCCGGGCTTCAACGAGTACGACTTCGTCCCGGAGAGCTT CGACCGGGACAAGACCATCGCCCTGATCATGAACAGCAGCGGCAGCACCGGCCTGCCGAA GGGGGTGGCCCTGCCGCACCGGACCGCCTGCGTGCGCTTCTCGCACGCCCGGGACCCCAT CTTCGGCAACCAGATCATCCCGGACACCGCCATCCTGAGCGTGGTGCCGTTCCACCACGG CTTCGGCATGTTCACGACCCTGGGCTACCTCATCTGCGGCTTCCGGGTGGTCCTGATGTA CCGGTTCGAGGAGGAGCTGTTCCTGCGGAGCCTGCAGGACTACAAGATCCAGAGCGCGCT GCTCGTGCCGACCCTGTTCAGCTTCTTCGCCAAGAGCACCCTGATCGACAAGTACGACCT GTCGAACCTGCACGAGATCGCCAGCGGGGGCGCCCCGCTGAGCAAGGAGGTGGGCGAGGC CGTGGCCAAGCGGTTCCACCTCCCGGGCATCCGCCAGGGCTACGGCCTGACCGAGACCAC GAGCGCGATCCTGATCACCCCCGAGGGGGACGACAAGCCGGGCGCCGTGGGCAAGGTGGT CCCGTTCTTCGAGGCCAAGGTGGTGGACCTGGACACCGGCAAGACCCTGGGCGTGAACCA GCGGGGCGAGCTGTGCGTGCGGGGGCCGATGATCATGAGCGGCTACGTGAACAACCCGGA GGCCACCAACGCCCTCATCGACAAGGACGGCTGGCTGCACAGCGGCGACATCGCCTACTG GGACGAGGACGAGCACTTCTTCATCGTCGACCGGCTGAAGTCGCTGATCAAGTACAAGGG CTACCAGGTGGCGCCGGCCGAGCTGGAGAGCATCCTGCTCCAGCACCCCAACATCTTCGA CGCCGGCGTGGCCGGGCTGCCGGACGACGACGCCGGCGAGCTGCCGGCCGCGGTGGTGGT GCTGGAGCACGGCAAGACCATGACGGAGAAGGAGATCGTCGACTACGTGGCCAGCCAGGT GACCACCGCCAAGAAGCTGCGGGGCGGCGTGGTGTTCGTGGACGAGGTCCCGAAGGGCCT GACCGGGAAGCTCGACGCCCGGAAGATCCGCGAGATCCTGATCAAGGCCAAGAAGGGCGG CAAGATCGCCGTGTAAGACTAGTTATAAGACTGACTAGCCCGATGGGCCTCCCAACGGGC CCTCCTCCCCTCCTTGCACCGAGATTAATAAAAAAAAAAAAAAAAAAAAAAAAAAAAAAA AAAAAAAAAAAAAAAAAAAAAAAAAAAAAAAAA (SEQ ID NO: 1470)

COX6C - PpLuc(GC) - ag - A64

GGAGTCAGGAAGGACGTTGGTGTTGAGGTTAGCATACGTATCAAGGACAGTAACTACCAA

GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTTATAAGACTGAC
TAGCCCGATGGGCCTCCCAACGGGCCCTCCTCCCCTCCTTGCACCGAGATTAATAAAAAA
AAAAAAAAAAAAAAAAAAAAAAAAAAAAAAAAAAAAAAAAAAAAAAAAAAAAAAAAA (SEQ ID NO: 1471)

ASAH1 - PpLuc(GC) - ag - A64

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG

GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAA (SEQ ID NO: 1472)

RPL35 - PpLuc(GC) - ag - A64 - histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAATGCATCAAAGGCTCTTTTCAGAGC
CACCA (SEQ ID NO: 1473)

RPL21 - PpLuc(GC) - ag - A64 - histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA

GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAATGCATCAAAGGCTCTTTTCAGAGC
CACCA (SEQ ID NO: 1474)

atp5a1 - PpLuc(GC) - ag - A64 - histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC

AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTTATAAGACTGACTAGCCCGATGGGCCTCCCAACGGGCCCTCCTCCCCTCCTTG
CACCGAGATTAATAAAAAAAAAAAAAAAAAAAAAAAAAAAAAAAAAAAAAAAAAAAAAA
AAAAAAAAAAAAAAAAATGCATCAAAGGCTCTTTTCAGAGCCACCA (SEQ ID NO: 1475)

HSD17B4 - PpLuc(GC) - ag - A64 - histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTTATAAG
ACTGACTAGCCCGATGGGCCTCCCAACGGGCCCTCCTCCCCTCCTTGCACCGAGATTAAT
AAAAAAAAAAAAAAAAAAAAAAAAAAAAAAAAAAAAAAAAAAAAAAAAAAAAAAAAAAAA
AAAATGCATCAAAGGCTCTTTTCAGAGCCACCA (SEQ ID NO: 1476)

AIG1 - PpLuc(GC) - ag - A64 - histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT

CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTTATAAGACTGACTAGCCCGATGGGCCTCCCAACGGGC
CCTCCTCCCCTCCTTGCACCGAGATTAATAAAAAAAAAAAAAAAAAAAAAAAAAAAAAAA
AAAAAAAAAAAAAAAAAAAAAAAAAAAAAAAAAATGCATCAAAGGCTCTTTTCAGAGCCAC
CA(SEQ ID NO: 1477)

COX6C - PpLuc(GC) - ag - A64 - histoneSL

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA

CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTTATAAGACTGAC
TAGCCCGATGGGCCTCCCAACGGGCCCTCCTCCCCTCCTTGCACCGAGATTAATAAAAAA
AAAAAAAAAAAAAAAAAAAAAAAAAAAAAAAAAAAAAAAAAAAAAAAAAAAAAAAAAA
TGCATCAAAGGCTCTTTTCAGAGCCACCA (SEQ ID NO: 1478)

ASAH1 - PpLuc(GC) - ag - A64 - histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAATGCATCAAAGGCTCTT
TTCAGAGCCACCA (SEQ ID NO: 1479)

RPL32 - PpLuc(GC) - ag - A64 - histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC

CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAATGCATCAAAGGCTCTT
TTCAGAGCCACCA (SEQ ID NO: 1480)

RPL32 - PpLuc(GC) - albumin? - A64 - C30 - histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC

AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCCCAAAGGCTCTTTTCAGAGCCACCAGAATT (SEQ ID
NO: 1481)

## Claims

1. An artificial nucleic acid molecule comprising:

    a. at least one 5'-untranslated region element (5'UTR element) which comprises or consists of a nucleic acid sequence which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a functional fragment or a functional variant thereof,
    wherein the functional fragment or variant fulfils at least one function of the naturally occuring 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit VIc gene (COX6C), or a N-acylsphingosine amidohydrolase (acid ceramidase) 1 gene (ASAH1), wherein the at least one function is stabilizing the mRNA and/or stabilizing and/or prolonging protein production from an mRNA; and
    b. at least one open reading frame (ORF),

    wherein the 5'UTR element and the open reading frame are heterologous.

2. The artificial nucleic acid molecule according to claim 1, further comprising:
    c. at least one histone stem-loop.

3. The artificial nucleic acid molecule according to claim 1 or 2, wherein the 5'UTR element is suitable for increasing protein production from the artificial nucleic acid molecule.

4. The artificial nucleic acid molecule according to any one of claims 1-3, wherein the 5'UTR element does not comprise a TOP-motif, preferably wherein the nucleic acid sequence, which is derived from a 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a functional fragment or a functional variant thereof, preferably the 5'UTR element, starts at its 5'-end with a nucleotide located at position 1, 2, 3, 4, 5, 6, 7, 8, 9 or 10 downstream of the polypyrimidine tract.

5. The artificial nucleic acid molecule according to any one of claims 1-4, wherein the nucleic acid sequence, which is derived from a 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a functional fragment or a functional variant thereof, preferably the 5'UTR element, terminates at its 3'-end with a nucleotide located at position 1, 2, 3, 4, 5, 6, 7, 8, 9 or 10 upstream of the start codon of the gene it is derived from.

6. The artificial nucleic acid molecule according to any one of claims 1-5, wherein the 5'UTR element does not comprise a start codon or an open reading frame.

7. The artificial nucleic acid molecule according to any one of claims 2-6, wherein the at least one histone stem-loop is selected from following formulae (I) or (II):

formula (I) (stem-loop sequence without stem bordering elements):

$$\underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}$$

formula (II) (stem-loop sequence with stem bordering elements):

$$\underbrace{N_{1-6}}_{\text{stem1 bordering element}}\ \underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}\ \underbrace{N_{1-6}}_{\text{stem2 bordering element}}$$

wherein:

stem1 or stem2 bordering elements $N_{1-6}$ is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
stem1 $[N_{0-2}GN_{3-5}]$ is reverse complementary or partially reverse complementary with element stem2, and is a consecutive sequence between of 5 to 7 nucleotides;
wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof, and
wherein G is guanosine or an analogue thereof, and may be optionally replaced by a cytidine or an analogue thereof, provided that its complementary nucleotide cytidine in stem2 is replaced by guanosine;
loop sequence $[N_{0-4}(U/T)N_{0-4}]$is located between elements stem1 and stem2, and is a consecutive sequence of 3 to 5 nucleotides, more preferably of 4 nucleotides;
wherein each $N_{0-4}$ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and
wherein U/T represents uridine, or optionally thymidine;
stem2 $[N_{3-5}CN_{0-2}]$ is reverse complementary or partially reverse complementary with element stem1, and is a consecutive sequence between of 5 to 7 nucleotides;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof;
wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof; and
wherein C is cytidine or an analogue thereof, and may be optionally replaced by a guanosine or an analogue thereof provided that its complementary nucleotide guanosine in stem1 is replaced by cytidine;

wherein
stem1 and stem2 are capable of base pairing with each other forming a reverse complementary sequence, wherein base pairing may occur between stem1 and stem2, or
forming a partially reverse complementary sequence, wherein an incomplete base pairing may occur between stem1 and stem2.

**8.** The artificial nucleic acid molecule according to any one of claims 2-7, wherein the at least one histone stem-loop is selected from at least one of following formulae (Ia) or (IIa):

$$\underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}$$

formula (Ia) (stem-loop sequence without stem bordering elements)

$$\underbrace{N_{2-5}}_{\substack{\text{stem1}\\ \text{bordering element}}}\ \underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}\ \underbrace{N_{2-5}}_{\substack{\text{stem2}\\ \text{bordering element}}}$$

formula (IIa) (stem-loop sequence with stem bordering elements)

**9.** The artificial nucleic acid molecule according to any one of claims 1-8, further comprising
d. a poly(A) sequence and/or a polyadenylation signal.

**10.** The artificial nucleic acid molecule according to any of claims 1-9, further comprising:
e. a poly(C) sequence.

**11.** The artificial nucleic acid molecule according to any one of claims 1-10, further comprising:
f. at least one 3'UTR element.

**12.** The artificial nucleic acid molecule according to claim 11, wherein the at least one 3'UTR element comprises or consists of a nucleic acid sequence, which is derived from a 3'UTR of a gene providing a stable mRNA or from a variant of the 3'UTR of a gene providing a stable mRNA.

**13.** The artificial nucleic acid molecule according to any one of claims 1-12, wherein the 5'UTR element comprises or consists of a nucleic acid sequence which is derived from the 5'UTR of a vertebrate ribosomal protein Large 32 (L32) gene, a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (1 7-beta) dehydrogenase 4 gene 15 (HSD17B4), a vertebrate androgen-induced 1 gene (AIG1), vertebrate cytochrome c oxidase subunit VIc gene (COX6C), a vertebrate N-acylsphingosine amidohydrolase (acid ceramidase) 1 gene (ASAH1) or from a functional fragment or from a functional variant thereof, more preferably from the 5'UTR of a mammalian ribosomal protein Large 32 (L32) gene, a mammalian ribosomal protein Large 35 gene (RPL35), a mammalian ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene 15 (HSD17B4), a mammalian androgen-induced 1 gene (AIG1), mammalian cytochrome c oxidase subunit VIc gene (COX6C), a mammalian N-acylsphingosine amidohydrolase (acid ceramidase) 1 gene (ASAH1) or from a functional fragment or a functional variant thereof, most preferably from the 5'UTR of a human ribosomal protein Large 32 (L32) gene, a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene 15 (HSD17B4), a human androgen-induced 1 gene (AIG1), human cytochrome c oxidase subunit

VIc gene (COX6C), a human N-acylsphingosine amidohydrolase (acid ceramidase) 1 gene (ASAH1) or from a functional fragment or a functional variant thereof, wherein preferably the 5'UTR element, preferably the artificial nucleic acid molecule does not comprise the 5'TOP of said gene.

**14.** The artificial nucleic acid molecule according to any one of claims 2-13, wherein the at least one histone stem-loop comprises or consists of a nucleic acid sequence selected from the group consisting of SEQ ID NOs. 1391-1433, preferably from the group consisting of SEQ ID NOs. 1403-1433.

**15.** The artificial nucleic acid molecule according to any one of claims 2-14, wherein the histone stem-loop comprises or consists of a nucleic acid sequence having a sequence identity of at least about 80%, preferably of at least about 85%, more preferably of at least about 90%, even more preferably of at least about 95% to the sequence according to SEQ ID NO. 1433 or to the corresponding RNA sequence, wherein preferably positions 6, 13 and 20 of the sequence having a sequence identity of at least about 80%, preferably at least about 85%, more preferably at least about 90%, even more preferably at least about 95% to the sequence according to SEQ ID NO. 1433 or to the corresponding RNA sequence are conserved, i.e. are identical to the nucleotides at positions 6, 13 and 20 of SEQ ID NO. 1433 or to the corresponding RNA nucleotides.

**16.** The artificial nucleic acid molecule according to any one of claims 11-15, wherein the 3'UTR element comprises or consists of a nucleic acid sequence derived from a 3'UTR of a gene selected from the group consisting of an albumin gene, an $\alpha$-globin gene, a $\beta$-globin gene, a tyrosine hydroxylase gene, a lipoxygenase gene, and a collagen alpha gene, or from a variant of a 3'UTR of a gene selected from the group consisting of an albumin gene, an $\alpha$-globin gene, a $\beta$-globin gene, a tyrosine hydroxylase gene, a lipoxygenase gene, and a collagen alpha gene, wherein the at least one 3'UTR element preferably comprises or consists of

a nucleic acid sequence which is derived from the 3'UTR of a vertebrate albumin gene or from a variant thereof, preferably from the 3'UTR of a mammalian albumin gene or from a variant thereof, more preferably from the 3'UTR of a human albumin gene or from a variant thereof, even more preferably from the 3'UTR of the human albumin gene according to GenBank Accession number NM_000477.5 or from a variant thereof; or
a nucleic acid sequence, which is derived from the 3'UTR of a vertebrate $\alpha$-globin gene or from a variant thereof, preferably from the 3'UTR of a mammalian $\alpha$-globin gene or from a variant thereof, more preferably from the 3'UTR of a human $\alpha$-globin gene or from a variant thereof.

**17.** The artificial nucleic acid molecule according to any one of claims 11-16, wherein the at least one 3'UTR element comprises or consists of a nucleic acid sequence which has an identity of at least about 80%, more preferably of at least about 90%, even more preferably of at least about 95%, even more preferably of at least about 99% to a nucleic acid sequence selected from SEQ ID NOs. 1369-1377 and 1434 or to a corresponding RNA sequence, or wherein the at least one 3'UTR element comprises or consists of a functional fragment of a nucleic acid sequence which has an identity of at least about 80%, more preferably of at least about 90%, even more preferably of at least about 95%, even more preferably of at least about 99% to a nucleic acid sequence selected from SEQ ID NOs. 1369-1377 and 1434 or to a corresponding RNA sequence.

**18.** The artificial nucleic acid molecule according to any one of claims 1-17, wherein the artificial nucleic acid molecule, preferably the open reading frame, is at least partially G/C modified, preferably wherein the G/C content of the open reading frame is increased compared to the wild type open reading frame.

**19.** The artificial nucleic acid molecule according to any one of claims 1-18, which is an RNA, preferably an mRNA molecule.

**20.** A vector comprising:

a. at least one 5'-untranslated region element (5'UTR element) which comprises or consists of a nucleic acid sequence which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (1 7-beta) dehydrogenase 4 gene 15 (HSD1 7B4), an androgen-induced 1 gene (AIG1), cytochrome c oxidase subunit VIc gene (COX6C), or a N-acylsphingosine amidohydrolase (acid ceramidase) 1 gene (ASAH1) or from a functional fragment or a functional variant thereof,
wherein the functional fragment or variant fulfils at least one function of the naturally occuring 5'UTR of a

ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit Vlc gene (COX6C), or a N-acylsphingosine amidohydrolase (acid ceramidase) 1 gene (ASAH1), wherein the at least one function is stabilizing the mRNA and/or stabilizing and/or prolonging protein production from an mRNA; and
b. at least one open reading frame (ORF) and/or at least one cloning site,

wherein the 5'UTR element and the open reading frame are heterologous.

**21.** The vector according to claim 20, further comprising:
c. at least one histone-stem loop.

**22.** The vector according to claim 20 or 21, which is a DNA vector, preferably a plasmid vector, or a viral vector.

**23.** The vector according to any one of claims 20-22, which comprises or codes for an artificial nucleic acid molecule according to any one of claims 1-19.

**24.** A cell comprising the artificial nucleic acid molecule according to any one of claims 1-19 or the vector according to any one of claims 20-23, wherein the cell is preferably a mammalian cell, more preferably an isolated cell of a mammalian subject, most preferably of a human subject.

**25.** A pharmaceutical composition comprising the artificial nucleic acid molecule according to any one of claims 1-19, the vector according to any one of claims 20-23, or the cell according to claim 24, optionally further comprising one or more pharmaceutically acceptable diluents and/or excipients and/or one or more adjuvants.

**26.** The artificial nucleic acid molecule according to any one of claims 1-19, the vector according to any one of claims 20-23, the cell according to claim 24, or the pharmaceutical composition according to claim 25 for use as a medicament, preferably for use as a vaccine or for use in gene therapy.

**27.** An in vitro method for increasing protein production from an artificial nucleic acid molecule, comprising the step of providing the artificial nucleic acid molecule with

i. at least one 5'-untranslated region element (5'UTR element) which comprises or consists of a nucleic acid sequence, which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (1 7-beta) dehydrogenase 4 gene 15 (HSD1 7B4), an androgen-induced 1 gene (AIG1), cytochrome c oxidase subunit Vlc gene (COX6C), or a N-acylsphingosine amidohydrolase (acid ceramidase) 1 gene (ASAH1) or from a functional fragment or a functional variant thereof,
wherein the functional fragment or variant fulfils at least one function of the naturally occuring 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene 15 (HSD17B4), an androgen-induced 1 gene (AIG1), cytochrome c oxidase subunit Vlc gene (COX6C), or a N-acylsphingosine amidohydrolase (acid ceramidase) 1 gene (ASAH1), wherein the at least one function is stabilizing the mRNA and/or stabilizing and/or prolonging protein production from an mRNA,
ii. preferably at least one histone stem-loop; and
iii. optionally, a poly(A) sequence and/or a polyadenylation signal.

**28.** In vitro use of a 5'UTR element, which comprises or consists of a nucleic acid sequence, which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (1 7-beta) dehydrogenase 4 gene 15 (HSD17B4), an androgen-induced 1 gene (AIG1), cytochrome c oxidase subunit Vlc gene (COX6C), or a N-acylsphingosine amidohydrolase (acid ceramidase) 1 gene (ASAH1) or from a functional fragment or a functional variant thereof, wherein the functional fragment or variant fulfils at least one function of the naturally occuring 5'UTR of a ribosomal protein Large 32 gene (RPL32), a ribosomal protein Large 35 gene (RPL35), a ribosomal protein Large 21 gene (RPL21), an ATP synthase,

H+ transporting, mitochondrial F1 complex, alpha subunit 1, cardiac muscle (ATP5A1) gene, an hydroxysteroid (1 7-beta) dehydrogenase 4 gene 15 (HSD17B4), an androgen-induced 1 gene (AIG1), cytochrome c oxidase subunit Vlc gene (COX6C), or a N-acylsphingosine amidohydrolase (acid ceramidase) 1 gene (ASAH1), wherein the at least one function is stabilizing the mRNA and/or stabilizing and/or prolonging protein production from an mRNA, and preferably further comprises at least one histone stem-loop for increasing protein production from a nucleic acid molecule.

**29.** A kit or kit of parts comprising an artificial nucleic acid molecule according to any one of claims 1-19, the vector according to any one of claims 20-23, the cell according to claim 24, and/or the pharmaceutical composition according to claim 25, the kit or kit of parts preferably further comprising instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector or the pharmaceutical composition.

## Patentansprüche

**1.** Artifizielles Nukleinsäuremolekül, umfassend:

a. mindestens ein Element der 5'-untranslatierten Region (5'-UTR-Element), das eine Nukleinsäuresequenz umfasst oder aus einer Nukleinsäuresequenz besteht, die von der 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1-Gens (ATP-Synthase, H+-transportierend, mitochondrialer F1-Komplex, alpha-Untereinheit 1, Herzmuskel), eines Hydroxysteroid (17-beta)-Dehydrogenase-4-Gens 15 (HSD17B4), eines Androgen-induziertes Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) oder von einem funktionellen Fragment oder einer funktionellen Variante davon abgeleitet ist,
wobei das funktionelle Fragment oder die funktionelle Variante mindestens eine Funktion der natürlich vorkommenden 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1-Gens (ATP-Synthase, H+-transportierend, mitochondrialer F1-Komplex, alpha-Untereinheit 1, Herzmuskel), eines Hydroxysteroid (17-beta)-Dehydrogenase-4-Gens 15 (HSD17B4), eines Androgen-induziertes Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) hat, wobei die mindestens eine Funktion die Stabilisierung der mRNA und/oder die Stabilisierung und/oder Verlängerung der Proteinproduktion von einer mRNA ist; und
b. mindestens einen offenen Leserahmen (ORF),

wobei das 5'-UTR-Element und der offene Leserahmen heterolog sind.

**2.** Artifizielles Nukleinsäuremolekül gemäß Anspruch 1, ferner umfassend:
c. mindestens einen Histon-Stem-Loop.

**3.** Artifizielles Nukleinsäuremolekül gemäß Anspruch 1 oder 2, wobei das 5'-UTR-Element zur Erhöhung der Proteinproduktion von dem artifiziellen Nukleinsäuremolekül geeignet ist.

**4.** Artifizielles Nukleinsäuremolekül gemäß irgendeinem der Ansprüche 1 bis 3, wobei das 5'-UTR-Element kein TOP-Motiv umfasst, wobei vorzugsweise die Nukleinsäuresequenz, die von einer 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1-Gens (ATP-Synthase, H+-transportierend, mitochondrialer F1-Komplex, alpha-Untereinheit 1, Herzmuskel), eines Hydroxysteroid (17-beta)-Dehydrogenase-4-Gens 15 (HSD17B4), eines Androgen-induziertes Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) oder von einem funktionellen Fragment oder einer funktionellen Variante davon abgeleitet ist, vorzugsweise das 5'-UTR-Element, an seinem 5'-Ende mit einem Nukleotid beginnt, das sich an Position 1, 2, 3, 4, 5, 6, 7, 8, 9 oder 10 stromabwärts des Polypyrimidinstranges befindet.

**5.** Artifizielles Nukleinsäuremolekül gemäß irgendeinem der Ansprüche 1 bis 4, wobei die Nukleinsäuresequenz, die von einer 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1-Gens (ATP-Synthase,

H+-transportierend, mitochondrialer F1-Komplex, alpha-Untereinheit 1, Herzmuskel), eines Hydroxysteroid (17-beta)-Dehydrogenase-4-Gens 15 (HSD17B4), eines Androgen-induziertes Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) oder von einem funktionellen Fragment oder einer funktionellen Variante davon abgeleitet ist, vorzugsweise das 5'-UTR-Element, an seinem 3'-Ende mit einem Nukleotid endet, das sich an Position 1, 2, 3, 4, 5, 6, 7, 8, 9 oder 10 stromaufwärts des Startcodons des Gens, von dem es abgeleitet ist, befindet.

**6.** Artifizielles Nukleinsäuremolekül gemäß irgendeinem der Ansprüche 1 bis 5, wobei das 5'-UTR-Element kein Startcodon oder keinen offenen Leserahmen umfasst.

**7.** Artifizielles Nukleinsäuremolekül gemäß irgendeinem der Ansprüche 2 bis 6, wobei der mindestens eine Histon-Stem-Loop aus den folgenden Formeln (I) oder (II) ausgewählt ist:

Formel (I) (Stem-Loop-Sequenz ohne an den Stamm („stem") angrenzende Elemente):

$$\underbrace{[N_{0-2}GN_{3-5}]}_{\text{stem1}}\ \underbrace{[N_{0-4}(U/T)N_{0-4}]}_{\text{Loop}}\ \underbrace{[N_{3-5}CN_{0-2}]}_{\text{stem2}}$$

Formel (II) (Stem-Loop-Sequenz mit an den Stamm („stem") angrenzenden Elementen):

$$\underbrace{N_{1-6}}_{\text{stem1 angrenzendes Element}}\ \underbrace{[N_{0-2}GN_{3-5}]}_{\text{stem1}}\ \underbrace{[N_{0-4}(U/T)N_{0-4}]}_{\text{Loop}}\ \underbrace{[N_{3-5}CN_{0-2}]}_{\text{stem2}}\ \underbrace{N_{1-6}}_{\text{stem2 angrenzendes Element}}$$

wobei:

das an stem1 oder stem2 angrenzende Element $N_{1-6}$ eine konsekutive Sequenz von 1 bis 6, bevorzugt von 2 bis 6, bevorzugter von 2 bis 5, noch bevorzugter von 3 bis 5, am meisten bevorzugt von 4 bis 5 oder 5 N ist, wobei jedes N unabhängig von den anderen ausgewählt ist aus einem Nukleotid ausgewählt aus A, U, T, G und C oder einem Nukleotidanalogon davon;
stem1 $[N_{0-2}GN_{3-5}]$ revers-komplementär oder teilweise revers-komplementär zu Element stem2 ist, und eine konsekutive Sequenz von 5 bis 7 Nukleotiden ist;
wobei $N_{0-2}$ eine konsekutive Sequenz von 0 bis 2, bevorzugt von 0 bis 1, bevorzugter von 1 N ist, wobei jedes N unabhängig von den anderen ausgewählt ist aus einem Nukleotid, ausgewählt aus A, U, T, G und C oder einem Nukleotidanalogon davon;
wobei $N_{3-5}$ eine konsekutive Sequenz von 3 bis 5, bevorzugt von 4 bis 5, bevorzugter von 4 N ist, wobei jedes N unabhängig von den anderen ausgewählt ist aus einem Nukleotid, ausgewählt aus A, U, T, G und C oder einem Nukleotidanalogon davon, und
wobei G Guanosin oder ein Analogon davon ist und optional durch ein Cytidin oder ein Analogon davon ersetzt sein kann, vorausgesetzt, dass sein komplementäres Nukleotid Cytidin in stem2 durch Guanosin ersetzt ist;
Loop-Sequenz $[N_{0-4}(U/T)N_{0-4}]$ zwischen den Elementen stem1 und stem2 gelegen ist und eine konsekutive Sequenz von 3 bis 5 Nukleotiden, bevorzugter von 4 Nukleotiden ist;
wobei jedes $N_{0-4}$ unabhängig von den anderen eine konsekutive Sequenz von 0 bis 4, bevorzugt von 1 bis 3, bevorzugter von 1 bis 2 N ist, wobei jedes N unabhängig von den anderen ausgewählt ist aus einem Nukleotid, ausgewählt aus A, U, T, G und C oder einem Nukleotidanalogon davon; und
wobei U/T Uridin oder optional Thymidin darstellt;
stem2 $[N_{3-5}CN_{0-2}]$ revers-komplementär oder teilweise revers-komplementär zu Element stem1 ist und eine konsekutive Sequenz von 5 bis 7 Nukleotiden ist;

wobei $N_{3-5}$ eine konsekutive Sequenz von 3 bis 5, bevorzugt von 4 bis 5, bevorzugter von 4 N ist, wobei jedes N unabhängig von den anderen ausgewählt ist aus einem Nukleotid, ausgewählt aus A, U, T, G und C oder einem Nukleotidanalogon davon;
wobei $N_{0-2}$ eine konsekutive Sequenz von 0 bis 2, bevorzugt von 0 bis 1, bevorzugter von 1 N ist, wobei jedes N unabhängig von den anderen ausgewählt ist aus einem Nukleotid, ausgewählt aus A, U, T, G und C oder einem Nukleotidanalogon davon; und
wobei C Cytidin oder ein Analogon davon ist, und optional durch ein Guanosin oder ein Analogon davon ersetzt sein kann, vorausgesetzt, dass sein komplementäres Nukleotid Guanosin in stem1 durch Cytidin ersetzt ist;
wobei
stem1 und stem2 zur Basenpaarung miteinander fähig sind und eine revers-komplementäre Sequenz bilden, wobei zwischen stem1 und stem2 Basenpaarung auftreten kann, oder miteinander eine teilweise revers-komplementäre Sequenz bilden können, wobei eine unvollständige Basenpaarung zwischen stem1 und stem2 auftreten kann.

**8.** Artifizielles Nukleinsäuremolekül gemäß irgendeinem der Ansprüche 2 bis 7, wobei der mindestens eine Histon-Stem-Loop aus mindestens einer der folgenden Formeln (Ia) oder (IIa) ausgewählt ist:

$$\underbrace{[N_{0-1}GN_{3-5}]}_{\text{stem1}}\ \underbrace{[N_{1-3}(U/T)N_{0-2}]}_{\text{Loop}}\ \underbrace{[N_{3-5}CN_{0-1}]}_{\text{stem2}}$$

Formel (Ia) (Stem-Loop-Sequenz ohne an den Stamm angrenzende Elemente)

$$\underbrace{N_{2-5}}_{\text{stem1}}\ \underbrace{[N_{0-1}GN_{3-5}]}_{\text{stem1}}\ \underbrace{[N_{1-3}(U/T)N_{0-2}]}_{\text{Loop}}\ \underbrace{[N_{3-5}CN_{0-1}]}_{\text{stem2}}\ \underbrace{N_{2-5}}_{\text{stem2}}$$

angrenzendes Element ... angrenzendes Element

Formel (IIa) (Stem-Loop-Sequenz mit an den Stamm angrenzenden Elementen)

**9.** Artifizielles Nukleinsäuremolekül gemäß irgendeinem der Ansprüche 1 bis 8, ferner umfassend
d. eine Poly(A)-Sequenz und/oder ein Polyadenylierungssignal.

**10.** Artifizielles Nukleinsäuremolekül gemäß irgendeinem der Ansprüche 1 bis 9, ferner umfassend:
e. eine Poly(C)-Sequenz.

**11.** Artifizielles Nukleinsäuremolekül gemäß irgendeinem der Ansprüche 1 bis 10, ferner umfassend:
f. mindestens ein 3'-UTR-Element.

**12.** Artifizielles Nukleinsäuremolekül gemäß Anspruch 11, wobei das mindestens eine 3'-UTR-Element eine Nukleinsäuresequenz umfasst oder aus einer Nukleinsäuresequenz besteht, die von einer 3'-UTR eines Gens, das eine stabile mRNA liefert, oder von einer Variante der 3'-UTR eines Gens, das eine stabile mRNA liefert, abgeleitet ist.

**13.** Artifizielles Nukleinsäuremolekül gemäß irgendeinem der Ansprüche 1 bis 12, wobei das 5'-UTR-Element eine Nukleinsäuresequenz umfasst oder aus einer Nukleinsäuresequenz besteht, die von der 5'-UTR eines Vertebraten-Gens des ribosomalen Proteins Large 32 (RPL32), eines Vertebraten-Gens des ribosomalen Proteins Large 35 (RPL35), eines Vertebraten-Gens des ribosomalen Proteins Large 21 (RPL21), eines Vertebraten-ATP5A1-Gens (ATP-Synthase, H+-transportierend, mitochondrialer F1-Komplex, alpha-Untereinheit 1, Herzmuskel), eines Vertebraten-Hydroxysteroid-(17-beta)-dehydrogenase-4-Gens 15 (HSD17B4), eines Vertebraten-Androgen-induzierten Gens 1 (AIG1), eines Vertebraten-Cytochrom-C-Oxidase-Untereinheit-VIc-Gens (COX6C) oder eines Vertebraten-N-Acylsphingosin-Amidohydrolase (saure Ceramidase)-1-Gens (ASAH1) oder von einem funktionellen Fragment oder einer funktionellen Variante davon, bevorzugter von der 5'-UTR eines Säugetier-Gens des ribosomalen Proteins Large 32 (RPL32), eines Säugetier-Gens des ribosomalen Proteins Large 35 (RPL35), eines Säugetier-Gens des

ribosomalen Proteins Large 21 (RPL21), eines Säugetier-ATP5A1-Gens (ATP-Synthase, H+-transportierend, mitochondrialer F1-Komplex, alpha-Untereinheit 1, Herzmuskel), eines Säugetier-Hydroxysteroid-(17-beta)-dehydrogenase-4-Gens (HSD17B4), eines Säugetier-Androgen-induzierten Gens 1 (AIG1), eines Säugetier-Cytochrom-C-Oxidase-Untereinheit-Vlc-Gens (COX6C) oder eines Säugetier-N-Acylsphingosin-Amidohydrolase (saure Ceramidase)-1-Gens (ASAH1) oder von einem funktionellen Fragment oder einer funktionellen Variante davon, am meisten bevorzugt von der 5'-UTR eines humanen Gens des ribosomalen Proteins Large 32 (RPL32), eines humanen Gens des ribosomalen Proteins Large 35 (RPL35), eines humanen Gens des ribosomalen Proteins Large 21 (RPL21), eines humanen ATP5A1-Gens (ATP-Synthase, H+-transportierend, mitochondrialer F1-Komplex, alpha-Untereinheit 1, Herzmuskel), eines humanen Hydroxysteroid-(17-beta)-dehydrogenase-4-Gens (HSD17B4), eines humanen Androgen-induzierten Gens 1 (AIG1), eines humanen Cytochrom-C-Oxidase-Untereinheit-Vlc-Gens (COX6C) oder eines humanen N-Acylsphingosin-Amidohydrolase (saure Ceramidase)-1-Gens (ASAH1) oder von einem funktionellen Fragment oder einer funktionellen Variante davon abgeleitet ist, wobei vorzugsweise das 5'-UTR-Element nicht den 5'-TOP des besagten Gens umfasst.

**14.** Artifizielles Nukleinsäuremolekül gemäß irgendeinem der Ansprüche 2 bis 13, wobei der mindestens eine Histon-Stem-Loop eine Nukleinsäuresequenz umfasst oder aus einer Nukleinsäuresequenz besteht, die ausgewählt ist aus der Gruppe bestehend aus SEQ ID NOs. 1391-1433, vorzugsweise aus der Gruppe bestehend aus SEQ ID NOs. 1403-1433.

**15.** Artifizielles Nukleinsäuremolekül gemäß irgendeinem der Ansprüche 2 bis 14, wobei der Histon-Stem-Loop eine Nukleinsäuresequenz umfasst oder aus einer Nukleinsäuresequenz besteht, die eine Sequenzidentität von mindestens etwa 80%, bevorzugt von mindestens etwa 85%, bevorzugter von mindestens etwa 90%, noch bevorzugter von mindestens etwa 95% mit der Sequenz gemäß SEQ ID NO. 1433 oder der entsprechenden RNA-Sequenz aufweist, wobei vorzugsweise die Positionen 6, 13 und 20 der Sequenz, die eine Sequenzidentität von mindestens etwa 80%, bevorzugt mindestens etwa 85%, bevorzugter mindestens etwa 90%, noch bevorzugter mindestens etwa 95% mit der Sequenz gemäß SEQ ID NO. 1433 oder der entsprechenden RNA-Sequenz aufweist, konserviert sind, d.h. mit den Nukleotiden an den Positionen 6, 13 und 20 der SEQ ID NO. 1433 oder den entsprechenden RNA-Nukleotiden identisch sind.

**16.** Artifizielles Nukleinsäuremolekül gemäß irgendeinem der Ansprüche 11 bis 15, wobei das 3'-UTR-Element eine Nukleinsäuresequenz umfasst oder aus einer Nukleinsäuresequenz besteht, die von einer 3'-UTR eines Gens, ausgewählt aus der Gruppe bestehend aus einem Albumin-Gen, einem $\alpha$-Globin-Gen, einem $\beta$-Globin-Gen, einem Tyrosinhydroxylase-Gen, einem Lipoxygenase-Gen und einem Kollagen-alpha-Gen, oder von einer Variante einer 3'-UTR eines Gens, ausgewählt aus der Gruppe bestehend aus einem Albumin-Gen, einem $\alpha$-Globin-Gen, einem $\beta$-Globin-Gen, einem Tyrosin-Hydroxylase-Gen, einem Lipoxygenase-Gen und einem Kollagen-alpha-Gen, abgeleitet ist, wobei das mindestens eine 3'-UTR-Element vorzugsweise

eine Nukleinsäuresequenz, die von der 3'-UTR eines Vertebraten-Albumin-Gens oder einer Variante davon, bevorzugt von der 3'-UTR eines Säugetier-Albumin-Gens oder einer Variante davon, bevorzugter von der 3'-UTR eines humanen Albumin-Gens oder einer Variante davon, noch bevorzugter von der 3'-UTR des humanen Albumin-Gens gemäß der GenBank-Zugangsnummer NM_000477.5 oder einer Variante davon, abgeleitet ist;
oder
eine Nukleinsäuresequenz, die von der 3'-UTR eines Vertebraten-$\alpha$-Globin-Gens oder einer Variante davon, bevorzugt von der 3'-UTR eines Säugetier-$\alpha$-Globin-Gens oder einer Variante davon, bevorzugter von der 3'-UTR eines humanen $\alpha$ - Globin-Gens oder einer Variante davon abgeleitet ist,
umfasst oder aus dieser besteht.

**17.** Artifizielles Nukleinsäuremolekül gemäß irgendeinem der Ansprüche 11 bis 16, wobei das mindestens eine 3'-UTR-Element eine Nukleinsäuresequenz umfasst oder aus einer Nukleinsäuresequenz besteht, die eine Identität von mindestens etwa 80%, bevorzugter von mindestens etwa 90%, noch bevorzugter von mindestens etwa 95%, noch bevorzugter von mindestens etwa 99% mit einer Nukleinsäuresequenz, ausgewählt aus SEQ ID NOs. 1369-1377 und 1434, oder einer entsprechenden RNA-Sequenz aufweist, oder wobei das mindestens eine 3'-UTR-Element ein funktionelles Fragment einer Nukleinsäuresequenz umfasst oder aus dieser besteht, die eine Identität von mindestens etwa 80%, bevorzugter von mindestens etwa 90%, noch bevorzugter von mindestens etwa 95%, noch bevorzugter von mindestens etwa 99% mit einer Nukleinsäuresequenz, ausgewählt aus SEQ ID NOs. 1369-1377 und 1434, oder mit einer entsprechenden RNA-Sequenz aufweist.

**18.** Artifizielles Nukleinsäuremolekül gemäß irgendeinem der Ansprüche 1 bis 17, wobei das artifizielle Nukleinsäuremolekül, vorzugsweise der offene Leserahmen, zumindest teilweise G/C-modifiziert ist, wobei bevorzugt der G/C-Gehalt des offenen Leserahmens im Vergleich zum offenen Leserahmen des Wildtyps erhöht ist.

**19.** Artifizielles Nukleinsäuremolekül gemäß irgendeinem der Ansprüche 1 bis 18, das ein RNA-Molekül, vorzugsweise ein mRNA-Molekül, ist.

**20.** Vektor, umfassend:

a. mindestens ein Element der 5'-untranslatierten Region (5'-UTR-Element), das eine Nukleinsäuresequenz umfasst oder aus einer Nukleinsäuresequenz besteht, die von der 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1-Gens (ATP-Synthase, H+-transportierend, mitochondrialer F1-Komplex, alpha-Untereinheit 1, Herzmuskel), eines Hydroxysteroid (17-beta)-Dehydrogenase-4-Gens 15 (HSD17B4), eines Androgen-induziertes Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) oder von einem funktionellen Fragment oder einer funktionellen Variante davon abgeleitet ist,
wobei das funktionelle Fragment oder die Variante mindestens eine Funktion der natürlich vorkommenden 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1 -Gens (ATP-Synthase, H+-transportierend, mitochondrialer F1-Komplex, alpha-Untereinheit 1, Herzmuskel), eines Hydroxysteroid (1 7-beta)-Dehydrogenase-4-Gens 15 (HSD17B4), eines Androgen-induziertes Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) hat, wobei die mindestens eine Funktion die Stabilisierung der mRNA und/oder die Stabilisierung und/oder Verlängerung der Proteinproduktion von einer mRNA ist; und
b. mindestens einen offenen Leserahmen (ORF) und/oder mindestens eine Klonierungsstelle,

wobei das 5'-UTR-Element und der offene Leserahmen heterolog sind.

**21.** Vektor gemäß Anspruch 20, ferner umfassend:
c. mindestens einen Histon-Stem-Loop.

**22.** Vektor gemäß Anspruch 20 oder 21, der ein DNA-Vektor, vorzugsweise ein Plasmidvektor, oder ein viraler Vektor ist.

**23.** Vektor gemäß irgendeinem der Ansprüche 20 bis 22, der ein artifizielles Nukleinsäuremolekül gemäß irgendeinem der Ansprüche 1 bis 19 umfasst oder dafür kodiert.

**24.** Zelle umfassend das artifizielle Nukleinsäuremolekül gemäß irgendeinem der Ansprüche 1 bis 19 oder den Vektor gemäß irgendeinem der Ansprüche 20 bis 23, wobei die Zelle bevorzugt eine Säugetierzelle, bevorzugter eine isolierte Zelle eines Säugetiers, am meisten bevorzugt eines Menschen, ist.

**25.** Pharmazeutische Zusammensetzung, umfassend das artifizielle Nukleinsäuremolekül gemäß irgendeinem der Ansprüche 1 bis 19, den Vektor gemäß irgendeinem der Ansprüche 20 bis 23 oder die Zelle gemäß Anspruch 24, optional ferner umfassend ein oder mehrere pharmazeutisch akzeptable Verdünnungsmittel und/oder Exzipienten und/oder ein oder mehrere Adjuvanzien.

**26.** Artifizielles Nukleinsäuremolekül gemäß irgendeinem der Ansprüche 1 bis 19, Vektor gemäß irgendeinem der Ansprüche 20 bis 23, Zelle gemäß Anspruch 24 oder pharmazeutische Zusammensetzung gemäß Anspruch 25 zur Verwendung als Medikament, vorzugsweise zur Verwendung als Vakzine oder zur Verwendung in der Gentherapie.

**27.** In-vitro-Verfahren zur Erhöhung der Proteinproduktion von einem artifiziellen Nukleinsäuremolekül, umfassend den Schritt des Versehens des artifiziellen Nukleinsäuremoleküls mit

i. mindestens einem Element der 5'-untranslatierten Region (5'UTR-Element), das eine Nukleinsäuresequenz umfasst oder aus einer Nukleinsäuresequenz besteht, die von der 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1-Gens (ATP-Synthase, H+-transportierend, mitochondrialer F1-Komplex, alpha-Untereinheit 1, Herzmuskel), eines Hydroxysteroid (17-beta)-Dehydrogenase-4-Gens 15 (HSD17B4), eines Androgen-induziertes Gens 1 (AIG1), eines Cytochrom-C-Oxidase-Untereinheit-Vlc-Gens

(COX6C) oder eines N-Acylsphingosin-Amidohydrolase (saure Ceramidase)-1-Gens (ASAH1) oder von einem funktionellen Fragment oder einer funktionellen Variante davon abgeleitet ist,
wobei das funktionelle Fragment oder die Variante mindestens eine Funktion der natürlich vorkommenden 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1-Gens (ATP-Synthase, H+-transportierend, mitochondrialer F1-Komplex, alpha-Untereinheit 1, Herzmuskel), eines Hydroxysteroid (17-beta)-Dehydrogenase-4-Gens 15 (HSD17B4), eines Androgen-induziertes Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) hat, wobei die mindestens eine Funktion die Stabilisierung der mRNA und/oder die Stabilisierung und/oder Verlängerung der Proteinproduktion von einer mRNA ist,
ii. vorzugsweise mindestens einen Histon-Stem-Loop; und
iii. optional eine Poly(A)-Sequenz und/oder ein Polyadenylierungssignal.

**28.** In-vitro-Verwendung eines 5'-UTR-Elements, das eine Nukleinsäuresequenz umfasst oder aus einer Nukleinsäuresequenz besteht, die von der 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1-Gens (ATP-Synthase, H+-transportierend, mitochondrialer F1-Komplex, alpha-Untereinheit 1, Herzmuskel), eines Hydroxysteroid (17-beta)-Dehydrogenase-4-Gens 15 (HSD17B4), eines Androgen-induziertes Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) oder von einem funktionellen Fragment oder einer funktionellen Variante davon abgeleitet ist, wobei das funktionelle Fragment oder die Variante mindestens eine Funktion der natürlich vorkommenden 5'-UTR eines Gens des ribosomalen Proteins Large 32 (RPL32), eines Gens des ribosomalen Proteins Large 35 (RPL35), eines Gens des ribosomalen Proteins Large 21 (RPL21), eines ATP5A1-Gens (ATP-Synthase, H+-transportierend, mitochondrialer F1-Komplex, alpha-Untereinheit 1, Herzmuskel), eines Hydroxysteroid (17-beta)-Dehydrogenase-4-Gens 15 (HSD17B4), eines Androgen-induziertes Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) hat, wobei die mindestens eine Funktion die Stabilisierung der mRNA und/oder die Stabilisierung und/oder Verlängerung der Proteinproduktion von einer mRNA ist, und vorzugsweise außerdem mindestens einen Histon-Stem-Loop zur Erhöhung der Proteinproduktion von einem Nukleinsäuremolekül umfasst.

**29.** Kit oder Kit von Teilen, umfassend ein artifizielles Nukleinsäuremolekül gemäß irgendeinem der Ansprüche 1 bis 19, den Vektor gemäß irgendeinem der Ansprüche 20 bis 23, die Zelle gemäß Anspruch 24 und/oder die pharmazeutische Zusammensetzung gemäß Anspruch 25, wobei das Kit oder Kit von Teilen vorzugsweise außerdem Gebrauchsanweisungen, Zellen für die Transfektion, ein Adjuvans, Mittel zur Verabreichung der pharmazeutischen Zusammensetzung, einen pharmazeutisch akzeptablen Träger und/oder eine pharmazeutisch akzeptable Lösung zur Auflösung oder Verdünnung des artifiziellen Nukleinsäuremoleküls, des Vektors oder der pharmazeutischen Zusammensetzung umfasst.

## Revendications

**1.** Molécule d'acide nucléique artificielle comprenant :

a. au moins un élément de région non traduite en 5' (élément 5'UTR) qui comprend ou est constitué d'une séquence d'acide nucléique qui est dérivée de la 5'UTR d'un gène de protéine ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité Vlc de la cytochrome c oxydase (COX6C), ou d'un gène de la N-acylsphingosine amidohydrolase (céramidase acide) 1 (ASAH1) ou d'un fragment fonctionnel ou d'un variant fonctionnel de celle-ci,
dans lequel le fragment ou variant fonctionnel remplit au moins une fonction de la 5'UTR d'origine naturelle d'un gène de protéine ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité Vlc de la cytochrome c oxydase (COX6C), ou d'un gène de la N-acylsphingosine amidohydrolase (céramidase acide) 1 (ASAH1), dans lequel la au moins une fonction est la stabilisation de l'ARNm

et/ou la stabilisation et/ou la prolongation de la production de protéines à partir d'un ARNm ; et
b. au moins un cadre de lecture ouvert (ORF),

dans laquelle l'élément 5'UTR et le cadre de lecture ouvert sont hétérologues.

2. Molécule d'acide nucléique artificielle selon la revendication 1, comprenant en outre :
c. au moins une tige-boucle d'histone.

3. Molécule d'acide nucléique artificielle selon la revendication 1 ou 2, dans laquelle l'élément 5'UTR est approprié pour augmenter la production de protéines à partir de la molécule d'acide nucléique artificielle.

4. Molécule d'acide nucléique artificielle selon l'une quelconque des revendications 1 à 3, dans laquelle l'élément 5'UTR ne comprend pas de motif TOP, de préférence dans laquelle la séquence d'acide nucléique, qui est dérivée d'une 5'UTR d'un gène de protéine ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité Vlc de la cytochrome c oxydase (COX6C), ou d'un gène de la N-acylsphingosine amidohydrolase (céramidase acide) 1 (ASAH1) ou d'un fragment fonctionnel ou d'un variant fonctionnel de celle-ci, de préférence l'élément 5'UTR, commence à son extrémité 5' avec un nucléotide localisé en position 1, 2, 3, 4, 5, 6, 7, 8, 9 ou 10 en aval de l'étendue de polypyrimidine.

5. Molécule d'acide nucléique artificielle selon l'une quelconque des revendications 1 à 4, dans laquelle la séquence d'acide nucléique, qui est dérivée d'une 5'UTR d'un gène de protéine ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité Vlc de la cytochrome c oxydase (COX6C), ou d'un gène de la N-acylsphingosine amidohydrolase (céramidase acide) 1 (ASAH1) ou d'un fragment fonctionnel ou d'un variant fonctionnel de celle-ci, de préférence l'élément 5'UTR, se termine à son extrémité 3' avec un nucléotide localisé en position 1, 2, 3, 4, 5, 6, 7, 8, 9 ou 10 en amont du codon de départ du gène dont elle est dérivée.

6. Molécule d'acide nucléique artificielle selon l'une quelconque des revendications 1 à 5, dans laquelle l'élément 5'UTR ne comprend pas de codon de départ ni de cadre de lecture ouvert.

7. Molécule d'acide nucléique artificielle selon l'une quelconque des revendications 2 à 6, dans laquelle l'au moins une tige-boucle d'histone est choisie parmi les formules (I) ou (II) suivantes :

formule (I) (séquence de tige-boucle sans éléments bordant la tige) :

$$\underbrace{[N_{0-2}GN_{3-5}]}_{\text{tige1}}\ \underbrace{[N_{0-4}(U/T)N_{0-4}]}_{\text{boucle}}\ \underbrace{[N_{3-5}CN_{0-2}]}_{\text{tige2}}$$

formule (II) (séquence de tige-boucle avec éléments bordant la tige) :

$$\underbrace{N_{1-6}}_{\text{élément bordant la tige1}}\ \underbrace{[N_{0-2}GN_{3-5}]}_{\text{tige1}}\ \underbrace{[N_{0-4}(U/T)N_{0-4}]}_{\text{boucle}}\ \underbrace{[N_{3-5}CN_{0-2}]}_{\text{tige2}}\ \underbrace{N_{1-6}}_{\text{élément bordant la tige2}}$$

dans lesquelles :

les éléments bordant la tige1 ou la tige2 $N_{1-6}$ sont une séquence consécutive de 1 à 6, de préférence de 2 à 6, plus préférablement de 2 à 5, encore plus préférablement de 3 à 5, de manière préférée entre toutes de 4 à 5 ou 5 N, dans laquelle chaque N est choisi indépendamment d'un autre choisi parmi un nucléotide choisi parmi A, U, T, G et C, ou un analogue nucléotidique de celui-ci ;
tige1 [$N_{0-2}GN_{3-5}$] est le complémentaire inverse ou partiellement le complémentaire inverse de l'élément tige2, et est une séquence consécutive de 5 à 7 nucléotides ;
dans laquelle $N_{0-2}$ est une séquence consécutive de 0 à 2, de préférence de 0 à 1, plus préférablement de 1 N, dans laquelle chaque N est choisi indépendamment d'un autre choisi parmi un nucléotide choisi parmi A, U, T, G et C ou un analogue nucléotidique de celui-ci ;
dans laquelle $N_{3-5}$ est une séquence consécutive de 3 à 5, de préférence de 4 à 5, plus préférablement de 4 N, dans laquelle chaque N est choisi indépendamment d'un autre choisi parmi un nucléotide choisi parmi A, U, T, G et C ou un analogue nucléotidique de celui-ci, et
dans laquelle G est la guanosine ou un analogue de celle-ci, et peut éventuellement être remplacé par une cytidine ou un analogue de celle-ci, à condition que son nucléotide complémentaire de cytidine dans la tige2 soit remplacé par une guanosine ;
la séquence de boucle [$N_{0-4}$(U/T)$N_{0-4}$] est localisée entre les éléments tige1 et tige2, et est une séquence consécutive de 3 à 5 nucléotides, plus préférablement de 4 nucléotides ;
dans laquelle chaque $N_{0-4}$ est indépendant des autres une séquence consécutive de 0 à 4, de préférence de 1 à 3, plus préférablement de 1 à 2 N, dans laquelle chaque N est choisi indépendamment d'un autre choisi parmi un nucléotide choisi parmi A, U, T, G et C ou un analogue nucléotidique de celui-ci ; et
dans lesquelles U/T représente l'uridine, ou éventuellement la thymidine ;
tige2 [$N_{3-5}CN_{0-2}$] est le complémentaire inverse ou partiellement le complémentaire inverse de l'élément tige1, et est une séquence consécutive de 5 à 7 nucléotides ;
dans laquelle $N_{3-5}$ est une séquence consécutive de 3 à 5, de préférence de 4 à 5, plus préférablement de 4 N, dans laquelle chaque N est choisi indépendamment d'un autre choisi parmi un nucléotide choisi parmi A, U, T, G et
C ou un analogue nucléotidique de celui-ci ;
dans laquelle $N_{0-2}$ est une séquence consécutive de 0 à 2, de préférence de 0 à 1, plus préférablement de 1 N, dans laquelle chaque N est choisi indépendamment d'un autre choisi parmi un nucléotide choisi parmi A, U, T, G et C ou un analogue nucléotidique de celui-ci ; et
dans laquelle C est la cytidine ou un analogue de celle-ci, et peut éventuellement être remplacé par une guanosine ou un analogue de celle-ci, à condition que son nucléotide complémentaire de guanosine dans la tige1 soit remplacé par la cytidine ;
dans laquelle
la tige1 et la tige2 sont aptes à effectuer un appariement des bases l'une avec l'autre
formant une séquence complémentaire inverse, dans laquelle un appariement de bases peut se produire entre la tige1 et la tige2, ou
formant une séquence partiellement complémentaire inverse, dans laquelle un appariement des bases incomplet peut se produire entre la tige1 et la tige2.

**8.** Molécule d'acide nucléique artificielle selon l'une quelconque des revendications 2 à 7, dans laquelle l'au moins une tige-boucle d'histone est choisie parmi au moins l'une des formules (Ia) ou (IIa) suivantes :

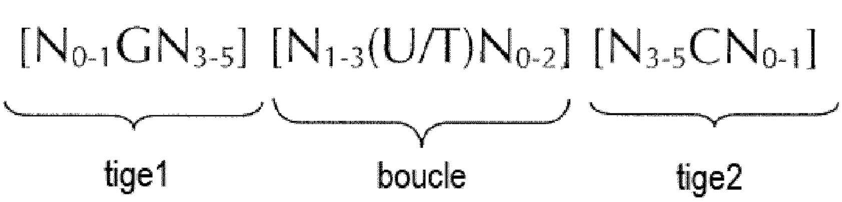


formule (Ia) (séquence de tige-boucle sans éléments bordant la tige)

$$\underbrace{N_{2-5}}_{\text{élément bordant la tige1}}\ \underbrace{[N_{0-1}GN_{3-5}]}_{\text{tige1}}\ \underbrace{[N_{1-3}(U/T)N_{0-2}]}_{\text{boucle}}\ \underbrace{[N_{3-5}CN_{0-1}]}_{\text{tige2}}\ \underbrace{N_{2-5}}_{\text{élément bordant la tige2}}$$

formule (IIa) (séquence de tige-boucle avec éléments bordant la tige)

**9.** Molécule d'acide nucléique artificielle selon l'une quelconque des revendications 1 à 8, comprenant en outre d. une séquence poly(A) et/ou un signal de polyadénylation.

**10.** Molécule d'acide nucléique artificielle selon l'une quelconque des revendications 1 à 9, comprenant en outre : e. une séquence poly(C).

**11.** Molécule d'acide nucléique artificielle selon l'une quelconque des revendications 1 à 10, comprenant en outre : f. au moins un élément 3'UTR.

**12.** Molécule d'acide nucléique artificielle selon la revendication 11, dans laquelle l'au moins un élément 3'UTR comprend ou est constitué d'une séquence d'acide nucléique, qui est dérivée d'une 3'UTR d'un gène fournissant un ARNm stable ou d'un variant de la 3'UTR d'un gène fournissant un ARNm stable.

**13.** Molécule d'acide nucléique artificielle selon l'une quelconque des revendications 1 à 12, dans laquelle l'élément 5'UTR comprend ou est constitué d'une séquence d'acide nucléique qui est dérivée de la 5'UTR d'un gène de protéine ribosomique Large 32 (L32) de vertébré, d'un gène de protéine ribosomique Large 35 (RPL35) de vertébré, d'un gène de protéine ribosomique Large 21 (RPL21) de vertébré, d'une ATP synthase de vertébré, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4) de vertébré, d'un gène 1 induit par les androgènes (AIG1) de vertébré, d'un gène de sous-unité VIc de la cytochrome c oxydase (COX6C) de vertébré, d'un gène de la N-acylsphingosine amidohydrolase (céramidase acide) 1 (ASAH1) de vertébré ou d'un fragment fonctionnel ou d'un variant fonctionnel de celle-ci, plus préférablement issue de la 5'UTR d'un gène de protéine ribosomique Large 32 (L32) de mammifère, d'un gène de protéine ribosomique Large 35 (RPL35) de mammifère, d'un gène de protéine ribosomique Large 21 (RPL21) de mammifère, d'une ATP synthase de mammifère, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4) de mammifère, d'un gène 1 induit par les androgènes (AIG1) de mammifère, d'un gène de sous-unité VIc de la cytochrome c oxydase (COX6C) de mammifère, d'un gène de la N-acylsphingosine amidohydrolase (céramidase acide) 1 (ASAH1) de mammifère ou d'un fragment fonctionnel ou d'un variant fonctionnel de celle-ci, de manière préférée entre toutes issue de la 5'UTR d'un gène de la protéine ribosomique Large 32 (RPL32) humaine, d'un gène de la protéine ribosomique Large 35 (RPL35) humaine, d'un gène de la protéine ribosomique Large 21 (RPL21) humaine, d'une ATP synthase humaine, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4) humaine, d'un gène 1 induit par les androgènes (AIG1) humain, d'un gène de la sous-unité VIc de la cytochrome c oxydase (COX6C) humaine, d'un gène de la N-acylsphingosine amidohydrolase (céramidase acide) 1 (ASAH1) humaine ou d'un fragment fonctionnel ou d'un variant fonctionnel de celle-ci, dans laquelle de préférence l'élément 5'UTR, de préférence la molécule d'acide nucléique artificielle ne comprend pas le 5'TOP dudit gène.

**14.** Molécule d'acide nucléique artificielle selon l'une quelconque des revendications 2 à 13, dans laquelle l'au moins une tige-boucle d'histone comprend ou est constituée d'une séquence d'acide nucléique choisie dans le groupe constitué des SEQ ID NO. 1391 à 1433, de préférence dans le groupe constitué des SEQ ID NO. 1403 à 1433.

**15.** Molécule d'acide nucléique artificielle selon l'une quelconque des revendications 2 à 14, dans laquelle la tige-boucle d'histone comprend ou est constituée d'une séquence d'acide nucléique ayant une identité de séquence d'au moins environ 80 %, de préférence d'au moins environ 85 %, plus préférablement d'au moins environ 90 %, encore plus préférablement d'au moins environ 95 % à la séquence selon SEQ ID NO. 1433 ou à la séquence d'ARN correspondante, dans laquelle de préférence les positions 6, 13 et 20 de la séquence ayant une identité de séquence

d'au moins environ 80 %, de préférence d'au moins environ 85 %, plus préférablement d'au moins environ 90 %, encore plus préférablement d'au moins environ 95 % à la séquence selon SEQ ID NO. 1433 ou à la séquence d'ARN correspondante sont conservées, c'est-à-dire sont identiques aux nucléotides en positions 6, 13 et 20 de SEQ ID NO. 1433 ou aux nucléotides d'ARN correspondants.

**16.** Molécule d'acide nucléique artificielle selon l'une quelconque des revendications 11 à 15, dans laquelle l'élément de la 3'UTR comprend ou est constitué d'une séquence d'acide nucléique dérivée d'une 3'UTR d'un gène choisi dans le groupe constitué d'un gène d'albumine, d'un gène d'a-globine, d'un gène de β-globine, d'un gène de tyrosine hydroxylase, d'un gène de lipoxygénase et d'un gène de collagène alpha, ou d'un variant d'une 3'UTR d'un gène choisi dans le groupe constitué d'un gène d'albumine, d'un gène d'a-globine, d'un gène de β-globine, d'un gène de tyrosine hydroxylase, d'un gène de lipoxygénase et d'un gène de collagène alpha, dans laquelle l'au moins un élément 3'UTR comprend ou est constituée de préférence de

une séquence d'acide nucléique qui est dérivée de la 3'UTR d'un gène d'albumine de vertébré ou d'un variant de celle-ci, de préférence de la 3'UTR d'un gène d'albumine de mammifère ou d'un variant de celle-ci, plus préférablement de la 3'UTR d'un gène d'albumine humaine ou d'un variant de celle-ci, encore plus préférablement issue de la 3'UTR du gène de l'albumine humaine selon le numéro d'accès GenBank NM_000477.5 ou issue d'un variant de celle-ci ; ou
une séquence d'acide nucléique, qui est dérivée de la 3'UTR d'un gène d'a-globine de vertébré ou d'un variant de celle-ci, de préférence de la 3'UTR d'un gène d'a-globine de mammifère ou d'un variant de celle-ci, plus préférablement de la 3'UTR d'un gène de l'a-globine humaine ou d'un variant de celle-ci.

**17.** Molécule d'acide nucléique artificielle selon l'une quelconque des revendications 11 à 16, dans laquelle l'au moins un élément 3'UTR comprend ou est constitué d'une séquence d'acide nucléique qui a une identité d'au moins environ 80 %, plus préférablement d'au moins environ 90 %, encore plus préférablement d'au moins environ 95 %, encore plus préférablement d'au moins environ 99 % à une séquence d'acide nucléique choisie parmi les SEQ ID NO. 1369 à 1377 et 1434 ou à une séquence d'ARN correspondante, ou dans laquelle l'au moins un élément 3'UTR comprend ou est constitué d'un fragment fonctionnel d'une séquence d'acide nucléique qui a une identité d'au moins environ 80 %, plus préférablement d'au moins environ 90 %, encore plus préférablement d'au moins environ 95 %, encore plus préférablement d'au moins environ 99 % à une séquence d'acide nucléique choisie parmi les SEQ ID NO. 1369 à 1377 et 1434 ou à une séquence d'ARN correspondante.

**18.** Molécule d'acide nucléique artificielle selon l'une quelconque des revendications 1 à 17, dans laquelle la molécule d'acide nucléique artificielle, de préférence le cadre de lecture ouvert, est au moins partiellement modifié(e) par G/C, de préférence dans laquelle la teneur en G/C du cadre de lecture ouvert est augmentée par rapport au cadre de lecture ouvert de type sauvage.

**19.** Molécule d'acide nucléique artificielle selon l'une quelconque des revendications 1 à 18, qui est un ARN, de préférence une molécule d'ARNm.

**20.** Vecteur comprenant :

a. au moins un élément de région non traduite en 5' (élément 5'UTR) qui comprend ou est constitué d'une séquence d'acide nucléique qui est dérivée de la 5'UTR d'un gène de protéine ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité VIc de la cytochrome c oxydase (COX6C), ou d'un gène de la N-acylsphingosine amidohydrolase (céramidase acide) 1 (ASAH1) ou d'un fragment fonctionnel ou d'un variant fonctionnel de celle-ci,
dans lequel le fragment ou variant fonctionnel remplit au moins une fonction de la 5'UTR d'origine naturelle d'un gène de protéine ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité VIc de la cytochrome c oxydase (COX6C), ou d'un gène de la N-acylsphingosine amidohydrolase (céramidase acide) 1 (ASAH1), dans laquelle l'au moins une fonction est la stabilisation de l'ARNm et/ou la stabilisation et/ou la prolongation de la production de protéines à partir d'un ARNm ; et

b. au moins un cadre de lecture ouvert (ORF) et/ou au moins un site de clonage,

dans lequel l'élément 5'UTR et le cadre de lecture ouvert sont hétérologues.

**21.** Vecteur selon la revendication 20, comprenant en outre :
c. au moins une tige-boucle d'histone.

**22.** Vecteur selon la revendication 20 ou 21, qui est un vecteur d'ADN, de préférence un vecteur plasmidique, ou un vecteur viral.

**23.** Vecteur selon l'une quelconque des revendications 20 à 22, qui comprend ou code pour une molécule d'acide nucléique artificielle selon l'une quelconque des revendications 1 à 19.

**24.** Cellule comprenant la molécule d'acide nucléique artificielle selon l'une quelconque des revendications 1 à 19 ou le vecteur selon l'une quelconque des revendications 20 à 23, dans laquelle la cellule est de préférence une cellule de mammifère, plus préférablement une cellule isolée d'un sujet mammifère, de manière préférée entre toutes d'un sujet humain.

**25.** Composition pharmaceutique comprenant la molécule d'acide nucléique artificielle selon l'une quelconque des revendications 1 à 19, le vecteur selon l'une quelconque des revendications 20 à 23, ou la cellule selon la revendication 24, comprenant éventuellement, en outre, un ou plusieurs diluants et/ou excipients pharmaceutiquement acceptables et/ou un ou plusieurs adjuvants.

**26.** Molécule d'acide nucléique artificielle selon l'une quelconque des revendications 1 à 19, vecteur selon l'une quelconque des revendications 20 à 23, cellule selon la revendication 24, ou composition pharmaceutique selon la revendication 25 pour une utilisation comme médicament, de préférence pour une utilisation comme vaccin ou pour une utilisation en thérapie génique.

**27.** Procédé in vitro pour augmenter la production de protéines à partir d'une molécule d'acide nucléique artificielle, comprenant l'étape consistant à fournir à la molécule d'acide nucléique artificielle avec

i. au moins un élément de région non traduite en 5' (élément 5'UTR) qui comprend ou est constitué d'une séquence d'acide nucléique qui est dérivée de la 5'UTR d'un gène de protéine ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité VIc de la cytochrome c oxydase (COX6C), ou d'un gène de la N-acylsphingosine amidohydrolase (céramidase acide) 1 (ASAH1) ou d'un fragment fonctionnel ou d'un variant fonctionnel de celle-ci,
dans lequel le fragment ou variant fonctionnel remplit au moins une fonction de la 5'UTR d'origine naturelle d'un gène de protéine ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité VIc de la cytochrome c oxydase (COX6C), ou d'un gène de la N-acylsphingosine amidohydrolase (céramidase acide) 1 (ASAH1), dans laquelle la au moins une fonction est la stabilisation de l'ARNm et/ou la stabilisation et/ou la prolongation de la production de protéines à partir d'un ARNm,
ii. de préférence au moins une tige-boucle d'histone ; et
iii. éventuellement, une séquence poly(A) et/ou un signal de polyadénylation.

**28.** Utilisation in vitro d'un élément 5'UTR, qui comprend ou est constitué d'une séquence d'acide nucléique qui est dérivée de la 5'UTR d'un gène de protéine ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité VIc de la cytochrome c oxydase (COX6C), ou d'un gène de la N-acylsphingosine amidohydrolase (céramidase acide) 1 (ASAH1) ou d'un fragment fonctionnel ou d'un variant fonctionnel de celle-ci, dans lequel le fragment ou variant fonctionnel remplit au moins une fonction de la 5'UTR d'origine naturelle d'un gène de protéine

ribosomique Large 32 (RPL32), d'un gène de protéine ribosomique Large 35 (RPL35), d'un gène de protéine ribosomique Large 21 (RPL21), d'une ATP synthase, du transport de H+, d'un complexe mitochondrial F1, d'une sous-unité alpha 1, d'un gène de muscle cardiaque (ATP5A1), d'un gène 15 de l'hydroxystéroïde (17-bêta) déshydrogénase 4 (HSD17B4), d'un gène 1 induit par les androgènes (AIG1), d'un gène de sous-unité VIc de la cytochrome c oxydase (COX6C), ou d'un gène de la N-acylsphingosine amidohydrolase (céramidase acide) 1 (ASAH1), dans laquelle la au moins une fonction est la stabilisation de l'ARNm et/ou la stabilisation et/ou la prolongation de la production de protéines à partir d'un ARNm, et de préférence comprend en outre au moins une tige-boucle d'histone pour augmenter la production de protéine à partir d'une molécule d'acide nucléique.

**29.** Kit ou kit d'éléments comprenant une molécule d'acide nucléique artificielle selon l'une quelconque des revendications 1 à 19, le vecteur selon l'une quelconque des revendications 20 à 23, la cellule selon la revendication 24, et/ou la composition pharmaceutique selon la revendication 25, le kit ou kit d'éléments de préférence comprend en outre des instructions d'utilisation, des cellules pour la transfection, un adjuvant, un moyen pour l'administration de la composition pharmaceutique, un support pharmaceutiquement acceptable et/ou une solution pharmaceutiquement acceptable pour la dissolution ou la dilution de la molécule d'acide nucléique artificielle, du vecteur ou de la composition pharmaceutique.

| | | | | | | Stem 1 | | | | | | Loop | | | | Stem 2 | | | | | | | | | | |
|---|---|---|---|---|---|---|---|---|---|---|---|---|---|---|---|---|---|---|---|---|---|---|---|---|---|---|
| | | | | | | < | < | < | < | < | < | • | • | • | • | > | > | > | > | > | > | | | | | |
| # A | 2224 | 1586 | 3075 | 2872 | 1204 | 184 | 0 | 13 | 12 | 9 | 1 | 47 | 59 | 0 | 675 | 3818 | 195 | 1596 | 523 | 0 | 14 | 3727 | 61 | 771 | 2012 | 2499 |
| # T | 172 | 188 | 47 | 205 | 19 | 6 | 0 | 569 | 1620 | 199 | 3947 | 3830 | 3704 | 4001 | 182 | 1 | 21 | 15 | 11 | 0 | 179 | 8 | 64 | 557 | 201 | 690 |
| # C | 1557 | 2211 | 875 | 918 | 2675 | 270 | 0 | 3394 | 2342 | 3783 | 51 | 119 | 227 | 0 | 3140 | 7 | 50 | 31 | 16 | 4001 | 3543 | 154 | 3870 | 2636 | 1744 | 674 |
| # G | 25 | 16 | 4 | 6 | 23 | 3541 | 4001 | 25 | 27 | 10 | 2 | 5 | 11 | 0 | 4 | 175 | 3735 | 2359 | 3451 | 0 | 265 | 112 | 4 | 37 | 43 | 130 |
| Cons | N* | N* | N | N | N | N | G | N | N | N | N | N | N | T | N | N | N | N | N | C | N | N | N | N* | N* | N* |
| 99% | H* | H* | H | H | V | V | G | Y | Y | Y | Y | H | H | T | H | R | V | V | R | C | B | V | H | H* | N* | N* |
| 95% | M* | H* | M | H | M | S | G | Y | Y | Y | T | T | Y | T | M | A | R | R | R | C | S | M | C | H* | H* | H* |
| 90% | M* | M* | M | M | M | S | G | Y | Y | C | T | T | T | T | M | A | G | R | R | C | S | A | C | H* | M* | H* |

Figure 1

Stem 1 Loop Stem 2

| | | | | | | | | | | | | | | | | | | | | | | | | | | |
|---|---|---|---|---|---|---|---|---|---|---|---|---|---|---|---|---|---|---|---|---|---|---|---|---|---|---|
| | | | | | | < | < | < | < | < | < | • | • | • | • | > | > | > | > | > | > | | | | | |
| #A | 52 | 32 | 71 | 82 | 76 | 13 | 0 | 12 | 12 | 9 | 1 | 46 | 3 | 0 | 75 | 82 | 53 | 79 | 20 | 0 | 4 | 94 | 17 | 35 | 74 | 56 |
| #T | 20 | 32 | 37 | 21 | 8 | 3 | 0 | 21 | 85 | 58 | 86 | 70 | 65 | 131 | 28 | 1 | 17 | 13 | 10 | 0 | 15 | 7 | 31 | 32 | 20 | 28 |
| #C | 45 | 59 | 20 | 25 | 38 | 0 | 0 | 86 | 8 | 54 | 42 | 13 | 58 | 0 | 27 | 2 | 6 | 31 | 10 | 131 | 112 | 5 | 82 | 50 | 30 | 40 |
| #G | 14 | 8 | 3 | 3 | 9 | 115 | 131 | 12 | 26 | 10 | 2 | 2 | 5 | 0 | 1 | 46 | 55 | 8 | 91 | 0 | 0 | 25 | 1 | 6 | 7 | 7 |
| Cons | N* | N* | N | N | N | D | G | N | N | N | N | N | N | T | N | N | N | N | N | C | H | N | N | N* | N* | N* |
| 99% | N* | N* | N | N | N | D | G | N | N | N | B | N | N | T | H | V | N | N | N | C | H | N | H | N* | N* | N* |
| 95% | N* | N* | H | H | N | R | G | N | N | N | Y | H | B | T | H | R | D | N | N | C | Y | D | H | H* | N* | N* |
| 90% | N* | H* | H | H | V | R | G | N | D | B | Y | H | Y | T | H | R | D | H | N | C | Y | R | H | H* | H* | H* |

Figure 2

| | | | | | | Stem 1 | | | | | | Loop | | | | Stem 2 | | | | | | | | | | |
|---|---|---|---|---|---|---|---|---|---|---|---|---|---|---|---|---|---|---|---|---|---|---|---|---|---|---|
| | | | | | | < | < | < | < | < | < | • | • | • | • | > | > | > | > | > | > | | | | | |
| #A | 2172 | 1554 | 3004 | 2790 | 1208 | 171 | 0 | 1 | 0 | 0 | 0 | 1 | 56 | 0 | 600 | 3736 | 142 | 1517 | 503 | 0 | 10 | 3633 | 44 | 736 | 1938 | 2443 |
| #T | 152 | 156 | 10 | 184 | 11 | 3 | 0 | 548 | 1535 | 141 | 3861 | 3760 | 3639 | 3870 | 154 | 0 | 4 | 2 | 1 | 0 | 164 | 1 | 33 | 525 | 181 | 662 |
| #C | 1512 | 2152 | 855 | 893 | 2637 | 270 | 0 | 3308 | 2334 | 3729 | 9 | 106 | 169 | 0 | 3113 | 5 | 44 | 0 | 6 | 3870 | 3431 | 149 | 3788 | 2578 | 1714 | 634 |
| #G | 11 | 6 | 1 | 3 | 14 | 3426 | 3870 | 13 | 1 | 0 | 0 | 3 | 6 | 0 | 3 | 129 | 3680 | 2351 | 3360 | 0 | 265 | 87 | 3 | 31 | 36 | 131 |
| Cons | N* | N* | N | N | N | N | G | N | B | Y | Y | N | N | T | N | V | N | D | N | C | N | N | N | N* | N* | N* |
| 99% | H* | H* | M | H | M | V | G | Y | Y | Y | T | Y | H | T | H | R | V | R | R | C | B | V | M | H* | H* | N* |
| 95% | M* | M* | M | M | M | S | G | Y | Y | C | T | T | Y | T | M | A | G | R | R | C | S | M | C | H* | H* | H* |
| 90% | M* | M* | M | M | M | S | G | Y | Y | C | T | T | T | T | M | A | G | R | R | C | S | A | C | H* | M* | H* |

Figure 3

| | | | | | | Stem 1 | | | | | | Loop | | | | Stem 2 | | | | | | | | | | |
|---|---|---|---|---|---|---|---|---|---|---|---|---|---|---|---|---|---|---|---|---|---|---|---|---|---|---|
| | | | | | | < | < | < | < | < | < | • | • | • | • | > | > | > | > | > | > | | | | | |
| # A | 661 | 146 | 1315 | 1323 | 920 | 8 | 0 | 1 | 0 | 0 | 0 | 1 | 4 | 0 | 441 | 1333 | 0 | 1199 | 21 | 0 | 1 | 1126 | 26 | 81 | 380 | 960 |
| # T | 63 | 121 | 2 | 2 | 6 | 2 | 0 | 39 | 1217 | 2 | 1331 | 1329 | 1207 | 1333 | 30 | 0 | 1 | 0 | 1 | 0 | 2 | 1 | 22 | 91 | 91 | 12 |
| # C | 601 | 1062 | 16 | 6 | 403 | 1 | 0 | 1293 | 116 | 1331 | 2 | 0 | 121 | 0 | 862 | 0 | 2 | 0 | 0 | 1333 | 1328 | 128 | 1284 | 1143 | 834 | 361 |
| # G | 8 | 4 | 0 | 2 | 4 | 1322 | 1333 | 0 | 0 | 0 | 0 | 3 | 1 | 0 | 0 | 0 | 1330 | 134 | 1311 | 0 | 2 | 70 | 1 | 18 | 28 | 0 |
| Cons | N* | N* | H | N | N | N | G | H | Y | Y | Y | D | N | T | H | A | B | R | D | C | N | N | N | N* | N* | H* |
| 99% | H* | H* | M | A | M | G | G | Y | Y | C | T | T | Y | T | H | A | G | R | R | C | C | V | H | N* | N* | M* |
| 95% | H* | H* | A | A | M | G | G | C | Y | C | T | T | Y | T | M | A | G | R | G | C | C | V | C | H* | H* | M* |
| 90% | M* | M* | A | A | M | G | G | C | T | C | T | T | T | T | M | A | G | R | G | C | C | M | C | Y* | M* | M* |

Figure 4

| | #A | #T | #C | #G | Cons | 99% | 95% | 90% | |
|---|---|---|---|---|---|---|---|---|---|
| | 10 | 8 | 62 | 4 | N* | N* | H* | H* | |
| | 17 | 6 | 61 | 0 | H* | H* | H* | M* | |
| | 84 | 0 | 0 | 0 | A | A | A | A | |
| | 84 | 0 | 0 | 0 | A | A | A | A | |
| | 76 | 2 | 6 | 0 | H | H | M | A | |
| < | 1 | 2 | 0 | 81 | D | D | G | G | |
| < | 0 | 0 | 0 | 84 | G | G | G | G | |
| < | 1 | 1 | 82 | 0 | H | H | C | C | |
| < | 0 | 67 | 17 | 0 | Y | Y | Y | Y | Stem 1 |
| < | 0 | 0 | 84 | 0 | C | C | C | C | |
| < | 0 | 84 | 0 | 0 | T | T | T | T | |
| • | 1 | 80 | 0 | 3 | D | D | T | T | |
| • | 0 | 81 | 3 | 0 | Y | Y | T | T | Loop |
| • | 0 | 84 | 0 | 0 | T | T | T | T | |
| • | 12 | 5 | 67 | 0 | H | H | H | M | |
| > | 84 | 0 | 0 | 0 | A | A | A | A | |
| > | 0 | 0 | 1 | 83 | S | S | G | G | |
| > | 65 | 0 | 0 | 19 | R | R | R | R | Stem 2 |
| > | 3 | 0 | 0 | 81 | R | R | G | G | |
| > | 0 | 0 | 84 | 0 | C | C | C | C | |
| > | 0 | 0 | 84 | 0 | C | C | C | C | |
| | 69 | 0 | 5 | 10 | V | V | V | R | |
| | 5 | 4 | 75 | 0 | H | H | M | M | |
| | 0 | 25 | 57 | 2 | B* | B* | Y* | Y* | |
| | 10 | 24 | 44 | 6 | N* | N* | N* | H* | |
| | 64 | 3 | 17 | 0 | H* | H* | M* | M* | |

Figure 5

nucleotide sequence of PpLuc(GC) – ag – A64

GGGAGAAAGCTTGAGG*ATGGAGGACGCCAAGAACATCAAGAAGGGCCCGGCGCCCTTCTA*
*CCCGCTGGAGGACGGGACCGCCGGCGAGCAGCTCCACAAGGCCATGAAGCGGTACGCCCT*
*GGTGCCGGGCACGATCGCCTTCACCGACGCCCACATCGAGGTCGACATCACCTACGCGGA*
*GTACTTCGAGATGAGCGTGCGCCTGGCCGAGGCCATGAAGCGGTACGGCCTGAACACCAA*
*CCACCGGATCGTGGTGTGCTCGGAGAACAGCCTGCAGTTCTTCATGCCGGTGCTGGGCGC*
*CCTCTTCATCGGCGTGGCCGTCGCCCCGGCGAACGACATCTACAACGAGCGGGAGCTGCT*
*GAACAGCATGGGGATCAGCCAGCCGACCGTGGTGTTCGTGAGCAAGAAGGGCCTGCAGAA*
*GATCCTGAACGTGCAGAAGAAGCTGCCCATCATCCAGAAGATCATCATCATGGACAGCAA*
*GACCGACTACCAGGGCTTCCAGTCGATGTACACGTTCGTGACCAGCCACCTCCCGCCGGG*
*CTTCAACGAGTACGACTTCGTCCCGGAGAGCTTCGACCGGGACAAGACCATCGCCCTGAT*
*CATGAACAGCAGCGGCAGCACCGGCCTGCCGAAGGGGGTGGCCCTGCCGCACCGGACCGC*
*CTGCGTGCGCTTCTCGCACGCCCGGGACCCCATCTTCGGCAACCAGATCATCCCGGACAC*
*CGCCATCCTGAGCGTGGTGCCGTTCCACCACGGCTTCGGCATGTTCACGACCCTGGGCTA*
*CCTCATCTGCGGCTTCCGGGTGGTCCTGATGTACCGGTTCGAGGAGGAGCTGTTCCTGCG*
*GAGCCTGCAGGACTACAAGATCCAGAGCGCGCTGCTCGTGCCGACCCTGTTCAGCTTCTT*
*CGCCAAGAGCACCCTGATCGACAAGTACGACCTGTCGAACCTGCACGAGATCGCCAGCGG*
*GGGCGCCCCGCTGAGCAAGGAGGTGGGCGAGGCCGTGGCCAAGCGGTTCCACCTCCCGGG*
*CATCCGCCAGGGCTACGGCCTGACCGAGACCACGAGCGCGATCCTGATCACCCCCGAGGG*
*GGACGACAAGCCGGGCGCCGTGGGCAAGGTGGTCCCGTTCTTCGAGGCCAAGGTGGTGGA*
*CCTGGACACCGGCAAGACCCTGGGCGTGAACCAGCGGGGCGAGCTGTGCGTGCGGGGGCC*
*GATGATCATGAGCGGCTACGTGAACAACCCGGAGGCCACCAACGCCCTCATCGACAAGGA*
*CGGCTGGCTGCACAGCGGCGACATCGCCTACTGGGACGAGGACGAGCACTTCTTCATCGT*
*CGACCGGCTGAAGTCGCTGATCAAGTACAAGGGCTACCAGGTGGCGCCGGCCGAGCTGGA*
*GAGCATCCTGCTCCAGCACCCCAACATCTTCGACGCCGGCGTGGCCGGGCTGCCGGACGA*
*CGACGCCGGCGAGCTGCCGGCCGCGGTGGTGGTGCTGGAGCACGGCAAGACCATGACGGA*
*GAAGGAGATCGTCGACTACGTGGCCAGCCAGGTGACCACCGCCAAGAAGCTGCGGGGCGG*
*CGTGGTGTTCGTGGACGAGGTCCCGAAGGGCCTGACCGGGAAGCTCGACGCCCGGAAGAT*
*CCGCGAGATCCTGATCAAGGCCAAGAAGGGCGGCAAGATCGCCGTGTAA*GACTAGTTATA
AGACTGACTAGCCCGATGGGCCTCCCAACGGGCCCTCCTCCCCTCCTTGCACCGAGATTA
ATAGATCTAAAAAAAAAAAAAAAAAAAAAAAAAAAAAAAAAAAAAAAAAAAAAAAA
AAAAAAAAAAAA

Figure 6

nucleotide sequence of RPL32 – PpLuc(GC) – ag – A64 – C30 – histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAGATCTAAAAAAAAAAAAAA
AAAAAAAAAAAAAAAAAAAAAAAAAAAAAAAAAAAAAAAAAAAAAAAAAATGCATCCCCC
CCCCCCCCCCCCCCCCCCCCCCCCCCAAAGGCTCTTTTCAGAGCCACCAGAATT

Figure 7

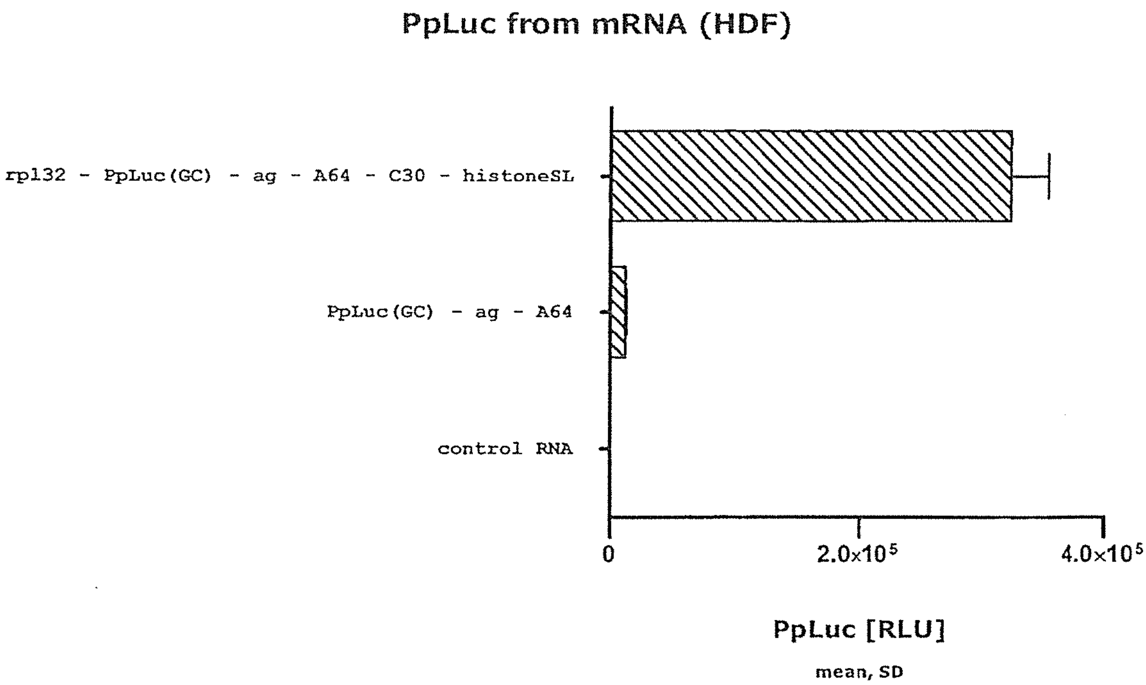
PpLuc from mRNA (HDF)
rpl32 - PpLuc(GC) - ag - A64 - C30 - histoneSL
PpLuc(GC) - ag - A64
control RNA
0
2.0×10⁵
4.0×10⁵
PpLuc [RLU]
mean, SD

Figure 8

nucleotide sequence of PpLuc(GC) – ag – A64 – histoneSL

GGGAGAAAGCTTGAGG*ATGGAGGACGCCAAGAACATCAAGAAGGGCCCGGCGCCCTTCTA*
*CCCGCTGGAGGACGGGACCGCCGGCGAGCAGCTCCACAAGGCCATGAAGCGGTACGCCCT*
*GGTGCCGGGCACGATCGCCTTCACCGACGCCCACATCGAGGTCGACATCACCTACGCGGA*
*GTACTTCGAGATGAGCGTGCGCCTGGCCGAGGCCATGAAGCGGTACGGCCTGAACACCAA*
*CCACCGGATCGTGGTGTGCTCGGAGAACAGCCTGCAGTTCTTCATGCCGGTGCTGGGCGC*
*CCTCTTCATCGGCGTGGCCGTCGCCCCGGCGAACGACATCTACAACGAGCGGGAGCTGCT*
*GAACAGCATGGGGATCAGCCAGCCGACCGTGGTGTTCGTGAGCAAGAAGGGCCTGCAGAA*
*GATCCTGAACGTGCAGAAGAAGCTGCCCATCATCCAGAAGATCATCATCATGGACAGCAA*
*GACCGACTACCAGGGCTTCCAGTCGATGTACACGTTCGTGACCAGCCACCTCCCGCCGGG*
*CTTCAACGAGTACGACTTCGTCCCGGAGAGCTTCGACCGGGACAAGACCATCGCCCTGAT*
*CATGAACAGCAGCGGCAGCACCGGCCTGCCGAAGGGGGTGGCCCTGCCGCACCGGACCGC*
*CTGCGTGCGCTTCTCGCACGCCCGGGACCCCATCTTCGGCAACCAGATCATCCCGGACAC*
*CGCCATCCTGAGCGTGGTGCCGTTCCACCACGGCTTCGGCATGTTCACGACCCTGGGCTA*
*CCTCATCTGCGGCTTCCGGGTGGTCCTGATGTACCGGTTCGAGGAGGAGCTGTTCCTGCG*
*GAGCCTGCAGGACTACAAGATCCAGAGCGCGCTGCTCGTGCCGACCCTGTTCAGCTTCTT*
*CGCCAAGAGCACCCTGATCGACAAGTACGACCTGTCGAACCTGCACGAGATCGCCAGCGG*
*GGGCGCCCCGCTGAGCAAGGAGGTGGGCGAGGCCGTGGCCAAGCGGTTCCACCTCCCGGG*
*CATCCGCCAGGGCTACGGCCTGACCGAGACCACGAGCGCGATCCTGATCACCCCCGAGGG*
*GGACGACAAGCCGGGCGCCGTGGGCAAGGTGGTCCCGTTCTTCGAGGCCAAGGTGGTGGA*
*CCTGGACACCGGCAAGACCCTGGGCGTGAACCAGCGGGGCGAGCTGTGCGTGCGGGGGCC*
*GATGATCATGAGCGGCTACGTGAACAACCCGGAGGCCACCAACGCCCTCATCGACAAGGA*
*CGGCTGGCTGCACAGCGGCGACATCGCCTACTGGGACGAGGACGAGCACTTCTTCATCGT*
*CGACCGGCTGAAGTCGCTGATCAAGTACAAGGGCTACCAGGTGGCGCCGGCCGAGCTGGA*
*GAGCATCCTGCTCCAGCACCCCAACATCTTCGACGCCGGCGTGGCCGGGCTGCCGGACGA*
*CGACGCCGGCGAGCTGCCGGCCGCGGTGGTGGTGCTGGAGCACGGCAAGACCATGACGGA*
*GAAGGAGATCGTCGACTACGTGGCCAGCCAGGTGACCACCGCCAAGAAGCTGCGGGGCGG*
*CGTGGTGTTCGTGGACGAGGTCCCGAAGGGCCTGACCGGGAAGCTCGACGCCCGGAAGAT*
*CCGCGAGATCCTGATCAAGGCCAAGAAGGGCGGCAAGATCGCCGTGTAA*GACTAGTTATA
AGACTGACTAGCCCGATGGGCCTCCCAACGGGCCCTCCTCCCCTCCTTGCACCGAGATTA
ATAAAAAAAAAAAAAAAAAAAAAAAAAAAAAAAAAAAAAAAAAAAAAAAAAAAAAAAAAA
AAAAAATGCATCAAAGGCTCTTTTCAGAGCCACCA

Figure 9

nucleotide sequence of RPL32 – PpLuc(GC) – ag – A64

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAGATCTAAAAAAAAAAAAAA
AAAAAAAAAAAAAAAAAAAAAAAAAAAAAAAAAAAAAAAAAAAAAAAAAA

Figure 10

nucleotide sequence of RPL32 – PpLuc(GC) – ag – A64 – histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAAA
AAAAAAAAAAAAAAAAAAAAAAAAAAAAAAAAAAAAAAAAAAAAATGCATCAAAGGCTCTT
TTCAGAGCCACCA

Figure 11

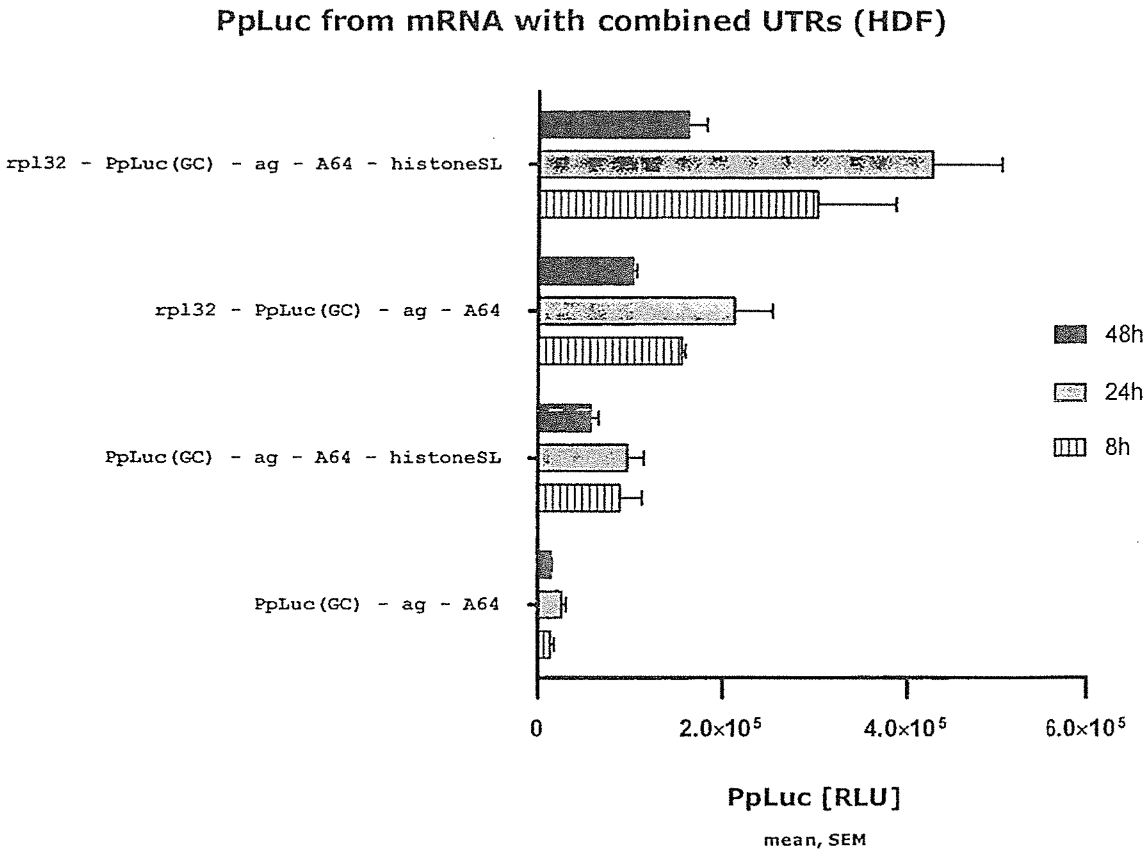
PpLuc from mRNA with combined UTRs (HDF)
rpl32 - PpLuc(GC) - ag - A64 - histoneSL
rpl32 - PpLuc(GC) - ag - A64
PpLuc(GC) - ag - A64 - histoneSL
PpLuc(GC) - ag - A64
48h
24h
8h
0
2.0×10⁵
4.0×10⁵
6.0×10⁵
PpLuc [RLU]
mean, SEM

Figure 12

nucleotide sequence of RPL32 – PpLuc(GC) – albumin7 – A64 – C30 – histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCCCAAAGGCTCTTTTCAGAGCCACCAGAATT

Figure 13

nucleotide sequence of RPL35 – PpLuc(GC) – albumin7 – A64 – C30 – histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCAAAGGCTCTTTTCAGAGCCACCAGAATT

**Figure 14**

nucleotide sequence of RPL21 – PpLuc(GC) – albumin7 – A64 – C30 – histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCAAAGGCTCTTTTCAGAGCCACCAGAATT

Figure 15

nucleotide sequence of atp5a1 – PpLuc(GC) – albumin7 – A64 – C30 – histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTGCATCACATTTAAAAGCATCTCAGCCTACCATGAGAATAAGAGAAAGAAAATG
AAGATCAATAGCTTATTCATCTCTTTTTCTTTTTCGTTGGTGTAAAGCCAACACCCTGTC
TAAAAAACATAAATTTCTTTAATCATTTTGCCTCTTTTCTCTGTGCTTCAATTAATAAAA
AATGGAAAGAACCTAGATCTAAAAAAAAAAAAAAAAAAAAAAAAAAAAAAAAAAAAAAAA
AAAAAAAAAAAAAAAAAAAAAAAATGCATCCCCCCCCCCCCCCCCCCCCCCCCCCCCCC
AAAGGCTCTTTTCAGAGCCACCAGAATT

Figure 16

nucleotide sequence of HSD17B4 – PpLuc(GC) – albumin7 – A64 – C30 – histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTGCATCA
CATTTAAAAGCATCTCAGCCTACCATGAGAATAAGAGAAAGAAAATGAAGATCAATAGCT
TATTCATCTCTTTTTCTTTTTCGTTGGTGTAAAGCCAACACCCTGTCTAAAAAACATAAA
TTTCTTTAATCATTTTGCCTCTTTTCTCTGTGCTTCAATTAATAAAAAATGGAAAGAACC
TAGATCTAAAAAAAAAAAAAAAAAAAAAAAAAAAAAAAAAAAAAAAAAAAAAAAAAAAAAA
AAAAAAAAAAATGCATCCCCCCCCCCCCCCCCCCCCCCCCCCCCCCCAAAGGCTCTTTTC
AGAGCCACCAGAATT

**Figure 17**

nucleotide sequence of AIG1 – PpLuc(GC) – albumin7 – A64 – C30 – histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTGCATCACATTTAAAAGCATCTCAGCCTACCATGAGAA
TAAGAGAAAGAAAATGAAGATCAATAGCTTATTCATCTCTTTTTCTTTTTCGTTGGTGTA
AAGCCAACACCCTGTCTAAAAAACATAAATTTCTTTAATCATTTTGCCTCTTTTCTCTGT
GCTTCAATTAATAAAAAATGGAAAGAACCTAGATCTAAAAAAAAAAAAAAAAAAAAAAAA
AAAAAAAAAAAAAAAAAAAAAAAAAAAAAAAAAAAAAAAATGCATCCCCCCCCCCCCCCC
CCCCCCCCCCCCCCCAAAGGCTCTTTTCAGAGCCACCAGAATT

# Figure 18

nucleotide sequence of COX6C – PpLuc(GC) – albumin7 – A64 – C30 – histoneSL

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTGCATCACATTTA
AAAGCATCTCAGCCTACCATGAGAATAAGAGAAAGAAAATGAAGATCAATAGCTTATTCA
TCTCTTTTTCTTTTTCGTTGGTGTAAAGCCAACACCCTGTCTAAAAAACATAAATTTCTT
TAATCATTTTGCCTCTTTTCTCTGTGCTTCAATTAATAAAAAATGGAAAGAACCTAGATC
TAAAAAAAAAAAAAAAAAAAAAAAAAAAAAAAAAAAAAAAAAAAAAAAAAAAAAAAAAAA
AAAAATGCATCCCCCCCCCCCCCCCCCCCCCCCCCCCCCCCAAAGGCTCTTTTCAGAGCC
ACCAGAATT

**Figure 19**

nucleotide sequence of ASAH1 – PpLuc(GC) – albumin7 – A64 – C30 – histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT

**Figure 20**

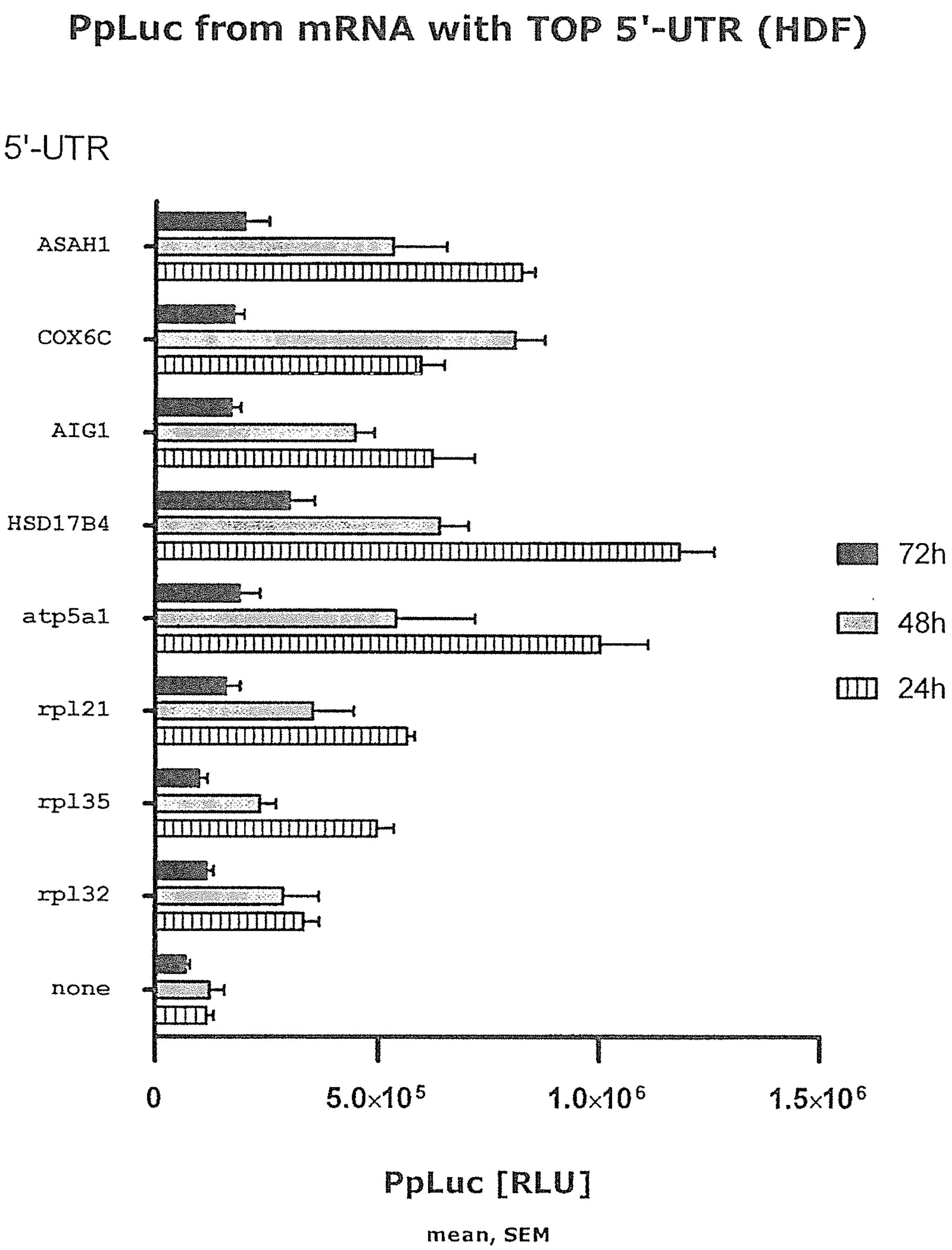
PpLuc from mRNA with TOP 5'-UTR (HDF)
5'-UTR
ASAH1
COX6C
AIG1
HSD17B4
atp5a1
rpl21
rpl35
rpl32
none
72h
48h
24h
0
5.0×10^5
1.0×10^6
1.5×10^6
PpLuc [RLU]
mean, SEM

Figure 21

nucleotide sequence of RPL35 – PpLuc(GC) – ag – A64

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAA

Figure 22

nucleotide sequence of rpl21 – PpLuc(GC) – ag – A64

GGGGCCGGAACCGCCATCTTCCAGTAATTCGCCAAAAAGCTTGAGG*ATGGAGGACGCCAA*
*GAACATCAAGAAGGGCCCGGCGCCCTTCTACCCGCTGGAGGACGGGACCGCCGGCGAGCA*
*GCTCCACAAGGCCATGAAGCGGTACGCCCTGGTGCCGGGCACGATCGCCTTCACCGACGC*
*CCACATCGAGGTCGACATCACCTACGCGGAGTACTTCGAGATGAGCGTGCGCCTGGCCGA*
*GGCCATGAAGCGGTACGGCCTGAACACCAACCACCGGATCGTGGTGTGCTCGGAGAACAG*
*CCTGCAGTTCTTCATGCCGGTGCTGGGCGCCCTCTTCATCGGCGTGGCCGTCGCCCCGGC*
*GAACGACATCTACAACGAGCGGGAGCTGCTGAACAGCATGGGGATCAGCCAGCCGACCGT*
*GGTGTTCGTGAGCAAGAAGGGCCTGCAGAAGATCCTGAACGTGCAGAAGAAGCTGCCCAT*
*CATCCAGAAGATCATCATCATGGACAGCAAGACCGACTACCAGGGCTTCCAGTCGATGTA*
*CACGTTCGTGACCAGCCACCTCCCGCCGGGCTTCAACGAGTACGACTTCGTCCCGGAGAG*
*CTTCGACCGGGACAAGACCATCGCCCTGATCATGAACAGCAGCGGCAGCACCGGCCTGCC*
*GAAGGGGGTGGCCCTGCCGCACCGGACCGCCTGCGTGCGCTTCTCGCACGCCCGGGACCC*
*CATCTTCGGCAACCAGATCATCCCGGACACCGCCATCCTGAGCGTGGTGCCGTTCCACCA*
*CGGCTTCGGCATGTTCACGACCCTGGGCTACCTCATCTGCGGCTTCCGGGTGGTCCTGAT*
*GTACCGGTTCGAGGAGGAGCTGTTCCTGCGGAGCCTGCAGGACTACAAGATCCAGAGCGC*
*GCTGCTCGTGCCGACCCTGTTCAGCTTCTTCGCCAAGAGCACCCTGATCGACAAGTACGA*
*CCTGTCGAACCTGCACGAGATCGCCAGCGGGGGCGCCCCGCTGAGCAAGGAGGTGGGCGA*
*GGCCGTGGCCAAGCGGTTCCACCTCCCGGGCATCCGCCAGGGCTACGGCCTGACCGAGAC*
*CACGAGCGCGATCCTGATCACCCCCGAGGGGGACGACAAGCCGGGCGCCGTGGGCAAGGT*
*GGTCCCGTTCTTCGAGGCCAAGGTGGTGGACCTGGACACCGGCAAGACCCTGGGCGTGAA*
*CCAGCGGGGCGAGCTGTGCGTGCGGGGGCCGATGATCATGAGCGGCTACGTGAACAACCC*
*GGAGGCCACCAACGCCCTCATCGACAAGGACGGCTGGCTGCACAGCGGCGACATCGCCTA*
*CTGGGACGAGGACGAGCACTTCTTCATCGTCGACCGGCTGAAGTCGCTGATCAAGTACAA*
*GGGCTACCAGGTGGCGCCGGCCGAGCTGGAGAGCATCCTGCTCCAGCACCCCAACATCTT*
*CGACGCCGGCGTGGCCGGGCTGCCGGACGACGACGCCGGCGAGCTGCCGGCCGCGGTGGT*
*GGTGCTGGAGCACGGCAAGACCATGACGGAGAAGGAGATCGTCGACTACGTGGCCAGCCA*
*GGTGACCACCGCCAAGAAGCTGCGGGGCGGCGTGGTGTTCGTGGACGAGGTCCCGAAGGG*
*CCTGACCGGGAAGCTCGACGCCCGGAAGATCCGCGAGATCCTGATCAAGGCCAAGAAGGG*
*CGGCAAGATCGCCGTGTAA*GACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAA

Figure 23

nucleotide sequence of atp5a1 – PpLuc(GC) – ag – A64

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTTATAAGACTGACTAGCCCGATGGGCCTCCCAACGGGCCCTCCTCCCCTCCTTG
CACCGAGATTAATAAAAAAAAAAAAAAAAAAAAAAAAAAAAAAAAAAAAAAAAAAAAAA
AAAAAAAAAAAAAAAAA

Figure 24

nucleotide sequence of HSD17B4 – PpLuc(GC) – ag – A64

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTTATAAG
ACTGACTAGCCCGATGGGCCTCCCAACGGGCCCTCCTCCCCTCCTTGCACCGAGATTAAT
AAAAAAAAAAAAAAAAAAAAAAAAAAAAAAAAAAAAAAAAAAAAAAAAAAAAAAAAAAAA
AAAA

Figure 25

nucleotide sequence of AIG1 – PpLuc(GC) – ag – A64

GGGCCGCCCAGCCGGTCCAGGCCTCTGGCGAACAAGCTTGAGG*ATGGAGGACGCCAAGAA*
*CATCAAGAAGGGCCCGGCGCCCTTCTACCCGCTGGAGGACGGGACCGCCGGCGAGCAGCT*
*CCACAAGGCCATGAAGCGGTACGCCCTGGTGCCGGGCACGATCGCCTTCACCGACGCCCA*
*CATCGAGGTCGACATCACCTACGCGGAGTACTTCGAGATGAGCGTGCGCCTGGCCGAGGC*
*CATGAAGCGGTACGGCCTGAACACCAACCACCGGATCGTGGTGTGCTCGGAGAACAGCCT*
*GCAGTTCTTCATGCCGGTGCTGGGCGCCCTCTTCATCGGCGTGGCCGTCGCCCCGGCGAA*
*CGACATCTACAACGAGCGGGAGCTGCTGAACAGCATGGGGATCAGCCAGCCGACCGTGGT*
*GTTCGTGAGCAAGAAGGGCCTGCAGAAGATCCTGAACGTGCAGAAGAAGCTGCCCATCAT*
*CCAGAAGATCATCATCATGGACAGCAAGACCGACTACCAGGGCTTCCAGTCGATGTACAC*
*GTTCGTGACCAGCCACCTCCCGCCGGGCTTCAACGAGTACGACTTCGTCCCGGAGAGCTT*
*CGACCGGGACAAGACCATCGCCCTGATCATGAACAGCAGCGGCAGCACCGGCCTGCCGAA*
*GGGGGTGGCCCTGCCGCACCGGACCGCCTGCGTGCGCTTCTCGCACGCCCGGGACCCCAT*
*CTTCGGCAACCAGATCATCCCGGACACCGCCATCCTGAGCGTGGTGCCGTTCCACCACGG*
*CTTCGGCATGTTCACGACCCTGGGCTACCTCATCTGCGGCTTCCGGGTGGTCCTGATGTA*
*CCGGTTCGAGGAGGAGCTGTTCCTGCGGAGCCTGCAGGACTACAAGATCCAGAGCGCGCT*
*GCTCGTGCCGACCCTGTTCAGCTTCTTCGCCAAGAGCACCCTGATCGACAAGTACGACCT*
*GTCGAACCTGCACGAGATCGCCAGCGGGGGCGCCCCGCTGAGCAAGGAGGTGGGCGAGGC*
*CGTGGCCAAGCGGTTCCACCTCCCGGGCATCCGCCAGGGCTACGGCCTGACCGAGACCAC*
*GAGCGCGATCCTGATCACCCCCGAGGGGGACGACAAGCCGGGCGCCGTGGGCAAGGTGGT*
*CCCGTTCTTCGAGGCCAAGGTGGTGGACCTGGACACCGGCAAGACCCTGGGCGTGAACCA*
*GCGGGGCGAGCTGTGCGTGCGGGGGCCGATGATCATGAGCGGCTACGTGAACAACCCGGA*
*GGCCACCAACGCCCTCATCGACAAGGACGGCTGGCTGCACAGCGGCGACATCGCCTACTG*
*GGACGAGGACGAGCACTTCTTCATCGTCGACCGGCTGAAGTCGCTGATCAAGTACAAGGG*
*CTACCAGGTGGCGCCGGCCGAGCTGGAGAGCATCCTGCTCCAGCACCCCAACATCTTCGA*
*CGCCGGCGTGGCCGGGCTGCCGGACGACGACGCCGGCGAGCTGCCGGCCGCGGTGGTGGT*
*GCTGGAGCACGGCAAGACCATGACGGAGAAGGAGATCGTCGACTACGTGGCCAGCCAGGT*
*GACCACCGCCAAGAAGCTGCGGGGCGGCGTGGTGTTCGTGGACGAGGTCCCGAAGGGCCT*
*GACCGGGAAGCTCGACGCCCGGAAGATCCGCGAGATCCTGATCAAGGCCAAGAAGGGCGG*
*CAAGATCGCCGTGTAA*GACTAGTTATAAGACTGACTAGCCCGATGGGCCTCCCAACGGGC
CCTCCTCCCCTCCTTGCACCGAGATTAATAAAAAAAAAAAAAAAAAAAAAAAAAAAAAAA
AAAAAAAAAAAAAAAAAAAAAAAAAAAAAAAAA

Figure 26

nucleotide sequence of COX6C – PpLuc(GC) – ag – A64

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTTATAAGACTGAC
TAGCCCGATGGCCTCCCAACGGCCCTCCTCCCCTCCTTGCACCGAGATTAATAAAAAA
AAAAAAAAAAAAAAAAAAAAAAAAAAAAAAAAAAAAAAAAAAAAAAAAAAAAAAAAAA

Figure 27

nucleotide sequence of ASAH1 – PpLuc(GC) – ag – A64

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGCCCTCCTCCCCTCCTTGCACCGAGATTAATAAAAAAAAAAAAAAAAAAAAA
AAAAAAAAAAAAAAAAAAAAAAAAAAAAAAAAAAAAAAAAAAAA

Figure 28

nucleotide sequence of RPL35 – PpLuc(GC) – ag – A64 – histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAATGCATCAAAGGCTCTTTTCAGAGC
CACCA

Figure 29

nucleotide sequence of rpl21 – PpLuc(GC) – ag – A64 – histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAATGCATCAAAGGCTCTTTTCAGAGC
CACCA

Figure 30

nucleotide sequence of atp5a1 – PpLuc(GC) – ag – A64 – histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTTATAAGACTGACTAGCCCGATGGGCCTCCCAACGGGCCCTCCTCCCCTCCTTG
CACCGAGATTAATAAAAAAAAAAAAAAAAAAAAAAAAAAAAAAAAAAAAAAAAAAAAAA
AAAAAAAAAAAAAAAAATGCATCAAAGGCTCTTTTCAGAGCCACCA

Figure 31

nucleotide sequence of HSD17B4 – PpLuc(GC) – ag – A64 – histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTTATAAG
ACTGACTAGCCCGATGGGCCTCCCAACGGGCCCTCCTCCCCTCCTTGCACCGAGATTAAT
AAAAAAAAAAAAAAAAAAAAAAAAAAAAAAAAAAAAAAAAAAAAAAAAAAAAAAAAAAAA
AAAATGCATCAAAGGCTCTTTTCAGAGCCACCA

Figure 32

nucleotide sequence of AIG1 – PpLuc(GC) – ag – A64 – histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTTATAAGACTGACTAGCCCGATGGGCCTCCCAACGGGC
CCTCCTCCCCTCCTTGCACCGAGATTAATAAAAAAAAAAAAAAAAAAAAAAAAAAAAAAA
AAAAAAAAAAAAAAAAAAAAAAAAAAAAAAAAATGCATCAAAGGCTCTTTTCAGAGCCAC
CA

Figure 33

nucleotide sequence of COX6C – PpLuc(GC) – ag – A64 – histoneSL

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTTATAAGACTGAC
TAGCCCGATGGGCCTCCCAACGGGCCCTCCTCCCCTCCTTGCACCGAGATTAATAAAAAA
AAAAAAAAAAAAAAAAAAAAAAAAAAAAAAAAAAAAAAAAAAAAAAAAAAAAAAAAAA
TGCATCAAAGGCTCTTTTCAGAGCCACCA

Figure 34

nucleotide sequence of ASAH1 – PpLuc(GC) – ag – A64 – histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAATGCATCAAAGGCTCTT
TTCAGAGCCACCA

Figure 35

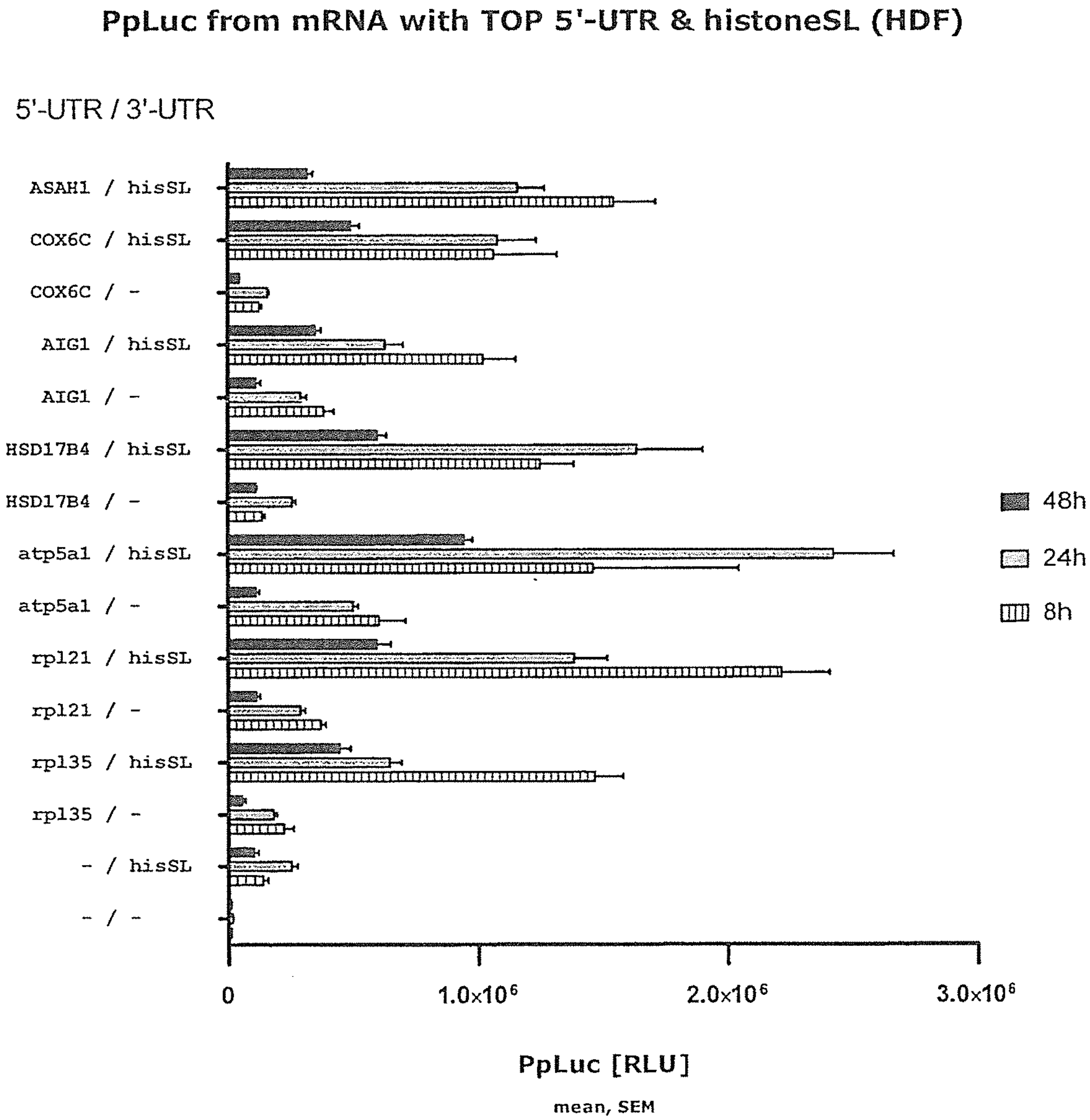
PpLuc from mRNA with TOP 5'-UTR & histoneSL (HDF)
5'-UTR / 3'-UTR
ASAH1 / hisSL
COX6C / hisSL
COX6C / -
AIG1 / hisSL
AIG1 / -
HSD17B4 / hisSL
HSD17B4 / -
atp5a1 / hisSL
atp5a1 / -
rpl21 / hisSL
rpl21 / -
rpl35 / hisSL
rpl35 / -
- / hisSL
- / -
48h
24h
8h
0
1.0×10^6
2.0×10^6
3.0×10^6
PpLuc [RLU]
mean, SEM

Figure 36

nucleotide sequence of mRPL21 – PpLuc(GC) – albumin7 – A64 – C30 – histoneSL

GGGGCCGCCGCAGCCATCTTCCAGTAACTCGCCAAAAAGCTTGAGG*ATGGAGGACGCCAA*
*GAACATCAAGAAGGGCCCGGCGCCCTTCTACCCGCTGGAGGACGGGACCGCCGGCGAGCA*
*GCTCCACAAGGCCATGAAGCGGTACGCCCTGGTGCCGGGCACGATCGCCTTCACCGACGC*
*CCACATCGAGGTCGACATCACCTACGCGGAGTACTTCGAGATGAGCGTGCGCCTGGCCGA*
*GGCCATGAAGCGGTACGGCCTGAACACCAACCACCGGATCGTGGTGTGCTCGGAGAACAG*
*CCTGCAGTTCTTCATGCCGGTGCTGGGCGCCCTCTTCATCGGCGTGGCCGTCGCCCCGGC*
*GAACGACATCTACAACGAGCGGGAGCTGCTGAACAGCATGGGGATCAGCCAGCCGACCGT*
*GGTGTTCGTGAGCAAGAAGGGCCTGCAGAAGATCCTGAACGTGCAGAAGAAGCTGCCCAT*
*CATCCAGAAGATCATCATCATGGACAGCAAGACCGACTACCAGGGCTTCCAGTCGATGTA*
*CACGTTCGTGACCAGCCACCTCCCGCCGGGCTTCAACGAGTACGACTTCGTCCCGGAGAG*
*CTTCGACCGGGACAAGACCATCGCCCTGATCATGAACAGCAGCGGCAGCACCGGCCTGCC*
*GAAGGGGGTGGCCCTGCCGCACCGGACCGCCTGCGTGCGCTTCTCGCACGCCCGGGACCC*
*CATCTTCGGCAACCAGATCATCCCGGACACCGCCATCCTGAGCGTGGTGCCGTTCCACCA*
*CGGCTTCGGCATGTTCACGACCCTGGGCTACCTCATCTGCGGCTTCCGGGTGGTCCTGAT*
*GTACCGGTTCGAGGAGGAGCTGTTCCTGCGGAGCCTGCAGGACTACAAGATCCAGAGCGC*
*GCTGCTCGTGCCGACCCTGTTCAGCTTCTTCGCCAAGAGCACCCTGATCGACAAGTACGA*
*CCTGTCGAACCTGCACGAGATCGCCAGCGGGGGCGCCCCGCTGAGCAAGGAGGTGGGCGA*
*GGCCGTGGCCAAGCGGTTCCACCTCCCGGGCATCCGCCAGGGCTACGGCCTGACCGAGAC*
*CACGAGCGCGATCCTGATCACCCCCGAGGGGGACGACAAGCCGGGCGCCGTGGGCAAGGT*
*GGTCCCGTTCTTCGAGGCCAAGGTGGTGGACCTGGACACCGGCAAGACCCTGGGCGTGAA*
*CCAGCGGGGCGAGCTGTGCGTGCGGGGGCCGATGATCATGAGCGGCTACGTGAACAACCC*
*GGAGGCCACCAACGCCCTCATCGACAAGGACGGCTGGCTGCACAGCGGCGACATCGCCTA*
*CTGGGACGAGGACGAGCACTTCTTCATCGTCGACCGGCTGAAGTCGCTGATCAAGTACAA*
*GGGCTACCAGGTGGCGCCGGCCGAGCTGGAGAGCATCCTGCTCCAGCACCCCAACATCTT*
*CGACGCCGGCGTGGCCGGGCTGCCGGACGACGACGCCGGCGAGCTGCCGGCCGCGGTGGT*
*GGTGCTGGAGCACGGCAAGACCATGACGGAGAAGGAGATCGTCGACTACGTGGCCAGCCA*
*GGTGACCACCGCCAAGAAGCTGCGGGGCGGCGTGGTGTTCGTGGACGAGGTCCCGAAGGG*
*CCTGACCGGGAAGCTCGACGCCCGGAAGATCCGCGAGATCCTGATCAAGGCCAAGAAGGG*
*CGGCAAGATCGCCGTGTAA*GACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCCAAAGGCTCTTTTCAGAGCCACCAGAATT

Figure 37

nucleotide sequence of mRPL35A – PpLuc(GC) – albumin7 – A64 – C30 – histoneSL

GGGCCATCTTGGCGCCTGTGGAGGCCTGCTGGGAACAGGACTTCTAACAGCAAGTAAGCT
TGAGGATGGAGGACGCCAAGAACATCAAGAAGGGCCCGGCGCCCTTCTACCCGCTGGAGG
ACGGGACCGCCGGCGAGCAGCTCCACAAGGCCATGAAGCGGTACGCCCTGGTGCCGGGCA
CGATCGCCTTCACCGACGCCCACATCGAGGTCGACATCACCTACGCGGAGTACTTCGAGA
TGAGCGTGCGCCTGGCCGAGGCCATGAAGCGGTACGGCCTGAACACCAACCACCGGATCG
TGGTGTGCTCGGAGAACAGCCTGCAGTTCTTCATGCCGGTGCTGGGCGCCCTCTTCATCG
GCGTGGCCGTCGCCCCGGCGAACGACATCTACAACGAGCGGGAGCTGCTGAACAGCATGG
GGATCAGCCAGCCGACCGTGGTGTTCGTGAGCAAGAAGGGCCTGCAGAAGATCCTGAACG
TGCAGAAGAAGCTGCCCATCATCCAGAAGATCATCATCATGGACAGCAAGACCGACTACC
AGGGCTTCCAGTCGATGTACACGTTCGTGACCAGCCACCTCCCGCCGGGCTTCAACGAGT
ACGACTTCGTCCCGGAGAGCTTCGACCGGGACAAGACCATCGCCCTGATCATGAACAGCA
GCGGCAGCACCGGCCTGCCGAAGGGGGTGGCCCTGCCGCACCGGACCGCCTGCGTGCGCT
TCTCGCACGCCCGGGACCCCATCTTCGGCAACCAGATCATCCCGGACACCGCCATCCTGA
GCGTGGTGCCGTTCCACCACGGCTTCGGCATGTTCACGACCCTGGGCTACCTCATCTGCG
GCTTCCGGGTGGTCCTGATGTACCGGTTCGAGGAGGAGCTGTTCCTGCGGAGCCTGCAGG
ACTACAAGATCCAGAGCGCGCTGCTCGTGCCGACCCTGTTCAGCTTCTTCGCCAAGAGCA
CCCTGATCGACAAGTACGACCTGTCGAACCTGCACGAGATCGCCAGCGGGGGCGCCCCGC
TGAGCAAGGAGGTGGGCGAGGCCGTGGCCAAGCGGTTCCACCTCCCGGGCATCCGCCAGG
GCTACGGCCTGACCGAGACCACGAGCGCGATCCTGATCACCCCCGAGGGGGACGACAAGC
CGGGCGCCGTGGGCAAGGTGGTCCCGTTCTTCGAGGCCAAGGTGGTGGACCTGGACACCG
GCAAGACCCTGGGCGTGAACCAGCGGGGCGAGCTGTGCGTGCGGGGGCCGATGATCATGA
GCGGCTACGTGAACAACCCGGAGGCCACCAACGCCCTCATCGACAAGGACGGCTGGCTGC
ACAGCGGCGACATCGCCTACTGGGACGAGGACGAGCACTTCTTCATCGTCGACCGGCTGA
AGTCGCTGATCAAGTACAAGGGCTACCAGGTGGCGCCGGCCGAGCTGGAGAGCATCCTGC
TCCAGCACCCCAACATCTTCGACGCCGGCGTGGCCGGGCTGCCGGACGACGACGCCGGCG
AGCTGCCGGCCGCGGTGGTGGTGCTGGAGCACGGCAAGACCATGACGGAGAAGGAGATCG
TCGACTACGTGGCCAGCCAGGTGACCACCGCCAAGAAGCTGCGGGGCGGCGTGGTGTTCG
TGGACGAGGTCCCGAAGGGCCTGACCGGGAAGCTCGACGCCCGGAAGATCCGCGAGATCC
TGATCAAGGCCAAGAAGGGCGGCAAGATCGCCGTGTAAGACTAGTGCATCACATTTAAAA
GCATCTCAGCCTACCATGAGAATAAGAGAAAGAAAATGAAGATCAATAGCTTATTCATCT
CTTTTTCTTTTTCGTTGGTGTAAAGCCAACACCCTGTCTAAAAAACATAAATTTCTTTAA
TCATTTTGCCTCTTTTCTCTGTGCTTCAATTAATAAAAAATGGAAAGAACCTAGATCTAA
AAAAAAAAAAAAAAAAAAAAAAAAAAAAAAAAAAAAAAAAAAAAAAAAAAAAAAAAAAAA
AATGCATCCCCCCCCCCCCCCCCCCCCCCCCCCCCCCCAAAGGCTCTTTTCAGAGCCACC
AGAATT

Figure 38

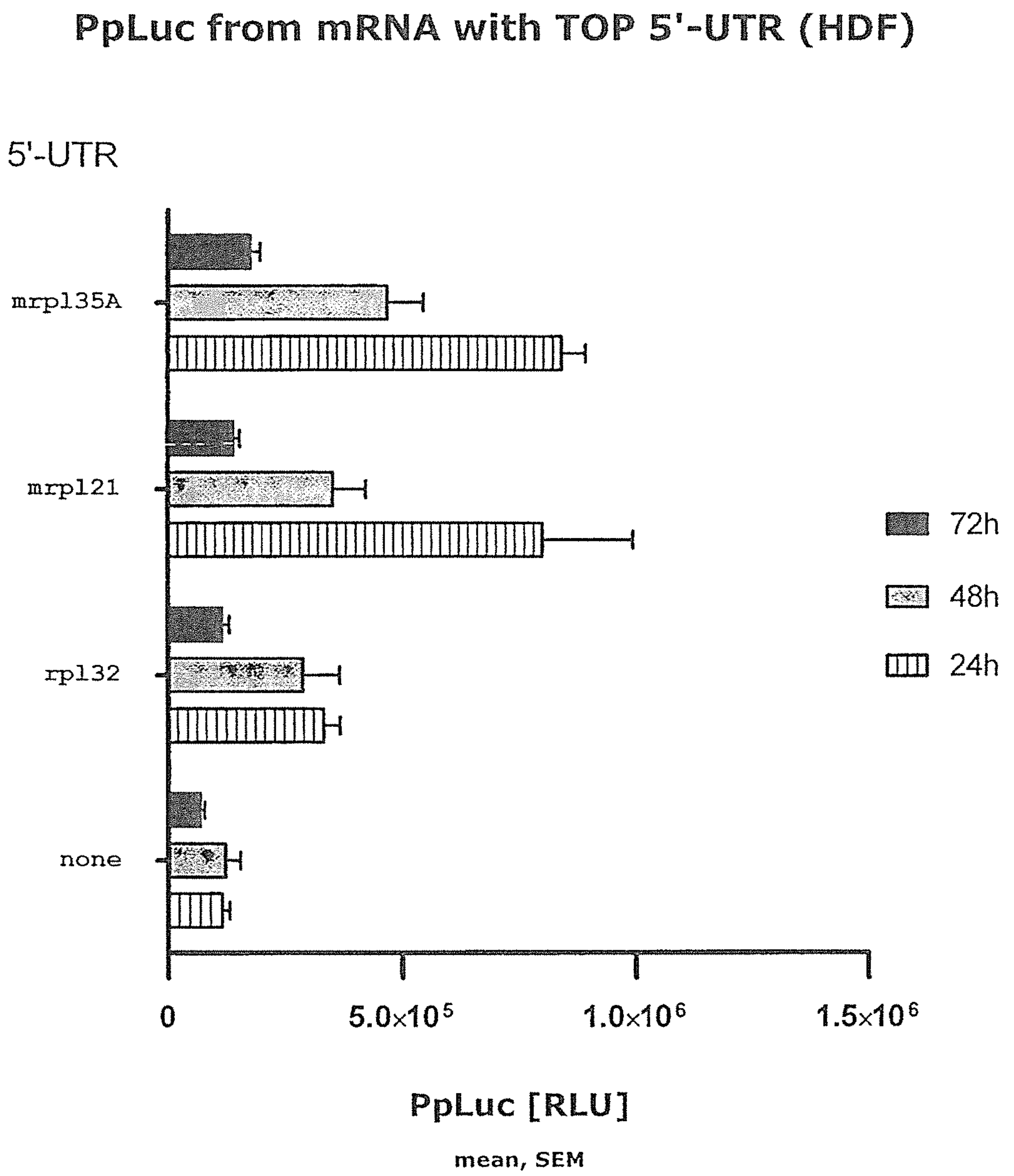
PpLuc from mRNA with TOP 5'-UTR (HDF)
5'-UTR
mrpl35A
mrpl21
rpl32
none
72h
48h
24h
0
5.0×10⁵
1.0×10⁶
1.5×10⁶
PpLuc [RLU]
mean, SEM

Figure 39

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02098443 A
- WO 0112824 A

### Non-patent literature cited in the description


- **FRIEDEL et al.** Conserved principles of mammalian transcriptional regulation revealed by RNA half-life. *Nucleic Acid Research,* 2009, 1-12
- **JOHNSON et al.** Newly synthesized RNA: Simultaneous measurement in intact cells of transcription rates and RNA stability of insulin-like growth factor I, actin, and albumin in growth hormone-stimulated hepatocytes. *Proc. Natl. Acad. Sci.,* 1991, vol. 88, 5287-5291
- **DOMINSKI, Z. ; W. F. MARZLUFF.** *Gene,* 2007, vol. 396 (2), 373-90
- **DÁVILA LÓPEZ, M. ; SAMUELSSON, T.** *RNA (New York, N.Y.),* 2008, vol. 14 (1), 1-10
- **PANDEY, N. B. et al.** *Molecular and Cellular Biology,* 1994, vol. 14 (3), 1709-1720
- **WILLIAMS, A. S. ; MARZLUFF, W. F.** *Nucleic Acids Research,* 1995, vol. 23 (4), 654-662
- **SANCHEZ, R. ; W. F. MARZLUFF.** *Mol Cell Biol,* 2004, vol. 24 (6), 2513-25
- **WHITELAW, E. et al.** *Nucleic Acids Research,* 1986, vol. 14 (17), 7059-7070
- **PANDEY, N. B. ; MARZLUFF, W. F.** *Molecular and Cellular Biology,* 1987, vol. 7 (12), 4557-4559
- **PANDEY, N. B. et al.** *Nucleic Acids Research,* 1990, vol. 18 (11), 3161-3170
- **RODGERS et al.** Regulated α-globin mRNA decay is a cytoplasmic event proceeding through 3'-to-5' exosome-dependent decapping. *RNA,* 2002, vol. 8, 1526-1537
- **WANG et al.** An mRNA stability complex functions with poly(A)-binding protein to stabilize mRNA in vitro. *Molecular and Cellular biology,* July 1999, vol. 19 (7), 4552-4560
- **CHAKRABARTI P. et al.** The Mammalian Target of Rapamycin Complex 1 Regulates Leptin Biosynthesis in Adipocytes at the Level of Translation: The Role of the 5'-Untranslated Region in the Expression of Leptin Messenger Ribonucleic Acid;. *Molecular Endocrinology,* 2008, vol. 22 (10), 2260-2267
- **LADEVAIA et al.** All translation elongation factors and the e, f, and h subunits of translation initiation factor 3 are encoded by 5'-terminal oligopyrimidine (TOP) mRNAs. *RNA,* 2008, vol. 14, 1730-1736
- **AVNI et al.** Vertebrate mRNAs with a 5'-terminal pyrimidine tract are Candidates for translational repression in quiescent cells: characterization of the translational cis-regulatory element. *Molecular and Cellular Biology,* 1994, 3822-3833
- Translational Control of Ribosomal Protein mRNAs in Eukaryotes, Translational Control. **MEYUHAS et al.** Cold Spring Harbor Monograph Archive. Cold Spring Harbor Laboratory Press, 1996, 363-388
- **ZHU J.** Binding of the La autoantigen to the 5' untranslated region of a chimeric human translation elongation factor 1A reporter mRNA inhibits translation in vitro. *Biochemica et Biophysica Acta,* 2001, vol. 1521, 19-29
- **LEDDA, M. et al.** Effect of 3' UTR length on the translational regulation of 5'-terminal oligopyrimidine mRNAs. *Gene,* 2005, vol. 344, 213-220
- **AKASHI.** *Curr. Opin. Genet. Dev.,* 2001, vol. 11 (6), 660-666